(19)

Europäisches
Patentamt
European
Patent Office
Office européen
des brevets

(11) **EP 4 697 021 A2**

(12) **EUROPEAN PATENT APPLICATION**

(43) Date of publication:
**18.02.2026 Bulletin 2026/08**

(21) Application number: **25205653.6**

(22) Date of filing: **12.02.2019**

(51) International Patent Classification (IPC):
***G01N 33/53*** (2006.01)

(52) Cooperative Patent Classification (CPC):
**C12Q 1/6883;** C12Q 2600/158; C12Q 2600/178

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.02.2018 JP 2018023283**
**26.04.2018 JP 2018085652**
**24.07.2018 JP 2018138487**

(62) Document number(s) of the earlier application(s) in accordance with Art. 76 EPC:
**24217565.1 / 4 498 086**
**23216032.5 / 4 328 587**
**19754972.8 / 3 754 019**

(71) Applicants:
• **Toray Industries, Inc.**
**Tokyo 103-8666 (JP)**
• **National Center for Geriatrics and Gerontology**
**Obu-shi, Aichi 474-8511 (JP)**

(72) Inventors:
• **SUZUKI, Kana**
**Kanagawa, 248-8555 (JP)**
• **YOSHIMOTO, Makiko**
**Kanagawa, 248-8555 (JP)**
• **KAWAUCHI, Junpei**
**Kanagawa, 248-8555 (JP)**
• **SUDO, Hiroko**
**Kanagawa, 248-8555 (JP)**

• **KIDA, Yuho**
**Kanagawa, 248-8555 (JP)**
• **KOZONO, Satoko**
**Kanagawa, 248-8555 (JP)**
• **KONDOU, Satoshi**
**Kanagawa, 248-8555 (JP)**
• **NIIDA, Shumpei**
**Aichi, 474-8511 (JP)**
• **ASANOMI, Yuya**
**Aichi, 474-8511 (JP)**
• **SHIGEMIZU, Daichi**
**Aichi, 474-8511 (JP)**
• **SAKURAI, Takashi**
**Aichi, 474-8511 (JP)**

(74) Representative: **Mewburn Ellis LLP**
**Aurora Building**
**Counterslip**
**Bristol BS1 6BX (GB)**

Remarks:
•The complete document including Reference Table(s) and the Sequence Listing(s) can be downloaded from the EPO website
•This application was filed on 30-09-2025 as a divisional application to the application mentioned under INID code 62.

(54) **KIT OR DEVICE AND METHOD FOR DETECTING DEMENTIA**

(57) An embodiment according to the present invention provides a kit or device for detection of dementia, and a method for detecting dementia. An embodiment according to the present invention relates to: a kit or device for detection of dementia, including a nucleic acid(s) capable of specifically binding to an miRNA(s) or a complementary strand(s) thereof in a sample from a subject; and a method for detecting dementia, including measuring the miRNA(s) *in vitro.*

Fig. 1

EP 4 697 021 A2

**Description**

TECHNICAL FIELD

The present invention relates to a kit or device for detection of dementia, comprising a nucleic acid capable of specifically binding to a specific miRNA or a complementary strand thereof, which is used for examining the presence or absence of dementia in a subject, and a method for detecting dementia, comprising measuring the expression level of the miRNA.

BACKGROUND ART

In future estimation of the number of dementia patients and the prevalence in the elderly aged 65 or over, the number of dementia patients in Japan is estimated to reach about 7 million in 2025 (corresponding to 1/5 of the elderly aged 65 or over) while the number was 4.62 million in 2012 (corresponding to 1/7 of the elderly aged 65 or over (a prevalence of 15.0%)) (the 2016 White Paper on Aged Society by Cabinet Office). Meanwhile, it was reported that the cost for medical and nursing care of dementia patients reached 14.5 trillion yen in 2014 (including: medical expenses of 1.9 trillion yen (inpatient expenses of about 970.3 billion yen and outpatient expenses of about 941.2 billion yen), nursing expenses of 6.4 trillion yen (at-home care expenses of about 3.5281 trillion yen and at-facility care expenses of 2.9160 trillion yen), and informal care costs of 6.2 trillion yen) ("Estimation of Social Cost for Dementia" by the research team of the Ministry of Health, Labor and Welfare in May, 2015). The number of dementia patients worldwide will have increased to 74.7 million by 2030 and medical costs for dementia are estimated to reach 240 trillion yen (ADI "World Alzheimer's Report 2015"). Dementia, in general, is a progressive disease and is accompanied by irreversible degeneration of brain neurons. Because no therapeutic drug for dementia is available today, it is critical to test/diagnose dementia and implement an early medical intervention for suppressing progress dementia so as to reduce burden on patients/caregivers and social costs.

Currently, dementia is diagnosed by differential diagnosis including medical history, present symptoms, physical finding, a neuropsychological test, a blood test, imaging test, and the like.

As a method for testing dementia by a neuropsychological test, most internationally widely used is Folstein's "Mini-Mental State Examination" (MMSE). This screening test is most recommended from the viewpoint of sensitivity, specificity, convenience, and past data accumulation. MMSE can simply assess, while the total score is 30, multiple cognitive functions such as orientation, retention, attention/calculation, language function, verbal command response, and figure copying. Generally speaking, when the score is 23 or less, it is determined to be suspected of dementia. However, a high score is sometimes exhibited, depending on education history and/or carrier history, even in a case of dementia. Such an interview method is thus just used for screening and does not give a definite diagnosis.

As a method for testing dementia by imaging, development of diagnostic imaging such as CT, MRI, or PET/SPECT has been in progress. Unfortunately, diagnostic imaging requires special equipment and can thus be implemented by limited medical institutions. In addition, in current diagnostic imaging technology, it is difficult to grasp a difference between a pre-dementia stage and a state in which cognitive functions are normal, etc. Accordingly, different image-reading doctors may give different diagnoses. Thus, there is a disadvantage such as lack of objectivity.

A blood test recommended in the Disease Guideline for Dementia Diagnosis is necessary for exclusion diagnosis of internal diseases, in which dementia and dementia-like symptoms are manifested, during diagnosis of degenerative dementia such as Alzheimer's dementia. However, there is no currently available blood test item that can identify dementia. The low concentration level of amyloid protein and the high concentration level of tau in cerebrospinal fluid (CSF) are being utilized as surrogate markers for Alzheimer's dementia. However, the test is highly invasive to patients.

Recently, markers have been developed that can diagnose dementia such as Alzheimer's dementia or neurodegenerative disease.

For instance, a report (Non-Patent Literature 1) shows that use of 6 miRNAs (miR-483-5p, miR-486-5p, miR-30b-5p, miR-200a-3p, miR-502-3p, miR-142-3p) included in plasma enables diagnosis of early Alzheimer's included in mild cognitive impairment (MCI), which is a pre-dementia stage. In addition, a report (Patent Literature 1) shows that after subjects with mild dementia were enrolled, they were given a supplement for alleviating dementia symptoms, and expressions of miRNAs in serum before and after the intake were compared to search for dementia markers. 21 markers (hsa-miR-486-5p, hsa-miR-21-5p, hsa-miR-92a-3p, hsa-miR-451a, hsa-let-7b-5p, hsa-miR-126-3p, hsa-miR-25-3p, hsa-miR-10b-5p, hsa-miR-30e-5p, hsa-miR-140-3p, hsa-miR-101-3p, hsa-miR-222-3p, hsa-miR-10a-5p, hsa-miR-221-3p, hsa-miR-375, hsa-miR-30a-5p, hsa-miR-584-5p, hsa-miR-27b-3p, hsa-miR-484, hsa-miR-125a-5p, hsa-miR-93-5p) were extracted.

miR-206 was identified during search of miRNA targets specific to prevention and treatment of neurodegenerative disease, in particular, Alzheimer's disease (Patent Literature 2). Patent Literature 2 reports that this antisense strand caused an increase in the levels of nerve growth factor BDNF and IGF-1 and synapses were found to regenerate in Alzheimer's disease model mice; and this marker was also applicable to diagnosis.

A report (Patent Literature 3) shows that it was found in a try at diagnosis of Alzheimer's disease that Alzheimer's disease could be diagnosed by measuring the expression levels of 67 kinds of miRNAs (e.g., hsa-miR-1296, hsa-miR-424*, hsa-miR-424, hsa-miR-629) in a sample.

A report (Patent Literature 4) shows that measurement of miRNAs (e.g., miR-7, miR-25, miR-26a) in a nerve tissue and/or synapses in body fluid allows for diagnosis and/or treatment monitoring of mild dementia and Alzheimer's disease.

The invention described in Patent Literature 5 aims to treat and prevent Alzheimer's disease and/or neurofibrillary degeneration due to abnormal tau expression. It reports that after search for miRNAs that bind to 3' UTR of tau mRNA and regulate expression of tau the resulting markers (e.g., miR-185-5p, miR-151-5p) for therapeutic purposes were applicable to diagnosis.

A report (Patent Literature 6) shows that vesicles derived from micro-glial cells present in body fluid were isolated and the expression of miRNAs encapsulated therein were analyzed. 36 miRNAs were identified in order to conduct diagnosis, prediction, or treatment monitoring of neurodegenerative disease or the like.

The invention described in Patent Literature 7 ailed to develop a microfluidic device for detecting a microRNA after hybridization. It reports that cancer-, inflammatory disease-, or dementia-related microRNAs in body fluid including blood, serum, and plasma have been detected, as an example of detection,

Meanwhile, it has been increasingly evident that dementia is sometimes complicated by many disease types. For instance, in a report where the doctors' diagnosis results had been compared to the corresponding pathological diagnosis results, just 14 (3.13%) of 447 patients diagnosed as Alzheimer's dementia by the doctors had Alzheimer's dementia alone after the pathological diagnosis. 366 patients (81.88%) who had Alzheimer's dementia complicated by vascular dementia and/or Lewy body dementia were also included. In addition, 67 (14.98%) of 477 patients diagnosed as Alzheimer's dementia by the doctors had pathologically neither Alzheimer's dementia nor Alzheimer's dementia complicated by non-Alzheimer's dementia, but had dementia different from Alzheimer's dementia, such as vascular dementia, Lewy body dementia, hippocampal atrophy, and so on (Non-Patent Literature 2).

CITATION LIST

Patent Literature

Patent Literature 1: JP Patent Publication (Kokai) No. 2017-184642
Patent Literature 2: U.S. Patent Application Publication No. 2013/0184331
Patent Literature 3: U.S. Patent Application Publication No. 2014/0206777
Patent Literature 4: U.S. Patent Application Publication No. 2014/0120545
Patent Literature 5: U.S. Patent Application Publication No. 2017/0002348
Patent Literature 6: International Publication No. WO 2017/084770
Patent Literature 7: International Publication No. WO 2017/059094

Non-Patent Literature

Non-Patent Literature 1: Siranjeevi N. et al., Oncotarget, 2017 Feb 05, vol.8, No.10, p.16122-16143
Non-Patent Literature 2: Alifiya Kapasi et al., Acta Neuropathol, 2017, vol.134, p.171-186

SUMMARY OF INVENTION

Problem to be Solved by Invention

Because dementia, in general, is a progressive disease and is accompanied by irreversible degeneration of brain neurons as mentioned above, it is essential to find dementia without misidentification and implement a medical intervention to suppress the progress. To prescribe a progress inhibitor or implement a medical intervention such as "Cognicize" for suppressing the progress, a diagnosis method has been desired that can objectively detect, without misidentification, different disease types of dementia regardless of dementia types. However, it is uneasy to diagnose dementia as stated above, because used is a subjective diagnosis method in which a difference in diagnosis is large among doctors. In addition, while majority of dementia is a complication type, when only one of dementia types is diagnosed, the other complicated dementia types are neglected, leading to a possibility of loss of treatment opportunities.

Thus, markers have been sought that can objectively and comprehensively diagnose each dementia type. However, the markers described in the above Non-Patent Literature 1 and Patent Literatures 1 to 7 cannot be said to exert enough performance as markers for diagnosing dementia as described below.

In Non-Patent Literature 1, only Alzheimer's dementia and early Alzheimer's dementia included in mild dementia (MCI) have been validated. Thus, it is unclear whether any dementia disease type other than Alzheimer's dementia can be detected.

In Patent Literature 1, just MCI, which is a pre-dementia stage, has been validated, and it is unclear whether or not dementia can be detected.

In Patent Literature 2, just Alzheimer's model mice and human brain samples have been examined, and blood samples, which can be collected in a low-invasive manner, have not been examined.

In Patent Literature 3, only Alzheimer's dementia is a target, and it is unclear whether any dementia disease type other than Alzheimer's dementia can be detected.

In Patent Literature 4, it was demonstrated discriminatation between MCI and healthy subjects and between MCI and Alzheimer's dementia. However, it was not demonstrated discrimination between healthy subjects and Alzheimer's dementia.

In Patent Literature 5, just brain tissues have been used for validation in Examples, and it is unclear whether or not even blood samples can be likewise used for detection.

In Patent Literature 6, whether or not markers are expressed in micro-glial cells in model mice was examined. However, it is unclear whether or not the markers can be detected using human blood samples.

Patent Literature 7 relates to a diagnostic device using microRNAs in body fluid, and dementia is exemplified together with cancers and inflammatory diseases. Detection of microRNAs by using samples including blood, serum, and plasma is described. However, it is unclear whether or not the markers can be detected using human blood samples because there is no description of Examples.

Collectively, in the past reports regarding dementia markers, samples for just one type of dementia disease were used for validation. Thus, when these markers are used for diagnosis, treatment opportunities may be lost because patients with any other dementia disease type are not found. In addition, in these reports, model mice and/or human brain samples were used for assessment, and validation using human blood was not conducted. As a result, they may be unsuitable for use in diagnosis of dementia.

Further, collection of cerebrospinal fluid as a sample for measurement is not preferable because invasiveness to patients is high. Accordingly, comprehensive dementia markers are desired that allow for detection using blood samples, which can be collected in a low-invasive manner, and can accurately discriminate the presence or absence of dementia. If dementia, in particular, can be found without misidentification, it should be possible to suppress progress of dementia by a medical intervention.

The object of the present invention is to provide a dementia marker that makes it possible to discriminate between dementia and normal cognitive functions (non-dementia) and to diagnose one or more dementia disease types, and to provide a method that makes it possible to objectively and effectively detect the presence or absence of dementia by using a nucleic acid capable of specifically binding to the marker. Specifically, the object is to provide a test method satisfying one or more and preferably all of the 4 points including: 1. there is no difference among medical institutions and among doctors; 2. different dementia types can be comprehensively detected; 3. the detection sensitivity and the specificity for dementia are high; and 4. invasiveness is low.

Means for Solution to Problem

The present inventors have conducted diligent studies to attain the object and completed the present invention by finding novel dementia markers that can be used to detect one or more disease types of dementia selected from Alzheimer's dementia, vascular dementia, Lewy body dementia, normal pressure hydrocephalus, frontotemporal lobar degeneration, or the like.

<Summary of Invention>

Specifically, the present invention includes the following aspects.

(1) A kit for detection of dementia, comprising nucleic acid(s) capable of specifically binding to one or more polynucleotide(s) selected from the group consisting of dementia markers: miR-4274, miR-4272, miR-4728-5p, miR-4443, miR-4506, miR-6773-5p, miR-4662a-5p, miR-3184-3p, miR-4281, miR-320d, miR-6729-3p, miR-5192, miR-6853-5p, miR-1234-3p, miR-1233-3p, miR-4539, miR-3914, miR-4738-5p, miR-548au-3p, miR-1539, miR-4720-3p, miR-365b-5p, miR-4486, miR-1227-5p, miR-4667-5p, miR-6088, miR-6820-5p, miR-4505, miR-548q, miR-4658, miR-450a-5p, miR-1260b, miR-3677-5p, miR-6777-3p, miR-6826-3p, miR-6832-3p, miR-4725-3p, miR-7161-3p, miR-2277-5p, miR-7110-3p, miR-4312, miR-4461, miR-6766-5p, miR-1266-3p, miR-6729-5p, miR-526b-3p, miR-519e-5p, miR-512-5p, miR-5088-5p, miR-1909-3p, miR-6511a-5p, miR-4734, miR-936, miR-1249-3p, miR-6777-5p, miR-4487, miR-3155a, miR-563, miR-4741, miR-6788-5p, miR-4433b-5p, miR-323a-5p, miR-6811-5p, miR-6721-5p, miR-5004-5p, miR-6509-3p, miR-648, miR-3917, miR-6087, miR-1470, miR-586, miR-3150a-5p, miR-105-3p, miR-7973, miR-1914-5p, miR-4749-3p, miR-15b-5p, miR-1289, miR-4433a-5p, miR-3666, miR-3186-3p, miR-4725-5p, miR-4488, miR-4474-3p, miR-6731-3p, miR-4640-3p, miR-202-5p, miR-6816-5p, miR-638, miR-6821-5p, miR-1247-3p, miR-6765-5p, miR-6800-5p, miR-3928-3p, miR-3940-5p, miR-3960, miR-6775-5p, miR-3178, miR-1202, miR-6790-5p, miR-4731-3p, miR-2681-3p, miR-6758-5p, miR-8072, miR-518d-3p, miR-3606-3p, miR-4800-5p, miR-1292-3p, miR-6784-3p, miR-4450, miR-6132, miR-4716-5p, miR-6860, miR-1268b, miR-378d, miR-4701-5p, miR-4329, miR-185-3p, miR-552-3p, miR-1273g-5p, miR-6769b-3p, miR-520a-3p, miR-4524b-5p, miR-4291, miR-6734-3p, miR-143-5p, miR-939-3p, miR-6889-3p, miR-6842-3p, miR-4511, miR-4318, miR-4653-5p, miR-6867-3p, miR-133b, miR-3196, miR-193b-3p, miR-3162-3p, miR-6819-3p, miR-1908-3p, miR-6786-5p, miR-3648, miR-4513, miR-3652, miR-4640-5p, miR-6871-5p, miR-7845-5p, miR-3138, miR-6884-5p, miR-4653-3p, miR-636, miR-4652-3p, miR-6823-5p, miR-4502, miR-7113-5p, miR-8087, miR-7154-3p, miR-5189-5p, miR-1253, miR-518c-5p, miR-7151-5p, miR-3614-3p, miR-4727-5p, miR-3682-5p, miR-5090, miR-337-3p, miR-488-5p, miR-100-5p, miR-4520-3p, miR-373-3p, miR-6499-5p, miR-3909, miR-32-5p, miR-302a-3p, miR-4686, miR-4659a-3p, miR-4287, miR-1301-5p, miR-593-3p, miR-517a-3p, miR-517b-3p, miR-142-3p, miR-1185-2-3p, miR-602, miR-527, miR-518a-5p, miR-4682, miR-28-5p, miR-4252, miR-452-5p, miR-525-5p, miR-3622a-3p, miR-6813-3p, miR-4769-3p, miR-5698, miR-1915-3p, miR-1343-5p, miR-6861-5p, miR-6781-5p, miR-4508, miR-6743-5p, miR-6726-5p, miR-4525, miR-4651, miR-6813-5p, miR-5787, miR-1290, miR-6075, miR-4758-5p, miR-4690-5p, miR-762, miR-371a-5p, miR-6765-3p, miR-6784-5p, miR-6778-5p, miR-6875-5p, miR-4534, miR-4721, miR-6756-5p, miR-615-5p, miR-6727-5p, miR-6887-5p, miR-8063, miR-6880-5p, miR-6805-3p, miR-4726-5p, miR-4710, miR-7111-5p, miR-3619-3p, miR-6795-5p, miR-1254, miR-1233-5p, miR-6836-3p, miR-6769a-5p, miR-4532, miR-365a-5p, miR-1231, miR-1228-5p, miR-4430, miR-296-3p, miR-1237-5p, miR-4466, miR-6789-5p, miR-4632-5p, miR-4745-5p, miR-4665-5p, miR-6807-5p, miR-7114-5p, miR-516a-5p, miR-769-3p, miR-3692-5p, miR-3945, miR-4433a-3p, miR-4485-3p, miR-6831-5p, miR-519c-5p, miR-551b-5p, miR-1343-3p, miR-4286, miR-4634, miR-4733-3p, and miR-6086, or to complementary strand(s) of the polynucleotide(s).

(2) The kit according to (1), wherein the nucleic acid(s) are polynucleotide(s) selected from the group consisting of the following polynucleotides (a) to (e):

(a) a polynucleotide consisting of a nucleotide sequence represented by any of SEQ ID NOs: 1 to 210, 374, 1315 to 1350, and 1435 to 1448 or a nucleotide sequence derived from the nucleotide sequence by the replacement of u with t, a variant thereof, a derivative thereof, or a fragment thereof comprising 15 or more consecutive nucleotides,

(b) a polynucleotide comprising a nucleotide sequence represented by any of SEQ ID NOs: 1 to 210, 374, 1315 to 1350, and 1435 to 1448,

(c) a polynucleotide consisting of a nucleotide sequence complementary to a nucleotide sequence represented by any of SEQ ID NOs: 1 to 210, 374, 1315 to 1350, and 1435 to 1448 or a nucleotide sequence derived from the nucleotide sequence by the replacement of u with t, a variant thereof, a derivative thereof, or a fragment thereof comprising 15 or more consecutive nucleotides,

(d) a polynucleotide comprising a nucleotide sequence complementary to a nucleotide sequence represented by any of SEQ ID NOs: 1 to 210, 374, 1315 to 1350, and 1435 to 1448 or a nucleotide sequence derived from the nucleotide sequence by the replacement of u with t, and

(e) a polynucleotide hybridizing under stringent conditions to any of the polynucleotides (a) to (d).

(3) The kit according to (1) or (2), wherein the kit further comprises nucleic acid(s) capable of specifically binding to one or more polynucleotide(s) selected from the group consisting of other dementia markers: miR-1225-3p, miR-3184-5p, miR-665, miR-211-5p, miR-1247-5p, miR-3656, miR-149-5p, miR-744-5p, miR-345-5p, miR-150-5p, miR-191-3p, miR-651-5p, miR-34a-5p, miR-409-5p, miR-369-5p, miR-1915-5p, miR-204-5p, miR-137, miR-382-5p, miR-517-5p, miR-532-5p, miR-22-5p, miR-1237-3p, miR-1224-3p, miR-625-3p, miR-328-3p, miR-122-5p, miR-202-3p, miR-4781-5p, miR-718, miR-342-3p, miR-26b-3p, miR-140-3p, miR-200a-3p, miR-378a-3p, miR-484, miR-296-5p, miR-205-5p, miR-431-5p, miR-150-3p, miR-423-5p, miR-575, miR-671-5p, miR-939-5p, miR-3665, miR-30d-5p, miR-30b-3p, miR-92a-3p, miR-371b-5p, and miR-486-5p, or to complementary strand(s) of the polynucleotide(s).

(4) The kit according to (3), wherein the nucleic acid(s) are polynucleotide(s) selected from the group consisting of the following polynucleotides (f) to (j):

(f) a polynucleotide consisting of a nucleotide sequence represented by any of SEQ ID NOs: 211 to 249, 1351 to 1356, and 1449 to 1453 or a nucleotide sequence derived from the nucleotide sequence by the replacement of u with t, a variant thereof, a derivative thereof, or a fragment thereof comprising 15 or more consecutive nucleotides,

(g) a polynucleotide comprising a nucleotide sequence represented by any of SEQ ID NOs: 211 to 249, 1351 to 1356, and 1449 to 1453,

(h) a polynucleotide consisting of a nucleotide sequence complementary to a nucleotide sequence represented by any of SEQ ID NOs: 211 to 249, 1351 to 1356, and 1449 to 1453 or a nucleotide sequence derived from the nucleotide sequence by the replacement of u with t, a variant thereof, a derivative thereof, or a fragment thereof comprising 15 or more consecutive nucleotides,

(i) a polynucleotide comprising a nucleotide sequence complementary to a nucleotide sequence represented by any of SEQ ID NOs: 211 to 249, 1351 to 1356, and 1449 to 1453 or a nucleotide sequence derived from the nucleotide sequence by the replacement of u with t, and

(j) a polynucleotide hybridizing under stringent conditions to any of the polynucleotides (f) to (i).

(5) The kit according to any one of (1) to (4), wherein the kit further comprises nucleic acid(s) capable of specifically binding to one or more polynucleotide(s) selected from the group consisting of other dementia markers: miR-1471, miR-1538, miR-449b-3p, miR-1976, miR-4268, miR-4279, miR-3620-3p, miR-3944-3p, miR-3156-3p, miR-3187-5p, miR-4685-3p, miR-4695-3p, miR-4697-3p, miR-4713-5p, miR-4723-3p, miR-371b-3p, miR-3151-3p, miR-3192-3p, miR-6728-3p, miR-6736-3p, miR-6740-3p, miR-6741-3p, miR-6743-3p, miR-6747-3p, miR-6750-3p, miR-6754-3p, miR-6759-3p, miR-6761-3p, miR-6762-3p, miR-6769a-3p, miR-6776-3p, miR-6778-3p, miR-6779-3p, miR-6786-3p, miR-6787-3p, miR-6792-3p, miR-6794-3p, miR-6801-3p, miR-6802-3p, miR-6803-3p, miR-6804-3p, miR-6810-5p, miR-6823-3p, miR-6825-3p, miR-6829-3p, miR-6833-3p, miR-6834-3p, miR-6780b-3p, miR-6845-3p, miR-6862-3p, miR-6865-3p, miR-6870-3p, miR-6875-3p, miR-6877-3p, miR-6879-3p, miR-6882-3p, miR-6885-3p, miR-6886-3p, miR-6887-3p, miR-6890-3p, miR-6893-3p, miR-6894-3p, miR-7106-3p, miR-7109-3p, miR-7114-3p, miR-7155-5p, miR-7160-5p, miR-615-3p, miR-920, miR-1825, miR-675-3p, miR-1910-5p, miR-2278, miR-2682-3p, miR-3122, miR-3151-5p, miR-3175, miR-4323, miR-4326, miR-4284, miR-3605-3p, miR-3622b-5p, miR-3646, miR-3158-5p, miR-4722-3p, miR-4728-3p, miR-4747-3p, miR-4436b-5p, miR-5196-3p, miR-5589-5p, miR-345-3p, miR-642b-5p, miR-6716-3p, miR-6511b-3p, miR-208a-5p, miR-6726-3p, miR-6744-5p, miR-6782-3p, miR-6789-3p, miR-6797-3p, miR-6800-3p, miR-6806-5p, miR-6824-3p, miR-6837-5p, miR-6846-3p, miR-6858-3p, miR-6859-3p, miR-6861-3p, miR-6880-3p, miR-7111-3p, miR-7152-5p, miR-642a-5p, miR-657, miR-1236-3p, miR-764, miR-4314, miR-3675-3p, miR-5703, miR-3191-5p, miR-6511a-3p, miR-6809-3p, miR-6815-5p, miR-6857-3p, and miR-6878-3p, or to complementary strand(s) of the polynucleotide(s).

(6) The kit according to (5), wherein the nucleic acid(s) are polynucleotide(s) selected from the group consisting of the following polynucleotides (k) to (o):

(k) a polynucleotide consisting of a nucleotide sequence represented by any of SEQ ID NOs: 250 to 373 or a nucleotide sequence derived from the nucleotide sequence by the replacement of u with t, a variant thereof, a derivative thereof, or a fragment thereof comprising 15 or more consecutive nucleotides,

(l) a polynucleotide comprising a nucleotide sequence represented by any of SEQ ID NOs: 250 to 373,

(m) a polynucleotide consisting of a nucleotide sequence complementary to a nucleotide sequence represented by any of SEQ ID NOs: 250 to 373 or a nucleotide sequence derived from the nucleotide sequence by the replacement of u with t, a variant thereof, a derivative thereof, or a fragment thereof comprising 15 or more consecutive nucleotides,

(n) a polynucleotide comprising a nucleotide sequence complementary to a nucleotide sequence represented by any of SEQ ID NOs: 250 to 373 or a nucleotide sequence derived from the nucleotide sequence by the replacement of u with t, and

(o) a polynucleotide hybridizing under stringent conditions to any of the polynucleotides (k) to (n).

(7) The kit according to any one of (1) to (6), wherein the kit further comprises nucleic acid(s) capable of specifically binding to one or more polynucleotide(s) selected from the group consisting of other dementia markers: miR-766-3p, miR-1229-3p, miR-1306-5p, miR-210-5p, miR-198, miR-485-3p, miR-668-3p, miR-532-3p, miR-877-3p, miR-1238-3p, miR-3130-5p, miR-4298, miR-4290, miR-3943, miR-346, and miR-767-3p, or to complementary strand(s) of the polynucleotide(s).

(8) The kit according to (7), wherein the nucleic acid(s) are polynucleotide(s) selected from the group consisting of the following polynucleotides (p) to (t):

(p) a polynucleotide consisting of a nucleotide sequence represented by any of SEQ ID NOs: 375 to 390 or a nucleotide sequence derived from the nucleotide sequence by the replacement of u with t, a variant thereof, a derivative thereof, or a fragment thereof comprising 15 or more consecutive nucleotides,

(q) a polynucleotide comprising a nucleotide sequence represented by any of SEQ ID NOs: 375 to 390,

(r) a polynucleotide consisting of a nucleotide sequence complementary to a nucleotide sequence represented by any of SEQ ID NOs: 375 to 390 or a nucleotide sequence derived from the nucleotide sequence by the replacement of u with t, a variant thereof, a derivative thereof, or a fragment thereof comprising 15 or more consecutive nucleotides,

(s) a polynucleotide comprising a nucleotide sequence complementary to a nucleotide sequence represented by any of SEQ ID NOs: 375 to 390 or a nucleotide sequence derived from the nucleotide sequence by the replacement of u with t, and

(t) a polynucleotide hybridizing under stringent conditions to any of the polynucleotides (p) to (s).

(9) A kit for detection or prediction of dementia, comprising nucleic acid(s) I capable of specifically binding to one or more polynucleotide(s) selected from the group consisting of dementia markers: miR-4728-5p, miR-3184-3p, miR-1233-3p, miR-6088, miR-6777-3p, miR-7110-3p, miR-936, miR-6087, miR-1202, miR-4731-3p, miR-4800-5p, miR-6784-3p, miR-4716-5p, miR-6734-3p, miR-6889-3p, miR-6867-3p, miR-3622a-3p, miR-6813-3p, miR-4769-3p, miR-5698, miR-3184-5p, miR-1471, miR-1538, miR-449b-3p, miR-1976, miR-4268, miR-4279, miR-3620-3p, miR-3944-3p, miR-3156-3p, miR-3187-5p, miR-4685-3p, miR-4695-3p, miR-4697-3p, miR-4713-5p, miR-4723-3p, miR-371b-3p, miR-3151-3p, miR-3192-3p, miR-6728-3p, miR-6736-3p, miR-6740-3p, miR-6741-3p, miR-6743-3p, miR-6747-3p, miR-6750-3p, miR-6754-3p, miR-6759-3p, miR-6761-3p, miR-6762-3p, miR-6769a-3p, miR-6776-3p, miR-6778-3p, miR-6779-3p, miR-6786-3p, miR-6787-3p, miR-6792-3p, miR-6794-3p, miR-6801-3p, miR-6802-3p, miR-6803-3p, miR-6804-3p, miR-6810-5p, miR-6823-3p, miR-6825-3p, miR-6829-3p, miR-6833-3p, miR-6834-3p, miR-6780b-3p, miR-6845-3p, miR-6862-3p, miR-6865-3p, miR-6870-3p, miR-6875-3p, miR-6877-3p, miR-6879-3p, miR-6882-3p, miR-6885-3p, miR-6886-3p, miR-6887-3p, miR-6890-3p, miR-6893-3p, miR-6894-3p, miR-7106-3p, miR-7109-3p, miR-7114-3p, miR-7155-5p, miR-7160-5p, miR-615-3p, miR-920, miR-1825, miR-675-3p, miR-1910-5p, miR-2278, miR-2682-3p, miR-3122, miR-3151-5p, miR-3175, miR-4323, miR-4326, miR-4284, miR-3605-3p, miR-3622b-5p, miR-3646, miR-3158-5p, miR-4722-3p, miR-4728-3p, miR-4747-3p, miR-4436b-5p, miR-5196-3p, miR-5589-5p, miR-345-3p, miR-642b-5p, miR-6716-3p, miR-6511b-3p, miR-208a-5p, miR-6726-3p, miR-6744-5p, miR-6782-3p, miR-6789-3p, miR-6797-3p, miR-6800-3p, miR-6806-5p, miR-6824-3p, miR-6837-5p, miR-6846-3p, miR-6858-3p, miR-6859-3p, miR-6861-3p, miR-6880-3p, miR-7111-3p, miR-7152-5p, miR-642a-5p, miR-657, miR-1236-3p, miR-764, miR-4314, miR-3675-3p, miR-5703, miR-3191-5p, miR-6511a-3p, miR-6809-3p, miR-6815-5p, miR-6857-3p, miR-6878-3p, and miR-371a-5p, or to complementary strand(s) of the polynucleotide(s), and optionally further comprising nucleic acid(s) II capable of specifically binding to one or more polynucleotide(s) selected from the group consisting of other dementia markers: miR-766-3p, miR-1229-3p, miR-1306-5p, miR-210-5p, miR-198, miR-485-3p, miR-668-3p, miR-532-3p, miR-877-3p, miR-1238-3p, miR-3130-5p, miR-4298, miR-4290, miR-3943, miR-346, and miR-767-3p, or to complementary strand(s) of the polynucleotide(s).

(10) The kit according to (9), wherein the nucleic acid(s) I are polynucleotide(s) selected from the group consisting of the following polynucleotides (u) to (y):

(u) a polynucleotide consisting of a nucleotide sequence represented by any of SEQ ID NOs: 3, 8, 15, 26, 34, 40, 53, 69, 99, 101, 107, 109, 112, 125, 128, 133, 191 to 194, 212, and 250 to 374 or a nucleotide sequence derived from the nucleotide sequence by the replacement of u with t, a variant thereof, a derivative thereof, or a fragment thereof comprising 15 or more consecutive nucleotides,

(v) a polynucleotide comprising a nucleotide sequence represented by any of SEQ ID NOs: 3, 8, 15, 26, 34, 40, 53, 69, 99, 101, 107, 109, 112, 125, 128, 133, 191 to 194, 212, and 250 to 374,

(w) a polynucleotide consisting of a nucleotide sequence complementary to a nucleotide sequence represented by any of SEQ ID NOs: 3, 8, 15, 26, 34, 40, 53, 69, 99, 101, 107, 109, 112, 125, 128, 133, 191 to 194, 212, and 250 to 374 or a nucleotide sequence derived from the nucleotide sequence by the replacement of u with t, a variant thereof, a derivative thereof, or a fragment thereof comprising 15 or more consecutive nucleotides,

(x) a polynucleotide comprising a nucleotide sequence complementary to a nucleotide sequence represented by any of SEQ ID NOs: 3, 8, 15, 26, 34, 40, 53, 69, 99, 101, 107, 109, 112, 125, 128, 133, 191 to 194, 212, and 250 to 374 or a nucleotide sequence derived from the nucleotide sequence by the replacement of u with t, and

(y) a polynucleotide hybridizing under stringent conditions to any of the polynucleotides (u) to (x),

wherein the nucleic acid(s) II are polynucleotides selected from the group consisting of the following polynucleotides (p) to (t):

(p) a polynucleotide consisting of a nucleotide sequence represented by any of SEQ ID NOs: 375 to 390 or a nucleotide sequence derived from the nucleotide sequence by the replacement of u with t, a variant thereof, a derivative thereof, or a fragment thereof comprising 15 or more consecutive nucleotides,

(q) a polynucleotide comprising a nucleotide sequence represented by any of SEQ ID NOs: 375 to 390,

(r) a polynucleotide consisting of a nucleotide sequence complementary to a nucleotide sequence represented by any of SEQ ID NOs: 375 to 390 or a nucleotide sequence derived from the nucleotide sequence by the replacement of u with t, a variant thereof, a derivative thereof, or a fragment thereof comprising 15 or more consecutive nucleotides,

(s) a polynucleotide comprising a nucleotide sequence complementary to a nucleotide sequence represented by any of SEQ ID NOs: 375 to 390 or a nucleotide sequence derived from the nucleotide sequence by the replacement of u with t, and

(t) a polynucleotide hybridizing under stringent conditions to any of the polynucleotides (p) to (s).

(11) A device for detection of dementia, comprising nucleic acid(s) capable of specifically binding to one or more polynucleotide(s) selected from the group consisting of dementia markers: miR-4274, miR-4272, miR-4728-5p, miR-4443, miR-4506, miR-6773-5p, miR-4662a-5p, miR-3184-3p, miR-4281, miR-320d, miR-6729-3p, miR-5192, miR-6853-5p, miR-1234-3p, miR-1233-3p, miR-4539, miR-3914, miR-4738-5p, miR-548au-3p, miR-1539, miR-4720-3p, miR-365b-5p, miR-4486, miR-1227-5p, miR-4667-5p, miR-6088, miR-6820-5p, miR-4505, miR-548q, miR-4658, miR-450a-5p, miR-1260b, miR-3677-5p, miR-6777-3p, miR-6826-3p, miR-6832-3p, miR-4725-3p, miR-7161-3p, miR-2277-5p, miR-7110-3p, miR-4312, miR-4461, miR-6766-5p, miR-1266-3p, miR-6729-5p, miR-526b-3p, miR-519e-5p, miR-512-5p, miR-5088-5p, miR-1909-3p, miR-6511a-5p, miR-4734, miR-936, miR-1249-3p, miR-6777-5p, miR-4487, miR-3155a, miR-563, miR-4741, miR-6788-5p, miR-4433b-5p, miR-323a-5p, miR-6811-5p, miR-6721-5p, miR-5004-5p, miR-6509-3p, miR-648, miR-3917, miR-6087, miR-1470, miR-586, miR-3150a-5p, miR-105-3p, miR-7973, miR-1914-5p, miR-4749-3p, miR-15b-5p, miR-1289, miR-4433a-5p, miR-3666, miR-3186-3p, miR-4725-5p, miR-4488, miR-4474-3p, miR-6731-3p, miR-4640-3p, miR-202-5p, miR-6816-5p, miR-638, miR-6821-5p, miR-1247-3p, miR-6765-5p, miR-6800-5p, miR-3928-3p, miR-3940-5p, miR-3960, miR-6775-5p, miR-3178, miR-1202, miR-6790-5p, miR-4731-3p, miR-2681-3p, miR-6758-5p, miR-8072, miR-518d-3p, miR-3606-3p, miR-4800-5p, miR-1292-3p, miR-6784-3p, miR-4450, miR-6132, miR-4716-5p, miR-6860, miR-1268b, miR-378d, miR-4701-5p, miR-4329, miR-185-3p, miR-552-3p, miR-1273g-5p, miR-6769b-3p, miR-520a-3p, miR-4524b-5p, miR-4291, miR-6734-3p, miR-143-5p, miR-939-3p, miR-6889-3p, miR-6842-3p, miR-4511, miR-4318, miR-4653-5p, miR-6867-3p, miR-133b, miR-3196, miR-193b-3p, miR-3162-3p, miR-6819-3p, miR-1908-3p, miR-6786-5p, miR-3648, miR-4513, miR-3652, miR-4640-5p, miR-6871-5p, miR-7845-5p, miR-3138, miR-6884-5p, miR-4653-3p, miR-636, miR-4652-3p, miR-6823-5p, miR-4502, miR-7113-5p, miR-8087, miR-7154-3p, miR-5189-5p, miR-1253, miR-518c-5p, miR-7151-5p, miR-3614-3p, miR-4727-5p, miR-3682-5p, miR-5090, miR-337-3p, miR-488-5p, miR-100-5p, miR-4520-3p, miR-373-3p, miR-6499-5p, miR-3909, miR-32-5p, miR-302a-3p, miR-4686, miR-4659a-3p, miR-4287, miR-1301-5p, miR-593-3p, miR-517a-3p, miR-517b-3p, miR-142-3p, miR-1185-2-3p, miR-602, miR-527, miR-518a-5p, miR-4682, miR-28-5p, miR-4252, miR-452-5p, miR-525-5p, miR-3622a-3p, miR-6813-3p, miR-4769-3p, miR-5698, miR-1915-3p, miR-1343-5p, miR-6861-5p, miR-6781-5p, miR-4508, miR-6743-5p, miR-6726-5p, miR-4525, miR-4651, miR-6813-5p, miR-5787, miR-1290, miR-6075, miR-4758-5p, miR-4690-5p, miR-762, miR-371a-5p, miR-6765-3p, miR-6784-5p, miR-6778-5p, miR-6875-5p, miR-4534, miR-4721, miR-6756-5p, miR-615-5p, miR-6727-5p, miR-6887-5p, miR-8063, miR-6880-5p, miR-6805-3p, miR-4726-5p, miR-4710, miR-7111-5p, miR-3619-3p, miR-6795-5p, miR-1254, miR-1233-5p, miR-6836-3p, miR-6769a-5p, miR-4532, miR-365a-5p, miR-1231, miR-1228-5p, miR-4430, miR-296-3p, miR-1237-5p, miR-4466, miR-6789-5p, miR-4632-5p, miR-4745-5p, miR-4665-5p, miR-6807-5p, miR-7114-5p, miR-516a-5p, miR-769-3p, miR-3692-5p, miR-3945, miR-4433a-3p, miR-4485-3p, miR-6831-5p, miR-519c-5p, miR-551b-5p, miR-1343-3p, miR-4286, miR-4634, miR-4733-3p, and miR-6086, or to complementary strand(s) of the polynucleotide(s).

(12) The device according to (11), wherein the nucleic acid(s) are polynucleotide(s) selected from the group consisting of the following polynucleotides (a) to (e):

(a) a polynucleotide consisting of a nucleotide sequence represented by any of SEQ ID NOs: 1 to 210, 374, 1315 to 1350, and 1435 to 1448 or a nucleotide sequence derived from the nucleotide sequence by the replacement of u with t, a variant thereof, a derivative thereof, or a fragment thereof comprising 15 or more consecutive nucleotides,

(b) a polynucleotide comprising a nucleotide sequence represented by any of SEQ ID NOs: 1 to 210, 374, 1315 to 1350, and 1435 to 1448,

(c) a polynucleotide consisting of a nucleotide sequence complementary to a nucleotide sequence represented by any of SEQ ID NOs: 1 to 210, 374, 1315 to 1350, and 1435 to 1448 or a nucleotide sequence derived from the nucleotide sequence by the replacement of u with t, a variant thereof, a derivative thereof, or a fragment thereof comprising 15 or more consecutive nucleotides,

(d) a polynucleotide comprising a nucleotide sequence complementary to a nucleotide sequence represented by any of SEQ ID NOs: 1 to 210, 374, 1315 to 1350, and 1435 to 1448 or a nucleotide sequence derived from the nucleotide sequence by the replacement of u with t, and

(e) a polynucleotide hybridizing under stringent conditions to any of the polynucleotides (a) to (d).

(13) The device according to (11) or (12), wherein the device further comprises nucleic acid(s) capable of specifically binding to one or more polynucleotide(s) selected from the group consisting of other dementia markers: miR-1225-3p, miR-3184-5p, miR-665, miR-211-5p, miR-1247-5p, miR-3656, miR-149-5p, miR-744-5p, miR-345-5p, miR-150-5p, miR-191-3p, miR-651-5p, miR-34a-5p, miR-409-5p, miR-369-5p, miR-1915-5p, miR-204-5p, miR-137, miR-382-5p, miR-517-5p, miR-532-5p, miR-22-5p, miR-1237-3p, miR-1224-3p, miR-625-3p, miR-328-3p, miR-122-5p, miR-202-3p, miR-4781-5p, miR-718, miR-342-3p, miR-26b-3p, miR-140-3p, miR-200a-3p, miR-378a-3p, miR-484, miR-296-5p, miR-205-5p, miR-431-5p, miR-150-3p, miR-423-5p, miR-575, miR-671-5p, miR-939-5p, miR-3665, miR-30d-5p, miR-30b-3p, miR-92a-3p, miR-371b-5p, and miR-486-5p, or to complementary strand(s) of the polynucleotide(s).

(14) The device according to (13), wherein the nucleic acid(s) are polynucleotide(s) selected from the group consisting of the following polynucleotides (f) to (j):

(f) a polynucleotide consisting of a nucleotide sequence represented by any of SEQ ID NOs: 211 to 249, 1351 to 1356, and 1449 to 1453 or a nucleotide sequence derived from the nucleotide sequence by the replacement of u with t, a variant thereof, a derivative thereof, or a fragment thereof comprising 15 or more consecutive nucleotides,

(g) a polynucleotide comprising a nucleotide sequence represented by any of SEQ ID NOs: 211 to 249, 1351 to 1356, and 1449 to 1453,

(h) a polynucleotide consisting of a nucleotide sequence complementary to a nucleotide sequence represented by any of SEQ ID NOs: 211 to 249, 1351 to 1356, and 1449 to 1453 or a nucleotide sequence derived from the nucleotide sequence by the replacement of u with t, a variant thereof, a derivative thereof, or a fragment thereof comprising 15 or more consecutive nucleotides,

(i) a polynucleotide comprising a nucleotide sequence complementary to a nucleotide sequence represented by any of SEQ ID NOs: 211 to 249, 1351 to 1356, and 1449 to 1453 or a nucleotide sequence derived from the nucleotide sequence by the replacement of u with t, and

(j) a polynucleotide hybridizing under stringent conditions to any of the polynucleotides (f) to (i).

(15) The device according to any one of (11) to (14), wherein the device further comprises nucleic acid(s) capable of specifically binding to one or more polynucleotide(s) selected from the group consisting of other dementia markers: miR-1471, miR-1538, miR-449b-3p, miR-1976, miR-4268, miR-4279, miR-3620-3p, miR-3944-3p, miR-3156-3p, miR-3187-5p, miR-4685-3p, miR-4695-3p, miR-4697-3p, miR-4713-5p, miR-4723-3p, miR-371b-3p, miR-3151-3p, miR-3192-3p, miR-6728-3p, miR-6736-3p, miR-6740-3p, miR-6741-3p, miR-6743-3p, miR-6747-3p, miR-6750-3p, miR-6754-3p, miR-6759-3p, miR-6761-3p, miR-6762-3p, miR-6769a-3p, miR-6776-3p, miR-6778-3p, miR-6779-3p, miR-6786-3p, miR-6787-3p, miR-6792-3p, miR-6794-3p, miR-6801-3p, miR-6802-3p, miR-6803-3p, miR-6804-3p, miR-6810-5p, miR-6823-3p, miR-6825-3p, miR-6829-3p, miR-6833-3p, miR-6834-3p, miR-6780b-3p, miR-6845-3p, miR-6862-3p, miR-6865-3p, miR-6870-3p, miR-6875-3p, miR-6877-3p, miR-6879-3p, miR-6882-3p, miR-6885-3p, miR-6886-3p, miR-6887-3p, miR-6890-3p, miR-6893-3p, miR-6894-3p, miR-7106-3p, miR-7109-3p, miR-7114-3p, miR-7155-5p, miR-7160-5p, miR-615-3p, miR-920, miR-1825, miR-675-3p, miR-1910-5p, miR-2278, miR-2682-3p, miR-3122, miR-3151-5p, miR-3175, miR-4323, miR-4326, miR-4284, miR-3605-3p, miR-3622b-5p, miR-3646, miR-3158-5p, miR-4722-3p, miR-4728-3p, miR-4747-3p, miR-4436b-5p, miR-5196-3p, miR-5589-5p, miR-345-3p, miR-642b-5p, miR-6716-3p, miR-6511b-3p, miR-208a-5p, miR-6726-3p, miR-6744-5p, miR-6782-3p, miR-6789-3p, miR-6797-3p, miR-6800-3p, miR-6806-5p, miR-6824-3p, miR-6837-5p, miR-6846-3p, miR-6858-3p, miR-6859-3p, miR-6861-3p, miR-6880-3p, miR-7111-3p, miR-7152-5p, miR-642a-5p, miR-657, miR-1236-3p, miR-764, miR-4314, miR-3675-3p, miR-5703, miR-3191-5p, miR-6511a-3p, miR-6809-3p, miR-6815-5p, miR-6857-3p, and miR-6878-3p, or to complementary strand(s) of the polynucleotide(s).

(16) The device according to (15), wherein the nucleic acid(s) are polynucleotide(s) selected from the group consisting of the following polynucleotides (k) to (o):

(k) a polynucleotide consisting of a nucleotide sequence represented by any of SEQ ID NOs: 250 to 373 or a nucleotide sequence derived from the nucleotide sequence by the replacement of u with t, a variant thereof, a derivative thereof, or a fragment thereof comprising 15 or more consecutive nucleotides,

(l) a polynucleotide comprising a nucleotide sequence represented by any of SEQ ID NOs: 250 to 373,

(m) a polynucleotide consisting of a nucleotide sequence complementary to a nucleotide sequence represented by any of SEQ ID NOs: 250 to 373 or a nucleotide sequence derived from the nucleotide sequence by the replacement of u with t, a variant thereof, a derivative thereof, or a fragment thereof comprising 15 or more consecutive nucleotides,

(n) a polynucleotide comprising a nucleotide sequence complementary to a nucleotide sequence represented by any of SEQ ID NOs: 250 to 373 or a nucleotide sequence derived from the nucleotide sequence by the replacement of u with t, and

(o) a polynucleotide hybridizing under stringent conditions to any of the polynucleotides (k) to (n).

(17) The device according to any one of (11) to (16), wherein the device further comprises nucleic acid(s) capable of specifically binding to one or more polynucleotide(s) selected from the group consisting of other dementia markers: miR-766-3p, miR-1229-3p, miR-1306-5p, miR-210-5p, miR-198, miR-485-3p, miR-668-3p, miR-532-3p, miR-877-3p, miR-1238-3p, miR-3130-5p, miR-4298, miR-4290, miR-3943, miR-346, and miR-767-3p, or to complementary strand(s) of the polynucleotide(s).

(18) The device according to (17), wherein the nucleic acid(s) are polynucleotide(s) selected from the group consisting of the following polynucleotides (p) to (t):

(p) a polynucleotide consisting of a nucleotide sequence represented by any of SEQ ID NOs: 375 to 390 or a nucleotide sequence derived from the nucleotide sequence by the replacement of u with t, a variant thereof, a derivative thereof, or a fragment thereof comprising 15 or more consecutive nucleotides,

(q) a polynucleotide comprising a nucleotide sequence represented by any of SEQ ID NOs: 375 to 390,

(r) a polynucleotide consisting of a nucleotide sequence complementary to a nucleotide sequence represented by any of SEQ ID NOs: 375 to 390 or a nucleotide sequence derived from the nucleotide sequence by the replacement of u with t, a variant thereof, a derivative thereof, or a fragment thereof comprising 15 or more consecutive nucleotides,

(s) a polynucleotide comprising a nucleotide sequence complementary to a nucleotide sequence represented by any of SEQ ID NOs: 375 to 390 or a nucleotide sequence derived from the nucleotide sequence by the replacement of u with t, and

(t) a polynucleotide hybridizing under stringent conditions to any of the polynucleotides (p) to (s).

(19) A device for detection or prediction of dementia, comprising nucleic acid(s) I capable of specifically binding to one or more polynucleotide(s) selected from the group consisting of dementia markers: miR-4728-5p, miR-3184-3p, miR-1233-3p, miR-6088, miR-6777-3p, miR-7110-3p, miR-936, miR-6087, miR-1202, miR-4731-3p, miR-4800-5p, miR-6784-3p, miR-4716-5p, miR-6734-3p, miR-6889-3p, miR-6867-3p, miR-3622a-3p, miR-6813-3p, miR-4769-3p, miR-5698, miR-3184-5p, miR-1471, miR-1538, miR-449b-3p, miR-1976, miR-4268, miR-4279, miR-3620-3p, miR-3944-3p, miR-3156-3p, miR-3187-5p, miR-4685-3p, miR-4695-3p, miR-4697-3p, miR-4713-5p, miR-4723-3p, miR-371b-3p, miR-3151-3p, miR-3192-3p, miR-6728-3p, miR-6736-3p, miR-6740-3p, miR-6741-3p, miR-6743-3p, miR-6747-3p, miR-6750-3p, miR-6754-3p, miR-6759-3p, miR-6761-3p, miR-6762-3p, miR-6769a-3p, miR-6776-3p, miR-6778-3p, miR-6779-3p, miR-6786-3p, miR-6787-3p, miR-6792-3p, miR-6794-3p, miR-6801-3p, miR-6802-3p, miR-6803-3p, miR-6804-3p, miR-6810-5p, miR-6823-3p, miR-6825-3p, miR-6829-3p, miR-6833-3p, miR-6834-3p, miR-6780b-3p, miR-6845-3p, miR-6862-3p, miR-6865-3p, miR-6870-3p, miR-6875-3p, miR-6877-3p, miR-6879-3p, miR-6882-3p, miR-6885-3p, miR-6886-3p, miR-6887-3p, miR-6890-3p, miR-6893-3p, miR-6894-3p, miR-7106-3p, miR-7109-3p, miR-7114-3p, miR-7155-5p, miR-7160-5p, miR-615-3p, miR-920, miR-1825, miR-675-3p, miR-1910-5p, miR-2278, miR-2682-3p, miR-3122, miR-3151-5p, miR-3175, miR-4323, miR-4326, miR-4284, miR-3605-3p, miR-3622b-5p, miR-3646, miR-3158-5p, miR-4722-3p, miR-4728-3p, miR-4747-3p, miR-4436b-5p, miR-5196-3p, miR-5589-5p, miR-345-3p, miR-642b-5p, miR-6716-3p, miR-6511b-3p, miR-208a-5p, miR-6726-3p, miR-6744-5p, miR-6782-3p, miR-6789-3p, miR-6797-3p, miR-6800-3p, miR-6806-5p, miR-6824-3p, miR-6837-5p, miR-6846-3p, miR-6858-3p, miR-6859-3p, miR-6861-3p, miR-6880-3p, miR-7111-3p, miR-7152-5p, miR-642a-5p, miR-657, miR-1236-3p, miR-764, miR-4314, miR-3675-3p, miR-5703, miR-3191-5p, miR-6511a-3p, miR-6809-3p, miR-6815-5p, miR-6857-3p, miR-6878-3p, and miR-371a-5p, or to complementary strand(s) of the polynucleotide(s), and

optionally further comprising nucleic acid(s) II capable of specifically binding to one or more polynucleotide(s) selected from the group consisting of other dementia markers: miR-766-3p, miR-1229-3p, miR-1306-5p, miR-210-5p, miR-198, miR-485-3p, miR-668-3p, miR-532-3p, miR-877-3p, miR-1238-3p, miR-3130-5p, miR-4298, miR-4290, miR-3943, miR-346, and miR-767-3p, or to complementary strand(s) of the polynucleotide(s).

(20) The device according to (19), wherein the nucleic acid(s) I are polynucleotide(s) selected from the group consisting of the following polynucleotides (u) to (y):

(u) a polynucleotide consisting of a nucleotide sequence represented by any of SEQ ID NOs: 3, 8, 15, 26, 34, 40, 53, 69, 99, 101, 107, 109, 112, 125, 128, 133, 191 to 194, 212, and 250 to 374 or a nucleotide sequence derived from the nucleotide sequence by the replacement of u with t, a variant thereof, a derivative thereof, or a fragment thereof comprising 15 or more consecutive nucleotides,

(v) a polynucleotide comprising a nucleotide sequence represented by any of SEQ ID NOs: 3, 8, 15, 26, 34, 40, 53, 69, 99, 101, 107, 109, 112, 125, 128, 133, 191 to 194, 212, and 250 to 374,

(w) a polynucleotide consisting of a nucleotide sequence complementary to a nucleotide sequence represented by any of SEQ ID NOs: 3, 8, 15, 26, 34, 40, 53, 69, 99, 101, 107, 109, 112, 125, 128, 133, 191 to 194, 212, and 250 to 374 or a nucleotide sequence derived from the nucleotide sequence by the replacement of u with t, a variant thereof, a derivative thereof, or a fragment thereof comprising 15 or more consecutive nucleotides,

(x) a polynucleotide comprising a nucleotide sequence complementary to a nucleotide sequence represented by any of SEQ ID NOs: 3, 8, 15, 26, 34, 40, 53, 69, 99, 101, 107, 109, 112, 125, 128, 133, 191 to 194, 212, and 250 to 374 or a nucleotide sequence derived from the nucleotide sequence by the replacement of u with t, and

(y) a polynucleotide hybridizing under stringent conditions to any of the polynucleotides (u) to (x), wherein the nucleic acid(s) II are polynucleotide(s) selected from the group consisting of the following polynucleotides (p) to (t):

(p) a polynucleotide consisting of a nucleotide sequence represented by any of SEQ ID NOs: 375 to 390 or a nucleotide sequence derived from the nucleotide sequence by the replacement of u with t, a variant thereof, a derivative thereof, or a fragment thereof comprising 15 or more consecutive nucleotides,

(q) a polynucleotide comprising a nucleotide sequence represented by any of SEQ ID NOs: 375 to 390,

(r) a polynucleotide consisting of a nucleotide sequence complementary to a nucleotide sequence represented by any of SEQ ID NOs: 375 to 390 or a nucleotide sequence derived from the nucleotide sequence by the replacement of u with t, a variant thereof, a derivative thereof, or a fragment thereof comprising 15 or more consecutive nucleotides,

(s) a polynucleotide comprising a nucleotide sequence complementary to a nucleotide sequence represented by any of SEQ ID NOs: 375 to 390 or a nucleotide sequence derived from the nucleotide sequence by the replacement of u with t, and

(t) a polynucleotide hybridizing under stringent conditions to any of the polynucleotides (p) to (s).

(21) The device according to any one of (11) to (20), wherein the device is a device for measurement by a hybridization technique.

(22) The device according to (21), wherein the hybridization technique is a nucleic acid array technique.

(23) A method for detecting dementia, comprising measuring expression level(s) of one or more polynucleotide(s) selected from the group consisting of dementia markers: miR-4274, miR-4272, miR-4728-5p, miR-4443, miR-4506, miR-6773-5p, miR-4662a-5p, miR-3184-3p, miR-4281, miR-320d, miR-6729-3p, miR-5192, miR-6853-5p, miR-1234-3p, miR-1233-3p, miR-4539, miR-3914, miR-4738-5p, miR-548au-3p, miR-1539, miR-4720-3p, miR-365b-5p, miR-4486, miR-1227-5p, miR-4667-5p, miR-6088, miR-6820-5p, miR-4505, miR-548q, miR-4658, miR-450a-5p, miR-1260b, miR-3677-5p, miR-6777-3p, miR-6826-3p, miR-6832-3p, miR-4725-3p, miR-7161-3p, miR-2277-5p, miR-7110-3p, miR-4312, miR-4461, miR-6766-5p, miR-1266-3p, miR-6729-5p, miR-526b-3p, miR-519e-5p, miR-512-5p, miR-5088-5p, miR-1909-3p, miR-6511a-5p, miR-4734, miR-936, miR-1249-3p, miR-6777-5p, miR-4487, miR-3155a, miR-563, miR-4741, miR-6788-5p, miR-4433b-5p, miR-323a-5p, miR-6811-5p, miR-6721-5p, miR-5004-5p, miR-6509-3p, miR-648, miR-3917, miR-6087, miR-1470, miR-586, miR-3150a-5p, miR-105-3p, miR-7973, miR-1914-5p, miR-4749-3p, miR-15b-5p, miR-1289, miR-4433a-5p, miR-3666, miR-3186-3p, miR-4725-5p, miR-4488, miR-4474-3p, miR-6731-3p, miR-4640-3p, miR-202-5p, miR-6816-5p, miR-638, miR-6821-5p, miR-1247-3p, miR-6765-5p, miR-6800-5p, miR-3928-3p, miR-3940-5p, miR-3960, miR-6775-5p, miR-3178, miR-1202, miR-6790-5p, miR-4731-3p, miR-2681-3p, miR-6758-5p, miR-8072, miR-518d-3p, miR-3606-3p, miR-4800-5p, miR-1292-3p, miR-6784-3p, miR-4450, miR-6132, miR-4716-5p, miR-6860, miR-1268b, miR-378d, miR-4701-5p, miR-4329, miR-185-3p, miR-552-3p, miR-1273g-5p, miR-6769b-3p, miR-520a-3p, miR-4524b-5p, miR-4291, miR-6734-3p, miR-143-5p, miR-939-3p, miR-6889-3p, miR-6842-3p, miR-4511, miR-4318, miR-4653-5p, miR-6867-3p, miR-133b, miR-3196, miR-193b-3p, miR-3162-3p, miR-6819-3p, miR-1908-3p, miR-6786-5p, miR-3648, miR-4513, miR-3652, miR-4640-5p, miR-6871-5p, miR-7845-5p, miR-3138, miR-6884-5p, miR-4653-3p, miR-636, miR-4652-3p, miR-6823-5p, miR-4502, miR-7113-5p, miR-8087, miR-7154-3p, miR-5189-5p, miR-1253, miR-518c-5p, miR-7151-5p, miR-3614-3p, miR-4727-5p, miR-3682-5p, miR-5090, miR-337-3p, miR-488-5p, miR-100-5p, miR-4520-3p, miR-373-3p, miR-6499-5p, miR-3909, miR-32-5p, miR-302a-3p, miR-4686, miR-4659a-3p, miR-4287, miR-1301-5p, miR-593-3p, miR-517a-3p, miR-517b-3p, miR-142-3p, miR-1185-2-3p, miR-602, miR-527, miR-518a-5p, miR-4682, miR-28-5p, miR-4252, miR-452-5p, miR-525-5p, miR-3622a-3p, miR-6813-3p, miR-4769-3p, miR-5698, miR-1915-3p, miR-1343-5p, miR-6861-5p, miR-6781-5p, miR-4508, miR-6743-5p, miR-6726-5p, miR-4525, miR-4651, miR-6813-5p, miR-5787, miR-1290, miR-6075, miR-4758-5p, miR-4690-5p, miR-762, miR-371a-5p, miR-6765-3p, miR-6784-5p, miR-6778-5p, miR-6875-5p, miR-4534, miR-4721, miR-6756-5p, miR-615-5p, miR-6727-5p, miR-6887-5p, miR-8063, miR-6880-5p, miR-6805-3p, miR-4726-5p, miR-4710, miR-7111-5p, miR-3619-3p, miR-6795-5p, miR-1254, miR-1233-5p, miR-6836-3p, miR-6769a-5p, miR-4532, miR-365a-5p, miR-1231, miR-1228-5p, miR-4430, miR-296-3p, miR-1237-5p, miR-4466, miR-6789-5p, miR-4632-5p, miR-4745-5p, miR-4665-5p, miR-6807-5p, miR-7114-5p, miR-516a-5p, miR-769-3p, miR-3692-5p, miR-3945, miR-4433a-3p, miR-4485-3p, miR-6831-5p, miR-519c-5p, miR-551b-5p, miR-1343-3p, miR-4286, miR-4634, miR-4733-3p, and miR-6086 in a sample from a subject; and evaluating *in vitro* whether or not the subject has dementia using the measured expression level(s).

(24) The method according to (23), comprising plugging the gene expression level(s) of the one or more polynucleotide(s) in the sample from the subject into a discriminant formula capable of discriminating dementia or non-dementia distinctively, wherein the discriminant formula is created by using gene expression levels in samples from subjects known to have dementia and gene expression levels in samples from non-dementia subjects as training samples; and thereby evaluating whether or not the subject has dementia.

(25) The method according to (23) or (24), wherein the expression level(s) of the polynucleotide(s) are measured by using nucleic acid(s) capable of specifically binding to the polynucleotide(s) or to complementary strand(s) of the polynucleotide(s), and

wherein the nucleic acid(s) are polynucleotide(s) selected from the group consisting of the following polynucleotides (a) to (e):

(a) a polynucleotide consisting of a nucleotide sequence represented by any of SEQ ID NOs: 1 to 210, 374, 1315 to 1350, and 1435 to 1448 or a nucleotide sequence derived from the nucleotide sequence by the replacement of u with t, a variant thereof, a derivative thereof, or a fragment thereof comprising 15 or more consecutive nucleotides,

(b) a polynucleotide comprising a nucleotide sequence represented by any of SEQ ID NOs: 1 to 210, 374, 1315 to 1350, and 1435 to 1448,

(c) a polynucleotide consisting of a nucleotide sequence complementary to a nucleotide sequence represented by any of SEQ ID NOs: 1 to 210, 374, 1315 to 1350, and 1435 to 1448 or a nucleotide sequence derived from the nucleotide sequence by the replacement of u with t, a variant thereof, a derivative thereof, or a fragment thereof comprising 15 or more consecutive nucleotides,

(d) a polynucleotide comprising a nucleotide sequence complementary to a nucleotide sequence represented by any of SEQ ID NOs: 1 to 210, 374, 1315 to 1350, and 1435 to 1448 or a nucleotide sequence derived from the nucleotide sequence by the replacement of u with t, and

(e) a polynucleotide hybridizing under stringent conditions to any of the polynucleotides (a) to (d).

(26) The method according to any one of (23) to (25), further comprising measuring expression level(s) of one or more polynucleotide(s) selected from the group consisting of other dementia markers: miR-1225-3p, miR-3184-5p, miR-665, miR-211-5p, miR-1247-5p, miR-3656, miR-149-5p, miR-744-5p, miR-345-5p, miR-150-5p, miR-191-3p, miR-651-5p, miR-34a-5p, miR-409-5p, miR-369-5p, miR-1915-5p, miR-204-5p, miR-137, miR-382-5p, miR-517-5p, miR-532-5p, miR-22-5p, miR-1237-3p, miR-1224-3p, miR-625-3p, miR-328-3p, miR-122-5p, miR-202-3p, miR-4781-5p, miR-718, miR-342-3p, miR-26b-3p, miR-140-3p, miR-200a-3p, miR-378a-3p, miR-484, miR-296-5p, miR-205-5p, miR-431-5p, miR-150-3p, miR-423-5p, miR-575, miR-671-5p, miR-939-5p, miR-3665, miR-30d-5p, miR-30b-3p, miR-92a-3p, miR-371b-5p, and miR-486-5p.

(27) The method according to (26), wherein the expression level(s) of the polynucleotide(s) are measured by using nucleic acid(s) capable of specifically binding to the polynucleotide(s) or to complementary strand(s) of the polynucleotide(s), and

wherein the nucleic acid(s) are polynucleotide(s) selected from the group consisting of the following polynucleotides (f) to (j):

(f) a polynucleotide consisting of a nucleotide sequence represented by any of SEQ ID NOs: 211 to 249, 1351 to 1356, and 1449 to 1453 or a nucleotide sequence derived from the nucleotide sequence by the replacement of u with t, a variant thereof, a derivative thereof, or a fragment thereof comprising 15 or more consecutive nucleotides,

(g) a polynucleotide comprising a nucleotide sequence represented by any of SEQ ID NOs: 211 to 249, 1351 to 1356, and 1449 to 1453,

(h) a polynucleotide consisting of a nucleotide sequence complementary to a nucleotide sequence represented by any of SEQ ID NOs: 211 to 249, 1351 to 1356, and 1449 to 1453 or a nucleotide sequence derived from the nucleotide sequence by the replacement of u with t, a variant thereof, a derivative thereof, or a fragment thereof comprising 15 or more consecutive nucleotides,

(i) a polynucleotide comprising a nucleotide sequence complementary to a nucleotide sequence represented by any of SEQ ID NOs: 211 to 249, 1351 to 1356, and 1449 to 1453 or a nucleotide sequence derived from the nucleotide sequence by the replacement of u with t, and

(j) a polynucleotide hybridizing under stringent conditions to any of the polynucleotides (f) to (i).

(28) The method according to any one of (23) to (27), further comprising measuring expression level(s) of one or more polynucleotide(s) selected from the group consisting of other dementia markers: miR-1471, miR-1538, miR-449b-3p, miR-1976, miR-4268, miR-4279, miR-3620-3p, miR-3944-3p, miR-3156-3p, miR-3187-5p, miR-4685-3p, miR-4695-3p, miR-4697-3p, miR-4713-5p, miR-4723-3p, miR-371b-3p, miR-3151-3p, miR-3192-3p, miR-6728-3p, miR-6736-3p, miR-6740-3p, miR-6741-3p, miR-6743-3p, miR-6747-3p, miR-6750-3p, miR-6754-3p, miR-6759-3p, miR-6761-3p, miR-6762-3p, miR-6769a-3p, miR-6776-3p, miR-6778-3p, miR-6779-3p, miR-6786-3p, miR-6787-3p, miR-6792-3p, miR-6794-3p, miR-6801-3p, miR-6802-3p, miR-6803-3p, miR-6804-3p, miR-6810-5p, miR-6823-3p, miR-6825-3p, miR-6829-3p, miR-6833-3p, miR-6834-3p, miR-6780b-3p, miR-6845-3p, miR-6862-3p, miR-6865-3p, miR-6870-3p, miR-6875-3p, miR-6877-3p, miR-6879-3p, miR-6882-3p, miR-6885-3p, miR-6886-3p, miR-6887-3p, miR-6890-3p, miR-6893-3p, miR-6894-3p, miR-7106-3p, miR-7109-3p, miR-7114-3p, miR-7155-5p, miR-7160-5p, miR-615-3p, miR-920, miR-1825, miR-675-3p, miR-1910-5p, miR-2278, miR-2682-3p, miR-3122, miR-3151-5p, miR-3175, miR-4323, miR-4326, miR-4284, miR-3605-3p, miR-3622b-5p, miR-3646, miR-3158-5p, miR-4722-3p, miR-4728-3p, miR-4747-3p, miR-4436b-5p, miR-5196-3p, miR-5589-5p, miR-345-3p, miR-642b-5p, miR-6716-3p, miR-6511b-3p, miR-208a-5p, miR-6726-3p, miR-6744-5p, miR-6782-3p, miR-6789-3p, miR-6797-3p, miR-6800-3p, miR-6806-5p, miR-6824-3p, miR-6837-5p, miR-6846-3p, miR-6858-3p, miR-6859-3p, miR-6861-3p, miR-6880-3p, miR-7111-3p, miR-7152-5p, miR-642a-5p, miR-657, miR-1236-3p, miR-764, miR-4314, miR-3675-3p, miR-5703, miR-3191-5p, miR-6511a-3p, miR-6809-3p, miR-6815-5p, miR-6857-3p, and miR-6878-3p.

(29) The method according to (28), wherein the expression level(s) of the polynucleotide(s) are measured by using nucleic acid(s) capable of specifically binding to the polynucleotide(s) or to complementary strand(s) of the polynucleotide(s), and
wherein the nucleic acid(s) are polynucleotide(s) selected from the group consisting of the following polynucleotides (k) to (o):

(k) a polynucleotide consisting of a nucleotide sequence represented by any of SEQ ID NOs: 250 to 373 or a nucleotide sequence derived from the nucleotide sequence by the replacement of u with t, a variant thereof, a derivative thereof, or a fragment thereof comprising 15 or more consecutive nucleotides,
(l) a polynucleotide comprising a nucleotide sequence represented by any of SEQ ID NOs: 250 to 373,
(m) a polynucleotide consisting of a nucleotide sequence complementary to a nucleotide sequence represented by any of SEQ ID NOs: 250 to 373 or a nucleotide sequence derived from the nucleotide sequence by the replacement of u with t, a variant thereof, a derivative thereof, or a fragment thereof comprising 15 or more consecutive nucleotides,
(n) a polynucleotide comprising a nucleotide sequence complementary to a nucleotide sequence represented by any of SEQ ID NOs: 250 to 373 or a nucleotide sequence derived from the nucleotide sequence by the replacement of u with t, and
(o) a polynucleotide hybridizing under stringent conditions to any of the polynucleotides (k) to (n).

(30) The method according to any one of (23) to (29), further comprising measuring expression level(s) of one or more polynucleotide(s) selected from the group consisting of other dementia markers: miR-766-3p, miR-1229-3p, miR-1306-5p, miR-210-5p, miR-198, miR-485-3p, miR-668-3p, miR-532-3p, miR-877-3p, miR-1238-3p, miR-3130-5p, miR-4298, miR-4290, miR-3943, miR-346, and miR-767-3p.

(31) The method according to (30), wherein the expression level(s) of the polynucleotide(s) are measured by using nucleic acid(s) capable of specifically binding to the polynucleotide(s) or to the complementary strand(s) of the polynucleotide(s), and
wherein the nucleic acid(s) are polynucleotide(s) selected from the group consisting of the following polynucleotides (p) to (t):

(p) a polynucleotide consisting of a nucleotide sequence represented by any of SEQ ID NOs: 375 to 390 or a nucleotide sequence derived from the nucleotide sequence by the replacement of u with t, a variant thereof, a derivative thereof, or a fragment thereof comprising 15 or more consecutive nucleotides,
(q) a polynucleotide comprising a nucleotide sequence represented by any of SEQ ID NOs: 375 to 390,
(r) a polynucleotide consisting of a nucleotide sequence complementary to a nucleotide sequence represented by any of SEQ ID NOs: 375 to 390 or a nucleotide sequence derived from the nucleotide sequence by the replacement of u with t, a variant thereof, a derivative thereof, or a fragment thereof comprising 15 or more consecutive nucleotides,
(s) a polynucleotide comprising a nucleotide sequence complementary to a nucleotide sequence represented by any of SEQ ID NOs: 375 to 390 or a nucleotide sequence derived from the nucleotide sequence by the replacement of u with t, and
(t) a polynucleotide hybridizing under stringent conditions to any of the polynucleotides (p) to (s).

(32) A method for detecting or predicting dementia, comprising:

measuring expression level(s) of one or more polynucleotide(s) I selected from the group consisting of dementia markers: miR-4728-5p, miR-3184-3p, miR-1233-3p, miR-6088, miR-6777-3p, miR-7110-3p, miR-936, miR-6087, miR-1202, miR-4731-3p, miR-4800-5p, miR-6784-3p, miR-4716-5p, miR-6734-3p, miR-6889-3p, miR-6867-3p, miR-3622a-3p, miR-6813-3p, miR-4769-3p, miR-5698, miR-3184-5p, miR-1471, miR-1538, miR-449b-3p, miR-1976, miR-4268, miR-4279, miR-3620-3p, miR-3944-3p, miR-3156-3p, miR-3187-5p, miR-4685-3p, miR-4695-3p, miR-4697-3p, miR-4713-5p, miR-4723-3p, miR-371b-3p, miR-3151-3p, miR-3192-3p, miR-6728-3p, miR-6736-3p, miR-6740-3p, miR-6741-3p, miR-6743-3p, miR-6747-3p, miR-6750-3p, miR-6754-3p, miR-6759-3p, miR-6761-3p, miR-6762-3p, miR-6769a-3p, miR-6776-3p, miR-6778-3p, miR-6779-3p, miR-6786-3p, miR-6787-3p, miR-6792-3p, miR-6794-3p, miR-6801-3p, miR-6802-3p, miR-6803-3p, miR-6804-3p, miR-6810-5p, miR-6823-3p, miR-6825-3p, miR-6829-3p, miR-6833-3p, miR-6834-3p, miR-6780b-3p, miR-6845-3p, miR-6862-3p, miR-6865-3p, miR-6870-3p, miR-6875-3p, miR-6877-3p, miR-6879-3p, miR-6882-3p, miR-6885-3p, miR-6886-3p, miR-6887-3p, miR-6890-3p, miR-6893-3p, miR-6894-3p, miR-7106-3p, miR-7109-3p, miR-7114-3p, miR-7155-5p, miR-7160-5p, miR-615-3p, miR-920, miR-1825, miR-675-3p, miR-1910-5p, miR-2278, miR-2682-3p, miR-3122, miR-3151-5p, miR-3175, miR-4323, miR-4326, miR-4284, miR-3605-3p, miR-3622b-5p, miR-3646, miR-3158-5p, miR-4722-3p, miR-4728-3p, miR-4747-3p, miR-4436b-5p, miR-5196-3p, miR-5589-5p, miR-345-3p, miR-642b-5p, miR-6716-3p, miR-6511b-3p, miR-208a-5p, miR-6726-3p, miR-6744-5p, miR-6782-3p, miR-6789-3p, miR-6797-3p, miR-6800-3p, miR-6806-5p, miR-6824-3p, miR-6837-5p, miR-6846-3p, miR-6858-3p, miR-6859-3p, miR-6861-3p, miR-6880-3p, miR-7111-3p, miR-7152-5p, miR-642a-5p, miR-657, miR-1236-3p, miR-764, miR-4314, miR-3675-3p, miR-5703, miR-3191-5p, miR-6511a-3p, miR-6809-3p, miR-6815-5p, miR-6857-3p, miR-6878-3p, and miR-371a-5p, further optionally measuring expression level(s) of one or more polynucleotide(s) II selected from the group consisting of other dementia markers: miR-766-3p, miR-1229-3p, miR-1306-5p, miR-210-5p, miR-198, miR-485-3p, miR-668-3p, miR-532-3p, miR-877-3p, miR-1238-3p, miR-3130-5p, miR-4298, miR-4290, miR-3943, miR-346, and miR-767-3p, and
evaluating *in vitro* whether or not the subject has dementia or predicting *in vitro* the possibility for the subject to develop dementia using the measured expression level(s).

(33) The method according to (32), wherein the expression level(s) of the polynucleotide(s) I are measured by using nucleic acid(s) capable of specifically binding to the polynucleotide(s) I or to complementary strand(s) of the polynucleotide(s),
wherein the nucleic acid(s) are polynucleotide(s) selected from the group consisting of the following polynucleotides (u) to (y):

(u) a polynucleotide consisting of a nucleotide sequence represented by any of SEQ ID NOs: 3, 8, 15, 26, 34, 40, 53, 69, 99, 101, 107, 109, 112, 125, 128, 133, 191 to 194, 212, and 250 to 374 or a nucleotide sequence derived from the nucleotide sequence by the replacement of u with t, a variant thereof, a derivative thereof, or a fragment thereof comprising 15 or more consecutive nucleotides,
(v) a polynucleotide comprising a nucleotide sequence represented by any of SEQ ID NOs: 3, 8, 15, 26, 34, 40, 53, 69, 99, 101, 107, 109, 112, 125, 128, 133, 191 to 194, 212, and 250 to 374,
(w) a polynucleotide consisting of a nucleotide sequence complementary to a nucleotide sequence represented by any of SEQ ID NOs: 3, 8, 15, 26, 34, 40, 53, 69, 99, 101, 107, 109, 112, 125, 128, 133, 191 to 194, 212, and 250 to 374 or a nucleotide sequence derived from the nucleotide sequence by the replacement of u with t, a variant thereof, a derivative thereof, or a fragment thereof comprising 15 or more consecutive nucleotides,
(x) a polynucleotide comprising a nucleotide sequence complementary to a nucleotide sequence represented by any of SEQ ID NOs: 3, 8, 15, 26, 34, 40, 53, 69, 99, 101, 107, 109, 112, 125, 128, 133, 191 to 194, 212, and 250 to 374 or a nucleotide sequence derived from the nucleotide sequence by the replacement of u with t, and
(y) a polynucleotide hybridizing under stringent conditions to any of the polynucleotides (u) to (x),
wherein the expression level(s) of the polynucleotide(s) II are measured by using nucleic acid(s) capable of specifically binding to the polynucleotide(s) II or to complementary strand(s) of the polynucleotide(s), wherein the nucleic acid(s) are polynucleotide(s) selected from the group consisting of the following polynucleotides (p) to (t):
(p) a polynucleotide consisting of a nucleotide sequence represented by any of SEQ ID NOs: 375 to 390 or a nucleotide sequence derived from the nucleotide sequence by the replacement of u with t, a variant thereof, a derivative thereof, or a fragment thereof comprising 15 or more consecutive nucleotides,

(q) a polynucleotide comprising a nucleotide sequence represented by any of SEQ ID NOs: 375 to 390,

(r) a polynucleotide consisting of a nucleotide sequence complementary to a nucleotide sequence represented by any of SEQ ID NOs: 375 to 390 or a nucleotide sequence derived from the nucleotide sequence by the replacement of u with t, a variant thereof, a derivative thereof, or a fragment thereof comprising 15 or more consecutive nucleotides,

(s) a polynucleotide comprising a nucleotide sequence complementary to a nucleotide sequence represented by any of SEQ ID NOs: 375 to 390 or a nucleotide sequence derived from the nucleotide sequence by the replacement of u with t, and

(t) a polynucleotide hybridizing under stringent conditions to any of the polynucleotides (p) to (s).

(34) The method according to any one of (23) to (33), wherein the expression level(s) of the target gene(s) in the sample from the subject are measured by using the kit according to any one of (1) to (10) or the device according to any one of (11) to (22).

(35) The method according to any one of (23) to (34), wherein the subject is a human.

(36) The method according to any one of (23) to (35), wherein the sample is blood, serum, or plasma.

(1') A kit for detection of dementia, comprising nucleic acid(s) capable of specifically binding to one or more polynucleotide(s) selected from the group consisting of dementia markers: miR-5698, miR-1915-3p, miR-1343-5p, miR-6861-5p, miR-6781-5p, miR-4508, miR-6743-5p, miR-6726-5p, miR-4525, miR-4651, miR-6813-5p, miR-5787, miR-1290, miR-6075, miR-4758-5p, miR-4690-5p, miR-762, miR-371a-5p, miR-6765-3p, miR-6784-5p, miR-6778-5p, miR-6875-5p, miR-4534, miR-4721, miR-6756-5p, miR-615-5p, miR-6727-5p, miR-6887-5p, miR-8063, miR-6880-5p, miR-6805-3p, miR-4726-5p, miR-4710, miR-7111-5p, miR-3619-3p, miR-6795-5p, miR-1254, miR-1233-5p, miR-6836-3p, miR-6769a-5p, miR-4532, miR-365a-5p, miR-1231, miR-1228-5p, miR-4430, miR-296-3p, miR-1237-5p, miR-4466, miR-6789-5p, miR-4632-5p, miR-4745-5p, miR-4665-5p, miR-6807-5p, and miR-7114-5p.

(2') The kit according to (1'), wherein the nucleic acid(s) are polynucleotide(s) selected from the group consisting of the following polynucleotides (a) to (e):

(a) a polynucleotide consisting of a nucleotide sequence represented by any of SEQ ID NOs: 194 to 210, 374, and 1315 to 1350 or a nucleotide sequence derived from the nucleotide sequence by the replacement of u with t, a variant thereof, a derivative thereof, or a fragment thereof comprising 15 or more consecutive nucleotides,

(b) a polynucleotide comprising a nucleotide sequence represented by any of SEQ ID NOs: 194 to 210, 374, and 1315 to 1350,

(c) a polynucleotide consisting of a nucleotide sequence complementary to a nucleotide sequence represented by any of SEQ ID NOs: 194 to 210, 374, and 1315 to 1350 or a nucleotide sequence derived from the nucleotide sequence by the replacement of u with t, a variant thereof, a derivative thereof, or a fragment thereof comprising 15 or more consecutive nucleotides,

(d) a polynucleotide comprising a nucleotide sequence complementary to a nucleotide sequence represented by any of SEQ ID NOs: 194 to 210, 374, and 1315 to 1350 or a nucleotide sequence derived from the nucleotide sequence by the replacement of u with t, and

(e) a polynucleotide hybridizing under stringent conditions to any of the polynucleotides (a) to (d).

(3') The kit according to (1') or (2'), wherein the kit further comprises nucleic acid(s) capable of specifically binding to one or more polynucleotide(s) selected from the group consisting of other dementia markers: miR-1225-3p, miR-3184-5p, miR-150-3p, miR-423-5p, miR-575, miR-671-5p, miR-939-5p, and miR-3665.

(4') The kit according to (3'), wherein the nucleic acid(s) are polynucleotide(s) selected from the group consisting of the following polynucleotides (f) to (j):

(f) a polynucleotide consisting of a nucleotide sequence represented by any of SEQ ID NOs: 211 to 212 and 1351 to 1356 or a nucleotide sequence derived from the nucleotide sequence by the replacement of u with t, a variant thereof, a derivative thereof, or a fragment thereof comprising 15 or more consecutive nucleotides,

(g) a polynucleotide comprising a nucleotide sequence represented by any of SEQ ID NOs: 211 to 212 and 1351 to 1356,

(h) a polynucleotide consisting of a nucleotide sequence complementary to a nucleotide sequence represented by any of SEQ ID NOs: 211 to 212 and 1351 to 1356 or a nucleotide sequence derived from the nucleotide sequence by the replacement of u with t, a variant thereof, a derivative thereof, or a fragment thereof comprising 15 or more consecutive nucleotides,

(i) a polynucleotide comprising a nucleotide sequence complementary to a nucleotide sequence represented by any of SEQ ID NOs: 211 to 212 and 1351 to 1356 or a nucleotide sequence derived from the nucleotide sequence by the replacement of u with t, and

(j) a polynucleotide hybridizing under stringent conditions to any of the polynucleotides (f) to (i).

(5') A device for detection of dementia, comprising nucleic acid(s) capable of specifically binding to one or more polynucleotide(s) selected from the group consisting of dementia markers: miR-5698, miR-1915-3p, miR-1343-5p, miR-6861-5p, miR-6781-5p, miR-4508, miR-6743-5p, miR-6726-5p, miR-4525, miR-4651, miR-6813-5p, miR-5787, miR-1290, miR-6075, miR-4758-5p, miR-4690-5p, miR-762, miR-371a-5p, miR-6765-3p, miR-6784-5p, miR-6778-5p, miR-6875-5p, miR-4534, miR-4721, miR-6756-5p, miR-615-5p, miR-6727-5p, miR-6887-5p, miR-8063, miR-6880-5p, miR-6805-3p, miR-4726-5p, miR-4710, miR-7111-5p, miR-3619-3p, miR-6795-5p, miR-1254, miR-1233-5p, miR-6836-3p, miR-6769a-5p, miR-4532, miR-365a-5p, miR-1231, miR-1228-5p, miR-4430, miR-296-3p, miR-1237-5p, miR-4466, miR-6789-5p, miR-4632-5p, miR-4745-5p, miR-4665-5p, miR-6807-5p, and miR-7114-5p.

(6') The device according to (5'), wherein the nucleic acid(s) are polynucleotide(s) selected from the group consisting of the following polynucleotides (a) to (e):

(a) a polynucleotide consisting of a nucleotide sequence represented by any of SEQ ID NOs: 194 to 210, 374, and 1315 to 1350 or a nucleotide sequence derived from the nucleotide sequence by the replacement of u with t, a variant thereof, a derivative thereof, or a fragment thereof comprising 15 or more consecutive nucleotides,

(b) a polynucleotide comprising a nucleotide sequence represented by any of SEQ ID NOs: 194 to 210, 374, and 1315 to 1350,

(c) a polynucleotide consisting of a nucleotide sequence complementary to a nucleotide sequence represented by any of SEQ ID NOs: 194 to 210, 374, and 1315 to 1350 or a nucleotide sequence derived from the nucleotide sequence by the replacement of u with t, a variant thereof, a derivative thereof, or a fragment thereof comprising 15 or more consecutive nucleotides,

(d) a polynucleotide comprising a nucleotide sequence complementary to a nucleotide sequence represented by any of SEQ ID NOs: 194 to 210, 374, and 1315 to 1350 or a nucleotide sequence derived from the nucleotide sequence by the replacement of u with t, and

(e) a polynucleotide hybridizing under stringent conditions to any of the polynucleotides (a) to (d).

(7') The device according to (5') or (6'), wherein the device further comprises nucleic acid(s) capable of specifically binding to one or more polynucleotide(s) selected from the group consisting of other dementia markers: miR-1225-3p, miR-3184-5p, miR-150-3p, miR-423-5p, miR-575, miR-671-5p, miR-939-5p, and miR-3665.

(8') The device according to (7'), wherein the nucleic acid(s) are polynucleotide(s) selected from the group consisting of the following polynucleotides (f) to (j):

(f) a polynucleotide consisting of a nucleotide sequence represented by any of SEQ ID NOs: 211 to 212 and 1351 to 1356 or a nucleotide sequence derived from the nucleotide sequence by the replacement of u with t, a variant thereof, a derivative thereof, or a fragment thereof comprising 15 or more consecutive nucleotides,

(g) a polynucleotide comprising a nucleotide sequence represented by any of SEQ ID NOs: 211 to 212 and 1351 to 1356,

(h) a polynucleotide consisting of a nucleotide sequence complementary to a nucleotide sequence represented by any of SEQ ID NOs: 211 to 212 and 1351 to 1356 or a nucleotide sequence derived from the nucleotide sequence by the replacement of u with t, a variant thereof, a derivative thereof, or a fragment thereof comprising 15 or more consecutive nucleotides,

(i) a polynucleotide comprising a nucleotide sequence complementary to a nucleotide sequence represented by any of SEQ ID NOs: 211 to 212 and 1351 to 1356 or a nucleotide sequence derived from the nucleotide sequence by the replacement of u with t, and

(j) a polynucleotide hybridizing under stringent conditions to any of the polynucleotides (f) to (i).

(9') The device according to any one of (5') to (8'), wherein the device is a device for measurement by a hybridization technique.

(10') The device according to (9'), wherein the hybridization technique is a nucleic acid array technique.

(11') A method for detecting dementia, comprising measuring expression level(s) of target nucleic acid(s) by using the kit according to any one of (1') to (4') or the device according to any one of (5') to (10') including nucleic acid(s) capable of specifically binding to one or more polynucleotide(s) selected from the group consisting of dementia markers: miR-5698, miR-1915-3p, miR-1343-5p, miR-6861-5p, miR-6781-5p, miR-4508, miR-6743-5p, miR-6726-5p, miR-4525, miR-4651, miR-6813-5p, miR-5787, miR-1290, miR-6075, miR-4758-5p, miR-4690-5p, miR-762, miR-371a-5p, miR-6765-3p, miR-6784-5p, miR-6778-5p, miR-6875-5p, miR-4534, miR-4721, miR-6756-5p, miR-615-5p, miR-6727-5p, miR-6887-5p, miR-8063, miR-6880-5p, miR-6805-3p, miR-4726-5p, miR-4710, miR-7111-5p, miR-3619-3p, miR-6795-5p, miR-1254, miR-1233-5p, miR-6836-3p, miR-6769a-5p, miR-4532, miR-365a-5p, miR-1231, miR-1228-5p, miR-4430, miR-296-3p, miR-1237-5p, miR-4466, miR-6789-5p, miR-4632-5p, miR-4745-5p, miR-4665-5p, miR-6807-5p, and miR-7114-5p in a sample from a subject; and evaluating *in vitro* whether or not the subject has dementia using the measured expression level(s) and likewise measured control expression level(s) in a non-dementia sample.

(12') A method for detecting dementia in a subject, comprising measuring expression level(s) of target gene(s) by using the kit according to any one of (1') to (4') or the device according to any one of (5') to (10') including nucleic acid(s) capable of specifically binding to one or more polynucleotide(s) selected from the group consisting of dementia markers: miR-5698, miR-1915-3p, miR-1343-5p, miR-6861-5p, miR-6781-5p, miR-4508, miR-6743-5p, miR-6726-5p, miR-4525, miR-4651, miR-6813-5p, miR-5787, miR-1290, miR-6075, miR-4758-5p, miR-4690-5p, miR-762, miR-371a-5p, miR-6765-3p, miR-6784-5p, miR-6778-5p, miR-6875-5p, miR-4534, miR-4721, miR-6756-5p, miR-615-5p, miR-6727-5p, miR-6887-5p, miR-8063, miR-6880-5p, miR-6805-3p, miR-4726-5p, miR-4710, miR-7111-5p, miR-3619-3p, miR-6795-5p, miR-1254, miR-1233-5p, miR-6836-3p, miR-6769a-5p, miR-4532, miR-365a-5p, miR-1231, miR-1228-5p, miR-4430, miR-296-3p, miR-1237-5p, miR-4466, miR-6789-5p, miR-4632-5p, miR-4745-5p, miR-4665-5p, miR-6807-5p, and miR-7114-5p in a sample from the subject; plugging the expression level(s) of the target gene(s) in the sample from the subject into a discriminant formula capable of discriminating dementia or non-dementia distinctively, wherein the discriminant formula is created by using gene expression levels in samples from subjects known to have dementia and gene expression levels in samples from non-dementia subjects as training samples; and thereby evaluating whether or not the subject has dementia.

(13') The method according to (11') or (12'), wherein the nucleic acid(s) are polynucleotide(s) selected from the group consisting of the following polynucleotides (a) to (e):

(a) a polynucleotide consisting of a nucleotide sequence represented by any of SEQ ID NOs: 194 to 210, 374, and 1315 to 1350 or a nucleotide sequence derived from the nucleotide sequence by the replacement of u with t, a variant thereof, a derivative thereof, or a fragment thereof comprising 15 or more consecutive nucleotides,

(b) a polynucleotide comprising a nucleotide sequence represented by any of SEQ ID NOs: 194 to 210, 374, and 1315 to 1350,

(c) a polynucleotide consisting of a nucleotide sequence complementary to a nucleotide sequence represented by any of SEQ ID NOs: 194 to 210, 374, and 1315 to 1350 or a nucleotide sequence derived from the nucleotide sequence by the replacement of u with t, a variant thereof, a derivative thereof, or a fragment thereof comprising 15 or more consecutive nucleotides,

(d) a polynucleotide comprising a nucleotide sequence complementary to a nucleotide sequence represented by any of SEQ ID NOs: 194 to 210, 374, and 1315 to 1350 or a nucleotide sequence derived from the nucleotide sequence by the replacement of u with t, and

(e) a polynucleotide hybridizing under stringent conditions to any of the polynucleotides (a) to (d).

(14') The method according to any one of (11') to (13'), wherein the kit or device further comprises nucleic acid(s) capable of specifically binding to one or more polynucleotide(s) selected from the group consisting of other dementia markers: miR-1225-3p, miR-3184-5p, miR-150-3p, miR-423-5p, miR-575, miR-671-5p, miR-939-5p, and miR-3665.

(15') The method according to (14'), wherein the nucleic acid(s) are polynucleotide(s) selected from the group consisting of the following polynucleotides (f) to (j):

(f) a polynucleotide consisting of a nucleotide sequence represented by any of SEQ ID NOs: 211 to 212 and 1351 to 1356 or a nucleotide sequence derived from the nucleotide sequence by the replacement of u with t, a variant thereof, a derivative thereof, or a fragment thereof comprising 15 or more consecutive nucleotides,

(g) a polynucleotide comprising a nucleotide sequence represented by any of SEQ ID NOs: 211 to 212 and 1351 to 1356,

(h) a polynucleotide consisting of a nucleotide sequence complementary to a nucleotide sequence represented by any of SEQ ID NOs: 211 to 212 and 1351 to 1356 or a nucleotide sequence derived from the nucleotide sequence by the replacement of u with t, a variant thereof, a derivative thereof, or a fragment thereof comprising 15 or more consecutive nucleotides,
(i) a polynucleotide comprising a nucleotide sequence complementary to a nucleotide sequence represented by any of SEQ ID NOs: 211 to 212 and 1351 to 1356 or a nucleotide sequence derived from the nucleotide sequence by the replacement of u with t, and
(j) a polynucleotide hybridizing under stringent conditions to any of the polynucleotides (f) to (i).

(16') The method according to any one of (11') to (15'), wherein the subject is a human.
(17') The method according to any one of (11') to (16'), wherein the sample is blood, serum, or plasma.

In a preferable aspect of a kit or device for detection of dementia or a method for detecting dementia according to the present invention, a disease type of the dementia may be Alzheimer's dementia, vascular dementia, Lewy body dementia, normal pressure hydrocephalus, frontotemporal lobar degeneration, senile dementia for neurofibrillary tangle type, dementia resulting from diseases (e.g. infectious diseases), alcoholic dementia, vitamin deficiency dementia, or the like, mixed dementia of any of these types, or type-unknown or unspecified dementia.

In another preferable aspect of a kit or device for detection of dementia or a method for detecting dementia according to the present invention, dementia marker miR-4274 is hsa-miR-4274, miR-4272 is hsa-miR-4272, miR-4728-5p is hsa-miR-4728-5p, miR-4443 is hsa-miR-4443, miR-4506 is hsa-miR-4506, miR-6773-5p is hsa-miR-6773-5p, miR-4662a-5p is hsa-miR-4662a-5p, miR-3184-3p is hsa-miR-3184-3p, miR-4281 is hsa-miR-4281, miR-320d is hsa-miR-320d, miR-6729-3p is hsa-miR-6729-3p, miR-5192 is hsa-miR-5192, miR-6853-5p is hsa-miR-6853-5p, miR-1234-3p is hsa-miR-1234-3p, miR-1233-3p is hsa-miR-1233-3p, miR-4539 is hsa-miR-4539, miR-3914 is hsa-miR-3914, miR-4738-5p is hsa-miR-4738-5p, miR-548au-3p is hsa-miR-548au-3p, miR-1539 is hsa-miR-1539, miR-4720-3p is hsa-miR-4720-3p, miR-365b-5p is hsa-miR-365b-5p, miR-4486 is hsa-miR-4486, miR-1227-5p is hsa-miR-1227-5p, miR-4667-5p is hsa-miR-4667-5p, miR-6088 is hsa-miR-6088, miR-6820-5p is hsa-miR-6820-5p, miR-4505 is hsa-miR-4505, miR-548q is hsa-miR-548q, miR-4658 is hsa-miR-4658, miR-450a-5p is hsa-miR-450a-5p, miR-1260b is hsa-miR-1260b, miR-3677-5p is hsa-miR-3677-5p, miR-6777-3p is hsa-miR-6777-3p, miR-6826-3p is hsa-miR-6826-3p, miR-6832-3p is hsa-miR-6832-3p, miR-4725-3p is hsa-miR-4725-3p, miR-7161-3p is hsa-miR-7161-3p, miR-2277-5p is hsa-miR-2277-5p, miR-7110-3p is hsa-miR-7110-3p, miR-4312 is hsa-miR-4312, miR-4461 is hsa-miR-4461, miR-6766-5p is hsa-miR-6766-5p, miR-1266-3p is hsa-miR-1266-3p, miR-6729-5p is hsa-miR-6729-5p, miR-526b-3p is hsa-miR-526b-3p, miR-519e-5p is hsa-miR-519e-5p, miR-512-5p is hsa-miR-512-5p, miR-5088-5p is hsa-miR-5088-5p, miR-1909-3p is hsa-miR-1909-3p, miR-6511a-5p is hsa-miR-6511a-5p, miR-4734 is hsa-miR-4734, miR-936 is hsa-miR-936, miR-1249-3p is hsa-miR-1249-3p, miR-6777-5p is hsa-miR-6777-5p, miR-4487 is hsa-miR-4487, miR-3155a is hsa-miR-3155a, miR-563 is hsa-miR-563, miR-4741 is hsa-miR-4741, miR-6788-5p is hsa-miR-6788-5p, miR-4433b-5p is hsa-miR-4433b-5p, miR-323a-5p is hsa-miR-323a-5p, miR-6811-5p is hsa-miR-6811-5p, miR-6721-5p is hsa-miR-6721-5p, miR-5004-5p is hsa-miR-5004-5p, miR-6509-3p is hsa-miR-6509-3p, miR-648 is hsa-miR-648, miR-3917 is hsa-miR-3917, miR-6087 is hsa-miR-6087, miR-1470 is hsa-miR-1470, miR-586 is hsa-miR-586, miR-3150a-5p is hsa-miR-3150a-5p, miR-105-3p is hsa-miR-105-3p, miR-7973 is hsa-miR-7973, miR-1914-5p is hsa-miR-1914-5p, miR-4749-3p is hsa-miR-4749-3p, miR-15b-5p is hsa-miR-15b-5p, miR-1289 is hsa-miR-1289, miR-4433a-5p is hsa-miR-4433a-5p, miR-3666 is hsa-miR-3666, miR-3186-3p is hsa-miR-3186-3p, miR-4725-5p is hsa-miR-4725-5p, miR-4488 is hsa-miR-4488, miR-4474-3p is hsa-miR-4474-3p, miR-6731-3p is hsa-miR-6731-3p, miR-4640-3p is hsa-miR-4640-3p, miR-202-5p is hsa-miR-202-5p, miR-6816-5p is hsa-miR-6816-5p, miR-638 is hsa-miR-638, miR-6821-5p is hsa-miR-6821-5p, miR-1247-3p is hsa-miR-1247-3p, miR-6765-5p is hsa-miR-6765-5p, miR-6800-5p is hsa-miR-6800-5p, miR-3928-3p is hsa-miR-3928-3p, miR-3940-5p is hsa-miR-3940-5p, miR-3960 is hsa-miR-3960, miR-6775-5p is hsa-miR-6775-5p, miR-3178 is hsa-miR-3178, miR-1202 is hsa-miR-1202, miR-6790-5p is hsa-miR-6790-5p, miR-4731-3p is hsa-miR-4731-3p, miR-2681-3p is hsa-miR-2681-3p, miR-6758-5p is hsa-miR-6758-5p, miR-8072 is hsa-miR-8072, miR-518d-3p is hsa-miR-518d-3p, miR-3606-3p is hsa-miR-3606-3p, miR-4800-5p is hsa-miR-4800-5p, miR-1292-3p is hsa-miR-1292-3p, miR-6784-3p is hsa-miR-6784-3p, miR-4450 is hsa-miR-4450, miR-6132 is hsa-miR-6132, miR-4716-5p is hsa-miR-4716-5p, miR-6860 is hsa-miR-6860, miR-1268b is hsa-miR-1268b, miR-378d is hsa-miR-378d, miR-4701-5p is hsa-miR-4701-5p, miR-4329 is hsa-miR-4329, miR-185-3p is hsa-miR-185-3p, miR-552-3p is hsa-miR-552-3p, miR-1273g-5p is hsa-miR-1273g-5p, miR-6769b-3p is hsa-miR-6769b-3p, miR-520a-3p is hsa-miR-520a-3p, miR-4524b-5p is hsa-miR-4524b-5p, miR-4291 is hsa-miR-4291, miR-6734-3p is hsa-miR-6734-3p, miR-143-5p is hsa-miR-143-5p, miR-939-3p is hsa-miR-939-3p, miR-6889-3p is hsa-miR-6889-3p, miR-6842-3p is hsa-miR-6842-3p, miR-4511 is hsa-miR-4511, miR-4318 is hsa-miR-4318, miR-4653-5p is hsa-miR-4653-5p, miR-6867-3p is hsa-miR-6867-3p, miR-133b is hsa-miR-133b, miR-3196 is hsa-miR-3196, miR-193b-3p is hsa-miR-193b-3p, miR-3162-3p is hsa-miR-3162-3p, miR-6819-3p is hsa-miR-6819-3p, miR-1908-3p is hsa-miR-1908-3p, miR-6786-5p is hsa-miR-6786-5p, miR-3648 is hsa-miR-3648, miR-4513 is hsa-miR-4513, miR-3652 is hsa-miR-3652, miR-4640-5p is hsa-miR-4640-5p, miR-6871-5p is hsa-miR-6871-5p, miR-7845-5p is hsa-miR-7845-5p, miR-3138 is hsa-miR-3138, miR-6884-5p is hsa-miR-6884-5p, miR-4653-3p is hsa-miR-4653-3p, miR-636 is hsa-miR-636, miR-4652-3p is hsa-miR-4652-3p, miR-6823-5p is hsa-miR-6823-5p, miR-4502 is hsa-miR-4502, miR-7113-5p is hsa-miR-7113-5p, miR-8087 is hsa-miR-8087, miR-7154-3p is hsa-miR-7154-3p, miR-5189-5p is hsa-miR-5189-5p, miR-1253 is hsa-miR-1253, miR-518c-5p is hsa-miR-518c-5p, miR-7151-5p is hsa-miR-7151-5p, miR-3614-3p is hsa-miR-3614-3p, miR-4727-5p is hsa-miR-4727-5p, miR-3682-5p is hsa-miR-3682-5p, miR-5090 is hsa-miR-5090, miR-337-3p is hsa-miR-337-3p, miR-488-5p is hsa-miR-488-5p, miR-100-5p is hsa-miR-100-5p, miR-4520-3p is hsa-miR-4520-3p, miR-373-3p is hsa-miR-373-3p, miR-6499-5p is hsa-miR-6499-5p, miR-3909 is hsa-miR-3909, miR-32-5p is hsa-miR-32-5p, miR-302a-3p is hsa-miR-302a-3p, miR-4686 is hsa-miR-4686, miR-4659a-3p is hsa-miR-4659a-3p, miR-4287 is hsa-miR-4287, miR-1301-5p is hsa-miR-1301-5p, miR-593-3p is hsa-miR-593-3p, miR-517a-3p is hsa-miR-517a-3p, miR-517b-3p is hsa-miR-517b-3p, miR-142-3p is hsa-miR-142-3p, miR-1185-2-3p is hsa-miR-1185-2-3p, miR-602 is hsa-miR-602, miR-527 is hsa-miR-527, miR-518a-5p is hsa-miR-518a-5p, miR-4682 is hsa-miR-4682, miR-28-5p is hsa-miR-28-5p, miR-4252 is hsa-miR-4252, miR-452-5p is hsa-miR-452-5p, miR-525-5p is hsa-miR-525-5p, miR-3622a-3p is hsa-miR-3622a-3p, miR-6813-3p is hsa-miR-6813-3p, miR-4769-3p is hsa-miR-4769-3p, miR-5698 is hsa-miR-5698, miR-1915-3p is hsa-miR-1915-3p, miR-1343-5p is hsa-miR-1343-5p, miR-6861-5p is hsa-miR-6861-5p, miR-6781-5p is hsa-miR-6781-5p, miR-4508 is hsa-miR-4508, miR-6743-5p is hsa-miR-6743-5p, miR-6726-5p is hsa-miR-6726-5p, miR-4525 is hsa-miR-4525, miR-4651 is hsa-miR-4651, miR-6813-5p is hsa-miR-6813-5p, miR-5787 is hsa-miR-5787, miR-1290 is hsa-miR-1290, miR-6075 is hsa-miR-6075, miR-4758-5p is hsa-miR-4758-5p, miR-4690-5p is hsa-miR-4690-5p, miR-762 is hsa-miR-762, miR-1225-3p is hsa-miR-1225-3p, miR-3184-5p is hsa-miR-3184-5p, miR-665 is hsa-miR-665, miR-211-5p is hsa-miR-211-5p, miR-1247-5p is hsa-miR-1247-5p, miR-3656 is hsa-miR-3656, miR-149-5p is hsa-miR-149-5p, miR-744-5p is hsa-miR-744-5p, miR-345-5p is hsa-miR-345-5p, miR-150-5p is hsa-miR-150-5p, miR-191-3p is hsa-miR-191-3p, miR-651-5p is hsa-miR-651-5p, miR-34a-5p is hsa-miR-34a-5p, miR-409-5p is hsa-miR-409-5p, miR-369-5p is hsa-miR-369-5p, miR-1915-5p is hsa-miR-1915-5p, miR-204-5p is hsa-miR-204-5p, miR-137 is hsa-miR-137, miR-382-5p is hsa-miR-382-5p, miR-517-5p is hsa-miR-517-5p, miR-532-5p is hsa-miR-532-5p, miR-22-5p is hsa-miR-22-5p, miR-1237-3p is hsa-miR-1237-3p, miR-1224-3p is hsa-miR-1224-3p, miR-625-3p is hsa-miR-625-3p, miR-328-3p is hsa-miR-328-3p, miR-122-5p is hsa-miR-122-5p, miR-202-3p is hsa-miR-202-3p, miR-4781-5p is hsa-miR-4781-5p, miR-718 is hsa-miR-718, miR-342-3p is hsa-miR-342-3p, miR-26b-3p is hsa-miR-26b-3p, miR-140-3p is hsa-miR-140-3p, miR-200a-3p is hsa-miR-200a-3p, miR-378a-3p is hsa-miR-378a-3p, miR-484 is hsa-miR-484, miR-296-5p is hsa-miR-296-5p, miR-205-5p is hsa-miR-205-5p, miR-431-5p is hsa-miR-431-5p, miR-1471 is hsa-miR-1471, miR-1538 is hsa-miR-1538, miR-449b-3p is hsa-miR-449b-3p, miR-1976 is hsa-miR-1976, miR-4268 is hsa-miR-4268, miR-4279 is hsa-miR-4279, miR-3620-3p is hsa-miR-3620-3p, miR-3944-3p is hsa-miR-3944-3p, miR-3156-3p is hsa-miR-3156-3p, miR-3187-5p is hsa-miR-3187-5p, miR-4685-3p is hsa-miR-4685-3p, miR-4695-3p is hsa-miR-4695-3p, miR-4697-3p is hsa-miR-4697-3p, miR-4713-5p is hsa-miR-4713-5p, miR-4723-3p is hsa-miR-4723-3p, miR-371b-3p is hsa-miR-371b-3p, miR-3151-3p is hsa-miR-3151-3p, miR-3192-3p is hsa-miR-3192-3p, miR-6728-3p is hsa-miR-6728-3p, miR-6736-3p is hsa-miR-6736-3p, miR-6740-3p is hsa-miR-6740-3p, miR-6741-3p is hsa-miR-6741-3p, miR-6743-3p is hsa-miR-6743-3p, miR-6747-3p is hsa-miR-6747-3p, miR-6750-3p is hsa-miR-6750-3p, miR-6754-3p is hsa-miR-6754-3p, miR-6759-3p is hsa-miR-6759-3p, miR-6761-3p is hsa-miR-6761-3p, miR-6762-3p is hsa-miR-6762-3p, miR-6769a-3p is hsa-miR-6769a-3p, miR-6776-3p is hsa-miR-6776-3p, miR-6778-3p is hsa-miR-6778-3p, miR-6779-3p is hsa-miR-6779-3p, miR-6786-3p is hsa-miR-6786-3p, miR-6787-3p is hsa-miR-6787-3p, miR-6792-3p is hsa-miR-6792-3p, miR-6794-3p is hsa-miR-6794-3p, miR-6801-3p is hsa-miR-6801-3p, miR-6802-3p is hsa-miR-6802-3p, miR-6803-3p is hsa-miR-6803-3p, miR-6804-3p is hsa-miR-6804-3p, miR-6810-5p is hsa-miR-6810-5p, miR-6823-3p is hsa-miR-6823-3p, miR-6825-3p is hsa-miR-6825-3p, miR-6829-3p is hsa-miR-6829-3p, miR-6833-3p is hsa-miR-6833-3p, miR-6834-3p is hsa-miR-6834-3p, miR-6780b-3p is hsa-miR-6780b-3p, miR-6845-3p is hsa-miR-6845-3p, miR-6862-3p is hsa-miR-6862-3p, miR-6865-3p is hsa-miR-6865-3p, miR-6870-3p is hsa-miR-6870-3p, miR-6875-3p is hsa-miR-6875-3p, miR-6877-3p is hsa-miR-6877-3p, miR-6879-3p is hsa-miR-6879-3p, miR-6882-3p is hsa-miR-6882-3p, miR-6885-3p is hsa-miR-6885-3p, miR-6886-3p is hsa-miR-6886-3p, miR-6887-3p is hsa-miR-6887-3p, miR-6890-3p is hsa-miR-6890-3p, miR-6893-3p is hsa-miR-6893-3p, miR-6894-3p is hsa-miR-6894-3p, miR-7106-3p is hsa-miR-7106-3p, miR-7109-3p is hsa-miR-7109-3p, miR-7114-3p is hsa-miR-7114-3p, miR-7155-5p is hsa-miR-7155-5p, miR-7160-5p is hsa-miR-7160-5p, miR-615-3p is hsa-miR-615-3p, miR-920 is hsa-miR-920, miR-1825 is hsa-miR-1825, miR-675-3p is hsa-miR-675-3p, miR-1910-5p is hsa-miR-1910-5p, miR-2278 is hsa-miR-2278, miR-2682-3p is hsa-miR-2682-3p, miR-3122 is hsa-miR-3122, miR-3151-5p is hsa-miR-3151-5p, miR-3175 is hsa-miR-3175, miR-4323 is hsa-miR-4323, miR-4326 is hsa-miR-4326, miR-4284 is hsa-miR-4284, miR-3605-3p is hsa-miR-3605-3p, miR-3622b-5p is hsa-miR-3622b-5p, miR-3646 is hsa-miR-3646, miR-3158-5p is hsa-miR-3158-5p, miR-4722-3p is hsa-miR-4722-3p, miR-4728-3p is hsa-miR-4728-3p, miR-4747-3p is hsa-miR-4747-3p, miR-4436b-5p is hsa-miR-4436b-5p, miR-5196-3p is hsa-miR-5196-3p, miR-5589-5p is hsa-miR-5589-5p, miR-345-3p is hsa-miR-345-3p, miR-642b-5p is hsa-miR-642b-5p, miR-6716-3p is hsa-miR-6716-3p, miR-6511b-3p is hsa-miR-6511b-3p, miR-208a-5p is hsa-miR-208a-5p, miR-6726-3p is hsa-miR-6726-3p, miR-6744-5p is hsa-miR-6744-5p, miR-6782-3p is hsa-miR-6782-3p, miR-6789-3p is hsa-miR-6789-3p, miR-6797-3p is hsa-miR-6797-3p, miR-6800-3p is hsa-miR-6800-3p, miR-6806-5p is hsa-miR-6806-5p, miR-6824-3p is hsa-miR-6824-3p, miR-6837-5p is hsa-miR-6837-5p, miR-6846-3p is hsa-miR-6846-3p, miR-6858-3p is hsa-miR-6858-3p, miR-6859-3p is hsa-miR-6859-3p, miR-6861-3p is hsa-miR-6861-3p, miR-6880-3p is hsa-miR-6880-3p, miR-7111-3p is hsa-miR-7111-3p, miR-7152-5p is hsa-miR-7152-5p, miR-642a-5p is hsa-miR-642a-5p, miR-657 is hsa-miR-657, miR-1236-3p is hsa-miR-1236-3p, miR-764 is hsa-miR-764, miR-4314 is hsa-miR-4314, miR-3675-3p is hsa-miR-3675-3p, miR-5703 is hsa-miR-5703, miR-3191-5p is hsa-miR-3191-5p, miR-6511a-3p is hsa-miR-6511a-3p, miR-6809-3p is hsa-miR-6809-3p, miR-6815-5p is hsa-miR-6815-5p, miR-6857-3p is hsa-miR-6857-3p, miR-6878-3p is hsa-miR-6878-3p, miR-371a-5p is hsa-miR-371a-5p, miR-766-3p is hsa-miR-766-3p, miR-1229-3p is hsa-miR-1229-3p, miR-1306-5p is hsa-miR-1306-5p, miR-210-5p is hsa-miR-210-5p, miR-198 is hsa-miR-198, miR-485-3p is hsa-miR-485-3p, miR-668-3p is hsa-miR-668-3p, miR-532-3p is hsa-miR-532-3p, miR-877-3p is hsa-miR-877-3p, miR-1238-3p is hsa-miR-1238-3p, miR-3130-5p is hsa-miR-3130-5p, miR-4298 is hsa-miR-4298, miR-4290 is hsa-miR-4290, miR-3943 is hsa-miR-3943, miR-346 is hsa-miR-346, miR-767-3p is hsa-miR-767-3p, miR-516a-5p is hsa-miR-516a-5p, miR-769-3p is hsa-miR-769-3p, miR-3692-5p is hsa-miR-3692-5p, miR-3945 is hsa-miR-3945, miR-4433a-3p is hsa-miR-4433a-3p, miR-4485-3p is hsa-miR-4485-3p, miR-6831-5p is hsa-miR-6831-5p, miR-519c-5p is hsa-miR-519c-5p, miR-551b-5p is hsa-miR-551b-5p, miR-1343-3p is hsa-miR-1343-3p, miR-4286 is hsa-miR-4286, miR-4634 is hsa-miR-4634, miR-4733-3p is hsa-miR-4733-3p, miR-6086 is hsa-miR-6086, miR-30d-5p is hsa-miR-30d-5p, miR-30b-3p is hsa-miR-30b-3p, miR-92a-3p is hsa-miR-92a-3p, miR-371b-5p is hsa-miR-371b-5p, and miR-486-5p is hsa-miR-486-5p.

In another preferable aspect of a kit or device for detection of dementia or a method for detecting dementia according to the present invention, dementia marker miR-6765-3p is hsa-miR-6765-3p, miR-6784-5p is hsa-miR-6784-5p, miR-5698 is hsa-miR-5698, miR-6778-5p is hsa-miR-6778-5p, miR-1915-3p is hsa-miR-1915-3p, miR-6875-5p is hsa-miR-6875-5p, miR-1343-5p is hsa-miR-1343-5p, miR-4534 is hsa-miR-4534, miR-6861-5p is hsa-miR-6861-5p, miR-4721 is hsa-miR-4721, miR-6756-5p is hsa-miR-6756-5p, miR-615-5p is hsa-miR-615-5p, miR-6727-5p is hsa-miR-6727-5p, miR-6887-5p is hsa-miR-6887-5p, miR-8063 is hsa-miR-8063, miR-6880-5p is hsa-miR-6880-5p, miR-6805-3p is hsa-miR-6805-3p, miR-6781-5p is hsa-miR-6781-5p, miR-4508 is hsa-miR-4508, miR-4726-5p is hsa-miR-4726-5p, miR-4710 is hsa-miR-4710, miR-7111-5p is hsa-miR-7111-5p, miR-3619-3p is hsa-miR-3619-3p, miR-6743-5p is hsa-miR-6743-5p, miR-6795-5p is hsa-miR-6795-5p, miR-6726-5p is hsa-miR-6726-5p, miR-4525 is hsa-miR-4525, miR-1254 is hsa-miR-1254, miR-1233-5p is hsa-miR-1233-5p, miR-4651 is hsa-miR-4651, miR-6836-3p is hsa-miR-6836-3p, miR-6769a-5p is hsa-miR-6769a-5p, miR-6813-5p is hsa-miR-6813-5p, miR-4532 is hsa-miR-4532, miR-365a-5p is hsa-miR-365a-5p, miR-1231 is hsa-miR-1231, miR-5787 is hsa-miR-5787, miR-1290 is hsa-miR-1290, miR-1228-5p is hsa-miR-1228-5p, miR-371a-5p is hsa-miR-371a-5p, miR-4430 is hsa-miR-4430, miR-296-3p is hsa-miR-296-3p, miR-6075 is hsa-miR-6075, miR-1237-5p is hsa-miR-1237-5p, miR-4758-5p is hsa-miR-4758-5p, miR-4690-5p is hsa-miR-4690-5p, miR-4466 is hsa-miR-4466, miR-6789-5p is hsa-miR-6789-5p, miR-4632-5p is hsa-miR-4632-5p, miR-4745-5p is hsa-miR-4745-5p, miR-4665-5p is hsa-miR-4665-5p, miR-6807-5p is hsa-miR-6807-5p, miR-762 is hsa-miR-762, miR-7114-5p is hsa-miR-7114-5p, miR-150-3p is hsa-miR-150-3p, miR-423-5p is hsa-miR-423-5p, miR-575 is hsa-miR-575, miR-671-5p is hsa-miR-671-5p, miR-939-5p is hsa-miR-939-5p, miR-1225-3p is hsa-miR-1225-3p, miR-3184-5p is hsa-miR-3184-5p, and miR-3665 is hsa-miR-3665.

\<Definition of Terms\>

The terms used herein are defined as described below.

Abbreviations or terms such as nucleotide, polynucleotide, DNA, and RNA abide by "Guidelines for the preparation of specification which contain nucleotide and/or amino acid sequences" (edited by Japan Patent Office) and common use in the art.

The term "polynucleotide" used herein refers to a nucleic acid including any of RNA, DNA, and RNA/DNA (chimera). The DNA includes any of cDNA, genomic DNA, and synthetic DNA. The RNA includes any of total RNA, mRNA, rRNA, miRNA, siRNA, snoRNA, snRNA, non-coding RNA and synthetic RNA. Here the "synthetic DNA" and the "synthetic RNA" refer to a DNA and an RNA artificially prepared using, for example, an automatic nucleic acid synthesizer, on the basis of predetermined nucleotide sequences (which may be any of natural and non-natural sequences). The "non-natural sequence" is intended to be used in a broad sense and includes, for example, a sequence comprising substitution, deletion, insertion, and/or addition of one or more nucleotides (i.e., a variant sequence) and a sequence comprising one or more modified nucleotides (i.e., a modified sequence), which are different from the natural sequence. Herein, the term "polynucleotide" is used interchangeably with the term "nucleic acid."

The term "fragment" used herein is a polynucleotide having a nucleotide sequence that consists of a consecutive portion of a polynucleotide and desirably has a length of 15 or more nucleotides, preferably 17 or more nucleotides, more preferably 19 or more nucleotides.

The term "gene" used herein is intended to include not only RNA and double-stranded DNA but also each single-stranded DNA such as a plus(+) strand (or a sense strand) or a complementary strand (or an antisense strand) constituting the duplex. The gene is not particularly limited by its length.

Thus, the "gene" used herein includes any of double-stranded DNA including human genomic DNA, single-stranded DNA (plus strand), single-stranded DNA having a sequence complementary to the plus strand (complementary strand), cDNA, microRNA (miRNA), their fragments, and human genome, and their transcripts, unless otherwise specified. The "gene" includes not only a "gene" represented by a particular nucleotide sequence (or SEQ ID NO) but also "nucleic acids" encoding RNAs having biological functions equivalent to RNA encoded by the gene, for example, a congener (i.e., a homolog or an ortholog), a variant (e.g., a genetic polymorph), and a derivative. Specific examples of such a "nucleic acid" encoding a congener, a variant, or a derivative can include a "nucleic acid" having a nucleotide sequence hybridizing under stringent conditions described later to a complementary sequence of a nucleotide sequence represented by any of SEQ ID NOs: 1 to 1314, 1315 to 1434, and 1435 to 1505 (e.g., SEQ ID NOs: 194 to 212, 374, 398, 490, 591, 593 to 609, 766, 1015 to 1017, 1019 to 1024, 1026 to 1031, 1285 to 1286, and 1315 to 1434) or a nucleotide sequence derived from the nucleotide sequence by the replacement of u with t. Regardless whether or not there is a difference in functional region, the "gene" can comprise, for example, expression control regions, coding region, exons, or introns. The "gene" may be contained in a cell or may exist alone after being released from a cell. Alternatively, the "gene" may be in a state enclosed in a vesicle called exosome.

The term "exosome" used herein is a vesicle that is encapsulated by lipid bilayer and secreted from a cell. The exosome is derived from a multivesicular endosome and may incorporate biomaterials such as "genes" (e.g., RNA or DNA) or proteins when released into an extracellular environment. The exosome is known to be contained in a body fluid such as blood, serum, plasma or lymph.

The term "transcript" used herein refers to an RNA synthesized from the DNA sequence of a gene as a template. RNA polymerase binds to a site called promoter located upstream of the gene and adds ribonucleotides complementary to the nucleotide sequence of the DNA to the 3' end to synthesize an RNA. This RNA contains not only the gene itself but also the whole sequence from a transcription initiation site to the end of a polyA sequence, including expression control regions, coding region, exons, or introns.

Unless otherwise specified, the term "microRNA (miRNA)" used herein is intended to mean a 15- to 25-nucleotide non-coding RNA that is transcribed as an RNA precursor having a hairpin-like structure, cleaved by a dsRNA-cleaving enzyme having RNase III cleavage activity, and integrated into a protein complex called RISC, and that is involved in the suppression of translation of mRNA. The term "miRNA" used herein includes not only a "miRNA" represented by a particular nucleotide sequence (or SEQ ID NO) but also a "miRNA" comprising a precursor of the "miRNA" (pre-miRNA or pri-miRNA) and having biological functions equivalent to miRNAs encoded by these, for example, a "miRNA" encoding a congener (i.e., a homolog or an ortholog), a variant such as a genetic polymorph, and a derivative. Such a "miRNA" encoding a precursor, a congener, a variant, or a derivative can be specifically identified using "miRBase" (version 21), and examples thereof can include a "miRNA" having a nucleotide sequence hybridizing under stringent conditions described later to a complementary sequence of any particular nucleotide sequence represented by any of SEQ ID NOs: 1 to 1314, 1315 to 1434, and 1435 to 1505 (e.g., SEQ ID NOs: 194 to 212, 374, 398, 490, 591, 593 to 609, 766, 1015 to 1017, 1019 to 1024, 1026 to 1031, 1285 to 1286, and 1315 to 1434). Note that the "miRBase" (version 21) (http://www.mirbase.org/) is a database on the Web that provides miRNA nucleotide sequences, annotations, and prediction of their target genes. All the miRNAs registered in the "miRBase" have been cloned or limited to miRNAs expressed *in vivo* and processed. The term "miRNA" used herein may be a gene product of a miR gene. Such a gene product includes a mature miRNA (e.g., a 15- to 25-nucleotide or 19- to 25-nucleotide non-coding RNA involved in the suppression of translation of mRNA as described above) or a miRNA precursor (e.g., pre-miRNA or pri-miRNA as described above).

The term "probe" used herein includes a polynucleotide that is used for specifically detecting an RNA resulting from the expression of a gene or a polynucleotide derived from the RNA, and/or a polynucleotide complementary thereto.

The term "primer" used herein includes consecutive polynucleotides that specifically recognize and amplify an RNA resulting from the expression of a gene or a polynucleotide derived from the RNA, and/or a polynucleotide complementary thereto.

In this context, the "complementary polynucleotide" (complementary strand or reverse strand) means a polynucleotide in a complementary relationship based on A:T (U) and G:C base pairs with the full-length sequence of a polynucleotide consisting of a nucleotide sequence defined by any of SEQ ID NOs: 1 to 1314, 1315 to 1434, and 1435 to 1505 (e.g., SEQ ID NOs: 194 to 212, 374, 398, 490, 591, 593 to 609, 766, 1015 to 1017, 1019 to 1024, 1026 to 1031, 1285 to 1286, and 1315 to 1434) or a nucleotide sequence derived from the nucleotide sequence by the replacement of u with t, or a partial sequence thereof (here, this full-length or partial sequence is referred to as a plus strand for the sake of convenience). However, such a complementary strand is not limited to a sequence completely complementary to the nucleotide sequence of the target plus strand and may have a complementary relationship to an extent that permits hybridization under stringent conditions to the target plus strand.

The term "stringent conditions" used herein refers to conditions under which a nucleic acid probe hybridizes to its target sequence to a detectably larger extent (e.g., a measurement value equal to or larger than "(a mean of background measurement values) + (a standard deviation of the background measurement values) $\times$ 2") than that for other sequences. The stringent conditions are dependent on a sequence and differ depending on an environment where hybridization is performed. A target sequence complementary 100% to the nucleic acid probe can be identified by controlling the stringency of hybridization and/or washing conditions. Specific examples of the "stringent conditions" will be mentioned later.

The term "Tm value" used herein means a temperature at which the double-stranded moiety of a polynucleotide is denatured into single strands so that the double strands and the single strands exist at a ratio of 1:1.

The term "variant" used herein means, in the case of a nucleic acid, a natural variant attributed to polymorphism, mutation, or the like; a variant containing the deletion, substitution, addition, or insertion of 1, 2 or 3 or more (e.g., 1 to several) nucleotides in a nucleotide sequence represented by a SEQ ID NO or a nucleotide sequence derived from the nucleotide sequence by the replacement of u with t, or a partial sequence thereof; a variant containing the deletion, substitution, addition, or insertion of one or more nucleotides in a nucleotide sequence of a premature miRNA of the sequence of any of SEQ ID NOs: 1 to 210, 211 to 249, 250 to 374, and 375 to 390, 1315 to 1350, 1351 to 1356, 1435 to 1448, and 1449 to 1453 or a nucleotide sequence derived from the nucleotide sequence by the replacement of u with t, or a partial sequence thereof; a variant that exhibits percent (%) identity of approximately 90% or higher, approximately 95% or higher, approximately 97% or higher, approximately 98% or higher, approximately 99% or higher to each of these nucleotide sequences or the partial sequences thereof; or a nucleic acid hybridizing under the stringent conditions defined above to a polynucleotide or an oligonucleotide comprising each of these nucleotide sequences or the partial sequences thereof.

The term "several" used herein means an integer of approximately 10, 9, 8, 7, 6, 5, 4, 3, or 2.

The variant as used herein can be prepared by use of a well-known technique such as site-directed mutagenesis or mutagenesis using PCR.

The term "percent (%) identity" used herein can be determined with or without an introduced gap, using a protein or gene search system based on BLAST (https://blast.ncbi.nlm.nih.gov/Blast.cgi) or FASTA (http://www.genome.jp/tools/fasta/) (Zheng Zhang et al., 2000, J. Comput. Biol., Vol. 7, p. 203-214; Altschul, S.F. et al., 1990, Journal of Molecular Biology, Vol. 215, p. 403-410; and Pearson, W.R. et al., 1988, Proc. Natl. Acad. Sci. U.S.A., Vol. 85, p. 2444-2448).

The term "derivative" used herein is meant to include unlimitedly a modified nucleic acid, for example, a derivative labeled with a fluorophore or the like, a derivative containing a modified nucleotide (e.g., a nucleotide containing a group such as halogen, alkyl such as methyl, alkoxy such as methoxy, thio, or carboxymethyl, and a nucleotide that has undergone base rearrangement, double bond saturation, deamination, replacement of an oxygen molecule with a sulfur molecule, etc.), PNA (peptide nucleic acid; Nielsen, P.E. et al., 1991, Science, Vol. 254, p. 1497-500), and LNA (locked nucleic acid; Obika, S. et al., 1998, Tetrahedron Lett., Vol. 39, p. 5401-5404).

As used herein, the "nucleic acid" capable of specifically binding to a polynucleotide selected from the dementia marker miRNAs described above or to a complementary strand of the polynucleotide is a synthesized or prepared nucleic acid and, for example, includes a "nucleic acid probe" or a "primer", and is utilized directly or indirectly for detecting the presence or absence of dementia in a subject, for diagnosing the presence or absence or the severity of dementia, the presence or absence or the degree of amelioration of dementia, or the therapeutic sensitivity of dementia, or for screening for a candidate substance useful in the prevention, amelioration, or treatment of dementia. The "nucleic acid" includes a nucleotide, an oligonucleotide, and a polynucleotide capable of specifically recognizing and binding to a transcript represented by any of SEQ ID NOs: 1 to 1314, 1315 to 1434, and 1435 to 1505 (e.g., SEQ ID NOs: 194 to 212, 374, 398, 490, 591, 593 to 609, 766, 1015 to 1017, 1019 to 1024, 1026 to 1031, 1285 to 1286, and 1315 to 1434) or a synthetic cDNA nucleic acid thereof, or a complementary strand thereto *in vivo,* particularly, in a sample such as a body fluid (e.g., blood or urine), in relation to the development of dementia. The nucleotide, the oligonucleotide, and the polynucleotide can be effectively used as probes for detecting the aforementioned gene expressed *in vivo,* in tissues, in cells, or the like on the basis of the properties described above, or as primers for amplifying the aforementioned gene expressed *in vivo.*

The term "detection" used herein is interchangeable with the term "examination", "measurement", "detection", or "decision support". As used herein, the term "evaluation" is meant to include diagnosing- or evaluation-supporting on the basis of examination results or measurement results.

The term "subject" used herein means a mammal such as a primate including a human and a chimpanzee, a pet animal including a dog and a cat, a livestock animal including cattle, a horse, sheep, and a goat, a rodent including a mouse and a rat, and animals raised in a zoo. The subject is preferably a human. Meanwhile, the "subject with normal cognitive functions" also means such a mammal, which is an animal without dementia to be detected. The subject with normal cognitive functions is preferably a human.

As used herein, the term "dementia" refers to a state in which once normal cognitive functions are persistently lowered due to acquired brain damage. Generally speaking, dementia is diagnosed when the functions decrease to a level where daily living activity and social activity are impaired.

Dementia, in general, is classified, depending on a cause of disease, into disease types such as Alzheimer's dementia, vascular dementia, Lewy body dementia, normal pressure hydrocephalus, and frontotemporal lobar degeneration. Examples of the "dementia" in the present invention includes: Alzheimer's dementia, vascular dementia, Lewy body dementia, normal pressure hydrocephalus, frontotemporal lobar degeneration, senile dementia of the neurofibrillary tangle type, dementia resulting from other diseases (e.g., infectious diseases) alcoholic dementia or vitamin deficiency dementia or the like. In addition, examples of the "dementia" in the present invention also include mixed dementia of any of these types. Further, type-unknown or unspecified dementia is also included in the "dementia" in the present invention.

As used herein, the term "Alzheimer's dementia" refers to dementia in which imagind test such as CT, MRI, and PET shows no abnormal structure in the brain, but atrophy of medial temporal lobe is detected, and as a result of which, as a main symptom of cognitive/memory impairment, short-term memory impairment occurs.

As used herein, the term "vascular dementia" refers to dementia which is accompanied by cerebrovascular disease and in which consciousness of disease and judgment is relatively well preserved while cognitive/memory impairment is ununiform or sporadic and memory and mental capacity are decreased.

As used herein, the term "Lewy body dementia" refers to dementia in which cerebral blood flow SPECT or PET imaging shows a decrease in the uptake of dopamine across a transporter in the basal ganglia and which is accompanied by, as a symptom, fluctuating cognitive impairment, hallucination in which a specific detailed event appears repeatedly, and/or parkinsonism.

As used herein, the term "normal pressure hydrocephalus" refers to dementia in which spinal fluid accumulates in the cerebral ventricle and then pressurizes the surrounding brain, causing cognitive function impairment, gait disturbance, and/or dysuria. This dementia can be ameliorated by surgical treatment.

As used herein, the term "frontotemporal lobar degeneration" refers to dementia in which frontal and temporal lobes are apparently atrophied, which causes language disturbance and mental manifestation to be displayed.

The term "P" or "P value" used herein refers to a probability at which a more extreme statistic than that actually calculated from data under null hypothesis is observed in a statistical test. Thus, smaller "P" or "P value" is regarded as being a more significant difference between subjects to be compared.

The term "sensitivity" used herein means a value of (the number of true positives) / (the number of true positives + the number of false negatives). High sensitivity allows dementia to be detected early and enables an early medical intervention.

The term "specificity" used herein means a value of (the number of true negatives) / (the number of true negatives + the number of false positives). High specificity prevents needless extra examination for subjects with normal cognitive functions misjudged as being dementia patients, leading to reduction in burden on patients and reduction in medical expense.

The term "accuracy" used herein means a value of (the number of true positives + the number of true negatives) / (the total number of cases). The accuracy indicates the ratio of samples that are identified correctly to all samples, and serves as a primary index for evaluating detection performance.

As used herein, the "sample" that is subjected to determination, detection, or diagnosis refers to a tissue and a biological material in which the expression of the gene of the present invention varies as dementia develops, as dementia progresses, or as therapeutic effects on dementia are exerted. Specifically, the sample refers to a brain tissue and neurons, a nerve tissue, cerebrospinal fluid, bone marrow aspirate, an organ, skin, and a body fluid such as blood, urine, saliva, sweat, and tissue exudate, serum or plasma prepared from blood, and others such as feces, hair, or the like. The "sample" further refers to a biological sample extracted therefrom, specifically, a gene such as RNA or miRNA.

The term "hsa-miR-4274 gene" or "hsa-miR-4274" used herein includes the hsa-miR-4274 gene (miRBase Accession No. MIMAT0016906) shown in SEQ ID NO: 1, a homolog or an ortholog of a different organism species, and the like. The hsa-miR-4274 gene can be obtained by a method described in Goff LA et al., 2009, PLoS One, Vol. 4, e7192. Also, "hsa-mir-4274" (miRBase Accession No. MI0015884; SEQ ID NO: 391) having a hairpin-like structure is known as a precursor of "hsa-miR-4274."

The term "hsa-miR-4272 gene" or "hsa-miR-4272" used herein includes the hsa-miR-4272 gene (miRBase Accession No. MIMAT0016902) shown in SEQ ID NO: 2, a homolog or an ortholog of a different organism species, and the like. The hsa-miR-4272 gene can be obtained by a method described in Goff LA et al., 2009, PLoS One, Vol. 4, e7192. Also, "hsa-mir-4272" (miRBase Accession No. MI0015880; SEQ ID NO: 392) having a hairpin-like structure is known as a precursor of "hsa-miR-4272."

The term "hsa-miR-4728-5p gene" or "hsa-miR-4728-5p" used herein includes the hsa-miR-4728-5p gene (miRBase Accession No. MIMAT0019849) shown in SEQ ID NO: 3, a homolog or an ortholog of a different organism species, and the like. The hsa-miR-4728-5p gene can be obtained by a method described in Persson H et al., 2011, Cancer Res., Vol. 71, pp. 78-86. Also, "hsa-mir-4728" (miRBase Accession No. MI0017365; SEQ ID NO: 393) having a hairpin-like structure is known as a precursor of "hsa-miR-4728-5p."

The term "hsa-miR-4443 gene" or "hsa-miR-4443" used herein includes the hsa-miR-4443 gene (miRBase Accession No. MIMAT0018961) shown in SEQ ID NO: 4, a homolog or an ortholog of a different organism species, and the like. The hsa-miR-4443 gene can be obtained by a method described in Jima DD et al., 2010, Blood, Vol. 116, e118-e127. Also, "hsa-mir-4443" (miRBase Accession No. MI0016786; SEQ ID NO: 394) having a hairpin-like structure is known as a precursor of "hsa-miR-4443."

The term "hsa-miR-4506 gene" or "hsa-miR-4506" used herein includes the hsa-miR-4506 gene (miRBase Accession No. MIMAT0019042) shown in SEQ ID NO: 5, a homolog or an ortholog of a different organism species, and the like. The hsa-miR-4506 gene can be obtained by a method described in Jima DD et al., 2010, Blood, Vol. 116, e118-e127.

Also, "hsa-mir-4506" (miRBase Accession No. MI0016869; SEQ ID NO: 395) having a hairpin-like structure is known as a precursor of "hsa-miR-4506."

The term "hsa-miR-6773-5p gene" or "hsa-miR-6773-5p" used herein includes the hsa-miR-6773-5p gene (miRBase Accession No. MIMAT0027446) shown in SEQ ID NO: 6, a homolog or an ortholog of a different organism species, and the like. The hsa-miR-6773-5p gene can be obtained by a method described in Ladewig E et al., 2012, Genome Res., Vol. 22, pp. 1634-1645. Also, "hsa-mir-6773" (miRBase Accession No. MI0022618; SEQ ID NO: 396) having a hairpin-like structure is known as a precursor of "hsa-miR-6773-5p."

The term "hsa-miR-4662a-5p gene" or "hsa-miR-4662a-5p" used herein includes the hsa-miR-4662a-5p gene (miRBase Accession No. MIMAT0019731) shown in SEQ ID NO: 7, a homolog or an ortholog of a different organism species, and the like. The hsa-miR-4662a-5p gene can be obtained by a method described in Persson H et al., 2011, Cancer Res., Vol. 71, pp. 78-86. Also, "hsa-mir-4662a" (miRBase Accession No. MI0017290; SEQ ID NO: 397) having a hairpin-like structure is known as a precursor of "hsa-miR-4662a-5p."

The term "hsa-miR-3184-3p gene" or "hsa-miR-3184-3p" used herein includes the hsa-miR-3184-3p gene (miRBase Accession No. MIMAT0022731) shown in SEQ ID NO: 8, a homolog or an ortholog of a different organism species, and the like. The hsa-miR-3184-3p gene can be obtained by a method described in Stark MS et al., 2010, PLoS One, Vol. 5, e9685. Also, "hsa-mir-3184" (miRBase Accession No. MI0014226; SEQ ID NO: 398) having a hairpin-like structure is known as a precursor of "hsa-miR-3184-3p."

The term "hsa-miR-4281 gene" or "hsa-miR-4281" used herein includes the hsa-miR-4281 gene (miRBase Accession No. MIMAT0016907) shown in SEQ ID NO: 9, a homolog or an ortholog of a different organism species, and the like. The hsa-miR-4281 gene can be obtained by a method described in Goff LA et al., 2009, PLoS One, Vol. 4, e7192. Also, "hsa-mir-4281" (miRBase Accession No. MI0015885; SEQ ID NO: 399) having a hairpin-like structure is known as a precursor of "hsa-miR-4281."

The term "hsa-miR-320d gene" or "hsa-miR-320d" used herein includes the hsa-miR-320d gene (miRBase Accession No. MIMAT0006764) shown in SEQ ID NO: 10, a homolog or an ortholog of a different organism species, and the like. The hsa-miR-320d gene can be obtained by a method described in Friedlander MR et al., 2008, Nat. Biotechnol., Vol. 26, 407-415. Also, "hsa-mir-320d-1 and hsa-mir-320d-2" (miRBase Accession Nos. MI0008190 and MI0008192; SEQ ID NOs: 400 and 401) each having a hairpin-like structure are known as precursors of "hsa-miR-320d."

The term "hsa-miR-6729-3p gene" or "hsa-miR-6729-3p" used herein includes the hsa-miR-6729-3p gene (miRBase Accession No. MIMAT0027360) shown in SEQ ID NO: 11, a homolog or an ortholog of a different organism species, and the like. The hsa-miR-6729-3p gene can be obtained by a method described in Ladewig E et al., 2012, Genome Res., Vol. 22, pp. 1634-1645. Also, "hsa-mir-6729" (miRBase Accession No. MI0022574; SEQ ID NO: 402) having a hairpin-like structure is known as a precursor of "hsa-miR-6729-3p."

The term "hsa-miR-5192 gene" or "hsa-miR-5192" used herein includes the hsa-miR-5192 gene (miRBase Accession No. MIMAT0021123) shown in SEQ ID NO: 12, a homolog or an ortholog of a different organism species, and the like. The hsa-miR-5192 gene can be obtained by a method described in Schotte D et al., 2011, Leukemia, Vol. 25, pp. 1389-1399. Also, "hsa-mir-5192" (miRBase Accession No. MI0018171; SEQ ID NO: 403) having a hairpin-like structure is known as a precursor of "hsa-miR-5192."

The term "hsa-miR-6853-5p gene" or "hsa-miR-6853-5p" used herein includes the hsa-miR-6853-5p gene (miRBase Accession No. MIMAT0027606) shown in SEQ ID NO: 13, a homolog or an ortholog of a different organism species, and the like. The hsa-miR-6853-5p gene can be obtained by a method described in Ladewig E et al., 2012, Genome Res., Vol. 22, pp. 1634-1645. Also, "hsa-mir-6853" (miRBase Accession No. MI0022699; SEQ ID NO: 404) having a hairpin-like structure is known as a precursor of "hsa-miR-6853-5p."

The term "hsa-miR-1234-3p gene" or "hsa-miR-1234-3p" used herein includes the hsa-miR-1234-3p gene (miRBase Accession No. MIMAT0005589) shown in SEQ ID NO: 14, a homolog or an ortholog of a different organism species, and the like. The hsa-miR-1234-3p gene can be obtained by a method described in Berezikov E et al., 2007, Mol. Cell, Vol. 28, pp. 328-336. Also, "hsa-mir-1234" (miRBase Accession No. MI0006324; SEQ ID NO: 405) having a hairpin-like structure is known as a precursor of "hsa-miR-1234-3p."

The term "hsa-miR-1233-3p gene" or "hsa-miR-1233-3p" used herein includes the hsa-miR-1233-3p gene (miRBase Accession No. MIMAT0005588) shown in SEQ ID NO: 15, a homolog or an ortholog of a different organism species, and the like. The hsa-miR-1233-3p gene can be obtained by a method described in Berezikov E et al., 2007, Mol. Cell, Vol. 28, pp. 328-336. Also, "hsa-mir-1233-1 and hsa-mir-1233-2" (miRBase Accession Nos. MI0006323 and MI0015973; SEQ ID NOs: 406 and 407) each having a hairpin-like structure are known as precursors of "hsa-miR-1233-3p."

The term "hsa-miR-4539 gene" or "hsa-miR-4539" used herein includes the hsa-miR-4539 gene (miRBase Accession No. MIMAT0019082) shown in SEQ ID NO: 16, a homolog or an ortholog of a different organism species, and the like. The hsa-miR-4539 gene can be obtained by a method described in Jima DD et al., 2010, Blood, Vol. 116, e118-e127. Also, "hsa-mir-4539" (miRBase Accession No. MI0016910; SEQ ID NO: 408) having a hairpin-like structure is known as a precursor of "hsa-miR-4539."

The term "hsa-miR-3914 gene" or "hsa-miR-3914" used herein includes the hsa-miR-3914 gene (miRBase Accession No. MIMAT0018188) shown in SEQ ID NO: 17, a homolog or an ortholog of a different organism species, and the like. The hsa-miR-3914 gene can be obtained by a method described in Creighton CJ et al., 2010, PLoS One, Vol. 5, e9637. Also, "hsa-mir-3914-1 and hsa-mir-3914-2" (miRBase Accession Nos. MI0016419 and MI0016421; SEQ ID NOs: 409 and 410) each having a hairpin-like structure are known as precursors of "hsa-miR-3914."

The term "hsa-miR-4738-5p gene" or "hsa-miR-4738-5p" used herein includes the hsa-miR-4738-5p gene (miRBase Accession No. MIMAT0019866) shown in SEQ ID NO: 18, a homolog or an ortholog of a different organism species, and the like. The hsa-miR-4738-5p gene can be obtained by a method described in Persson H et al., 2011, Cancer Res., Vol. 71, pp. 78-86. Also, "hsa-mir-4738" (miRBase Accession No. MI0017376; SEQ ID NO: 411) having a hairpin-like structure is known as a precursor of "hsa-miR-4738-5p."

The term "hsa-miR-548au-3p gene" or "hsa-miR-548au-3p" used herein includes the hsa-miR-548au-3p gene (miRBase Accession No. MIMAT0022292) shown in SEQ ID NO: 19, a homolog or an ortholog of a different organism species, and the like. The hsa-miR-548au-3p gene can be obtained by a method described in Friedlander MR et al., 2012, Nucleic Acids Res., Vol. 40, pp. 37-52. Also, "hsa-mir-548au" (miRBase Accession No. MI0019145; SEQ ID NO: 412) having a hairpin-like structure is known as a precursor of "hsa-miR-548au-3p."

The term "hsa-miR-1539 gene" or "hsa-miR-1539" used herein includes the hsa-miR-1539 gene (miRBase Accession No. MIMAT0007401) shown in SEQ ID NO: 20, a homolog or an ortholog of a different organism species, and the like. The hsa-miR-1539 gene can be obtained by a method described in Azuma-Mukai, A et al., 2008, Proc. Natl. Acad. Sci. U.S.A., Vol. 105, pp. 7964-7969. Also, "hsa-mir-1539" (miRBase Accession No. MI0007260; SEQ ID NO: 413) having a hairpin-like structure is known as a precursor of "hsa-miR-1539."

The term "hsa-miR-4720-3p gene" or "hsa-miR-4720-3p" used herein includes the hsa-miR-4720-3p gene (miRBase Accession No. MIMAT0019834) shown in SEQ ID NO: 21, a homolog or an ortholog of a different organism species, and the like. The hsa-miR-4720-3p gene can be obtained by a method described in Persson H et al., 2011, Cancer Res., Vol. 71, pp. 78-86. Also, "hsa-mir-4720" (miRBase Accession No. MI0017355; SEQ ID NO: 414) having a hairpin-like structure is known as a precursor of "hsa-miR-4720-3p."

The term "hsa-miR-365b-5p gene" or "hsa-miR-365b-5p" used herein includes the hsa-miR-365b-5p gene (miRBase Accession No. MIMAT0022833) shown in SEQ ID NO: 22, a homolog or an ortholog of a different organism species, and the like. The hsa-miR-365b-5p gene can be obtained by a method described in Xie X et al., 2005, Nature, Vol. 434, pp. 338-345. Also, "hsa-mir-365b" (miRBase Accession No. MI0000769; SEQ ID NO: 415) having a hairpin-like structure is known as a precursor of "hsa-miR-365b-5p."

The term "hsa-miR-4486 gene" or "hsa-miR-4486" used herein includes the hsa-miR-4486 gene (miRBase Accession No. MIMAT0019020) shown in SEQ ID NO: 23, a homolog or an ortholog of a different organism species, and the like. The hsa-miR-4486 gene can be obtained by a method described in Jima DD et al., 2010, Blood, Vol. 116, e118-e127. Also, "hsa-mir-4486" (miRBase Accession No. MI0016847; SEQ ID NO: 416) having a hairpin-like structure is known as a precursor of "hsa-miR-4486."

The term "hsa-miR-1227-5p gene" or "hsa-miR-1227-5p" used herein includes the hsa-miR-1227-5p gene (miRBase Accession No. MIMAT0022941) shown in SEQ ID NO: 24, a homolog or an ortholog of a different organism species, and the like. The hsa-miR-1227-5p gene can be obtained by a method described in Berezikov E et al., 2007, Mol. Cell., Vol. 28, pp. 328-336. Also, "hsa-mir-1227" (miRBase Accession No. MI0006316; SEQ ID NO: 417) having a hairpin-like structure is known as a precursor of "hsa-miR-1227-5p."

The term "hsa-miR-4667-5p gene" or "hsa-miR-4667-5p" used herein includes the hsa-miR-4667-5p gene (miRBase Accession No. MIMAT0019743) shown in SEQ ID NO: 25, a homolog or an ortholog of a different organism species, and the like. The hsa-miR-4667-5p gene can be obtained by a method described in Persson H et al., 2011, Cancer Res., Vol. 71, pp. 78-86. Also, "hsa-mir-4667" (miRBase Accession No. MI0017297; SEQ ID NO: 418) having a hairpin-like structure is known as a precursor of "hsa-miR-4667-5p."

The term "hsa-miR-6088 gene" or "hsa-miR-6088" used herein includes the hsa-miR-6088 gene (miRBase Accession No. MIMAT0023713) shown in SEQ ID NO: 26, a homolog or an ortholog of a different organism species, and the like. The hsa-miR-6088 gene can be obtained by a method described in Yoo JK et al., 2012, Stem Cells Dev., Vol. 21, pp. 2049-2057. Also, "hsa-mir-6088" (miRBase Accession No. MI0020365; SEQ ID NO: 419) having a hairpin-like structure is known as a precursor of "hsa-miR-6088."

The term "hsa-miR-6820-5p gene" or "hsa-miR-6820-5p" used herein includes the hsa-miR-6820-5p gene (miRBase Accession No. MIMAT0027540) shown in SEQ ID NO: 27, a homolog or an ortholog of a different organism species, and the like. The hsa-miR-6820-5p gene can be obtained by a method described in Ladewig E et al., 2012, Genome Res., Vol. 22, pp. 1634-1645. Also, "hsa-mir-6820" (miRBase Accession No. MI0022665; SEQ ID NO: 420) having a hairpin-like structure is known as a precursor of "hsa-miR-6820-5p."

The term "hsa-miR-4505 gene" or "hsa-miR-4505" used herein includes the hsa-miR-4505 gene (miRBase Accession No. MIMAT0019041) shown in SEQ ID NO: 28, a homolog or an ortholog of a different organism species, and the like. The hsa-miR-4505 gene can be obtained by a method described in Jima DD et al., 2010, Blood, Vol. 116, e118-e127. Also, "hsa-mir-4505" (miRBase Accession No. MI0016868; SEQ ID NO: 421) having a hairpin-like structure is known as a precursor of "hsa-miR-4505."

The term "hsa-miR-548q gene" or "hsa-miR-548q" used herein includes the hsa-miR-548q gene (miRBase Accession No. MIMAT0011163) shown in SEQ ID NO: 29, a homolog or an ortholog of a different organism species, and the like. The hsa-miR-548q gene can be obtained by a method described in Wyman SK et al., 2009, PLoS One, Vol. 4, e5311. Also, "hsa-mir-548q" (miRBase Accession No. MI0010637; SEQ ID NO: 422) having a hairpin-like structure is known as a precursor of "hsa-miR-548q."

The term "hsa-miR-4658 gene" or "hsa-miR-4658" used herein includes the hsa-miR-4658 gene (miRBase Accession No. MIMAT0019725) shown in SEQ ID NO: 30, a homolog or an ortholog of a different organism species, and the like. The hsa-miR-4658 gene can be obtained by a method described in Persson H et al., 2011, Cancer Res., Vol. 71, pp. 78-86. Also, "hsa-mir-4658" (miRBase Accession No. MI0017286; SEQ ID NO: 423) having a hairpin-like structure is known as a precursor of "hsa-miR-4658."

The term "hsa-miR-450a-5p gene" or "hsa-miR-450a-5p" used herein includes the hsa-miR-450a-5p gene (miRBase Accession No. MIMAT0001545) shown in SEQ ID NO: 31, a homolog or an ortholog of a different organism species, and the like. The hsa-miR-450a-5p gene can be obtained by a method described in Xie X et al., 2005, Nature, Vol. 434, pp. 338-345. Also, "hsa-mir-450a-1 and hsa-mir-450a-2" (miRBase Accession Nos. MI0001652 and MI0003187; SEQ ID NOs: 424 and 425) each having a hairpin-like structure are known as precursors of "hsa-miR-450a-5p."

The term "hsa-miR-1260b gene" or "hsa-miR-1260b" used herein includes the hsa-miR-1260b gene (miRBase Accession No. MIMAT0015041) shown in SEQ ID NO: 32, a homolog or an ortholog of a different organism species, and the like. The hsa-miR-1260b gene can be obtained by a method described in Stark MS et al., 2010, PLoS One, Vol. 5, e9685. Also, "hsa-mir-1260b" (miRBase Accession No. MI0014197; SEQ ID NO: 426) having a hairpin-like structure is known as a precursor of "hsa-miR-1260b."

The term "hsa-miR-3677-5p gene" or "hsa-miR-3677-5p" used herein includes the hsa-miR-3677-5p gene (miRBase Accession No. MIMAT0019221) shown in SEQ ID NO: 33, a homolog or an ortholog of a different organism species, and the like. The hsa-miR-3677-5p gene can be obtained by a method described in Vaz C et al., 2010, BMC Genomics, Vol. 11, p. 288. Also, "hsa-mir-3677" (miRBase Accession No. MI0016078; SEQ ID NO: 427) having a hairpin-like structure is known as a precursor of "hsa-miR-3677-5p."

The term "hsa-miR-6777-3p gene" or "hsa-miR-6777-3p" used herein includes the hsa-miR-6777-3p gene (miRBase Accession No. MIMAT0027455) shown in SEQ ID NO: 34, a homolog or an ortholog of a different organism species, and the like. The hsa-miR-6777-3p gene can be obtained by a method described in Ladewig E et al., 2012, Genome Res., Vol. 22, pp. 1634-1645. Also, "hsa-mir-6777" (miRBase Accession No. MI0022622; SEQ ID NO: 428) having a hairpin-like structure is known as a precursor of "hsa-miR-6777-3p."

The term "hsa-miR-6826-3p gene" or "hsa-miR-6826-3p" used herein includes the hsa-miR-6826-3p gene (miRBase Accession No. MIMAT0027553) shown in SEQ ID NO: 35, a homolog or an ortholog of a different organism species, and the like. The hsa-miR-6826-3p gene can be obtained by a method described in Ladewig E et al., 2012, Genome Res., Vol. 22, pp. 1634-1645. Also, "hsa-mir-6826" (miRBase Accession No. MI0022671; SEQ ID NO: 429) having a hairpin-like structure is known as a precursor of "hsa-miR-6826-3p."

The term "hsa-miR-6832-3p gene" or "hsa-miR-6832-3p" used herein includes the hsa-miR-6832-3p gene (miRBase Accession No. MIMAT0027565) shown in SEQ ID NO: 36, a homolog or an ortholog of a different organism species, and the like. The hsa-miR-6832-3p gene can be obtained by a method described in Ladewig E et al., 2012, Genome Res., Vol. 22, pp. 1634-1645. Also, "hsa-mir-6832" (miRBase Accession No. MI0022677; SEQ ID NO: 430) having a hairpin-like structure is known as a precursor of "hsa-miR-6832-3p."

The term "hsa-miR-4725-3p gene" or "hsa-miR-4725-3p" used herein includes the hsa-miR-4725-3p gene (miRBase Accession No. MIMAT0019844) shown in SEQ ID NO: 37, a homolog or an ortholog of a different organism species, and the like. The hsa-miR-4725-3p gene can be obtained by a method described in Persson H et al., 2011, Cancer Res., Vol. 71, pp. 78-86. Also, "hsa-mir-4725" (miRBase Accession No. MI0017362; SEQ ID NO: 431) having a hairpin-like structure is known as a precursor of "hsa-miR-4725-3p."

The term "hsa-miR-7161-3p gene" or "hsa-miR-7161-3p" used herein includes the hsa-miR-7161-3p gene (miRBase Accession No. MIMAT0028233) shown in SEQ ID NO: 38, a homolog or an ortholog of a different organism species, and the like. The hsa-miR-7161-3p gene can be obtained by a method described in Meunier J et al., 2013, Genome Res., Vol. 23, pp. 34-45. Also, "hsa-mir-7161" (miRBase Accession No. MI0023619; SEQ ID NO: 432) having a hairpin-like structure is known as a precursor of "hsa-miR-7161-3p."

The term "hsa-miR-2277-5p gene" or "hsa-miR-2277-5p" used herein includes the hsa-miR-2277-5p gene (miRBase Accession No. MIMAT0017352) shown in SEQ ID NO: 39, a homolog or an ortholog of a different organism species, and the like. The hsa-miR-2277-5p gene can be obtained by a method described in Nygaard S et al., 2009, BMC Med. Genomics, Vol. 2, p. 35. Also, "hsa-mir-2277" (miRBase Accession No. MI0011284; SEQ ID NO: 433) having a hairpin-like structure is known as a precursor of "hsa-miR-2277-5p."

The term "hsa-miR-7110-3p gene" or "hsa-miR-7110-3p" used herein includes the hsa-miR-7110-3p gene (miRBase Accession No. MIMAT0028118) shown in SEQ ID NO: 40, a homolog or an ortholog of a different organism species, and the like. The hsa-miR-7110-3p gene can be obtained by a method described in Ladewig E et al., 2012, Genome Res., Vol. 22, pp. 1634-1645. Also, "hsa-mir-7110" (miRBase Accession No. MI0022961; SEQ ID NO: 434) having a hairpin-like structure is known as a precursor of "hsa-miR-7110-3p."

The term "hsa-miR-4312 gene" or "hsa-miR-4312" used herein includes the hsa-miR-4312 gene (miRBase Accession No. MIMAT0016864) shown in SEQ ID NO: 41, a homolog or an ortholog of a different organism species, and the like. The hsa-miR-4312 gene can be obtained by a method described in Goff LA et al., 2009, PLoS One, Vol. 4, e7192. Also, "hsa-mir-4312" (miRBase Accession No. MI0015842; SEQ ID NO: 435) having a hairpin-like structure is known as a precursor of "hsa-miR-4312."

The term "hsa-miR-4461 gene" or "hsa-miR-4461" used herein includes the hsa-miR-4461 gene (miRBase Accession No. MIMAT0018983) shown in SEQ ID NO: 42, a homolog or an ortholog of a different organism species, and the like. The hsa-miR-4461 gene can be obtained by a method described in Jima DD et al., 2010, Blood, Vol. 116, e118-e127. Also, "hsa-mir-4461" (miRBase Accession No. MI0016807; SEQ ID NO: 436) having a hairpin-like structure is known as a precursor of "hsa-miR-4461."

The term "hsa-miR-6766-5p gene" or "hsa-miR-6766-5p" used herein includes the hsa-miR-6766-5p gene (miRBase Accession No. MIMAT0027432) shown in SEQ ID NO: 43, a homolog or an ortholog of a different organism species, and the like. The hsa-miR-6766-5p gene can be obtained by a method described in Ladewig E et al., 2012, Genome Res., Vol. 22, pp. 1634-1645. Also, "hsa-mir-6766" (miRBase Accession No. MI10022611; SEQ ID NO: 437) having a hairpin-like structure is known as a precursor of "hsa-miR-6766-5p."

The term "hsa-miR-1266-3p gene" or "hsa-miR-1266-3p" used herein includes the hsa-miR-1266-3p gene (miRBase Accession No. MIMAT0026742) shown in SEQ ID NO: 44, a homolog or an ortholog of a different organism species, and the like. The hsa-miR-1266-3p gene can be obtained by a method described in Morin RD et al., 2008, Genome Res., Vol. 18, pp. 610-621. Also, "hsa-mir-1266" (miRBase Accession No. MI0006403; SEQ ID NO: 438) having a hairpin-like structure is known as a precursor of "hsa-miR-1266-3p."

The term "hsa-miR-6729-5p gene" or "hsa-miR-6729-5p" used herein includes the hsa-miR-6729-5p gene (miRBase Accession No. MIMAT0027359) shown in SEQ ID NO: 45, a homolog or an ortholog of a different organism species, and the like. The hsa-miR-6729-5p gene can be obtained by a method described in Ladewig E et al., 2012, Genome Res., Vol. 22, pp. 1634-1645. Also, "hsa-mir-6729" (miRBase Accession No. MI0022574; SEQ ID NO: 402) having a hairpin-like structure is known as a precursor of "hsa-miR-6729-5p."

The term "hsa-miR-526b-3p gene" or "hsa-miR-526b-3p" used herein includes the hsa-miR-526b-3p gene (miRBase Accession No. MIMAT0002836) shown in SEQ ID NO: 46, a homolog or an ortholog of a different organism species, and the like. The hsa-miR-526b-3p gene can be obtained by a method described in Bentwich I et al., 2005, Nat. Genet., Vol. 37, pp. 766-770. Also, "hsa-mir-526b" (miRBase Accession No. MI0003150; SEQ ID NO: 439) having a hairpin-like structure is known as a precursor of "hsa-miR-526b-3p."

The term "hsa-miR-519e-5p gene" or "hsa-miR-519e-5p" used herein includes the hsa-miR-519e-5p gene (miRBase Accession No. MIMAT0002828) shown in SEQ ID NO: 47, a homolog or an ortholog of a different organism species, and the like. The hsa-miR-519e-5p gene can be obtained by a method described in Bentwich I et al., 2005, Nat. Genet., Vol. 37, pp. 766-770. Also, "hsa-mir-519e" (miRBase Accession No. MI0003145; SEQ ID NO: 440) having a hairpin-like structure is known as a precursor of "hsa-miR-519e-5p."

The term "hsa-miR-512-5p gene" or "hsa-miR-512-5p" used herein includes the hsa-miR-512-5p gene (miRBase Accession No. MIMAT0002822) shown in SEQ ID NO: 48, a homolog or an ortholog of a different organism species, and the like. The hsa-miR-512-5p gene can be obtained by a method described in Bentwich I et al., 2005, Nat. Genet., Vol. 37, pp. 766-770. Also, "hsa-mir-512-1 and hsa-mir-512-2" (miRBase Accession Nos. MI0003140 and MI0003141; SEQ ID NOs: 441 and 442) each having a hairpin-like structure are known as precursors of "hsa-miR-512-5p."

The term "hsa-miR-5088-5p gene" or "hsa-miR-5088-5p" used herein includes the hsa-miR-5088-5p gene (miRBase Accession No. MIMAT0021080) shown in SEQ ID NO: 49, a homolog or an ortholog of a different organism species, and the like. The hsa-miR-5088-5p gene can be obtained by a method described in Ding N et al., 2011, J. Radiat. Res., Vol. 52, pp. 425-432. Also, "hsa-mir-5088" (miRBase Accession No. MI0017977; SEQ ID NO: 443) having a hairpin-like structure is known as a precursor of "hsa-miR-5088-5p."

The term "hsa-miR-1909-3p gene" or "hsa-miR-1909-3p" used herein includes the hsa-miR-1909-3p gene (miRBase Accession No. MIMAT0007883) shown in SEQ ID NO: 50, a homolog or an ortholog of a different organism species, and the like. The hsa-miR-1909-3p gene can be obtained by a method described in Bar M et al., 2008, Stem Cells, Vol. 26, pp. 2496-2505. Also, "hsa-mir-1909" (miRBase Accession No. MI0008330; SEQ ID NO: 444) having a hairpin-like structure is known as a precursor of "hsa-miR-1909-3p."

The term "hsa-miR-6511a-5p gene" or "hsa-miR-6511a-5p" used herein includes the hsa-miR-6511a-5p gene (miRBase Accession No. MIMAT0025478) shown in SEQ ID NO: 51, a homolog or an ortholog of a different organism species, and the like. The hsa-miR-6511a-5p gene can be obtained by a method described in Joyce CE et al., 2011, Hum. Mol. Genet., Vol. 20, pp. 4025-4040. Also, "hsa-mir-6511a-1, hsa-mir-6511a-2, hsa-mir-6511a-3, and hsa-mir-6511a-4" (miRBase Accession Nos. MI0022223, MI0023564, MI0023565, and MI0023566; SEQ ID NOs: 445, 446, 447, and 448) each having a hairpin-like structure are known as precursors of "hsa-miR-651 la-5p."

The term "hsa-miR-4734 gene" or "hsa-miR-4734" used herein includes the hsa-miR-4734 gene (miRBase Accession No. MIMAT0019859) shown in SEQ ID NO: 52, a homolog or an ortholog of a different organism species, and the like. The hsa-miR-4734 gene can be obtained by a method described in Persson H et al., 2011, Cancer Res., Vol. 71, pp. 78-86. Also, "hsa-mir-4734" (miRBase Accession No. MI0017371; SEQ ID NO: 449) having a hairpin-like structure is known as a precursor of "hsa-miR-4734."

The term "hsa-miR-936 gene" or "hsa-miR-936" used herein includes the hsa-miR-936 gene (miRBase Accession No. MIMAT0004979) shown in SEQ ID NO: 53, a homolog or an ortholog of a different organism species, and the like. The hsa-miR-936 gene can be obtained by a method described in Lui WO et al., 2007, Cancer Res., Vol. 67, pp. 6031-6043. Also, "hsa-mir-936" (miRBase Accession No. MI0005758; SEQ ID NO: 450) having a hairpin-like structure is known as a precursor of "hsa-miR-936."

The term "hsa-miR-1249-3p gene" or "hsa-miR-1249-3p" used herein includes the hsa-miR-1249-3p gene (miRBase Accession No. MIMAT0005901) shown in SEQ ID NO: 54, a homolog or an ortholog of a different organism species, and the like. The hsa-miR-1249-3p gene can be obtained by a method described in Morin RD et al., 2008, Genome Res., Vol. 18, pp. 610-621. Also, "hsa-mir-1249" (miRBase Accession No. MI0006384; SEQ ID NO: 451) having a hairpin-like structure is known as a precursor of "hsa-miR-1249-3p."

The term "hsa-miR-6777-5p gene" or "hsa-miR-6777-5p" used herein includes the hsa-miR-6777-5p gene (miRBase Accession No. MIMAT0027454) shown in SEQ ID NO: 55, a homolog or an ortholog of a different organism species, and the like. The hsa-miR-6777-5p gene can be obtained by a method described in Ladewig E et al., 2012, Genome Res., Vol. 22, pp. 1634-1645. Also, "hsa-mir-6777" (miRBase Accession No. MI0022622; SEQ ID NO: 428) having a hairpin-like structure is known as a precursor of "hsa-miR-6777-5p."

The term "hsa-miR-4487 gene" or "hsa-miR-4487" used herein includes the hsa-miR-4487 gene (miRBase Accession No. MIMAT0019021) shown in SEQ ID NO: 56, a homolog or an ortholog of a different organism species, and the like. The hsa-miR-4487 gene can be obtained by a method described in Jima DD et al., 2010, Blood, Vol. 116, e118-e127. Also, "hsa-mir-4487" (miRBase Accession No. MI0016848; SEQ ID NO: 452) having a hairpin-like structure is known as a precursor of "hsa-miR-4487."

The term "hsa-miR-3155a gene" or "hsa-miR-3155a" used herein includes the hsa-miR-3155a gene (miRBase Accession No. MIMAT0015029) shown in SEQ ID NO: 57, a homolog or an ortholog of a different organism species, and the like. The hsa-miR-3155a gene can be obtained by a method described in Stark MS et al., 2010, PLoS One, Vol. 5, e9685. Also, "hsa-mir-3155a" (miRBase Accession No. MI0014183; SEQ ID NO: 453) having a hairpin-like structure is known as a precursor of "hsa-miR-3155a."

The term "hsa-miR-563 gene" or "hsa-miR-563" used herein includes the hsa-miR-563 gene (miRBase Accession No. MIMAT0003227) shown in SEQ ID NO: 58, a homolog or an ortholog of a different organism species, and the like. The hsa-miR-563 gene can be obtained by a method described in Cummins JM et al., 2006, Proc. Natl. Acad. Sci. U.S.A., Vol. 103, pp. 3687-3692. Also, "hsa-mir-563" (miRBase Accession No. MI0003569; SEQ ID NO: 454) having a hairpin-like structure is known as a precursor of "hsa-miR-563."

The term "hsa-miR-4741 gene" or "hsa-miR-4741" used herein includes the hsa-miR-4741 gene (miRBase Accession No. MIMAT0019871) shown in SEQ ID NO: 59, a homolog or an ortholog of a different organism species, and the like. The hsa-miR-4741 gene can be obtained by a method described in Persson H et al., 2011, Cancer Res., Vol. 71, pp. 78-86. Also, "hsa-mir-4741" (miRBase Accession No. MI0017379; SEQ ID NO: 455) having a hairpin-like structure is known as a precursor of "hsa-miR-4741."

The term "hsa-miR-6788-5p gene" or "hsa-miR-6788-5p" used herein includes the hsa-miR-6788-5p gene (miRBase Accession No. MIMAT0027476) shown in SEQ ID NO: 60, a homolog or an ortholog of a different organism species, and the like. The hsa-miR-6788-5p gene can be obtained by a method described in Ladewig E et al., 2012, Genome Res., Vol. 22, pp. 1634-1645. Also, "hsa-mir-6788" (miRBase Accession No. MI0022633; SEQ ID NO: 456) having a hairpin-like structure is known as a precursor of "hsa-miR-6788-5p."

The term "hsa-miR-4433b-5p gene" or "hsa-miR-4433b-5p" used herein includes the hsa-miR-4433b-5p gene (miRBase Accession No. MIMAT0030413) shown in SEQ ID NO: 61, a homolog or an ortholog of a different organism species, and the like. The hsa-miR-4433b-5p gene can be obtained by a method described in Ple H et al., 2012, PLoS One, Vol. 7, e50746. Also, "hsa-mir-4433b" (miRBase Accession No. MI0025511; SEQ ID NO: 457) having a hairpin-like structure is known as a precursor of "hsa-miR-4433b-5p."

The term "hsa-miR-323a-5p gene" or "hsa-miR-323a-5p" used herein includes the hsa-miR-323a-5p gene (miRBase Accession No. MIMAT0004696) shown in SEQ ID NO: 62, a homolog or an ortholog of a different organism species, and the like. The hsa-miR-323a-5p gene can be obtained by a method described in Kim J et al., 2004, Proc. Natl. Acad. Sci. U.S.A., Vol. 101, pp. 360-365. Also, "hsa-mir-323a" (miRBase Accession No. MI0000807; SEQ ID NO: 458) having a hairpin-like structure is known as a precursor of "hsa-miR-323a-5p."

The term "hsa-miR-6811-5p gene" or "hsa-miR-6811-5p" used herein includes the hsa-miR-6811-5p gene (miRBase Accession No. MIMAT0027522) shown in SEQ ID NO: 63, a homolog or an ortholog of a different organism species, and the like. The hsa-miR-6811-5p gene can be obtained by a method described in Ladewig E et al., 2012, Genome Res., Vol. 22, pp. 1634-1645. Also, "hsa-mir-6811" (miRBase Accession No. MI0022656; SEQ ID NO: 459) having a hairpin-like structure is known as a precursor of "hsa-miR-6811-5p."

The term "hsa-miR-6721-5p gene" or "hsa-miR-6721-5p" used herein includes the hsa-miR-6721-5p gene (miRBase Accession No. MIMAT0025852) shown in SEQ ID NO: 64, a homolog or an ortholog of a different organism species, and the like. The hsa-miR-6721-5p gene can be obtained by a method described in Li Y et al., 2012, Gene, Vol. 497, pp. 330-335. Also, "hsa-mir-6721" (miRBase Accession No. MI0022556; SEQ ID NO: 460) having a hairpin-like structure is known as a precursor of "hsa-miR-6721-5p."

The term "hsa-miR-5004-5p gene" or "hsa-miR-5004-5p" used herein includes the hsa-miR-5004-5p gene (miRBase Accession No. MIMAT0021027) shown in SEQ ID NO: 65, a homolog or an ortholog of a different organism species, and the like. The hsa-miR-5004-5p gene can be obtained by a method described in Hansen TB et al., 2011, RNA Biol., Vol. 8, pp. 378-383. Also, "hsa-mir-5004" (miRBase Accession No. MI0017870; SEQ ID NO: 461) having a hairpin-like structure is known as a precursor of "hsa-miR-5004-5p."

The term "hsa-miR-6509-3p gene" or "hsa-miR-6509-3p" used herein includes the hsa-miR-6509-3p gene (miRBase Accession No. MIMAT0025475) shown in SEQ ID NO: 66, a homolog or an ortholog of a different organism species, and the like. The hsa-miR-6509-3p gene can be obtained by a method described in Joyce CE et al., 2011, Hum. Mol. Genet., Vol. 20, pp. 4025-4040. Also, "hsa-mir-6509" (miRBase Accession No. MI0022221; SEQ ID NO: 462) having a hairpin-like structure is known as a precursor of "hsa-miR-6509-3p."

The term "hsa-miR-648 gene" or "hsa-miR-648" used herein includes the hsa-miR-648 gene (miRBase Accession No. MIMAT0003318) shown in SEQ ID NO: 67, a homolog or an ortholog of a different organism species, and the like. The hsa-miR-648 gene can be obtained by a method described in Cummins JM et al., 2006, Proc. Natl. Acad. Sci. U.S.A., Vol. 103, pp. 3687-3692. Also, "hsa-mir-648" (miRBase Accession No. MI0003663; SEQ ID NO: 463) having a hairpin-like structure is known as a precursor of "hsa-miR-648."

The term "hsa-miR-3917 gene" or "hsa-miR-3917" used herein includes the hsa-miR-3917 gene (miRBase Accession No. MIMAT0018191) shown in SEQ ID NO: 68, a homolog or an ortholog of a different organism species, and the like. The hsa-miR-3917 gene can be obtained by a method described in Creighton CJ et al., 2010, PLoS One, Vol. 5, e9637. Also, "hsa-mir-3917" (miRBase Accession No. MI0016423; SEQ ID NO: 464) having a hairpin-like structure is known as a precursor of "hsa-miR-3917."

The term "hsa-miR-6087 gene" or "hsa-miR-6087" used herein includes the hsa-miR-6087 gene (miRBase Accession No. MIMAT0023712) shown in SEQ ID NO: 69, a homolog or an ortholog of a different organism species, and the like. The hsa-miR-6087 gene can be obtained by a method described in Yoo JK et al., 2012, Stem Cells Dev., Vol. 21, pp. 2049-2057. Also, "hsa-mir-6087" (miRBase Accession No. MI0020364; SEQ ID NO: 465) having a hairpin-like structure is known as a precursor of "hsa-miR-6087."

The term "hsa-miR-1470 gene" or "hsa-miR-1470" used herein includes the hsa-miR-1470 gene (miRBase Accession No. MIMAT0007348) shown in SEQ ID NO: 70, a homolog or an ortholog of a different organism species, and the like. The hsa-miR-1470 gene can be obtained by a method described in Kawaji H et al., 2008, BMC Genomics, Vol. 9, p. 157. Also, "hsa-mir-1470" (miRBase Accession No. MI0007075; SEQ ID NO: 466) having a hairpin-like structure is known as a precursor of "hsa-miR-1470."

The term "hsa-miR-586 gene" or "hsa-miR-586" used herein includes the hsa-miR-586 gene (miRBase Accession No. MIMAT0003252) shown in SEQ ID NO: 71, a homolog or an ortholog of a different organism species, and the like. The hsa-miR-586 gene can be obtained by a method described in Cummins JM et al., 2006, Proc. Natl. Acad. Sci. U.S.A., Vol. 103, pp. 3687-3692. Also, "hsa-mir-586" (miRBase Accession No. MI0003594; SEQ ID NO: 467) having a hairpin-like structure is known as a precursor of "hsa-miR-586."

The term "hsa-miR-3150a-5p gene" or "hsa-miR-3150a-5p" used herein includes the hsa-miR-3150a-5p gene (miRBase Accession No. MIMAT0019206) shown in SEQ ID NO: 72, a homolog or an ortholog of a different organism species, and the like. The hsa-miR-3150a-5p gene can be obtained by a method described in Stark MS et al., 2010, PLoS One, Vol. 5, e9685. Also, "hsa-mir-3150a" (miRBase Accession No. MI0014177; SEQ ID NO: 468) having a hairpin-like structure is known as a precursor of "hsa-miR-3150a-5p."

The term "hsa-miR-105-3p gene" or "hsa-miR-105-3p" used herein includes the hsa-miR-105-3p gene (miRBase Accession No. MIMAT0004516) shown in SEQ ID NO: 73, a homolog or an ortholog of a different organism species, and the like. The hsa-miR-105-3p gene can be obtained by a method described in Mourelatos Z et al., 2002, Genes Dev., Vol. 16, pp. 720-728. Also, "hsa-mir-105-1 and hsa-mir-105-2" (miRBase Accession Nos. MI0000111 and MI0000112; SEQ ID NOs: 469 and 470) each having a hairpin-like structure are known as precursors of "hsa-miR-105-3p."

The term "hsa-miR-7973 gene" or "hsa-miR-7973" used herein includes the hsa-miR-7973 gene (miRBase Accession No. MIMAT0031176) shown in SEQ ID NO: 74, a homolog or an ortholog of a different organism species, and the like. The hsa-miR-7973 gene can be obtained by a method described in Velthut-Meikas A et al., 2013, Mol. Endocrinol., Vol. 27, pp. 1128-1141. Also, "hsa-mir-7973-1 and hsa-mir-7973-2" (miRBase Accession Nos. MI0025748 and MI0025749; SEQ ID NOs: 471 and 472) each having a hairpin-like structure are known as precursors of "hsa-miR-7973."

The term "hsa-miR-1914-5p gene" or "hsa-miR-1914-5p" used herein includes the hsa-miR-1914-5p gene (miRBase Accession No. MIMAT0007889) shown in SEQ ID NO: 75, a homolog or an ortholog of a different organism species, and the like. The hsa-miR-1914-5p gene can be obtained by a method described in Bar M et al., 2008, Stem Cells, Vol. 26, pp. 2496-2505. Also, "hsa-mir-1914" (miRBase Accession No. MI0008335; SEQ ID NO: 473) having a hairpin-like structure is known as a precursor of "hsa-miR-1914-5p."

The term "hsa-miR-4749-3p gene" or "hsa-miR-4749-3p" used herein includes the hsa-miR-4749-3p gene (miRBase Accession No. MIMAT0019886) shown in SEQ ID NO: 76, a homolog or an ortholog of a different organism species, and the like. The hsa-miR-4749-3p gene can be obtained by a method described in Persson H et al., 2011, Cancer Res., Vol. 71, pp. 78-86. Also, "hsa-mir-4749" (miRBase Accession No. MI0017388; SEQ ID NO: 474) having a hairpin-like structure is known as a precursor of "hsa-miR-4749-3p."

The term "hsa-miR-15b-5p gene" or "hsa-miR-15b-5p" used herein includes the hsa-miR-15b-5p gene (miRBase Accession No. MIMAT0000417) shown in SEQ ID NO: 77, a homolog or an ortholog of a different organism species, and the like. The hsa-miR-15b-5p gene can be obtained by a method described in Lagos-Quintana M et al., 2002, Curr. Biol., Vol. 12, pp. 735-739. Also, "hsa-mir-15b" (miRBase Accession No. MI0000438; SEQ ID NO: 475) having a hairpin-like structure is known as a precursor of "hsa-miR-15b-5p."

The term "hsa-miR-1289 gene" or "hsa-miR-1289" used herein includes the hsa-miR-1289 gene (miRBase Accession No. MIMAT0005879) shown in SEQ ID NO: 78, a homolog or an ortholog of a different organism species, and the like. The hsa-miR-1289 gene can be obtained by a method described in Morin RD et al., 2008, Genome Res., Vol. 18, pp. 610-621. Also, "hsa-mir-1289-1 and hsa-mir-1289-2" (miRBase Accession Nos. MI0006350 and MI0006351; SEQ ID NOs: 476 and 477) each having a hairpin-like structure are known as precursors of "hsa-miR-1289."

The term "hsa-miR-4433a-5p gene" or "hsa-miR-4433a-5p" used herein includes the hsa-miR-4433a-5p gene (miRBase Accession No. MIMAT0020956) shown in SEQ ID NO: 79, a homolog or an ortholog of a different organism species, and the like. The hsa-miR-4433a-5p gene can be obtained by a method described in Jima DD et al., 2010, Blood, Vol. 116, e118-e127. Also, "hsa-mir-4433a" (miRBase Accession No. MI0016773; SEQ ID NO: 478) having a hairpin-like structure is known as a precursor of "hsa-miR-4433a-5p."

The term "hsa-miR-3666 gene" or "hsa-miR-3666" used herein includes the hsa-miR-3666 gene (miRBase Accession No. MIMAT0018088) shown in SEQ ID NO: 80, a homolog or an ortholog of a different organism species, and the like. The hsa-miR-3666 gene can be obtained by a method described in Xie X et al., 2005, Nature, Vol. 434, pp. 338-345. Also, "hsa-mir-3666" (miRBase Accession No. MI0016067; SEQ ID NO: 479) having a hairpin-like structure is known as a precursor of "hsa-miR-3666."

The term "hsa-miR-3186-3p gene" or "hsa-miR-3186-3p" used herein includes the hsa-miR-3186-3p gene (miRBase Accession No. MIMAT0015068) shown in SEQ ID NO: 81, a homolog or an ortholog of a different organism species, and the like. The hsa-miR-3186-3p gene can be obtained by a method described in Creighton CJ et al., 2010, PLoS One, Vol. 5, e9637. Also, "hsa-mir-3186" (miRBase Accession No. MI0014229; SEQ ID NO: 480) having a hairpin-like structure is known as a precursor of "hsa-miR-3186-3p."

The term "hsa-miR-4725-5p gene" or "hsa-miR-4725-5p" used herein includes the hsa-miR-4725-5p gene (miRBase Accession No. MIMAT0019843) shown in SEQ ID NO: 82, a homolog or an ortholog of a different organism species, and the like. The hsa-miR-4725-5p gene can be obtained by a method described in Persson H et al., 2011, Cancer Res., Vol. 71, pp. 78-86. Also, "hsa-mir-4725" (miRBase Accession No. MI0017362; SEQ ID NO: 431) having a hairpin-like structure is known as a precursor of "hsa-miR-4725-5p."

The term "hsa-miR-4488 gene" or "hsa-miR-4488" used herein includes the hsa-miR-4488 gene (miRBase Accession No. MIMAT0019022) shown in SEQ ID NO: 83, a homolog or an ortholog of a different organism species, and the like. The hsa-miR-4488 gene can be obtained by a method described in Jima DD et al., 2010, Blood, Vol. 116, e118-e127. Also, "hsa-mir-4488" (miRBase Accession No. MI0016849; SEQ ID NO: 481) having a hairpin-like structure is known as a precursor of "hsa-miR-4488."

The term "hsa-miR-4474-3p gene" or "hsa-miR-4474-3p" used herein includes the hsa-miR-4474-3p gene (miRBase Accession No. MIMAT0019001) shown in SEQ ID NO: 84, a homolog or an ortholog of a different organism species, and the like. The hsa-miR-4474-3p gene can be obtained by a method described in Jima DD et al., 2010, Blood, Vol. 116, e118-e127. Also, "hsa-mir-4474" (miRBase Accession No. MI0016826; SEQ ID NO: 482) having a hairpin-like structure is known as a precursor of "hsa-miR-4474-3p."

The term "hsa-miR-6731-3p gene" or "hsa-miR-6731-3p" used herein includes the hsa-miR-6731-3p gene (miRBase Accession No. MIMAT0027364) shown in SEQ ID NO: 85, a homolog or an ortholog of a different organism species, and the like. The hsa-miR-6731-3p gene can be obtained by a method described in Ladewig E et al., 2012, Genome Res., Vol. 22, pp. 1634-1645. Also, "hsa-mir-6731" (miRBase Accession No. MI0022576; SEQ ID NO: 483) having a hairpin-like structure is known as a precursor of "hsa-miR-6731-3p."

The term "hsa-miR-4640-3p gene" or "hsa-miR-4640-3p" used herein includes the hsa-miR-4640-3p gene (miRBase Accession No. MIMAT0019700) shown in SEQ ID NO: 86, a homolog or an ortholog of a different organism species, and the like. The hsa-miR-4640-3p gene can be obtained by a method described in Persson H et al., 2011, Cancer Res., Vol. 71, pp. 78-86. Also, "hsa-mir-4640" (miRBase Accession No. MI0017267; SEQ ID NO: 484) having a hairpin-like structure is known as a precursor of "hsa-miR-4640-3p."

The term "hsa-miR-202-5p gene" or "hsa-miR-202-5p" used herein includes the hsa-miR-202-5p gene (miRBase Accession No. MIMAT0002810) shown in SEQ ID NO: 87, a homolog or an ortholog of a different organism species, and the like. The hsa-miR-202-5p gene can be obtained by a method described in Bentwich I et al., 2005, Nat. Genet., Vol. 37, pp. 766-770. Also, "hsa-mir-202" (miRBase Accession No. MI0003130; SEQ ID NO: 485) having a hairpin-like structure is known as a precursor of "hsa-miR-202-5p."

The term "hsa-miR-6816-5p gene" or "hsa-miR-6816-5p" used herein includes the hsa-miR-6816-5p gene (miRBase Accession No. MIMAT0027532) shown in SEQ ID NO: 88, a homolog or an ortholog of a different organism species, and the like. The hsa-miR-6816-5p gene can be obtained by a method described in Ladewig E et al., 2012, Genome Res., Vol. 22, pp. 1634-1645. Also, "hsa-mir-6816" (miRBase Accession No. MI0022661; SEQ ID NO: 486) having a hairpin-like structure is known as a precursor of "hsa-miR-6816-5p."

The term "hsa-miR-638 gene" or "hsa-miR-638" used herein includes the hsa-miR-638 gene (miRBase Accession No. MIMAT0003308) shown in SEQ ID NO: 89, a homolog or an ortholog of a different organism species, and the like. The hsa-miR-638 gene can be obtained by a method described in Cummins JM et al., 2006, Proc. Natl. Acad. Sci. U.S.A., Vol. 103, pp. 3687-3692. Also, "hsa-mir-638" (miRBase Accession No. MI0003653; SEQ ID NO: 487) having a hairpin-like structure is known as a precursor of "hsa-miR-638."

The term "hsa-miR-6821-5p gene" or "hsa-miR-6821-5p" used herein includes the hsa-miR-6821-5p gene (miRBase Accession No. MIMAT0027542) shown in SEQ ID NO: 90, a homolog or an ortholog of a different organism species, and the like. The hsa-miR-6821-5p gene can be obtained by a method described in Ladewig E et al., 2012, Genome Res., Vol. 22, pp. 1634-1645. Also, "hsa-mir-6821" (miRBase Accession No. MI0022666; SEQ ID NO: 488) having a hairpin-like structure is known as a precursor of "hsa-miR-6821-5p."

The term "hsa-miR-1247-3p gene" or "hsa-miR-1247-3p" used herein includes the hsa-miR-1247-3p gene (miRBase Accession No. MIMAT0022721) shown in SEQ ID NO: 91, a homolog or an ortholog of a different organism species, and the like. The hsa-miR-1247-3p gene can be obtained by a method described in Morin RD et al., 2008, Genome Res., Vol. 18, pp. 610-621. Also, "hsa-mir-1247" (miRBase Accession No. MI0006382; SEQ ID NO: 489) having a hairpin-like structure is known as a precursor of "hsa-miR-1247-3p."

The term "hsa-miR-6765-5p gene" or "hsa-miR-6765-5p" used herein includes the hsa-miR-6765-5p gene (miRBase Accession No. MIMAT0027430) shown in SEQ ID NO: 92, a homolog or an ortholog of a different organism species, and the like. The hsa-miR-6765-5p gene can be obtained by a method described in Ladewig E et al., 2012, Genome Res., Vol. 22, pp. 1634-1645. Also, "hsa-mir-6765" (miRBase Accession No. MI0022610; SEQ ID NO: 490) having a hairpin-like structure is known as a precursor of "hsa-miR-6765-5p."

The term "hsa-miR-6800-5p gene" or "hsa-miR-6800-5p" used herein includes the hsa-miR-6800-5p gene (miRBase Accession No. MIMAT0027500) shown in SEQ ID NO: 93, a homolog or an ortholog of a different organism species, and the like. The hsa-miR-6800-5p gene can be obtained by a method described in Ladewig E et al., 2012, Genome Res., Vol. 22, pp. 1634-1645. Also, "hsa-mir-6800" (miRBase Accession No. MI0022645; SEQ ID NO: 491) having a hairpin-like structure is known as a precursor of "hsa-miR-6800-5p."

The term "hsa-miR-3928-3p gene" or "hsa-miR-3928-3p" used herein includes the hsa-miR-3928-3p gene (miRBase Accession No. MIMAT0018205) shown in SEQ ID NO: 94, a homolog or an ortholog of a different organism species, and the like. The hsa-miR-3928-3p gene can be obtained by a method described in Creighton CJ et al., 2010, PLoS One, Vol. 5, e9637. Also, "hsa-mir-3928" (miRBase Accession No. MI0016438; SEQ ID NO: 492) having a hairpin-like structure is known as a precursor of "hsa-miR-3928-3p."

The term "hsa-miR-3940-5p gene" or "hsa-miR-3940-5p" used herein includes the hsa-miR-3940-5p gene (miRBase Accession No. MIMAT0019229) shown in SEQ ID NO: 95, a homolog or an ortholog of a different organism species, and the like. The hsa-miR-3940-5p gene can be obtained by a method described in Liao JY et al., 2010, PLoS One, Vol. 5, e10563. Also, "hsa-mir-3940" (miRBase Accession No. MI0016597; SEQ ID NO: 493) having a hairpin-like structure is known as a precursor of "hsa-miR-3940-5p."

The term "hsa-miR-3960 gene" or "hsa-miR-3960" used herein includes the hsa-miR-3960 gene (miRBase Accession No. MIMAT0019337) shown in SEQ ID NO: 96, a homolog or an ortholog of a different organism species, and the like. The hsa-miR-3960 gene can be obtained by a method described in Hu R et al., 2011, J. Biol. Chem., Vol. 286, pp. 12328-12339. Also, "hsa-mir-3960" (miRBase Accession No. MI0016964; SEQ ID NO: 494) having a hairpin-like structure is known as a precursor of "hsa-miR-3960."

The term "hsa-miR-6775-5p gene" or "hsa-miR-6775-5p" used herein includes the hsa-miR-6775-5p gene (miRBase Accession No. MIMAT0027450) shown in SEQ ID NO: 97, a homolog or an ortholog of a different organism species, and the like. The hsa-miR-6775-5p gene can be obtained by a method described in Ladewig E et al., 2012, Genome Res., Vol. 22, pp. 1634-1645. Also, "hsa-mir-6775" (miRBase Accession No. MI0022620; SEQ ID NO: 495) having a hairpin-like structure is known as a precursor of "hsa-miR-6775-5p."

The term "hsa-miR-3178 gene" or "hsa-miR-3178" used herein includes the hsa-miR-3178 gene (miRBase Accession No. MIMAT0015055) shown in SEQ ID NO: 98, a homolog or an ortholog of a different organism species, and the like. The hsa-miR-3178 gene can be obtained by a method described in Stark MS et al., 2010, PLoS One, Vol. 5, e9685. Also, "hsa-mir-3178" (miRBase Accession No. MI0014212; SEQ ID NO: 496) having a hairpin-like structure is known as a precursor of "hsa-miR-3178."

The term "hsa-miR-1202 gene" or "hsa-miR-1202" used herein includes the hsa-miR-1202 gene (miRBase Accession No. MIMAT0005865) shown in SEQ ID NO: 99, a homolog or an ortholog of a different organism species, and the like. The hsa-miR-1202 gene can be obtained by a method described in Marton S et al., 2008, Leukemia, Vol. 22, pp. 330-338. Also, "hsa-mir-1202" (miRBase Accession No. MI0006334; SEQ ID NO: 497) having a hairpin-like structure is known as a precursor of "hsa-miR-1202."

The term "hsa-miR-6790-5p gene" or "hsa-miR-6790-5p" used herein includes the hsa-miR-6790-5p gene (miRBase Accession No. MIMAT0027480) shown in SEQ ID NO: 100, a homolog or an ortholog of a different organism species, and the like. The hsa-miR-6790-5p gene can be obtained by a method described in Ladewig E et al., 2012, Genome Res., Vol. 22, pp. 1634-1645. Also, "hsa-mir-6790" (miRBase Accession No. MI0022635; SEQ ID NO: 498) having a hairpin-like structure is known as a precursor of "hsa-miR-6790-5p."

The term "hsa-miR-4731-3p gene" or "hsa-miR-4731-3p" used herein includes the hsa-miR-4731-3p gene (miRBase Accession No. MIMAT0019854) shown in SEQ ID NO: 101, a homolog or an ortholog of a different organism species, and the like. The hsa-miR-4731-3p gene can be obtained by a method described in Persson H et al., 2011, Cancer Res., Vol. 71, pp. 78-86. Also, "hsa-mir-4731" (miRBase Accession No. MI0017368; SEQ ID NO: 499) having a hairpin-like structure is known as a precursor of "hsa-miR-4731-3p."

The term "hsa-miR-2681-3p gene" or "hsa-miR-2681-3p" used herein includes the hsa-miR-2681-3p gene (miRBase Accession No. MIMAT0013516) shown in SEQ ID NO: 102, a homolog or an ortholog of a different organism species, and the like. The hsa-miR-2681-3p gene can be obtained by a method described in Persson H et al., 2011, Cancer Res., Vol. 71, pp. 78-86. Also, "hsa-mir-2681" (miRBase Accession No. MI0012062; SEQ ID NO: 500) having a hairpin-like structure is known as a precursor of "hsa-miR-2681-3p."

The term "hsa-miR-6758-5p gene" or "hsa-miR-6758-5p" used herein includes the hsa-miR-6758-5p gene (miRBase Accession No. MIMAT0027416) shown in SEQ ID NO: 103, a homolog or an ortholog of a different organism species, and the like. The hsa-miR-6758-5p gene can be obtained by a method described in Ladewig E et al., 2012, Genome Res., Vol. 22, pp. 1634-1645. Also, "hsa-mir-6758" (miRBase Accession No. MI0022603; SEQ ID NO: 501) having a hairpin-like structure is known as a precursor of "hsa-miR-6758-5p."

The term "hsa-miR-8072 gene" or "hsa-miR-8072" used herein includes the hsa-miR-8072 gene (miRBase Accession No. MIMAT0030999) shown in SEQ ID NO: 104, a homolog or an ortholog of a different organism species, and the like. The hsa-miR-8072 gene can be obtained by a method described in Wang HJ et al., 2013, Shock, Vol. 39, pp. 480-487. Also, "hsa-mir-8072" (miRBase Accession No. MI0025908; SEQ ID NO: 502) having a hairpin-like structure is known as a precursor of "hsa-miR-8072."

The term "hsa-miR-518d-3p gene" or "hsa-miR-518d-3p" used herein includes the hsa-miR-518d-3p gene (miRBase Accession No. MIMAT0002864) shown in SEQ ID NO: 105, a homolog or an ortholog of a different organism species, and the like. The hsa-miR-518d-3p gene can be obtained by a method described in Bentwich I et al., 2005, Nat. Genet., Vol. 37, pp. 766-770. Also, "hsa-mir-518d" (miRBase Accession No. MI0003171; SEQ ID NO: 503) having a hairpin-like structure is known as a precursor of "hsa-miR-518d-3p."

The term "hsa-miR-3606-3p gene" or "hsa-miR-3606-3p" used herein includes the hsa-miR-3606-3p gene (miRBase Accession No. MIMAT0022965) shown in SEQ ID NO: 106, a homolog or an ortholog of a different organism species, and the like. The hsa-miR-3606-3p gene can be obtained by a method described in Witten D et al., 2010, BMC Biol., Vol. 8, p. 58. Also, "hsa-mir-3606" (miRBase Accession No. MI0015996; SEQ ID NO: 504) having a hairpin-like structure is known as a precursor of "hsa-miR-3606-3p."

The term "hsa-miR-4800-5p gene" or "hsa-miR-4800-5p" used herein includes the hsa-miR-4800-5p gene (miRBase Accession No. MIMAT0019978) shown in SEQ ID NO: 107, a homolog or an ortholog of a different organism species, and the like. The hsa-miR-4800-5p gene can be obtained by a method described in Persson H et al., 2011, Cancer Res., Vol. 71, pp. 78-86. Also, "hsa-mir-4800" (miRBase Accession No. MI0017448; SEQ ID NO: 505) having a hairpin-like structure is known as a precursor of "hsa-miR-4800-5p."

The term "hsa-miR-1292-3p gene" or "hsa-miR-1292-3p" used herein includes the hsa-miR-1292-3p gene (miRBase Accession No. MIMAT0022948) shown in SEQ ID NO: 108, a homolog or an ortholog of a different organism species, and the like. The hsa-miR-1292-3p gene can be obtained by a method described in Morin RD et al., 2008, Genome Res., Vol. 18, pp. 610-621. Also, "hsa-mir-1292" (miRBase Accession No. MI0006433; SEQ ID NO: 506) having a hairpin-like structure is known as a precursor of "hsa-miR-1292-3p."

The term "hsa-miR-6784-3p gene" or "hsa-miR-6784-3p" used herein includes the hsa-miR-6784-3p gene (miRBase Accession No. MIMAT0027469) shown in SEQ ID NO: 109, a homolog or an ortholog of a different organism species, and the like. The hsa-miR-6784-3p gene can be obtained by a method described in Ladewig E et al., 2012, Genome Res., Vol. 22, pp. 1634-1645. Also, "hsa-mir-6784" (miRBase Accession No. MI0022629; SEQ ID NO: 507) having a hairpin-like structure is known as a precursor of "hsa-miR-6784-3p."

The term "hsa-miR-4450 gene" or "hsa-miR-4450" used herein includes the hsa-miR-4450 gene (miRBase Accession No. MIMAT0018971) shown in SEQ ID NO: 110, a homolog or an ortholog of a different organism species, and the like. The hsa-miR-4450 gene can be obtained by a method described in Jima DD et al., 2010, Blood, Vol. 116, e118-e127. Also, "hsa-mir-4450" (miRBase Accession No. MI0016795; SEQ ID NO: 508) having a hairpin-like structure is known as a precursor of "hsa-miR-4450."

The term "hsa-miR-6132 gene" or "hsa-miR-6132" used herein includes the hsa-miR-6132 gene (miRBase Accession No. MIMAT0024616) shown in SEQ ID NO: 111, a homolog or an ortholog of a different organism species, and the like. The hsa-miR-6132 gene can be obtained by a method described in Dannemann M et al., 2012, Genome Biol. Evol., Vol. 4, pp. 552-564. Also, "hsa-mir-6132" (miRBase Accession No. MI0021277; SEQ ID NO: 509) having a hairpin-like structure is known as a precursor of "hsa-miR-6132."

The term "hsa-miR-4716-5p gene" or "hsa-miR-4716-5p" used herein includes the hsa-miR-4716-5p gene (miRBase Accession No. MIMAT0019826) shown in SEQ ID NO: 112, a homolog or an ortholog of a different organism species, and the like. The hsa-miR-4716-5p gene can be obtained by a method described in Persson H et al., 2011, Cancer Res., Vol. 71, pp. 78-86. Also, "hsa-mir-4716" (miRBase Accession No. MI0017350; SEQ ID NO: 510) having a hairpin-like structure is known as a precursor of "hsa-miR-4716-5p."

The term "hsa-miR-6860 gene" or "hsa-miR-6860" used herein includes the hsa-miR-6860 gene (miRBase Accession No. MIMAT0027622) shown in SEQ ID NO: 113, a homolog or an ortholog of a different organism species, and the like. The hsa-miR-6860 gene can be obtained by a method described in Ladewig E et al., 2012, Genome Res., Vol. 22, pp. 1634-1645. Also, "hsa-mir-6860" (miRBase Accession No. MI0022707; SEQ ID NO: 511) having a hairpin-like structure is known as a precursor of "hsa-miR-6860."

The term "hsa-miR-1268b gene" or "hsa-miR-1268b" used herein includes the hsa-miR-1268b gene (miRBase Accession No. MIMAT0018925) shown in SEQ ID NO: 114, a homolog or an ortholog of a different organism species, and the like. The hsa-miR-1268b gene can be obtained by a method described in Jima DD et al., 2010, Blood, Vol. 116, e118-e127. Also, "hsa-mir-1268b" (miRBase Accession No. MI0016748; SEQ ID NO: 512) having a hairpin-like structure is known as a precursor of "hsa-miR-1268b."

The term "hsa-miR-378d gene" or "hsa-miR-378d" used herein includes the hsa-miR-378d gene (miRBase Accession No. MIMAT0018926) shown in SEQ ID NO: 115, a homolog or an ortholog of a different organism species, and the like. The hsa-miR-378d gene can be obtained by a method described in Berezikov E et al., 2006, Genome Res., Vol. 16, pp. 1289-1298. Also, "hsa-mir-378d-1 and hsa-mir-378d-2" (miRBase Accession Nos. MI0016749 and MI0003840; SEQ ID NOs: 513 and 514) each having a hairpin-like structure are known as precursors of "hsa-miR-378d."

The term "hsa-miR-4701-5p gene" or "hsa-miR-4701-5p" used herein includes the hsa-miR-4701-5p gene (miRBase Accession No. MIMAT0019798) shown in SEQ ID NO: 116, a homolog or an ortholog of a different organism species, and the like. The hsa-miR-4701-5p gene can be obtained by a method described in Persson H et al., 2011, Cancer Res., Vol. 71, pp. 78-86. Also, "hsa-mir-4701" (miRBase Accession No. MI0017334; SEQ ID NO: 515) having a hairpin-like structure is known as a precursor of "hsa-miR-4701-5p."

The term "hsa-miR-4329 gene" or "hsa-miR-4329" used herein includes the hsa-miR-4329 gene (miRBase Accession No. MIMAT0016923) shown in SEQ ID NO: 117, a homolog or an ortholog of a different organism species, and the like. The hsa-miR-4329 gene can be obtained by a method described in Goff LA et al., 2009, PLoS One, Vol. 4, e7192. Also, "hsa-mir-4329" (miRBase Accession No. MI0015901; SEQ ID NO: 516) having a hairpin-like structure is known as a precursor of "hsa-miR-4329."

The term "hsa-miR-185-3p gene" or "hsa-miR-185-3p" used herein includes the hsa-miR-185-3p gene (miRBase Accession No. MIMAT0004611) shown in SEQ ID NO: 118, a homolog or an ortholog of a different organism species, and the like. The hsa-miR-185-3p gene can be obtained by a method described in Lagos-Quintana M et al., 2003, RNA, Vol. 9, pp. 175-179. Also, "hsa-mir-185" (miRBase Accession No. MI0000482; SEQ ID NO: 517) having a hairpin-like structure is known as a precursor of "hsa-miR-185-3p."

The term "hsa-miR-552-3p gene" or "hsa-miR-552-3p" used herein includes the hsa-miR-552-3p gene (miRBase Accession No. MIMAT0003215) shown in SEQ ID NO: 119, a homolog or an ortholog of a different organism species, and the like. The hsa-miR-552-3p gene can be obtained by a method described in Cummins JM et al., 2006, Proc. Natl. Acad. Sci. U.S.A., Vol. 103, pp. 3687-3692. Also, "hsa-mir-552" (miRBase Accession No. MI0003557; SEQ ID NO: 518) having a hairpin-like structure is known as a precursor of "hsa-miR-552-3p."

The term "hsa-miR-1273g-5p gene" or "hsa-miR-1273g-5p" used herein includes the hsa-miR-1273g-5p gene (miRBase Accession No. MIMAT0020602) shown in SEQ ID NO: 120, a homolog or an ortholog of a different organism species, and the like. The hsa-miR-1273g-5p gene can be obtained by a method described in Reshmi G et al., 2011, Genomics, Vol. 97, pp. 333-340. Also, "hsa-mir-1273g" (miRBase Accession No. MI0018003; SEQ ID NO: 519) having a hairpin-like structure is known as a precursor of "hsa-miR-1273g-5p."

The term "hsa-miR-6769b-3p gene" or "hsa-miR-6769b-3p" used herein includes the hsa-miR-6769b-3p gene (miRBase Accession No. MIMAT0027621) shown in SEQ ID NO: 121, a homolog or an ortholog of a different organism species, and the like. The hsa-miR-6769b-3p gene can be obtained by a method described in Ladewig E et al., 2012, Genome Res., Vol. 22, pp. 1634-1645. Also, "hsa-mir-6769b" (miRBase Accession No. MI0022706; SEQ ID NO: 520) having a hairpin-like structure is known as a precursor of "hsa-miR-6769b-3p."

The term "hsa-miR-520a-3p gene" or "hsa-miR-520a-3p" used herein includes the hsa-miR-520a-3p gene (miRBase Accession No. MIMAT0002834) shown in SEQ ID NO: 122, a homolog or an ortholog of a different organism species, and the like. The hsa-miR-520a-3p gene can be obtained by a method described in Bentwich I et al., 2005, Nat. Genet., Vol. 37, pp. 766-770. Also, "hsa-mir-520a" (miRBase Accession No. MI0003149; SEQ ID NO: 521) having a hairpin-like structure is known as a precursor of "hsa-miR-520a-3p."

The term "hsa-miR-4524b-5p gene" or "hsa-miR-4524b-5p" used herein includes the hsa-miR-4524b-5p gene (miRBase Accession No. MIMAT0022255) shown in SEQ ID NO: 123, a homolog or an ortholog of a different organism species, and the like. The hsa-miR-4524b-5p gene can be obtained by a method described in Tandon M et al., 2012, Oral Dis., Vol. 18, pp. 127-131. Also, "hsa-mir-4524b" (miRBase Accession No. MI0019114; SEQ ID NO: 522) having a hairpin-like structure is known as a precursor of "hsa-miR-4524b-5p."

The term "hsa-miR-4291 gene" or "hsa-miR-4291" used herein includes the hsa-miR-4291 gene (miRBase Accession No. MIMAT0016922) shown in SEQ ID NO: 124, a homolog or an ortholog of a different organism species, and the like. The hsa-miR-4291 gene can be obtained by a method described in Goff LA et al., 2009, PLoS One, Vol. 4, e7192. Also, "hsa-mir-4291" (miRBase Accession No. MI0015900; SEQ ID NO: 523) having a hairpin-like structure is known as a precursor of "hsa-miR-4291."

The term "hsa-miR-6734-3p gene" or "hsa-miR-6734-3p" used herein includes the hsa-miR-6734-3p gene (miRBase Accession No. MIMAT0027370) shown in SEQ ID NO: 125, a homolog or an ortholog of a different organism species, and the like. The hsa-miR-6734-3p gene can be obtained by a method described in Ladewig E et al., 2012, Genome Res., Vol. 22, pp. 1634-1645. Also, "hsa-mir-6734" (miRBase Accession No. MI0022579; SEQ ID NO: 524) having a hairpin-like structure is known as a precursor of "hsa-miR-6734-3p."

The term "hsa-miR-143-5p gene" or "hsa-miR-143-5p" used herein includes the hsa-miR-143-5p gene (miRBase Accession No. MIMAT0004599) shown in SEQ ID NO: 126, a homolog or an ortholog of a different organism species, and the like. The hsa-miR-143-5p gene can be obtained by a method described in Lagos-Quintana M et al., 2002, Curr. Biol., Vol. 12, pp. 735-739. Also, "hsa-mir-143" (miRBase Accession No. MI0000459; SEQ ID NO: 525) having a hairpin-like structure is known as a precursor of "hsa-miR-143-5p."

The term "hsa-miR-939-3p gene" or "hsa-miR-939-3p" used herein includes the hsa-miR-939-3p gene (miRBase Accession No. MIMAT0022939) shown in SEQ ID NO: 127, a homolog or an ortholog of a different organism species, and the like. The hsa-miR-939-3p gene can be obtained by a method described in Lui WO et al., 2007, Cancer Res., Vol. 67, pp. 6031-6043. Also, "hsa-mir-939" (miRBase Accession No. MI0005761; SEQ ID NO: 526) having a hairpin-like structure is known as a precursor of "hsa-miR-939-3p."

The term "hsa-miR-6889-3p gene" or "hsa-miR-6889-3p" used herein includes the hsa-miR-6889-3p gene (miRBase Accession No. MIMAT0027679) shown in SEQ ID NO: 128, a homolog or an ortholog of a different organism species, and the like. The hsa-miR-6889-3p gene can be obtained by a method described in Ladewig E et al., 2012, Genome Res., Vol. 22, pp. 1634-1645. Also, "hsa-mir-6889" (miRBase Accession No. MI0022736; SEQ ID NO: 527) having a hairpin-like structure is known as a precursor of "hsa-miR-6889-3p."

The term "hsa-miR-6842-3p gene" or "hsa-miR-6842-3p" used herein includes the hsa-miR-6842-3p gene (miRBase Accession No. MIMAT0027587) shown in SEQ ID NO: 129, a homolog or an ortholog of a different organism species, and the like. The hsa-miR-6842-3p gene can be obtained by a method described in Ladewig E et al., 2012, Genome Res., Vol. 22, pp. 1634-1645. Also, "hsa-mir-6842" (miRBase Accession No. MI0022688; SEQ ID NO: 528) having a hairpin-like structure is known as a precursor of "hsa-miR-6842-3p."

The term "hsa-miR-4511 gene" or "hsa-miR-4511" used herein includes the hsa-miR-4511 gene (miRBase Accession No. MIMAT0019048) shown in SEQ ID NO: 130, a homolog or an ortholog of a different organism species, and the like. The hsa-miR-4511 gene can be obtained by a method described in Jima DD et al., 2010, Blood, Vol. 116, e118-e127. Also, "hsa-mir-4511" (miRBase Accession No. MI0016877; SEQ ID NO: 529) having a hairpin-like structure is known as a precursor of "hsa-miR-4511."

The term "hsa-miR-4318 gene" or "hsa-miR-4318" used herein includes the hsa-miR-4318 gene (miRBase Accession No. MIMAT0016869) shown in SEQ ID NO: 131, a homolog or an ortholog of a different organism species, and the like. The hsa-miR-4318 gene can be obtained by a method described in Goff LA et al., 2009, PLoS One, Vol. 4, e7192. Also, "hsa-mir-4318" (miRBase Accession No. MI0015847; SEQ ID NO: 530) having a hairpin-like structure is known as a precursor of "hsa-miR-4318."

The term "hsa-miR-4653-5p gene" or "hsa-miR-4653-5p" used herein includes the hsa-miR-4653-5p gene (miRBase Accession No. MIMAT0019718) shown in SEQ ID NO: 132, a homolog or an ortholog of a different organism species, and the like. The hsa-miR-4653-5p gene can be obtained by a method described in Persson H et al., 2011, Cancer Res., Vol. 71, pp. 78-86. Also, "hsa-mir-4653" (miRBase Accession No. MI0017281; SEQ ID NO: 531) having a hairpin-like structure is known as a precursor of "hsa-miR-4653-5p."

The term "hsa-miR-6867-3p gene" or "hsa-miR-6867-3p" used herein includes the hsa-miR-6867-3p gene (miRBase Accession No. MIMAT0027635) shown in SEQ ID NO: 133, a homolog or an ortholog of a different organism species, and the like. The hsa-miR-6867-3p gene can be obtained by a method described in Ladewig E et al., 2012, Genome Res., Vol. 22, pp. 1634-1645. Also, "hsa-mir-6867" (miRBase Accession No. MI0022714; SEQ ID NO: 532) having a hairpin-like structure is known as a precursor of "hsa-miR-6867-3p."

The term "hsa-miR-133b gene" or "hsa-miR-133b" used herein includes the hsa-miR-133b gene (miRBase Accession No. MIMAT0000770) shown in SEQ ID NO: 134, a homolog or an ortholog of a different organism species, and the like. The hsa-miR-133b gene can be obtained by a method described in Lim LP et al., 2003, Science, Vol. 299, p. 1540. Also, "hsa-mir-133b" (miRBase Accession No. MI0000822; SEQ ID NO: 533) having a hairpin-like structure is known as a precursor of "hsa-miR-133b."

The term "hsa-miR-3196 gene" or "hsa-miR-3196" used herein includes the hsa-miR-3196 gene (miRBase Accession No. MIMAT0015080) shown in SEQ ID NO: 135, a homolog or an ortholog of a different organism species, and the like. The hsa-miR-3196 gene can be obtained by a method described in Stark MS et al., 2010, PLoS One, Vol. 5, e9685. Also, "hsa-mir-3196" (miRBase Accession No. MI0014241; SEQ ID NO: 534) having a hairpin-like structure is known as a precursor of "hsa-miR-3196."

The term "hsa-miR-193b-3p gene" or "hsa-miR-193b-3p" used herein includes the hsa-miR-193b-3p gene (miRBase Accession No. MIMAT0002819) shown in SEQ ID NO: 136, a homolog or an ortholog of a different organism species, and the like. The hsa-miR-193b-3p gene can be obtained by a method described in Bentwich I et al., 2005, Nat. Genet., Vol. 37, pp. 766-770. Also, "hsa-mir-193b" (miRBase Accession No. MI0003137; SEQ ID NO: 535) having a hairpin-like structure is known as a precursor of "hsa-miR-193b-3p."

The term "hsa-miR-3162-3p gene" or "hsa-miR-3162-3p" used herein includes the hsa-miR-3162-3p gene (miRBase Accession No. MIMAT0019213) shown in SEQ ID NO: 137, a homolog or an ortholog of a different organism species, and the like. The hsa-miR-3162-3p gene can be obtained by a method described in Stark MS et al., 2010, PLoS One, Vol. 5, e9685. Also, "hsa-mir-3162" (miRBase Accession No. MI0014192; SEQ ID NO: 536) having a hairpin-like structure is known as a precursor of "hsa-miR-3162-3p."

The term "hsa-miR-6819-3p gene" or "hsa-miR-6819-3p" used herein includes the hsa-miR-6819-3p gene (miRBase Accession No. MIMAT0027539) shown in SEQ ID NO: 138, a homolog or an ortholog of a different organism species, and the like. The hsa-miR-6819-3p gene can be obtained by a method described in Ladewig E et al., 2012, Genome Res., Vol. 22, pp. 1634-1645. Also, "hsa-mir-6819" (miRBase Accession No. MI0022664; SEQ ID NO: 537) having a hairpin-like structure is known as a precursor of "hsa-miR-6819-3p."

The term "hsa-miR-1908-3p gene" or "hsa-miR-1908-3p" used herein includes the hsa-miR-1908-3p gene (miRBase Accession No. MIMAT0026916) shown in SEQ ID NO: 139, a homolog or an ortholog of a different organism species, and the like. The hsa-miR-1908-3p gene can be obtained by a method described in Bar M et al., 2008, Stem Cells, Vol. 26, pp. 2496-2505. Also, "hsa-mir-1908" (miRBase Accession No. MI0008329; SEQ ID NO: 538) having a hairpin-like structure is known as a precursor of "hsa-miR-1908-3p."

The term "hsa-miR-6786-5p gene" or "hsa-miR-6786-5p" used herein includes the hsa-miR-6786-5p gene (miRBase Accession No. MIMAT0027472) shown in SEQ ID NO: 140, a homolog or an ortholog of a different organism species, and the like. The hsa-miR-6786-5p gene can be obtained by a method described in Ladewig E et al., 2012, Genome Res., Vol. 22, pp. 1634-1645. Also, "hsa-mir-6786" (miRBase Accession No. MI0022631; SEQ ID NO: 539) having a hairpin-like structure is known as a precursor of "hsa-miR-6786-5p."

The term "hsa-miR-3648 gene" or "hsa-miR-3648" used herein includes the hsa-miR-3648 gene (miRBase Accession No. MIMAT0018068) shown in SEQ ID NO: 141, a homolog or an ortholog of a different organism species, and the like. The hsa-miR-3648 gene can be obtained by a method described in Meiri E et al., 2010, Nucleic Acids Res., Vol. 38, p. 6234-6246. Also, "hsa-mir-3648-1 and hsa-mir-3648-2" (miRBase Accession Nos. MI0016048 and MI0031512; SEQ ID NOs: 540 and 541) each having a hairpin-like structure are known as precursors of "hsa-miR-3648."

The term "hsa-miR-4513 gene" or "hsa-miR-4513" used herein includes the hsa-miR-4513 gene (miRBase Accession No. MIMAT0019050) shown in SEQ ID NO: 142, a homolog or an ortholog of a different organism species, and the like. The hsa-miR-4513 gene can be obtained by a method described in Jima DD et al., 2010, Blood, Vol. 116, e118-e127. Also, "hsa-mir-4513" (miRBase Accession No. MI0016879; SEQ ID NO: 542) having a hairpin-like structure is known as a precursor of "hsa-miR-4513."

The term "hsa-miR-3652 gene" or "hsa-miR-3652" used herein includes the hsa-miR-3652 gene (miRBase Accession No. MIMAT0018072) shown in SEQ ID NO: 143, a homolog or an ortholog of a different organism species, and the like. The hsa-miR-3652 gene can be obtained by a method described in Meiri E et al., 2010, Nucleic Acids Res., Vol. 38, p. 6234-6246. Also, "hsa-mir-3652" (miRBase Accession No. MI0016052; SEQ ID NO: 543) having a hairpin-like structure is known as a precursor of "hsa-miR-3652."

The term "hsa-miR-4640-5p gene" or "hsa-miR-4640-5p" used herein includes the hsa-miR-4640-5p gene (miRBase Accession No. MIMAT0019699) shown in SEQ ID NO: 144, a homolog or an ortholog of a different organism species, and the like. The hsa-miR-4640-5p gene can be obtained by a method described in Persson H et al., 2011, Cancer Res., Vol. 71, pp. 78-86. Also, "hsa-mir-4640" (miRBase Accession No. MI0017267; SEQ ID NO: 484) having a hairpin-like structure is known as a precursor of "hsa-miR-4640-5p."

The term "hsa-miR-6871-5p gene" or "hsa-miR-6871-5p" used herein includes the hsa-miR-6871-5p gene (miRBase Accession No. MIMAT0027642) shown in SEQ ID NO: 145, a homolog or an ortholog of a different organism species, and the like. The hsa-miR-6871-5p gene can be obtained by a method described in Ladewig E et al., 2012, Genome Res., Vol. 22, pp. 1634-1645. Also, "hsa-mir-6871" (miRBase Accession No. MI0022718; SEQ ID NO: 544) having a hairpin-like structure is known as a precursor of "hsa-miR-6871-5p."

The term "hsa-miR-7845-5p gene" or "hsa-miR-7845-5p" used herein includes the hsa-miR-7845-5p gene (miRBase Accession No. MIMAT0030420) shown in SEQ ID NO: 146, a homolog or an ortholog of a different organism species, and the like. The hsa-miR-7845-5p gene can be obtained by a method described in Ple H et al., 2012, PLoS One, Vol. 7, e50746. Also, "hsa-mir-7845" (miRBase Accession No. MI0025515; SEQ ID NO: 545) having a hairpin-like structure is known as a precursor of "hsa-miR-7845-5p."

The term "hsa-miR-3138 gene" or "hsa-miR-3138" used herein includes the hsa-miR-3138 gene (miRBase Accession No. MIMAT0015006) shown in SEQ ID NO: 147, a homolog or an ortholog of a different organism species, and the like. The hsa-miR-3138 gene can be obtained by a method described in Creighton CJ et al., 2010, PLoS One, Vol. 5, e9637. Also, "hsa-mir-3138" (miRBase Accession No. MI0014161; SEQ ID NO: 546) having a hairpin-like structure is known as a precursor of "hsa-miR-3138."

The term "hsa-miR-6884-5p gene" or "hsa-miR-6884-5p" used herein includes the hsa-miR-6884-5p gene (miRBase Accession No. MIMAT0027668) shown in SEQ ID NO: 148, a homolog or an ortholog of a different organism species, and the like. The hsa-miR-6884-5p gene can be obtained by a method described in Ladewig E et al., 2012, Genome Res., Vol. 22, pp. 1634-1645. Also, "hsa-mir-6884" (miRBase Accession No. MI0022731; SEQ ID NO: 547) having a hairpin-like structure is known as a precursor of "hsa-miR-6884-5p."

The term "hsa-miR-4653-3p gene" or "hsa-miR-4653-3p" used herein includes the hsa-miR-4653-3p gene (miRBase Accession No. MIMAT0019719) shown in SEQ ID NO: 149, a homolog or an ortholog of a different organism species, and the like. The hsa-miR-4653-3p gene can be obtained by a method described in Persson H et al., 2011, Cancer Res., Vol. 71, pp. 78-86. Also, "hsa-mir-4653" (miRBase Accession No. MI0017281; SEQ ID NO: 531) having a hairpin-like structure is known as a precursor of "hsa-miR-4653-3p."

The term "hsa-miR-636 gene" or "hsa-miR-636" used herein includes the hsa-miR-636 gene (miRBase Accession No. MIMAT0003306) shown in SEQ ID NO: 150, a homolog or an ortholog of a different organism species, and the like. The hsa-miR-636 gene can be obtained by a method described in Cummins JM et al., 2006, Proc. Natl. Acad. Sci. U.S.A., Vol. 103, pp. 3687-3692. Also, "hsa-mir-636" (miRBase Accession No. MI0003651; SEQ ID NO: 548) having a hairpin-like structure is known as a precursor of "hsa-miR-636."

The term "hsa-miR-4652-3p gene" or "hsa-miR-4652-3p" used herein includes the hsa-miR-4652-3p gene (miRBase Accession No. MIMAT0019717) shown in SEQ ID NO: 151, a homolog or an ortholog of a different organism species, and the like. The hsa-miR-4652-3p gene can be obtained by a method described in Persson H et al., 2011, Cancer Res., Vol. 71, pp. 78-86. Also, "hsa-mir-4652" (miRBase Accession No. MI0017280; SEQ ID NO: 549) having a hairpin-like structure is known as a precursor of "hsa-miR-4652-3p."

The term "hsa-miR-6823-5p gene" or "hsa-miR-6823-5p" used herein includes the hsa-miR-6823-5p gene (miRBase Accession No. MIMAT0027546) shown in SEQ ID NO: 152, a homolog or an ortholog of a different organism species, and the like. The hsa-miR-6823-5p gene can be obtained by a method described in Ladewig E et al., 2012, Genome Res., Vol. 22, pp. 1634-1645. Also, "hsa-mir-6823" (miRBase Accession No. MI0022668; SEQ ID NO: 550) having a hairpin-like structure is known as a precursor of "hsa-miR-6823-5p."

The term "hsa-miR-4502 gene" or "hsa-miR-4502" used herein includes the hsa-miR-4502 gene (miRBase Accession No. MIMAT0019038) shown in SEQ ID NO: 153, a homolog or an ortholog of a different organism species, and the like. The hsa-miR-4502 gene can be obtained by a method described in Jima DD et al., 2010, Blood, Vol. 116, e118-e127. Also, "hsa-mir-4502" (miRBase Accession No. MI0016865; SEQ ID NO: 551) having a hairpin-like structure is known as a precursor of "hsa-miR-4502."

The term "hsa-miR-7113-5p gene" or "hsa-miR-7113-5p" used herein includes the hsa-miR-7113-5p gene (miRBase Accession No. MIMAT0028123) shown in SEQ ID NO: 154, a homolog or an ortholog of a different organism species, and the like. The hsa-miR-7113-5p gene can be obtained by a method described in Ladewig E et al., 2012, Genome Res., Vol. 22, pp. 1634-1645. Also, "hsa-mir-7113" (miRBase Accession No. MI0022964; SEQ ID NO: 552) having a hairpin-like structure is known as a precursor of "hsa-miR-7113-5p."

The term "hsa-miR-8087 gene" or "hsa-miR-8087" used herein includes the hsa-miR-8087 gene (miRBase Accession No. MIMAT0031014) shown in SEQ ID NO: 155, a homolog or an ortholog of a different organism species, and the like. The hsa-miR-8087 gene can be obtained by a method described in Wang HJ et al., 2013, Shock, Vol. 39, pp. 480-487. Also, "hsa-mir-8087" (miRBase Accession No. MI0025923; SEQ ID NO: 553) having a hairpin-like structure is known as a precursor of "hsa-miR-8087."

The term "hsa-miR-7154-3p gene" or "hsa-miR-7154-3p" used herein includes the hsa-miR-7154-3p gene (miRBase Accession No. MIMAT0028219) shown in SEQ ID NO: 156, a homolog or an ortholog of a different organism species, and the like. The hsa-miR-7154-3p gene can be obtained by a method described in Meunier J et al., 2013, Genome Res., Vol. 23, pp. 34-45. Also, "hsa-mir-7154" (miRBase Accession No. MI0023614; SEQ ID NO: 554) having a hairpin-like structure is known as a precursor of "hsa-miR-7154-3p."

The term "hsa-miR-5189-5p gene" or "hsa-miR-5189-5p" used herein includes the hsa-miR-5189-5p gene (miRBase Accession No. MIMAT0021120) shown in SEQ ID NO: 157, a homolog or an ortholog of a different organism species, and the like. The hsa-miR-5189-5p gene can be obtained by a method described in Schotte D et al., 2011, Leukemia, Vol. 25, pp. 1389-1399. Also, "hsa-mir-5189" (miRBase Accession No. MI0018168; SEQ ID NO: 555) having a hairpin-like structure is known as a precursor of "hsa-miR-5189-5p."

The term "hsa-miR-1253 gene" or "hsa-miR-1253" used herein includes the hsa-miR-1253 gene (miRBase Accession No. MIMAT0005904) shown in SEQ ID NO: 158, a homolog or an ortholog of a different organism species, and the like. The hsa-miR-1253 gene can be obtained by a method described in Morin RD et al., 2008, Genome Res., Vol. 18, pp. 610-621. Also, "hsa-mir-1253" (miRBase Accession No. MI0006387; SEQ ID NO: 556) having a hairpin-like structure is known as a precursor of "hsa-miR-1253."

The term "hsa-miR-518c-5p gene" or "hsa-miR-518c-5p" used herein includes the hsa-miR-518c-5p gene (miRBase Accession No. MIMAT0002847) shown in SEQ ID NO: 159, a homolog or an ortholog of a different organism species, and the like. The hsa-miR-518c-5p gene can be obtained by a method described in Bentwich I et al., 2005, Nat. Genet., Vol. 37, pp. 766-770. Also, "hsa-mir-518c" (miRBase Accession No. MI0003159; SEQ ID NO: 557) having a hairpin-like structure is known as a precursor of "hsa-miR-518c-5p."

The term "hsa-miR-7151-5p gene" or "hsa-miR-7151-5p" used herein includes the hsa-miR-7151-5p gene (miRBase Accession No. MIMAT0028212) shown in SEQ ID NO: 160, a homolog or an ortholog of a different organism species, and the like. The hsa-miR-7151-5p gene can be obtained by a method described in Meunier J et al., 2013, Genome Res., Vol. 23, pp. 34-45. Also, "hsa-mir-7151" (miRBase Accession No. MI0023611; SEQ ID NO: 558) having a hairpin-like structure is known as a precursor of "hsa-miR-7151-5p."

The term "hsa-miR-3614-3p gene" or "hsa-miR-3614-3p" used herein includes the hsa-miR-3614-3p gene (miRBase Accession No. MIMAT0017993) shown in SEQ ID NO: 161, a homolog or an ortholog of a different organism species, and the like. The hsa-miR-3614-3p gene can be obtained by a method described in Witten D et al., 2010, BMC Biol., Vol. 8, p. 58. Also, "hsa-mir-3614" (miRBase Accession No. MI0016004; SEQ ID NO: 559) having a hairpin-like structure is known as a precursor of "hsa-miR-3614-3p."

The term "hsa-miR-4727-5p gene" or "hsa-miR-4727-5p" used herein includes the hsa-miR-4727-5p gene (miRBase Accession No. MIMAT0019847) shown in SEQ ID NO: 162, a homolog or an ortholog of a different organism species, and the like. The hsa-miR-4727-5p gene can be obtained by a method described in Persson H et al., 2011, Cancer Res., Vol. 71, pp. 78-86. Also, "hsa-mir-4727" (miRBase Accession No. MI0017364; SEQ ID NO: 560) having a hairpin-like structure is known as a precursor of "hsa-miR-4727-5p."

The term "hsa-miR-3682-5p gene" or "hsa-miR-3682-5p" used herein includes the hsa-miR-3682-5p gene (miRBase Accession No. MIMAT0019222) shown in SEQ ID NO: 163, a homolog or an ortholog of a different organism species, and the like. The hsa-miR-3682-5p gene can be obtained by a method described in Vaz C et al., 2010, BMC Genomics, Vol. 11, p. 288. Also, "hsa-mir-3682" (miRBase Accession No. MI0016083; SEQ ID NO: 561) having a hairpin-like structure is known as a precursor of "hsa-miR-3682-5p."

The term "hsa-miR-5090 gene" or "hsa-miR-5090" used herein includes the hsa-miR-5090 gene (miRBase Accession No. MIMAT0021082) shown in SEQ ID NO: 164, a homolog or an ortholog of a different organism species, and the like. The hsa-miR-5090 gene can be obtained by a method described in Ding N et al., 2011, J. Radiat. Res., Vol. 52, pp. 425-432. Also, "hsa-mir-5090" (miRBase Accession No. MI0017979; SEQ ID NO: 562) having a hairpin-like structure is known as a precursor of "hsa-miR-5090."

The term "hsa-miR-337-3p gene" or "hsa-miR-337-3p" used herein includes the hsa-miR-337-3p gene (miRBase Accession No. MIMAT0000754) shown in SEQ ID NO: 165, a homolog or an ortholog of a different organism species, and the like. The hsa-miR-337-3p gene can be obtained by a method described in Kim J et al., 2004, Proc. Natl. Acad. Sci. U.S.A., Vol. 101, pp. 360-365. Also, "hsa-mir-337" (miRBase Accession No. MI0000806; SEQ ID NO: 563) having a hairpin-like structure is known as a precursor of "hsa-miR-337-3p."

The term "hsa-miR-488-5p gene" or "hsa-miR-488-5p" used herein includes the hsa-miR-488-5p gene (miRBase Accession No. MIMAT0002804) shown in SEQ ID NO: 166, a homolog or an ortholog of a different organism species, and the like. The hsa-miR-488-5p gene can be obtained by a method described in Bentwich I et al., 2005, Nat. Genet., Vol. 37, pp. 766-770. Also, "hsa-mir-488" (miRBase Accession No. MI0003123; SEQ ID NO: 564) having a hairpin-like structure is known as a precursor of "hsa-miR-488-5p."

The term "hsa-miR-100-5p gene" or "hsa-miR-100-5p" used herein includes the hsa-miR-100-5p gene (miRBase Accession No. MIMAT0000098) shown in SEQ ID NO: 167, a homolog or an ortholog of a different organism species, and the like. The hsa-miR-100-5p gene can be obtained by a method described in Mourelatos Z et al., 2002, Genes Dev.,

Vol. 16, pp. 720-728. Also, "hsa-mir-100" (miRBase Accession No. MI0000102; SEQ ID NO: 565) having a hairpin-like structure is known as a precursor of "hsa-miR-100-5p."

The term "hsa-miR-4520-3p gene" or "hsa-miR-4520-3p" used herein includes the hsa-miR-4520-3p gene (miRBase Accession No. MIMAT0019057) shown in SEQ ID NO: 168, a homolog or an ortholog of a different organism species, and the like. The hsa-miR-4520-3p gene can be obtained by a method described in Jima DD et al., 2010, Blood, Vol. 116, e118-e127. Also, "hsa-mir-4520-1" (miRBase Accession No. MI0016886; SEQ ID NO: 566) having a hairpin-like structure is known as a precursor of "hsa-miR-4520-3p."

The term "hsa-miR-373-3p gene" or "hsa-miR-373-3p" used herein includes the hsa-miR-373-3p gene (miRBase Accession No. MIMAT0000726) shown in SEQ ID NO: 169, a homolog or an ortholog of a different organism species, and the like. The hsa-miR-373-3p gene can be obtained by a method described in Suh MR et al., 2004, Dev. Biol., Vol. 270, pp. 488-498. Also, "hsa-mir-373" (miRBase Accession No. MI0000781; SEQ ID NO: 567) having a hairpin-like structure is known as a precursor of "hsa-miR-373-3p."

The term "hsa-miR-6499-5p gene" or "hsa-miR-6499-5p" used herein includes the hsa-miR-6499-5p gene (miRBase Accession No. MIMAT0025450) shown in SEQ ID NO: 170, a homolog or an ortholog of a different organism species, and the like. The hsa-miR-6499-5p gene can be obtained by a method described in Joyce CE et al., 2011, Hum. Mol. Genet., Vol. 20, pp. 4025-4040. Also, "hsa-mir-6499" (miRBase Accession No. MI0022209; SEQ ID NO: 568) having a hairpin-like structure is known as a precursor of "hsa-miR-6499-5p."

The term "hsa-miR-3909 gene" or "hsa-miR-3909" used herein includes the hsa-miR-3909 gene (miRBase Accession No. MIMAT0018183) shown in SEQ ID NO: 171, a homolog or an ortholog of a different organism species, and the like. The hsa-miR-3909 gene can be obtained by a method described in Creighton CJ et al., 2010, PLoS One, Vol. 5, e9637. Also, "hsa-mir-3909" (miRBase Accession No. MI0016413; SEQ ID NO: 569) having a hairpin-like structure is known as a precursor of "hsa-miR-3909."

The term "hsa-miR-32-5p gene" or "hsa-miR-32-5p" used herein includes the hsa-miR-32-5p gene (miRBase Accession No. MIMAT0000090) shown in SEQ ID NO: 172, a homolog or an ortholog of a different organism species, and the like. The hsa-miR-32-5p gene can be obtained by a method described in Lagos-Quintana M et al., 2001, Science, Vol. 294, pp. 853-858. Also, "hsa-mir-32" (miRBase Accession No. MI0000090; SEQ ID NO: 570) having a hairpin-like structure is known as a precursor of "hsa-miR-3909."

The term "hsa-miR-302a-3p gene" or "hsa-miR-302a-3p" used herein includes the hsa-miR-302a-3p gene (miRBase Accession No. MIMAT0000684) shown in SEQ ID NO: 173, a homolog or an ortholog of a different organism species, and the like. The hsa-miR-302a-3p gene can be obtained by a method described in Houbaviy HB et al., 2003, Dev. Cell., Vol. 5, pp. 351-358. Also, "hsa-mir-302a" (miRBase Accession No. MI0000738; SEQ ID NO: 571) having a hairpin-like structure is known as a precursor of "hsa-miR-302a-3p."

The term "hsa-miR-4686 gene" or "hsa-miR-4686" used herein includes the hsa-miR-4686 gene (miRBase Accession No. MIMAT0019773) shown in SEQ ID NO: 174, a homolog or an ortholog of a different organism species, and the like. The hsa-miR-4686 gene can be obtained by a method described in Persson H et al., 2011, Cancer Res., Vol. 71, pp. 78-86. Also, "hsa-mir-4686" (miRBase Accession No. MI0017318; SEQ ID NO: 572) having a hairpin-like structure is known as a precursor of "hsa-miR-4686."

The term "hsa-miR-4659a-3p gene" or "hsa-miR-4659a-3p" used herein includes the hsa-miR-4659a-3p gene (miRBase Accession No. MIMAT0019727) shown in SEQ ID NO: 175, a homolog or an ortholog of a different organism species, and the like. The hsa-miR-4659a-3p gene can be obtained by a method described in Persson H et al., 2011, Cancer Res., Vol. 71, pp. 78-86. Also, "hsa-mir-4659a" (miRBase Accession No. MI0017287; SEQ ID NO: 573) having a hairpin-like structure is known as a precursor of "hsa-miR-4659a-3p."

The term "hsa-miR-4287 gene" or "hsa-miR-4287" used herein includes the hsa-miR-4287 gene (miRBase Accession No. MIMAT0016917) shown in SEQ ID NO: 176, a homolog or an ortholog of a different organism species, and the like. The hsa-miR-4287 gene can be obtained by a method described in Goff LA et al., 2009, PLoS One, Vol. 4, e7192. Also, "hsa-mir-4287" (miRBase Accession No. MI0015895; SEQ ID NO: 574) having a hairpin-like structure is known as a precursor of "hsa-miR-4287."

The term "hsa-miR-1301-5p gene" or "hsa-miR-1301-5p" used herein includes the hsa-miR-1301-5p gene (miRBase Accession No. MIMAT0026639) shown in SEQ ID NO: 177, a homolog or an ortholog of a different organism species, and the like. The hsa-miR-1301-5p gene can be obtained by a method described in Berezikov E et al., 2006, Genome Res., Vol. 16, pp. 1289-1298. Also, "hsa-mir-1301" (miRBase Accession No. MI0003815; SEQ ID NO: 575) having a hairpin-like structure is known as a precursor of "hsa-miR-1301-5p."

The term "hsa-miR-593-3p gene" or "hsa-miR-593-3p" used herein includes the hsa-miR-593-3p gene (miRBase Accession No. MIMAT0004802) shown in SEQ ID NO: 178, a homolog or an ortholog of a different organism species, and the like. The hsa-miR-593-3p gene can be obtained by a method described in Cummins JM et al., 2006, Proc. Natl. Acad. Sci. U.S.A., Vol. 103, pp. 3687-3692. Also, "hsa-mir-593" (miRBase Accession No. MI0003605; SEQ ID NO: 576) having a hairpin-like structure is known as a precursor of "hsa-miR-593-3p."

The term "hsa-miR-517a-3p gene" or "hsa-miR-517a-3p" used herein includes the hsa-miR-517a-3p gene (miRBase Accession No. MIMAT0002852) shown in SEQ ID NO: 179, a homolog or an ortholog of a different organism species, and the like. The hsa-miR-517a-3p gene can be obtained by a method described in Bentwich I et al., 2005, Nat. Genet., Vol. 37, pp. 766-770. Also, "hsa-mir-517a" (miRBase Accession No. MI0003161; SEQ ID NO: 577) having a hairpin-like structure is known as a precursor of "hsa-miR-593-3p."

The term "hsa-miR-517b-3p gene" or "hsa-miR-517b-3p" used herein includes the hsa-miR-517b-3p gene (miRBase Accession No. MIMAT0002857) shown in SEQ ID NO: 180, a homolog or an ortholog of a different organism species, and the like. The hsa-miR-517b-3p gene can be obtained by a method described in Bentwich I et al., 2005, Nat. Genet., Vol. 37, pp. 766-770. Also, "hsa-mir-517b" (miRBase Accession No. MI0003165; SEQ ID NO: 578) having a hairpin-like structure is known as a precursor of "hsa-miR-517b-3p."

The term "hsa-miR-142-3p gene" or "hsa-miR-142-3p" used herein includes the hsa-miR-142-3p gene (miRBase Accession No. MIMAT0000434) shown in SEQ ID NO: 181, a homolog or an ortholog of a different organism species, and the like. The hsa-miR-142-3p gene can be obtained by a method described in Lagos-Quintana M et al., 2002, Curr. Biol., Vol. 12, pp. 735-739. Also, "hsa-mir-142" (miRBase Accession No. MI0000458; SEQ ID NO: 579) having a hairpin-like structure is known as a precursor of "hsa-miR-142-3p."

The term "hsa-miR-1185-2-3p gene" or "hsa-miR-1185-2-3p" used herein includes the hsa-miR-1185-2-3p gene (miRBase Accession No. MIMAT0022713) shown in SEQ ID NO: 182, a homolog or an ortholog of a different organism species, and the like. The hsa-miR-1185-2-3p gene can be obtained by a method described in Berezikov E et al., 2006, Genome Res., Vol. 16, pp. 1289-1298. Also, "hsa-mir-1185-2" (miRBase Accession No. MI0003821; SEQ ID NO: 580) having a hairpin-like structure is known as a precursor of "hsa-miR-1185-2-3p."

The term "hsa-miR-602 gene" or "hsa-miR-602" used herein includes the hsa-miR-602 gene (miRBase Accession No. MIMAT0003270) shown in SEQ ID NO: 183, a homolog or an ortholog of a different organism species, and the like. The hsa-miR-602 gene can be obtained by a method described in Cummins JM et al., 2006, Proc. Natl. Acad. Sci. U.S.A., Vol. 103, pp. 3687-3692. Also, "hsa-mir-602" (miRBase Accession No. MI0003615; SEQ ID NO: 581) having a hairpin-like structure is known as a precursor of "hsa-miR-1185-2-3p."

The term "hsa-miR-527 gene" or "hsa-miR-527" used herein includes the hsa-miR-527 gene (miRBase Accession No. MIMAT0002862) shown in SEQ ID NO: 184, a homolog or an ortholog of a different organism species, and the like. The hsa-miR-527 gene can be obtained by a method described in Bentwich I et al., 2005, Nat. Genet., Vol. 37, pp. 766-770. Also, "hsa-mir-527" (miRBase Accession No. MI0003179; SEQ ID NO: 582) having a hairpin-like structure is known as a precursor of "hsa-miR-527."

The term "hsa-miR-518a-5p gene" or "hsa-miR-518a-5p" used herein includes the hsa-miR-518a-5p gene (miRBase Accession No. MIMAT0005457) shown in SEQ ID NO: 185, a homolog or an ortholog of a different organism species, and the like. The hsa-miR-518a-5p gene can be obtained by a method described in Bentwich I et al., 2005, Nat. Genet., Vol. 37, pp. 766-770. Also, "hsa-mir-518a-1 and hsa-mir-518a-2" (miRBase Accession Nos. MI0003170 and MI0003173; SEQ ID NOs: 583 and 584) each having a hairpin-like structure are known as precursors of "hsa-miR-518a-5p."

The term "hsa-miR-4682 gene" or "hsa-miR-4682" used herein includes the hsa-miR-4682 gene (miRBase Accession No. MIMAT0019767) shown in SEQ ID NO: 186, a homolog or an ortholog of a different organism species, and the like. The hsa-miR-4682 gene can be obtained by a method described in Persson H et al., 2011, Cancer Res., Vol. 71, pp. 78-86. Also, "hsa-mir-4682" (miRBase Accession No. MI0017314; SEQ ID NO: 585) having a hairpin-like structure is known as a precursor of "hsa-miR-4682."

The term "hsa-miR-28-5p gene" or "hsa-miR-28-5p" used herein includes the hsa-miR-28-5p gene (miRBase Accession No. MIMAT0000085) shown in SEQ ID NO: 187, a homolog or an ortholog of a different organism species, and the like. The hsa-miR-28-5p gene can be obtained by a method described in Lagos-Quintana M et al., 2001, Science, Vol. 294, pp. 853-858. Also, "hsa-mir-28" (miRBase Accession No. MI0000086; SEQ ID NO: 586) having a hairpin-like structure is known as a precursor of "hsa-miR-28-5p."

The term "hsa-miR-4252 gene" or "hsa-miR-4252" used herein includes the hsa-miR-4252 gene (miRBase Accession No. MIMAT0016886) shown in SEQ ID NO: 188, a homolog or an ortholog of a different organism species, and the like. The hsa-miR-4252 gene can be obtained by a method described in Goff LA et al., 2009, PLoS One, Vol. 4, e7192. Also, "hsa-mir-4252" (miRBase Accession No. MI0015864; SEQ ID NO: 587) having a hairpin-like structure is known as a precursor of "hsa-miR-4252."

The term "hsa-miR-452-5p gene" or "hsa-miR-452-5p" used herein includes the hsa-miR-452-5p gene (miRBase Accession No. MIMAT0001635) shown in SEQ ID NO: 189, a homolog or an ortholog of a different organism species, and the like. The hsa-miR-452-5p gene can be obtained by a method described in Altuvia Y et al., 2005, Nucleic Acids Res., Vol. 33, pp. 2697-2706. Also, "hsa-mir-452" (miRBase Accession No. MI0001733; SEQ ID NO: 588) having a hairpin-like structure is known as a precursor of "hsa-miR-452-5p."

The term "hsa-miR-525-5p gene" or "hsa-miR-525-5p" used herein includes the hsa-miR-525-5p gene (miRBase Accession No. MIMAT0002838) shown in SEQ ID NO: 190, a homolog or an ortholog of a different organism species, and the like. The hsa-miR-525-5p gene can be obtained by a method described in Bentwich I et al., 2005, Nat. Genet., Vol.

37, pp. 766-770. Also, "hsa-mir-525" (miRBase Accession No. MI0003152; SEQ ID NO: 589) having a hairpin-like structure is known as a precursor of "hsa-miR-525-5p."

The term "hsa-miR-3622a-3p gene" or "hsa-miR-3622a-3p" used herein includes the hsa-miR-3622a-3p gene (miRBase Accession No. MIMAT0018004) shown in SEQ ID NO: 191, a homolog or an ortholog of a different organism species, and the like. The hsa-miR-3622a-3p gene can be obtained by a method described in Witten D et al., 2010, BMC Biol., Vol. 8, p. 58. Also, "hsa-mir-3622a" (miRBase Accession No. MI0016013; SEQ ID NO: 590) having a hairpin-like structure is known as a precursor of "hsa-miR-3622a-3p."

The term "hsa-miR-6813-3p gene" or "hsa-miR-6813-3p" used herein includes the hsa-miR-6813-3p gene (miRBase Accession No. MIMAT0027527) shown in SEQ ID NO: 192, a homolog or an ortholog of a different organism species, and the like. The hsa-miR-6813-3p gene can be obtained by a method described in Ladewig E et al., 2012, Genome Res., Vol. 22, pp. 1634-1645. Also, "hsa-mir-6813" (miRBase Accession No. MI0022658; SEQ ID NO: 591) having a hairpin-like structure is known as a precursor of "hsa-miR-6813-3p."

The term "hsa-miR-4769-3p gene" or "hsa-miR-4769-3p" used herein includes the hsa-miR-4769-3p gene (miRBase Accession No. MIMAT0019923) shown in SEQ ID NO: 193, a homolog or an ortholog of a different organism species, and the like. The hsa-miR-4769-3p gene can be obtained by a method described in Persson H et al., 2011, Cancer Res., Vol. 71, pp. 78-86. Also, "hsa-mir-4769" (miRBase Accession No. MI0017410; SEQ ID NO: 592) having a hairpin-like structure is known as a precursor of "hsa-miR-4769-3p."

The term "hsa-miR-5698 gene" or "hsa-miR-5698" used herein includes the hsa-miR-5698 gene (miRBase Accession No. MIMAT0022491) shown in SEQ ID NO: 194, a homolog or an ortholog of a different organism species, and the like. The hsa-miR-5698 gene can be obtained by a method described in Watahiki A et al., 2011, PLoS One, Vol. 6, e24950. Also, "hsa-mir-5698" (miRBase Accession No. MI0019305; SEQ ID NO: 593) having a hairpin-like structure is known as a precursor of "hsa-miR-5698."

The term "hsa-miR-1915-3p gene" or "hsa-miR-1915-3p" used herein includes the hsa-miR-1915-3p gene (miRBase Accession No. MIMAT0007892) shown in SEQ ID NO: 195, a homolog or an ortholog of a different organism species, and the like. The hsa-miR-1915-3p gene can be obtained by a method described in Bar M et al., 2008, Stem Cells, Vol. 26, pp. 2496-2505. Also, "hsa-mir-1915" (miRBase Accession No. MI0008336; SEQ ID NO: 594) having a hairpin-like structure is known as a precursor of "hsa-miR-1915-3p."

The term "hsa-miR-1343-5p gene" or "hsa-miR-1343-5p" used herein includes the hsa-miR-1343-5p gene (miRBase Accession No. MIMAT0027038) shown in SEQ ID NO: 196, a homolog or an ortholog of a different organism species, and the like. The hsa-miR-1343-5p gene can be obtained by a method described in Persson H et al., 2011, Cancer Res., Vol. 71, pp. 78-86. Also, "hsa-mir-1343" (miRBase Accession No. MI0017320; SEQ ID NO: 595) having a hairpin-like structure is known as a precursor of "hsa-miR-1343-5p."

The term "hsa-miR-6861-5p gene" or "hsa-miR-6861-5p" used herein includes the hsa-miR-6861-5p gene (miRBase Accession No. MIMAT0027623) shown in SEQ ID NO: 197, a homolog or an ortholog of a different organism species, and the like. The hsa-miR-6861-5p gene can be obtained by a method described in Ladewig E et al., 2012, Genome Res., Vol. 22, pp. 1634-1645. Also, "hsa-mir-6861" (miRBase Accession No. MI0022708; SEQ ID NO: 596) having a hairpin-like structure is known as a precursor of "hsa-miR-6861-5p."

The term "hsa-miR-6781-5p gene" or "hsa-miR-6781-5p" used herein includes the hsa-miR-6781-5p gene (miRBase Accession No. MIMAT0027462) shown in SEQ ID NO: 198, a homolog or an ortholog of a different organism species, and the like. The hsa-miR-6781-5p gene can be obtained by a method described in Ladewig E et al., 2012, Genome Res., Vol. 22, pp. 1634-1645. Also, "hsa-mir-6781" (miRBase Accession No. MI0022626; SEQ ID NO: 597) having a hairpin-like structure is known as a precursor of "hsa-miR-6781-5p."

The term "hsa-miR-4508 gene" or "hsa-miR-4508" used herein includes the hsa-miR-4508 gene (miRBase Accession No. MIMAT0019045) shown in SEQ ID NO: 199, a homolog or an ortholog of a different organism species, and the like. The hsa-miR-4508 gene can be obtained by a method described in Jima DD et al., 2010, Blood, Vol. 116, e118-e127. Also, "hsa-mir-4508" (miRBase Accession No. MI0016872; SEQ ID NO: 598) having a hairpin-like structure is known as a precursor of "hsa-miR-4508."

The term "hsa-miR-6743-5p gene" or "hsa-miR-6743-5p" used herein includes the hsa-miR-6743-5p gene (miRBase Accession No. MIMAT0027387) shown in SEQ ID NO: 200, a homolog or an ortholog of a different organism species, and the like. The hsa-miR-6743-5p gene can be obtained by a method described in Ladewig E et al., 2012, Genome Res., Vol. 22, pp. 1634-1645. Also, "hsa-mir-6743" (miRBase Accession No. MI0022588; SEQ ID NO: 599) having a hairpin-like structure is known as a precursor of "hsa-miR-6743-5p."

The term "hsa-miR-6726-5p gene" or "hsa-miR-6726-5p" used herein includes the hsa-miR-6726-5p gene (miRBase Accession No. MIMAT0027353) shown in SEQ ID NO: 201, a homolog or an ortholog of a different organism species, and the like. The hsa-miR-6726-5p gene can be obtained by a method described in Ladewig E et al., 2012, Genome Res., Vol. 22, pp. 1634-1645. Also, "hsa-mir-6726" (miRBase Accession No. MI0022571; SEQ ID NO: 600) having a hairpin-like structure is known as a precursor of "hsa-miR-6726-5p."

The term "hsa-miR-4525 gene" or "hsa-miR-4525" used herein includes the hsa-miR-4525 gene (miRBase Accession No. MIMAT0019064) shown in SEQ ID NO: 202, a homolog or an ortholog of a different organism species, and the like. The hsa-miR-4525 gene can be obtained by a method described in Jima DD et al., 2010, Blood, Vol. 116, e118-e127. Also, "hsa-mir-4525" (miRBase Accession No. MI0016892; SEQ ID NO: 601) having a hairpin-like structure is known as a precursor of "hsa-miR-4525."

The term "hsa-miR-4651 gene" or "hsa-miR-4651" used herein includes the hsa-miR-4651 gene (miRBase Accession No. MIMAT0019715) shown in SEQ ID NO: 203, a homolog or an ortholog of a different organism species, and the like. The hsa-miR-4651 gene can be obtained by a method described in Persson H et al., 2011, Cancer Res., Vol. 71, pp. 78-86. Also, "hsa-mir-4651" (miRBase Accession No. MI0017279; SEQ ID NO: 602) having a hairpin-like structure is known as a precursor of "hsa-miR-4651."

The term "hsa-miR-6813-5p gene" or "hsa-miR-6813-5p" used herein includes the hsa-miR-6813-5p gene (miRBase Accession No. MIMAT0027526) shown in SEQ ID NO: 204, a homolog or an ortholog of a different organism species, and the like. The hsa-miR-6813-5p gene can be obtained by a method described in Ladewig E et al., 2012, Genome Res., Vol. 22, pp. 1634-1645. Also, "hsa-mir-6813" (miRBase Accession No. MI0022658; SEQ ID NO: 591) having a hairpin-like structure is known as a precursor of "hsa-miR-6813-5p."

The term "hsa-miR-5787 gene" or "hsa-miR-5787" used herein includes the hsa-miR-5787 gene (miRBase Accession No. MIMAT0023252) shown in SEQ ID NO: 205, a homolog or an ortholog of a different organism species, and the like. The hsa-miR-5787 gene can be obtained by a method described in Yoo H et al., 2011, Biochem. Biophys. Res. Commun., Vol. 415, pp. 567-572. Also, "hsa-mir-5787" (miRBase Accession No. MI0019797; SEQ ID NO: 603) having a hairpin-like structure is known as a precursor of "hsa-miR-5787."

The term "hsa-miR-1290 gene" or "hsa-miR-1290" used herein includes the hsa-miR-1290 gene (miRBase Accession No. MIMAT0005880) shown in SEQ ID NO: 206, a homolog or an ortholog of a different organism species, and the like. The hsa-miR-1290 gene can be obtained by a method described in Morin RD et al., 2008, Genome Res., Vol. 18, pp. 610-621. Also, "hsa-mir-1290" (miRBase Accession No. MI0006352; SEQ ID NO: 604) having a hairpin-like structure is known as a precursor of "hsa-miR-1290."

The term "hsa-miR-6075 gene" or "hsa-miR-6075" used herein includes the hsa-miR-6075 gene (miRBase Accession No. MIMAT0023700) shown in SEQ ID NO: 207, a homolog or an ortholog of a different organism species, and the like. The hsa-miR-6075 gene can be obtained by a method described in Voellenkle C et al., 2012, RNA, Vol. 18, p. 472-484. Also, "hsa-mir-6075" (miRBase Accession No. MI0020352; SEQ ID NO: 605) having a hairpin-like structure is known as a precursor of "hsa-miR-6075."

The term "hsa-miR-4758-5p gene" or "hsa-miR-4758-5p" used herein includes the hsa-miR-4758-5p gene (miRBase Accession No. MIMAT0019903) shown in SEQ ID NO: 208, a homolog or an ortholog of a different organism species, and the like. The hsa-miR-4758-5p gene can be obtained by a method described in Persson H et al., 2011, Cancer Res., Vol. 71, pp. 78-86. Also, "hsa-mir-4758" (miRBase Accession No. MI0017399; SEQ ID NO: 606) having a hairpin-like structure is known as a precursor of "hsa-miR-4758-5p."

The term "hsa-miR-4690-5p gene" or "hsa-miR-4690-5p" used herein includes the hsa-miR-4690-5p gene (miRBase Accession No. MIMAT0019779) shown in SEQ ID NO: 209, a homolog or an ortholog of a different organism species, and the like. The hsa-miR-4690-5p gene can be obtained by a method described in Persson H et al., 2011, Cancer Res., Vol. 71, pp. 78-86. Also, "hsa-mir-4690" (miRBase Accession No. MI0017323; SEQ ID NO: 607) having a hairpin-like structure is known as a precursor of "hsa-miR-4690-5p."

The term "hsa-miR-762 gene" or "hsa-miR-762" used herein includes the hsa-miR-762 gene (miRBase Accession No. MIMAT0010313) shown in SEQ ID NO: 210, a homolog or an ortholog of a different organism species, and the like. The hsa-miR-762 gene can be obtained by a method described in Berezikov E et al., 2006, Genome Res., Vol. 16, pp. 1289-1298. Also, "hsa-mir-762" (miRBase Accession No. MI0003892; SEQ ID NO: 608) having a hairpin-like structure is known as a precursor of "hsa-miR-762."

The term "hsa-miR-1225-3p gene" or "hsa-miR-1225-3p" used herein includes the hsa-miR-1225-3p gene (miRBase Accession No. MIMAT0005573) shown in SEQ ID NO: 211, a homolog or an ortholog of a different organism species, and the like. The hsa-miR-1225-3p gene can be obtained by a method described in Berezikov E et al., 2007, Mol. Cell, Vol. 28, pp. 328-336. Also, "hsa-mir-1225" (miRBase Accession No. MI0006311; SEQ ID NO: 609) having a hairpin-like structure is known as a precursor of "hsa-miR-1225-3p."

The term "hsa-miR-3184-5p gene" or "hsa-miR-3184-5p" used herein includes the hsa-miR-3184-5p gene (miRBase Accession No. MIMAT0015064) shown in SEQ ID NO: 212, a homolog or an ortholog of a different organism species, and the like. The hsa-miR-3184-5p gene can be obtained by a method described in Stark MS et al., 2010, PLoS One, Vol. 5, e9685. Also, "hsa-mir-3184" (miRBase Accession No. MI0014226; SEQ ID NO: 398) having a hairpin-like structure is known as a precursor of "hsa-miR-3184-5p."

The term "hsa-miR-665 gene" or "hsa-miR-665" used herein includes the hsa-miR-665 gene (miRBase Accession No. MIMAT0004952) shown in SEQ ID NO: 213, a homolog or an ortholog of a different organism species, and the like. The hsa-miR-665 gene can be obtained by a method described in Berezikov E et al., 2006, Genome Res., Vol. 16, pp. 1289-1298. Also, "hsa-mir-665" (miRBase Accession No. MI0005563; SEQ ID NO: 610) having a hairpin-like structure is known as a precursor of "hsa-miR-665."

The term "hsa-miR-211-5p gene" or "hsa-miR-211-5p" used herein includes the hsa-miR-211-5p gene (miRBase Accession No. MIMAT0000268) shown in SEQ ID NO: 214, a homolog or an ortholog of a different organism species, and the like. The hsa-miR-211-5p gene can be obtained by a method described in Lim LP et al., 2003, Science, Vol. 299, p. 1540. Also, "hsa-mir-211" (miRBase Accession No. MI0000287; SEQ ID NO: 611) having a hairpin-like structure is known as a precursor of "hsa-miR-211-5p."

The term "hsa-miR-1247-5p gene" or "hsa-miR-1247-5p" used herein includes the hsa-miR-1247-5p gene (miRBase Accession No. MIMAT0005899) shown in SEQ ID NO: 215, a homolog or an ortholog of a different organism species, and the like. The hsa-miR-1247-5p gene can be obtained by a method described in Morin RD et al., 2008, Genome Res., Vol. 18, pp. 610-621. Also, "hsa-mir-1247" (miRBase Accession No. MI0006382; SEQ ID NO: 489) having a hairpin-like structure is known as a precursor of "hsa-miR-1247-5p."

The term "hsa-miR-3656 gene" or "hsa-miR-3656" used herein includes the hsa-miR-3656 gene (miRBase Accession No. MIMAT0018076) shown in SEQ ID NO: 216, a homolog or an ortholog of a different organism species, and the like. The hsa-miR-3656 gene can be obtained by a method described in Meiri E et al., 2010, Nucleic Acids Res., Vol. 38, p. 6234-6246. Also, "hsa-mir-3656" (miRBase Accession No. MI0016056; SEQ ID NO: 612) having a hairpin-like structure is known as a precursor of "hsa-miR-3656."

The term "hsa-miR-149-5p gene" or "hsa-miR-149-5p" used herein includes the hsa-miR-149-5p gene (miRBase Accession No. MIMAT0000450) shown in SEQ ID NO: 217, a homolog or an ortholog of a different organism species, and the like. The hsa-miR-149-5p gene can be obtained by a method described in Lagos-Quintana M et al., 2002, Curr. Biol., Vol. 12, pp. 735-739. Also, "hsa-mir-149" (miRBase Accession No. MI0000478; SEQ ID NO: 613) having a hairpin-like structure is known as a precursor of "hsa-miR-149-5p."

The term "hsa-miR-744-5p gene" or "hsa-miR-744-5p" used herein includes the hsa-miR-744-5p gene (miRBase Accession No. MIMAT0004945) shown in SEQ ID NO: 218, a homolog or an ortholog of a different organism species, and the like. The hsa-miR-744-5p gene can be obtained by a method described in Berezikov E et al., 2006, Genome Res., Vol. 16, pp. 1289-1298. Also, "hsa-mir-744" (miRBase Accession No. MI0005559; SEQ ID NO: 614) having a hairpin-like structure is known as a precursor of "hsa-miR-744-5p."

The term "hsa-miR-345-5p gene" or "hsa-miR-345-5p" used herein includes the hsa-miR-345-5p gene (miRBase Accession No. MIMAT0000772) shown in SEQ ID NO: 219, a homolog or an ortholog of a different organism species, and the like. The hsa-miR-345-5p gene can be obtained by a method described in Kim J et al., 2004, Proc. Natl. Acad. Sci.U.S.A., Vol. 101, pp. 360-365. Also, "hsa-mir-345" (miRBase Accession No. MI0000825; SEQ ID NO: 615) having a hairpin-like structure is known as a precursor of "hsa-miR-345-5p."

The term "hsa-miR-150-5p gene" or "hsa-miR-150-5p" used herein includes the hsa-miR-150-5p gene (miRBase Accession No. MIMAT0000451) shown in SEQ ID NO: 220, a homolog or an ortholog of a different organism species, and the like. The hsa-miR-150-5p gene can be obtained by a method described in Lagos-Quintana M et al., 2002, Curr. Biol., Vol. 12, pp. 735-739. Also, "hsa-mir-150" (miRBase Accession No. MI0000479; SEQ ID NO: 616) having a hairpin-like structure is known as a precursor of "hsa-miR-150-5p."

The term "hsa-miR-191-3p gene" or "hsa-miR-191-3p" used herein includes the hsa-miR-191-3p gene (miRBase Accession No. MIMAT0001618) shown in SEQ ID NO: 221, a homolog or an ortholog of a different organism species, and the like. The hsa-miR-191-3p gene can be obtained by a method described in Lagos-Quintana M et al., 2003, RNA, Vol. 9, pp. 175-179. Also, "hsa-mir-191" (miRBase Accession No. MI0000465; SEQ ID NO: 617) having a hairpin-like structure is known as a precursor of "hsa-miR-191-3p."

The term "hsa-miR-651-5p gene" or "hsa-miR-651-5p" used herein includes the hsa-miR-651-5p gene (miRBase Accession No. MIMAT0003321) shown in SEQ ID NO: 222, a homolog or an ortholog of a different organism species, and the like. The hsa-miR-651-5p gene can be obtained by a method described in Cummins JM et al., 2006, Proc. Natl. Acad. Sci., U.S.A., Vol. 103, pp. 3687-3692. Also, "hsa-mir-651" (miRBase Accession No. MI0003666; SEQ ID NO: 618) having a hairpin-like structure is known as a precursor of "hsa-miR-651-5p."

The term "hsa-miR-34a-5p gene" or "hsa-miR-34a-5p" used herein includes the hsa-miR-34a-5p gene (miRBase Accession No. MIMAT0000255) shown in SEQ ID NO: 223, a homolog or an ortholog of a different organism species, and the like. The hsa-miR-34a-5p gene can be obtained by a method described in Dostie J et al., 2003, RNA, Vol. 9, pp. 180-186. Also, "hsa-mir-34a" (miRBase Accession No. MI0000268; SEQ ID NO: 619) having a hairpin-like structure is known as a precursor of "hsa-miR-34a-5p."

The term "hsa-miR-409-5p gene" or "hsa-miR-409-5p" used herein includes the hsa-miR-409-5p gene (miRBase Accession No. MIMAT0001638) shown in SEQ ID NO: 224, a homolog or an ortholog of a different organism species, and the like. The hsa-miR-409-5p gene can be obtained by a method described in Altuvia Y et al., 2005, Nucleic Acids Res., Vol. 33, pp. 2697-2706. Also, "hsa-mir-409" (miRBase Accession No. MI0001735; SEQ ID NO: 620) having a hairpin-like structure is known as a precursor of "hsa-miR-409-5p."

The term "hsa-miR-369-5p gene" or "hsa-miR-369-5p" used herein includes the hsa-miR-369-5p gene (miRBase Accession No. MIMAT0001621) shown in SEQ ID NO: 225, a homolog or an ortholog of a different organism species, and the like. The hsa-miR-369-5p gene can be obtained by a method described in Suh MR et al., 2004, Dev. Biol., Vol. 270, pp. 488-498. Also, "hsa-mir-369" (miRBase Accession No. MI0000777; SEQ ID NO: 621) having a hairpin-like structure is known as a precursor of "hsa-miR-369-5p."

The term "hsa-miR-1915-5p gene" or "hsa-miR-1915-5p" used herein includes the hsa-miR-1915-5p gene (miRBase Accession No. MIMAT0007891) shown in SEQ ID NO: 226, a homolog or an ortholog of a different organism species, and the like. The hsa-miR-1915-5p gene can be obtained by a method described in Bar M et al., 2008, Stem Cells, Vol. 26, pp. 2496-2505. Also, "hsa-mir-1915" (miRBase Accession No. MI0008336; SEQ ID NO: 594) having a hairpin-like structure is known as a precursor of "hsa-miR-1915-5p."

The term "hsa-miR-204-5p gene" or "hsa-miR-204-5p" used herein includes the hsa-miR-204-5p gene (miRBase Accession No. MIMAT0000265) shown in SEQ ID NO: 227, a homolog or an ortholog of a different organism species, and the like. The hsa-miR-204-5p gene can be obtained by a method described in Lim LP et al., 2003, Science, Vol. 299, p. 1540. Also, "hsa-mir-204" (miRBase Accession No. MI0000284; SEQ ID NO: 622) having a hairpin-like structure is known as a precursor of "hsa-miR-204-5p."

The term "hsa-miR-137 gene" or "hsa-miR-137" used herein includes the hsa-miR-137 gene (miRBase Accession No. MIMAT0000429) shown in SEQ ID NO: 228, a homolog or an ortholog of a different organism species, and the like. The hsa-miR-137 gene can be obtained by a method described in Lagos-Quintana M et al., 2002, Curr. Biol., Vol. 12, pp. 735-739. Also, "hsa-mir-137" (miRBase Accession No. MI0000454; SEQ ID NO: 623) having a hairpin-like structure is known as a precursor of "hsa-miR-137."

The term "hsa-miR-382-5p gene" or "hsa-miR-382-5p" used herein includes the hsa-miR-382-5p gene (miRBase Accession No. MIMAT0000737) shown in SEQ ID NO: 229, a homolog or an ortholog of a different organism species, and the like. The hsa-miR-382-5p gene can be obtained by a method described in Altuvia Y et al., 2005, Nucleic Acids Res., Vol. 33, pp. 2697-2706. Also, "hsa-mir-382" (miRBase Accession No. MI0000790; SEQ ID NO: 624) having a hairpin-like structure is known as a precursor of "hsa-miR-382-5p."

The term "hsa-miR-517-5p gene" or "hsa-miR-517-5p" used herein includes the hsa-miR-517-5p gene (miRBase Accession No. MIMAT0002851) shown in SEQ ID NO: 230, a homolog or an ortholog of a different organism species, and the like. The hsa-miR-517-5p gene can be obtained by a method described in Bentwich I et al., 2005, Nat. Genet., Vol. 37, pp. 766-770. Also, "hsa-mir-517c" (miRBase Accession No. M10003174; SEQ ID NO: 625) having a hairpin-like structure is known as a precursor of "hsa-miR-517-5p."

The term "hsa-miR-532-5p gene" or "hsa-miR-532-5p" used herein includes the hsa-miR-532-5p gene (miRBase Accession No. MIMAT0002888) shown in SEQ ID NO: 231, a homolog or an ortholog of a different organism species, and the like. The hsa-miR-532-5p gene can be obtained by a method described in Lagos-Quintana M et al., 2001, Science, Vol. 294, pp. 853-858. Also, "hsa-mir-532" (miRBase Accession No. MI0003205; SEQ ID NO: 626) having a hairpin-like structure is known as a precursor of "hsa-miR-532-5p."

The term "hsa-miR-22-5p gene" or "hsa-miR-22-5p" used herein includes the hsa-miR-22-5p gene (miRBase Accession No. MIMAT0004495) shown in SEQ ID NO: 232, a homolog or an ortholog of a different organism species, and the like. The hsa-miR-22-5p gene can be obtained by a method described in Berezikov E et al., 2007, Mol. Cell, Vol. 28, pp. 328-336. Also, "hsa-mir-22" (miRBase Accession No. MI0000078; SEQ ID NO: 627) having a hairpin-like structure is known as a precursor of "hsa-miR-22-5p."

The term "hsa-miR-1237-3p gene" or "hsa-miR-1237-3p" used herein includes the hsa-miR-1237-3p gene (miRBase Accession No. MIMAT0005592) shown in SEQ ID NO: 233, a homolog or an ortholog of a different organism species, and the like. The hsa-miR-1237-3p gene can be obtained by a method described in Berezikov E et al., 2006, Genome Res., Vol. 16, pp. 1289-1298. Also, "hsa-mir-1237" (miRBase Accession No. MI0006327; SEQ ID NO: 628) having a hairpin-like structure is known as a precursor of "hsa-miR-1237-3p."

The term "hsa-miR-1224-3p gene" or "hsa-miR-1224-3p" used herein includes the hsa-miR-1224-3p gene (miRBase Accession No. MIMAT0005459) shown in SEQ ID NO: 234, a homolog or an ortholog of a different organism species, and the like. The hsa-miR-1224-3p gene can be obtained by a method described in Cummins JM et al., 2006, Proc. Natl. Acad. Sci. U.S.A., Vol. 103, pp. 3687-3692. Also, "hsa-mir-1224" (miRBase Accession No. MI0003764; SEQ ID NO: 629) having a hairpin-like structure is known as a precursor of "hsa-miR-1224-3p."

The term "hsa-miR-625-3p gene" or "hsa-miR-625-3p" used herein includes the hsa-miR-625-3p gene (miRBase Accession No. MIMAT0004808) shown in SEQ ID NO: 235, a homolog or an ortholog of a different organism species, and the like. The hsa-miR-625-3p gene can be obtained by a method described in Kim J et al., 2004, Proc. Natl. Acad. Sci., U.S.A. Vol. 101, pp. 360-365. Also, "hsa-mir-625" (miRBase Accession No. MI0003639; SEQ ID NO: 630) having a hairpin-like structure is known as a precursor of "hsa-miR-625-3p."

The term "hsa-miR-328-3p gene" or "hsa-miR-328-3p" used herein includes the hsa-miR-328-3p gene (miRBase Accession No. MIMAT0000752) shown in SEQ ID NO: 236, a homolog or an ortholog of a different organism species, and the like. The hsa-miR-328-3p gene can be obtained by a method described in Lagos-Quintana M et al., 2002, Curr. Biol., Vol. 12, pp. 735-739. Also, "hsa-mir-328" (miRBase Accession No. MI0000804; SEQ ID NO: 631) having a hairpin-like structure is known as a precursor of "hsa-miR-328-3p."

The term "hsa-miR-122-5p gene" or "hsa-miR-122-5p" used herein includes the hsa-miR-122-5p gene (miRBase Accession No. MIMAT0000421) shown in SEQ ID NO: 237, a homolog or an ortholog of a different organism species, and the like. The hsa-miR-122-5p gene can be obtained by a method described in Bentwich I et al., 2005, Nat. Genet., Vol. 37, pp. 766-770. Also, "hsa-mir-122" (miRBase Accession No. MI0000442; SEQ ID NO: 632) having a hairpin-like structure is known as a precursor of "hsa-miR-122-5p."

The term "hsa-miR-202-3p gene" or "hsa-miR-202-3p" used herein includes the hsa-miR-202-3p gene (miRBase Accession No. MIMAT0002811) shown in SEQ ID NO: 238, a homolog or an ortholog of a different organism species, and the like. The hsa-miR-202-3p gene can be obtained by a method described in Persson H et al., 2011, Cancer Res., Vol. 71, pp. 78-86. Also, "hsa-mir-202" (miRBase Accession No. MI0003130; SEQ ID NO: 485) having a hairpin-like structure is known as a precursor of "hsa-miR-202-3p."

The term "hsa-miR-4781-5p gene" or "hsa-miR-4781-5p" used herein includes the hsa-miR-4781-5p gene (miRBase Accession No. MIMAT0019942) shown in SEQ ID NO: 239, a homolog or an ortholog of a different organism species, and the like. The hsa-miR-4781-5p gene can be obtained by a method described in Artzi S et al., 2008, BMC Bioinformatics, Vol. 9, p. 39. Also, "hsa-mir-4781" (miRBase Accession No. MI0017426; SEQ ID NO: 633) having a hairpin-like structure is known as a precursor of "hsa-miR-4781-5p."

The term "hsa-miR-718 gene" or "hsa-miR-718" used herein includes the hsa-miR-718 gene (miRBase Accession No. MIMAT0012735) shown in SEQ ID NO: 240, a homolog or an ortholog of a different organism species, and the like. The hsa-miR-718 gene can be obtained by a method described in Kim J et al., 2004, Proc. Natl. Acad. Sci. U.S.A., Vol. 101, pp. 360-365. Also, "hsa-mir-718" (miRBase Accession No. MI0012489; SEQ ID NO: 634) having a hairpin-like structure is known as a precursor of "hsa-miR-718."

The term "hsa-miR-342-3p gene" or "hsa-miR-342-3p" used herein includes the hsa-miR-342-3p gene (miRBase Accession No. MIMAT0000753) shown in SEQ ID NO: 241, a homolog or an ortholog of a different organism species, and the like. The hsa-miR-342-3p gene can be obtained by a method described in Lagos-Quintana M et al., 2001, Science, Vol. 294, pp. 853-858. Also, "hsa-mir-342" (miRBase Accession No. MI0000805; SEQ ID NO: 635) having a hairpin-like structure is known as a precursor of "hsa-miR-342-3p."

The term "hsa-miR-26b-3p gene" or "hsa-miR-26b-3p" used herein includes the hsa-miR-26b-3p gene (miRBase Accession No. MIMAT0004500) shown in SEQ ID NO: 242, a homolog or an ortholog of a different organism species, and the like. The hsa-miR-26b-3p gene can be obtained by a method described in Lagos-Quintana M et al., 2002, Curr. Biol., Vol. 12, pp. 735-739. Also, "hsa-mir-26b" (miRBase Accession No. MI0000084; SEQ ID NO: 636) having a hairpin-like structure is known as a precursor of "hsa-miR-26b-3p."

The term "hsa-miR-140-3p gene" or "hsa-miR-140-3p" used herein includes the hsa-miR-140-3p gene (miRBase Accession No. MIMAT0004597) shown in SEQ ID NO: 243, a homolog or an ortholog of a different organism species, and the like. The hsa-miR-140-3p gene can be obtained by a method described in Lagos-Quintana M et al., 2003, RNA, Vol. 9, pp. 175-179. Also, "hsa-mir-140" (miRBase Accession No. MI0000456; SEQ ID NO: 637) having a hairpin-like structure is known as a precursor of "hsa-miR-140-3p."

The term "hsa-miR-200a-3p gene" or "hsa-miR-200a-3p" used herein includes the hsa-miR-200a-3p gene (miRBase Accession No. MIMAT0000682) shown in SEQ ID NO: 244, a homolog or an ortholog of a different organism species, and the like. The hsa-miR-200a-3p gene can be obtained by a method described in Kasashima K et al., 2004, Biochem. Biophys. Res. Commun., Vol. 322, pp. 403-410. Also, "hsa-mir-200a" (miRBase Accession No. MI0000737; SEQ ID NO: 638) having a hairpin-like structure is known as a precursor of "hsa-miR-200a-3p."

The term "hsa-miR-378a-3p gene" or "hsa-miR-378a-3p" used herein includes the hsa-miR-378a-3p gene (miRBase Accession No. MIMAT0000732) shown in SEQ ID NO: 245, a homolog or an ortholog of a different organism species, and the like. The hsa-miR-378a-3p gene can be obtained by a method described in Fu H et al., 2005, FEBS Lett., Vol. 579, pp. 3849-3854. Also, "hsa-mir-378a" (miRBase Accession No. MI0000786; SEQ ID NO: 639) having a hairpin-like structure is known as a precursor of "hsa-miR-378a-3p."

The term "hsa-miR-484 gene" or "hsa-miR-484" used herein includes the hsa-miR-484 gene (miRBase Accession No. MIMAT0002174) shown in SEQ ID NO: 246, a homolog or an ortholog of a different organism species, and the like. The hsa-miR-484 gene can be obtained by a method described in Houbaviy HB et al., 2003, Dev. Cell., Vol. 5, pp. 351-358. Also, "hsa-mir-484" (miRBase Accession No. MI0002468; SEQ ID NO: 640) having a hairpin-like structure is known as a precursor of "hsa-miR-484."

The term "hsa-miR-296-5p gene" or "hsa-miR-296-5p" used herein includes the hsa-miR-296-5p gene (miRBase Accession No. MIMAT0000690) shown in SEQ ID NO: 247, a homolog or an ortholog of a different organism species, and the like. The hsa-miR-296-5p gene can be obtained by a method described in Lim LP et al., 2003, Science, Vol. 299, p. 1540. Also, "hsa-mir-296" (miRBase Accession No. MI0000747; SEQ ID NO: 641) having a hairpin-like structure is known as a precursor of "hsa-miR-296-5p."

The term "hsa-miR-205-5p gene" or "hsa-miR-205-5p" used herein includes the hsa-miR-205-5p gene (miRBase Accession No. MIMAT0000266) shown in SEQ ID NO: 248, a homolog or an ortholog of a different organism species, and the like. The hsa-miR-205-5p gene can be obtained by a method described in Altuvia Y et al., 2005, Nucleic Acids Res.,

Vol. 33, pp. 2697-2706. Also, "hsa-mir-205" (miRBase Accession No. MI0000285; SEQ ID NO: 642) having a hairpin-like structure is known as a precursor of "hsa-miR-205-5p."

The term "hsa-miR-431-5p gene" or "hsa-miR-431-5p" used herein includes the hsa-miR-431-5p gene (miRBase Accession No. MIMAT0001625) shown in SEQ ID NO: 249, a homolog or an ortholog of a different organism species, and the like. The hsa-miR-431-5p gene can be obtained by a method described in Kawaji H et al., 2008, BMC Genomics, Vol. 9, p. 157. Also, "hsa-mir-431" (miRBase Accession No. MI0001721; SEQ ID NO: 643) having a hairpin-like structure is known as a precursor of "hsa-miR-431-5p."

The term "hsa-miR-1471 gene" or "hsa-miR-1471" used herein includes the hsa-miR-1471 gene (miRBase Accession No. MIMAT0007349) shown in SEQ ID NO: 250, a homolog or an ortholog of a different organism species, and the like. The hsa-miR-1471 gene can be obtained by a method described in Azuma, Mukai, A et al., 2008, Proc. Natl. Acad. Sci. U.S.A., Vol. 105, pp. 7964-7969. Also, "hsa-mir-1471" (miRBase Accession No. MI0007076; SEQ ID NO: 644) having a hairpin-like structure is known as a precursor of "hsa-miR-1471."

The term "hsa-miR-1538 gene" or "hsa-miR-1538" used herein includes the hsa-miR-1538 gene (miRBase Accession No. MIMAT0007400) shown in SEQ ID NO: 251, a homolog or an ortholog of a different organism species, and the like. The hsa-miR-1538 gene can be obtained by a method described in Cummins JM et al., 2006, Proc. Natl. Acad. Sci. U.S.A., Vol. 103, pp. 3687-3692. Also, "hsa-mir-1538" (miRBase Accession No. MI0007259; SEQ ID NO: 645) having a hairpin-like structure is known as a precursor of "hsa-miR-1538."

The term "hsa-miR-449b-3p gene" or "hsa-miR-449b-3p" used herein includes the hsa-miR-449b-3p gene (miRBase Accession No. MIMAT0009203) shown in SEQ ID NO: 252, a homolog or an ortholog of a different organism species, and the like. The hsa-miR-449b-3p gene can be obtained by a method described in Schotte D et al., 2009, Leukemia, Vol. 23, pp. 313-322. Also, "hsa-mir-449b" (miRBase Accession No. MI0003673; SEQ ID NO: 646) having a hairpin-like structure is known as a precursor of "hsa-miR-449b-3p."

The term "hsa-miR-1976 gene" or "hsa-miR-1976" used herein includes the hsa-miR-1976 gene (miRBase Accession No. MIMAT0009451) shown in SEQ ID NO: 253, a homolog or an ortholog of a different organism species, and the like. The hsa-miR-1976 gene can be obtained by a method described in Goff LA et al., 2009, PLoS One, Vol. 4, e7192. Also, "hsa-mir-1976" (miRBase Accession No. MI0009986; SEQ ID NO: 647) having a hairpin-like structure is known as a precursor of "hsa-miR-1976."

The term "hsa-miR-4268 gene" or "hsa-miR-4268" used herein includes the hsa-miR-4268 gene (miRBase Accession No. MIMAT0016896) shown in SEQ ID NO: 254, a homolog or an ortholog of a different organism species, and the like. The hsa-miR-4268 gene can be obtained by a method described in Goff LA et al., 2009, PLoS One, Vol. 4, e7192. Also, "hsa-mir-4268" (miRBase Accession No. MI0015874; SEQ ID NO: 648) having a hairpin-like structure is known as a precursor of "hsa-miR-4268."

The term "hsa-miR-4279 gene" or "hsa-miR-4279" used herein includes the hsa-miR-4279 gene (miRBase Accession No. MIMAT0016909) shown in SEQ ID NO: 255, a homolog or an ortholog of a different organism species, and the like. The hsa-miR-4279 gene can be obtained by a method described in Witten D et al., 2010, BMC Biol., Vol. 8, p. 58. Also, "hsa-mir-4279" (miRBase Accession No. MI0015887; SEQ ID NO: 649) having a hairpin-like structure is known as a precursor of "hsa-miR-4279."

The term "hsa-miR-3620-3p gene" or "hsa-miR-3620-3p" used herein includes the hsa-miR-3620-3p gene (miRBase Accession No. MIMAT0018001) shown in SEQ ID NO: 256, a homolog or an ortholog of a different organism species, and the like. The hsa-miR-3620-3p gene can be obtained by a method described in Witten D et al., 2010, BMC Biol., Vol. 8, p. 58. Also, "hsa-mir-3620" (miRBase Accession No. MI0016011; SEQ ID NO: 650) having a hairpin-like structure is known as a precursor of "hsa-miR-3620-3p."

The term "hsa-miR-3944-3p gene" or "hsa-miR-3944-3p" used herein includes the hsa-miR-3944-3p gene (miRBase Accession No. MIMAT0018360) shown in SEQ ID NO: 257, a homolog or an ortholog of a different organism species, and the like. The hsa-miR-3944-3p gene can be obtained by a method described in Liao JY et al., 2010, PLoS One, Vol. 5, e10563. Also, "hsa-mir-3944" (miRBase Accession No. MI0016601; SEQ ID NO: 651) having a hairpin-like structure is known as a precursor of "hsa-miR-3944-3p."

The term "hsa-miR-3156-3p gene" or "hsa-miR-3156-3p" used herein includes the hsa-miR-3156-3p gene (miRBase Accession No. MIMAT0019209) shown in SEQ ID NO: 258, a homolog or an ortholog of a different organism species, and the like. The hsa-miR-3156-3p gene can be obtained by a method described in Stark MS et al., 2010, PLoS One, Vol. 5, e9685. Also, "hsa-mir-3156-1 and hsa-mir-3156-2" (miRBase Accession Nos. MI0014184 and MI0014230; SEQ ID NOs: 652 and 653) each having a hairpin-like structure are known as precursors of "hsa-miR-3156-3p."

The term "hsa-miR-3187-5p gene" or "hsa-miR-3187-5p" used herein includes the hsa-miR-3187-5p gene (miRBase Accession No. MIMAT0019216) shown in SEQ ID NO: 259, a homolog or an ortholog of a different organism species, and the like. The hsa-miR-3187-5p gene can be obtained by a method described in Stark MS et al., 2010, PLoS One, Vol. 5, e9685. Also, "hsa-mir-3187" (miRBase Accession No. MI0014231; SEQ ID NO: 654) having a hairpin-like structure is known as a precursor of "hsa-miR-3187-5p."

The term "hsa-miR-4685-3p gene" or "hsa-miR-4685-3p" used herein includes the hsa-miR-4685-3p gene (miRBase Accession No. MIMAT0019772) shown in SEQ ID NO: 260, a homolog or an ortholog of a different organism species, and the like. The hsa-miR-4685-3p gene can be obtained by a method described in Persson H et al., 2011, Cancer Res., Vol. 71, pp. 78-86. Also, "hsa-mir-4685" (miRBase Accession No. MI0017317; SEQ ID NO: 655) having a hairpin-like structure is known as a precursor of "hsa-miR-4685-3p."

The term "hsa-miR-4695-3p gene" or "hsa-miR-4695-3p" used herein includes the hsa-miR-4695-3p gene (miRBase Accession No. MIMAT0019789) shown in SEQ ID NO: 261, a homolog or an ortholog of a different organism species, and the like. The hsa-miR-4695-3p gene can be obtained by a method described in Persson H et al., 2011, Cancer Res., Vol. 71, pp. 78-86. Also, "hsa-mir-4695" (miRBase Accession No. MI0017328; SEQ ID NO: 656) having a hairpin-like structure is known as a precursor of "hsa-miR-4695-3p."

The term "hsa-miR-4697-3p gene" or "hsa-miR-4697-3p" used herein includes the hsa-miR-4697-3p gene (miRBase Accession No. MIMAT0019792) shown in SEQ ID NO: 262, a homolog or an ortholog of a different organism species, and the like. The hsa-miR-4697-3p gene can be obtained by a method described in Persson H et al., 2011, Cancer Res., Vol. 71, pp. 78-86. Also, "hsa-mir-4697" (miRBase Accession No. MI0017330; SEQ ID NO: 657) having a hairpin-like structure is known as a precursor of "hsa-miR-4697-3p."

The term "hsa-miR-4713-5p gene" or "hsa-miR-4713-5p" used herein includes the hsa-miR-4713-5p gene (miRBase Accession No. MIMAT0019820) shown in SEQ ID NO: 263, a homolog or an ortholog of a different organism species, and the like. The hsa-miR-4713-5p gene can be obtained by a method described in Persson H et al., 2011, Cancer Res., Vol. 71, pp. 78-86. Also, "hsa-mir-4713" (miRBase Accession No. MI0017347; SEQ ID NO: 658) having a hairpin-like structure is known as a precursor of "hsa-miR-4713-5p."

The term "hsa-miR-4723-3p gene" or "hsa-miR-4723-3p" used herein includes the hsa-miR-4723-3p gene (miRBase Accession No. MIMAT0019839) shown in SEQ ID NO: 264, a homolog or an ortholog of a different organism species, and the like. The hsa-miR-4723-3p gene can be obtained by a method described in Persson H et al., 2011, Cancer Res., Vol. 71, pp. 78-86. Also, "hsa-mir-4723" (miRBase Accession No. MI0017359; SEQ ID NO: 659) having a hairpin-like structure is known as a precursor of "hsa-miR-4723-3p."

The term "hsa-miR-371b-3p gene" or "hsa-miR-371b-3p" used herein includes the hsa-miR-371b-3p gene (miRBase Accession No. MIMAT0019893) shown in SEQ ID NO: 265, a homolog or an ortholog of a different organism species, and the like. The hsa-miR-371b-3p gene can be obtained by a method described in Persson H et al., 2011, Cancer Res., Vol. 71, pp. 78-86. Also, "hsa-mir-371b" (miRBase Accession No. MI0017393; SEQ ID NO: 660) having a hairpin-like structure is known as a precursor of "hsa-miR-371b-3p."

The term "hsa-miR-3151-3p gene" or "hsa-miR-3151-3p" used herein includes the hsa-miR-3151-3p gene (miRBase Accession No. MIMAT0027026) shown in SEQ ID NO: 266, a homolog or an ortholog of a different organism species, and the like. The hsa-miR-3151-3p gene can be obtained by a method described in Stark MS et al., 2010, PLoS One, Vol. 5, e9685. Also, "hsa-mir-3151" (miRBase Accession No. MI0014178; SEQ ID NO: 661) having a hairpin-like structure is known as a precursor of "hsa-miR-3151-3p."

The term "hsa-miR-3192-3p gene" or "hsa-miR-3192-3p" used herein includes the hsa-miR-3192-3p gene (miRBase Accession No. MIMAT0027027) shown in SEQ ID NO: 267, a homolog or an ortholog of a different organism species, and the like. The hsa-miR-3192-3p gene can be obtained by a method described in Stark MS et al., 2010, PLoS One, Vol. 5, e9685. Also, "hsa-mir-3192" (miRBase Accession No. MI0014237; SEQ ID NO: 662) having a hairpin-like structure is known as a precursor of "hsa-miR-3192-3p."

The term "hsa-miR-6728-3p gene" or "hsa-miR-6728-3p" used herein includes the hsa-miR-6728-3p gene (miRBase Accession No. MIMAT0027358) shown in SEQ ID NO: 268, a homolog or an ortholog of a different organism species, and the like. The hsa-miR-6728-3p gene can be obtained by a method described in Ladewig E et al., 2012, Genome Res., Vol. 22, pp. 1634-1645. Also, "hsa-mir-6728" (miRBase Accession No. MI0022573; SEQ ID NO: 663) having a hairpin-like structure is known as a precursor of "hsa-miR-6728-3p."

The term "hsa-miR-6736-3p gene" or "hsa-miR-6736-3p" used herein includes the hsa-miR-6736-3p gene (miRBase Accession No. MIMAT0027374) shown in SEQ ID NO: 269, a homolog or an ortholog of a different organism species, and the like. The hsa-miR-6736-3p gene can be obtained by a method described in Ladewig E et al., 2012, Genome Res., Vol. 22, pp. 1634-1645. Also, "hsa-mir-6736" (miRBase Accession No. MI0022581; SEQ ID NO: 664) having a hairpin-like structure is known as a precursor of "hsa-miR-6736-3p."

The term "hsa-miR-6740-3p gene" or "hsa-miR-6740-3p" used herein includes the hsa-miR-6740-3p gene (miRBase Accession No. MIMAT0027382) shown in SEQ ID NO: 270, a homolog or an ortholog of a different organism species, and the like. The hsa-miR-6740-3p gene can be obtained by a method described in Ladewig E et al., 2012, Genome Res., Vol. 22, pp. 1634-1645. Also, "hsa-mir-6740" (miRBase Accession No. MI0022585; SEQ ID NO: 665) having a hairpin-like structure is known as a precursor of "hsa-miR-6740-3p."

The term "hsa-miR-6741-3p gene" or "hsa-miR-6741-3p" used herein includes the hsa-miR-6741-3p gene (miRBase Accession No. MIMAT0027384) shown in SEQ ID NO: 271, a homolog or an ortholog of a different organism species, and the like. The hsa-miR-6741-3p gene can be obtained by a method described in Ladewig E et al., 2012, Genome Res., Vol. 22, pp. 1634-1645. Also, "hsa-mir-6741" (miRBase Accession No. MI0022586; SEQ ID NO: 666) having a hairpin-like structure is known as a precursor of "hsa-miR-6741-3p."

The term "hsa-miR-6743-3p gene" or "hsa-miR-6743-3p" used herein includes the hsa-miR-6743-3p gene (miRBase Accession No. MIMAT0027388) shown in SEQ ID NO: 272, a homolog or an ortholog of a different organism species, and the like. The hsa-miR-6743-3p gene can be obtained by a method described in Ladewig E et al., 2012, Genome Res., Vol. 22, pp. 1634-1645. Also, "hsa-mir-6743" (miRBase Accession No. MI0022588; SEQ ID NO: 599) having a hairpin-like structure is known as a precursor of "hsa-miR-6743-3p."

The term "hsa-miR-6747-3p gene" or "hsa-miR-6747-3p" used herein includes the hsa-miR-6747-3p gene (miRBase Accession No. MIMAT0027395) shown in SEQ ID NO: 273, a homolog or an ortholog of a different organism species, and the like. The hsa-miR-6747-3p gene can be obtained by a method described in Ladewig E et al., 2012, Genome Res., Vol. 22, pp. 1634-1645. Also, "hsa-mir-6747" (miRBase Accession No. MI0022592; SEQ ID NO: 667) having a hairpin-like structure is known as a precursor of "hsa-miR-6747-3p."

The term "hsa-miR-6750-3p gene" or "hsa-miR-6750-3p" used herein includes the hsa-miR-6750-3p gene (miRBase Accession No. MIMAT0027401) shown in SEQ ID NO: 274, a homolog or an ortholog of a different organism species, and the like. The hsa-miR-6750-3p gene can be obtained by a method described in Ladewig E et al., 2012, Genome Res., Vol. 22, pp. 1634-1645. Also, "hsa-mir-6750" (miRBase Accession No. MI0022595; SEQ ID NO: 668) having a hairpin-like structure is known as a precursor of "hsa-miR-6747-3p."

The term "hsa-miR-6754-3p gene" or "hsa-miR-6754-3p" used herein includes the hsa-miR-6754-3p gene (miRBase Accession No. MIMAT0027409) shown in SEQ ID NO: 275, a homolog or an ortholog of a different organism species, and the like. The hsa-miR-6754-3p gene can be obtained by a method described in Ladewig E et al., 2012, Genome Res., Vol. 22, pp. 1634-1645. Also, "hsa-mir-6754" (miRBase Accession No. MI0022599; SEQ ID NO: 669) having a hairpin-like structure is known as a precursor of "hsa-miR-6754-3p."

The term "hsa-miR-6759-3p gene" or "hsa-miR-6759-3p" used herein includes the hsa-miR-6759-3p gene (miRBase Accession No. MIMAT0027419) shown in SEQ ID NO: 276, a homolog or an ortholog of a different organism species, and the like. The hsa-miR-6759-3p gene can be obtained by a method described in Ladewig E et al., 2012, Genome Res., Vol. 22, pp. 1634-1645. Also, "hsa-mir-6759" (miRBase Accession No. MI0022604; SEQ ID NO: 670) having a hairpin-like structure is known as a precursor of "hsa-miR-6759-3p."

The term "hsa-miR-6761-3p gene" or "hsa-miR-6761-3p" used herein includes the hsa-miR-6761-3p gene (miRBase Accession No. MIMAT0027423) shown in SEQ ID NO: 277, a homolog or an ortholog of a different organism species, and the like. The hsa-miR-6761-3p gene can be obtained by a method described in Ladewig E et al., 2012, Genome Res., Vol. 22, pp. 1634-1645. Also, "hsa-mir-6761" (miRBase Accession No. MI0022606; SEQ ID NO: 671) having a hairpin-like structure is known as a precursor of "hsa-miR-6761-3p."

The term "hsa-miR-6762-3p gene" or "hsa-miR-6762-3p" used herein includes the hsa-miR-6762-3p gene (miRBase Accession No. MIMAT0027425) shown in SEQ ID NO: 278, a homolog or an ortholog of a different organism species, and the like. The hsa-miR-6762-3p gene can be obtained by a method described in Ladewig E et al., 2012, Genome Res., Vol. 22, pp. 1634-1645. Also, "hsa-mir-6762" (miRBase Accession No. MI0022607; SEQ ID NO: 672) having a hairpin-like structure is known as a precursor of "hsa-miR-6762-3p."

The term "hsa-miR-6769a-3p gene" or "hsa-miR-6769a-3p" used herein includes the hsa-miR-6769a-3p gene (miRBase Accession No. MIMAT0027439) shown in SEQ ID NO: 279, a homolog or an ortholog of a different organism species, and the like. The hsa-miR-6769a-3p gene can be obtained by a method described in Ladewig E et al., 2012, Genome Res., Vol. 22, pp. 1634-1645. Also, "hsa-mir-6769a" (miRBase Accession No. MI0022614; SEQ ID NO: 673) having a hairpin-like structure is known as a precursor of "hsa-miR-6769a-3p."

The term "hsa-miR-6776-3p gene" or "hsa-miR-6776-3p" used herein includes the hsa-miR-6776-3p gene (miRBase Accession No. MIMAT0027453) shown in SEQ ID NO: 280, a homolog or an ortholog of a different organism species, and the like. The hsa-miR-6776-3p gene can be obtained by a method described in Ladewig E et al., 2012, Genome Res., Vol. 22, pp. 1634-1645. Also, "hsa-mir-6776" (miRBase Accession No. MI0022621; SEQ ID NO: 674) having a hairpin-like structure is known as a precursor of "hsa-miR-6776-3p."

The term "hsa-miR-6778-3p gene" or "hsa-miR-6778-3p" used herein includes the hsa-miR-6778-3p gene (miRBase Accession No. MIMAT0027457) shown in SEQ ID NO: 281, a homolog or an ortholog of a different organism species, and the like. The hsa-miR-6778-3p gene can be obtained by a method described in Ladewig E et al., 2012, Genome Res., Vol. 22, pp. 1634-1645. Also, "hsa-mir-6778" (miRBase Accession No. MI0022623; SEQ ID NO: 675) having a hairpin-like structure is known as a precursor of "hsa-miR-6778-3p."

The term "hsa-miR-6779-3p gene" or "hsa-miR-6779-3p" used herein includes the hsa-miR-6779-3p gene (miRBase Accession No. MIMAT0027459) shown in SEQ ID NO: 282, a homolog or an ortholog of a different organism species, and the like. The hsa-miR-6779-3p gene can be obtained by a method described in Ladewig E et al., 2012, Genome Res., Vol. 22, pp. 1634-1645. Also, "hsa-mir-6779" (miRBase Accession No. MI0022624; SEQ ID NO: 676) having a hairpin-like structure is known as a precursor of "hsa-miR-6779-3p."

The term "hsa-miR-6786-3p gene" or "hsa-miR-6786-3p" used herein includes the hsa-miR-6786-3p gene (miRBase Accession No. MIMAT0027473) shown in SEQ ID NO: 283, a homolog or an ortholog of a different organism species, and the like. The hsa-miR-6786-3p gene can be obtained by a method described in Ladewig E et al., 2012, Genome Res., Vol. 22, pp. 1634-1645. Also, "hsa-mir-6786" (miRBase Accession No. MI0022631; SEQ ID NO: 539) having a hairpin-like structure is known as a precursor of "hsa-miR-6786-3p."

The term "hsa-miR-6787-3p gene" or "hsa-miR-6787-3p" used herein includes the hsa-miR-6787-3p gene (miRBase Accession No. MIMAT0027475) shown in SEQ ID NO: 284, a homolog or an ortholog of a different organism species, and the like. The hsa-miR-6787-3p gene can be obtained by a method described in Ladewig E et al., 2012, Genome Res., Vol. 22, pp. 1634-1645. Also, "hsa-mir-6787" (miRBase Accession No. MI0022632; SEQ ID NO: 677) having a hairpin-like structure is known as a precursor of "hsa-miR-6787-3p."

The term "hsa-miR-6792-3p gene" or "hsa-miR-6792-3p" used herein includes the hsa-miR-6792-3p gene (miRBase Accession No. MIMAT0027485) shown in SEQ ID NO: 285, a homolog or an ortholog of a different organism species, and the like. The hsa-miR-6792-3p gene can be obtained by a method described in Ladewig E et al., 2012, Genome Res., Vol. 22, pp. 1634-1645. Also, "hsa-mir-6792" (miRBase Accession No. MI0022637; SEQ ID NO: 678) having a hairpin-like structure is known as a precursor of "hsa-miR-6792-3p."

The term "hsa-miR-6794-3p gene" or "hsa-miR-6794-3p" used herein includes the hsa-miR-6794-3p gene (miRBase Accession No. MIMAT0027489) shown in SEQ ID NO: 286, a homolog or an ortholog of a different organism species, and the like. The hsa-miR-6794-3p gene can be obtained by a method described in Ladewig E et al., 2012, Genome Res., Vol. 22, pp. 1634-1645. Also, "hsa-mir-6794" (miRBase Accession No. MI0022639; SEQ ID NO: 679) having a hairpin-like structure is known as a precursor of "hsa-miR-6794-3p."

The term "hsa-miR-6801-3p gene" or "hsa-miR-6801-3p" used herein includes the hsa-miR-6801-3p gene (miRBase Accession No. MIMAT0027503) shown in SEQ ID NO: 287, a homolog or an ortholog of a different organism species, and the like. The hsa-miR-6801-3p gene can be obtained by a method described in Ladewig E et al., 2012, Genome Res., Vol. 22, pp. 1634-1645. Also, "hsa-mir-6801" (miRBase Accession No. MI0022646; SEQ ID NO: 680) having a hairpin-like structure is known as a precursor of "hsa-miR-6801-3p."

The term "hsa-miR-6802-3p gene" or "hsa-miR-6802-3p" used herein includes the hsa-miR-6802-3p gene (miRBase Accession No. MIMAT0027505) shown in SEQ ID NO: 288, a homolog or an ortholog of a different organism species, and the like. The hsa-miR-6802-3p gene can be obtained by a method described in Ladewig E et al., 2012, Genome Res., Vol. 22, pp. 1634-1645. Also, "hsa-mir-6802" (miRBase Accession No. MI0022647; SEQ ID NO: 681) having a hairpin-like structure is known as a precursor of "hsa-miR-6802-3p."

The term "hsa-miR-6803-3p gene" or "hsa-miR-6803-3p" used herein includes the hsa-miR-6803-3p gene (miRBase Accession No. MIMAT0027507) shown in SEQ ID NO: 289, a homolog or an ortholog of a different organism species, and the like. The hsa-miR-6803-3p gene can be obtained by a method described in Ladewig E et al., 2012, Genome Res., Vol. 22, pp. 1634-1645. Also, "hsa-mir-6803" (miRBase Accession No. MI0022648; SEQ ID NO: 682) having a hairpin-like structure is known as a precursor of "hsa-miR-6803-3p."

The term "hsa-miR-6804-3p gene" or "hsa-miR-6804-3p" used herein includes the hsa-miR-6804-3p gene (miRBase Accession No. MIMAT0027509) shown in SEQ ID NO: 290, a homolog or an ortholog of a different organism species, and the like. The hsa-miR-6804-3p gene can be obtained by a method described in Ladewig E et al., 2012, Genome Res., Vol. 22, pp. 1634-1645. Also, "hsa-mir-6804" (miRBase Accession No. MI0022649; SEQ ID NO: 683) having a hairpin-like structure is known as a precursor of "hsa-miR-6804-3p."

The term "hsa-miR-6810-5p gene" or "hsa-miR-6810-5p" used herein includes the hsa-miR-6810-5p gene (miRBase Accession No. MIMAT0027520) shown in SEQ ID NO: 291, a homolog or an ortholog of a different organism species, and the like. The hsa-miR-6810-5p gene can be obtained by a method described in Ladewig E et al., 2012, Genome Res., Vol. 22, pp. 1634-1645. Also, "hsa-mir-6810" (miRBase Accession No. MI0022655; SEQ ID NO: 684) having a hairpin-like structure is known as a precursor of "hsa-miR-6810-5p."

The term "hsa-miR-6823-3p gene" or "hsa-miR-6823-3p" used herein includes the hsa-miR-6823-3p gene (miRBase Accession No. MIMAT0027547) shown in SEQ ID NO: 292, a homolog or an ortholog of a different organism species, and the like. The hsa-miR-6823-3p gene can be obtained by a method described in Ladewig E et al., 2012, Genome Res., Vol. 22, pp. 1634-1645. Also, "hsa-mir-6823" (miRBase Accession No. MI0022668; SEQ ID NO: 550) having a hairpin-like structure is known as a precursor of "hsa-miR-6823-3p."

The term "hsa-miR-6825-3p gene" or "hsa-miR-6825-3p" used herein includes the hsa-miR-6825-3p gene (miRBase Accession No. MIMAT0027551) shown in SEQ ID NO: 293, a homolog or an ortholog of a different organism species, and the like. The hsa-miR-6825-3p gene can be obtained by a method described in Ladewig E et al., 2012, Genome Res., Vol. 22, pp. 1634-1645. Also, "hsa-mir-6825" (miRBase Accession No. MI0022670; SEQ ID NO: 685) having a hairpin-like structure is known as a precursor of "hsa-miR-6825-3p."

The term "hsa-miR-6829-3p gene" or "hsa-miR-6829-3p" used herein includes the hsa-miR-6829-3p gene (miRBase Accession No. MIMAT0027559) shown in SEQ ID NO: 294, a homolog or an ortholog of a different organism species, and the like. The hsa-miR-6829-3p gene can be obtained by a method described in Ladewig E et al., 2012, Genome Res., Vol. 22, pp. 1634-1645. Also, "hsa-mir-6829" (miRBase Accession No. MI0022674; SEQ ID NO: 686) having a hairpin-like structure is known as a precursor of "hsa-miR-6829-3p."

The term "hsa-miR-6833-3p gene" or "hsa-miR-6833-3p" used herein includes the hsa-miR-6833-3p gene (miRBase Accession No. MIMAT0027567) shown in SEQ ID NO: 295, a homolog or an ortholog of a different organism species, and the like. The hsa-miR-6833-3p gene can be obtained by a method described in Ladewig E et al., 2012, Genome Res., Vol. 22, pp. 1634-1645. Also, "hsa-mir-6833" (miRBase Accession No. MI0022678; SEQ ID NO: 687) having a hairpin-like structure is known as a precursor of "hsa-miR-6833-3p."

The term "hsa-miR-6834-3p gene" or "hsa-miR-6834-3p" used herein includes the hsa-miR-6834-3p gene (miRBase Accession No. MIMAT0027569) shown in SEQ ID NO: 296, a homolog or an ortholog of a different organism species, and the like. The hsa-miR-6834-3p gene can be obtained by a method described in Ladewig E et al., 2012, Genome Res., Vol. 22, pp. 1634-1645. Also, "hsa-mir-6834" (miRBase Accession No. MI0022679; SEQ ID NO: 688) having a hairpin-like structure is known as a precursor of "hsa-miR-6834-3p."

The term "hsa-miR-6780b-3p gene" or "hsa-miR-6780b-3p" used herein includes the hsa-miR-6780b-3p gene (miRBase Accession No. MIMAT0027573) shown in SEQ ID NO: 297, a homolog or an ortholog of a different organism species, and the like. The hsa-miR-6780b-3p gene can be obtained by a method described in Ladewig E et al., 2012, Genome Res., Vol. 22, pp. 1634-1645. Also, "hsa-mir-6780b" (miRBase Accession No. MI0022681; SEQ ID NO: 689) having a hairpin-like structure is known as a precursor of "hsa-miR-6780b-3p."

The term "hsa-miR-6845-3p gene" or "hsa-miR-6845-3p" used herein includes the hsa-miR-6845-3p gene (miRBase Accession No. MIMAT0027591) shown in SEQ ID NO: 298, a homolog or an ortholog of a different organism species, and the like. The hsa-miR-6845-3p gene can be obtained by a method described in Ladewig E et al., 2012, Genome Res., Vol. 22, pp. 1634-1645. Also, "hsa-mir-6845" (miRBase Accession No. MI0022691; SEQ ID NO: 690) having a hairpin-like structure is known as a precursor of "hsa-miR-6845-3p."

The term "hsa-miR-6862-3p gene" or "hsa-miR-6862-3p" used herein includes the hsa-miR-6862-3p gene (miRBase Accession No. MIMAT0027626) shown in SEQ ID NO: 299, a homolog or an ortholog of a different organism species, and the like. The hsa-miR-6862-3p gene can be obtained by a method described in Ladewig E et al., 2012, Genome Res., Vol. 22, pp. 1634-1645. Also, "hsa-mir-6862-1 and hsa-mir-6862-2" (miRBase Accession Nos. MI0022709 and M10026415; SEQ ID NOs: 691 and 692) each having a hairpin-like structure are known as precursors of "hsa-miR-6862-3p."

The term "hsa-miR-6865-3p gene" or "hsa-miR-6865-3p" used herein includes the hsa-miR-6865-3p gene (miRBase Accession No. MIMAT0027631) shown in SEQ ID NO: 300, a homolog or an ortholog of a different organism species, and the like. The hsa-miR-6865-3p gene can be obtained by a method described in Ladewig E et al., 2012, Genome Res., Vol. 22, pp. 1634-1645. Also, "hsa-mir-6865" (miRBase Accession No. MI0022712; SEQ ID NO: 693) having a hairpin-like structure is known as a precursor of "hsa-miR-6865-3p."

The term "hsa-miR-6870-3p gene" or "hsa-miR-6870-3p" used herein includes the hsa-miR-6870-3p gene (miRBase Accession No. MIMAT0027641) shown in SEQ ID NO: 301, a homolog or an ortholog of a different organism species, and the like. The hsa-miR-6870-3p gene can be obtained by a method described in Ladewig E et al., 2012, Genome Res., Vol. 22, pp. 1634-1645. Also, "hsa-mir-6870" (miRBase Accession No. MI0022717; SEQ ID NO: 694) having a hairpin-like structure is known as a precursor of "hsa-miR-6870-3p."

The term "hsa-miR-6875-3p gene" or "hsa-miR-6875-3p" used herein includes the hsa-miR-6875-3p gene (miRBase Accession No. MIMAT0027651) shown in SEQ ID NO: 302, a homolog or an ortholog of a different organism species, and the like. The hsa-miR-6875-3p gene can be obtained by a method described in Ladewig E et al., 2012, Genome Res., Vol. 22, pp. 1634-1645. Also, "hsa-mir-6875" (miRBase Accession No. MI0022722; SEQ ID NO: 695) having a hairpin-like structure is known as a precursor of "hsa-miR-6875-3p."

The term "hsa-miR-6877-3p gene" or "hsa-miR-6877-3p" used herein includes the hsa-miR-6877-3p gene (miRBase Accession No. MIMAT0027655) shown in SEQ ID NO: 303, a homolog or an ortholog of a different organism species, and the like. The hsa-miR-6877-3p gene can be obtained by a method described in Ladewig E et al., 2012, Genome Res., Vol. 22, pp. 1634-1645. Also, "hsa-mir-6877" (miRBase Accession No. MI0022724; SEQ ID NO: 696) having a hairpin-like structure is known as a precursor of "hsa-miR-6877-3p."

The term "hsa-miR-6879-3p gene" or "hsa-miR-6879-3p" used herein includes the hsa-miR-6879-3p gene (miRBase Accession No. MIMAT0027659) shown in SEQ ID NO: 304, a homolog or an ortholog of a different organism species, and the like. The hsa-miR-6879-3p gene can be obtained by a method described in Ladewig E et al., 2012, Genome Res., Vol. 22, pp. 1634-1645. Also, "hsa-mir-6879" (miRBase Accession No. MI0022726; SEQ ID NO: 697) having a hairpin-like structure is known as a precursor of "hsa-miR-6879-3p."

The term "hsa-miR-6882-3p gene" or "hsa-miR-6882-3p" used herein includes the hsa-miR-6882-3p gene (miRBase Accession No. MIMAT0027665) shown in SEQ ID NO: 305, a homolog or an ortholog of a different organism species, and the like. The hsa-miR-6882-3p gene can be obtained by a method described in Ladewig E et al., 2012, Genome Res., Vol. 22, pp. 1634-1645. Also, "hsa-mir-6882" (miRBase Accession No. MI0022729; SEQ ID NO: 698) having a hairpin-like structure is known as a precursor of "hsa-miR-6882-3p."

The term "hsa-miR-6885-3p gene" or "hsa-miR-6885-3p" used herein includes the hsa-miR-6885-3p gene (miRBase Accession No. MIMAT0027671) shown in SEQ ID NO: 306, a homolog or an ortholog of a different organism species, and the like. The hsa-miR-6885-3p gene can be obtained by a method described in Ladewig E et al., 2012, Genome Res., Vol. 22, pp. 1634-1645. Also, "hsa-mir-6885" (miRBase Accession No. MI0022732; SEQ ID NO: 699) having a hairpin-like structure is known as a precursor of "hsa-miR-6885-3p."

The term "hsa-miR-6886-3p gene" or "hsa-miR-6886-3p" used herein includes the hsa-miR-6886-3p gene (miRBase Accession No. MIMAT0027673) shown in SEQ ID NO: 307, a homolog or an ortholog of a different organism species, and the like. The hsa-miR-6886-3p gene can be obtained by a method described in Ladewig E et al., 2012, Genome Res., Vol. 22, pp. 1634-1645. Also, "hsa-mir-6886" (miRBase Accession No. MI0022733; SEQ ID NO: 700) having a hairpin-like structure is known as a precursor of "hsa-miR-6886-3p."

The term "hsa-miR-6887-3p gene" or "hsa-miR-6887-3p" used herein includes the hsa-miR-6887-3p gene (miRBase Accession No. MIMAT0027675) shown in SEQ ID NO: 308, a homolog or an ortholog of a different organism species, and the like. The hsa-miR-6887-3p gene can be obtained by a method described in Ladewig E et al., 2012, Genome Res., Vol. 22, pp. 1634-1645. Also, "hsa-mir-6887" (miRBase Accession No. MI0022734; SEQ ID NO: 701) having a hairpin-like structure is known as a precursor of "hsa-miR-6887-3p."

The term "hsa-miR-6890-3p gene" or "hsa-miR-6890-3p" used herein includes the hsa-miR-6890-3p gene (miRBase Accession No. MIMAT0027681) shown in SEQ ID NO: 309, a homolog or an ortholog of a different organism species, and the like. The hsa-miR-6890-3p gene can be obtained by a method described in Ladewig E et al., 2012, Genome Res., Vol. 22, pp. 1634-1645. Also, "hsa-mir-6890" (miRBase Accession No. MI0022737; SEQ ID NO: 702) having a hairpin-like structure is known as a precursor of "hsa-miR-6890-3p."

The term "hsa-miR-6893-3p gene" or "hsa-miR-6893-3p" used herein includes the hsa-miR-6893-3p gene (miRBase Accession No. MIMAT0027687) shown in SEQ ID NO: 310, a homolog or an ortholog of a different organism species, and the like. The hsa-miR-6893-3p gene can be obtained by a method described in Ladewig E et al., 2012, Genome Res., Vol. 22, pp. 1634-1645. Also, "hsa-mir-6893" (miRBase Accession No. MI0022740; SEQ ID NO: 703) having a hairpin-like structure is known as a precursor of "hsa-miR-6893-3p."

The term "hsa-miR-6894-3p gene" or "hsa-miR-6894-3p" used herein includes the hsa-miR-6894-3p gene (miRBase Accession No. MIMAT0027689) shown in SEQ ID NO: 311, a homolog or an ortholog of a different organism species, and the like. The hsa-miR-6894-3p gene can be obtained by a method described in Ladewig E et al., 2012, Genome Res., Vol. 22, pp. 1634-1645. Also, "hsa-mir-6894" (miRBase Accession No. MI0022741; SEQ ID NO: 704) having a hairpin-like structure is known as a precursor of "hsa-miR-6894-3p."

The term "hsa-miR-7106-3p gene" or "hsa-miR-7106-3p" used herein includes the hsa-miR-7106-3p gene (miRBase Accession No. MIMAT0028110) shown in SEQ ID NO: 312, a homolog or an ortholog of a different organism species, and the like. The hsa-miR-7106-3p gene can be obtained by a method described in Ladewig E et al., 2012, Genome Res., Vol. 22, pp. 1634-1645. Also, "hsa-mir-7106" (miRBase Accession No. MI0022957; SEQ ID NO: 705) having a hairpin-like structure is known as a precursor of "hsa-miR-7106-3p."

The term "hsa-miR-7109-3p gene" or "hsa-miR-7109-3p" used herein includes the hsa-miR-7109-3p gene (miRBase Accession No. MIMAT0028116) shown in SEQ ID NO: 313, a homolog or an ortholog of a different organism species, and the like. The hsa-miR-7109-3p gene can be obtained by a method described in Ladewig E et al., 2012, Genome Res., Vol. 22, pp. 1634-1645. Also, "hsa-mir-7109" (miRBase Accession No. MI0022960; SEQ ID NO: 706) having a hairpin-like structure is known as a precursor of "hsa-miR-7109-3p."

The term "hsa-miR-7114-3p gene" or "hsa-miR-7114-3p" used herein includes the hsa-miR-7114-3p gene (miRBase Accession No. MIMAT0028126) shown in SEQ ID NO: 314, a homolog or an ortholog of a different organism species, and the like. The hsa-miR-7114-3p gene can be obtained by a method described in Ladewig E et al., 2012, Genome Res., Vol. 22, pp. 1634-1645. Also, "hsa-mir-7114" (miRBase Accession No. MI0022965; SEQ ID NO: 707) having a hairpin-like structure is known as a precursor of "hsa-miR-7114-3p."

The term "hsa-miR-7155-5p gene" or "hsa-miR-7155-5p" used herein includes the hsa-miR-7155-5p gene (miRBase Accession No. MIMAT0028220) shown in SEQ ID NO: 315, a homolog or an ortholog of a different organism species, and the like. The hsa-miR-7155-5p gene can be obtained by a method described in Meunier J et al., 2013, Genome Res., Vol. 23, pp. 34-45. Also, "hsa-mir-7155" (miRBase Accession No. MI0023615; SEQ ID NO: 708) having a hairpin-like structure is known as a precursor of "hsa-miR-7155-5p."

The term "hsa-miR-7160-5p gene" or "hsa-miR-7160-5p" used herein includes the hsa-miR-7160-5p gene (miRBase Accession No. MIMAT0028230) shown in SEQ ID NO: 316, a homolog or an ortholog of a different organism species, and the like. The hsa-miR-7160-5p gene can be obtained by a method described in Meunier J et al., 2013, Genome Res., Vol. 23, pp. 34-45. Also, "hsa-mir-7160" (miRBase Accession No. MI0023621; SEQ ID NO: 709) having a hairpin-like structure is known as a precursor of "hsa-miR-7160-5p."

The term "hsa-miR-615-3p gene" or "hsa-miR-615-3p" used herein includes the hsa-miR-615-3p gene (miRBase Accession No. MIMAT0003283) shown in SEQ ID NO: 317, a homolog or an ortholog of a different organism species, and the like. The hsa-miR-615-3p gene can be obtained by a method described in Cummins JM et al., 2006, Proc. Natl. Acad. Sci. U.S.A., Vol. 103, pp. 3687-3692. Also, "hsa-mir-615" (miRBase Accession No. MI0003628; SEQ ID NO: 710) having a hairpin-like structure is known as a precursor of "hsa-miR-615-3p."

The term "hsa-miR-920 gene" or "hsa-miR-920" used herein includes the hsa-miR-920 gene (miRBase Accession No. MIMAT0004970) shown in SEQ ID NO: 318, a homolog or an ortholog of a different organism species, and the like. The hsa-miR-920 gene can be obtained by a method described in Novotny GW et al., 2007, Int. J. Androl., Vol. 30, pp. 316-326. Also, "hsa-mir-920" (miRBase Accession No. MI0005712; SEQ ID NO: 711) having a hairpin-like structure is known as a precursor of "hsa-miR-920."

The term "hsa-miR-1825 gene" or "hsa-miR-1825" used herein includes the hsa-miR-1825 gene (miRBase Accession No. MIMAT0006765) shown in SEQ ID NO: 319, a homolog or an ortholog of a different organism species, and the like. The hsa-miR-1825 gene can be obtained by a method described in Friedlander MR et al., 2008, Nat. Biotechnol., Vol. 26, pp. 407-415. Also, "hsa-mir-1825" (miRBase Accession No. MI0008193; SEQ ID NO: 712) having a hairpin-like structure is known as a precursor of "hsa-miR-1825."

The term "hsa-miR-675-3p gene" or "hsa-miR-675-3p" used herein includes the hsa-miR-675-3p gene (miRBase Accession No. MIMAT0006790) shown in SEQ ID NO: 320, a homolog or an ortholog of a different organism species, and the like. The hsa-miR-675-3p gene can be obtained by a method described in Cai X et al., 2007, RNA, Vol. 13, pp. 313-316. Also, "hsa-mir-675" (miRBase Accession No. MI0005416; SEQ ID NO: 713) having a hairpin-like structure is known as a precursor of "hsa-miR-675-3p."

The term "hsa-miR-1910-5p gene" or "hsa-miR-1910-5p" used herein includes the hsa-miR-1910-5p gene (miRBase Accession No. MIMAT0007884) shown in SEQ ID NO: 321, a homolog or an ortholog of a different organism species, and the like. The hsa-miR-1910-5p gene can be obtained by a method described in Bar M et al., 2008, Stem Cells, Vol. 26, pp. 2496-2505. Also, "hsa-mir-1910" (miRBase Accession No. MI0008331; SEQ ID NO: 714) having a hairpin-like structure is known as a precursor of "hsa-miR-1910-5p."

The term "hsa-miR-2278 gene" or "hsa-miR-2278" used herein includes the hsa-miR-2278 gene (miRBase Accession No. MIMAT0011778) shown in SEQ ID NO: 322, a homolog or an ortholog of a different organism species, and the like. The hsa-miR-2278 gene can be obtained by a method described in Nygaard S et al., 2009, BMC Med. Genomics, Vol. 2, p. 35. Also, "hsa-mir-2278" (miRBase Accession No. MI0011285; SEQ ID NO: 715) having a hairpin-like structure is known as a precursor of "hsa-miR-2278."

The term "hsa-miR-2682-3p gene" or "hsa-miR-2682-3p" used herein includes the hsa-miR-2682-3p gene (miRBase Accession No. MIMAT0013518) shown in SEQ ID NO: 323, a homolog or an ortholog of a different organism species, and the like. The hsa-miR-2682-3p gene can be obtained by a method described in Creighton CJ et al., 2010, PLoS One, Vol. 5, e9637. Also, "hsa-mir-2682" (miRBase Accession No. MI0012063; SEQ ID NO: 716) having a hairpin-like structure is known as a precursor of "hsa-miR-2682-3p."

The term "hsa-miR-3122 gene" or "hsa-miR-3122" used herein includes the hsa-miR-3122 gene (miRBase Accession No. MIMAT0014984) shown in SEQ ID NO: 324, a homolog or an ortholog of a different organism species, and the like. The hsa-miR-3122 gene can be obtained by a method described in Stark MS et al., 2010, PLoS One, Vol. 5, e9685. Also, "hsa-mir-3122" (miRBase Accession No. MI0014138; SEQ ID NO: 717) having a hairpin-like structure is known as a precursor of "hsa-miR-3122."

The term "hsa-miR-3151-5p gene" or "hsa-miR-3151-5p" used herein includes the hsa-miR-3151-5p gene (miRBase Accession No. MIMAT0015024) shown in SEQ ID NO: 325, a homolog or an ortholog of a different organism species, and the like. The hsa-miR-3151-5p gene can be obtained by a method described in Stark MS et al., 2010, PLoS One, Vol. 5, e9685. Also, "hsa-mir-3151" (miRBase Accession No. MI0014178; SEQ ID NO: 661) having a hairpin-like structure is known as a precursor of "hsa-miR-3151-5p."

The term "hsa-miR-3175 gene" or "hsa-miR-3175" used herein includes the hsa-miR-3175 gene (miRBase Accession No. MIMAT0015052) shown in SEQ ID NO: 326, a homolog or an ortholog of a different organism species, and the like. The hsa-miR-3175 gene can be obtained by a method described in Creighton CJ et al., 2010, PLoS One, Vol. 5, e9637. Also, "hsa-mir-3175" (miRBase Accession No. MI0014209; SEQ ID NO: 718) having a hairpin-like structure is known as a precursor of "hsa-miR-3175."

The term "hsa-miR-4323 gene" or "hsa-miR-4323" used herein includes the hsa-miR-4323 gene (miRBase Accession No. MIMAT0016875) shown in SEQ ID NO: 327, a homolog or an ortholog of a different organism species, and the like. The hsa-miR-4323 gene can be obtained by a method described in Goff LA et al., 2009, PLoS One, Vol. 4, e7192. Also, "hsa-mir-4323" (miRBase Accession No. MI0015853; SEQ ID NO: 719) having a hairpin-like structure is known as a precursor of "hsa-miR-4323."

The term "hsa-miR-4326 gene" or "hsa-miR-4326" used herein includes the hsa-miR-4326 gene (miRBase Accession No. MIMAT0016888) shown in SEQ ID NO: 328, a homolog or an ortholog of a different organism species, and the like. The hsa-miR-4326 gene can be obtained by a method described in Goff LA et al., 2009, PLoS One, Vol. 4, e7192. Also, "hsa-mir-4326" (miRBase Accession No. MI0015866; SEQ ID NO: 720) having a hairpin-like structure is known as a precursor of "hsa-miR-4326."

The term "hsa-miR-4284 gene" or "hsa-miR-4284" used herein includes the hsa-miR-4284 gene (miRBase Accession No. MIMAT0016915) shown in SEQ ID NO: 329, a homolog or an ortholog of a different organism species, and the like. The hsa-miR-4284 gene can be obtained by a method described in Goff LA et al., 2009, PLoS One, Vol. 4, e7192.

Also, "hsa-mir-4284" (miRBase Accession No. MI0015893; SEQ ID NO: 721) having a hairpin-like structure is known as a precursor of "hsa-miR-4284."

The term "hsa-miR-3605-3p gene" or "hsa-miR-3605-3p" used herein includes the hsa-miR-3605-3p gene (miRBase Accession No. MIMAT0017982) shown in SEQ ID NO: 330, a homolog or an ortholog of a different organism species, and the like. The hsa-miR-3605-3p gene can be obtained by a method described in Creighton CJ et al., 2010, PLoS One, Vol. 5, e9637. Also, "hsa-mir-3605" (miRBase Accession No. MI0015995; SEQ ID NO: 722) having a hairpin-like structure is known as a precursor of "hsa-miR-3605-3p."

The term "hsa-miR-3622b-5p gene" or "hsa-miR-3622b-5p" used herein includes the hsa-miR-3622b-5p gene (miRBase Accession No. MIMAT0018005) shown in SEQ ID NO: 331, a homolog or an ortholog of a different organism species, and the like. The hsa-miR-3622b-5p gene can be obtained by a method described in Witten D et al., 2010, BMC Biol., Vol. 8, p. 58. Also, "hsa-mir-3622b" (miRBase Accession No. MI0016014; SEQ ID NO: 723) having a hairpin-like structure is known as a precursor of "hsa-miR-3622b-5p."

The term "hsa-miR-3646 gene" or "hsa-miR-3646" used herein includes the hsa-miR-3646 gene (miRBase Accession No. MIMAT0018065) shown in SEQ ID NO: 332, a homolog or an ortholog of a different organism species, and the like. The hsa-miR-3646 gene can be obtained by a method described in Meiri E et al., 2010, Nucleic Acids Res., Vol. 38, pp. 6234-6246. Also, "hsa-mir-3646" (miRBase Accession No. MI0016046; SEQ ID NO: 724) having a hairpin-like structure is known as a precursor of "hsa-miR-3646."

The term "hsa-miR-3158-5p gene" or "hsa-miR-3158-5p" used herein includes the hsa-miR-3158-5p gene (miRBase Accession No. MIMAT0019211) shown in SEQ ID NO: 333, a homolog or an ortholog of a different organism species, and the like. The hsa-miR-3158-5p gene can be obtained by a method described in Creighton CJ et al., 2010, PLoS One, Vol. 5, e9637. Also, "hsa-mir-3158-1 and hsa-mir-3158-2" (miRBase Accession Nos. MI0014186 and MI0014187; SEQ ID NOs: 725 and 726) each having a hairpin-like structure are known as precursors of "hsa-miR-3158-5p."

The term "hsa-miR-4722-3p gene" or "hsa-miR-4722-3p" used herein includes the hsa-miR-4722-3p gene (miRBase Accession No. MIMAT0019837) shown in SEQ ID NO: 334, a homolog or an ortholog of a different organism species, and the like. The hsa-miR-4722-3p gene can be obtained by a method described in Persson H et al., 2011, Cancer Res., Vol. 71, pp. 78-86. Also, "hsa-mir-4722" (miRBase Accession No. MI0017357; SEQ ID NO: 727) having a hairpin-like structure is known as a precursor of "hsa-miR-4722-3p."

The term "hsa-miR-4728-3p gene" or "hsa-miR-4728-3p" used herein includes the hsa-miR-4728-3p gene (miRBase Accession No. MIMAT0019850) shown in SEQ ID NO: 335, a homolog or an ortholog of a different organism species, and the like. The hsa-miR-4728-3p gene can be obtained by a method described in Persson H et al., 2011, Cancer Res., Vol. 71, pp. 78-86. Also, "hsa-mir-4728" (miRBase Accession No. MI0017365; SEQ ID NO: 393) having a hairpin-like structure is known as a precursor of "hsa-miR-4728-3p."

The term "hsa-miR-4747-3p gene" or "hsa-miR-4747-3p" used herein includes the hsa-miR-4747-3p gene (miRBase Accession No. MIMAT0019883) shown in SEQ ID NO: 336, a homolog or an ortholog of a different organism species, and the like. The hsa-miR-4747-3p gene can be obtained by a method described in Persson H et al., 2011, Cancer Res., Vol. 71, pp. 78-86. Also, "hsa-mir-4747" (miRBase Accession No. MI0017386; SEQ ID NO: 728) having a hairpin-like structure is known as a precursor of "hsa-miR-4747-3p."

The term "hsa-miR-4436b-5p gene" or "hsa-miR-4436b-5p" used herein includes the hsa-miR-4436b-5p gene (miRBase Accession No. MIMAT0019940) shown in SEQ ID NO: 337, a homolog or an ortholog of a different organism species, and the like. The hsa-miR-4436b-5p gene can be obtained by a method described in Persson H et al., 2011, Cancer Res., Vol. 71, pp. 78-86. Also, "hsa-mir-4436b-1 and hsa-mir-4436b-2" (miRBase Accession Nos. MI0017425 and MI0019110; SEQ ID NOs: 729 and 730) each having a hairpin-like structure are known as precursors of "hsa-miR-4436b-5p."

The term "hsa-miR-5196-3p gene" or "hsa-miR-5196-3p" used herein includes the hsa-miR-5196-3p gene (miRBase Accession No. MIMAT0021129) shown in SEQ ID NO: 338, a homolog or an ortholog of a different organism species, and the like. The hsa-miR-5196-3p gene can be obtained by a method described in Schotte D et al., 2011, Leukemia, Vol. 25, pp. 1389-1399. Also, "hsa-mir-5196" (miRBase Accession No. MI0018175; SEQ ID NO: 731) having a hairpin-like structure is known as a precursor of "hsa-miR-4436b-5p."

The term "hsa-miR-5589-5p gene" or "hsa-miR-5589-5p" used herein includes the hsa-miR-5589-5p gene (miRBase Accession No. MIMAT0022297) shown in SEQ ID NO: 339, a homolog or an ortholog of a different organism species, and the like. The hsa-miR-5589-5p gene can be obtained by a method described in Friedlander MR et al., 2012, Nucleic Acids Res., Vol. 40, pp. 37-52. Also, "hsa-mir-5589" (miRBase Accession No. M10019148; SEQ ID NO: 732) having a hairpin-like structure is known as a precursor of "hsa-miR-5589-5p."

The term "hsa-miR-345-3p gene" or "hsa-miR-345-3p" used herein includes the hsa-miR-345-3p gene (miRBase Accession No. MIMAT0022698) shown in SEQ ID NO: 340, a homolog or an ortholog of a different organism species, and the like. The hsa-miR-345-3p gene can be obtained by a method described in Kim J et al., 2004, Proc. Natl. Acad. Sci.U.S.A., Vol. 101, pp. 360-365. Also, "hsa-mir-345" (miRBase Accession No. MI0000825; SEQ ID NO: 615) having a hairpin-like structure is known as a precursor of "hsa-miR-345-3p."

The term "hsa-miR-642b-5p gene" or "hsa-miR-642b-5p" used herein includes the hsa-miR-642b-5p gene (miRBase Accession No. MIMAT0022736) shown in SEQ ID NO: 341, a homolog or an ortholog of a different organism species, and the like. The hsa-miR-642b-5p gene can be obtained by a method described in Witten D et al., 2010, BMC Biol., Vol. 8, p. 58. Also, "hsa-mir-642b" (miRBase Accession No. MI0016685; SEQ ID NO: 733) having a hairpin-like structure is known as a precursor of "hsa-miR-642b-5p."

The term "hsa-miR-6716-3p gene" or "hsa-miR-6716-3p" used herein includes the hsa-miR-6716-3p gene (miRBase Accession No. MIMAT0025845) shown in SEQ ID NO: 342, a homolog or an ortholog of a different organism species, and the like. The hsa-miR-6716-3p gene can be obtained by a method described in Li Y et al., 2012, Gene, Vol. 497, pp. 330-335. Also, "hsa-mir-6716" (miRBase Accession No. MI0022550; SEQ ID NO: 734) having a hairpin-like structure is known as a precursor of "hsa-miR-6716-3p."

The term "hsa-miR-6511b-3p gene" or "hsa-miR-6511b-3p" used herein includes the hsa-miR-6511b-3p gene (miRBase Accession No. MIMAT0025848) shown in SEQ ID NO: 343, a homolog or an ortholog of a different organism species, and the like. The hsa-miR-6511b-3p gene can be obtained by a method described in Li Y et al., 2012, Gene, Vol. 497, pp. 330-335. Also, "hsa-mir-6511b-1 and hsa-mir-6511b-2" (miRBase Accession Nos. MI0022552 and MI0023431; SEQ ID NOs: 735 and 736) each having a hairpin-like structure are known as precursors of "hsa-miR-6511b-3p."

The term "hsa-miR-208a-5p gene" or "hsa-miR-208a-5p" used herein includes the hsa-miR-208a-5p gene (miRBase Accession No. MIMAT0026474) shown in SEQ ID NO: 344, a homolog or an ortholog of a different organism species, and the like. The hsa-miR-208a-5p gene can be obtained by a method described in Lagos-Quintana M et al., 2003, RNA, Vol. 9, pp. 175-179. Also, "hsa-mir-208a" (miRBase Accession No. MI0000251; SEQ ID NO: 737) having a hairpin-like structure is known as a precursor of "hsa-miR-208a-5p."

The term "hsa-miR-6726-3p gene" or "hsa-miR-6726-3p" used herein includes the hsa-miR-6726-3p gene (miRBase Accession No. MIMAT0027354) shown in SEQ ID NO: 345, a homolog or an ortholog of a different organism species, and the like. The hsa-miR-6726-3p gene can be obtained by a method described in Ladewig E et al., 2012, Genome Res., Vol. 22, pp. 1634-1645. Also, "hsa-mir-6726" (miRBase Accession No. MI0022571; SEQ ID NO: 600) having a hairpin-like structure is known as a precursor of "hsa-miR-6726-3p."

The term "hsa-miR-6744-5p gene" or "hsa-miR-6744-5p" used herein includes the hsa-miR-6744-5p gene (miRBase Accession No. MIMAT0027389) shown in SEQ ID NO: 346, a homolog or an ortholog of a different organism species, and the like. The hsa-miR-6744-5p gene can be obtained by a method described in Ladewig E et al., 2012, Genome Res., Vol. 22, pp. 1634-1645. Also, "hsa-mir-6744" (miRBase Accession No. MI0022589; SEQ ID NO: 738) having a hairpin-like structure is known as a precursor of "hsa-miR-6744-5p."

The term "hsa-miR-6782-3p gene" or "hsa-miR-6782-3p" used herein includes the hsa-miR-6782-3p gene (miRBase Accession No. MIMAT0027465) shown in SEQ ID NO: 347, a homolog or an ortholog of a different organism species, and the like. The hsa-miR-6782-3p gene can be obtained by a method described in Ladewig E et al., 2012, Genome Res., Vol. 22, pp. 1634-1645. Also, "hsa-mir-6782" (miRBase Accession No. MI0022627; SEQ ID NO: 739) having a hairpin-like structure is known as a precursor of "hsa-miR-6782-3p."

The term "hsa-miR-6789-3p gene" or "hsa-miR-6789-3p" used herein includes the hsa-miR-6789-3p gene (miRBase Accession No. MIMAT0027479) shown in SEQ ID NO: 348, a homolog or an ortholog of a different organism species, and the like. The hsa-miR-6789-3p gene can be obtained by a method described in Ladewig E et al., 2012, Genome Res., Vol. 22, pp. 1634-1645. Also, "hsa-mir-6789" (miRBase Accession No. MI0022634; SEQ ID NO: 740) having a hairpin-like structure is known as a precursor of "hsa-miR-6789-3p."

The term "hsa-miR-6797-3p gene" or "hsa-miR-6797-3p" used herein includes the hsa-miR-6797-3p gene (miRBase Accession No. MIMAT0027495) shown in SEQ ID NO: 349, a homolog or an ortholog of a different organism species, and the like. The hsa-miR-6797-3p gene can be obtained by a method described in Ladewig E et al., 2012, Genome Res., Vol. 22, pp. 1634-1645. Also, "hsa-mir-6797" (miRBase Accession No. MI0022642; SEQ ID NO: 741) having a hairpin-like structure is known as a precursor of "hsa-miR-6797-3p."

The term "hsa-miR-6800-3p gene" or "hsa-miR-6800-3p" used herein includes the hsa-miR-6800-3p gene (miRBase Accession No. MIMAT0027501) shown in SEQ ID NO: 350, a homolog or an ortholog of a different organism species, and the like. The hsa-miR-6800-3p gene can be obtained by a method described in Ladewig E et al., 2012, Genome Res., Vol. 22, pp. 1634-1645. Also, "hsa-mir-6800" (miRBase Accession No. MI0022645; SEQ ID NO: 491) having a hairpin-like structure is known as a precursor of "hsa-miR-6800-3p."

The term "hsa-miR-6806-5p gene" or "hsa-miR-6806-5p" used herein includes the hsa-miR-6806-5p gene (miRBase Accession No. MIMAT0027512) shown in SEQ ID NO: 351, a homolog or an ortholog of a different organism species, and the like. The hsa-miR-6806-5p gene can be obtained by a method described in Ladewig E et al., 2012, Genome Res., Vol. 22, pp. 1634-1645. Also, "hsa-mir-6806" (miRBase Accession No. MI0022651; SEQ ID NO: 742) having a hairpin-like structure is known as a precursor of "hsa-miR-6806-5p."

The term "hsa-miR-6824-3p gene" or "hsa-miR-6824-3p" used herein includes the hsa-miR-6824-3p gene (miRBase Accession No. MIMAT0027549) shown in SEQ ID NO: 352, a homolog or an ortholog of a different organism species, and the like. The hsa-miR-6824-3p gene can be obtained by a method described in Ladewig E et al., 2012, Genome Res., Vol. 22, pp. 1634-1645. Also, "hsa-mir-6824" (miRBase Accession No. MI0022669; SEQ ID NO: 743) having a hairpin-like structure is known as a precursor of "hsa-miR-6824-3p."

The term "hsa-miR-6837-5p gene" or "hsa-miR-6837-5p" used herein includes the hsa-miR-6837-5p gene (miRBase Accession No. MIMAT0027576) shown in SEQ ID NO: 353, a homolog or an ortholog of a different organism species, and the like. The hsa-miR-6837-5p gene can be obtained by a method described in Ladewig E et al., 2012, Genome Res., Vol. 22, pp. 1634-1645. Also, "hsa-mir-6837" (miRBase Accession No. MI0022683; SEQ ID NO: 744) having a hairpin-like structure is known as a precursor of "hsa-miR-6837-5p."

The term "hsa-miR-6846-3p gene" or "hsa-miR-6846-3p" used herein includes the hsa-miR-6846-3p gene (miRBase Accession No. MIMAT0027593) shown in SEQ ID NO: 354, a homolog or an ortholog of a different organism species, and the like. The hsa-miR-6846-3p gene can be obtained by a method described in Ladewig E et al., 2012, Genome Res., Vol. 22, pp. 1634-1645. Also, "hsa-mir-6846" (miRBase Accession No. MI0022692; SEQ ID NO: 745) having a hairpin-like structure is known as a precursor of "hsa-miR-6846-3p."

The term "hsa-miR-6858-3p gene" or "hsa-miR-6858-3p" used herein includes the hsa-miR-6858-3p gene (miRBase Accession No. MIMAT0027617) shown in SEQ ID NO: 355, a homolog or an ortholog of a different organism species, and the like. The hsa-miR-6858-3p gene can be obtained by a method described in Ladewig E et al., 2012, Genome Res., Vol. 22, pp. 1634-1645. Also, "hsa-mir-6858" (miRBase Accession No. MI0022704; SEQ ID NO: 746) having a hairpin-like structure is known as a precursor of "hsa-miR-6858-3p."

The term "hsa-miR-6859-3p gene" or "hsa-miR-6859-3p" used herein includes the hsa-miR-6859-3p gene (miRBase Accession No. MIMAT0027619) shown in SEQ ID NO: 356, a homolog or an ortholog of a different organism species, and the like. The hsa-miR-6859-3p gene can be obtained by a method described in Ladewig E et al., 2012, Genome Res., Vol. 22, pp. 1634-1645. Also, "hsa-mir-6859-1, hsa-mir-6859-2, hsa-mir-6859-3, and hsa-mir-6859-4" (miRBase Accession Nos. MI0022705, MI0026420, MI0026421, and MI0031521; SEQ ID NOs: 747, 748, 749, and 750) each having a hairpin-like structure are known as precursors of "hsa-miR-6858-3p."

The term "hsa-miR-6861-3p gene" or "hsa-miR-6861-3p" used herein includes the hsa-miR-6861-3p gene (miRBase Accession No. MIMAT0027624) shown in SEQ ID NO: 357, a homolog or an ortholog of a different organism species, and the like. The hsa-miR-6861-3p gene can be obtained by a method described in Ladewig E et al., 2012, Genome Res., Vol. 22, pp. 1634-1645. Also, "hsa-mir-6861" (miRBase Accession No. MI0022708; SEQ ID NO: 596) having a hairpin-like structure is known as a precursor of "hsa-miR-6861-3p."

The term "hsa-miR-6880-3p gene" or "hsa-miR-6880-3p" used herein includes the hsa-miR-6880-3p gene (miRBase Accession No. MIMAT0027661) shown in SEQ ID NO: 358, a homolog or an ortholog of a different organism species, and the like. The hsa-miR-6880-3p gene can be obtained by a method described in Ladewig E et al., 2012, Genome Res., Vol. 22, pp. 1634-1645. Also, "hsa-mir-6880" (miRBase Accession No. MI0022727; SEQ ID NO: 751) having a hairpin-like structure is known as a precursor of "hsa-miR-6880-3p."

The term "hsa-miR-7111-3p gene" or "hsa-miR-7111-3p" used herein includes the hsa-miR-7111-3p gene (miRBase Accession No. MIMAT0028120) shown in SEQ ID NO: 359, a homolog or an ortholog of a different organism species, and the like. The hsa-miR-7111-3p gene can be obtained by a method described in Ladewig E et al., 2012, Genome Res., Vol. 22, pp. 1634-1645. Also, "hsa-mir-7111" (miRBase Accession No. MI0022962; SEQ ID NO: 752) having a hairpin-like structure is known as a precursor of "hsa-miR-7111-3p."

The term "hsa-miR-7152-5p gene" or "hsa-miR-7152-5p" used herein includes the hsa-miR-7152-5p gene (miRBase Accession No. MIMAT0028214) shown in SEQ ID NO: 360, a homolog or an ortholog of a different organism species, and the like. The hsa-miR-7152-5p gene can be obtained by a method described in Meunier J et al., 2013, Genome Res., Vol. 23, pp. 34-45. Also, "hsa-mir-7152" (miRBase Accession No. MI0023612; SEQ ID NO: 753) having a hairpin-like structure is known as a precursor of "hsa-miR-7152-5p."

The term "hsa-miR-642a-5p gene" or "hsa-miR-642a-5p" used herein includes the hsa-miR-642a-5p gene (miRBase Accession No. MIMAT0003312) shown in SEQ ID NO: 361, a homolog or an ortholog of a different organism species, and the like. The hsa-miR-642a-5p gene can be obtained by a method described in Cummins JM et al., 2006, Proc. Natl. Acad. Sci. U.S.A., Vol. 103, pp. 3687-3692. Also, "hsa-mir-642a" (miRBase Accession No. MI0003657; SEQ ID NO: 754) having a hairpin-like structure is known as a precursor of "hsa-miR-642a-5p."

The term "hsa-miR-657 gene" or "hsa-miR-657" used herein includes the hsa-miR-657 gene (miRBase Accession No. MIMAT0003335) shown in SEQ ID NO: 362, a homolog or an ortholog of a different organism species, and the like. The hsa-miR-657 gene can be obtained by a method described in Cummins JM et al., 2006, Proc. Natl. Acad. Sci. U.S.A., Vol. 103, pp. 3687-3692. Also, "hsa-mir-657" (miRBase Accession No. MI0003681; SEQ ID NO: 755) having a hairpin-like structure is known as a precursor of "hsa-miR-657."

The term "hsa-miR-1236-3p gene" or "hsa-miR-1236-3p" used herein includes the hsa-miR-1236-3p gene (miRBase Accession No. MIMAT0005591) shown in SEQ ID NO: 363, a homolog or an ortholog of a different organism species, and the like. The hsa-miR-1236-3p gene can be obtained by a method described in Berezikov E et al., 2007, Mol. Cell, Vol. 28, pp. 328-336. Also, "hsa-mir-1236" (miRBase Accession No. MI0006326; SEQ ID NO: 756) having a hairpin-like structure is known as a precursor of "hsa-miR-1236-3p."

The term "hsa-miR-764 gene" or "hsa-miR-764" used herein includes the hsa-miR-764 gene (miRBase Accession No. MIMAT0010367) shown in SEQ ID NO: 364, a homolog or an ortholog of a different organism species, and the like. The hsa-miR-764 gene can be obtained by a method described in Berezikov E et al., 2006, Genome Res., Vol. 16, pp. 1289-1298. Also, "hsa-mir-764" (miRBase Accession No. MI0003944; SEQ ID NO: 757) having a hairpin-like structure is known as a precursor of "hsa-miR-764."

The term "hsa-miR-4314 gene" or "hsa-miR-4314" used herein includes the hsa-miR-4314 gene (miRBase Accession No. MIMAT0016868) shown in SEQ ID NO: 365, a homolog or an ortholog of a different organism species, and the like. The hsa-miR-4314 gene can be obtained by a method described in Goff LA et al., 2009, PLoS One, Vol. 4, e7192. Also, "hsa-mir-4314" (miRBase Accession No. MI0015846; SEQ ID NO: 758) having a hairpin-like structure is known as a precursor of "hsa-miR-4314."

The term "hsa-miR-3675-3p gene" or "hsa-miR-3675-3p" used herein includes the hsa-miR-3675-3p gene (miRBase Accession No. MIMAT0018099) shown in SEQ ID NO: 366, a homolog or an ortholog of a different organism species, and the like. The hsa-miR-3675-3p gene can be obtained by a method described in Vaz C et al., 2010, BMC Genomics, Vol. 11, p. 288. Also, "hsa-mir-3675" (miRBase Accession No. MI0016076; SEQ ID NO: 759) having a hairpin-like structure is known as a precursor of "hsa-miR-3675-3p."

The term "hsa-miR-5703 gene" or "hsa-miR-5703" used herein includes the hsa-miR-5703 gene (miRBase Accession No. MIMAT0022496) shown in SEQ ID NO: 367, a homolog or an ortholog of a different organism species, and the like. The hsa-miR-5703 gene can be obtained by a method described in Watahiki A et al., 2011, PLoS One, Vol. 6, e24950. Also, "hsa-mir-5703" (miRBase Accession No. MI0019310; SEQ ID NO: 760) having a hairpin-like structure is known as a precursor of "hsa-miR-5703."

The term "hsa-miR-3191-5p gene" or "hsa-miR-3191-5p" used herein includes the hsa-miR-3191-5p gene (miRBase Accession No. MIMAT0022732) shown in SEQ ID NO: 368, a homolog or an ortholog of a different organism species, and the like. The hsa-miR-3191-5p gene can be obtained by a method described in Stark MS et al., 2010, PLoS One, Vol. 5, e9685. Also, "hsa-mir-3191" (miRBase Accession No. MI0014236; SEQ ID NO: 761) having a hairpin-like structure is known as a precursor of "hsa-miR-3191-5p."

The term "hsa-miR-6511a-3p gene" or "hsa-miR-6511a-3p" used herein includes the hsa-miR-6511a-3p gene (miRBase Accession No. MIMAT0025479) shown in SEQ ID NO: 369, a homolog or an ortholog of a different organism species, and the like. The hsa-miR-6511a-3p gene can be obtained by a method described in Joyce CE et al., 2011, Hum. Mol. Genet., Vol. 20, pp. 4025-4040. Also, "hsa-mir-6511a-1" (miRBase Accession No. MI0022223; SEQ ID NO: 445) having a hairpin-like structure is known as a precursor of "hsa-miR-6511a-3p."

The term "hsa-miR-6809-3p gene" or "hsa-miR-6809-3p" used herein includes the hsa-miR-6809-3p gene (miRBase Accession No. MIMAT0027519) shown in SEQ ID NO: 370, a homolog or an ortholog of a different organism species, and the like. The hsa-miR-6809-3p gene can be obtained by a method described in Ladewig E et al., 2012, Genome Res., Vol. 22, pp. 1634-1645. Also, "hsa-mir-6809" (miRBase Accession No. MI0022654; SEQ ID NO: 762) having a hairpin-like structure is known as a precursor of "hsa-miR-6809-3p."

The term "hsa-miR-6815-5p gene" or "hsa-miR-6815-5p" used herein includes the hsa-miR-6815-5p gene (miRBase Accession No. MIMAT0027530) shown in SEQ ID NO: 371, a homolog or an ortholog of a different organism species, and the like. The hsa-miR-6815-5p gene can be obtained by a method described in Ladewig E et al., 2012, Genome Res., Vol. 22, pp. 1634-1645. Also, "hsa-mir-6815" (miRBase Accession No. MI0022660; SEQ ID NO: 763) having a hairpin-like structure is known as a precursor of "hsa-miR-6815-5p."

The term "hsa-miR-6857-3p gene" or "hsa-miR-6857-3p" used herein includes the hsa-miR-6857-3p gene (miRBase Accession No. MIMAT0027615) shown in SEQ ID NO: 372, a homolog or an ortholog of a different organism species, and the like. The hsa-miR-6857-3p gene can be obtained by a method described in Ladewig E et al., 2012, Genome Res., Vol. 22, pp. 1634-1645. Also, "hsa-mir-6857" (miRBase Accession No. MI0022703; SEQ ID NO: 764) having a hairpin-like structure is known as a precursor of "hsa-miR-6857-3p."

The term "hsa-miR-6878-3p gene" or "hsa-miR-6878-3p" used herein includes the hsa-miR-6878-3p gene (miRBase Accession No. MIMAT0027657) shown in SEQ ID NO: 373, a homolog or an ortholog of a different organism species, and the like. The hsa-miR-6878-3p gene can be obtained by a method described in Ladewig E et al., 2012, Genome Res., Vol. 22, pp. 1634-1645. Also, "hsa-mir-6878" (miRBase Accession No. MI0022725; SEQ ID NO: 765) having a hairpin-like structure is known as a precursor of "hsa-miR-6878-3p."

The term "hsa-miR-371a-5p gene" or "hsa-miR-371a-5p" used herein includes the hsa-miR-371a-5p gene (miRBase Accession No. MIMAT0004687) shown in SEQ ID NO: 374, a homolog or an ortholog of a different organism species, and the like. The hsa-miR-371a-5p gene can be obtained by a method described in Suh MR et al., 2004, Dev. Biol., Vol. 270, pp. 488-498. Also, "hsa-mir-371a" (miRBase Accession No. MI0000779; SEQ ID NO: 766) having a hairpin-like structure is known as a precursor of "hsa-miR-371a-5p."

The term "hsa-miR-766-3p gene" or "hsa-miR-766-3p" used herein includes the hsa-miR-766-3p gene (miRBase Accession No. MIMAT0003888) shown in SEQ ID NO: 375, a homolog or an ortholog of a different organism species, and the like. The hsa-miR-766-3p gene can be obtained by a method described in Berezikov E et al., 2006, Genome Res., Vol. 16, pp. 1289-1298. Also, "hsa-mir-766" (miRBase Accession No. MI0003836; SEQ ID NO: 767) having a hairpin-like structure is known as a precursor of "hsa-miR-766-3p."

The term "hsa-miR-1229-3p gene" or "hsa-miR-1229-3p" used herein includes the hsa-miR-1229-3p gene (miRBase Accession No. MIMAT0005584) shown in SEQ ID NO: 376, a homolog or an ortholog of a different organism species, and the like. The hsa-miR-1229-3p gene can be obtained by a method described in Berezikov E et al., 2007, Mol. Cell, Vol. 28, pp. 328-336. Also, "hsa-mir-1229" (miRBase Accession No. MI0006319; SEQ ID NO: 768) having a hairpin-like structure is known as a precursor of "hsa-miR-1229-3p."

The term "hsa-miR-1306-5p gene" or "hsa-miR-1306-5p" used herein includes the hsa-miR-1306-5p gene (miRBase Accession No. MIMAT0022726) shown in SEQ ID NO: 377, a homolog or an ortholog of a different organism species, and the like. The hsa-miR-1306-5p gene can be obtained by a method described in Morin RD et al., 2008, Genome Res., Vol. 18, pp. 610-621. Also, "hsa-mir-1306" (miRBase Accession No. MI0006443; SEQ ID NO: 769) having a hairpin-like structure is known as a precursor of "hsa-miR-1306-5p."

The term "hsa-miR-210-5p gene" or "hsa-miR-210-5p" used herein includes the hsa-miR-210-5p gene (miRBase Accession No. MIMAT0026475) shown in SEQ ID NO: 378, a homolog or an ortholog of a different organism species, and the like. The hsa-miR-210-5p gene can be obtained by a method described in Lim LP et al., 2003, Science, Vol. 299, p. 1540. Also, "hsa-mir-210" (miRBase Accession No. MI0000286; SEQ ID NO: 770) having a hairpin-like structure is known as a precursor of "hsa-miR-210-5p."

The term "hsa-miR-198 gene" or "hsa-miR-198" used herein includes the hsa-miR-198 gene (miRBase Accession No. MIMAT0000228) shown in SEQ ID NO: 379, a homolog or an ortholog of a different organism species, and the like. The hsa-miR-198 gene can be obtained by a method described in Lagos-Quintana M et al., 2003, RNA, Vol. 9, pp. 175-179. Also, "hsa-mir-198" (miRBase Accession No. MI0000240; SEQ ID NO: 771) having a hairpin-like structure is known as a precursor of "hsa-miR-198."

The term "hsa-miR-485-3p gene" or "hsa-miR-485-3p" used herein includes the hsa-miR-485-3p gene (miRBase Accession No. MIMAT0002176) shown in SEQ ID NO: 380, a homolog or an ortholog of a different organism species, and the like. The hsa-miR-485-3p gene can be obtained by a method described in Bentwich I et al., 2005, Nat. Genet., Vol. 37, pp. 766-770. Also, "hsa-mir-485" (miRBase Accession No. MI0002469; SEQ ID NO: 772) having a hairpin-like structure is known as a precursor of "hsa-miR-485-3p."

The term "hsa-miR-668-3p gene" or "hsa-miR-668-3p" used herein includes the hsa-miR-668-3p gene (miRBase Accession No. MIMAT0003881) shown in SEQ ID NO: 381, a homolog or an ortholog of a different organism species, and the like. The hsa-miR-668-3p gene can be obtained by a method described in Berezikov E et al., 2006, Genome Res., Vol. 16, pp. 1289-1298. Also, "hsa-mir-668" (miRBase Accession No. MI0003761; SEQ ID NO: 773) having a hairpin-like structure is known as a precursor of "hsa-miR-668-3p."

The term "hsa-miR-532-3p gene" or "hsa-miR-532-3p" used herein includes the hsa-miR-532-3p gene (miRBase Accession No. MIMAT0004780) shown in SEQ ID NO: 382, a homolog or an ortholog of a different organism species, and the like. The hsa-miR-532-3p gene can be obtained by a method described in Lagos-Quintana M et al., 2001, Science, Vol. 294, pp. 853-858. Also, "hsa-mir-532" (miRBase Accession No. MI0003205; SEQ ID NO: 626) having a hairpin-like structure is known as a precursor of "hsa-miR-532-3p."

The term "hsa-miR-877-3p gene" or "hsa-miR-877-3p" used herein includes the hsa-miR-877-3p gene (miRBase Accession No. MIMAT0004950) shown in SEQ ID NO: 383, a homolog or an ortholog of a different organism species, and the like. The hsa-miR-877-3p gene can be obtained by a method described in Berezikov E et al., 2006, Genome Res., Vol. 16, pp. 1289-1298. Also, "hsa-mir-877" (miRBase Accession No. MI0005561; SEQ ID NO: 774) having a hairpin-like structure is known as a precursor of "hsa-miR-877-3p."

The term "hsa-miR-1238-3p gene" or "hsa-miR-1238-3p" used herein includes the hsa-miR-1238-3p gene (miRBase Accession No. MIMAT0005593) shown in SEQ ID NO: 384, a homolog or an ortholog of a different organism species, and the like. The hsa-miR-1238-3p gene can be obtained by a method described in Berezikov E et al., 2007, Mol. Cell, Vol. 28, pp. 328-336. Also, "hsa-mir-1238" (miRBase Accession No. MI0006328; SEQ ID NO: 775) having a hairpin-like structure is known as a precursor of "hsa-miR-1238-3p."

The term "hsa-miR-3130-5p gene" or "hsa-miR-3130-5p" used herein includes the hsa-miR-3130-5p gene (miRBase Accession No. MIMAT0014995) shown in SEQ ID NO: 385, a homolog or an ortholog of a different organism species, and the like. The hsa-miR-3130-5p gene can be obtained by a method described in Creighton CJ et al., 2010, PLoS One, Vol. 5, e9637. Also, "hsa-mir-3130-1 and hsa-mir-3130-2" (miRBase Accession Nos. MI0014147 and M10014148; SEQ ID NOs: 776 and 777) each having a hairpin-like structure are known as precursors of "hsa-miR-3130-5p."

The term "hsa-miR-4298 gene" or "hsa-miR-4298" used herein includes the hsa-miR-4298 gene (miRBase Accession No. MIMAT0016852) shown in SEQ ID NO: 386, a homolog or an ortholog of a different organism species, and the like. The hsa-miR-4298 gene can be obtained by a method described in Goff LA et al., 2009, PLoS One, Vol. 4, e7192. Also, "hsa-mir-4298" (miRBase Accession No. MI0015830; SEQ ID NO: 778) having a hairpin-like structure is known as a precursor of "hsa-miR-4298."

The term "hsa-miR-4290 gene" or "hsa-miR-4290" used herein includes the hsa-miR-4290 gene (miRBase Accession No. MIMAT0016921) shown in SEQ ID NO: 387, a homolog or an ortholog of a different organism species, and the like. The hsa-miR-4290 gene can be obtained by a method described in Goff LA et al., 2009, PLoS One, Vol. 4, e7192. Also, "hsa-mir-4290" (miRBase Accession No. MI0015899; SEQ ID NO: 779) having a hairpin-like structure is known as a precursor of "hsa-miR-4290."

The term "hsa-miR-3943 gene" or "hsa-miR-3943" used herein includes the hsa-miR-3943 gene (miRBase Accession No. MIMAT0018359) shown in SEQ ID NO: 388, a homolog or an ortholog of a different organism species, and the like. The hsa-miR-3943 gene can be obtained by a method described in Liao JY et al., 2010, PLoS One, Vol. 5, e10563. Also, "hsa-mir-3943" (miRBase Accession No. MI0016600; SEQ ID NO: 780) having a hairpin-like structure is known as a precursor of "hsa-miR-3943."

The term "hsa-miR-346 gene" or "hsa-miR-346" used herein includes the hsa-miR-346 gene (miRBase Accession No. MIMAT0000773) shown in SEQ ID NO: 389, a homolog or an ortholog of a different organism species, and the like. The hsa-miR-346 gene can be obtained by a method described in Kim J et al., 2004, Proc. Natl. Acad. Sci. U.S.A., Vol. 101, pp. 360-365. Also, "hsa-mir-346" (miRBase Accession No. M10000826; SEQ ID NO: 781) having a hairpin-like structure is known as a precursor of "hsa-miR-346."

The term "hsa-miR-767-3p gene" or "hsa-miR-767-3p" used herein includes the hsa-miR-767-3p gene (miRBase Accession No. MIMAT0003883) shown in SEQ ID NO: 390, a homolog or an ortholog of a different organism species, and the like. The hsa-miR-767-3p gene can be obtained by a method described in Berezikov E et al., 2006, Genome Res., Vol. 16, pp. 1289-1298. Also, "hsa-mir-767" (miRBase Accession No. MI0003763; SEQ ID NO: 782) having a hairpin-like structure is known as a precursor of "hsa-miR-767-3p."

The term "hsa-miR-6765-3p gene" or "hsa-miR-6765-3p" used herein includes the hsa-miR-6765-3p gene (miRBase Accession No. MIMAT0027431) shown in SEQ ID NO: 1315, a homolog or an ortholog of a different organism species, and the like. The hsa-miR-6765-3p gene can be obtained by a method described in Ladewig E et al., 2012, Genome Res., Vol. 22, pp. 1634-1645. Also, "hsa-mir-6765" (miRBase Accession No. MI0022610; SEQ ID NO: 490) having a hairpin-like structure is known as a precursor of "hsa-miR-6765-3p."

The term "hsa-miR-6784-5p gene" or "hsa-miR-6784-5p" used herein includes the hsa-miR-6784-5p gene (miRBase Accession No. MIMAT0027468) shown in SEQ ID NO: 1316, a homolog or an ortholog of a different organism species, and the like. The hsa-miR-6784-5p gene can be obtained by a method described in Ladewig E et al., 2012, Genome Res., Vol. 22, pp. 1634-1645. Also, "hsa-mir-6784" (miRBase Accession No. M10022629; SEQ ID NO: 1357) having a hairpin-like structure is known as a precursor of "hsa-miR-6784-5p."

The term "hsa-miR-6778-5p gene" or "hsa-miR-6778-5p" used herein includes the hsa-miR-6778-5p gene (miRBase Accession No. MIMAT0027456) shown in SEQ ID NO: 1317, a homolog or an ortholog of a different organism species, and the like. The hsa-miR-6778-5p gene can be obtained by a method described in Ladewig E et al., 2012, Genome Res., Vol. 22, pp. 1634-1645. Also, "hsa-mir-6778" (miRBase Accession No. MI0022623; SEQ ID NO: 1358) having a hairpin-like structure is known as a precursor of "hsa-miR-6778-5p."

The term "hsa-miR-6875-5p gene" or "hsa-miR-6875-5p" used herein includes the hsa-miR-6875-5p gene (miRBase Accession No. MIMAT0027650) shown in SEQ ID NO: 1318, a homolog or an ortholog of a different organism species, and the like. The hsa-miR-6875-5p gene can be obtained by a method described in Ladewig E et al., 2012, Genome Res., Vol. 22, pp. 1634-1645. Also, "hsa-mir-6875" (miRBase Accession No. MI0022722; SEQ ID NO: 1359) having a hairpin-like structure is known as a precursor of "hsa-miR-6875-5p."

The term "hsa-miR-4534 gene" or "hsa-miR-4534" used herein includes the hsa-miR-4534 gene (miRBase Accession No. MIMAT0019073) shown in SEQ ID NO: 1319, a homolog or an ortholog of a different organism species, and the like. The hsa-miR-4534 gene can be obtained by a method described in Jima DD et al., 2010, Blood, Vol. 116, e118-e127. Also, "hsa-mir-4534" (miRBase Accession No. MI0016901; SEQ ID NO: 1360) having a hairpin-like structure is known as a precursor of "hsa-miR-4534."

The term "hsa-miR-4721 gene" or "hsa-miR-4721" used herein includes the hsa-miR-4721 gene (miRBase Accession No. MIMAT0019835) shown in SEQ ID NO: 1320, a homolog or an ortholog of a different organism species, and the like. The hsa-miR-4721 gene can be obtained by a method described in Persson H et al., 2011, Cancer Res., Vol. 71, pp. 78-86. Also, "hsa-mir-4721" (miRBase Accession No. MI0017356; SEQ ID NO: 1361) having a hairpin-like structure is known as a precursor of "hsa-miR-4721."

The term "hsa-miR-6756-5p gene" or "hsa-miR-6756-5p" used herein includes the hsa-miR-6756-5p gene (miRBase Accession No. MIMAT0027412) shown in SEQ ID NO: 1321, a homolog or an ortholog of a different organism species, and the like. The hsa-miR-6756-5p gene can be obtained by a method described in Ladewig E et al., 2012, Genome Res., Vol. 22, pp. 1634-1645. Also, "hsa-mir-6756" (miRBase Accession No. MI0022601; SEQ ID NO: 1362) having a hairpin-like structure is known as a precursor of "hsa-miR-6756-5p."

The term "hsa-miR-615-5p gene" or "hsa-miR-615-5p" used herein includes the hsa-miR-615-5p gene (miRBase Accession No. MIMAT0004804) shown in SEQ ID NO: 1322, a homolog or an ortholog of a different organism species, and the like. The hsa-miR-615-5p gene can be obtained by a method described in Cummins JM et al., 2006, Proc. Natl. Acad. Sci. U.S.A., Vol. 103, pp. 3687-3692. Also, "hsa-mir-615" (miRBase Accession No. MI0003628; SEQ ID NO: 1363) having a hairpin-like structure is known as a precursor of "hsa-miR-615-5p."

The term "hsa-miR-6727-5p gene" or "hsa-miR-6727-5p" used herein includes the hsa-miR-6727-5p gene (miRBase Accession No. MIMAT0027355) shown in SEQ ID NO: 1323, a homolog or an ortholog of a different organism species, and the like. The hsa-miR-6727-5p gene can be obtained by a method described in Ladewig E et al., 2012, Genome Res., Vol. 22, pp. 1634-1645. Also, "hsa-mir-6727" (miRBase Accession No. MI0022572; SEQ ID NO: 1364) having a hairpin-like structure is known as a precursor of "hsa-miR-6727-5p."

The term "hsa-miR-6887-5p gene" or "hsa-miR-6887-5p" used herein includes the hsa-miR-6887-5p gene (miRBase Accession No. MIMAT0027674) shown in SEQ ID NO: 1324, a homolog or an ortholog of a different organism species, and the like. The hsa-miR-6887-5p gene can be obtained by a method described in Ladewig E et al., 2012, Genome Res., Vol. 22, pp. 1634-1645. Also, "hsa-mir-6887" (miRBase Accession No. MI0022734; SEQ ID NO: 1365) having a hairpin-like structure is known as a precursor of "hsa-miR-6887-5p."

The term "hsa-miR-8063 gene" or "hsa-miR-8063" used herein includes the hsa-miR-8063 gene (miRBase Accession No. MIMAT0030990) shown in SEQ ID NO: 1325, a homolog or an ortholog of a different organism species, and the like. The hsa-miR-8063 gene can be obtained by a method described in Wang HJ et al., 2013, Shock, Vol. 39, pp. 480-487. Also, "hsa-mir-8063" (miRBase Accession No. MI0025899; SEQ ID NO: 1366) having a hairpin-like structure is known as a precursor of "hsa-miR-8063."

The term "hsa-miR-6880-5p gene" or "hsa-miR-6880-5p" used herein includes the hsa-miR-6880-5p gene (miRBase Accession No. MIMAT0027660) shown in SEQ ID NO: 1326, a homolog or an ortholog of a different organism species, and the like. The hsa-miR-6880-5p gene can be obtained by a method described in Ladewig E et al., 2012, Genome Res., Vol. 22, pp. 1634-1645. Also, "hsa-mir-6880" (miRBase Accession No. MI0022727; SEQ ID NO: 1367) having a hairpin-like structure is known as a precursor of "hsa-miR-6880-5p."

The term "hsa-miR-6805-3p gene" or "hsa-miR-6805-3p" used herein includes the hsa-miR-6805-3p gene (miRBase Accession No. MIMAT0027511) shown in SEQ ID NO: 1327, a homolog or an ortholog of a different organism species, and the like. The hsa-miR-6805-3p gene can be obtained by a method described in Ladewig E et al., 2012, Genome Res., Vol. 22, pp. 1634-1645. Also, "hsa-mir-6805" (miRBase Accession No. MI0022650; SEQ ID NO: 1368) having a hairpin-like structure is known as a precursor of "hsa-miR-6805-3p."

The term "hsa-miR-4726-5p gene" or "hsa-miR-4726-5p" used herein includes the hsa-miR-4726-5p gene (miRBase Accession No. MIMAT0019845) shown in SEQ ID NO: 1328, a homolog or an ortholog of a different organism species, and the like. The hsa-miR-4726-5p gene can be obtained by a method described in Persson H et al., 2011, Cancer Res., Vol. 71, pp. 78-86. Also, "hsa-mir-4726" (miRBase Accession No. MI0017363; SEQ ID NO: 1369) having a hairpin-like structure is known as a precursor of "hsa-miR-4726-5p."

The term "hsa-miR-4710 gene" or "hsa-miR-4710" used herein includes the hsa-miR-4710 gene (miRBase Accession No. MIMAT0019815) shown in SEQ ID NO: 1329, a homolog or an ortholog of a different organism species, and the like. The hsa-miR-4710 gene can be obtained by a method described in Persson H et al., 2011, Cancer Res., Vol. 71, pp. 78-86. Also, "hsa-mir-4710" (miRBase Accession No. MI0017344; SEQ ID NO: 1370) having a hairpin-like structure is known as a precursor of "hsa-miR-4710."

The term "hsa-miR-7111-5p gene" or "hsa-miR-7111-5p" used herein includes the hsa-miR-7111-5p gene (miRBase Accession No. MIMAT0028119) shown in SEQ ID NO: 1330, a homolog or an ortholog of a different organism species, and the like. The hsa-miR-7111-5p gene can be obtained by a method described in Ladewig E et al., 2012, Genome Res., Vol. 22, pp. 1634-1645. Also, "hsa-mir-7111" (miRBase Accession No. MI0022962; SEQ ID NO: 1371) having a hairpin-like structure is known as a precursor of "hsa-miR-7111-5p."

The term "hsa-miR-3619-3p gene" or "hsa-miR-3619-3p" used herein includes the hsa-miR-3619-3p gene (miRBase Accession No. MIMAT0019219) shown in SEQ ID NO: 1331, a homolog or an ortholog of a different organism species, and the like. The hsa-miR-3619-3p gene can be obtained by a method described in Witten D et al., 2010, BMC Biol., Vol. 8, p. 58. Also, "hsa-mir-3619" (miRBase Accession No. MI0016009; SEQ ID NO: 1372) having a hairpin-like structure is known as a precursor of "hsa-miR-3619-3p."

The term "hsa-miR-6795-5p gene" or "hsa-miR-6795-5p" used herein includes the hsa-miR-6795-5p gene (miRBase Accession No. MIMAT0027490) shown in SEQ ID NO: 1332, a homolog or an ortholog of a different organism species, and the like. The hsa-miR-6795-5p gene can be obtained by a method described in Ladewig E et al., 2012, Genome Res., Vol. 22, pp. 1634-1645. Also, "hsa-mir-6795" (miRBase Accession No. MI0022640; SEQ ID NO: 1373) having a hairpin-like structure is known as a precursor of "hsa-miR-6795-5p."

The term "hsa-miR-1254 gene" or "hsa-miR-1254" used herein includes the hsa-miR-1254 gene (miRBase Accession No. MIMAT0005905) shown in SEQ ID NO: 1333, a homolog or an ortholog of a different organism species, and the like. The hsa-miR-1254 gene can be obtained by a method described in Morin RD et al., 2008, Genome Res., Vol. 18, pp. 610-621. Also, "hsa-mir-1254-1 and hsa-mir-1254-2" (miRBase Accession Nos. MI0006388 and MI0016747; SEQ ID NOs: 1374 and 1375) each having a hairpin-like structure are known as precursors of "hsa-miR-1254."

The term "hsa-miR-1233-5p gene" or "hsa-miR-1233-5p" used herein includes the hsa-miR-1233-5p gene (miRBase Accession No. MIMAT0022943) shown in SEQ ID NO: 1334, a homolog or an ortholog of a different organism species, and the like. The hsa-miR-1233-5p gene can be obtained by a method described in Berezikov E et al., 2007, Mol. Cell, Vol. 28, pp. 328-336. Also, "hsa-mir-1233-1 and hsa-mir-1233-2" (miRBase Accession Nos. MI0006323 and MI0015973; SEQ ID NOs: 1376 and 1377) each having a hairpin-like structure are known as precursors of "hsa-miR-1233-5p."

The term "hsa-miR-6836-3p gene" or "hsa-miR-6836-3p" used herein includes the hsa-miR-6836-3p gene (miRBase Accession No. MIMAT0027575) shown in SEQ ID NO: 1335, a homolog or an ortholog of a different organism species, and the like. The hsa-miR-6836-3p gene can be obtained by a method described in Ladewig E et al., 2012, Genome Res., Vol. 22, pp. 1634-1645. Also, "hsa-mir-6836" (miRBase Accession No. MI0022682; SEQ ID NO: 1378) having a hairpin-like structure is known as a precursor of "hsa-miR-6836-3p."

The term "hsa-miR-6769a-5p gene" or "hsa-miR-6769a-5p" used herein includes the hsa-miR-6769a-5p gene (miRBase Accession No. MIMAT0027438) shown in SEQ ID NO: 1336, a homolog or an ortholog of a different organism species, and the like. The hsa-miR-6769a-5p gene can be obtained by a method described in Ladewig E et al., 2012, Genome Res., Vol. 22, pp. 1634-1645. Also, "hsa-mir-6769a" (miRBase Accession No. MI0022614; SEQ ID NO: 1379) having a hairpin-like structure is known as a precursor of "hsa-miR-6769a-5p."

The term "hsa-miR-4532 gene" or "hsa-miR-4532" used herein includes the hsa-miR-4532 gene (miRBase Accession No. MIMAT0019071) shown in SEQ ID NO: 1337, a homolog or an ortholog of a different organism species, and the like. The hsa-miR-4532 gene can be obtained by a method described in Jima DD et al., 2010, Blood, Vol. 116, e118-e127. Also, "hsa-mir-4532" (miRBase Accession No. MI0016899; SEQ ID NO: 1380) having a hairpin-like structure is known as a precursor of "hsa-miR-4532."

The term "hsa-miR-365a-5p gene" or "hsa-miR-365a-5p" used herein includes the hsa-miR-365a-5p gene (miRBase Accession No. MIMAT0009199) shown in SEQ ID NO: 1338, a homolog or an ortholog of a different organism species, and the like. The hsa-miR-365a-5p gene can be obtained by a method described in Xie X et al., 2005, Nature, Vol. 434, pp. 338-345. Also, "hsa-mir-365a" (miRBase Accession No. MI0000767; SEQ ID NO: 1381) having a hairpin-like structure is known as a precursor of "hsa-miR-365a-5p."

The term "hsa-miR-1231 gene" or "hsa-miR-1231" used herein includes the hsa-miR-1231 gene (miRBase Accession No. MIMAT0005586) shown in SEQ ID NO: 1339, a homolog or an ortholog of a different organism species, and the like. The hsa-miR-1231 gene can be obtained by a method described in Berezikov E et al., 2007, Mol. Cell, Vol. 28, pp. 328-336. Also, "hsa-mir-1231" (miRBase Accession No. MI0006321; SEQ ID NO: 1382) having a hairpin-like structure is known as a precursor of "hsa-miR-1231."

The term "hsa-miR-1228-5p gene" or "hsa-miR-1228-5p" used herein includes the hsa-miR-1228-5p gene (miRBase Accession No. MIMAT0005582) shown in SEQ ID NO: 1340, a homolog or an ortholog of a different organism species, and the like. The hsa-miR-1228-5p gene can be obtained by a method described in Berezikov E et al., 2007, Mol. Cell, Vol. 28, pp. 328-336. Also, "hsa-mir-1228" (miRBase Accession No. MI0006318; SEQ ID NO: 1383) having a hairpin-like structure is known as a precursor of "hsa-miR-1228-5p."

The term "hsa-miR-4430 gene" or "hsa-miR-4430" used herein includes the hsa-miR-4430 gene (miRBase Accession No. MIMAT0018945) shown in SEQ ID NO: 1341, a homolog or an ortholog of a different organism species, and the like. The hsa-miR-4430 gene can be obtained by a method described in Jima DD et al., 2010, Blood, Vol. 116, e118-e127. Also, "hsa-mir-4430" (miRBase Accession No. MI0016769; SEQ ID NO: 1384) having a hairpin-like structure is known as a precursor of "hsa-miR-4430."

The term "hsa-miR-296-3p gene" or "hsa-miR-296-3p" used herein includes the hsa-miR-296-3p gene (miRBase Accession No. MIMAT0004679) shown in SEQ ID NO: 1342, a homolog or an ortholog of a different organism species, and the like. The hsa-miR-296-3p gene can be obtained by a method described in Houbaviy HB et al., 2003, Dev. Cell, Vol. 5, pp. 351-358. Also, "hsa-mir-296" (miRBase Accession No. MI0000747; SEQ ID NO: 1385) having a hairpin-like structure is known as a precursor of "hsa-miR-296-3p."

The term "hsa-miR-1237-5p gene" or "hsa-miR-1237-5p" used herein includes the hsa-miR-1237-5p gene (miRBase Accession No. MIMAT0022946) shown in SEQ ID NO: 1343, a homolog or an ortholog of a different organism species, and the like. The hsa-miR-1237-5p gene can be obtained by a method described in Berezikov E et al., 2007, Mol. Cell, Vol. 28, pp. 328-336. Also, "hsa-mir-1237" (miRBase Accession No. MI0006327; SEQ ID NO: 1386) having a hairpin-like structure is known as a precursor of "miR-1237-5p."

The term "hsa-miR-4466 gene" or "hsa-miR-4466" used herein includes the hsa-miR-4466 gene (miRBase Accession No. MIMAT0018993) shown in SEQ ID NO: 1344, a homolog or an ortholog of a different organism species, and the like. The hsa-miR-4466 gene can be obtained by a method described in Jima DD et al., 2010, Blood, Vol. 116, e118-e127. Also, "hsa-mir-4466" (miRBase Accession No. MI0016817; SEQ ID NO: 1387) having a hairpin-like structure is known as a precursor of "hsa-miR-4466."

The term "hsa-miR-6789-5p gene" or "hsa-miR-6789-5p" used herein includes the hsa-miR-6789-5p gene (miRBase Accession No. MIMAT0027478) shown in SEQ ID NO: 1345, a homolog or an ortholog of a different organism species, and the like. The hsa-miR-6789-5p gene can be obtained by a method described in Ladewig E et al., 2012, Genome Res., Vol. 22, pp. 1634-1645. Also, "hsa-mir-6789" (miRBase Accession No. MI0022634; SEQ ID NO: 1388) having a hairpin-like structure is known as a precursor of "hsa-miR-6789-5p."

The term "hsa-miR-4632-5p gene" or "hsa-miR-4632-5p" used herein includes the hsa-miR-4632-5p gene (miRBase Accession No. MIMAT0022977) shown in SEQ ID NO: 1346, a homolog or an ortholog of a different organism species, and the like. The hsa-miR-4632-5p gene can be obtained by a method described in Persson H et al., 2011, Cancer Res., Vol. 71, pp. 78-86. Also, "hsa-mir-4632" (miRBase Accession No. MI0017259; SEQ ID NO: 1389) having a hairpin-like structure is known as a precursor of "hsa-miR-4632-5p."

The term "hsa-miR-4745-5p gene" or "hsa-miR-4745-5p" used herein includes the hsa-miR-4745-5p gene (miRBase Accession No. MIMAT0019878) shown in SEQ ID NO: 1347, a homolog or an ortholog of a different organism species, and the like. The hsa-miR-4745-5p gene can be obtained by a method described in Persson H et al., 2011, Cancer Res., Vol. 71, pp. 78-86. Also, "hsa-mir-4745" (miRBase Accession No. MI0017384; SEQ ID NO: 1390) having a hairpin-like structure is known as a precursor of "hsa-miR-4745-5p."

The term "hsa-miR-4665-5p gene" or "hsa-miR-4665-5p" used herein includes the hsa-miR-4665-5p gene (miRBase Accession No. MIMAT0019739) shown in SEQ ID NO: 1348, a homolog or an ortholog of a different organism species, and the like. The hsa-miR-4665-5p gene can be obtained by a method described in Persson H et al., 2011, Cancer Res., Vol. 71, pp. 78-86. Also, "hsa-mir-4665" (miRBase Accession No. MI0017295; SEQ ID NO: 1391) having a hairpin-like structure is known as a precursor of "hsa-miR-4665-5p."

The term "hsa-miR-6807-5p gene" or "hsa-miR-6807-5p" used herein includes the hsa-miR-6807-5p gene (miRBase Accession No. MIMAT0027514) shown in SEQ ID NO: 1349, a homolog or an ortholog of a different organism species, and the like. The hsa-miR-6807-5p gene can be obtained by a method described in Ladewig E et al., 2012, Genome Res., Vol. 22, pp. 1634-1645. Also, "hsa-mir-6807" (miRBase Accession No. MI0022652; SEQ ID NO: 1392) having a hairpin-like structure is known as a precursor of "hsa-miR-6807-5p."

The term "hsa-miR-7114-5p gene" or "hsa-miR-7114-5p" used herein includes the hsa-miR-7114-5p gene (miRBase Accession No. MIMAT0028125) shown in SEQ ID NO: 1350, a homolog or an ortholog of a different organism species, and the like. The hsa-miR-7114-5p gene can be obtained by a method described in Ladewig E et al., 2012, Genome Res., Vol. 22, pp. 1634-1645. Also, "hsa-mir-7114" (miRBase Accession No. MI0022965; SEQ ID NO: 1393) having a hairpin-like structure is known as a precursor of "hsa-miR-7114-5p."

The term "hsa-miR-150-3p gene" or "hsa-miR-150-3p" used herein includes the hsa-miR-150-3p gene (miRBase Accession No. MIMAT0004610) shown in SEQ ID NO: 1351, a homolog or an ortholog of a different organism species, and the like. The hsa-miR-150-3p gene can be obtained by a method described in Lagos-Quintana M et al., 2002, Curr. Biol., Vol. 12, pp. 735-739. Also, "hsa-mir-150" (miRBase Accession No. MI0000479; SEQ ID NO: 1394) having a hairpin-like structure is known as a precursor of "hsa-miR-150-3p."

The term "hsa-miR-423-5p gene" or "hsa-miR-423-5p" used herein includes the hsa-miR-423-5p gene (miRBase Accession No. MIMAT0004748) shown in SEQ ID NO: 1352, a homolog or an ortholog of a different organism species, and the like. The hsa-miR-423-5p gene can be obtained by a method described in Kasashima K et al., 2004, Biochem. Biophys. Res. Commun., Vol. 322, pp. 403-410. Also, "hsa-mir-423" (miRBase Accession No. MI0001445; SEQ ID NO: 1395) having a hairpin-like structure is known as a precursor of "hsa-miR-423-5p."

The term "hsa-miR-575 gene" or "hsa-miR-575" used herein includes the hsa-miR-575 gene (miRBase Accession No. MIMAT0003240) shown in SEQ ID NO: 1353, a homolog or an ortholog of a different organism species, and the like. The hsa-miR-575 gene can be obtained by a method described in Cummins JM et al., 2006, Proc. Natl. Acad. Sci. U.S.A., Vol. 103, pp. 3687-3692. Also, "hsa-mir-575" (miRBase Accession No. MI0003582; SEQ ID NO: 1396) having a hairpin-like structure is known as a precursor of "hsa-miR-575."

The term "hsa-miR-671-5p gene" or "hsa-miR-671-5p" used herein includes the hsa-miR-671-5p gene (miRBase Accession No. MIMAT0003880) shown in SEQ ID NO: 1354, a homolog or an ortholog of a different organism species, and the like. The hsa-miR-671-5p gene can be obtained by a method described in Berezikov E et al., 2006, Genome Res., Vol. 16, pp. 1289-1298. Also, "hsa-mir-671" (miRBase Accession No. MI0003760; SEQ ID NO: 1397) having a hairpin-like structure is known as a precursor of "hsa-miR-671-5p."

The term "hsa-miR-939-5p gene" or "hsa-miR-939-5p" used herein includes the hsa-miR-939-5p gene (miRBase Accession No. MIMAT0004982) shown in SEQ ID NO: 1355, a homolog or an ortholog of a different organism species, and the like. The hsa-miR-939-5p gene can be obtained by a method described in Lui WO et al., 2007, Cancer Res., Vol. 67, pp. 6031-6043. Also, "hsa-mir-939" (miRBase Accession No. MI0005761; SEQ ID NO: 1398) having a hairpin-like structure is known as a precursor of "hsa-miR-939-5p."

The term "hsa-miR-3665 gene" or "hsa-miR-3665" used herein includes the hsa-miR-3665 gene (miRBase Accession No. MIMAT0018087) shown in SEQ ID NO: 1356, a homolog or an ortholog of a different organism species, and the like. The hsa-miR-3665 gene can be obtained by a method described in Xie X et al., 2005, Nature, Vol. 434, pp. 338-345. Also, "hsa-mir-3665" (miRBase Accession No. MI0016066; SEQ ID NO: 1399) having a hairpin-like structure is known as a precursor of "hsa-miR-3665."

The term "hsa-miR-516a-5p gene" or "hsa-miR-516a-5p" used herein includes the hsa-miR-516a-5p gene (miRBase Accession No. MIMAT0004770) shown in SEQ ID NO: 1435, a homolog or an ortholog of a different organism species, and the like. The hsa-miR-516a-5p gene can be obtained by a method described in Bentwich I et al., 2005, Nat. Genet., Vol. 37, pp. 766-770. Also, "hsa-miR-516a-1" (miRBase Accession No. MI0003180; SEQ ID NO: 1454) having a hairpin-like structure is known as a precursor of "hsa-miR-516a-5p."

The term "hsa-miR-769-3p gene" or "hsa-miR-769-3p" used herein includes the hsa-miR-769-3p gene (miRBase Accession No. MIMAT0003887) shown in SEQ ID NO: 1436, a homolog or an ortholog of a different organism species, and the like. The hsa-miR-769-3p gene can be obtained by a method described in Berezikov E et al., 2006, Genome Res., Vol. 16, pp. 1289-1298. Also, "hsa-miR-769-3p" (miRBase Accession No. MI0003834; SEQ ID NO: 1465) having a hairpin-like structure is known as a precursor of "hsa-miR-769-3p."

The term "hsa-miR-3692-5p gene" or "hsa-miR-3692-5p" used herein includes the hsa-miR-3692-5p gene (miRBase Accession No. MIMAT0018121) shown in SEQ ID NO: 1437, a homolog or an ortholog of a different organism species, and the like. The hsa-miR-3692-5p gene can be obtained by a method described in Vaz C et al., 2010, BMC Genomics, Vol. 11, p. 288. Also, "hsa-miR-3692-5p" (miRBase Accession No. MI0016093; SEQ ID NO: 1456) having a hairpin-like structure is known as a precursor of "hsa-miR-3692-5p."

The term "hsa-miR-3945 gene" or "hsa-miR-3945" used herein includes the hsa-miR-3945 gene (miRBase Accession No. MIMAT0018361) shown in SEQ ID NO: 1438, a homolog or an ortholog of a different organism species, and the like. The hsa-miR-3945 gene can be obtained by a method described in Liao JY et al., 2010, PLoS One, Vol. 5, e10563. Also, "hsa-miR-3945" (miRBase Accession No. MI0016602; SEQ ID NO: 1457) having a hairpin-like structure is known as a precursor of "hsa-miR-3945."

The term "hsa-miR-4433a-3p gene" or "hsa-miR-4433a-3p" used herein includes the hsa-miR-4433a-3p gene (miRBase Accession No. MIMAT0018949) shown in SEQ ID NO: 1439, a homolog or an ortholog of a different organism species, and the like. The hsa-miR-4433a-3p gene can be obtained by a method described in Jima DD et al., 2010, Blood, Vol. 116, e118-e127. Also, "hsa-miR-4433a-3p" (miRBase Accession No. MI0016773; SEQ ID NO: 1458) having a hairpin-like structure is known as a precursor of "hsa-miR-4433a-3p."

The term "hsa-miR-4485-3p gene" or "hsa-miR-4485-3p" used herein includes the hsa-miR-4485-3p gene (miRBase Accession No. MIMAT0019019) shown in SEQ ID NO: 1440, a homolog or an ortholog of a different organism species, and the like. The hsa-miR-4485-3p gene can be obtained by a method described in Jima DD et al., 2010, Blood, Vol. 116, e118-e127. Also, "hsa-miR-4485-3p" (miRBase Accession No. MI0016846; SEQ ID NO: 1459) having a hairpin-like structure is known as a precursor of "hsa-miR-4485-3p."

The term "hsa-miR-6831-5p gene" or "hsa-miR-6831-5p" used herein includes the hsa-miR-6831-5p gene (miRBase Accession No. MIMAT0027562) shown in SEQ ID NO: 1441, a homolog or an ortholog of a different organism species, and the like. The hsa-miR-6831-5p gene can be obtained by a method described in Ladewig E et al., 2012, Genome Res., Vol. 22, pp. 1634-1645. Also, "hsa-miR-6831-5p" (miRBase Accession No. MI0022676; SEQ ID NO: 1460) having a hairpin-like structure is known as a precursor of "hsa-miR-6831-5p."

The term "hsa-miR-519c-5p gene" or "hsa-miR-519c-5p" used herein includes the hsa-miR-519c-5p gene (miRBase Accession No. MIMAT0002831) shown in SEQ ID NO: 1442, a homolog or an ortholog of a different organism species, and the like. The hsa-miR-519c-5p gene can be obtained by a method described in Bentwich I et al., 2005, Nat. Genet., Vol. 37, pp. 766-770. Also, "hsa-miR-519c-5p" (miRBase Accession No. MI0003148; SEQ ID NO: 1461) having a hairpin-like structure is known as a precursor of "hsa-miR-519c-5p."

The term "hsa-miR-551b-5p gene" or "hsa-miR-551b-5p" used herein includes the hsa-miR-551b-5p gene (miRBase Accession No. MIMAT0004794) shown in SEQ ID NO: 1443, a homolog or an ortholog of a different organism species, and the like. The hsa-miR-551b-5p gene can be obtained by a method described in Cummins JM et al., 2006, Proc. Natl. Acad. Sci. U.S.A., Vol. 103, pp. 3687-3692. Also, "hsa-miR-551b-5p" (miRBase Accession No. MI0003575; SEQ ID NO: 1462) having a hairpin-like structure is known as a precursor of "hsa-miR-551b-5p."

The term "hsa-miR-1343-3p gene" or "hsa-miR-1343-3p" used herein includes the hsa-miR-1343-3p gene (miRBase Accession No. MIMAT0019776) shown in SEQ ID NO: 1444, a homolog or an ortholog of a different organism species, and the like. The hsa-miR-1343-3p gene can be obtained by a method described in Persson H et al., 2011, Cancer Res., Vol. 71, pp. 78-86. Also, "hsa-miR-1343-3p" (miRBase Accession No. MI0017320; SEQ ID NO: 1463) having a hairpin-like structure is known as a precursor of "hsa-miR-1343-3p."

The term "hsa-miR-4286 gene" or "hsa-miR-4286" used herein includes the hsa-miR-4286 gene (miRBase Accession No. MIMAT0016916) shown in SEQ ID NO: 1445, a homolog or an ortholog of a different organism species, and the like. The hsa-miR-4286 gene can be obtained by a method described in Goff LA et al., 2009, PLoS One, Vol. 4, e7192. Also, "hsa-miR-4286" (miRBase Accession No. MI0015894; SEQ ID NO: 1464) having a hairpin-like structure is known as a precursor of "hsa-miR-4286."

The term "hsa-miR-4634 gene" or "hsa-miR-4634" used herein includes the hsa-miR-4634 gene (miRBase Accession No. MIMAT0019691) shown in SEQ ID NO: 1446, a homolog or an ortholog of a different organism species, and the like. The hsa-miR-4634 gene can be obtained by a method described in Persson H et al., 2011, Cancer Res., Vol. 71, pp. 78-86. Also, "hsa-miR-4634" (miRBase Accession No. MI0017261; SEQ ID NO: 1465) having a hairpin-like structure is known as a precursor of "hsa-miR-4634."

The term "hsa-miR-4733-3p gene" or "hsa-miR-4733-3p" used herein includes the hsa-miR-4733-3p gene (miRBase Accession No. MIMAT0019858) shown in SEQ ID NO: 1447, a homolog or an ortholog of a different organism species, and the like. The hsa-miR-4733-3p gene can be obtained by a method described in Persson H et al., 2011, Cancer Res., Vol. 71, pp. 78-86. Also, "hsa-miR-4733-3p" (miRBase Accession No. MI0017370; SEQ ID NO: 1466) having a hairpin-like structure is known as a precursor of "hsa-miR-4733-3p."

The term "hsa-miR-6086 gene" or "hsa-miR-6086" used herein includes the hsa-miR-6086 gene (miRBase Accession No. MIMAT0023711) shown in SEQ ID NO: 1448, a homolog or an ortholog of a different organism species, and the like. The hsa-miR-6086 gene can be obtained by a method described in Yoo JK et al., 2012, Stem Cells Dev., Vol. 21, pp. 2049-2057. Also, "hsa-miR-6086" (miRBase Accession No. MI0020363; SEQ ID NO: 1467) having a hairpin-like structure is known as a precursor of "hsa-miR-6086."

The term "hsa-miR-30d-5p gene" or "hsa-miR-30d-5p" used herein includes the hsa-miR-30d-5p gene (miRBase Accession No. MIMAT0000245) shown in SEQ ID NO: 1449, a homolog or an ortholog of a different organism species, and the like. The hsa-miR-30d-5p gene can be obtained by a method described in Lagos-Quintana M et al., 2002, Curr. Biol., Vol. 12, pp. 735-739. Also, "hsa-miR-30d-5p" (miRBase Accession No. MI0000255; SEQ ID NO: 1468) having a hairpin-like structure is known as a precursor of "hsa-miR-30d-5p."

The term "hsa-miR-30b-3p gene" or "hsa-miR-30b-3p" used herein includes the hsa-miR-30b-3p gene (miRBase Accession No. MIMAT0004589) shown in SEQ ID NO: 1450, a homolog or an ortholog of a different organism species, and the like. The hsa-miR-30b-3p gene can be obtained by a method described in Lagos-Quintana M et al., 2002, Curr. Biol., Vol. 12, pp. 735-739. Also, "hsa-miR-30b-3p" (miRBase Accession No. M10000441; SEQ ID NO: 1469) having a hairpin-like structure is known as a precursor of "hsa-miR-30b-3p."

The term "hsa-miR-92a-3p gene" or "hsa-miR-92a-3p" used herein includes the hsa-miR-92a-3p gene (miRBase Accession No. MIMAT0000092) shown in SEQ ID NO: 1451, a homolog or an ortholog of a different organism species, and the like. The hsa-miR-92a-3p gene can be obtained by a method described in Mourelatos Z et al., 2002, Genes Dev., Vol. 16, pp. 720-728. Also, "hsa-miR-92a-3p" (miRBase Accession No. MI0000093; SEQ ID NO: 1470) having a hairpin-like structure is known as a precursor of "hsa-miR-92a-3p."

The term "hsa-miR-371b-5p gene" or "hsa-miR-371b-5p" used herein includes the hsa-miR-371b-5p gene (miRBase Accession No. MIMAT0019892) shown in SEQ ID NO: 1452, a homolog or an ortholog of a different organism species, and the like. The hsa-miR-371b-5p gene can be obtained by a method described in Persson H et al., 2011, Cancer Res., Vol. 71, pp. 78-86. Also, "hsa-miR-371b-5p" (miRBase Accession No. MI0017393; SEQ ID NO: 1471) having a hairpin-like structure is known as a precursor of "hsa-miR-371b-5p."

The term "hsa-miR-486-5p gene" or "hsa-miR-486-5p" used herein includes the hsa-miR-486-5p gene (miRBase Accession No. MIMAT0002177) shown in SEQ ID NO: 1453, a homolog or an ortholog of a different organism species, and the like. The hsa-miR-486-5p gene can be obtained by a method described in Fu H et al., 2005, FEBS Lett., Vol. 579, pp. 3849-3854. Also, "hsa-miR-486-5p" (miRBase Accession No. MI0002470; SEQ ID NO: 1472) having a hairpin-like structure is known as a precursor of "hsa-miR-486-5p."

A mature miRNA may become a variant due to the sequence cleaved shorter or longer by one to several flanking nucleotides, or due to substitution of nucleotides, when cut out as the mature miRNA from its RNA precursor having a hairpin-like structure. This variant is called isomiR (Morin RD. et al., 2008, Genome Res., Vol. 18, p. 610-621). The miRBase (version 21) shows the nucleotide sequences represented by any of SEQ ID NOs: 1 to 210, 211 to 249, 250 to 374, and 375 to 390 as well as a large number of the nucleotide sequence variants and fragments represented by any of SEQ ID NOs: 783 to 1314, called isomiRs. These variants can also be obtained as miRNAs having a nucleotide sequence represented by any of SEQ ID NOs: 1 to 210, 211 to 249, 250 to 374, and 375 to 390. Specifically, among the variants of polynucleotides consisting of the nucleotide sequence represented by any of SEQ ID NOs: 3, 4, 5, 6, 7, 8, 10, 11, 12, 13, 15, 16, 17, 19, 22, 23, 25, 26, 28, 29, 30, 31, 32, 33, 34, 35, 36, 37, 39, 40, 41, 44, 47, 48, 49, 50, 51, 52, 54, 56, 57, 59, 60, 61, 62, 63, 64, 65, 66, 67, 68, 69, 70, 71, 72, 73, 75, 77, 78, 79, 81, 82, 83, 84, 85, 86, 87, 89, 91, 94, 95, 96, 98, 99, 100, 101, 102, 103, 105, 107, 109, 111, 112, 113, 114, 115, 116, 118, 119, 121, 122, 125, 126, 127, 128, 129, 130, 133, 134, 135, 136, 137, 138, 139, 141, 142, 143, 144, 145, 147, 148, 149, 150, 151, 152, 153, 154, 157, 158, 159, 161, 162, 163, 164, 165, 166, 167, 168, 169, 170, 171, 172, 173, 175, 177, 179, 180, 181, 182, 185, 187, 189, 190, 191, 192, 194, 195, 199, 202, 203, 205, 206, 208, 209, 213, 214, 215, 216, 217, 218, 219, 220, 221, 222, 223, 224, 225, 226, 227, 228, 229, 230, 231, 232, 233, 234, 235, 236, 237, 238, 239, 241, 242, 243, 244, 245, 246, 247, 248, 249, 251, 252, 253, 255, 256, 257, 259, 260, 261, 262, 263, 264, 265, 268, 269, 270, 271, 272, 273, 274, 275, 276, 277, 278, 279, 280, 281, 282, 283, 284, 286, 287, 288, 289, 290, 291, 292, 293, 295, 296, 297, 298, 299, 300, 301, 302, 303, 304, 305, 306, 307, 308, 309, 310, 311, 312, 313, 314, 315, 316, 317, 320, 321, 322, 323, 324, 325, 326, 327, 328, 329, 330, 331, 332, 333, 335, 338, 340, 341, 342, 343, 345, 347, 348, 349, 350, 352, 353, 354, 355, 356, 357, 359, 361, 362, 363, 367, 368, 369, 371, 372, 373, 374, 375, 376, 377, 378, 379, 380, 381, 382, 383, 385, 386, 388, 389, and 390 or a nucleotide sequence derived from the nucleotide sequence by the replacement of u with t according to the present invention, polynucleotides represented by SEQ ID NOs:

241 and 243 are included as the longest variants registered in miRBase (version 21),. polynucleotides having sequences represented by SEQ ID NOs: 4, 25, 26, 68, 69, 70, 83, 91, 96, 99, 114, 135, 141, 182, 199, 202, 205, 206, 208, 213, 218, 235, 350, and 386 are also included as the shortest variants, in the same manner. In addition to these variants and fragments, a large number of isomiR polynucleotides of SEQ ID NOs: 1 to 210, 211 to 249, 250 to 374, and 375 to 390 registered in the miRBase are included. Further, polynucleotides represented by any of SEQ ID NOs: 391 to 782, which are their respective precursors are included as polynucleotides comprising nucleotide sequences represented by any of SEQ ID NOs: 1 to 210, 211 to 249, 250 to 374, and 375 to 390.

The miRBase (version 21) shows the nucleotide sequences represented by any of SEQ ID NOs: 194 to 210, 374, and 1315 to 1350, and 211 to 212 and 1351 to 1356 as well as a large number of the nucleotide sequence variants and fragments represented by any of SEQ ID NOs: 1015 to 1017, 1019 to 1024, 1026 to 1031, 1285 to 1286, and 1400 to 1434, called isomiRs. These variants can also be obtained as miRNAs having a nucleotide sequence represented by any of SEQ ID NOs: 194 to 210, 374, and 1315 to 1350, and 211 to 212 and 1351 to 1356. Specifically, among the variants of polynucleotides consisting of the nucleotide sequence represented by any of SEQ ID NOs: 194, 195, 1320, 1322, 199, 1328, 1329, 202, 1333, 1334, 203, 1337, 1338, 205, 206, 1340, 374, 1341, 1342, 1343, 208, 209, 1344, 1346, 1347, 1348, 1351, 1352, 1354, 1355, and 1356 or a nucleotide sequence derived from the nucleotide sequence by the replacement of u with t according to the present invention, , a polynucleotide represented by SEQ ID NO: 1427 are included as the longest variants registered in miRBase (version 21). polynucleotides having sequences represented by SEQ ID NOs: 1017, 1020, 1405, 1022, 1407, 1409, 1411, 1024, 1027, 1415, 1417, 1418, 1419, 1029, 1421, 1428, 1430, 1432, and 1434 are also included as the shortest variants in the same manner. In addition to these variants and fragments, a large number of isomiR polynucleotides of SEQ ID NOs: 194 to 210, 374, and 1315 to 1350, and 211 to 212 and 1351 to 1356 registered in the miRBase are included. Further, polynucleotides represented by any of SEQ ID NOs: 398, 490, 591, 593 to 609, 766, and 1357 to 1399, which are their respective precursors are included as the polynucleotides comprising nucleotide sequences represented by any of SEQ ID NOs: 194 to 210, 374, and 1315 to 1350, and 211 to 212 and 1351 to 1356.

In addition, the miRBase (version 21) shows the nucleotide sequences represented by SEQ ID NOs: 1435 to 1448 and 1449 to 1453 as well as a large number of the nucleotide sequence variants and fragments represented by any of SEQ ID NOs: 1473 to 1505, called isomiRs. These variants can also be obtained as miRNAs having a nucleotide sequence represented by any of SEQ ID NOs: 1435 to 1448 and 1449 to 1453. Specifically, among the variants of polynucleotides consisting of the nucleotide sequence represented by any of SEQ ID NOs: 1435, 1436, 1437, 1438, 1439, 1440, 1441, 1442, 1443, 1444, 1445, 1447, 1449, 1450, 1451, 1452, and 1453 or a nucleotide sequence derived from the nucleotide sequence by the replacement of u with t according to the present invention, a polynucleotide represented by SEQ ID NO: 1496 is included as the longest variant registered in miRBase (version 21), Also, polynucleotides having sequences represented by SEQ ID NOs: 1494, 1501, and 1505 also included as the shortest variants in the same manner. In addition to these variants and fragments, a large number of isomiR polynucleotides of SEQ ID NOs: 1435 to 1448 and 1449 to 1453 registered in the miRBase are included. Further, polynucleotides represented by any of SEQ ID NOs: 1454 to 1467 and 1468 to 1472, which are their respective precursors are included as the polynucleotide comprising a nucleotide sequence represented by any of SEQ ID NOs: 1435 to 1448 and 1449 to 1453.

The names and miRBase Accession Nos. (registration numbers) of the genes represented by SEQ ID NOs: 1 to 1314 and 1435 to 1505 are shown in Table 1-1.

In addition, the names and miRBase Accession Nos. (registration numbers) of the genes represented by SEQ ID NOs: 1315 to 1434 are shown in Table 1-2.

As used herein, the term "capable of specifically binding" means that the nucleic acid probe or the primer used in the present invention binds to a specific target nucleic acid and cannot substantially bind to other nucleic acids.

[Table 1-1]

| SEQ ID NO | Name of gene | Accession No. of miRBase |
|---|---|---|
| 1 | hsa-miR-4274 | MIMAT0016906 |
| 2 | hsa-miR-4272 | MIMAT0016902 |
| 3 | hsa-miR-4728-5p | MIMAT0019849 |
| 4 | hsa-miR-4443 | MIMAT0018961 |
| 5 | hsa-miR-4506 | MIMAT0019042 |
| 6 | hsa-miR-6773-5p | MIMAT0027446 |
| 7 | hsa-miR-4662a-5p | MIMAT0019731 |
| 8 | hsa-miR-3184-3p | MIMAT0022731 |
| 9 | hsa-miR-4281 | MIMAT0016907 |

(continued)

| SEQ ID NO | Name of gene | Accession No. of miRBase |
|---|---|---|
| 10 | hsa-miR-320d | MIMAT0006764 |
| 11 | hsa-miR-6729-3p | MIMAT0027360 |
| 12 | hsa-miR-5192 | MIMAT0021123 |
| 13 | hsa-miR-6853-5p | MIMAT0027606 |
| 14 | hsa-miR-1234-3p | MIMAT0005589 |
| 15 | hsa-miR-1233-3p | MIMAT0005588 |
| 16 | hsa-miR-4539 | MIMAT0019082 |
| 17 | hsa-miR-3914 | MIMAT0018188 |
| 18 | hsa-miR-4738-5p | MIMAT0019866 |
| 19 | hsa-miR-548au-3p | MIMAT0022292 |
| 20 | hsa-miR-1539 | MIMAT0007401 |
| 21 | hsa-miR-4720-3p | MIMAT0019834 |
| 22 | hsa-miR-365b-5p | MIMAT0022833 |
| 23 | hsa-miR-4486 | MIMAT0019020 |
| 24 | hsa-miR-1227-5p | MIMAT0022941 |
| 25 | hsa-miR-4667-5p | MIMAT0019743 |
| 26 | hsa-miR-6088 | MIMAT0023713 |
| 27 | hsa-miR-6820-5p | MIMAT0027540 |
| 28 | hsa-miR-4505 | MIMAT0019041 |
| 29 | hsa-miR-548q | MIMAT0011163 |
| 30 | hsa-miR-4658 | MIMAT0019725 |
| 31 | hsa-miR-450a-5p | MIMAT0001545 |
| 32 | hsa-miR-1260b | MIMAT0015041 |
| 33 | hsa-miR-3677-5p | MIMAT0019221 |
| 34 | hsa-miR-6777-3p | MIMAT0027455 |
| 35 | hsa-miR-6826-3p | MIMAT0027553 |
| 36 | hsa-miR-6832-3p | MIMAT0027565 |
| 37 | hsa-miR-4725-3p | MIMAT0019844 |
| 38 | hsa-miR-7161-3p | MIMAT0028233 |
| 39 | hsa-miR-2277-5p | MIMAT0017352 |
| 40 | hsa-miR-7110-3p | MIMAT0028118 |
| 41 | hsa-miR-4312 | MIMAT0016864 |
| 42 | hsa-miR-4461 | MIMAT0018983 |
| 43 | hsa-miR-6766-5p | MIMAT0027432 |
| 44 | hsa-miR-1266-3p | MIMAT0026742 |
| 45 | hsa-miR-6729-5p | MIMAT0027359 |
| 46 | hsa-miR-526b-3p | MIMAT0002836 |
| 47 | hsa-miR-519e-5p | MIMAT0002828 |
| 48 | hsa-miR-512-5p | MIMAT0002822 |

(continued)

| SEQ ID NO | Name of gene | Accession No. of miRBase |
|---|---|---|
| 49 | hsa-miR-5088-5p | MIMAT0021080 |
| 50 | hsa-miR-1909-3p | MIMAT0007883 |
| 51 | hsa-miR-6511a-5p | MIMAT0025478 |
| 52 | hsa-miR-4734 | MIMAT0019859 |
| 53 | hsa-miR-936 | MIMAT0004979 |
| 54 | hsa-miR-1249-3p | MIMAT0005901 |
| 55 | hsa-miR-6777-5p | MIMAT0027454 |
| 56 | hsa-miR-4487 | MIMAT0019021 |
| 57 | hsa-miR-3155a | MIMAT0015029 |
| 58 | hsa-miR-563 | MIMAT0003227 |
| 59 | hsa-miR-4741 | MIMAT0019871 |
| 60 | hsa-miR-6788-5p | MIMAT0027476 |
| 61 | hsa-miR-4433b-5p | MIMAT0030413 |
| 62 | hsa-miR-323a-5p | MIMAT0004696 |
| 63 | hsa-miR-6811-5p | MIMAT0027522 |
| 64 | hsa-miR-6721-5p | MIMAT0025852 |
| 65 | hsa-miR-5004-5p | MIMAT0021027 |
| 66 | hsa-miR-6509-3p | MIMAT0025475 |
| 67 | hsa-miR-648 | MIMAT0003318 |
| 68 | hsa-miR-3917 | MIMAT0018191 |
| 69 | hsa-miR-6087 | MIMAT0023712 |
| 70 | hsa-miR-1470 | MIMAT0007348 |
| 71 | hsa-miR-586 | MIMAT0003252 |
| 72 | hsa-miR-3150a-5p | MIMAT0019206 |
| 73 | hsa-miR-105-3p | MIMAT0004516 |
| 74 | hsa-miR-7973 | MIMAT0031176 |
| 75 | hsa-miR-1914-5p | MIMAT0007889 |
| 76 | hsa-miR-4749-3p | MIMAT0019886 |
| 77 | hsa-miR-15b-5p | MIMAT0000417 |
| 78 | hsa-miR-1289 | MIMAT0005879 |
| 79 | hsa-miR-4433a-5p | MIMAT0020956 |
| 80 | hsa-miR-3666 | MIMAT0018088 |
| 81 | hsa-miR-3186-3p | MIMAT0015068 |
| 82 | hsa-miR-4725-5p | MIMAT0019843 |
| 83 | hsa-miR-4488 | MIMAT0019022 |
| 84 | hsa-miR-4474-3p | MIMAT0019001 |
| 85 | hsa-miR-6731-3p | MIMAT0027364 |
| 86 | hsa-miR-4640-3p | MIMAT0019700 |
| 87 | hsa-miR-202-5p | MIMAT0002810 |

(continued)

| SEQ ID NO | Name of gene | Accession No. of miRBase |
|---|---|---|
| 88 | hsa-miR-6816-5p | MIMAT0027532 |
| 89 | hsa-miR-638 | MIMAT0003308 |
| 90 | hsa-miR-6821-5p | MIMAT0027542 |
| 91 | hsa-miR-1247-3p | MIMAT0022721 |
| 92 | hsa-miR-6765-5p | MIMAT0027430 |
| 93 | hsa-miR-6800-5p | MIMAT0027500 |
| 94 | hsa-miR-3928-3p | MIMAT0018205 |
| 95 | hsa-miR-3940-5p | MIMAT0019229 |
| 96 | hsa-miR-3960 | MIMAT0019337 |
| 97 | hsa-miR-6775-5p | MIMAT0027450 |
| 98 | hsa-miR-3178 | MIMAT0015055 |
| 99 | hsa-miR-1202 | MIMAT0005865 |
| 100 | hsa-miR-6790-5p | MIMAT0027480 |
| 101 | hsa-miR-4731-3p | MIMAT0019854 |
| 102 | hsa-miR-2681-3p | MIMAT0013516 |
| 103 | hsa-miR-6758-5p | MIMAT0027416 |
| 104 | hsa-miR-8072 | MIMAT0030999 |
| 105 | hsa-miR-518d-3p | MIMAT0002864 |
| 106 | hsa-miR-3606-3p | MIMAT0022965 |
| 107 | hsa-miR-4800-5p | MIMAT0019978 |
| 108 | hsa-miR-1292-3p | MIMAT0022948 |
| 109 | hsa-miR-6784-3p | MIMAT0027469 |
| 110 | hsa-miR-4450 | MIMAT0018971 |
| 111 | hsa-miR-6132 | MIMAT0024616 |
| 112 | hsa-miR-4716-5p | MIMAT0019826 |
| 113 | hsa-miR-6860 | MIMAT0027622 |
| 114 | hsa-miR-1268b | MIMAT0018925 |
| 115 | hsa-miR-378d | MIMAT0018926 |
| 116 | hsa-miR-4701-5p | MIMAT0019798 |
| 117 | hsa-miR-4329 | MIMAT0016923 |
| 118 | hsa-miR-185-3p | MIMAT0004611 |
| 119 | hsa-miR-552-3p | MIMAT0003215 |
| 120 | hsa-miR-1273g-5p | MIMAT0020602 |
| 121 | hsa-miR-6769b-3p | MIMAT0027621 |
| 122 | hsa-miR-520a-3p | MIMAT0002834 |
| 123 | hsa-miR-4524b-5p | MIMAT0022255 |
| 124 | hsa-miR-4291 | MIMAT0016922 |
| 125 | hsa-miR-6734-3p | MIMAT0027370 |
| 126 | hsa-miR-143-5p | MIMAT0004599 |

(continued)

| SEQ ID NO | Name of gene | Accession No. of miRBase |
|---|---|---|
| 127 | hsa-miR-939-3p | MIMAT0022939 |
| 128 | hsa-miR-6889-3p | MIMAT0027679 |
| 129 | hsa-miR-6842-3p | MIMAT0027587 |
| 130 | hsa-miR-4511 | MIMAT0019048 |
| 131 | hsa-miR-4318 | MIMAT0016869 |
| 132 | hsa-miR-4653-5p | MIMAT0019718 |
| 133 | hsa-miR-6867-3p | MIMAT0027635 |
| 134 | hsa-miR-133b | MIMAT0000770 |
| 135 | hsa-miR-3196 | MIMAT0015080 |
| 136 | hsa-miR-193b-3p | MIMAT0002819 |
| 137 | hsa-miR-3162-3p | MIMAT0019213 |
| 138 | hsa-miR-6819-3p | MIMAT0027539 |
| 139 | hsa-miR-1908-3p | MIMAT0026916 |
| 140 | hsa-miR-6786-5p | MIMAT0027472 |
| 141 | hsa-miR-3648 | MIMAT0018068 |
| 142 | hsa-miR-4513 | MIMAT0019050 |
| 143 | hsa-miR-3652 | MIMAT0018072 |
| 144 | hsa-miR-4640-5p | MIMAT0019699 |
| 145 | hsa-miR-6871-5p | MIMAT0027642 |
| 146 | hsa-miR-7845-5p | MIMAT0030420 |
| 147 | hsa-miR-3138 | MIMAT0015006 |
| 148 | hsa-miR-6884-5p | MIMAT0027668 |
| 149 | hsa-miR-4653-3p | MIMAT0019719 |
| 150 | hsa-miR-636 | MIMAT0003306 |
| 151 | hsa-miR-4652-3p | MIMAT0019717 |
| 152 | hsa-miR-6823-5p | MIMAT0027546 |
| 153 | hsa-miR-4502 | MIMAT0019038 |
| 154 | hsa-miR-7113-5p | MIMAT0028123 |
| 155 | hsa-miR-8087 | MIMAT0031014 |
| 156 | hsa-miR-7154-3p | MIMAT0028219 |
| 157 | hsa-miR-5189-5p | MIMAT0021120 |
| 158 | hsa-miR-1253 | MIMAT0005904 |
| 159 | hsa-miR-518c-5p | MIMAT0002847 |
| 160 | hsa-miR-7151-5p | MIMAT0028212 |
| 161 | hsa-miR-3614-3p | MIMAT0017993 |
| 162 | hsa-miR-4727-5p | MIMAT0019847 |
| 163 | hsa-miR-3682-5p | MIMAT0019222 |
| 164 | hsa-miR-5090 | MIMAT0021082 |
| 165 | hsa-miR-337-3p | MIMAT0000754 |

(continued)

| SEQ ID NO | Name of gene | Accession No. of miRBase |
|---|---|---|
| 166 | hsa-miR-488-5p | MIMAT0002804 |
| 167 | hsa-miR-100-5p | MIMAT0000098 |
| 168 | hsa-miR-4520-3p | MIMAT0019057 |
| 169 | hsa-miR-373-3p | MIMAT0000726 |
| 170 | hsa-miR-6499-5p | MIMAT0025450 |
| 171 | hsa-miR-3909 | MIMAT0018183 |
| 172 | hsa-miR-32-5p | MIMAT0000090 |
| 173 | hsa-miR-302a-3p | MIMAT0000684 |
| 174 | hsa-miR-4686 | MIMAT0019773 |
| 175 | hsa-miR-4659a-3p | MIMAT0019727 |
| 176 | hsa-miR-4287 | MIMAT0016917 |
| 177 | hsa-miR-1301-5p | MIMAT0026639 |
| 178 | hsa-miR-593-3p | MIMAT0004802 |
| 179 | hsa-miR-517a-3p | MIMAT0002852 |
| 180 | hsa-miR-517b-3p | MIMAT0002857 |
| 181 | hsa-miR-142-3p | MIMAT0000434 |
| 182 | hsa-miR-1185-2-3p | MIMAT0022713 |
| 183 | hsa-miR-602 | MIMAT0003270 |
| 184 | hsa-miR-527 | MIMAT0002862 |
| 185 | hsa-miR-518a-5p | MIMAT0005457 |
| 186 | hsa-miR-4682 | MIMAT0019767 |
| 187 | hsa-miR-28-5p | MIMAT0000085 |
| 188 | hsa-miR-4252 | MIMAT0016886 |
| 189 | hsa-miR-452-5p | MIMAT0001635 |
| 190 | hsa-miR-525-5p | MIMAT0002838 |
| 191 | hsa-miR-3622a-3p | MIMAT0018004 |
| 192 | hsa-miR-6813-3p | MIMAT0027527 |
| 193 | hsa-miR-4769-3p | MIMAT0019923 |
| 194 | hsa-miR-5698 | MIMAT0022491 |
| 195 | hsa-miR-1915-3p | MIMAT0007892 |
| 196 | hsa-miR-1343-5p | MIMAT0027038 |
| 197 | hsa-miR-6861-5p | MIMAT0027623 |
| 198 | hsa-miR-6781-5p | MIMAT0027462 |
| 199 | hsa-miR-4508 | MIMAT0019045 |
| 200 | hsa-miR-6743-5p | MIMAT0027387 |
| 201 | hsa-miR-6726-5p | MIMAT0027353 |
| 202 | hsa-miR-4525 | MIMAT0019064 |
| 203 | hsa-miR-4651 | MIMAT0019715 |
| 204 | hsa-miR-6813-5p | MIMAT0027526 |

(continued)

| SEQ ID NO | Name of gene | Accession No. of miRBase |
|---|---|---|
| 205 | hsa-miR-5787 | MIMAT0023252 |
| 206 | hsa-miR-1290 | MIMAT0005880 |
| 207 | hsa-miR-6075 | MIMAT0023700 |
| 208 | hsa-miR-4758-5p | MIMAT0019903 |
| 209 | hsa-miR-4690-5p | MIMAT0019779 |
| 210 | hsa-miR-762 | MIMAT0010313 |
| 211 | hsa-miR-1225-3p | MIMAT0005573 |
| 212 | hsa-miR-3184-5p | MIMAT0015064 |
| 213 | hsa-miR-665 | MIMAT0004952 |
| 214 | hsa-miR-211-5p | MIMAT0000268 |
| 215 | hsa-miR-1247-5p | MIMAT0005899 |
| 216 | hsa-miR-3656 | MIMAT0018076 |
| 217 | hsa-miR-149-5p | MIMAT0000450 |
| 218 | hsa-miR-744-5p | MIMAT0004945 |
| 219 | hsa-miR-345-5p | MIMAT0000772 |
| 220 | hsa-miR-150-5p | MIMAT0000451 |
| 221 | hsa-miR-191-3p | MIMAT0001618 |
| 222 | hsa-miR-651-5p | MIMAT0003321 |
| 223 | hsa-miR-34a-5p | MIMAT0000255 |
| 224 | hsa-miR-409-5p | MIMAT0001638 |
| 225 | hsa-miR-369-5p | MIMAT0001621 |
| 226 | hsa-miR-1915-5p | MIMAT0007891 |
| 227 | hsa-miR-204-5p | MIMAT0000265 |
| 228 | hsa-miR-137 | MIMAT0000429 |
| 229 | hsa-miR-382-5p | MIMAT0000737 |
| 230 | hsa-miR-517-5p | MIMAT0002851 |
| 231 | hsa-miR-532-5p | MIMAT0002888 |
| 232 | hsa-miR-22-5p | MIMAT0004495 |
| 233 | hsa-miR-1237-3p | MIMAT0005592 |
| 234 | hsa-miR-1224-3p | MIMAT0005459 |
| 235 | hsa-miR-625-3p | MIMAT0004808 |
| 236 | hsa-miR-328-3p | MIMAT0000752 |
| 237 | hsa-miR-122-5p | MIMAT0000421 |
| 238 | hsa-miR-202-3p | MIMAT0002811 |
| 239 | hsa-miR-4781-5p | MIMAT0019942 |
| 240 | hsa-miR-718 | MIMAT0012735 |
| 241 | hsa-miR-342-3p | MIMAT0000753 |
| 242 | hsa-miR-26b-3p | MIMAT0004500 |
| 243 | hsa-miR-140-3p | MIMAT0004597 |

(continued)

| SEQ ID NO | Name of gene | Accession No. of miRBase |
|---|---|---|
| 244 | hsa-miR-200a-3p | MIMAT0000682 |
| 245 | hsa-miR-378a-3p | MIMAT0000732 |
| 246 | hsa-miR-484 | MIMAT0002174 |
| 247 | hsa-miR-296-5p | MIMAT0000690 |
| 248 | hsa-miR-205-5p | MIMAT0000266 |
| 249 | hsa-miR-431-5p | MIMAT0001625 |
| 250 | hsa-miR-1471 | MIMAT0007349 |
| 251 | hsa-miR-1538 | MIMAT0007400 |
| 252 | hsa-miR-449b-3p | MIMAT0009203 |
| 253 | hsa-miR-1976 | MIMAT0009451 |
| 254 | hsa-miR-4268 | MIMAT0016896 |
| 255 | hsa-miR-4279 | MIMAT0016909 |
| 256 | hsa-miR-3620-3p | MIMAT0018001 |
| 257 | hsa-miR-3944-3p | MIMAT0018360 |
| 258 | hsa-miR-3156-3p | MIMAT0019209 |
| 259 | hsa-miR-3187-5p | MIMAT0019216 |
| 260 | hsa-miR-4685-3p | MIMAT0019772 |
| 261 | hsa-miR-4695-3p | MIMAT0019789 |
| 262 | hsa-miR-4697-3p | MIMAT0019792 |
| 263 | hsa-miR-4713-5p | MIMAT0019820 |
| 264 | hsa-miR-4723-3p | MIMAT0019839 |
| 265 | hsa-miR-371b-3p | MIMAT0019893 |
| 266 | hsa-miR-3151-3p | MIMAT0027026 |
| 267 | hsa-miR-3192-3p | MIMAT0027027 |
| 268 | hsa-miR-6728-3p | MIMAT0027358 |
| 269 | hsa-miR-6736-3p | MIMAT0027374 |
| 270 | hsa-miR-6740-3p | MIMAT0027382 |
| 271 | hsa-miR-6741-3p | MIMAT0027384 |
| 272 | hsa-miR-6743-3p | MIMAT0027388 |
| 273 | hsa-miR-6747-3p | MIMAT0027395 |
| 274 | hsa-miR-6750-3p | MIMAT0027401 |
| 275 | hsa-miR-6754-3p | MIMAT0027409 |
| 276 | hsa-miR-6759-3p | MIMAT0027419 |
| 277 | hsa-miR-6761-3p | MIMAT0027423 |
| 278 | hsa-miR-6762-3p | MIMAT0027425 |
| 279 | hsa-miR-6769a-3p | MIMAT0027439 |
| 280 | hsa-miR-6776-3p | MIMAT0027453 |
| 281 | hsa-miR-6778-3p | MIMAT0027457 |
| 282 | hsa-miR-6779-3p | MIMAT0027459 |

(continued)

| SEQ ID NO | Name of gene | Accession No. of miRBase |
|---|---|---|
| 283 | hsa-miR-6786-3p | MIMAT0027473 |
| 284 | hsa-miR-6787-3p | MIMAT0027475 |
| 285 | hsa-miR-6792-3p | MIMAT0027485 |
| 286 | hsa-miR-6794-3p | MIMAT0027489 |
| 287 | hsa-miR-6801-3p | MIMAT0027503 |
| 288 | hsa-miR-6802-3p | MIMAT0027505 |
| 289 | hsa-miR-6803-3p | MIMAT0027507 |
| 290 | hsa-miR-6804-3p | MIMAT0027509 |
| 291 | hsa-miR-6810-5p | MIMAT0027520 |
| 292 | hsa-miR-6823-3p | MIMAT0027547 |
| 293 | hsa-miR-6825-3p | MIMAT0027551 |
| 294 | hsa-miR-6829-3p | MIMAT0027559 |
| 295 | hsa-miR-6833-3p | MIMAT0027567 |
| 296 | hsa-miR-6834-3p | MIMAT0027569 |
| 297 | hsa-miR-6780b-3p | MIMAT0027573 |
| 298 | hsa-miR-6845-3p | MIMAT0027591 |
| 299 | hsa-miR-6862-3p | MIMAT0027626 |
| 300 | hsa-miR-6865-3p | MIMAT0027631 |
| 301 | hsa-miR-6870-3p | MIMAT0027641 |
| 302 | hsa-miR-6875-3p | MIMAT0027651 |
| 303 | hsa-miR-6877-3p | MIMAT0027655 |
| 304 | hsa-miR-6879-3p | MIMAT0027659 |
| 305 | hsa-miR-6882-3p | MIMAT0027665 |
| 306 | hsa-miR-6885-3p | MIMAT0027671 |
| 307 | hsa-miR-6886-3p | MIMAT0027673 |
| 308 | hsa-miR-6887-3p | MIMAT0027675 |
| 309 | hsa-miR-6890-3p | MIMAT0027681 |
| 310 | hsa-miR-6893-3p | MIMAT0027687 |
| 311 | hsa-miR-6894-3p | MIMAT0027689 |
| 312 | hsa-miR-7106-3p | MIMAT0028110 |
| 313 | hsa-miR-7109-3p | MIMAT0028116 |
| 314 | hsa-miR-7114-3p | MIMAT0028126 |
| 315 | hsa-miR-7155-5p | MIMAT0028220 |
| 316 | hsa-miR-7160-5p | MIMAT0028230 |
| 317 | hsa-miR-615-3p | MIMAT0003283 |
| 318 | hsa-miR-920 | MIMAT0004970 |
| 319 | hsa-miR-1825 | MIMAT0006765 |
| 320 | hsa-miR-675-3p | MIMAT0006790 |
| 321 | hsa-miR-1910-5p | MIMAT0007884 |

(continued)

| SEQ ID NO | Name of gene | Accession No. of miRBase |
|---|---|---|
| 322 | hsa-miR-2278 | MIMAT0011778 |
| 323 | hsa-miR-2682-3p | MIMAT0013518 |
| 324 | hsa-miR-3122 | MIMAT0014984 |
| 325 | hsa-miR-3151-5p | MIMAT0015024 |
| 326 | hsa-miR-3175 | MIMAT0015052 |
| 327 | hsa-miR-4323 | MIMAT0016875 |
| 328 | hsa-miR-4326 | MIMAT0016888 |
| 329 | hsa-miR-4284 | MIMAT0016915 |
| 330 | hsa-miR-3605-3p | MIMAT0017982 |
| 331 | hsa-miR-3622b-5p | MIMAT0018005 |
| 332 | hsa-miR-3646 | MIMAT0018065 |
| 333 | hsa-miR-3158-5p | MIMAT0019211 |
| 334 | hsa-miR-4722-3p | MIMAT0019837 |
| 335 | hsa-miR-4728-3p | MIMAT0019850 |
| 336 | hsa-miR-4747-3p | MIMAT0019883 |
| 337 | hsa-miR-4436b-5p | MIMAT0019940 |
| 338 | hsa-miR-5196-3p | MIMAT0021129 |
| 339 | hsa-miR-5589-5p | MIMAT0022297 |
| 340 | hsa-miR-345-3p | MIMAT0022698 |
| 341 | hsa-miR-642b-5p | MIMAT0022736 |
| 342 | hsa-miR-6716-3p | MIMAT0025845 |
| 343 | hsa-miR-6511b-3p | MIMAT0025848 |
| 344 | hsa-miR-208a-5p | MIMAT0026474 |
| 345 | hsa-miR-6726-3p | MIMAT0027354 |
| 346 | hsa-miR-6744-5p | MIMAT0027389 |
| 347 | hsa-miR-6782-3p | MIMAT0027465 |
| 348 | hsa-miR-6789-3p | MIMAT0027479 |
| 349 | hsa-miR-6797-3p | MIMAT0027495 |
| 350 | hsa-miR-6800-3p | MIMAT0027501 |
| 351 | hsa-miR-6806-5p | MIMAT0027512 |
| 352 | hsa-miR-6824-3p | MIMAT0027549 |
| 353 | hsa-miR-6837-5p | MIMAT0027576 |
| 354 | hsa-miR-6846-3p | MIMAT0027593 |
| 355 | hsa-miR-6858-3p | MIMAT0027617 |
| 356 | hsa-miR-6859-3p | MIMAT0027619 |
| 357 | hsa-miR-6861-3p | MIMAT0027624 |
| 358 | hsa-miR-6880-3p | MIMAT0027661 |
| 359 | hsa-miR-7111-3p | MIMAT0028120 |
| 360 | hsa-miR-7152-5p | MIMAT0028214 |

(continued)

| SEQ ID NO | Name of gene | Accession No. of miRBase |
|---|---|---|
| 361 | hsa-miR-642a-5p | MIMAT0003312 |
| 362 | hsa-miR-657 | MIMAT0003335 |
| 363 | hsa-miR-1236-3p | MIMAT0005591 |
| 364 | hsa-miR-764 | MIMAT0010367 |
| 365 | hsa-miR-4314 | MIMAT0016868 |
| 366 | hsa-miR-3675-3p | MIMAT0018099 |
| 367 | hsa-miR-5703 | MIMAT0022496 |
| 368 | hsa-miR-3191-5p | MIMAT0022732 |
| 369 | hsa-miR-6511a-3p | MIMAT0025479 |
| 370 | hsa-miR-6809-3p | MIMAT0027519 |
| 371 | hsa-miR-6815-5p | MIMAT0027530 |
| 372 | hsa-miR-6857-3p | MIMAT0027615 |
| 373 | hsa-miR-6878-3p | MIMAT0027657 |
| 374 | hsa-miR-371a-5p | MIMAT0004687 |
| 375 | hsa-miR-766-3p | MIMAT0003888 |
| 376 | hsa-miR-1229-3p | MIMAT0005584 |
| 377 | hsa-miR-1306-5p | MIMAT0022726 |
| 378 | hsa-miR-210-5p | MIMAT0026475 |
| 379 | hsa-miR-198 | MIMAT0000228 |
| 380 | hsa-miR-485-3p | MIMAT0002176 |
| 381 | hsa-miR-668-3p | MIMAT0003881 |
| 382 | hsa-miR-532-3p | MIMAT0004780 |
| 383 | hsa-miR-877-3p | MIMAT0004950 |
| 384 | hsa-miR-1238-3p | MIMAT0005593 |
| 385 | hsa-miR-3130-5p | MIMAT0014995 |
| 386 | hsa-miR-4298 | MIMAT0016852 |
| 387 | hsa-miR-4290 | MIMAT0016921 |
| 388 | hsa-miR-3943 | MIMAT0018359 |
| 389 | hsa-miR-346 | MIMAT0000773 |
| 390 | hsa-miR-767-3p | MIMAT0003883 |
| 391 | hsa-mir-4274 | MI0015884 |
| 392 | hsa-mir-4272 | MI0015880 |
| 393 | hsa-mir-4728 | MI0017365 |
| 394 | hsa-mir-4443 | MI0016786 |
| 395 | hsa-mir-4506 | MI0016869 |
| 396 | hsa-mir-6773 | MI0022618 |
| 397 | hsa-mir-4662a | MI0017290 |
| 398 | hsa-mir-3184 | MI0014226 |
| 399 | hsa-mir-4281 | MI0015885 |

(continued)

| SEQ ID NO | Name of gene | Accession No. of miRBase |
|---|---|---|
| 400 | hsa-mir-320d-1 | MI0008190 |
| 401 | hsa-mir-320d-2 | MI0008192 |
| 402 | hsa-mir-6729 | MI0022574 |
| 403 | hsa-mir-5192 | MI0018171 |
| 404 | hsa-mir-6853 | MI0022699 |
| 405 | hsa-mir-1234 | MI0006324 |
| 406 | hsa-mir-1233-1 | MI0006323 |
| 407 | hsa-mir-1233-2 | MI0015973 |
| 408 | hsa-mir-4539 | MI0016910 |
| 409 | hsa-mir-3914-1 | MI0016419 |
| 410 | hsa-mir-3914-2 | MI0016421 |
| 411 | hsa-mir-4738 | MI0017376 |
| 412 | hsa-mir-548au | MI0019145 |
| 413 | hsa-mir-1539 | MI0007260 |
| 414 | hsa-mir-4720 | MI0017355 |
| 415 | hsa-mir-365b | MI0000769 |
| 416 | hsa-mir-4486 | MI0016847 |
| 417 | hsa-mir-1227 | MI0006316 |
| 418 | hsa-mir-4667 | MI0017297 |
| 419 | hsa-mir-6088 | MI0020365 |
| 420 | hsa-mir-6820 | MI0022665 |
| 421 | hsa-mir-4505 | MI0016868 |
| 422 | hsa-mir-548q | MI0010637 |
| 423 | hsa-mir-4658 | MI0017286 |
| 424 | hsa-mir-450a-1 | MI0001652 |
| 425 | hsa-mir-450a-2 | MI0003187 |
| 426 | hsa-mir-1260b | MI0014197 |
| 427 | hsa-mir-3677 | MI0016078 |
| 428 | hsa-mir-6777 | MI0022622 |
| 429 | hsa-mir-6826 | MI0022671 |
| 430 | hsa-mir-6832 | MI0022677 |
| 431 | hsa-mir-4725 | MI0017362 |
| 432 | hsa-mir-7161 | MI0023619 |
| 433 | hsa-mir-2277 | MI0011284 |
| 434 | hsa-mir-7110 | MI0022961 |
| 435 | hsa-mir-4312 | MI0015842 |
| 436 | hsa-mir-4461 | MI0016807 |
| 437 | hsa-mir-6766 | MI0022611 |
| 438 | hsa-mir-1266 | MI0006403 |

(continued)

| SEQ ID NO | Name of gene | Accession No. of miRBase |
|---|---|---|
| 439 | hsa-mir-526b | MI0003150 |
| 440 | hsa-mir-519e | MI0003145 |
| 441 | hsa-mir-512-1 | MI0003140 |
| 442 | hsa-mir-512-2 | MI0003141 |
| 443 | hsa-mir-5088 | MI0017977 |
| 444 | hsa-mir-1909 | MI0008330 |
| 445 | hsa-mir-6511a-1 | MI0022223 |
| 446 | hsa-mir-6511a-2 | MI0023564 |
| 447 | hsa-mir-6511a-3 | MI0023565 |
| 448 | hsa-mir-6511a-4 | MI0023566 |
| 449 | hsa-mir-4734 | MI0017371 |
| 450 | hsa-mir-936 | MI0005758 |
| 451 | hsa-mir-1249 | MI0006384 |
| 452 | hsa-mir-4487 | MI0016848 |
| 453 | hsa-mir-3155a | MI0014183 |
| 454 | hsa-mir-563 | MI0003569 |
| 455 | hsa-mir-4741 | MI0017379 |
| 456 | hsa-mir-6788 | MI0022633 |
| 457 | hsa-mir-4433b | MI0025511 |
| 458 | hsa-mir-323a | MI0000807 |
| 459 | hsa-mir-6811 | MI0022656 |
| 460 | hsa-mir-6721 | MI0022556 |
| 461 | hsa-mir-5004 | MI0017870 |
| 462 | hsa-mir-6509 | MI0022221 |
| 463 | hsa-mir-648 | MI0003663 |
| 464 | hsa-mir-3917 | MI0016423 |
| 465 | hsa-mir-6087 | MI0020364 |
| 466 | hsa-mir-1470 | MI0007075 |
| 467 | hsa-mir-586 | MI0003594 |
| 468 | hsa-mir-3150a | MI0014177 |
| 469 | hsa-mir-105-1 | MI0000111 |
| 470 | hsa-mir-105-2 | MI0000112 |
| 471 | hsa-mir-7973-1 | MI0025748 |
| 472 | hsa-mir-7973-2 | MI0025749 |
| 473 | hsa-mir-1914 | MI0008335 |
| 474 | hsa-mir-4749 | MI0017388 |
| 475 | hsa-mir-15b | MI0000438 |
| 476 | hsa-mir-1289-1 | MI0006350 |
| 477 | hsa-mir-1289-2 | MI0006351 |

(continued)

| SEQ ID NO | Name of gene | Accession No. of miRBase |
|---|---|---|
| 478 | hsa-mir-4433a | MI0016773 |
| 479 | hsa-mir-3666 | MI0016067 |
| 480 | hsa-mir-3186 | MI0014229 |
| 481 | hsa-mir-4488 | MI0016849 |
| 482 | hsa-mir-4474 | MI0016826 |
| 483 | hsa-mir-6731 | MI0022576 |
| 484 | hsa-mir-4640 | MI0017267 |
| 485 | hsa-mir-202 | MI0003130 |
| 486 | hsa-mir-6816 | MI0022661 |
| 487 | hsa-mir-638 | MI0003653 |
| 488 | hsa-mir-6821 | MI0022666 |
| 489 | hsa-mir-1247 | MI0006382 |
| 490 | hsa-mir-6765 | MI0022610 |
| 491 | hsa-mir-6800 | MI0022645 |
| 492 | hsa-mir-3928 | MI0016438 |
| 493 | hsa-mir-3940 | MI0016597 |
| 494 | hsa-mir-3960 | MI0016964 |
| 495 | hsa-mir-6775 | MI0022620 |
| 496 | hsa-mir-3178 | MI0014212 |
| 497 | hsa-mir-1202 | MI0006334 |
| 498 | hsa-mir-6790 | MI0022635 |
| 499 | hsa-mir-4731 | MI0017368 |
| 500 | hsa-mir-2681 | MI0012062 |
| 501 | hsa-mir-6758 | MI0022603 |
| 502 | hsa-mir-8072 | MI0025908 |
| 503 | hsa-mir-518d | MI0003171 |
| 504 | hsa-mir-3606 | MI0015996 |
| 505 | hsa-mir-4800 | MI0017448 |
| 506 | hsa-mir-1292 | MI0006433 |
| 507 | hsa-mir-6784 | MI0022629 |
| 508 | hsa-mir-4450 | MI0016795 |
| 509 | hsa-mir-6132 | MI0021277 |
| 510 | hsa-mir-4716 | MI0017350 |
| 511 | hsa-mir-6860 | MI0022707 |
| 512 | hsa-mir-1268b | MI0016748 |
| 513 | hsa-mir-378d-2 | MI0003840 |
| 514 | hsa-mir-378d-1 | MI0016749 |
| 515 | hsa-mir-4701 | MI0017334 |
| 516 | hsa-mir-4329 | MI0015901 |

(continued)

| SEQ ID NO | Name of gene | Accession No. of miRBase |
|---|---|---|
| 517 | hsa-mir-185 | MI0000482 |
| 518 | hsa-mir-552 | MI0003557 |
| 519 | hsa-mir-1273g | MI0018003 |
| 520 | hsa-mir-6769b | MI0022706 |
| 521 | hsa-mir-520a | MI0003149 |
| 522 | hsa-mir-4524b | MI0019114 |
| 523 | hsa-mir-4291 | MI0015900 |
| 524 | hsa-mir-6734 | MI0022579 |
| 525 | hsa-mir-143 | MI0000459 |
| 526 | hsa-mir-939 | MI0005761 |
| 527 | hsa-mir-6889 | MI0022736 |
| 528 | hsa-mir-6842 | MI0022688 |
| 529 | hsa-mir-4511 | MI0016877 |
| 530 | hsa-mir-4318 | MI0015847 |
| 531 | hsa-mir-4653 | MI0017281 |
| 532 | hsa-mir-6867 | MI0022714 |
| 533 | hsa-mir-133b | MI0000822 |
| 534 | hsa-mir-3196 | MI0014241 |
| 535 | hsa-mir-193b | MI0003137 |
| 536 | hsa-mir-3162 | MI0014192 |
| 537 | hsa-mir-6819 | MI0022664 |
| 538 | hsa-mir-1908 | MI0008329 |
| 539 | hsa-mir-6786 | MI0022631 |
| 540 | hsa-mir-3648-1 | MI0016048 |
| 541 | hsa-mir-3648-2 | MI0031512 |
| 542 | hsa-mir-4513 | MI0016879 |
| 543 | hsa-mir-3652 | MI0016052 |
| 544 | hsa-mir-6871 | MI0022718 |
| 545 | hsa-mir-7845 | MI0025515 |
| 546 | hsa-mir-3138 | MI0014161 |
| 547 | hsa-mir-6884 | MI0022731 |
| 548 | hsa-mir-636 | MI0003651 |
| 549 | hsa-mir-4652 | MI0017280 |
| 550 | hsa-mir-6823 | MI0022668 |
| 551 | hsa-mir-4502 | MI0016865 |
| 552 | hsa-mir-7113 | MI0022964 |
| 553 | hsa-mir-8087 | MI0025923 |
| 554 | hsa-mir-7154 | MI0023614 |
| 555 | hsa-mir-5189 | MI0018168 |

(continued)

| SEQ ID NO | Name of gene | Accession No. of miRBase |
|---|---|---|
| 556 | hsa-mir-1253 | MI0006387 |
| 557 | hsa-mir-518c | MI0003159 |
| 558 | hsa-mir-7151 | MI0023611 |
| 559 | hsa-mir-3614 | MI0016004 |
| 560 | hsa-mir-4727 | MI0017364 |
| 561 | hsa-mir-3682 | MI0016083 |
| 562 | hsa-mir-5090 | MI0017979 |
| 563 | hsa-mir-337 | MI0000806 |
| 564 | hsa-mir-488 | MI0003123 |
| 565 | hsa-mir-100 | MI0000102 |
| 566 | hsa-mir-4520-1 | MI0016886 |
| 567 | hsa-mir-373 | MI0000781 |
| 568 | hsa-mir-6499 | MI0022209 |
| 569 | hsa-mir-3909 | MI0016413 |
| 570 | hsa-mir-32 | MI0000090 |
| 571 | hsa-mir-302a | MI0000738 |
| 572 | hsa-mir-4686 | MI0017318 |
| 573 | hsa-mir-4659a | MI0017287 |
| 574 | hsa-mir-4287 | MI0015895 |
| 575 | hsa-mir-1301 | MI0003815 |
| 576 | hsa-mir-593 | MI0003605 |
| 577 | hsa-mir-517a | MI0003161 |
| 578 | hsa-mir-517b | MI0003165 |
| 579 | hsa-mir-142 | MI0000458 |
| 580 | hsa-mir-1185-2 | MI0003821 |
| 581 | hsa-mir-602 | MI0003615 |
| 582 | hsa-mir-527 | MI0003179 |
| 583 | hsa-mir-518a-1 | MI0003170 |
| 584 | hsa-mir-518a-2 | MI0003173 |
| 585 | hsa-mir-4682 | MI0017314 |
| 586 | hsa-mir-28 | MI0000086 |
| 587 | hsa-mir-4252 | MI0015864 |
| 588 | hsa-mir-452 | MI0001733 |
| 589 | hsa-mir-525 | MI0003152 |
| 590 | hsa-mir-3622a | MI0016013 |
| 591 | hsa-mir-6813 | MI0022658 |
| 592 | hsa-mir-4769 | MI0017410 |
| 593 | hsa-mir-5698 | MI0019305 |
| 594 | hsa-mir-1915 | MI0008336 |

(continued)

| SEQ ID NO | Name of gene | Accession No. of miRBase |
|---|---|---|
| 595 | hsa-mir-1343 | MI0017320 |
| 596 | hsa-mir-6861 | MI0022708 |
| 597 | hsa-mir-6781 | MI0022626 |
| 598 | hsa-mir-4508 | MI0016872 |
| 599 | hsa-mir-6743 | MI0022588 |
| 600 | hsa-mir-6726 | MI0022571 |
| 601 | hsa-mir-4525 | MI0016892 |
| 602 | hsa-mir-4651 | MI0017279 |
| 603 | hsa-mir-5787 | MI0019797 |
| 604 | hsa-mir-1290 | MI0006352 |
| 605 | hsa-mir-6075 | MI0020352 |
| 606 | hsa-mir-4758 | MI0017399 |
| 607 | hsa-mir-4690 | MI0017323 |
| 608 | hsa-mir-762 | MI0003892 |
| 609 | hsa-mir-1225 | MI0006311 |
| 610 | hsa-mir-665 | MI0005563 |
| 611 | hsa-mir-211 | MI0000287 |
| 612 | hsa-mir-3656 | MI0016056 |
| 613 | hsa-mir-149 | MI0000478 |
| 614 | hsa-mir-744 | MI0005559 |
| 615 | hsa-mir-345 | MI0000825 |
| 616 | hsa-mir-150 | MI0000479 |
| 617 | hsa-mir-191 | MI0000465 |
| 618 | hsa-mir-651 | MI0003666 |
| 619 | hsa-mir-34a | MI0000268 |
| 620 | hsa-mir-409 | MI0001735 |
| 621 | hsa-mir-369 | MI0000777 |
| 622 | hsa-mir-204 | MI0000284 |
| 623 | hsa-mir-137 | MI0000454 |
| 624 | hsa-mir-382 | MI0000790 |
| 625 | hsa-mir-517c | MI0003174 |
| 626 | hsa-mir-532 | MI0003205 |
| 627 | hsa-mir-22 | MI0000078 |
| 628 | hsa-mir-1237 | MI0006327 |
| 629 | hsa-mir-1224 | MI0003764 |
| 630 | hsa-mir-625 | MI0003639 |
| 631 | hsa-mir-328 | MI0000804 |
| 632 | hsa-mir-122 | MI0000442 |
| 633 | hsa-mir-4781 | MI0017426 |

(continued)

| SEQ ID NO | Name of gene | Accession No. of miRBase |
|---|---|---|
| 634 | hsa-mir-718 | MI0012489 |
| 635 | hsa-mir-342 | MI0000805 |
| 636 | hsa-mir-26b | MI0000084 |
| 637 | hsa-mir-140 | MI0000456 |
| 638 | hsa-mir-200a | MI0000737 |
| 639 | hsa-mir-378a | MI0000786 |
| 640 | hsa-mir-484 | MI0002468 |
| 641 | hsa-mir-296 | MI0000747 |
| 642 | hsa-mir-205 | MI0000285 |
| 643 | hsa-mir-431 | MI0001721 |
| 644 | hsa-mir-1471 | MI0007076 |
| 645 | hsa-mir-1538 | MI0007259 |
| 646 | hsa-mir-449b | MI0003673 |
| 647 | hsa-mir-1976 | MI0009986 |
| 648 | hsa-mir-4268 | MI0015874 |
| 649 | hsa-mir-4279 | MI0015887 |
| 650 | hsa-mir-3620 | MI0016011 |
| 651 | hsa-mir-3944 | MI0016601 |
| 652 | hsa-mir-3156-1 | MI0014184 |
| 653 | hsa-mir-3156-2 | MI0014230 |
| 654 | hsa-mir-3187 | MI0014231 |
| 655 | hsa-mir-4685 | MI0017317 |
| 656 | hsa-mir-4695 | MI0017328 |
| 657 | hsa-mir-4697 | MI0017330 |
| 658 | hsa-mir-4713 | MI0017347 |
| 659 | hsa-mir-4723 | MI0017359 |
| 660 | hsa-mir-371b | MI0017393 |
| 661 | hsa-mir-3151 | MI0014178 |
| 662 | hsa-mir-3192 | MI0014237 |
| 663 | hsa-mir-6728 | MI0022573 |
| 664 | hsa-mir-6736 | MI0022581 |
| 665 | hsa-mir-6740 | MI0022585 |
| 666 | hsa-mir-6741 | MI0022586 |
| 667 | hsa-mir-6747 | MI0022592 |
| 668 | hsa-mir-6750 | MI0022595 |
| 669 | hsa-mir-6754 | MI0022599 |
| 670 | hsa-mir-6759 | MI0022604 |
| 671 | hsa-mir-6761 | MI0022606 |
| 672 | hsa-mir-6762 | MI0022607 |

(continued)

| SEQ ID NO | Name of gene | Accession No. of miRBase |
|-----------|--------------|--------------------------|
| 673 | hsa-mir-6769a | MI0022614 |
| 674 | hsa-mir-6776 | MI0022621 |
| 675 | hsa-mir-6778 | MI0022623 |
| 676 | hsa-mir-6779 | MI0022624 |
| 677 | hsa-mir-6787 | MI0022632 |
| 678 | hsa-mir-6792 | MI0022637 |
| 679 | hsa-mir-6794 | MI0022639 |
| 680 | hsa-mir-6801 | MI0022646 |
| 681 | hsa-mir-6802 | MI0022647 |
| 682 | hsa-mir-6803 | MI0022648 |
| 683 | hsa-mir-6804 | MI0022649 |
| 684 | hsa-mir-6810 | MI0022655 |
| 685 | hsa-mir-6825 | MI0022670 |
| 686 | hsa-mir-6829 | MI0022674 |
| 687 | hsa-mir-6833 | MI0022678 |
| 688 | hsa-mir-6834 | MI0022679 |
| 689 | hsa-mir-6780b | MI0022681 |
| 690 | hsa-mir-6845 | MI0022691 |
| 691 | hsa-mir-6862-1 | MI0022709 |
| 692 | hsa-mir-6862-2 | MI0026415 |
| 693 | hsa-mir-6865 | MI0022712 |
| 694 | hsa-mir-6870 | MI0022717 |
| 695 | hsa-mir-6875 | MI0022722 |
| 696 | hsa-mir-6877 | MI0022724 |
| 697 | hsa-mir-6879 | MI0022726 |
| 698 | hsa-mir-6882 | MI0022729 |
| 699 | hsa-mir-6885 | MI0022732 |
| 700 | hsa-mir-6886 | MI0022733 |
| 701 | hsa-mir-6887 | MI0022734 |
| 702 | hsa-mir-6890 | MI0022737 |
| 703 | hsa-mir-6893 | MI0022740 |
| 704 | hsa-mir-6894 | MI0022741 |
| 705 | hsa-mir-7106 | MI0022957 |
| 706 | hsa-mir-7109 | MI0022960 |
| 707 | hsa-mir-7114 | MI0022965 |
| 708 | hsa-mir-7155 | MI0023615 |
| 709 | hsa-mir-7160 | MI0023621 |
| 710 | hsa-mir-615 | MI0003628 |
| 711 | hsa-mir-920 | MI0005712 |

(continued)

| SEQ ID NO | Name of gene | Accession No. of miRBase |
|---|---|---|
| 712 | hsa-mir-1825 | MI0008193 |
| 713 | hsa-mir-675 | MI0005416 |
| 714 | hsa-mir-1910 | MI0008331 |
| 715 | hsa-mir-2278 | MI0011285 |
| 716 | hsa-mir-2682 | MI0012063 |
| 717 | hsa-mir-3122 | MI0014138 |
| 718 | hsa-mir-3175 | MI0014209 |
| 719 | hsa-mir-4323 | MI0015853 |
| 720 | hsa-mir-4326 | MI0015866 |
| 721 | hsa-mir-4284 | MI0015893 |
| 722 | hsa-mir-3605 | MI0015995 |
| 723 | hsa-mir-3622b | MI0016014 |
| 724 | hsa-mir-3646 | MI0016046 |
| 725 | hsa-mir-3158-1 | MI0014186 |
| 726 | hsa-mir-3158-2 | MI0014187 |
| 727 | hsa-mir-4722 | MI0017357 |
| 728 | hsa-mir-4747 | MI0017386 |
| 729 | hsa-mir-4436b-1 | MI0017425 |
| 730 | hsa-mir-4436b-2 | MI0019110 |
| 731 | hsa-mir-5196 | MI0018175 |
| 732 | hsa-mir-5589 | MI0019148 |
| 733 | hsa-mir-642b | MI0016685 |
| 734 | hsa-mir-6716 | MI0022550 |
| 735 | hsa-mir-6511b-1 | MI0022552 |
| 736 | hsa-mir-6511b-2 | MI0023431 |
| 737 | hsa-mir-208a | MI0000251 |
| 738 | hsa-mir-6744 | MI0022589 |
| 739 | hsa-mir-6782 | MI0022627 |
| 740 | hsa-mir-6789 | MI0022634 |
| 741 | hsa-mir-6797 | MI0022642 |
| 742 | hsa-mir-6806 | MI0022651 |
| 743 | hsa-mir-6824 | MI0022669 |
| 744 | hsa-mir-6837 | MI0022683 |
| 745 | hsa-mir-6846 | MI0022692 |
| 746 | hsa-mir-6858 | MI0022704 |
| 747 | hsa-mir-6859-1 | MI0022705 |
| 748 | hsa-mir-6859-2 | MI0026420 |
| 749 | hsa-mir-6859-3 | MI0026421 |
| 750 | hsa-mir-6859-4 | MI0031521 |

(continued)

| SEQ ID NO | Name of gene | Accession No. of miRBase |
|---|---|---|
| 751 | hsa-mir-6880 | MI0022727 |
| 752 | hsa-mir-7111 | MI0022962 |
| 753 | hsa-mir-7152 | MI0023612 |
| 754 | hsa-mir-642a | MI0003657 |
| 755 | hsa-mir-657 | MI0003681 |
| 756 | hsa-mir-1236 | MI0006326 |
| 757 | hsa-mir-764 | MI0003944 |
| 758 | hsa-mir-4314 | MI0015846 |
| 759 | hsa-mir-3675 | MI0016076 |
| 760 | hsa-mir-5703 | MI0019310 |
| 761 | hsa-mir-3191 | MI0014236 |
| 762 | hsa-mir-6809 | MI0022654 |
| 763 | hsa-mir-6815 | MI0022660 |
| 764 | hsa-mir-6857 | MI0022703 |
| 765 | hsa-mir-6878 | MI0022725 |
| 766 | hsa-mir-371a | MI0000779 |
| 767 | hsa-mir-766 | MI0003836 |
| 768 | hsa-mir-1229 | MI0006319 |
| 769 | hsa-mir-1306 | MI0006443 |
| 770 | hsa-mir-210 | MI0000286 |
| 771 | hsa-mir-198 | MI0000240 |
| 772 | hsa-mir-485 | MI0002469 |
| 773 | hsa-mir-668 | MI0003761 |
| 774 | hsa-mir-877 | MI0005561 |
| 775 | hsa-mir-1238 | MI0006328 |
| 776 | hsa-mir-3130-1 | MI0014147 |
| 777 | hsa-mir-3130-2 | MI0014148 |
| 778 | hsa-mir-4298 | MI0015830 |
| 779 | hsa-mir-4290 | MI0015899 |
| 780 | hsa-mir-3943 | MI0016600 |
| 781 | hsa-mir-346 | MI0000826 |
| 782 | hsa-mir-767 | MI0003763 |
| 783 | isomiR sequence 1 of SEQ ID NO: 3 | - |
| 784 | isomiR sequence 2 of SEQ ID NO: 3 | - |
| 785 | isomiR sequence 1 of SEQ ID NO: 4 | - |
| 786 | isomiR sequence 2 of SEQ ID NO: 4 | - |
| 787 | isomiR sequence 1 of SEQ ID NO: 5 | - |
| 788 | isomiR sequence 2 of SEQ ID NO: 5 | - |
| 789 | isomiR sequence 1 of SEQ ID NO: 6 | - |

(continued)

| SEQ ID NO | Name of gene | Accession No. of miRBase |
|---|---|---|
| 790 | isomiR sequence 2 of SEQ ID NO: 6 | - |
| 791 | isomiR sequence 1 of SEQ ID NO: 7 | - |
| 792 | isomiR sequence 1 of SEQ ID NO: 8 | - |
| 793 | isomiR sequence 1 of SEQ ID NO: 10 | - |
| 794 | isomiR sequence 2 of SEQ ID NO: 10 | - |
| 795 | isomiR sequence 1 of SEQ ID NO: 11 | - |
| 796 | isomiR sequence 1 of SEQ ID NO: 12 | - |
| 797 | isomiR sequence 1 of SEQ ID NO: 13 | - |
| 798 | isomiR sequence 1 of SEQ ID NO: 15 | - |
| 799 | isomiR sequence 2 of SEQ ID NO: 15 | - |
| 800 | isomiR sequence 1 of SEQ ID NO: 16 | - |
| 801 | isomiR sequence 1 of SEQ ID NO: 17 | - |
| 802 | isomiR sequence 1 of SEQ ID NO: 19 | - |
| 803 | isomiR sequence 1 of SEQ ID NO: 22 | - |
| 804 | isomiR sequence 2 of SEQ ID NO: 22 | - |
| 805 | isomiR sequence 1 of SEQ ID NO: 23 | - |
| 806 | isomiR sequence 1 of SEQ ID NO: 25 | - |
| 807 | isomiR sequence 2 of SEQ ID NO: 25 | - |
| 808 | isomiR sequence 1 of SEQ ID NO: 26 | - |
| 809 | isomiR sequence 2 of SEQ ID NO: 26 | - |
| 810 | isomiR sequence 1 of SEQ ID NO: 28 | - |
| 811 | isomiR sequence 1 of SEQ ID NO: 29 | - |
| 812 | isomiR sequence 2 of SEQ ID NO: 29 | - |
| 813 | isomiR sequence 1 of SEQ ID NO: 30 | - |
| 814 | isomiR sequence 1 of SEQ ID NO: 31 | - |
| 815 | isomiR sequence 2 of SEQ ID NO: 31 | - |
| 816 | isomiR sequence 1 of SEQ ID NO: 32 | - |
| 817 | isomiR sequence 2 of SEQ ID NO: 32 | - |
| 818 | isomiR sequence 1 of SEQ ID NO: 33 | - |
| 819 | isomiR sequence 1 of SEQ ID NO: 34 | - |
| 820 | isomiR sequence 1 of SEQ ID NO: 35 | - |
| 821 | isomiR sequence 1 of SEQ ID NO: 36 | - |
| 822 | isomiR sequence 1 of SEQ ID NO: 37 | - |
| 823 | isomiR sequence 2 of SEQ ID NO: 37 | - |
| 824 | isomiR sequence 1 of SEQ ID NO: 39 | - |
| 825 | isomiR sequence 2 of SEQ ID NO: 39 | - |
| 826 | isomiR sequence 1 of SEQ ID NO: 40 | - |
| 827 | isomiR sequence 2 of SEQ ID NO: 40 | - |
| 828 | isomiR sequence 1 of SEQ ID NO: 41 | - |

(continued)

| SEQ ID NO | Name of gene | Accession No. of miRBase |
|---|---|---|
| 829 | isomiR sequence 1 of SEQ ID NO: 44 | - |
| 830 | isomiR sequence 1 of SEQ ID NO: 47 | - |
| 831 | isomiR sequence 1 of SEQ ID NO: 48 | - |
| 832 | isomiR sequence 1 of SEQ ID NO: 49 | - |
| 833 | isomiR sequence 2 of SEQ ID NO: 49 | - |
| 834 | isomiR sequence 1 of SEQ ID NO: 50 | - |
| 835 | isomiR sequence 2 of SEQ ID NO: 50 | - |
| 836 | isomiR sequence 1 of SEQ ID NO: 51 | - |
| 837 | isomiR sequence 2 of SEQ ID NO: 51 | - |
| 838 | isomiR sequence 1 of SEQ ID NO: 52 | - |
| 839 | isomiR sequence 1 of SEQ ID NO: 54 | - |
| 840 | isomiR sequence 2 of SEQ ID NO: 54 | - |
| 841 | isomiR sequence 1 of SEQ ID NO: 56 | - |
| 842 | isomiR sequence 2 of SEQ ID NO: 56 | - |
| 843 | isomiR sequence 1 of SEQ ID NO: 57 | - |
| 844 | isomiR sequence 2 of SEQ ID NO: 57 | - |
| 845 | isomiR sequence 1 of SEQ ID NO: 59 | - |
| 846 | isomiR sequence 2 of SEQ ID NO: 59 | - |
| 847 | isomiR sequence 1 of SEQ ID NO: 60 | - |
| 848 | isomiR sequence 2 of SEQ ID NO: 60 | - |
| 849 | isomiR sequence 1 of SEQ ID NO: 61 | - |
| 850 | isomiR sequence 2 of SEQ ID NO: 61 | - |
| 851 | isomiR sequence 1 of SEQ ID NO: 62 | - |
| 852 | isomiR sequence 2 of SEQ ID NO: 62 | - |
| 853 | isomiR sequence 1 of SEQ ID NO: 63 | - |
| 854 | isomiR sequence 2 of SEQ ID NO: 63 | - |
| 855 | isomiR sequence 1 of SEQ ID NO: 64 | - |
| 856 | isomiR sequence 2 of SEQ ID NO: 64 | - |
| 857 | isomiR sequence 1 of SEQ ID NO: 65 | - |
| 858 | isomiR sequence 1 of SEQ ID NO: 66 | - |
| 859 | isomiR sequence 1 of SEQ ID NO: 67 | - |
| 860 | isomiR sequence 1 of SEQ ID NO: 68 | - |
| 861 | isomiR sequence 2 of SEQ ID NO: 68 | - |
| 862 | isomiR sequence 1 of SEQ ID NO: 69 | - |
| 863 | isomiR sequence 2 of SEQ ID NO: 69 | - |
| 864 | isomiR sequence 1 of SEQ ID NO: 70 | - |
| 865 | isomiR sequence 1 of SEQ ID NO: 71 | - |
| 866 | isomiR sequence 1 of SEQ ID NO: 72 | - |
| 867 | isomiR sequence 2 of SEQ ID NO: 72 | - |

(continued)

| SEQ ID NO | Name of gene | Accession No. of miRBase |
|---|---|---|
| 868 | isomiR sequence 1 of SEQ ID NO: 73 | - |
| 869 | isomiR sequence 2 of SEQ ID NO: 73 | - |
| 870 | isomiR sequence 1 of SEQ ID NO: 75 | - |
| 871 | isomiR sequence 1 of SEQ ID NO: 77 | - |
| 872 | isomiR sequence 2 of SEQ ID NO: 77 | - |
| 873 | isomiR sequence 1 of SEQ ID NO: 78 | - |
| 874 | isomiR sequence 1 of SEQ ID NO: 79 | - |
| 875 | isomiR sequence 1 of SEQ ID NO: 81 | - |
| 876 | isomiR sequence 2 of SEQ ID NO: 81 | - |
| 877 | isomiR sequence 1 of SEQ ID NO: 82 | - |
| 878 | isomiR sequence 1 of SEQ ID NO: 83 | - |
| 879 | isomiR sequence 2 of SEQ ID NO: 83 | - |
| 880 | isomiR sequence 1 of SEQ ID NO: 84 | - |
| 881 | isomiR sequence 1 of SEQ ID NO: 85 | - |
| 882 | isomiR sequence 1 of SEQ ID NO: 86 | - |
| 883 | isomiR sequence 1 of SEQ ID NO: 87 | - |
| 884 | isomiR sequence 2 of SEQ ID NO: 87 | - |
| 885 | isomiR sequence 1 of SEQ ID NO: 89 | - |
| 886 | isomiR sequence 2 of SEQ ID NO: 89 | - |
| 887 | isomiR sequence 1 of SEQ ID NO: 91 | - |
| 888 | isomiR sequence 2 of SEQ ID NO: 91 | - |
| 889 | isomiR sequence 1 of SEQ ID NO: 94 | - |
| 890 | isomiR sequence 2 of SEQ ID NO: 94 | - |
| 891 | isomiR sequence 1 of SEQ ID NO: 95 | - |
| 892 | isomiR sequence 2 of SEQ ID NO: 95 | - |
| 893 | isomiR sequence 1 of SEQ ID NO: 96 | - |
| 894 | isomiR sequence 2 of SEQ ID NO: 96 | - |
| 895 | isomiR sequence 1 of SEQ ID NO: 98 | - |
| 896 | isomiR sequence 2 of SEQ ID NO: 98 | - |
| 897 | isomiR sequence 1 of SEQ ID NO: 99 | - |
| 898 | isomiR sequence 1 of SEQ ID NO: 100 | - |
| 899 | isomiR sequence 1 of SEQ ID NO: 101 | - |
| 900 | isomiR sequence 1 of SEQ ID NO: 102 | - |
| 901 | isomiR sequence 1 of SEQ ID NO: 103 | - |
| 902 | isomiR sequence 2 of SEQ ID NO: 103 | - |
| 903 | isomiR sequence 1 of SEQ ID NO: 105 | - |
| 904 | isomiR sequence 1 of SEQ ID NO: 107 | - |
| 905 | isomiR sequence 1 of SEQ ID NO: 109 | - |
| 906 | isomiR sequence 1 of SEQ ID NO: 111 | - |

(continued)

| SEQ ID NO | Name of gene | Accession No. of miRBase |
|---|---|---|
| 907 | isomiR sequence 1 of SEQ ID NO: 112 | - |
| 908 | isomiR sequence 2 of SEQ ID NO: 112 | - |
| 909 | isomiR sequence 1 of SEQ ID NO: 113 | - |
| 910 | isomiR sequence 1 of SEQ ID NO: 114 | - |
| 911 | isomiR sequence 2 of SEQ ID NO: 114 | - |
| 912 | isomiR sequence 1 of SEQ ID NO: 115 | - |
| 913 | isomiR sequence 2 of SEQ ID NO: 115 | - |
| 914 | isomiR sequence 1 of SEQ ID NO: 116 | - |
| 915 | isomiR sequence 2 of SEQ ID NO: 116 | - |
| 916 | isomiR sequence 1 of SEQ ID NO: 118 | - |
| 917 | isomiR sequence 2 of SEQ ID NO: 118 | - |
| 918 | isomiR sequence 1 of SEQ ID NO: 119 | - |
| 919 | isomiR sequence 1 of SEQ ID NO: 121 | - |
| 920 | isomiR sequence 1 of SEQ ID NO: 122 | - |
| 921 | isomiR sequence 1 of SEQ ID NO: 125 | - |
| 922 | isomiR sequence 1 of SEQ ID NO: 126 | - |
| 923 | isomiR sequence 2 of SEQ ID NO: 126 | - |
| 924 | isomiR sequence 1 of SEQ ID NO: 127 | - |
| 925 | isomiR sequence 2 of SEQ ID NO: 127 | - |
| 926 | isomiR sequence 1 of SEQ ID NO: 128 | - |
| 927 | isomiR sequence 1 of SEQ ID NO: 129 | - |
| 928 | isomiR sequence 1 of SEQ ID NO: 130 | - |
| 929 | isomiR sequence 2 of SEQ ID NO: 130 | - |
| 930 | isomiR sequence 1 of SEQ ID NO: 133 | - |
| 931 | isomiR sequence 1 of SEQ ID NO: 134 | - |
| 932 | isomiR sequence 2 of SEQ ID NO: 134 | - |
| 933 | isomiR sequence 1 of SEQ ID NO: 135 | - |
| 934 | isomiR sequence 2 of SEQ ID NO: 135 | - |
| 935 | isomiR sequence 1 of SEQ ID NO: 136 | - |
| 936 | isomiR sequence 2 of SEQ ID NO: 136 | - |
| 937 | isomiR sequence 1 of SEQ ID NO: 137 | - |
| 938 | isomiR sequence 1 of SEQ ID NO: 138 | - |
| 939 | isomiR sequence 1 of SEQ ID NO: 139 | - |
| 940 | isomiR sequence 2 of SEQ ID NO: 139 | - |
| 941 | isomiR sequence 1 of SEQ ID NO: 141 | - |
| 942 | isomiR sequence 2 of SEQ ID NO: 141 | - |
| 943 | isomiR sequence 1 of SEQ ID NO: 142 | - |
| 944 | isomiR sequence 2 of SEQ ID NO: 142 | - |
| 945 | isomiR sequence 1 of SEQ ID NO: 143 | - |

(continued)

| SEQ ID NO | Name of gene | Accession No. of miRBase |
|---|---|---|
| 946 | isomiR sequence 2 of SEQ ID NO: 143 | - |
| 947 | isomiR sequence 1 of SEQ ID NO: 144 | - |
| 948 | isomiR sequence 2 of SEQ ID NO: 144 | - |
| 949 | isomiR sequence 1 of SEQ ID NO: 145 | - |
| 950 | isomiR sequence 2 of SEQ ID NO: 145 | - |
| 951 | isomiR sequence 1 of SEQ ID NO: 147 | - |
| 952 | isomiR sequence 2 of SEQ ID NO: 147 | - |
| 953 | isomiR sequence 1 of SEQ ID NO: 148 | - |
| 954 | isomiR sequence 2 of SEQ ID NO: 148 | - |
| 955 | isomiR sequence 1 of SEQ ID NO: 149 | - |
| 956 | isomiR sequence 2 of SEQ ID NO: 149 | - |
| 957 | isomiR sequence 1 of SEQ ID NO: 150 | - |
| 958 | isomiR sequence 2 of SEQ ID NO: 150 | - |
| 959 | isomiR sequence 1 of SEQ ID NO: 151 | - |
| 960 | isomiR sequence 2 of SEQ ID NO: 151 | - |
| 961 | isomiR sequence 1 of SEQ ID NO: 152 | - |
| 962 | isomiR sequence 2 of SEQ ID NO: 152 | - |
| 963 | isomiR sequence 1 of SEQ ID NO: 153 | - |
| 964 | isomiR sequence 2 of SEQ ID NO: 153 | - |
| 965 | isomiR sequence 1 of SEQ ID NO: 154 | - |
| 966 | isomiR sequence 2 of SEQ ID NO: 154 | - |
| 967 | isomiR sequence 1 of SEQ ID NO: 157 | - |
| 968 | isomiR sequence 2 of SEQ ID NO: 157 | - |
| 969 | isomiR sequence 1 of SEQ ID NO: 158 | - |
| 970 | isomiR sequence 1 of SEQ ID NO: 159 | - |
| 971 | isomiR sequence 1 of SEQ ID NO: 161 | - |
| 972 | isomiR sequence 2 of SEQ ID NO: 161 | - |
| 973 | isomiR sequence 1 of SEQ ID NO: 162 | - |
| 974 | isomiR sequence 1 of SEQ ID NO: 163 | - |
| 975 | isomiR sequence 2 of SEQ ID NO: 163 | - |
| 976 | isomiR sequence 1 of SEQ ID NO: 164 | - |
| 977 | isomiR sequence 2 of SEQ ID NO: 164 | - |
| 978 | isomiR sequence 1 of SEQ ID NO: 165 | - |
| 979 | isomiR sequence 2 of SEQ ID NO: 165 | - |
| 980 | isomiR sequence 1 of SEQ ID NO: 166 | - |
| 981 | isomiR sequence 1 of SEQ ID NO: 167 | - |
| 982 | isomiR sequence 2 of SEQ ID NO: 167 | - |
| 983 | isomiR sequence 1 of SEQ ID NO: 168 | - |
| 984 | isomiR sequence 2 of SEQ ID NO: 168 | - |

(continued)

| SEQ ID NO | Name of gene | Accession No. of miRBase |
|---|---|---|
| 985 | isomiR sequence 1 of SEQ ID NO: 169 | - |
| 986 | isomiR sequence 1 of SEQ ID NO: 170 | - |
| 987 | isomiR sequence 2 of SEQ ID NO: 170 | - |
| 988 | isomiR sequence 1 of SEQ ID NO: 171 | - |
| 989 | isomiR sequence 2 of SEQ ID NO: 171 | - |
| 990 | isomiR sequence 1 of SEQ ID NO: 172 | - |
| 991 | isomiR sequence 2 of SEQ ID NO: 172 | - |
| 992 | isomiR sequence 1 of SEQ ID NO: 173 | - |
| 993 | isomiR sequence 2 of SEQ ID NO: 173 | - |
| 994 | isomiR sequence 1 of SEQ ID NO: 175 | - |
| 995 | isomiR sequence 2 of SEQ ID NO: 175 | - |
| 996 | isomiR sequence 1 of SEQ ID NO: 177 | - |
| 997 | isomiR sequence 2 of SEQ ID NO: 177 | - |
| 998 | isomiR sequence 1 of SEQ ID NO: 179 | - |
| 999 | isomiR sequence 2 of SEQ ID NO: 179 | - |
| 1000 | isomiR sequence 1 of SEQ ID NO: 180 | - |
| 1001 | isomiR sequence 2 of SEQ ID NO: 180 | - |
| 1002 | isomiR sequence 1 of SEQ ID NO: 181 | - |
| 1003 | isomiR sequence 2 of SEQ ID NO: 181 | - |
| 1004 | isomiR sequence 1 of SEQ ID NO: 182 | - |
| 1005 | isomiR sequence 2 of SEQ ID NO: 182 | - |
| 1006 | isomiR sequence 1 of SEQ ID NO: 185 | - |
| 1007 | isomiR sequence 1 of SEQ ID NO: 187 | - |
| 1008 | isomiR sequence 2 of SEQ ID NO: 187 | - |
| 1009 | isomiR sequence 1 of SEQ ID NO: 189 | - |
| 1010 | isomiR sequence 2 of SEQ ID NO: 189 | - |
| 1011 | isomiR sequence 1 of SEQ ID NO: 190 | - |
| 1012 | isomiR sequence 1 of SEQ ID NO: 191 | - |
| 1013 | isomiR sequence 2 of SEQ ID NO: 191 | - |
| 1014 | isomiR sequence 1 of SEQ ID NO: 192 | - |
| 1015 | isomiR sequence 1 of SEQ ID NO: 194 | - |
| 1016 | isomiR sequence 2 of SEQ ID NO: 194 | - |
| 1017 | isomiR sequence 1 of SEQ ID NO: 195 | - |
| 1018 | isomiR sequence 2 of SEQ ID NO: 195 | - |
| 1019 | isomiR sequence 1 of SEQ ID NO: 199 | - |
| 1020 | isomiR sequence 2 of SEQ ID NO: 199 | - |
| 1021 | isomiR sequence 1 of SEQ ID NO: 202 | - |
| 1022 | isomiR sequence 2 of SEQ ID NO: 202 | - |
| 1023 | isomiR sequence 1 of SEQ ID NO: 203 | - |

(continued)

| SEQ ID NO | Name of gene | Accession No. of miRBase |
|---|---|---|
| 1024 | isomiR sequence 1 of SEQ ID NO: 205 | - |
| 1025 | isomiR sequence 2 of SEQ ID NO: 205 | - |
| 1026 | isomiR sequence 1 of SEQ ID NO: 206 | - |
| 1027 | isomiR sequence 2 of SEQ ID NO: 206 | - |
| 1028 | isomiR sequence 1 of SEQ ID NO: 208 | - |
| 1029 | isomiR sequence 2 of SEQ ID NO: 208 | - |
| 1030 | isomiR sequence 1 of SEQ ID NO: 209 | - |
| 1031 | isomiR sequence 2 of SEQ ID NO: 209 | - |
| 1032 | isomiR sequence 1 of SEQ ID NO: 213 | - |
| 1033 | isomiR sequence 2 of SEQ ID NO: 213 | - |
| 1034 | isomiR sequence 1 of SEQ ID NO: 214 | - |
| 1035 | isomiR sequence 2 of SEQ ID NO: 214 | - |
| 1036 | isomiR sequence 1 of SEQ ID NO: 215 | - |
| 1037 | isomiR sequence 2 of SEQ ID NO: 215 | - |
| 1038 | isomiR sequence 1 of SEQ ID NO: 216 | - |
| 1039 | isomiR sequence 2 of SEQ ID NO: 216 | - |
| 1040 | isomiR sequence 1 of SEQ ID NO: 217 | - |
| 1041 | isomiR sequence 2 of SEQ ID NO: 217 | - |
| 1042 | isomiR sequence 1 of SEQ ID NO: 218 | - |
| 1043 | isomiR sequence 2 of SEQ ID NO: 218 | - |
| 1044 | isomiR sequence 1 of SEQ ID NO: 219 | - |
| 1045 | isomiR sequence 2 of SEQ ID NO: 219 | - |
| 1046 | isomiR sequence 1 of SEQ ID NO: 220 | - |
| 1047 | isomiR sequence 2 of SEQ ID NO: 220 | - |
| 1048 | isomiR sequence 1 of SEQ ID NO: 221 | - |
| 1049 | isomiR sequence 2 of SEQ ID NO: 221 | - |
| 1050 | isomiR sequence 1 of SEQ ID NO: 222 | - |
| 1051 | isomiR sequence 2 of SEQ ID NO: 222 | - |
| 1052 | isomiR sequence 1 of SEQ ID NO: 223 | - |
| 1053 | isomiR sequence 1 of SEQ ID NO: 223 | - |
| 1054 | isomiR sequence 1 of SEQ ID NO: 224 | - |
| 1055 | isomiR sequence 2 of SEQ ID NO: 224 | - |
| 1056 | isomiR sequence 1 of SEQ ID NO: 225 | - |
| 1057 | isomiR sequence 2 of SEQ ID NO: 225 | - |
| 1058 | isomiR sequence 1 of SEQ ID NO: 226 | - |
| 1059 | isomiR sequence 2 of SEQ ID NO: 226 | - |
| 1060 | isomiR sequence 1 of SEQ ID NO: 227 | - |
| 1061 | isomiR sequence 2 of SEQ ID NO: 227 | - |
| 1062 | isomiR sequence 1 of SEQ ID NO: 228 | - |

(continued)

| SEQ ID NO | Name of gene | Accession No. of miRBase |
|-----------|--------------|--------------------------|
| 1063 | isomiR sequence 2 of SEQ ID NO: 228 | - |
| 1064 | isomiR sequence 1 of SEQ ID NO: 229 | - |
| 1065 | isomiR sequence 2 of SEQ ID NO: 229 | - |
| 1066 | isomiR sequence 1 of SEQ ID NO: 230 | - |
| 1067 | isomiR sequence 1 of SEQ ID NO: 231 | - |
| 1068 | isomiR sequence 2 of SEQ ID NO: 231 | - |
| 1069 | isomiR sequence 1 of SEQ ID NO: 232 | - |
| 1070 | isomiR sequence 2 of SEQ ID NO: 232 | - |
| 1071 | isomiR sequence 1 of SEQ ID NO: 233 | - |
| 1072 | isomiR sequence 2 of SEQ ID NO: 233 | - |
| 1073 | isomiR sequence 1 of SEQ ID NO: 234 | - |
| 1074 | isomiR sequence 2 of SEQ ID NO: 234 | - |
| 1075 | isomiR sequence 1 of SEQ ID NO: 235 | - |
| 1076 | isomiR sequence 2 of SEQ ID NO: 235 | - |
| 1077 | isomiR sequence 1 of SEQ ID NO: 236 | - |
| 1078 | isomiR sequence 2 of SEQ ID NO: 236 | - |
| 1079 | isomiR sequence 1 of SEQ ID NO: 237 | - |
| 1080 | isomiR sequence 2 of SEQ ID NO: 237 | - |
| 1081 | isomiR sequence 1 of SEQ ID NO: 238 | - |
| 1082 | isomiR sequence 2 of SEQ ID NO: 238 | - |
| 1083 | isomiR sequence 1 of SEQ ID NO: 239 | - |
| 1084 | isomiR sequence 2 of SEQ ID NO: 239 | - |
| 1085 | isomiR sequence 1 of SEQ ID NO: 241 | - |
| 1086 | isomiR sequence 2 of SEQ ID NO: 241 | - |
| 1087 | isomiR sequence 1 of SEQ ID NO: 242 | - |
| 1088 | isomiR sequence 2 of SEQ ID NO: 242 | - |
| 1089 | isomiR sequence 1 of SEQ ID NO: 243 | - |
| 1090 | isomiR sequence 2 of SEQ ID NO: 243 | - |
| 1091 | isomiR sequence 1 of SEQ ID NO: 244 | - |
| 1092 | isomiR sequence 2 of SEQ ID NO: 244 | - |
| 1093 | isomiR sequence 1 of SEQ ID NO: 245 | - |
| 1094 | isomiR sequence 2 of SEQ ID NO: 245 | - |
| 1095 | isomiR sequence 1 of SEQ ID NO: 246 | - |
| 1096 | isomiR sequence 2 of SEQ ID NO: 246 | - |
| 1097 | isomiR sequence 1 of SEQ ID NO: 247 | - |
| 1098 | isomiR sequence 2 of SEQ ID NO: 247 | - |
| 1099 | isomiR sequence 1 of SEQ ID NO: 248 | - |
| 1100 | isomiR sequence 2 of SEQ ID NO: 248 | - |
| 1101 | isomiR sequence 1 of SEQ ID NO: 249 | - |

(continued)

| SEQ ID NO | Name of gene | Accession No. of miRBase |
|---|---|---|
| 1102 | isomiR sequence 2 of SEQ ID NO: 249 | - |
| 1103 | isomiR sequence 1 of SEQ ID NO: 251 | - |
| 1104 | isomiR sequence 2 of SEQ ID NO: 251 | - |
| 1105 | isomiR sequence 1 of SEQ ID NO: 252 | - |
| 1106 | isomiR sequence 2 of SEQ ID NO: 252 | - |
| 1107 | isomiR sequence 1 of SEQ ID NO: 253 | - |
| 1108 | isomiR sequence 2 of SEQ ID NO: 253 | - |
| 1109 | isomiR sequence 1 of SEQ ID NO: 255 | - |
| 1110 | isomiR sequence 1 of SEQ ID NO: 256 | - |
| 1111 | isomiR sequence 2 of SEQ ID NO: 256 | - |
| 1112 | isomiR sequence 1 of SEQ ID NO: 257 | - |
| 1113 | isomiR sequence 2 of SEQ ID NO: 257 | - |
| 1114 | isomiR sequence 1 of SEQ ID NO: 259 | - |
| 1115 | isomiR sequence 2 of SEQ ID NO: 259 | - |
| 1116 | isomiR sequence 1 of SEQ ID NO: 260 | - |
| 1117 | isomiR sequence 2 of SEQ ID NO: 260 | - |
| 1118 | isomiR sequence 1 of SEQ ID NO: 261 | - |
| 1119 | isomiR sequence 2 of SEQ ID NO: 261 | - |
| 1120 | isomiR sequence 1 of SEQ ID NO: 262 | - |
| 1121 | isomiR sequence 2 of SEQ ID NO: 262 | - |
| 1122 | isomiR sequence 1 of SEQ ID NO: 263 | - |
| 1123 | isomiR sequence 2 of SEQ ID NO: 263 | - |
| 1124 | isomiR sequence 1 of SEQ ID NO: 264 | - |
| 1125 | isomiR sequence 2 of SEQ ID NO: 264 | - |
| 1126 | isomiR sequence 1 of SEQ ID NO: 265 | - |
| 1127 | isomiR sequence 1 of SEQ ID NO: 268 | - |
| 1128 | isomiR sequence 1 of SEQ ID NO: 269 | - |
| 1129 | isomiR sequence 2 of SEQ ID NO: 269 | - |
| 1130 | isomiR sequence 1 of SEQ ID NO: 270 | - |
| 1131 | isomiR sequence 2 of SEQ ID NO: 270 | - |
| 1132 | isomiR sequence 1 of SEQ ID NO: 271 | - |
| 1133 | isomiR sequence 2 of SEQ ID NO: 271 | - |
| 1134 | isomiR sequence 1 of SEQ ID NO: 272 | - |
| 1135 | isomiR sequence 2 of SEQ ID NO: 272 | - |
| 1136 | isomiR sequence 1 of SEQ ID NO: 273 | - |
| 1137 | isomiR sequence 2 of SEQ ID NO: 273 | - |
| 1138 | isomiR sequence 1 of SEQ ID NO: 274 | - |
| 1139 | isomiR sequence 2 of SEQ ID NO: 274 | - |
| 1140 | isomiR sequence 1 of SEQ ID NO: 275 | - |

(continued)

| SEQ ID NO | Name of gene | Accession No. of miRBase |
|---|---|---|
| 1141 | isomiR sequence 2 of SEQ ID NO: 275 | - |
| 1142 | isomiR sequence 1 of SEQ ID NO: 276 | - |
| 1143 | isomiR sequence 1 of SEQ ID NO: 277 | - |
| 1144 | isomiR sequence 2 of SEQ ID NO: 277 | - |
| 1145 | isomiR sequence 1 of SEQ ID NO: 278 | - |
| 1146 | isomiR sequence 2 of SEQ ID NO: 278 | - |
| 1147 | isomiR sequence 1 of SEQ ID NO: 279 | - |
| 1148 | isomiR sequence 1 of SEQ ID NO: 280 | - |
| 1149 | isomiR sequence 2 of SEQ ID NO: 280 | - |
| 1150 | isomiR sequence 1 of SEQ ID NO: 281 | - |
| 1151 | isomiR sequence 2 of SEQ ID NO: 281 | - |
| 1152 | isomiR sequence 1 of SEQ ID NO: 282 | - |
| 1153 | isomiR sequence 2 of SEQ ID NO: 282 | - |
| 1154 | isomiR sequence 1 of SEQ ID NO: 283 | - |
| 1155 | isomiR sequence 2 of SEQ ID NO: 283 | - |
| 1156 | isomiR sequence 1 of SEQ ID NO: 284 | - |
| 1157 | isomiR sequence 2 of SEQ ID NO: 284 | - |
| 1158 | isomiR sequence 1 of SEQ ID NO: 286 | - |
| 1159 | isomiR sequence 2 of SEQ ID NO: 286 | - |
| 1160 | isomiR sequence 1 of SEQ ID NO: 287 | - |
| 1161 | isomiR sequence 2 of SEQ ID NO: 287 | - |
| 1162 | isomiR sequence 1 of SEQ ID NO: 288 | - |
| 1163 | isomiR sequence 2 of SEQ ID NO: 288 | - |
| 1164 | isomiR sequence 1 of SEQ ID NO: 289 | - |
| 1165 | isomiR sequence 2 of SEQ ID NO: 289 | - |
| 1166 | isomiR sequence 1 of SEQ ID NO: 290 | - |
| 1167 | isomiR sequence 1 of SEQ ID NO: 291 | - |
| 1168 | isomiR sequence 2 of SEQ ID NO: 291 | - |
| 1169 | isomiR sequence 1 of SEQ ID NO: 292 | - |
| 1170 | isomiR sequence 1 of SEQ ID NO: 293 | - |
| 1171 | isomiR sequence 2 of SEQ ID NO: 293 | - |
| 1172 | isomiR sequence 1 of SEQ ID NO: 295 | - |
| 1173 | isomiR sequence 2 of SEQ ID NO: 295 | - |
| 1174 | isomiR sequence 1 of SEQ ID NO: 296 | - |
| 1175 | isomiR sequence 2 of SEQ ID NO: 296 | - |
| 1176 | isomiR sequence 1 of SEQ ID NO: 297 | - |
| 1177 | isomiR sequence 2 of SEQ ID NO: 297 | - |
| 1178 | isomiR sequence 1 of SEQ ID NO: 298 | - |
| 1179 | isomiR sequence 1 of SEQ ID NO: 299 | - |

(continued)

| SEQ ID NO | Name of gene | Accession No. of miRBase |
|-----------|--------------|--------------------------|
| 1180 | isomiR sequence 2 of SEQ ID NO: 299 | - |
| 1181 | isomiR sequence 1 of SEQ ID NO: 300 | - |
| 1182 | isomiR sequence 2 of SEQ ID NO: 300 | - |
| 1183 | isomiR sequence 1 of SEQ ID NO: 301 | - |
| 1184 | isomiR sequence 1 of SEQ ID NO: 302 | - |
| 1185 | isomiR sequence 2 of SEQ ID NO: 302 | - |
| 1186 | isomiR sequence 1 of SEQ ID NO: 303 | - |
| 1187 | isomiR sequence 1 of SEQ ID NO: 304 | - |
| 1188 | isomiR sequence 2 of SEQ ID NO: 304 | - |
| 1189 | isomiR sequence 1 of SEQ ID NO: 305 | - |
| 1190 | isomiR sequence 2 of SEQ ID NO: 305 | - |
| 1191 | isomiR sequence 1 of SEQ ID NO: 306 | - |
| 1192 | isomiR sequence 2 of SEQ ID NO: 306 | - |
| 1193 | isomiR sequence 1 of SEQ ID NO: 307 | - |
| 1194 | isomiR sequence 1 of SEQ ID NO: 308 | - |
| 1195 | isomiR sequence 2 of SEQ ID NO: 308 | - |
| 1196 | isomiR sequence 1 of SEQ ID NO: 309 | - |
| 1197 | isomiR sequence 1 of SEQ ID NO: 310 | - |
| 1198 | isomiR sequence 1 of SEQ ID NO: 311 | - |
| 1199 | isomiR sequence 2 of SEQ ID NO: 311 | - |
| 1200 | isomiR sequence 1 of SEQ ID NO: 312 | - |
| 1201 | isomiR sequence 1 of SEQ ID NO: 313 | - |
| 1202 | isomiR sequence 2 of SEQ ID NO: 313 | - |
| 1203 | isomiR sequence 1 of SEQ ID NO: 314 | - |
| 1204 | isomiR sequence 2 of SEQ ID NO: 314 | - |
| 1205 | isomiR sequence 1 of SEQ ID NO: 315 | - |
| 1206 | isomiR sequence 1 of SEQ ID NO: 316 | - |
| 1207 | isomiR sequence 2 of SEQ ID NO: 316 | - |
| 1208 | isomiR sequence 1 of SEQ ID NO: 317 | - |
| 1209 | isomiR sequence 2 of SEQ ID NO: 317 | - |
| 1210 | isomiR sequence 1 of SEQ ID NO: 320 | - |
| 1211 | isomiR sequence 2 of SEQ ID NO: 320 | - |
| 1212 | isomiR sequence 1 of SEQ ID NO: 321 | - |
| 1213 | isomiR sequence 2 of SEQ ID NO: 321 | - |
| 1214 | isomiR sequence 1 of SEQ ID NO: 322 | - |
| 1215 | isomiR sequence 2 of SEQ ID NO: 322 | - |
| 1216 | isomiR sequence 1 of SEQ ID NO: 323 | - |
| 1217 | isomiR sequence 2 of SEQ ID NO: 323 | - |
| 1218 | isomiR sequence 1 of SEQ ID NO: 324 | - |

(continued)

| SEQ ID NO | Name of gene | Accession No. of miRBase |
|---|---|---|
| 1219 | isomiR sequence 2 of SEQ ID NO: 324 | - |
| 1220 | isomiR sequence 1 of SEQ ID NO: 325 | - |
| 1221 | isomiR sequence 2 of SEQ ID NO: 325 | - |
| 1222 | isomiR sequence 1 of SEQ ID NO: 326 | - |
| 1223 | isomiR sequence 2 of SEQ ID NO: 326 | - |
| 1224 | isomiR sequence 1 of SEQ ID NO: 327 | - |
| 1225 | isomiR sequence 1 of SEQ ID NO: 328 | - |
| 1226 | isomiR sequence 2 of SEQ ID NO: 328 | - |
| 1227 | isomiR sequence 1 of SEQ ID NO: 329 | - |
| 1228 | isomiR sequence 2 of SEQ ID NO: 329 | - |
| 1229 | isomiR sequence 1 of SEQ ID NO: 330 | - |
| 1230 | isomiR sequence 2 of SEQ ID NO: 330 | - |
| 1231 | isomiR sequence 1 of SEQ ID NO: 331 | - |
| 1232 | isomiR sequence 1 of SEQ ID NO: 332 | - |
| 1233 | isomiR sequence 1 of SEQ ID NO: 333 | - |
| 1234 | isomiR sequence 2 of SEQ ID NO: 333 | - |
| 1235 | isomiR sequence 1 of SEQ ID NO: 335 | - |
| 1236 | isomiR sequence 2 of SEQ ID NO: 335 | - |
| 1237 | isomiR sequence 1 of SEQ ID NO: 338 | - |
| 1238 | isomiR sequence 2 of SEQ ID NO: 338 | - |
| 1239 | isomiR sequence 1 of SEQ ID NO: 340 | - |
| 1240 | isomiR sequence 2 of SEQ ID NO: 340 | - |
| 1241 | isomiR sequence 1 of SEQ ID NO: 341 | - |
| 1242 | isomiR sequence 2 of SEQ ID NO: 341 | - |
| 1243 | isomiR sequence 1 of SEQ ID NO: 342 | - |
| 1244 | isomiR sequence 2 of SEQ ID NO: 342 | - |
| 1245 | isomiR sequence 1 of SEQ ID NO: 343 | - |
| 1246 | isomiR sequence 2 of SEQ ID NO: 343 | - |
| 1247 | isomiR sequence 1 of SEQ ID NO: 345 | - |
| 1248 | isomiR sequence 2 of SEQ ID NO: 345 | - |
| 1249 | isomiR sequence 1 of SEQ ID NO: 347 | - |
| 1250 | isomiR sequence 2 of SEQ ID NO: 347 | - |
| 1251 | isomiR sequence 1 of SEQ ID NO: 348 | - |
| 1252 | isomiR sequence 2 of SEQ ID NO: 348 | - |
| 1253 | isomiR sequence 1 of SEQ ID NO: 349 | - |
| 1254 | isomiR sequence 2 of SEQ ID NO: 349 | - |
| 1255 | isomiR sequence 1 of SEQ ID NO: 350 | - |
| 1256 | isomiR sequence 1 of SEQ ID NO: 352 | - |
| 1257 | isomiR sequence 2 of SEQ ID NO: 352 | - |

(continued)

| SEQ ID NO | Name of gene | Accession No. of miRBase |
|---|---|---|
| 1258 | isomiR sequence 1 of SEQ ID NO: 353 | - |
| 1259 | isomiR sequence 2 of SEQ ID NO: 353 | - |
| 1260 | isomiR sequence 1 of SEQ ID NO: 354 | - |
| 1261 | isomiR sequence 1 of SEQ ID NO: 355 | - |
| 1262 | isomiR sequence 2 of SEQ ID NO: 355 | - |
| 1263 | isomiR sequence 1 of SEQ ID NO: 356 | - |
| 1264 | isomiR sequence 2 of SEQ ID NO: 356 | - |
| 1265 | isomiR sequence 1 of SEQ ID NO: 357 | - |
| 1266 | isomiR sequence 2 of SEQ ID NO: 357 | - |
| 1267 | isomiR sequence 1 of SEQ ID NO: 359 | - |
| 1268 | isomiR sequence 2 of SEQ ID NO: 359 | - |
| 1269 | isomiR sequence 1 of SEQ ID NO: 361 | - |
| 1270 | isomiR sequence 2 of SEQ ID NO: 361 | - |
| 1271 | isomiR sequence 1 of SEQ ID NO: 362 | - |
| 1272 | isomiR sequence 1 of SEQ ID NO: 363 | - |
| 1273 | isomiR sequence 2 of SEQ ID NO: 363 | - |
| 1274 | isomiR sequence 1 of SEQ ID NO: 367 | - |
| 1275 | isomiR sequence 2 of SEQ ID NO: 367 | - |
| 1276 | isomiR sequence 1 of SEQ ID NO: 368 | - |
| 1277 | isomiR sequence 2 of SEQ ID NO: 368 | - |
| 1278 | isomiR sequence 1 of SEQ ID NO: 369 | - |
| 1279 | isomiR sequence 2 of SEQ ID NO: 369 | - |
| 1280 | isomiR sequence 1 of SEQ ID NO: 371 | - |
| 1281 | isomiR sequence 2 of SEQ ID NO: 371 | - |
| 1282 | isomiR sequence 1 of SEQ ID NO: 372 | - |
| 1283 | isomiR sequence 2 of SEQ ID NO: 372 | - |
| 1284 | isomiR sequence 1 of SEQ ID NO: 373 | - |
| 1285 | isomiR sequence 1 of SEQ ID NO: 374 | - |
| 1286 | isomiR sequence 2 of SEQ ID NO: 374 | - |
| 1287 | isomiR sequence 1 of SEQ ID NO: 375 | - |
| 1288 | isomiR sequence 2 of SEQ ID NO: 375 | - |
| 1289 | isomiR sequence 1 of SEQ ID NO: 376 | - |
| 1290 | isomiR sequence 2 of SEQ ID NO: 376 | - |
| 1291 | isomiR sequence 1 of SEQ ID NO: 377 | - |
| 1292 | isomiR sequence 2 of SEQ ID NO: 377 | - |
| 1293 | isomiR sequence 1 of SEQ ID NO: 378 | - |
| 1294 | isomiR sequence 2 of SEQ ID NO: 378 | - |
| 1295 | isomiR sequence 1 of SEQ ID NO: 379 | - |
| 1296 | isomiR sequence 2 of SEQ ID NO: 379 | - |

(continued)

| SEQ ID NO | Name of gene | Accession No. of miRBase |
| --- | --- | --- |
| 1297 | isomiR sequence 1 of SEQ ID NO: 380 | - |
| 1298 | isomiR sequence 2 of SEQ ID NO: 380 | - |
| 1299 | isomiR sequence 1 of SEQ ID NO: 381 | - |
| 1300 | isomiR sequence 2 of SEQ ID NO: 381 | - |
| 1301 | isomiR sequence 1 of SEQ ID NO: 382 | - |
| 1302 | isomiR sequence 2 of SEQ ID NO: 382 | - |
| 1303 | isomiR sequence 1 of SEQ ID NO: 383 | - |
| 1304 | isomiR sequence 2 of SEQ ID NO: 383 | - |
| 1305 | isomiR sequence 1 of SEQ ID NO: 385 | - |
| 1306 | isomiR sequence 2 of SEQ ID NO: 385 | - |
| 1307 | isomiR sequence 1 of SEQ ID NO: 386 | - |
| 1308 | isomiR sequence 2 of SEQ ID NO: 386 | - |
| 1309 | isomiR sequence 1 of SEQ ID NO: 388 | - |
| 1310 | isomiR sequence 2 of SEQ ID NO: 388 | - |
| 1311 | isomiR sequence 1 of SEQ ID NO: 389 | - |
| 1312 | isomiR sequence 2 of SEQ ID NO: 389 | - |
| 1313 | isomiR sequence 1 of SEQ ID NO: 390 | - |
| 1314 | isomiR sequence 2 of SEQ ID NO: 390 | - |
| 1435 | hsa-miR-516a-5p | MIMAT0004770 |
| 1436 | hsa-miR-769-3p | MIMAT0003887 |
| 1437 | hsa-miR-3692-5p | MIMAT0018121 |
| 1438 | hsa-miR-3945 | MIMAT0018361 |
| 1439 | hsa-miR-4433a-3p | MIMAT0018949 |
| 1440 | hsa-miR-4485-3p | MIMAT0019019 |
| 1441 | hsa-miR-6831-5p | MIMAT0027562 |
| 1442 | hsa-miR-519c-5p | MIMAT0002831 |
| 1443 | hsa-miR-551b-5p | MIMAT0004794 |
| 1444 | hsa-miR-1343-3p | MIMAT0019776 |
| 1445 | hsa-miR-4286 | MIMAT0016916 |
| 1446 | hsa-miR-4634 | MIMAT0019691 |
| 1447 | hsa-miR-4733-3p | MIMAT0019858 |
| 1448 | hsa-miR-6086 | MIMAT0023711 |
| 1449 | hsa-miR-30d-5p | MIMAT0000245 |
| 1450 | hsa-miR-30b-3p | MIMAT0004589 |
| 1451 | hsa-miR-92a-3p | MIMAT0000092 |
| 1452 | hsa-miR-371b-5p | MIMAT0019892 |
| 1453 | hsa-miR-486-5p | MIMAT0002177 |
| 1454 | hsa-mir-516a-1 | MI0003180 |
| 1455 | hsa-mir-769 | MI0003834 |

(continued)

| SEQ ID NO | Name of gene | Accession No. of miRBase |
|---|---|---|
| 1456 | hsa-mir-3692 | MI0016093 |
| 1457 | hsa-mir-3945 | MI0016602 |
| 1458 | hsa-mir-4433a | MI0016773 |
| 1459 | hsa-mir-4485 | MI0016846 |
| 1460 | hsa-mir-6831 | MI0022676 |
| 1461 | hsa-mir-519c | MI0003148 |
| 1462 | hsa-mir-551b | MI0003575 |
| 1463 | hsa-mir-1343 | MI0017320 |
| 1464 | hsa-mir-4286 | MI0015894 |
| 1465 | hsa-mir-4634 | MI0017261 |
| 1466 | hsa-mir-4733 | MI0017370 |
| 1467 | hsa-mir-6086 | MI0020363 |
| 1468 | hsa-mir-30d | MI0000255 |
| 1469 | hsa-mir-30b | MI0000441 |
| 1470 | hsa-mir-92a-1 | MI0000093 |
| 1471 | hsa-mir-371b | MI0017393 |
| 1472 | hsa-mir-486-1 | MI0002470 |
| 1473 | isomiR sequence 1 of SEQ ID NO: 1435 | - |
| 1474 | isomiR sequence 2 of SEQ ID NO: 1435 | - |
| 1475 | isomiR sequence 1 of SEQ ID NO: 1436 | - |
| 1476 | isomiR sequence 2 of SEQ ID NO: 1436 | - |
| 1477 | isomiR sequence 1 of SEQ ID NO: 1438 | - |
| 1478 | isomiR sequence 2 of SEQ ID NO: 1438 | - |
| 1479 | isomiR sequence 1 of SEQ ID NO: 1439 | - |
| 1480 | isomiR sequence 2 of SEQ ID NO: 1439 | - |
| 1481 | isomiR sequence 1 of SEQ ID NO: 1440 | - |
| 1482 | isomiR sequence 2 of SEQ ID NO: 1440 | |
| 1483 | isomiR sequence 1 of SEQ ID NO: 1441 | - |
| 1484 | isomiR sequence 2 of SEQ ID NO: 1441 | - |
| 1485 | isomiR sequence 1 of SEQ ID NO: 1446 | - |
| 1486 | isomiR sequence 2 of SEQ ID NO: 1446 | - |
| 1487 | isomiR sequence 1 of SEQ ID NO: 1448 | - |
| 1488 | isomiR sequence 2 of SEQ ID NO: 1448 | - |
| 1489 | isomiR sequence 1 of SEQ ID NO: 1449 | - |
| 1490 | isomiR sequence 2 of SEQ ID NO: 1449 | - |
| 1491 | isomiR sequence 1 of SEQ ID NO: 1451 | - |
| 1492 | isomiR sequence 2 of SEQ ID NO: 1451 | - |
| 1493 | isomiR sequence 1 of SEQ ID NO: 1452 | - |
| 1494 | isomiR sequence 2 of SEQ ID NO: 1452 | - |

(continued)

| SEQ ID NO | Name of gene | Accession No. of miRBase |
|---|---|---|
| 1495 | isomiR sequence 1 of SEQ ID NO: 1455 | - |
| 1496 | isomiR sequence 1 of SEQ ID NO: 1458 | - |
| 1497 | isomiR sequence 2 of SEQ ID NO: 1458 | - |
| 1498 | isomiR sequence 1 of SEQ ID NO: 1460 | - |
| 1499 | isomiR sequence 2 of SEQ ID NO: 1460 | - |
| 1500 | isomiR sequence 1 of SEQ ID NO: 1461 | - |
| 1501 | isomiR sequence 2 of SEQ ID NO: 1461 | - |
| 1502 | isomiR sequence 1 of SEQ ID NO: 1462 | - |
| 1503 | isomiR sequence 2 of SEQ ID NO: 1462 | - |
| 1504 | isomiR sequence 1 of SEQ ID NO: 1463 | - |
| 1505 | isomiR sequence 2 of SEQ ID NO: 1463 | - |

[Table 1-2]

| SEQ ID NO | Name of gene | Accession No. of miRBase |
|---|---|---|
| 1315 | hsa-miR-6765-3p | MIMAT0027431 |
| 1316 | hsa-miR-6784-5p | MIMAT0027468 |
| 194 | hsa-miR-5698 | MIMAT0022491 |
| 1317 | hsa-miR-6778-5p | MIMAT0027456 |
| 195 | hsa-miR-1915-3p | MIMAT0007892 |
| 1318 | hsa-miR-6875-5p | MIMAT0027650 |
| 196 | hsa-miR-1343-5p | MIMAT0027038 |
| 1319 | hsa-miR-4534 | MIMAT0019073 |
| 197 | hsa-miR-6861-5p | MIMAT0027623 |
| 1320 | hsa-miR-4721 | MIMAT0019835 |
| 1321 | hsa-miR-6756-5p | MIMAT0027412 |
| 1322 | hsa-miR-615-5p | MIMAT0004804 |
| 1323 | hsa-miR-6727-5p | MIMAT0027355 |
| 1324 | hsa-miR-6887-5p | MIMAT0027674 |
| 1325 | hsa-miR-8063 | MIMAT0030990 |
| 1326 | hsa-miR-6880-5p | MIMAT0027660 |
| 1327 | hsa-miR-6805-3p | MIMAT0027511 |
| 198 | hsa-miR-6781-5p | MIMAT0027462 |
| 199 | hsa-miR-4508 | MIMAT0019045 |
| 1328 | hsa-miR-4726-5p | MIMAT0019845 |
| 1329 | hsa-miR-4710 | MIMAT0019815 |
| 1330 | hsa-miR-7111-5p | MIMAT0028119 |
| 1331 | hsa-miR-3619-3p | MIMAT0019219 |
| 200 | hsa-miR-6743-5p | MIMAT0027387 |
| 1332 | hsa-miR-6795-5p | MIMAT0027490 |

(continued)

| SEQ ID NO | Name of gene | Accession No. of miRBase |
|---|---|---|
| 201 | hsa-miR-6726-5p | MIMAT0027353 |
| 202 | hsa-miR-4525 | MIMAT0019064 |
| 1333 | hsa-miR-1254 | MIMAT0005905 |
| 1334 | hsa-miR-1233-5p | MIMAT0022943 |
| 203 | hsa-miR-4651 | MIMAT0019715 |
| 1335 | hsa-miR-6836-3p | MIMAT0027575 |
| 1336 | hsa-miR-6769a-5p | MIMAT0027438 |
| 204 | hsa-miR-6813-5p | MIMAT0027526 |
| 1337 | hsa-miR-4532 | MIMAT0019071 |
| 1338 | hsa-miR-365a-5p | MIMAT0009199 |
| 1339 | hsa-miR-1231 | MIMAT0005586 |
| 205 | hsa-miR-5787 | MIMAT0023252 |
| 206 | hsa-miR-1290 | MIMAT0005880 |
| 1340 | hsa-miR-1228-5p | MIMAT0005582 |
| 374 | hsa-miR-371a-5p | MIMAT0004687 |
| 1341 | hsa-miR-4430 | MIMAT0018945 |
| 1342 | hsa-miR-296-3p | MIMAT0004679 |
| 207 | hsa-miR-6075 | MIMAT0023700 |
| 1343 | hsa-miR-1237-5p | MIMAT0022946 |
| 208 | hsa-miR-4758-5p | MIMAT0019903 |
| 209 | hsa-miR-4690-5p | MIMAT0019779 |
| 1344 | hsa-miR-4466 | MIMAT0018993 |
| 1345 | hsa-miR-6789-5p | MIMAT0027478 |
| 1346 | hsa-miR-4632-5p | MIMAT0022977 |
| 1347 | hsa-miR-4745-5p | MIMAT0019878 |
| 1348 | hsa-miR-4665-5p | MIMAT0019739 |
| 1349 | hsa-miR-6807-5p | MIMAT0027514 |
| 210 | hsa-miR-762 | MIMAT0010313 |
| 1350 | hsa-miR-7114-5p | MIMAT0028125 |
| 1351 | hsa-miR-150-3p | MIMAT0004610 |
| 1352 | hsa-miR-423-5p | MIMAT0004748 |
| 1353 | hsa-miR-575 | MIMAT0003240 |
| 1354 | hsa-miR-671-5p | MIMAT0003880 |
| 1355 | hsa-miR-939-5p | MIMAT0004982 |
| 211 | hsa-miR-1225-3p | MIMAT0005573 |
| 212 | hsa-miR-3184-5p | MIMAT0015064 |
| 1356 | hsa-miR-3665 | MIMAT0018087 |
| 490 | hsa-mir-6765 | MI0022610 |
| 1357 | hsa-mir-6784 | MI0022629 |

(continued)

| SEQ ID NO | Name of gene | Accession No. of miRBase |
|---|---|---|
| 593 | hsa-mir-5698 | MI0019305 |
| 1358 | hsa-mir-6778 | MI0022623 |
| 594 | hsa-mir-1915 | MI0008336 |
| 1359 | hsa-mir-6875 | MI0022722 |
| 595 | hsa-mir-1343 | MI0017320 |
| 1360 | hsa-mir-4534 | MI0016901 |
| 596 | hsa-mir-6861 | MI0022708 |
| 1361 | hsa-mir-4721 | MI0017356 |
| 1362 | hsa-mir-6756 | MI0022601 |
| 1363 | hsa-mir-615 | MI0003628 |
| 1364 | hsa-mir-6727 | MI0022572 |
| 1365 | hsa-mir-6887 | MI0022734 |
| 1366 | hsa-mir-8063 | MI0025899 |
| 1367 | hsa-mir-6880 | MI0022727 |
| 1368 | hsa-mir-6805 | MI0022650 |
| 597 | hsa-mir-6781 | MI0022626 |
| 598 | hsa-mir-4508 | MI0016872 |
| 1369 | hsa-mir-4726 | MI0017363 |
| 1370 | hsa-mir-4710 | MI0017344 |
| 1371 | hsa-mir-7111 | MI0022962 |
| 1372 | hsa-mir-3619 | MI0016009 |
| 599 | hsa-mir-6743 | MI0022588 |
| 1373 | hsa-mir-6795 | MI0022640 |
| 600 | hsa-mir-6726 | MI0022571 |
| 601 | hsa-mir-4525 | MI0016892 |
| 1374 | hsa-mir-1254-1 | MI0006388 |
| 1375 | hsa-mir-1254-2 | MI0016747 |
| 1376 | hsa-mir-1233-1 | MI0006323 |
| 1377 | hsa-mir-1233-2 | MI0015973 |
| 602 | hsa-mir-4651 | MI0017279 |
| 1378 | hsa-mir-6836 | MI0022682 |
| 1379 | hsa-mir-6769a | MI0022614 |
| 591 | hsa-mir-6813 | MI0022658 |
| 1380 | hsa-mir-4532 | MI0016899 |
| 1381 | hsa-mir-365a | MI0000767 |
| 1382 | hsa-mir-1231 | MI0006321 |
| 603 | hsa-mir-5787 | MI0019797 |
| 604 | hsa-mir-1290 | MI0006352 |
| 1383 | hsa-mir-1228 | MI0006318 |

(continued)

| SEQ ID NO | Name of gene | Accession No. of miRBase |
|---|---|---|
| 766 | hsa-mir-371a | MI0000779 |
| 1384 | hsa-mir-4430 | MI0016769 |
| 1385 | hsa-mir-296 | MI0000747 |
| 605 | hsa-mir-6075 | MI0020352 |
| 1386 | hsa-mir-1237 | MI0006327 |
| 606 | hsa-mir-4758 | MI0017399 |
| 607 | hsa-mir-4690 | MI0017323 |
| 1387 | hsa-mir-4466 | MI0016817 |
| 1388 | hsa-mir-6789 | MI0022634 |
| 1389 | hsa-mir-4632 | MI0017259 |
| 1390 | hsa-mir-4745 | MI0017384 |
| 1391 | hsa-mir-4665 | MI0017295 |
| 1392 | hsa-mir-6807 | MI0022652 |
| 608 | hsa-mir-762 | MI0003892 |
| 1393 | hsa-mir-7114 | MI0022965 |
| 1394 | hsa-mir-150 | MI0000479 |
| 1395 | hsa-mir-423 | MI0001445 |
| 1396 | hsa-mir-575 | MI0003582 |
| 1397 | hsa-mir-671 | MI0003760 |
| 1398 | hsa-mir-939 | MI0005761 |
| 609 | hsa-mir-1225 | MI0006311 |
| 398 | hsa-mir-3184 | MI0014226 |
| 1399 | hsa-mir-3665 | MI0016066 |
| 1015 | isomiR sequence 1 of SEQ ID NO: 194 | - |
| 1016 | isomiR sequence 2 of SEQ ID NO: 194 | - |
| 1017 | isomiR sequence 2 of SEQ ID NO: 195 | - |
| 1400 | isomiR sequence 1 of SEQ ID NO: 1320 | - |
| 1401 | isomiR sequence 1 of SEQ ID NO: 1322 | - |
| 1402 | isomiR sequence 2 of SEQ ID NO: 1322 | - |
| 1019 | isomiR sequence 1 of SEQ ID NO: 199 | - |
| 1020 | isomiR sequence 2 of SEQ ID NO: 199 | - |
| 1403 | isomiR sequence 1 of SEQ ID NO: 1328 | - |
| 1404 | isomiR sequence 2 of SEQ ID NO: 1328 | - |
| 1405 | isomiR sequence 2 of SEQ ID NO: 1329 | - |
| 1021 | isomiR sequence 1 of SEQ ID NO: 202 | - |
| 1022 | isomiR sequence 2 of SEQ ID NO: 202 | - |
| 1406 | isomiR sequence 1 of SEQ ID NO: 1333 | - |
| 1407 | isomiR sequence 2 of SEQ ID NO: 1333 | - |
| 1408 | isomiR sequence 1 of SEQ ID NO: 1334 | - |

(continued)

| SEQ ID NO | Name of gene | Accession No. of miRBase |
| --- | --- | --- |
| 1409 | isomiR sequence 2 of SEQ ID NO: 1334 | - |
| 1023 | isomiR sequence 1 of SEQ ID NO: 203 | - |
| 1410 | isomiR sequence 1 of SEQ ID NO: 1337 | - |
| 1411 | isomiR sequence 2 of SEQ ID NO: 1337 | - |
| 1412 | isomiR sequence 1 of SEQ ID NO: 1338 | - |
| 1413 | isomiR sequence 2 of SEQ ID NO: 1338 | - |
| 1024 | isomiR sequence 2 of SEQ ID NO: 205 | - |
| 1026 | isomiR sequence 1 of SEQ ID NO: 206 | - |
| 1027 | isomiR sequence 2 of SEQ ID NO: 206 | - |
| 1414 | isomiR sequence 1 of SEQ ID NO: 1340 | - |
| 1415 | isomiR sequence 2 of SEQ ID NO: 1340 | - |
| 1285 | isomiR sequence 1 of SEQ ID NO: 374 | - |
| 1286 | isomiR sequence 2 of SEQ ID NO: 374 | - |
| 1416 | isomiR sequence 1 of SEQ ID NO: 1341 | - |
| 1417 | isomiR sequence 2 of SEQ ID NO: 1341 | - |
| 1418 | isomiR sequence 2 of SEQ ID NO: 1342 | - |
| 1419 | isomiR sequence 2 of SEQ ID NO: 1343 | - |
| 1028 | isomiR sequence 1 of SEQ ID NO: 208 | - |
| 1029 | isomiR sequence 2 of SEQ ID NO: 208 | - |
| 1030 | isomiR sequence 1 of SEQ ID NO: 209 | - |
| 1031 | isomiR sequence 2 of SEQ ID NO: 209 | - |
| 1420 | isomiR sequence 1 of SEQ ID NO: 1344 | - |
| 1421 | isomiR sequence 2 of SEQ ID NO: 1344 | - |
| 1422 | isomiR sequence 2 of SEQ ID NO: 1346 | - |
| 1423 | isomiR sequence 2 of SEQ ID NO: 1347 | - |
| 1424 | isomiR sequence 2 of SEQ ID NO: 1348 | - |
| 1425 | isomiR sequence 1 of SEQ ID NO: 1351 | - |
| 1426 | isomiR sequence 2 of SEQ ID NO: 1351 | - |
| 1427 | isomiR sequence 1 of SEQ ID NO: 1352 | - |
| 1428 | isomiR sequence 2 of SEQ ID NO: 1352 | - |
| 1429 | isomiR sequence 1 of SEQ ID NO: 1354 | - |
| 1430 | isomiR sequence 2 of SEQ ID NO: 1354 | - |
| 1431 | isomiR sequence 1 of SEQ ID NO: 1355 | - |
| 1432 | isomiR sequence 2 of SEQ ID NO: 1355 | - |
| 1433 | isomiR sequence 1 of SEQ ID NO: 1356 | - |
| 1434 | isomiR sequence 2 of SEQ ID NO: 1356 | - |

The present specification encompasses the contents disclosed in Japanese Patent Application Nos. 2018-023283, 2018-085652, and 2018-138487 from which the present application claims priority.

Advantageous Effect of Invention

The present invention makes it possible to detect a wide range of disease types of dementia comprehensively with high accuracy (or AUC), sensitivity, and specificity, thereby discriminating between dementia and normal cognitive functions (non-dementia). Use of a nucleic acid capable of specifically binding to a dementia marker in the present invention enables the disease type of dementia to be detected easily and objectively without causing a difference among physicians and/or among facilities of medical institutions. For instance, using the measured value(s) for the expression level(s) of at least one or several miRNAs in blood, serum, and/or plasma that can be collected less invasively from a subject as an indicator(s), whether or not the subject has dementia can be detected easily. .

BRIEF DESCRIPTION OF THE DRAWINGS

[Fig. 1] Fig. 1 illustrates the relationship between the nucleotide sequences of hsa-miR-4728-5p represented by SEQ ID NO: 3 and hsa-miR-4728-3p represented by SEQ ID NO: 335, which are generated from a precursor hsa-mir-4728 represented by SEQ ID NO: 393.

[Fig. 2] Fig. 2 shows plots of discriminant scores in each training cohort and each validation cohort as obtained in Example 1. Fig. 2A is a plot of discriminant scores for Alzheimer's dementia as obtained in Example 1-1; Fig. 2B is a plot of discriminant scores for vascular dementia as obtained in Example 1-2; and Fig. 2C is a plot of discriminant scores for Lewy body dementia as obtained in Example 1-3.

[Fig. 3] Fig. 3 shows plots of discriminant scores in a training cohort (A) and a validation cohort (B) and ROC curves for the training cohort (C) and the validation cohort (D) as obtained in Example 2-1.

[Fig. 4] Fig. 4 shows plots of discriminant scores in a training cohort (A) and a validation cohort (B) and ROC curves for the training cohort (C) and the validation cohort (D) as obtained in Example 2-2.

[Fig. 5] Fig. 5 shows plots of discriminant scores in a training cohort (A) and a validation cohort (B) and ROC curves for the training cohort (C) and the validation cohort (D) as obtained in Example 2-3.

[Fig. 6] Fig. 6 shows plots of discriminant scores in a training cohort (A) and a validation cohort (B) and ROC curves for the training cohort (C) and the validation cohort (D) as obtained in Example 2-4.

[Fig. 7] Fig. 7 shows plots of discriminant scores in a training cohort (A) and a validation cohort (B) and ROC curves for the training cohort (C) and the validation cohort (D) as obtained in Example 2-5.

[Fig. 8] Fig. 8 shows plots of discriminant scores in a training cohort (A) and a validation cohort (B) and ROC curves for the training cohort (C) and the validation cohort (D) as obtained in Example 3-1.

[Fig. 9] Fig. 9 shows plots of discriminant scores in a training cohort (A) and a validation cohort (B) and ROC curves for the training cohort (C) and the validation cohort (D) as obtained in Example 3-2.

[Fig. 10] Fig. 10 shows plots of discriminant scores in a training cohort (A) and a validation cohort (B) and ROC curves for the training cohort (C) and the validation cohort (D) as obtained in Example 3-3.

[Fig. 11] Fig. 11 shows plots of discriminant scores in a training cohort (A) and a validation cohort (B) and ROC curves for the training cohort (C) and the validation cohort (D) as obtained in Example 3-4.

[Fig. 12] Fig. 12 shows plots of discriminant scores in a training cohort (A) and a validation cohort (B) and ROC curves for the training cohort (C) and the validation cohort (D) as obtained in Example 3-5.

[Fig. 13] Fig. 13 shows ROC curves for validation cohorts as obtained in Example 9. Fig. 13A is a plot of discriminant scores for Alzheimer's dementia as obtained in Example 9-1; Fig. 13B is a plot of discriminant scores for vascular dementia as obtained in Example 9-2; and Fig. 13C is a plot of discriminant scores for Lewy body dementia as obtained in Example 9-3.

[Fig. 14] Fig. 14 illustrates the relationship between the nucleotide sequences of hsa-miR-6765-5p and hsa-miR-6765-3p represented by SEQ ID NO: 1315, which are generated from a precursor hsa-mir-6765 represented by SEQ ID NO: 490.

[Fig. 15] Fig. 15 shows plots of discriminant scores in training cohorts (A) and validation cohorts (B) as obtained in Example 15.

[Fig. 16] Fig. 16 shows plots of discriminant scores in training cohorts (A) and validation cohorts (B) as obtained in Example 20.

[Fig. 17] Fig. 17 shows ROC curves calculated in Example 21. In Fig. 17A, 42 miRNA markers obtained in Example 15 were used and in Fig. 17B, 51 miRNA markers obtained in Example 20 were used to calculate the sensitivity as the ordinate and the specificity as the abscissa expressed in ROC curves.

[Fig. 18] Fig. 18A is a plot of discriminant scores in validation cohorts in which 44 miRNA markers obtained in Example 24 were used. In Fig. 18B, 44 miRNA markers obtained in Example 24 were used to calculate the sensitivity as the ordinate and the specificity as the abscissa expressed in the ROC curve.

DESCRIPTION OF EMBODIMENTS

Hereinafter, the present invention will be further described in detail.

1. Target Nucleic acids for Dementia

The major target nucleic acids as dementia markers for detecting dementia or the presence or absence of dementia by using the nucleic acid probes or primers for detection of dementia as defined above according to the present invention include at least one miRNA selected from the group consisting of (i) miR-4274, miR-4272, miR-4728-5p, miR-4443, miR-4506, miR-6773-5p, miR-4662a-5p, miR-3184-3p, miR-4281, miR-320d, miR-6729-3p, miR-5192, miR-6853-5p, miR-1234-3p, miR-1233-3p, miR-4539, miR-3914, miR-4738-5p, miR-548au-3p, miR-1539, miR-4720-3p, miR-365b-5p, miR-4486, miR-1227-5p, miR-4667-5p, miR-6088, miR-6820-5p, miR-4505, miR-548q, miR-4658, miR-450a-5p, miR-1260b, miR-3677-5p, miR-6777-3p, miR-6826-3p, miR-6832-3p, miR-4725-3p, miR-7161-3p, miR-2277-5p, miR-7110-3p, miR-4312, miR-4461, miR-6766-5p, miR-1266-3p, miR-6729-5p, miR-526b-3p, miR-519e-5p, miR-512-5p, miR-5088-5p, miR-1909-3p, miR-6511a-5p, miR-4734, miR-936, miR-1249-3p, miR-6777-5p, miR-4487, miR-3155a, miR-563, miR-4741, miR-6788-5p, miR-4433b-5p, miR-323a-5p, miR-6811-5p, miR-6721-5p, miR-5004-5p, miR-6509-3p, miR-648, miR-3917, miR-6087, miR-1470, miR-586, miR-3150a-5p, miR-105-3p, miR-7973, miR-1914-5p, miR-4749-3p, miR-15b-5p, miR-1289, miR-4433a-5p, miR-3666, miR-3186-3p, miR-4725-5p, miR-4488, miR-4474-3p, miR-6731-3p, miR-4640-3p, miR-202-5p, miR-6816-5p, miR-638, miR-6821-5p, miR-1247-3p, miR-6765-5p, miR-6800-5p, miR-3928-3p, miR-3940-5p, miR-3960, miR-6775-5p, miR-3178, miR-1202, miR-6790-5p, miR-4731-3p, miR-2681-3p, miR-6758-5p, miR-8072, miR-518d-3p, miR-3606-3p, miR-4800-5p, miR-1292-3p, miR-6784-3p, miR-4450, miR-6132, miR-4716-5p, miR-6860, miR-1268b, miR-378d, miR-4701-5p, miR-4329, miR-185-3p, miR-552-3p, miR-1273g-5p, miR-6769b-3p, miR-520a-3p, miR-4524b-5p, miR-4291, miR-6734-3p, miR-143-5p, miR-939-3p, miR-6889-3p, miR-6842-3p, miR-4511, miR-4318, miR-4653-5p, miR-6867-3p, miR-133b, miR-3196, miR-193b-3p, miR-3162-3p, miR-6819-3p, miR-1908-3p, miR-6786-5p, miR-3648, miR-4513, miR-3652, miR-4640-5p, miR-6871-5p, miR-7845-5p, miR-3138, miR-6884-5p, miR-4653-3p, miR-636, miR-4652-3p, miR-6823-5p, miR-4502, miR-7113-5p, miR-8087, miR-7154-3p, miR-5189-5p, miR-1253, miR-518c-5p, miR-7151-5p, miR-3614-3p, miR-4727-5p, miR-3682-5p, miR-5090, miR-337-3p, miR-488-5p, miR-100-5p, miR-4520-3p, miR-373-3p, miR-6499-5p, miR-3909, miR-32-5p, miR-302a-3p, miR-4686, miR-4659a-3p, miR-4287, miR-1301-5p, miR-593-3p, miR-517a-3p, miR-517b-3p, miR-142-3p, miR-1185-2-3p, miR-602, miR-527, miR-518a-5p, miR-4682, miR-28-5p, miR-4252, miR-452-5p, miR-525-5p, miR-3622a-3p, miR-6813-3p, miR-4769-3p, miR-5698, miR-1915-3p, miR-1343-5p, miR-6861-5p, miR-6781-5p, miR-4508, miR-6743-5p, miR-6726-5p, miR-4525, miR-4651, miR-6813-5p, miR-5787, miR-1290, miR-6075, miR-4758-5p, miR-4690-5p, miR-762, miR-371a-5p, miR-6765-3p, miR-6784-5p, miR-6778-5p, miR-6875-5p, miR-4534, miR-4721, miR-6756-5p, miR-615-5p, miR-6727-5p, miR-6887-5p, miR-8063, miR-6880-5p, miR-6805-3p, miR-4726-5p, miR-4710, miR-7111-5p, miR-3619-3p, miR-6795-5p, miR-1254, miR-1233-5p, miR-6836-3p, miR-6769a-5p, miR-4532, miR-365a-5p, miR-1231, miR-1228-5p, miR-4430, miR-296-3p, miR-1237-5p, miR-4466, miR-6789-5p, miR-4632-5p, miR-4745-5p, miR-4665-5p, miR-6807-5p, miR-7114-5p, miR-516a-5p, miR-769-3p, miR-3692-5p, miR-3945, miR-4433a-3p, miR-4485-3p, miR-6831-5p, miR-519c-5p, miR-551b-5p, miR-1343-3p, miR-4286, miR-4634, miR-4733-3p, and miR-6086. Further, it is possible to preferably use, as target nucleic acid(s), other dementia marker(s) that can be combined with these miRNAs (i), specifically 1, 2, 3 or more, 10 or more, preferably 3 to 30 miRNAs selected from the group consisting of (ii) miR-1225-3p, miR-3184-5p, miR-665, miR-211-5p, miR-1247-5p, miR-3656, miR-149-5p, miR-744-5p, miR-345-5p, miR-150-5p, miR-191-3p, miR-651-5p, miR-34a-5p, miR-409-5p, miR-369-5p, miR-1915-5p, miR-204-5p, miR-137, miR-382-5p, miR-517-5p, miR-532-5p, miR-22-5p, miR-1237-3p, miR-1224-3p, miR-625-3p, miR-328-3p, miR-122-5p, miR-202-3p, miR-4781-5p, miR-718, miR-342-3p, miR-26b-3p, miR-140-3p, miR-200a-3p, miR-378a-3p, miR-484, miR-296-5p, miR-205-5p, miR-431-5p, miR-150-3p, miR-423-5p, miR-575, miR-671-5p, miR-939-5p, miR-3665, miR-30d-5p, miR-30b-3p, miR-92a-3p, miR-371b-5p, and miR-486-5p. In addition to the above miRNAs (i) to (ii) or aside from the above miRNAs (i) to (ii), it is possible to preferably use, as target nucleic acid(s), 1, 2, 3 or more, 10 or more, preferably 3 to 110 miRNAs selected from the group consisting of (iii) miR-4728-5p, miR-3184-3p, miR-1233-3p, miR-6088, miR-6777-3p, miR-7110-3p, miR-936, miR-6087, miR-1202, miR-4731-3p, miR-4800-5p, miR-6784-3p, miR-4716-5p, miR-6734-3p, miR-6889-3p, miR-6867-3p, miR-3622a-3p, miR-6813-3p, miR-4769-3p, miR-5698, miR-3184-5p, miR-1471, miR-1538, miR-449b-3p, miR-1976, miR-4268, miR-4279, miR-3620-3p, miR-3944-3p, miR-3156-3p, miR-3187-5p, miR-4685-3p, miR-4695-3p, miR-4697-3p, miR-4713-5p, miR-4723-3p, miR-371b-3p, miR-3151-3p, miR-3192-3p, miR-6728-3p, miR-6736-3p, miR-6740-3p, miR-6741-3p, miR-6743-3p, miR-6747-3p, miR-6750-3p, miR-6754-3p, miR-6759-3p, miR-6761-3p, miR-6762-3p, miR-6769a-3p, miR-6776-3p, miR-6778-3p, miR-6779-3p, miR-6786-3p, miR-6787-3p, miR-6792-3p, miR-6794-3p, miR-6801-3p, miR-6802-3p, miR-6803-3p, miR-6804-3p, miR-6810-5p, miR-6823-3p, miR-6825-3p, miR-6829-3p, miR-6833-3p, miR-6834-3p, miR-6780b-3p, miR-6845-3p, miR-6862-3p, miR-6865-3p, miR-6870-3p, miR-6875-3p, miR-6877-3p, miR-6879-3p, miR-6882-3p, miR-6885-3p, miR-6886-3p, miR-6887-3p, miR-6890-3p, miR-6893-3p, miR-6894-3p, miR-7106-3p, miR-7109-3p, miR-7114-3p, miR-7155-5p, miR-7160-5p, miR-615-3p, miR-920, miR-1825, miR-675-3p, miR-1910-5p, miR-2278, miR-2682-3p, miR-3122, miR-3151-5p, miR-3175, miR-4323, miR-4326, miR-4284, miR-3605-3p, miR-3622b-5p, miR-3646, miR-3158-5p, miR-4722-3p, miR-4728-3p, miR-4747-3p, miR-4436b-5p, miR-5196-3p, miR-5589-5p, miR-345-3p, miR-642b-5p, miR-6716-3p, miR-6511b-3p, miR-208a-5p, miR-6726-3p, miR-6744-5p, miR-6782-3p, miR-6789-3p, miR-6797-3p, miR-6800-3p, miR-6806-5p, miR-6824-3p, miR-6837-5p, miR-6846-3p, miR-6858-3p, miR-6859-3p, miR-6861-3p, miR-6880-3p, miR-7111-3p, miR-7152-5p, miR-642a-5p, miR-657, miR-1236-3p, miR-764, miR-4314, miR-3675-3p, miR-5703, miR-3191-5p, miR-6511a-3p, miR-6809-3p, miR-6815-5p, miR-6857-3p, and miR-6878-3p. Also, it is possible to preferably use, as target nucleic acid(s), other dementia marker(s) that can be combined with these miRNAs (i) to (iii), specifically, 1, 2, 3 or more, 10 or more, preferably 3 to 110 miRNAs selected from the group consisting of (iv) miR-766-3p, miR-1229-3p, miR-1306-5p, miR-210-5p, miR-198, miR-485-3p, miR-668-3p, miR-532-3p, miR-877-3p, miR-1238-3p, miR-3130-5p, miR-4298, miR-4290, miR-3943, miR-346, and miR-767-3p.

Above miRNAs include any human genes containing any nucleotide sequence represented by any of SEQ ID NOs: 1 to 210, 211 to 249, 250 to 374, 375 to 390, 1315 to 1350, 1351 to 1356, 1435 to 1448, and 1449 to 1453 (i.e., respective miR-4274, miR-4272, miR-4728-5p, miR-4443, miR-4506, miR-6773-5p, miR-4662a-5p, miR-3184-3p, miR-4281, miR-320d, miR-6729-3p, miR-5192, miR-6853-5p, miR-1234-3p, miR-1233-3p, miR-4539, miR-3914, miR-4738-5p, miR-548au-3p, miR-1539, miR-4720-3p, miR-365b-5p, miR-4486, miR-1227-5p, miR-4667-5p, miR-6088, miR-6820-5p, miR-4505, miR-548q, miR-4658, miR-450a-5p, miR-1260b, miR-3677-5p, miR-6777-3p, miR-6826-3p, miR-6832-3p, miR-4725-3p, miR-7161-3p, miR-2277-5p, miR-7110-3p, miR-4312, miR-4461, miR-6766-5p, miR-1266-3p, miR-6729-5p, miR-526b-3p, miR-519e-5p, miR-512-5p, miR-5088-5p, miR-1909-3p, miR-6511a-5p, miR-4734, miR-936, miR-1249-3p, miR-6777-5p, miR-4487, miR-3155a, miR-563, miR-4741, miR-6788-5p, miR-4433b-5p, miR-323a-5p, miR-6811-5p, miR-6721-5p, miR-5004-5p, miR-6509-3p, miR-648, miR-3917, miR-6087, miR-1470, miR-586, miR-3150a-5p, miR-105-3p, miR-7973, miR-1914-5p, miR-4749-3p, miR-15b-5p, miR-1289, miR-4433a-5p, miR-3666, miR-3186-3p, miR-4725-5p, miR-4488, miR-4474-3p, miR-6731-3p, miR-4640-3p, miR-202-5p, miR-6816-5p, miR-638, miR-6821-5p, miR-1247-3p, miR-6765-5p, miR-6800-5p, miR-3928-3p, miR-3940-5p, miR-3960, miR-6775-5p, miR-3178, miR-1202, miR-6790-5p, miR-4731-3p, miR-2681-3p, miR-6758-5p, miR-8072, miR-518d-3p, miR-3606-3p, miR-4800-5p, miR-1292-3p, miR-6784-3p, miR-4450, miR-6132, miR-4716-5p, miR-6860, miR-1268b, miR-378d, miR-4701-5p, miR-4329, miR-185-3p, miR-552-3p, miR-1273g-5p, miR-6769b-3p, miR-520a-3p, miR-4524b-5p, miR-4291, miR-6734-3p, miR-143-5p, miR-939-3p, miR-6889-3p, miR-6842-3p, miR-4511, miR-4318, miR-4653-5p, miR-6867-3p, miR-133b, miR-3196, miR-193b-3p, miR-3162-3p, miR-6819-3p, miR-1908-3p, miR-6786-5p, miR-3648, miR-4513, miR-3652, miR-4640-5p, miR-6871-5p, miR-7845-5p, miR-3138, miR-6884-5p, miR-4653-3p, miR-636, miR-4652-3p, miR-6823-5p, miR-4502, miR-7113-5p, miR-8087, miR-7154-3p, miR-5189-5p, miR-1253, miR-518c-5p, miR-7151-5p, miR-3614-3p, miR-4727-5p, miR-3682-5p, miR-5090, miR-337-3p, miR-488-5p, miR-100-5p, miR-4520-3p, miR-373-3p, miR-6499-5p, miR-3909, miR-32-5p, miR-302a-3p, miR-4686, miR-4659a-3p, miR-4287, miR-1301-5p, miR-593-3p, miR-517a-3p, miR-517b-3p, miR-142-3p, miR-1185-2-3p, miR-602, miR-527, miR-518a-5p, miR-4682, miR-28-5p, miR-4252, miR-452-5p, miR-525-5p, miR-3622a-3p, miR-6813-3p, miR-4769-3p, miR-5698, miR-1915-3p, miR-1343-5p, miR-6861-5p, miR-6781-5p, miR-4508, miR-6743-5p, miR-6726-5p, miR-4525, miR-4651, miR-6813-5p, miR-5787, miR-1290, miR-6075, miR-4758-5p, miR-4690-5p, miR-762, miR-1225-3p, miR-3184-5p, miR-665, miR-211-5p, miR-1247-5p, miR-3656, miR-149-5p, miR-744-5p, miR-345-5p, miR-150-5p, miR-191-3p, miR-651-5p, miR-34a-5p, miR-409-5p, miR-369-5p, miR-1915-5p, miR-204-5p, miR-137, miR-382-5p, miR-517-5p, miR-532-5p, miR-22-5p, miR-1237-3p, miR-1224-3p, miR-625-3p, miR-328-3p, miR-122-5p, miR-202-3p, miR-4781-5p, miR-718, miR-342-3p, miR-26b-3p, miR-140-3p, miR-200a-3p, miR-378a-3p, miR-484, miR-296-5p, miR-205-5p, miR-431-5p, miR-1471, miR-1538, miR-449b-3p, miR-1976, miR-4268, miR-4279, miR-3620-3p, miR-3944-3p, miR-3156-3p, miR-3187-5p, miR-4685-3p, miR-4695-3p, miR-4697-3p, miR-4713-5p, miR-4723-3p, miR-371b-3p, miR-3151-3p, miR-3192-3p, miR-6728-3p, miR-6736-3p, miR-6740-3p, miR-6741-3p, miR-6743-3p, miR-6747-3p, miR-6750-3p, miR-6754-3p, miR-6759-3p, miR-6761-3p, miR-6762-3p, miR-6769a-3p, miR-6776-3p, miR-6778-3p, miR-6779-3p, miR-6786-3p, miR-6787-3p, miR-6792-3p, miR-6794-3p, miR-6801-3p, miR-6802-3p, miR-6803-3p, miR-6804-3p, miR-6810-5p, miR-6823-3p, miR-6825-3p, miR-6829-3p, miR-6833-3p, miR-6834-3p, miR-6780b-3p, miR-6845-3p, miR-6862-3p, miR-6865-3p, miR-6870-3p, miR-6875-3p, miR-6877-3p, miR-6879-3p, miR-6882-3p, miR-6885-3p, miR-6886-3p, miR-6887-3p, miR-6890-3p, miR-6893-3p, miR-6894-3p, miR-7106-3p, miR-7109-3p, miR-7114-3p, miR-7155-5p, miR-7160-5p, miR-615-3p, miR-920, miR-1825, miR-675-3p, miR-1910-5p, miR-2278, miR-2682-3p, miR-3122, miR-3151-5p, miR-3175, miR-4323, miR-4326, miR-4284, miR-3605-3p, miR-3622b-5p, miR-3646, miR-3158-5p, miR-4722-3p, miR-4728-3p, miR-4747-3p, miR-4436b-5p, miR-5196-3p, miR-5589-5p, miR-345-3p, miR-642b-5p, miR-6716-3p, miR-6511b-3p, miR-208a-5p, miR-6726-3p, miR-6744-5p, miR-6782-3p, miR-6789-3p, miR-6797-3p, miR-6800-3p, miR-6806-5p, miR-6824-3p, miR-6837-5p, miR-6846-3p, miR-6858-3p, miR-6859-3p, miR-6861-3p, miR-6880-3p, miR-7111-3p, miR-7152-5p, miR-642a-5p, miR-657, miR-1236-3p, miR-764, miR-4314, miR-3675-3p, miR-5703, miR-3191-5p, miR-6511a-3p, miR-6809-3p, miR-6815-5p, miR-6857-3p, miR-6878-3p, miR-371a-5p, miR-766-3p, miR-1229-3p, miR-1306-5p, miR-210-5p, miR-198, miR-485-3p, miR-668-3p, miR-532-3p, miR-877-3p, miR-1238-3p, miR-3130-5p, miR-4298, miR-4290, miR-3943, miR-346 or miR-767-3p, miR-6765-3p, miR-6784-5p, miR-6778-5p, miR-6875-5p, miR-4534, miR-4721, miR-6756-5p, miR-615-5p, miR-6727-5p, miR-6887-5p, miR-8063, miR-6880-5p, miR-6805-3p, miR-4726-5p, miR-4710, miR-7111-5p, miR-3619-3p, miR-6795-5p, miR-1254, miR-1233-5p, miR-6836-3p, miR-6769a-5p, miR-4532, miR-365a-5p, miR-1231, miR-1228-5p, miR-4430, miR-296-3p, miR-1237-5p, miR-4466, miR-6789-5p, miR-4632-5p, miR-4745-5p, miR-4665-5p, miR-6807-5p, and miR-7114-5p, miR-150-3p, miR-423-5p, miR-575, miR-671-5p, miR-939-5p, miR-3665, miR-516a-5p, miR-769-3p, miR-3692-5p, miR-3945, miR-4433a-3p, miR-4485-3p, miR-6831-5p, miR-519c-5p, miR-551b-5p, miR-1343-3p, miR-4286, miR-4634, miR-4733-3p, miR-6086, miR-30d-5p, miR-30b-3p, miR-92a-3p, miR-371b-5p, and miR-486-5p), any congener thereof, any transcript thereof, and any variant or derivative thereof. Here, the gene, congener, transcript, variant, and derivative are as defined above.

Preferable target nucleic acid(s) is any human gene containing any nucleotide sequence represented by any of SEQ ID NOs: 1 to 210, 211 to 249, 250 to 374, and 375 to 390, 1315 to 1350, 1351 to 1356, 1435 to 1448, and 1449 to 1453 or any transcript thereof and more preferably is the corresponding transcript(s), i.e. miRNA(s) and any precursor RNA such as pri-miRNA or pre-miRNA thereof.

Additionally, in one embodiment, the major target nucleic acids as dementia markers for detecting dementia or the presence or absence of dementia by using the nucleic acid probes or primers for detection of dementia as defined above according to the present invention include at least one miRNA selected from the group consisting of miR-5698, miR-1915-3p, miR-1343-5p, miR-6861-5p, miR-6781-5p, miR-4508, miR-6743-5p, miR-6726-5p, miR-4525, miR-4651, miR-6813-5p, miR-5787, miR-1290, miR-6075, miR-4758-5p, miR-4690-5p, miR-762, miR-371a-5p, miR-6765-3p, miR-6784-5p, miR-6778-5p, miR-6875-5p, miR-4534, miR-4721, miR-6756-5p, miR-615-5p, miR-6727-5p, miR-6887-5p, miR-8063, miR-6880-5p, miR-6805-3p, miR-4726-5p, miR-4710, miR-7111-5p, miR-3619-3p, miR-6795-5p, miR-1254, miR-1233-5p, miR-6836-3p, miR-6769a-5p, miR-4532, miR-365a-5p, miR-1231, miR-1228-5p, miR-4430, miR-296-3p, miR-1237-5p, miR-4466, miR-6789-5p, miR-4632-5p, miR-4745-5p, miR-4665-5p, miR-6807-5p, and miR-7114-5p. Further, it is possible to include other dementia markers that can be combined with these miRNAs, specifically 1, 2, 3 or more, 10 or more, preferably 20 to 60, and more preferably 40 to 55 miRNAs selected from the group consisting of miR-1225-3p, miR-3184-5p, miR-150-3p, miR-423-5p, miR-575, miR-671-5p, miR-939-5p, and miR-3665.

The above miRNAs include, for instance, any human genes containing any nucleotide sequences represented by any of SEQ ID NOs: 194 to 210, 374, and 1315 to 1350, and 211 to 212, and 1351 to 1356 (i.e., respective miR-5698, miR-1915-3p, miR-1343-5p, miR-6861-5p, miR-6781-5p, miR-4508, miR-6743-5p, miR-6726-5p, miR-4525, miR-4651, miR-6813-5p, miR-5787, miR-1290, miR-6075, miR-4758-5p, miR-4690-5p, miR-762, miR-371a-5p, miR-6765-3p, miR-6784-5p, miR-6778-5p, miR-6875-5p, miR-4534, miR-4721, miR-6756-5p, miR-615-5p, miR-6727-5p, miR-6887-5p, miR-8063, miR-6880-5p, miR-6805-3p, miR-4726-5p, miR-4710, miR-7111-5p, miR-3619-3p, miR-6795-5p, miR-1254, miR-1233-5p, miR-6836-3p, miR-6769a-5p, miR-4532, miR-365a-5p, miR-1231, miR-1228-5p, miR-4430, miR-296-3p, miR-1237-5p, miR-4466, miR-6789-5p, miR-4632-5p, miR-4745-5p, miR-4665-5p, miR-6807-5p and miR-7114-5p, and miR-1225-3p, miR-3184-5p, miR-150-3p, miR-423-5p, miR-575, miR-671-5p, miR-939-5p, and miR-3665), any congener thereof, any transcript thereof, and/or any variant or derivative thereof. Here, the gene, congener, transcript, variant, and derivative are as defined above.

In one embodiment, the target nucleic acid(s) is any human gene containing any nucleotide sequence represented by any of SEQ ID NOs: 194 to 210, 374, and 1315 to 1350, and 211 to 212, and 1351 to 1356 or any transcript thereof and more preferably is the corresponding transcript(s), namely miRNA(s) and any precursor RNA such as pri-miRNA or pre-miRNA thereof.

The first target gene is the hsa-miR-4274 gene, a congener thereof, a transcript thereof, or a variant or derivative thereof. None of the previously known reports show that change in the expression of the gene or the transcript thereof can serve as a marker for dementia.

The second target gene is the hsa-miR-4272 gene, a congener thereof, a transcript thereof, or a variant or derivative thereof. None of the previously known reports show that change in the expression of the gene or the transcript thereof can serve as a marker for dementia.

The third target gene is the hsa-miR-4728-5p gene, a congener thereof, a transcript thereof, or a variant or derivative thereof. None of the previously known reports show that change in the expression of the gene or the transcript thereof can serve as a marker for dementia.

The 4th target gene is the hsa-miR-4443 gene, a congener thereof, a transcript thereof, or a variant or derivative thereof. None of the previously known reports show that change in the expression of the gene or the transcript thereof can serve as a marker for dementia.

The 5th target gene is the hsa-miR-4506 gene, a congener thereof, a transcript thereof, or a variant or derivative thereof. None of the previously known reports show that change in the expression of the gene or the transcript thereof can serve as a marker for dementia.

The 6th target gene is the hsa-miR-6773-5p gene, a congener thereof, a transcript thereof, or a variant or derivative thereof. None of the previously known reports show that change in the expression of the gene or the transcript thereof can serve as a marker for dementia.

The 7th target gene is the hsa-miR-4662a-5p gene, a congener thereof, a transcript thereof, or a variant or derivative thereof. None of the previously known reports show that change in the expression of the gene or the transcript thereof can serve as a marker for dementia.

The 8th target gene is the hsa-miR-3184-3p gene, a congener thereof, a transcript thereof, or a variant or derivative thereof. None of the previously known reports show that change in the expression of the gene or the transcript thereof can serve as a marker for dementia.

The 9th target gene is the hsa-miR-4281 gene, a congener thereof, a transcript thereof, or a variant or derivative thereof. None of the previously known reports show that change in the expression of the gene or the transcript thereof can serve as a marker for dementia.

The 10th target gene is the hsa-miR-320d gene, a congener thereof, a transcript thereof, or a variant or derivative thereof. None of the previously known reports show that change in the expression of the gene or the transcript thereof can serve as a marker for dementia.

The 11th target gene is the hsa-miR-6729-3p gene, a congener thereof, a transcript thereof, or a variant or derivative thereof. None of the previously known reports show that change in the expression of the gene or the transcript thereof can serve as a marker for dementia.

The 12th target gene is the hsa-miR-5192 gene, a congener thereof, a transcript thereof, or a variant or derivative thereof. None of the previously known reports show that change in the expression of the gene or the transcript thereof can serve as a marker for dementia.

The 13th target gene is the hsa-miR-6853-5p gene, a congener thereof, a transcript thereof, or a variant or derivative thereof. None of the previously known reports show that change in the expression of the gene or the transcript thereof can serve as a marker for dementia.

The 14th target gene is the hsa-miR-1234-3p gene, a congener thereof, a transcript thereof, or a variant or derivative thereof. None of the previously known reports show that change in the expression of the gene or the transcript thereof can serve as a marker for dementia.

The 15th target gene is the hsa-miR-1233-3p gene, a congener thereof, a transcript thereof, or a variant or derivative thereof. None of the previously known reports show that change in the expression of the gene or the transcript thereof can serve as a marker for dementia.

The 16th target gene is the hsa-miR-4539 gene, a congener thereof, a transcript thereof, or a variant or derivative thereof. None of the previously known reports show that change in the expression of the gene or the transcript thereof can serve as a marker for dementia.

The 17th target gene is the hsa-miR-3914 gene, a congener thereof, a transcript thereof, or a variant or derivative thereof. None of the previously known reports show that change in the expression of the gene or the transcript thereof can serve as a marker for dementia.

The 18th target gene is the hsa-miR-4738-5p gene, a congener thereof, a transcript thereof, or a variant or derivative thereof. None of the previously known reports show that change in the expression of the gene or the transcript thereof can serve as a marker for dementia.

The 19th target gene is the hsa-miR-548au-3p gene, a congener thereof, a transcript thereof, or a variant or derivative thereof. None of the previously known reports show that change in the expression of the gene or the transcript thereof can serve as a marker for dementia.

The 20th target gene is the hsa-miR-1539 gene, a congener thereof, a transcript thereof, or a variant or derivative thereof. None of the previously known reports show that change in the expression of the gene or the transcript thereof can serve as a marker for dementia.

The 21st target gene is the hsa-miR-4720-3p gene, a congener thereof, a transcript thereof, or a variant or derivative thereof. None of the previously known reports show that change in the expression of the gene or the transcript thereof can serve as a marker for dementia.

The 22nd target gene is the hsa-miR-365b-5p gene, a congener thereof, a transcript thereof, or a variant or derivative thereof. None of the previously known reports show that change in the expression of the gene or the transcript thereof can serve as a marker for dementia.

The 23rd target gene is the hsa-miR-4486 gene, a congener thereof, a transcript thereof, or a variant or derivative thereof. None of the previously known reports show that change in the expression of the gene or the transcript thereof can serve as a marker for dementia.

The 24th target gene is the hsa-miR-1227-5p gene, a congener thereof, a transcript thereof, or a variant or derivative thereof. None of the previously known reports show that change in the expression of the gene or the transcript thereof can serve as a marker for dementia.

The 25th target gene is the hsa-miR-4667-5p gene, a congener thereof, a transcript thereof, or a variant or derivative thereof. None of the previously known reports show that change in the expression of the gene or the transcript thereof can serve as a marker for dementia.

The 26th target gene is the hsa-miR-6088 gene, a congener thereof, a transcript thereof, or a variant or derivative thereof. None of the previously known reports show that change in the expression of the gene or the transcript thereof can serve as a marker for dementia.

The 27th target gene is the hsa-miR-6820-5p gene, a congener thereof, a transcript thereof, or a variant or derivative thereof. None of the previously known reports show that change in the expression of the gene or the transcript thereof can serve as a marker for dementia.

The 28th target gene is the hsa-miR-4505 gene, a congener thereof, a transcript thereof, or a variant or derivative thereof. None of the previously known reports show that change in the expression of the gene or the transcript thereof can serve as a marker for dementia.

The 29th target gene is the hsa-miR-548q gene, a congener thereof, a transcript thereof, or a variant or derivative thereof. None of the previously known reports show that change in the expression of the gene or the transcript thereof can serve as a marker for dementia.

The 30th target gene is the hsa-miR-4658 gene, a congener thereof, a transcript thereof, or a variant or derivative thereof. None of the previously known reports show that change in the expression of the gene or the transcript thereof can serve as a marker for dementia.

The 31st target gene is the hsa-miR-450a-5p gene, a congener thereof, a transcript thereof, or a variant or derivative thereof. None of the previously known reports show that change in the expression of the gene or the transcript thereof can serve as a marker for dementia.

The 32nd target gene is the hsa-miR-1260b gene, a congener thereof, a transcript thereof, or a variant or derivative thereof. None of the previously known reports show that change in the expression of the gene or the transcript thereof can serve as a marker for dementia.

The 33rd target gene is the hsa-miR-3677-5p gene, a congener thereof, a transcript thereof, or a variant or derivative thereof. None of the previously known reports show that change in the expression of the gene or the transcript thereof can serve as a marker for dementia.

The 34th target gene is the hsa-miR-6777-3p gene, a congener thereof, a transcript thereof, or a variant or derivative thereof. None of the previously known reports show that change in the expression of the gene or the transcript thereof can serve as a marker for dementia.

The 35th target gene is the hsa-miR-6826-3p gene, a congener thereof, a transcript thereof, or a variant or derivative thereof. None of the previously known reports show that change in the expression of the gene or the transcript thereof can serve as a marker for dementia.

The 36th target gene is the hsa-miR-6832-3p gene, a congener thereof, a transcript thereof, or a variant or derivative thereof. None of the previously known reports show that change in the expression of the gene or the transcript thereof can serve as a marker for dementia.

The 37th target gene is the hsa-miR-4725-3p gene, a congener thereof, a transcript thereof, or a variant or derivative thereof. None of the previously known reports show that change in the expression of the gene or the transcript thereof can serve as a marker for dementia.

The 38th target gene is the hsa-miR-7161-3p gene, a congener thereof, a transcript thereof, or a variant or derivative thereof. None of the previously known reports show that change in the expression of the gene or the transcript thereof can serve as a marker for dementia.

The 39th target gene is the hsa-miR-2277-5p gene, a congener thereof, a transcript thereof, or a variant or derivative thereof. None of the previously known reports show that change in the expression of the gene or the transcript thereof can serve as a marker for dementia.

The 40th target gene is the hsa-miR-7110-3p gene, a congener thereof, a transcript thereof, or a variant or derivative thereof. None of the previously known reports show that change in the expression of the gene or the transcript thereof can serve as a marker for dementia.

The 41st target gene is the hsa-miR-4312 gene, a congener thereof, a transcript thereof, or a variant or derivative thereof. None of the previously known reports show that change in the expression of the gene or the transcript thereof can serve as a marker for dementia.

The 42nd target gene is the hsa-miR-4461 gene, a congener thereof, a transcript thereof, or a variant or derivative thereof. None of the previously known reports show that change in the expression of the gene or the transcript thereof can serve as a marker for dementia.

The 43rd target gene is the hsa-miR-6766-5p gene, a congener thereof, a transcript thereof, or a variant or derivative thereof. None of the previously known reports show that change in the expression of the gene or the transcript thereof can serve as a marker for dementia.

The 44th target gene is the hsa-miR-1266-3p gene, a congener thereof, a transcript thereof, or a variant or derivative thereof. None of the previously known reports show that change in the expression of the gene or the transcript thereof can serve as a marker for dementia.

The 45th target gene is the hsa-miR-6729-5p gene, a congener thereof, a transcript thereof, or a variant or derivative thereof. None of the previously known reports show that change in the expression of the gene or the transcript thereof can serve as a marker for dementia.

The 46th target gene is the hsa-miR-526b-3p gene, a congener thereof, a transcript thereof, or a variant or derivative thereof. None of the previously known reports show that change in the expression of the gene or the transcript thereof can serve as a marker for dementia.

The 47th target gene is the hsa-miR-519e-5p gene, a congener thereof, a transcript thereof, or a variant or derivative thereof. None of the previously known reports show that change in the expression of the gene or the transcript thereof can serve as a marker for dementia.

The 48th target gene is the hsa-miR-512-5p gene, a congener thereof, a transcript thereof, or a variant or derivative thereof. None of the previously known reports show that change in the expression of the gene or the transcript thereof can serve as a marker for dementia.

The 49th target gene is the hsa-miR-5088-5p gene, a congener thereof, a transcript thereof, or a variant or derivative thereof. None of the previously known reports show that change in the expression of the gene or the transcript thereof can serve as a marker for dementia.

The 50th target gene is the hsa-miR-1909-3p gene, a congener thereof, a transcript thereof, or a variant or derivative thereof. None of the previously known reports show that change in the expression of the gene or the transcript thereof can serve as a marker for dementia.

The 51st target gene is the hsa-miR-6511a-5p gene, a congener thereof, a transcript thereof, or a variant or derivative thereof. None of the previously known reports show that change in the expression of the gene or the transcript thereof can serve as a marker for dementia.

The 52nd target gene is the hsa-miR-4734 gene, a congener thereof, a transcript thereof, or a variant or derivative thereof. None of the previously known reports show that change in the expression of the gene or the transcript thereof can serve as a marker for dementia.

The 53rd target gene is the hsa-miR-936 gene, a congener thereof, a transcript thereof, or a variant or derivative thereof. None of the previously known reports show that change in the expression of the gene or the transcript thereof can serve as a marker for dementia.

The 54th target gene is the hsa-miR-1249-3p gene, a congener thereof, a transcript thereof, or a variant or derivative thereof. None of the previously known reports show that change in the expression of the gene or the transcript thereof can serve as a marker for dementia.

The 55th target gene is the hsa-miR-6777-5p gene, a congener thereof, a transcript thereof, or a variant or derivative thereof. None of the previously known reports show that change in the expression of the gene or the transcript thereof can serve as a marker for dementia.

The 56th target gene is the hsa-miR-4487 gene, a congener thereof, a transcript thereof, or a variant or derivative thereof. None of the previously known reports show that change in the expression of the gene or the transcript thereof can serve as a marker for dementia.

The 57th target gene is the hsa-miR-3155a gene, a congener thereof, a transcript thereof, or a variant or derivative thereof. None of the previously known reports show that change in the expression of the gene or the transcript thereof can serve as a marker for dementia.

The 58th target gene is the hsa-miR-563 gene, a congener thereof, a transcript thereof, or a variant or derivative thereof. None of the previously known reports show that change in the expression of the gene or the transcript thereof can serve as a marker for dementia.

The 59th target gene is the hsa-miR-4741 gene, a congener thereof, a transcript thereof, or a variant or derivative thereof. None of the previously known reports show that change in the expression of the gene or the transcript thereof can serve as a marker for dementia.

The 60th target gene is the hsa-miR-6788-5p gene, a congener thereof, a transcript thereof, or a variant or derivative thereof. None of the previously known reports show that change in the expression of the gene or the transcript thereof can serve as a marker for dementia.

The 61st target gene is the hsa-miR-4433b-5p gene, a congener thereof, a transcript thereof, or a variant or derivative thereof. None of the previously known reports show that change in the expression of the gene or the transcript thereof can serve as a marker for dementia.

The 62nd target gene is the hsa-miR-323a-5p gene, a congener thereof, a transcript thereof, or a variant or derivative thereof. None of the previously known reports show that change in the expression of the gene or the transcript thereof can serve as a marker for dementia.

The 63rd target gene is the hsa-miR-6811-5p gene, a congener thereof, a transcript thereof, or a variant or derivative thereof. None of the previously known reports show that change in the expression of the gene or the transcript thereof can serve as a marker for dementia.

The 64th target gene is the hsa-miR-6721-5p gene, a congener thereof, a transcript thereof, or a variant or derivative thereof. None of the previously known reports show that change in the expression of the gene or the transcript thereof can serve as a marker for dementia.

The 65th target gene is the hsa-miR-5004-5p gene, a congener thereof, a transcript thereof, or a variant or derivative thereof. None of the previously known reports show that change in the expression of the gene or the transcript thereof can serve as a marker for dementia.

The 66th target gene is the hsa-miR-6509-3p gene, a congener thereof, a transcript thereof, or a variant or derivative thereof. None of the previously known reports show that change in the expression of the gene or the transcript thereof can serve as a marker for dementia.

The 67th target gene is the hsa-miR-648 gene, a congener thereof, a transcript thereof, or a variant or derivative thereof. None of the previously known reports show that change in the expression of the gene or the transcript thereof can serve as a marker for dementia.

The 68th target gene is the hsa-miR-3917 gene, a congener thereof, a transcript thereof, or a variant or derivative thereof. None of the previously known reports show that change in the expression of the gene or the transcript thereof can serve as a marker for dementia.

The 69th target gene is the hsa-miR-6087 gene, a congener thereof, a transcript thereof, or a variant or derivative thereof. None of the previously known reports show that change in the expression of the gene or the transcript thereof can serve as a marker for dementia.

The 70th target gene is the hsa-miR-1470 gene, a congener thereof, a transcript thereof, or a variant or derivative thereof. None of the previously known reports show that change in the expression of the gene or the transcript thereof can serve as a marker for dementia.

The 71st target gene is the hsa-miR-586 gene, a congener thereof, a transcript thereof, or a variant or derivative thereof. None of the previously known reports show that change in the expression of the gene or the transcript thereof can serve as a marker for dementia.

The 72nd target gene is the hsa-miR-3150a-5p gene, a congener thereof, a transcript thereof, or a variant or derivative thereof. None of the previously known reports show that change in the expression of the gene or the transcript thereof can serve as a marker for dementia.

The 73rd target gene is the hsa-miR-105-3p gene, a congener thereof, a transcript thereof, or a variant or derivative thereof. None of the previously known reports show that change in the expression of the gene or the transcript thereof can serve as a marker for dementia.

The 74th target gene is the hsa-miR-7973 gene, a congener thereof, a transcript thereof, or a variant or derivative thereof. None of the previously known reports show that change in the expression of the gene or the transcript thereof can serve as a marker for dementia.

The 75th target gene is the hsa-miR-1914-5p gene, a congener thereof, a transcript thereof, or a variant or derivative thereof. None of the previously known reports show that change in the expression of the gene or the transcript thereof can serve as a marker for dementia.

The 76th target gene is the hsa-miR-4749-3p gene, a congener thereof, a transcript thereof, or a variant or derivative thereof. None of the previously known reports show that change in the expression of the gene or the transcript thereof can serve as a marker for dementia.

The 77th target gene is the hsa-miR-15b-5p gene, a congener thereof, a transcript thereof, or a variant or derivative thereof. None of the previously known reports show that change in the expression of the gene or the transcript thereof can serve as a marker for dementia.

The 78th target gene is the hsa-miR-1289 gene, a congener thereof, a transcript thereof, or a variant or derivative thereof. None of the previously known reports show that change in the expression of the gene or the transcript thereof can serve as a marker for dementia.

The 79th target gene is the hsa-miR-4433a-5p gene, a congener thereof, a transcript thereof, or a variant or derivative thereof. None of the previously known reports show that change in the expression of the gene or the transcript thereof can serve as a marker for dementia.

The 80th target gene is the hsa-miR-3666 gene, a congener thereof, a transcript thereof, or a variant or derivative thereof. None of the previously known reports show that change in the expression of the gene or the transcript thereof can serve as a marker for dementia.

The 81st target gene is the hsa-miR-3186-3p gene, a congener thereof, a transcript thereof, or a variant or derivative thereof. None of the previously known reports show that change in the expression of the gene or the transcript thereof can serve as a marker for dementia.

The 82nd target gene is the hsa-miR-4725-5p gene, a congener thereof, a transcript thereof, or a variant or derivative thereof. None of the previously known reports show that change in the expression of the gene or the transcript thereof can serve as a marker for dementia.

The 83rd target gene is the hsa-miR-4488 gene, a congener thereof, a transcript thereof, or a variant or derivative thereof. None of the previously known reports show that change in the expression of the gene or the transcript thereof can serve as a marker for dementia.

The 84th target gene is the hsa-miR-4474-3p gene, a congener thereof, a transcript thereof, or a variant or derivative thereof. None of the previously known reports show that change in the expression of the gene or the transcript thereof can serve as a marker for dementia.

The 85th target gene is the hsa-miR-6731-3p gene, a congener thereof, a transcript thereof, or a variant or derivative thereof. None of the previously known reports show that change in the expression of the gene or the transcript thereof can serve as a marker for dementia.

The 86th target gene is the hsa-miR-4640-3p gene, a congener thereof, a transcript thereof, or a variant or derivative thereof. None of the previously known reports show that change in the expression of the gene or the transcript thereof can serve as a marker for dementia.

The 87th target gene is the hsa-miR-202-5p gene, a congener thereof, a transcript thereof, or a variant or derivative thereof. None of the previously known reports show that change in the expression of the gene or the transcript thereof can serve as a marker for dementia.

The 88th target gene is the hsa-miR-6816-5p gene, a congener thereof, a transcript thereof, or a variant or derivative thereof. None of the previously known reports show that change in the expression of the gene or the transcript thereof can serve as a marker for dementia.

The 89th target gene is the hsa-miR-638 gene, a congener thereof, a transcript thereof, or a variant or derivative thereof. None of the previously known reports show that change in the expression of the gene or the transcript thereof can serve as a marker for dementia.

The 90th target gene is the hsa-miR-6821-5p gene, a congener thereof, a transcript thereof, or a variant or derivative thereof. None of the previously known reports show that change in the expression of the gene or the transcript thereof can serve as a marker for dementia.

The 91st target gene is the hsa-miR-1247-3p gene, a congener thereof, a transcript thereof, or a variant or derivative thereof. None of the previously known reports show that change in the expression of the gene or the transcript thereof can serve as a marker for dementia.

The 92nd target gene is the hsa-miR-6765-5p gene, a congener thereof, a transcript thereof, or a variant or derivative thereof. None of the previously known reports show that change in the expression of the gene or the transcript thereof can serve as a marker for dementia.

The 93rd target gene is the hsa-miR-6800-5p gene, a congener thereof, a transcript thereof, or a variant or derivative thereof. None of the previously known reports show that change in the expression of the gene or the transcript thereof can serve as a marker for dementia.

The 94th target gene is the hsa-miR-3928-3p gene, a congener thereof, a transcript thereof, or a variant or derivative thereof. None of the previously known reports show that change in the expression of the gene or the transcript thereof can serve as a marker for dementia.

The 95th target gene is the hsa-miR-3940-5p gene, a congener thereof, a transcript thereof, or a variant or derivative thereof. None of the previously known reports show that change in the expression of the gene or the transcript thereof can serve as a marker for dementia.

The 96th target gene is the hsa-miR-3960 gene, a congener thereof, a transcript thereof, or a variant or derivative thereof. None of the previously known reports show that change in the expression of the gene or the transcript thereof can serve as a marker for dementia.

The 97th target gene is the hsa-miR-6775-5p gene, a congener thereof, a transcript thereof, or a variant or derivative thereof. None of the previously known reports show that change in the expression of the gene or the transcript thereof can serve as a marker for dementia.

The 98th target gene is the hsa-miR-3178 gene, a congener thereof, a transcript thereof, or a variant or derivative thereof. None of the previously known reports show that change in the expression of the gene or the transcript thereof can serve as a marker for dementia.

The 99th target gene is the hsa-miR-1202 gene, a congener thereof, a transcript thereof, or a variant or derivative thereof. None of the previously known reports show that change in the expression of the gene or the transcript thereof can serve as a marker for dementia.

The 100th target gene is the hsa-miR-6790-5p gene, a congener thereof, a transcript thereof, or a variant or derivative thereof. None of the previously known reports show that change in the expression of the gene or the transcript thereof can serve as a marker for dementia.

The 101st target gene is the hsa-miR-4731-3p gene, a congener thereof, a transcript thereof, or a variant or derivative thereof. None of the previously known reports show that change in the expression of the gene or the transcript thereof can serve as a marker for dementia.

The 102nd target gene is the hsa-miR-2681-3p gene, a congener thereof, a transcript thereof, or a variant or derivative thereof. None of the previously known reports show that change in the expression of the gene or the transcript thereof can serve as a marker for dementia.

The 103rd target gene is the hsa-miR-6758-5p gene, a congener thereof, a transcript thereof, or a variant or derivative thereof. None of the previously known reports show that change in the expression of the gene or the transcript thereof can serve as a marker for dementia.

The 104th target gene is the hsa-miR-8072 gene, a congener thereof, a transcript thereof, or a variant or derivative thereof. None of the previously known reports show that change in the expression of the gene or the transcript thereof can serve as a marker for dementia.

The 105th target gene is the hsa-miR-518d-3p gene, a congener thereof, a transcript thereof, or a variant or derivative thereof. None of the previously known reports show that change in the expression of the gene or the transcript thereof can serve as a marker for dementia.

The 106th target gene is the hsa-miR-3606-3p gene, a congener thereof, a transcript thereof, or a variant or derivative thereof. None of the previously known reports show that change in the expression of the gene or the transcript thereof can serve as a marker for dementia.

The 107th target gene is the hsa-miR-4800-5p gene, a congener thereof, a transcript thereof, or a variant or derivative thereof. None of the previously known reports show that change in the expression of the gene or the transcript thereof can serve as a marker for dementia.

The 108th target gene is the hsa-miR-1292-3p gene, a congener thereof, a transcript thereof, or a variant or derivative thereof. None of the previously known reports show that change in the expression of the gene or the transcript thereof can serve as a marker for dementia.

The 109th target gene is the hsa-miR-6784-3p gene, a congener thereof, a transcript thereof, or a variant or derivative thereof. None of the previously known reports show that change in the expression of the gene or the transcript thereof can serve as a marker for dementia.

The 110th target gene is the hsa-miR-4450 gene, a congener thereof, a transcript thereof, or a variant or derivative thereof. None of the previously known reports show that change in the expression of the gene or the transcript thereof can serve as a marker for dementia.

The 111st target gene is the hsa-miR-6132 gene, a congener thereof, a transcript thereof, or a variant or derivative thereof. None of the previously known reports show that change in the expression of the gene or the transcript thereof can serve as a marker for dementia.

The 112nd target gene is the hsa-miR-4716-5p gene, a congener thereof, a transcript thereof, or a variant or derivative thereof. None of the previously known reports show that change in the expression of the gene or the transcript thereof can serve as a marker for dementia.

The 113rd target gene is the hsa-miR-6860 gene, a congener thereof, a transcript thereof, or a variant or derivative thereof. None of the previously known reports show that change in the expression of the gene or the transcript thereof can serve as a marker for dementia.

The 114th target gene is the hsa-miR-1268b gene, a congener thereof, a transcript thereof, or a variant or derivative thereof. None of the previously known reports show that change in the expression of the gene or the transcript thereof can serve as a marker for dementia.

The 115th target gene is the hsa-miR-378d gene, a congener thereof, a transcript thereof, or a variant or derivative thereof. None of the previously known reports show that change in the expression of the gene or the transcript thereof can serve as a marker for dementia.

The 116th target gene is the hsa-miR-4701-5p gene, a congener thereof, a transcript thereof, or a variant or derivative thereof. None of the previously known reports show that change in the expression of the gene or the transcript thereof can serve as a marker for dementia.

The 117th target gene is the hsa-miR-4329 gene, a congener thereof, a transcript thereof, or a variant or derivative thereof. None of the previously known reports show that change in the expression of the gene or the transcript thereof can serve as a marker for dementia.

The 118th target gene is the hsa-miR-185-3p gene, a congener thereof, a transcript thereof, or a variant or derivative thereof. None of the previously known reports show that change in the expression of the gene or the transcript thereof can serve as a marker for dementia.

The 119th target gene is the hsa-miR-552-3p gene, a congener thereof, a transcript thereof, or a variant or derivative thereof. None of the previously known reports show that change in the expression of the gene or the transcript thereof can serve as a marker for dementia.

The 120th target gene is the hsa-miR-1273g-5p gene, a congener thereof, a transcript thereof, or a variant or derivative thereof. None of the previously known reports show that change in the expression of the gene or the transcript thereof can serve as a marker for dementia.

The 121st target gene is the hsa-miR-6769b-3p gene, a congener thereof, a transcript thereof, or a variant or derivative thereof. None of the previously known reports show that change in the expression of the gene or the transcript thereof can serve as a marker for dementia.

The 122nd target gene is the hsa-miR-520a-3p gene, a congener thereof, a transcript thereof, or a variant or derivative thereof. None of the previously known reports show that change in the expression of the gene or the transcript thereof can serve as a marker for dementia.

The 123rd target gene is the hsa-miR-4524b-5p gene, a congener thereof, a transcript thereof, or a variant or derivative thereof. None of the previously known reports show that change in the expression of the gene or the transcript thereof can serve as a marker for dementia.

The 124th target gene is the hsa-miR-4291 gene, a congener thereof, a transcript thereof, or a variant or derivative thereof. None of the previously known reports show that change in the expression of the gene or the transcript thereof can serve as a marker for dementia.

The 125th target gene is the hsa-miR-6734-3p gene, a congener thereof, a transcript thereof, or a variant or derivative thereof. None of the previously known reports show that change in the expression of the gene or the transcript thereof can serve as a marker for dementia.

The 126th target gene is the hsa-miR-143-5p gene, a congener thereof, a transcript thereof, or a variant or derivative thereof. None of the previously known reports show that change in the expression of the gene or the transcript thereof can serve as a marker for dementia.

The 127th target gene is the hsa-miR-939-3p gene, a congener thereof, a transcript thereof, or a variant or derivative thereof. None of the previously known reports show that change in the expression of the gene or the transcript thereof can serve as a marker for dementia.

The 128th target gene is the hsa-miR-6889-3p gene, a congener thereof, a transcript thereof, or a variant or derivative thereof. None of the previously known reports show that change in the expression of the gene or the transcript thereof can serve as a marker for dementia.

The 129th target gene is the hsa-miR-6842-3p gene, a congener thereof, a transcript thereof, or a variant or derivative thereof. None of the previously known reports show that change in the expression of the gene or the transcript thereof can serve as a marker for dementia.

The 130th target gene is the hsa-miR-4511 gene, a congener thereof, a transcript thereof, or a variant or derivative thereof. None of the previously known reports show that change in the expression of the gene or the transcript thereof can serve as a marker for dementia.

The 131st target gene is the hsa-miR-4318 gene, a congener thereof, a transcript thereof, or a variant or derivative thereof. None of the previously known reports show that change in the expression of the gene or the transcript thereof can serve as a marker for dementia.

The 132nd target gene is the hsa-miR-4653-5p gene, a congener thereof, a transcript thereof, or a variant or derivative thereof. None of the previously known reports show that change in the expression of the gene or the transcript thereof can serve as a marker for dementia.

The 133rd target gene is the hsa-miR-6867-3p gene, a congener thereof, a transcript thereof, or a variant or derivative thereof. None of the previously known reports show that change in the expression of the gene or the transcript thereof can serve as a marker for dementia.

The 134th target gene is the hsa-miR-133b gene, a congener thereof, a transcript thereof, or a variant or derivative thereof. None of the previously known reports show that change in the expression of the gene or the transcript thereof can serve as a marker for dementia.

The 135th target gene is the hsa-miR-3196 gene, a congener thereof, a transcript thereof, or a variant or derivative thereof. None of the previously known reports show that change in the expression of the gene or the transcript thereof can serve as a marker for dementia.

The 136th target gene is the hsa-miR-193b-3p gene, a congener thereof, a transcript thereof, or a variant or derivative thereof. None of the previously known reports show that change in the expression of the gene or the transcript thereof can serve as a marker for dementia.

The 137th target gene is the hsa-miR-3162-3p gene, a congener thereof, a transcript thereof, or a variant or derivative thereof. None of the previously known reports show that change in the expression of the gene or the transcript thereof can serve as a marker for dementia.

The 138th target gene is the hsa-miR-6819-3p gene, a congener thereof, a transcript thereof, or a variant or derivative thereof. None of the previously known reports show that change in the expression of the gene or the transcript thereof can serve as a marker for dementia.

The 139th target gene is the hsa-miR-1908-3p gene, a congener thereof, a transcript thereof, or a variant or derivative thereof. None of the previously known reports show that change in the expression of the gene or the transcript thereof can serve as a marker for dementia.

The 140th target gene is the hsa-miR-6786-5p gene, a congener thereof, a transcript thereof, or a variant or derivative thereof. None of the previously known reports show that change in the expression of the gene or the transcript thereof can serve as a marker for dementia.

The 141st target gene is the hsa-miR-3648 gene, a congener thereof, a transcript thereof, or a variant or derivative thereof. None of the previously known reports show that change in the expression of the gene or the transcript thereof can serve as a marker for dementia.

The 142nd target gene is the hsa-miR-4513 gene, a congener thereof, a transcript thereof, or a variant or derivative thereof. None of the previously known reports show that change in the expression of the gene or the transcript thereof can serve as a marker for dementia.

The 143rd target gene is the hsa-miR-3652 gene, a congener thereof, a transcript thereof, or a variant or derivative thereof. None of the previously known reports show that change in the expression of the gene or the transcript thereof can serve as a marker for dementia.

The 144th target gene is the hsa-miR-4640-5p gene, a congener thereof, a transcript thereof, or a variant or derivative thereof. None of the previously known reports show that change in the expression of the gene or the transcript thereof can serve as a marker for dementia.

The 145th target gene is the hsa-miR-6871-5p gene, a congener thereof, a transcript thereof, or a variant or derivative thereof. None of the previously known reports show that change in the expression of the gene or the transcript thereof can serve as a marker for dementia.

The 146th target gene is the hsa-miR-7845-5p gene, a congener thereof, a transcript thereof, or a variant or derivative thereof. None of the previously known reports show that change in the expression of the gene or the transcript thereof can serve as a marker for dementia.

The 147th target gene is the hsa-miR-3138 gene, a congener thereof, a transcript thereof, or a variant or derivative thereof. None of the previously known reports show that change in the expression of the gene or the transcript thereof can serve as a marker for dementia.

The 148th target gene is the hsa-miR-6884-5p gene, a congener thereof, a transcript thereof, or a variant or derivative thereof. None of the previously known reports show that change in the expression of the gene or the transcript thereof can serve as a marker for dementia.

The 149th target gene is the hsa-miR-4653-3p gene, a congener thereof, a transcript thereof, or a variant or derivative thereof. None of the previously known reports show that change in the expression of the gene or the transcript thereof can serve as a marker for dementia.

The 150th target gene is the hsa-miR-636 gene, a congener thereof, a transcript thereof, or a variant or derivative thereof. None of the previously known reports show that change in the expression of the gene or the transcript thereof can serve as a marker for dementia.

The 151st target gene is the hsa-miR-4652-3p gene, a congener thereof, a transcript thereof, or a variant or derivative thereof. None of the previously known reports show that change in the expression of the gene or the transcript thereof can serve as a marker for dementia.

The 152nd target gene is the hsa-miR-6823-5p gene, a congener thereof, a transcript thereof, or a variant or derivative thereof. None of the previously known reports show that change in the expression of the gene or the transcript thereof can serve as a marker for dementia.

The 153rd target gene is the hsa-miR-4502 gene, a congener thereof, a transcript thereof, or a variant or derivative thereof. None of the previously known reports show that change in the expression of the gene or the transcript thereof can serve as a marker for dementia.

The 154th target gene is the hsa-miR-7113-5p gene, a congener thereof, a transcript thereof, or a variant or derivative thereof. None of the previously known reports show that change in the expression of the gene or the transcript thereof can serve as a marker for dementia.

The 155th target gene is the hsa-miR-8087 gene, a congener thereof, a transcript thereof, or a variant or derivative thereof. None of the previously known reports show that change in the expression of the gene or the transcript thereof can serve as a marker for dementia.

The 156th target gene is the hsa-miR-7154-3p gene, a congener thereof, a transcript thereof, or a variant or derivative thereof. None of the previously known reports show that change in the expression of the gene or the transcript thereof can serve as a marker for dementia.

The 157th target gene is the hsa-miR-5189-5p gene, a congener thereof, a transcript thereof, or a variant or derivative thereof. None of the previously known reports show that change in the expression of the gene or the transcript thereof can serve as a marker for dementia.

The 158th target gene is the hsa-miR-1253 gene, a congener thereof, a transcript thereof, or a variant or derivative thereof. None of the previously known reports show that change in the expression of the gene or the transcript thereof can serve as a marker for dementia.

The 159th target gene is the hsa-miR-518c-5p gene, a congener thereof, a transcript thereof, or a variant or derivative thereof. None of the previously known reports show that change in the expression of the gene or the transcript thereof can serve as a marker for dementia.

The 160th target gene is the hsa-miR-7151-5p gene, a congener thereof, a transcript thereof, or a variant or derivative thereof. None of the previously known reports show that change in the expression of the gene or the transcript thereof can serve as a marker for dementia.

The 161st target gene is the hsa-miR-3614-3p gene, a congener thereof, a transcript thereof, or a variant or derivative thereof. None of the previously known reports show that change in the expression of the gene or the transcript thereof can serve as a marker for dementia.

The 162nd target gene is the hsa-miR-4727-5p gene, a congener thereof, a transcript thereof, or a variant or derivative thereof. None of the previously known reports show that change in the expression of the gene or the transcript thereof can serve as a marker for dementia.

The 163rd target gene is the hsa-miR-3682-5p gene, a congener thereof, a transcript thereof, or a variant or derivative thereof. None of the previously known reports show that change in the expression of the gene or the transcript thereof can serve as a marker for dementia.

The 164th target gene is the hsa-miR-5090 gene, a congener thereof, a transcript thereof, or a variant or derivative thereof. None of the previously known reports show that change in the expression of the gene or the transcript thereof can serve as a marker for dementia.

The 165th target gene is the hsa-miR-337-3p gene, a congener thereof, a transcript thereof, or a variant or derivative thereof. None of the previously known reports show that change in the expression of the gene or the transcript thereof can serve as a marker for dementia.

The 166th target gene is the hsa-miR-488-5p gene, a congener thereof, a transcript thereof, or a variant or derivative thereof. None of the previously known reports show that change in the expression of the gene or the transcript thereof can serve as a marker for dementia.

The 167th target gene is the hsa-miR-100-5p gene, a congener thereof, a transcript thereof, or a variant or derivative thereof. None of the previously known reports show that change in the expression of the gene or the transcript thereof can serve as a marker for dementia.

The 168th target gene is the hsa-miR-4520-3p gene, a congener thereof, a transcript thereof, or a variant or derivative thereof. None of the previously known reports show that change in the expression of the gene or the transcript thereof can serve as a marker for dementia.

The 169th target gene is the hsa-miR-373-3p gene, a congener thereof, a transcript thereof, or a variant or derivative thereof. None of the previously known reports show that change in the expression of the gene or the transcript thereof can serve as a marker for dementia.

The 170th target gene is the hsa-miR-6499-5p gene, a congener thereof, a transcript thereof, or a variant or derivative thereof. None of the previously known reports show that change in the expression of the gene or the transcript thereof can serve as a marker for dementia.

The 171st target gene is the hsa-miR-3909 gene, a congener thereof, a transcript thereof, or a variant or derivative thereof. None of the previously known reports show that change in the expression of the gene or the transcript thereof can serve as a marker for dementia.

The 172nd target gene is the hsa-miR-32-5p gene, a congener thereof, a transcript thereof, or a variant or derivative thereof. None of the previously known reports show that change in the expression of the gene or the transcript thereof can serve as a marker for dementia.

The 173rd target gene is the hsa-miR-302a-3p gene, a congener thereof, a transcript thereof, or a variant or derivative thereof. None of the previously known reports show that change in the expression of the gene or the transcript thereof can serve as a marker for dementia.

The 174th target gene is the hsa-miR-4686 gene, a congener thereof, a transcript thereof, or a variant or derivative thereof. None of the previously known reports show that change in the expression of the gene or the transcript thereof can serve as a marker for dementia.

The 175th target gene is the hsa-miR-4659a-3p gene, a congener thereof, a transcript thereof, or a variant or derivative thereof. None of the previously known reports show that change in the expression of the gene or the transcript thereof can serve as a marker for dementia.

The 176th target gene is the hsa-miR-4287 gene, a congener thereof, a transcript thereof, or a variant or derivative thereof. None of the previously known reports show that change in the expression of the gene or the transcript thereof can serve as a marker for dementia.

The 177th target gene is the hsa-miR-1301-5p gene, a congener thereof, a transcript thereof, or a variant or derivative thereof. None of the previously known reports show that change in the expression of the gene or the transcript thereof can serve as a marker for dementia.

The 178th target gene is the hsa-miR-593-3p gene, a congener thereof, a transcript thereof, or a variant or derivative thereof. None of the previously known reports show that change in the expression of the gene or the transcript thereof can serve as a marker for dementia.

The 179th target gene is the hsa-miR-517a-3p gene, a congener thereof, a transcript thereof, or a variant or derivative thereof. None of the previously known reports show that change in the expression of the gene or the transcript thereof can serve as a marker for dementia.

The 180th target gene is the hsa-miR-517b-3p gene, a congener thereof, a transcript thereof, or a variant or derivative thereof. None of the previously known reports show that change in the expression of the gene or the transcript thereof can serve as a marker for dementia.

The 181 st target gene is the hsa-miR-142-3p gene, a congener thereof, a transcript thereof, or a variant or derivative thereof. None of the previously known reports show that change in the expression of the gene or the transcript thereof can serve as a marker for dementia.

The 182nd target gene is the hsa-miR-1185-2-3p gene, a congener thereof, a transcript thereof, or a variant or derivative thereof. None of the previously known reports show that change in the expression of the gene or the transcript thereof can serve as a marker for dementia.

The 183rd target gene is the hsa-miR-602 gene, a congener thereof, a transcript thereof, or a variant or derivative thereof. None of the previously known reports show that change in the expression of the gene or the transcript thereof can serve as a marker for dementia.

The 184th target gene is the hsa-miR-527 gene, a congener thereof, a transcript thereof, or a variant or derivative thereof. None of the previously known reports show that change in the expression of the gene or the transcript thereof can serve as a marker for dementia.

The 185th target gene is the hsa-miR-518a-5p gene, a congener thereof, a transcript thereof, or a variant or derivative thereof. None of the previously known reports show that change in the expression of the gene or the transcript thereof can serve as a marker for dementia.

The 186th target gene is the hsa-miR-4682 gene, a congener thereof, a transcript thereof, or a variant or derivative thereof. None of the previously known reports show that change in the expression of the gene or the transcript thereof can serve as a marker for dementia.

The 187th target gene is the hsa-miR-28-5p gene, a congener thereof, a transcript thereof, or a variant or derivative thereof. None of the previously known reports show that change in the expression of the gene or the transcript thereof can serve as a marker for dementia.

The 188th target gene is the hsa-miR-4252 gene, a congener thereof, a transcript thereof, or a variant or derivative thereof. None of the previously known reports show that change in the expression of the gene or the transcript thereof can serve as a marker for dementia.

The 189th target gene is the hsa-miR-452-5p gene, a congener thereof, a transcript thereof, or a variant or derivative thereof. None of the previously known reports show that change in the expression of the gene or the transcript thereof can serve as a marker for dementia.

The 190th target gene is the hsa-miR-525-5p gene, a congener thereof, a transcript thereof, or a variant or derivative thereof. None of the previously known reports show that change in the expression of the gene or the transcript thereof can serve as a marker for dementia.

The 191st target gene is the hsa-miR-3622a-3p gene, a congener thereof, a transcript thereof, or a variant or derivative thereof. None of the previously known reports show that change in the expression of the gene or the transcript thereof can serve as a marker for dementia.

The 192nd target gene is the hsa-miR-6813-3p gene, a congener thereof, a transcript thereof, or a variant or derivative thereof. None of the previously known reports show that change in the expression of the gene or the transcript thereof can serve as a marker for dementia.

The 193rd target gene is the hsa-miR-4769-3p gene, a congener thereof, a transcript thereof, or a variant or derivative thereof. None of the previously known reports show that change in the expression of the gene or the transcript thereof can serve as a marker for dementia.

The 194th target gene is the hsa-miR-5698 gene, a congener thereof, a transcript thereof, or a variant or derivative thereof. None of the previously known reports show that change in the expression of the gene or the transcript thereof can serve as a marker for dementia.

The 195th target gene is the hsa-miR-1915-3p gene, a congener thereof, a transcript thereof, or a variant or derivative thereof. None of the previously known reports show that change in the expression of the gene or the transcript thereof can serve as a marker for dementia.

The 196th target gene is the hsa-miR-1343-5p gene, a congener thereof, a transcript thereof, or a variant or derivative thereof. None of the previously known reports show that change in the expression of the gene or the transcript thereof can serve as a marker for dementia.

The 197th target gene is the hsa-miR-6861-5p gene, a congener thereof, a transcript thereof, or a variant or derivative thereof. None of the previously known reports show that change in the expression of the gene or the transcript thereof can serve as a marker for dementia.

The 198th target gene is the hsa-miR-6781-5p gene, a congener thereof, a transcript thereof, or a variant or derivative thereof. None of the previously known reports show that change in the expression of the gene or the transcript thereof can serve as a marker for dementia.

The 199th target gene is the hsa-miR-4508 gene, a congener thereof, a transcript thereof, or a variant or derivative thereof. None of the previously known reports show that change in the expression of the gene or the transcript thereof can serve as a marker for dementia.

The 200th target gene is the hsa-miR-6743-5p gene, a congener thereof, a transcript thereof, or a variant or derivative thereof. None of the previously known reports show that change in the expression of the gene or the transcript thereof can serve as a marker for dementia.

The 201st target gene is the hsa-miR-6726-5p gene, a congener thereof, a transcript thereof, or a variant or derivative thereof. None of the previously known reports show that change in the expression of the gene or the transcript thereof can serve as a marker for dementia.

The 202nd target gene is the hsa-miR-4525 gene, a congener thereof, a transcript thereof, or a variant or derivative thereof. None of the previously known reports show that change in the expression of the gene or the transcript thereof can serve as a marker for dementia.

The 203rd target gene is the hsa-miR-4651 gene, a congener thereof, a transcript thereof, or a variant or derivative thereof. None of the previously known reports show that change in the expression of the gene or the transcript thereof can serve as a marker for dementia.

The 204th target gene is the hsa-miR-6813-5p gene, a congener thereof, a transcript thereof, or a variant or derivative thereof. None of the previously known reports show that change in the expression of the gene or the transcript thereof can serve as a marker for dementia.

The 205th target gene is the hsa-miR-5787 gene, a congener thereof, a transcript thereof, or a variant or derivative thereof. None of the previously known reports show that change in the expression of the gene or the transcript thereof can serve as a marker for dementia.

The 206th target gene is the hsa-miR-1290 gene, a congener thereof, a transcript thereof, or a variant or derivative thereof. None of the previously known reports show that change in the expression of the gene or the transcript thereof can serve as a marker for dementia.

The 207th target gene is the hsa-miR-6075 gene, a congener thereof, a transcript thereof, or a variant or derivative thereof. None of the previously known reports show that change in the expression of the gene or the transcript thereof can serve as a marker for dementia.

The 208th target gene is the hsa-miR-4758-5p gene, a congener thereof, a transcript thereof, or a variant or derivative thereof. None of the previously known reports show that change in the expression of the gene or the transcript thereof can serve as a marker for dementia.

The 209th target gene is the hsa-miR-4690-5p gene, a congener thereof, a transcript thereof, or a variant or derivative thereof. None of the previously known reports show that change in the expression of the gene or the transcript thereof can serve as a marker for dementia.

The 210th target gene is the hsa-miR-762 gene, a congener thereof, a transcript thereof, or a variant or derivative thereof. None of the previously known reports show that change in the expression of the gene or the transcript thereof can serve as a marker for dementia.

The 211st target gene is the hsa-miR-1225-3p gene, a congener thereof, a transcript thereof, or a variant or derivative thereof. The previously known report shows that change in the expression of the gene or the transcript thereof can serve as a marker for dementia (Patent Literature 5).

The 212nd target gene is the hsa-miR-3184-5p gene, a congener thereof, a transcript thereof, or a variant or derivative thereof. The previously known report shows that change in the expression of the gene or the transcript thereof can serve as a marker for dementia (Patent Literature 5).

The 213rd target gene is the hsa-miR-665 gene, a congener thereof, a transcript thereof, or a variant or derivative thereof. The previously known report shows that change in the expression of the gene or the transcript thereof can serve as a marker for dementia (Patent Literature 3).

The 214th target gene is the hsa-miR-211-5p gene, a congener thereof, a transcript thereof, or a variant or derivative thereof. The previously known report shows that change in the expression of the gene or the transcript thereof can serve as a marker for dementia (U.S. Patent Application Publication No. 2005/0261218).

The 215th target gene is the hsa-miR-1247-5p gene, a congener thereof, a transcript thereof, or a variant or derivative thereof. The previously known report shows that change in the expression of the gene or the transcript thereof can serve as a marker for dementia (Patent Literature 5).

The 216th target gene is the hsa-miR-3656 gene, a congener thereof, a transcript thereof, or a variant or derivative thereof. The previously known report shows that change in the expression of the gene or the transcript thereof can serve as a marker for dementia (Patent Literature 7).

The 217th target gene is the hsa-miR-149-5p gene, a congener thereof, a transcript thereof, or a variant or derivative thereof. The previously known report shows that change in the expression of the gene or the transcript thereof can serve as a marker for dementia (Patent Literature 5).

The 218th target gene is the hsa-miR-744-5p gene, a congener thereof, a transcript thereof, or a variant or derivative thereof. The previously known report shows that change in the expression of the gene or the transcript thereof can serve as a marker for dementia (Patent Literature 6).

The 219th target gene is the hsa-miR-345-5p gene, a congener thereof, a transcript thereof, or a variant or derivative thereof. The previously known report shows that change in the expression of the gene or the transcript thereof can serve as a marker for dementia (Patent Literature 3).

The 220th target gene is the hsa-miR-150-5p gene, a congener thereof, a transcript thereof, or a variant or derivative thereof. The previously known report shows that change in the expression of the gene or the transcript thereof can serve as a marker for dementia (Patent Literature 7).

The 221 st target gene is the hsa-miR-191-3p gene, a congener thereof, a transcript thereof, or a variant or derivative thereof. The previously known report shows that change in the expression of the gene or the transcript thereof can serve as a marker for dementia (Patent Literature 2).

The 222nd target gene is the hsa-miR-651-5p gene, a congener thereof, a transcript thereof, or a variant or derivative thereof. The previously known report shows that change in the expression of the gene or the transcript thereof can serve as a marker for dementia (U.S. Patent Application Publication No. 2014/0303025).

The 223rd target gene is the hsa-miR-34a-5p gene, a congener thereof, a transcript thereof, or a variant or derivative thereof. The previously known report shows that change in the expression of the gene or the transcript thereof can serve as a marker for dementia (U.S. Patent Application Publication No. 2013/0040303).

The 224th target gene is the hsa-miR-409-5p gene, a congener thereof, a transcript thereof, or a variant or derivative thereof. The previously known report shows that change in the expression of the gene or the transcript thereof can serve as a marker for dementia (Patent Literature 5).

The 225th target gene is the hsa-miR-369-5p gene, a congener thereof, a transcript thereof, or a variant or derivative thereof. The previously known report shows that change in the expression of the gene or the transcript thereof can serve as a marker for dementia (Patent Literature 5).

The 226th target gene is the hsa-miR-1915-5p gene, a congener thereof, a transcript thereof, or a variant or derivative thereof. The previously known report shows that change in the expression of the gene or the transcript thereof can serve as a marker for dementia (Patent Literature 5).

The 227th target gene is the hsa-miR-204-5p gene, a congener thereof, a transcript thereof, or a variant or derivative thereof. The previously known report shows that change in the expression of the gene or the transcript thereof can serve as a marker for dementia (Patent Literature 5).

The 228th target gene is the hsa-miR-137 gene, a congener thereof, a transcript thereof, or a variant or derivative thereof. The previously known report shows that change in the expression of the gene or the transcript thereof can serve as a marker for dementia (Patent Literature 4).

The 229th target gene is the hsa-miR-382-5p gene, a congener thereof, a transcript thereof, or a variant or derivative thereof. The previously known report shows that change in the expression of the gene or the transcript thereof can serve as a marker for dementia (Patent Literature 3).

The 230th target gene is the hsa-miR-517-5p gene, a congener thereof, a transcript thereof, or a variant or derivative thereof. The previously known report shows that change in the expression of the gene or the transcript thereof can serve as a marker for dementia (U.S. Patent Application Publication No. 2013/0040303).

The 231st target gene is the hsa-miR-532-5p gene, a congener thereof, a transcript thereof, or a variant or derivative thereof. The previously known report shows that change in the expression of the gene or the transcript thereof can serve as a marker for dementia (U.S. Patent Application Publication No. 2016/0273040).

The 232nd target gene is the hsa-miR-22-5p gene, a congener thereof, a transcript thereof, or a variant or derivative thereof. The previously known report shows that change in the expression of the gene or the transcript thereof can serve as a marker for dementia (International Publication No. WO 2017/186719).

The 233rd target gene is the hsa-miR-1237-3p gene, a congener thereof, a transcript thereof, or a variant or derivative thereof. The previously known report shows that change in the expression of the gene or the transcript thereof can serve as a marker for dementia (Patent Literature 5).

The 234th target gene is the hsa-miR-1224-3p gene, a congener thereof, a transcript thereof, or a variant or derivative thereof. The previously known report shows that change in the expression of the gene or the transcript thereof can serve as a marker for dementia (Patent Literature 5).

The 235th target gene is the hsa-miR-625-3p gene, a congener thereof, a transcript thereof, or a variant or derivative thereof. The previously known report shows that change in the expression of the gene or the transcript thereof can serve as a marker for dementia (Patent Literature 1).

The 236th target gene is the hsa-miR-328-3p gene, a congener thereof, a transcript thereof, or a variant or derivative thereof. The previously known report shows that change in the expression of the gene or the transcript thereof can serve as a marker for dementia (Patent Literature 7).

The 237th target gene is the hsa-miR-122-5p gene, a congener thereof, a transcript thereof, or a variant or derivative thereof. The previously known report shows that change in the expression of the gene or the transcript thereof can serve as a marker for dementia (Patent Literature 7).

The 238th target gene is the hsa-miR-202-3p gene, a congener thereof, a transcript thereof, or a variant or derivative thereof. The previously known report shows that change in the expression of the gene or the transcript thereof can serve as a marker for dementia (Patent Literature 7).

The 239th target gene is the hsa-miR-4781-5p gene, a congener thereof, a transcript thereof, or a variant or derivative thereof. The previously known report shows that change in the expression of the gene or the transcript thereof can serve as a marker for dementia (U.S. Patent Application Publication No. 2016/0273040).

The 240th target gene is the hsa-miR-718 gene, a congener thereof, a transcript thereof, or a variant or derivative thereof. The previously known report shows that change in the expression of the gene or the transcript thereof can serve as a marker for dementia (Patent Literature 5).

The 241st target gene is the hsa-miR-342-3p gene, a congener thereof, a transcript thereof, or a variant or derivative thereof. The previously known report shows that change in the expression of the gene or the transcript thereof can serve as a marker for dementia (Patent Literature 3).

The 242nd target gene is the hsa-miR-26b-3p gene, a congener thereof, a transcript thereof, or a variant or derivative thereof. The previously known report shows that change in the expression of the gene or the transcript thereof can serve as a marker for dementia (Satoh J., et al., Biomark Insights., 2015, vol. 10, pp. 21-23).

The 243rd target gene is the hsa-miR-140-3p gene, a congener thereof, a transcript thereof, or a variant or derivative thereof. The previously known report shows that change in the expression of the gene or the transcript thereof can serve as a marker for dementia (Patent Literature 1).

The 244th target gene is the hsa-miR-200a-3p gene, a congener thereof, a transcript thereof, or a variant or derivative thereof. The previously known report shows that change in the expression of the gene or the transcript thereof can serve as a marker for dementia (International Publication No. WO 2017/186719).

The 245th target gene is the hsa-miR-378a-3p gene, a congener thereof, a transcript thereof, or a variant or derivative thereof. The previously known report shows that change in the expression of the gene or the transcript thereof can serve as a marker for dementia (Patent Literature 5).

The 246th target gene is the hsa-miR-484 gene, a congener thereof, a transcript thereof, or a variant or derivative thereof. The previously known report shows that change in the expression of the gene or the transcript thereof can serve as a marker for dementia (Patent Literature 1).

The 247th target gene is the hsa-miR-296-5p gene, a congener thereof, a transcript thereof, or a variant or derivative thereof. The previously known report shows that change in the expression of the gene or the transcript thereof can serve as a marker for dementia (Patent Literature 5).

The 248th target gene is the hsa-miR-205-5p gene, a congener thereof, a transcript thereof, or a variant or derivative thereof. The previously known report shows that change in the expression of the gene or the transcript thereof can serve as a marker for dementia (Patent Literature 7).

The 249th target gene is the hsa-miR-431-5p gene, a congener thereof, a transcript thereof, or a variant or derivative thereof. The previously known report shows that change in the expression of the gene or the transcript thereof can serve as a marker for dementia (Patent Literature 7).

The 250th target gene is the hsa-miR-1471 gene, a congener thereof, a transcript thereof, or a variant or derivative thereof. None of the previously known reports show that change in the expression of the gene or the transcript thereof can serve as a marker for dementia.

The 251st target gene is the hsa-miR-1538 gene, a congener thereof, a transcript thereof, or a variant or derivative thereof. None of the previously known reports show that change in the expression of the gene or the transcript thereof can serve as a marker for dementia.

The 252nd target gene is the hsa-miR-449b-3p gene, a congener thereof, a transcript thereof, or a variant or derivative thereof. None of the previously known reports show that change in the expression of the gene or the transcript thereof can serve as a marker for dementia.

The 253rd target gene is the hsa-miR-1976 gene, a congener thereof, a transcript thereof, or a variant or derivative thereof. None of the previously known reports show that change in the expression of the gene or the transcript thereof can serve as a marker for dementia.

The 254th target gene is the hsa-miR-4268 gene, a congener thereof, a transcript thereof, or a variant or derivative thereof. None of the previously known reports show that change in the expression of the gene or the transcript thereof can serve as a marker for dementia.

The 255th target gene is the hsa-miR-4279 gene, a congener thereof, a transcript thereof, or a variant or derivative thereof. None of the previously known reports show that change in the expression of the gene or the transcript thereof can serve as a marker for dementia.

The 256th target gene is the hsa-miR-3620-3p gene, a congener thereof, a transcript thereof, or a variant or derivative thereof. None of the previously known reports show that change in the expression of the gene or the transcript thereof can serve as a marker for dementia.

The 257th target gene is the hsa-miR-3944-3p gene, a congener thereof, a transcript thereof, or a variant or derivative thereof. None of the previously known reports show that change in the expression of the gene or the transcript thereof can serve as a marker for dementia.

The 258th target gene is the hsa-miR-3156-3p gene, a congener thereof, a transcript thereof, or a variant or derivative thereof. None of the previously known reports show that change in the expression of the gene or the transcript thereof can serve as a marker for dementia.

The 259th target gene is the hsa-miR-3187-5p gene, a congener thereof, a transcript thereof, or a variant or derivative thereof. None of the previously known reports show that change in the expression of the gene or the transcript thereof can serve as a marker for dementia.

The 260th target gene is the hsa-miR-4685-3p gene, a congener thereof, a transcript thereof, or a variant or derivative thereof. None of the previously known reports show that change in the expression of the gene or the transcript thereof can serve as a marker for dementia.

The 261st target gene is the hsa-miR-4695-3p gene, a congener thereof, a transcript thereof, or a variant or derivative thereof. None of the previously known reports show that change in the expression of the gene or the transcript thereof can serve as a marker for dementia.

The 262nd target gene is the hsa-miR-4697-3p gene, a congener thereof, a transcript thereof, or a variant or derivative thereof. None of the previously known reports show that change in the expression of the gene or the transcript thereof can serve as a marker for dementia.

The 263rd target gene is the hsa-miR-4713-5p gene, a congener thereof, a transcript thereof, or a variant or derivative thereof. None of the previously known reports show that change in the expression of the gene or the transcript thereof can serve as a marker for dementia.

The 264th target gene is the hsa-miR-4723-3p gene, a congener thereof, a transcript thereof, or a variant or derivative thereof. None of the previously known reports show that change in the expression of the gene or the transcript thereof can serve as a marker for dementia.

The 265th target gene is the hsa-miR-371b-3p gene, a congener thereof, a transcript thereof, or a variant or derivative thereof. None of the previously known reports show that change in the expression of the gene or the transcript thereof can serve as a marker for dementia.

The 266th target gene is the hsa-miR-3151-3p gene, a congener thereof, a transcript thereof, or a variant or derivative thereof. None of the previously known reports show that change in the expression of the gene or the transcript thereof can serve as a marker for dementia.

The 267th target gene is the hsa-miR-3192-3p gene, a congener thereof, a transcript thereof, or a variant or derivative thereof. None of the previously known reports show that change in the expression of the gene or the transcript thereof can serve as a marker for dementia.

The 268th target gene is the hsa-miR-6728-3p gene, a congener thereof, a transcript thereof, or a variant or derivative thereof. None of the previously known reports show that change in the expression of the gene or the transcript thereof can serve as a marker for dementia.

The 269th target gene is the hsa-miR-6736-3p gene, a congener thereof, a transcript thereof, or a variant or derivative thereof. None of the previously known reports show that change in the expression of the gene or the transcript thereof can serve as a marker for dementia.

The 270th target gene is the hsa-miR-6740-3p gene, a congener thereof, a transcript thereof, or a variant or derivative thereof. None of the previously known reports show that change in the expression of the gene or the transcript thereof can serve as a marker for dementia.

The 271st target gene is the hsa-miR-6741-3p gene, a congener thereof, a transcript thereof, or a variant or derivative thereof. None of the previously known reports show that change in the expression of the gene or the transcript thereof can serve as a marker for dementia.

The 272nd target gene is the hsa-miR-6743-3p gene, a congener thereof, a transcript thereof, or a variant or derivative thereof. None of the previously known reports show that change in the expression of the gene or the transcript thereof can serve as a marker for dementia.

The 273rd target gene is the hsa-miR-6747-3p gene, a congener thereof, a transcript thereof, or a variant or derivative thereof. None of the previously known reports show that change in the expression of the gene or the transcript thereof can serve as a marker for dementia.

The 274th target gene is the hsa-miR-6750-3p gene, a congener thereof, a transcript thereof, or a variant or derivative thereof. None of the previously known reports show that change in the expression of the gene or the transcript thereof can serve as a marker for dementia.

The 275th target gene is the hsa-miR-6754-3p gene, a congener thereof, a transcript thereof, or a variant or derivative thereof. None of the previously known reports show that change in the expression of the gene or the transcript thereof can serve as a marker for dementia.

The 276th target gene is the hsa-miR-6759-3p gene, a congener thereof, a transcript thereof, or a variant or derivative thereof. None of the previously known reports show that change in the expression of the gene or the transcript thereof can serve as a marker for dementia.

The 277th target gene is the hsa-miR-6761-3p gene, a congener thereof, a transcript thereof, or a variant or derivative thereof. None of the previously known reports show that change in the expression of the gene or the transcript thereof can serve as a marker for dementia.

The 278th target gene is the hsa-miR-6762-3p gene, a congener thereof, a transcript thereof, or a variant or derivative thereof. None of the previously known reports show that change in the expression of the gene or the transcript thereof can serve as a marker for dementia.

The 279th target gene is the hsa-miR-6769a-3p gene, a congener thereof, a transcript thereof, or a variant or derivative thereof. None of the previously known reports show that change in the expression of the gene or the transcript thereof can serve as a marker for dementia.

The 280th target gene is the hsa-miR-6776-3p gene, a congener thereof, a transcript thereof, or a variant or derivative thereof. None of the previously known reports show that change in the expression of the gene or the transcript thereof can serve as a marker for dementia.

The 281st target gene is the hsa-miR-6778-3p gene, a congener thereof, a transcript thereof, or a variant or derivative thereof. None of the previously known reports show that change in the expression of the gene or the transcript thereof can serve as a marker for dementia.

The 282nd target gene is the hsa-miR-6779-3p gene, a congener thereof, a transcript thereof, or a variant or derivative thereof. None of the previously known reports show that change in the expression of the gene or the transcript thereof can serve as a marker for dementia.

The 283rd target gene is the hsa-miR-6786-3p gene, a congener thereof, a transcript thereof, or a variant or derivative thereof. None of the previously known reports show that change in the expression of the gene or the transcript thereof can serve as a marker for dementia.

The 284th target gene is the hsa-miR-6787-3p gene, a congener thereof, a transcript thereof, or a variant or derivative thereof. None of the previously known reports show that change in the expression of the gene or the transcript thereof can serve as a marker for dementia.

The 285th target gene is the hsa-miR-6792-3p gene, a congener thereof, a transcript thereof, or a variant or derivative thereof. None of the previously known reports show that change in the expression of the gene or the transcript thereof can serve as a marker for dementia.

The 286th target gene is the hsa-miR-6794-3p gene, a congener thereof, a transcript thereof, or a variant or derivative thereof. None of the previously known reports show that change in the expression of the gene or the transcript thereof can serve as a marker for dementia.

The 287th target gene is the hsa-miR-6801-3p gene, a congener thereof, a transcript thereof, or a variant or derivative thereof. None of the previously known reports show that change in the expression of the gene or the transcript thereof can serve as a marker for dementia.

The 288th target gene is the hsa-miR-6802-3p gene, a congener thereof, a transcript thereof, or a variant or derivative thereof. None of the previously known reports show that change in the expression of the gene or the transcript thereof can serve as a marker for dementia.

The 289th target gene is the hsa-miR-6803-3p gene, a congener thereof, a transcript thereof, or a variant or derivative thereof. None of the previously known reports show that change in the expression of the gene or the transcript thereof can serve as a marker for dementia.

The 290th target gene is the hsa-miR-6804-3p gene, a congener thereof, a transcript thereof, or a variant or derivative thereof. None of the previously known reports show that change in the expression of the gene or the transcript thereof can serve as a marker for dementia.

The 291st target gene is the hsa-miR-6810-5p gene, a congener thereof, a transcript thereof, or a variant or derivative thereof. None of the previously known reports show that change in the expression of the gene or the transcript thereof can serve as a marker for dementia.

The 292nd target gene is the hsa-miR-6823-3p gene, a congener thereof, a transcript thereof, or a variant or derivative thereof. None of the previously known reports show that change in the expression of the gene or the transcript thereof can serve as a marker for dementia.

The 293rd target gene is the hsa-miR-6825-3p gene, a congener thereof, a transcript thereof, or a variant or derivative thereof. None of the previously known reports show that change in the expression of the gene or the transcript thereof can serve as a marker for dementia.

The 294th target gene is the hsa-miR-6829-3p gene, a congener thereof, a transcript thereof, or a variant or derivative thereof. None of the previously known reports show that change in the expression of the gene or the transcript thereof can serve as a marker for dementia.

The 295th target gene is the hsa-miR-6833-3p gene, a congener thereof, a transcript thereof, or a variant or derivative thereof. None of the previously known reports show that change in the expression of the gene or the transcript thereof can serve as a marker for dementia.

The 296th target gene is the hsa-miR-6834-3p gene, a congener thereof, a transcript thereof, or a variant or derivative thereof. None of the previously known reports show that change in the expression of the gene or the transcript thereof can serve as a marker for dementia.

The 297th target gene is the hsa-miR-6780b-3p gene, a congener thereof, a transcript thereof, or a variant or derivative thereof. None of the previously known reports show that change in the expression of the gene or the transcript thereof can serve as a marker for dementia.

The 298th target gene is the hsa-miR-6845-3p gene, a congener thereof, a transcript thereof, or a variant or derivative thereof. None of the previously known reports show that change in the expression of the gene or the transcript thereof can serve as a marker for dementia.

The 299th target gene is the hsa-miR-6862-3p gene, a congener thereof, a transcript thereof, or a variant or derivative thereof. None of the previously known reports show that change in the expression of the gene or the transcript thereof can serve as a marker for dementia.

The 300th target gene is the hsa-miR-6865-3p gene, a congener thereof, a transcript thereof, or a variant or derivative thereof. None of the previously known reports show that change in the expression of the gene or the transcript thereof can serve as a marker for dementia.

The 301st target gene is the hsa-miR-6870-3p gene, a congener thereof, a transcript thereof, or a variant or derivative thereof. None of the previously known reports show that change in the expression of the gene or the transcript thereof can serve as a marker for dementia.

The 302nd target gene is the hsa-miR-6875-3p gene, a congener thereof, a transcript thereof, or a variant or derivative thereof. None of the previously known reports show that change in the expression of the gene or the transcript thereof can serve as a marker for dementia.

The 303rd target gene is the hsa-miR-6877-3p gene, a congener thereof, a transcript thereof, or a variant or derivative thereof. None of the previously known reports show that change in the expression of the gene or the transcript thereof can serve as a marker for dementia.

The 304th target gene is the hsa-miR-6879-3p gene, a congener thereof, a transcript thereof, or a variant or derivative thereof. None of the previously known reports show that change in the expression of the gene or the transcript thereof can serve as a marker for dementia.

The 305th target gene is the hsa-miR-6882-3p gene, a congener thereof, a transcript thereof, or a variant or derivative thereof. None of the previously known reports show that change in the expression of the gene or the transcript thereof can serve as a marker for dementia.

The 306th target gene is the hsa-miR-6885-3p gene, a congener thereof, a transcript thereof, or a variant or derivative thereof. None of the previously known reports show that change in the expression of the gene or the transcript thereof can serve as a marker for dementia.

The 307th target gene is the hsa-miR-6886-3p gene, a congener thereof, a transcript thereof, or a variant or derivative thereof. None of the previously known reports show that change in the expression of the gene or the transcript thereof can serve as a marker for dementia.

The 308th target gene is the hsa-miR-6887-3p gene, a congener thereof, a transcript thereof, or a variant or derivative thereof. None of the previously known reports show that change in the expression of the gene or the transcript thereof can serve as a marker for dementia.

The 309th target gene is the hsa-miR-6890-3p gene, a congener thereof, a transcript thereof, or a variant or derivative thereof. None of the previously known reports show that change in the expression of the gene or the transcript thereof can serve as a marker for dementia.

The 310th target gene is the hsa-miR-6893-3p gene, a congener thereof, a transcript thereof, or a variant or derivative thereof. None of the previously known reports show that change in the expression of the gene or the transcript thereof can serve as a marker for dementia.

The 311st target gene is the hsa-miR-6894-3p gene, a congener thereof, a transcript thereof, or a variant or derivative thereof. None of the previously known reports show that change in the expression of the gene or the transcript thereof can serve as a marker for dementia.

The 312nd target gene is the hsa-miR-7106-3p gene, a congener thereof, a transcript thereof, or a variant or derivative thereof. None of the previously known reports show that change in the expression of the gene or the transcript thereof can serve as a marker for dementia.

The 313rd target gene is the hsa-miR-7109-3p gene, a congener thereof, a transcript thereof, or a variant or derivative thereof. None of the previously known reports show that change in the expression of the gene or the transcript thereof can serve as a marker for dementia.

The 314th target gene is the hsa-miR-7114-3p gene, a congener thereof, a transcript thereof, or a variant or derivative thereof. None of the previously known reports show that change in the expression of the gene or the transcript thereof can serve as a marker for dementia.

The 315th target gene is the hsa-miR-7155-5p gene, a congener thereof, a transcript thereof, or a variant or derivative thereof. None of the previously known reports show that change in the expression of the gene or the transcript thereof can serve as a marker for dementia.

The 316th target gene is the hsa-miR-7160-5p gene, a congener thereof, a transcript thereof, or a variant or derivative thereof. None of the previously known reports show that change in the expression of the gene or the transcript thereof can serve as a marker for dementia.

The 317th target gene is the hsa-miR-615-3p gene, a congener thereof, a transcript thereof, or a variant or derivative thereof. None of the previously known reports show that change in the expression of the gene or the transcript thereof can serve as a marker for dementia.

The 318th target gene is the hsa-miR-920 gene, a congener thereof, a transcript thereof, or a variant or derivative thereof. None of the previously known reports show that change in the expression of the gene or the transcript thereof can serve as a marker for dementia.

The 319th target gene is the hsa-miR-1825 gene, a congener thereof, a transcript thereof, or a variant or derivative thereof. None of the previously known reports show that change in the expression of the gene or the transcript thereof can serve as a marker for dementia.

The 320th target gene is the hsa-miR-675-3p gene, a congener thereof, a transcript thereof, or a variant or derivative thereof. None of the previously known reports show that change in the expression of the gene or the transcript thereof can serve as a marker for dementia.

The 321st target gene is the hsa-miR-1910-5p gene, a congener thereof, a transcript thereof, or a variant or derivative thereof. None of the previously known reports show that change in the expression of the gene or the transcript thereof can serve as a marker for dementia.

The 322nd target gene is the hsa-miR-2278 gene, a congener thereof, a transcript thereof, or a variant or derivative thereof. None of the previously known reports show that change in the expression of the gene or the transcript thereof can serve as a marker for dementia.

The 323rd target gene is the hsa-miR-2682-3p gene, a congener thereof, a transcript thereof, or a variant or derivative thereof. None of the previously known reports show that change in the expression of the gene or the transcript thereof can serve as a marker for dementia.

The 324th target gene is the hsa-miR-3122 gene, a congener thereof, a transcript thereof, or a variant or derivative thereof. None of the previously known reports show that change in the expression of the gene or the transcript thereof can serve as a marker for dementia.

The 325th target gene is the hsa-miR-3151-5p gene, a congener thereof, a transcript thereof, or a variant or derivative thereof. None of the previously known reports show that change in the expression of the gene or the transcript thereof can serve as a marker for dementia.

The 326th target gene is the hsa-miR-3175 gene, a congener thereof, a transcript thereof, or a variant or derivative thereof. None of the previously known reports show that change in the expression of the gene or the transcript thereof can serve as a marker for dementia.

The 327th target gene is the hsa-miR-4323 gene, a congener thereof, a transcript thereof, or a variant or derivative thereof. None of the previously known reports show that change in the expression of the gene or the transcript thereof can serve as a marker for dementia.

The 328th target gene is the hsa-miR-4326 gene, a congener thereof, a transcript thereof, or a variant or derivative thereof. None of the previously known reports show that change in the expression of the gene or the transcript thereof can serve as a marker for dementia.

The 329th target gene is the hsa-miR-4284 gene, a congener thereof, a transcript thereof, or a variant or derivative thereof. None of the previously known reports show that change in the expression of the gene or the transcript thereof can serve as a marker for dementia.

The 330th target gene is the hsa-miR-3605-3p gene, a congener thereof, a transcript thereof, or a variant or derivative thereof. None of the previously known reports show that change in the expression of the gene or the transcript thereof can serve as a marker for dementia.

The 331st target gene is the hsa-miR-3622b-5p gene, a congener thereof, a transcript thereof, or a variant or derivative thereof. None of the previously known reports show that change in the expression of the gene or the transcript thereof can serve as a marker for dementia.

The 332nd target gene is the hsa-miR-3646 gene, a congener thereof, a transcript thereof, or a variant or derivative thereof. None of the previously known reports show that change in the expression of the gene or the transcript thereof can serve as a marker for dementia.

The 333rd target gene is the hsa-miR-3158-5p gene, a congener thereof, a transcript thereof, or a variant or derivative thereof. None of the previously known reports show that change in the expression of the gene or the transcript thereof can serve as a marker for dementia.

The 334th target gene is the hsa-miR-4722-3p gene, a congener thereof, a transcript thereof, or a variant or derivative thereof. None of the previously known reports show that change in the expression of the gene or the transcript thereof can serve as a marker for dementia.

The 335th target gene is the hsa-miR-4728-3p gene, a congener thereof, a transcript thereof, or a variant or derivative thereof. None of the previously known reports show that change in the expression of the gene or the transcript thereof can serve as a marker for dementia.

The 336th target gene is the hsa-miR-4747-3p gene, a congener thereof, a transcript thereof, or a variant or derivative thereof. None of the previously known reports show that change in the expression of the gene or the transcript thereof can serve as a marker for dementia.

The 337th target gene is the hsa-miR-4436b-5p gene, a congener thereof, a transcript thereof, or a variant or derivative thereof. None of the previously known reports show that change in the expression of the gene or the transcript thereof can serve as a marker for dementia.

The 338th target gene is the hsa-miR-5196-3p gene, a congener thereof, a transcript thereof, or a variant or derivative thereof. None of the previously known reports show that change in the expression of the gene or the transcript thereof can serve as a marker for dementia.

The 339th target gene is the hsa-miR-5589-5p gene, a congener thereof, a transcript thereof, or a variant or derivative thereof. None of the previously known reports show that change in the expression of the gene or the transcript thereof can serve as a marker for dementia.

The 340th target gene is the hsa-miR-345-3p gene, a congener thereof, a transcript thereof, or a variant or derivative thereof. None of the previously known reports show that change in the expression of the gene or the transcript thereof can serve as a marker for dementia.

The 341st target gene is the hsa-miR-642b-5p gene, a congener thereof, a transcript thereof, or a variant or derivative thereof. None of the previously known reports show that change in the expression of the gene or the transcript thereof can serve as a marker for dementia.

The 342nd target gene is the hsa-miR-6716-3p gene, a congener thereof, a transcript thereof, or a variant or derivative thereof. None of the previously known reports show that change in the expression of the gene or the transcript thereof can serve as a marker for dementia.

The 343rd target gene is the hsa-miR-6511b-3p gene, a congener thereof, a transcript thereof, or a variant or derivative thereof. None of the previously known reports show that change in the expression of the gene or the transcript thereof can serve as a marker for dementia.

The 344th target gene is the hsa-miR-208a-5p gene, a congener thereof, a transcript thereof, or a variant or derivative thereof. None of the previously known reports show that change in the expression of the gene or the transcript thereof can serve as a marker for dementia.

The 345th target gene is the hsa-miR-6726-3p gene, a congener thereof, a transcript thereof, or a variant or derivative thereof. None of the previously known reports show that change in the expression of the gene or the transcript thereof can serve as a marker for dementia.

The 346th target gene is the hsa-miR-6744-5p gene, a congener thereof, a transcript thereof, or a variant or derivative thereof. None of the previously known reports show that change in the expression of the gene or the transcript thereof can serve as a marker for dementia.

The 347th target gene is the hsa-miR-6782-3p gene, a congener thereof, a transcript thereof, or a variant or derivative thereof. None of the previously known reports show that change in the expression of the gene or the transcript thereof can serve as a marker for dementia.

The 348th target gene is the hsa-miR-6789-3p gene, a congener thereof, a transcript thereof, or a variant or derivative thereof. None of the previously known reports show that change in the expression of the gene or the transcript thereof can serve as a marker for dementia.

The 349th target gene is the hsa-miR-6797-3p gene, a congener thereof, a transcript thereof, or a variant or derivative thereof. None of the previously known reports show that change in the expression of the gene or the transcript thereof can serve as a marker for dementia.

The 350th target gene is the hsa-miR-6800-3p gene, a congener thereof, a transcript thereof, or a variant or derivative thereof. None of the previously known reports show that change in the expression of the gene or the transcript thereof can serve as a marker for dementia.

The 351st target gene is the hsa-miR-6806-5p gene, a congener thereof, a transcript thereof, or a variant or derivative thereof. None of the previously known reports show that change in the expression of the gene or the transcript thereof can serve as a marker for dementia.

The 352nd target gene is the hsa-miR-6824-3p gene, a congener thereof, a transcript thereof, or a variant or derivative thereof. None of the previously known reports show that change in the expression of the gene or the transcript thereof can serve as a marker for dementia.

The 353rd target gene is the hsa-miR-6837-5p gene, a congener thereof, a transcript thereof, or a variant or derivative thereof. None of the previously known reports show that change in the expression of the gene or the transcript thereof can serve as a marker for dementia.

The 354th target gene is the hsa-miR-6846-3p gene, a congener thereof, a transcript thereof, or a variant or derivative thereof. None of the previously known reports show that change in the expression of the gene or the transcript thereof can serve as a marker for dementia.

The 355th target gene is the hsa-miR-6858-3p gene, a congener thereof, a transcript thereof, or a variant or derivative thereof. None of the previously known reports show that change in the expression of the gene or the transcript thereof can serve as a marker for dementia.

The 356th target gene is the hsa-miR-6859-3p gene, a congener thereof, a transcript thereof, or a variant or derivative thereof. None of the previously known reports show that change in the expression of the gene or the transcript thereof can serve as a marker for dementia.

The 357th target gene is the hsa-miR-6861-3p gene, a congener thereof, a transcript thereof, or a variant or derivative thereof. None of the previously known reports show that change in the expression of the gene or the transcript thereof can serve as a marker for dementia.

The 358th target gene is the hsa-miR-6880-3p gene, a congener thereof, a transcript thereof, or a variant or derivative thereof. None of the previously known reports show that change in the expression of the gene or the transcript thereof can serve as a marker for dementia.

The 359th target gene is the hsa-miR-7111-3p gene, a congener thereof, a transcript thereof, or a variant or derivative thereof. None of the previously known reports show that change in the expression of the gene or the transcript thereof can serve as a marker for dementia.

The 360th target gene is the hsa-miR-7152-5p gene, a congener thereof, a transcript thereof, or a variant or derivative thereof. None of the previously known reports show that change in the expression of the gene or the transcript thereof can serve as a marker for dementia.

The 361st target gene is the hsa-miR-642a-5p gene, a congener thereof, a transcript thereof, or a variant or derivative thereof. None of the previously known reports show that change in the expression of the gene or the transcript thereof can serve as a marker for dementia.

The 362nd target gene is the hsa-miR-657 gene, a congener thereof, a transcript thereof, or a variant or derivative thereof. None of the previously known reports show that change in the expression of the gene or the transcript thereof can serve as a marker for dementia.

The 363rd target gene is the hsa-miR-1236-3p gene, a congener thereof, a transcript thereof, or a variant or derivative thereof. None of the previously known reports show that change in the expression of the gene or the transcript thereof can serve as a marker for dementia.

The 364th target gene is the hsa-miR-764 gene, a congener thereof, a transcript thereof, or a variant or derivative thereof. None of the previously known reports show that change in the expression of the gene or the transcript thereof can serve as a marker for dementia.

The 365th target gene is the hsa-miR-4314 gene, a congener thereof, a transcript thereof, or a variant or derivative thereof. None of the previously known reports show that change in the expression of the gene or the transcript thereof can serve as a marker for dementia.

The 366th target gene is the hsa-miR-3675-3p gene, a congener thereof, a transcript thereof, or a variant or derivative thereof. None of the previously known reports show that change in the expression of the gene or the transcript thereof can serve as a marker for dementia.

The 367th target gene is the hsa-miR-5703 gene, a congener thereof, a transcript thereof, or a variant or derivative thereof. None of the previously known reports show that change in the expression of the gene or the transcript thereof can serve as a marker for dementia.

The 368th target gene is the hsa-miR-3191-5p gene, a congener thereof, a transcript thereof, or a variant or derivative thereof. None of the previously known reports show that change in the expression of the gene or the transcript thereof can serve as a marker for dementia.

The 369th target gene is the hsa-miR-6511a-3p gene, a congener thereof, a transcript thereof, or a variant or derivative thereof. None of the previously known reports show that change in the expression of the gene or the transcript thereof can serve as a marker for dementia.

The 370th target gene is the hsa-miR-6809-3p gene, a congener thereof, a transcript thereof, or a variant or derivative thereof. None of the previously known reports show that change in the expression of the gene or the transcript thereof can serve as a marker for dementia.

The 371st target gene is the hsa-miR-6815-5p gene, a congener thereof, a transcript thereof, or a variant or derivative thereof. None of the previously known reports show that change in the expression of the gene or the transcript thereof can serve as a marker for dementia.

The 372nd target gene is the hsa-miR-6857-3p gene, a congener thereof, a transcript thereof, or a variant or derivative thereof. None of the previously known reports show that change in the expression of the gene or the transcript thereof can serve as a marker for dementia.

The 373rd target gene is the hsa-miR-6878-3p gene, a congener thereof, a transcript thereof, or a variant or derivative thereof. None of the previously known reports show that change in the expression of the gene or the transcript thereof can serve as a marker for dementia.

The 374th target gene is the hsa-miR-371a-5p gene, a congener thereof, a transcript thereof, or a variant or derivative thereof. None of the previously known reports show that change in the expression of the gene or the transcript thereof can serve as a marker for dementia.

The 375th target gene is the hsa-miR-766-3p gene, a congener thereof, a transcript thereof, or a variant or derivative thereof. The previously known report shows that change in the expression of the gene or the transcript thereof can serve as a marker for dementia (Patent Literature 5).

The 376th target gene is the hsa-miR-1229-3p gene, a congener thereof, a transcript thereof, or a variant or derivative thereof. The previously known report shows that change in the expression of the gene or the transcript thereof can serve as a marker for dementia (Patent Literature 7).

The 377th target gene is the hsa-miR-1306-5p gene, a congener thereof, a transcript thereof, or a variant or derivative thereof. The previously known report shows that change in the expression of the gene or the transcript thereof can serve as a marker for dementia (Patent Literature 6).

The 378th target gene is the hsa-miR-210-5p gene, a congener thereof, a transcript thereof, or a variant or derivative thereof. The previously known report shows that change in the expression of the gene or the transcript thereof can serve as a marker for dementia (Patent Literature 5).

The 379th target gene is the hsa-miR-198 gene, a congener thereof, a transcript thereof, or a variant or derivative thereof. The previously known report shows that change in the expression of the gene or the transcript thereof can serve as a marker for dementia (Patent Literature 7).

The 380th target gene is the hsa-miR-485-3p gene, a congener thereof, a transcript thereof, or a variant or derivative thereof. The previously known report shows that change in the expression of the gene or the transcript thereof can serve as a marker for dementia (Patent Literature 7).

The 381st target gene is the hsa-miR-668-3p gene, a congener thereof, a transcript thereof, or a variant or derivative thereof. The previously known report shows that change in the expression of the gene or the transcript thereof can serve as a marker for dementia (Patent Literature 4).

The 382nd target gene is the hsa-miR-532-3p gene, a congener thereof, a transcript thereof, or a variant or derivative thereof. The previously known report shows that change in the expression of the gene or the transcript thereof can serve as a marker for dementia (Patent Literature 5).

The 383rd target gene is the hsa-miR-877-3p gene, a congener thereof, a transcript thereof, or a variant or derivative thereof. The previously known report shows that change in the expression of the gene or the transcript thereof can serve as a marker for dementia (Patent Literature 5).

The 384th target gene is the hsa-miR-1238-3p gene, a congener thereof, a transcript thereof, or a variant or derivative thereof. The previously known report shows that change in the expression of the gene or the transcript thereof can serve as a marker for dementia (Patent Literature 2).

The 385th target gene is the hsa-miR-3130-5p gene, a congener thereof, a transcript thereof, or a variant or derivative thereof. The previously known report shows that change in the expression of the gene or the transcript thereof can serve as a marker for dementia (Patent Literature 5).

The 386th target gene is the hsa-miR-4298 gene, a congener thereof, a transcript thereof, or a variant or derivative thereof. The previously known report shows that change in the expression of the gene or the transcript thereof can serve as a marker for dementia (Patent Literature 5).

The 387th target gene is the hsa-miR-4290 gene, a congener thereof, a transcript thereof, or a variant or derivative thereof. The previously known report shows that change in the expression of the gene or the transcript thereof can serve as a marker for dementia (Patent Literature 5).

The 388th target gene is the hsa-miR-3943 gene, a congener thereof, a transcript thereof, or a variant or derivative thereof. The previously known report shows that change in the expression of the gene or the transcript thereof can serve as a marker for dementia (Patent Literature 5).

The 389th target gene is the hsa-miR-346 gene, a congener thereof, a transcript thereof, or a variant or derivative thereof. The previously known report shows that change in the expression of the gene or the transcript thereof can serve as a marker for dementia (Patent Literature 3).

The 390th target gene is the hsa-miR-767-3p gene, a congener thereof, a transcript thereof, or a variant or derivative thereof. The previously known report shows that change in the expression of the gene or the transcript thereof can serve as a marker for dementia (Patent Literature 5).

The 391st target gene is the hsa-miR-6765-3p gene, a congener thereof, a transcript thereof, or a variant or derivative thereof. None of the previously known reports show that change in the expression of the gene or the transcript thereof can serve as a marker for dementia.

The 392nd target gene is the hsa-miR-6784-5p gene, a congener thereof, a transcript thereof, or a variant or derivative thereof. None of the previously known reports show that change in the expression of the gene or the transcript thereof can serve as a marker for dementia.

The 393rd target gene is the hsa-miR-6778-5p gene, a congener thereof, a transcript thereof, or a variant or derivative thereof. None of the previously known reports show that change in the expression of the gene or the transcript thereof can serve as a marker for dementia.

The 394th target gene is the hsa-miR-6875-5p gene, a congener thereof, a transcript thereof, or a variant or derivative thereof. None of the previously known reports show that change in the expression of the gene or the transcript thereof can serve as a marker for dementia.

The 395th target gene is the hsa-miR-4534 gene, a congener thereof, a transcript thereof, or a variant or derivative thereof. None of the previously known reports show that change in the expression of the gene or the transcript thereof can serve as a marker for dementia.

The 396th target gene is the hsa-miR-4721 gene, a congener thereof, a transcript thereof, or a variant or derivative thereof. None of the previously known reports show that change in the expression of the gene or the transcript thereof can serve as a marker for dementia.

The 397th target gene is the hsa-miR-6756-5p gene, a congener thereof, a transcript thereof, or a variant or derivative thereof. None of the previously known reports show that change in the expression of the gene or the transcript thereof can serve as a marker for dementia.

The 398th target gene is the hsa-miR-615-5p gene, a congener thereof, a transcript thereof, or a variant or derivative thereof. None of the previously known reports show that change in the expression of the gene or the transcript thereof can serve as a marker for dementia.

The 399th target gene is the hsa-miR-6727-5p gene, a congener thereof, a transcript thereof, or a variant or derivative thereof. None of the previously known reports show that change in the expression of the gene or the transcript thereof can serve as a marker for dementia.

The 400th target gene is the hsa-miR-6887-5p gene, a congener thereof, a transcript thereof, or a variant or derivative thereof. None of the previously known reports show that change in the expression of the gene or the transcript thereof can serve as a marker for dementia.

The 401st target gene is the hsa-miR-8063 gene, a congener thereof, a transcript thereof, or a variant or derivative thereof. None of the previously known reports show that change in the expression of the gene or the transcript thereof can serve as a marker for dementia.

The 402nd target gene is the hsa-miR-6880-5p gene, a congener thereof, a transcript thereof, or a variant or derivative thereof. None of the previously known reports show that change in the expression of the gene or the transcript thereof can serve as a marker for dementia.

The 403rd target gene is the hsa-miR-6805-3p gene, a congener thereof, a transcript thereof, or a variant or derivative thereof. None of the previously known reports show that change in the expression of the gene or the transcript thereof can serve as a marker for dementia.

The 404th target gene is the hsa-miR-4726-5p gene, a congener thereof, a transcript thereof, or a variant or derivative thereof. None of the previously known reports show that change in the expression of the gene or the transcript thereof can serve as a marker for dementia.

The 405th target gene is the hsa-miR-4710 gene, a congener thereof, a transcript thereof, or a variant or derivative thereof. None of the previously known reports show that change in the expression of the gene or the transcript thereof can serve as a marker for dementia.

The 406th target gene is the hsa-miR-7111-5p gene, a congener thereof, a transcript thereof, or a variant or derivative thereof. None of the previously known reports show that change in the expression of the gene or the transcript thereof can serve as a marker for dementia.

The 407th target gene is the hsa-miR-3619-3p gene, a congener thereof, a transcript thereof, or a variant or derivative thereof. None of the previously known reports show that change in the expression of the gene or the transcript thereof can serve as a marker for dementia.

The 408th target gene is the hsa-miR-6795-5p gene, a congener thereof, a transcript thereof, or a variant or derivative thereof. None of the previously known reports show that change in the expression of the gene or the transcript thereof can serve as a marker for dementia.

The 409th target gene is the hsa-miR-1254 gene, a congener thereof, a transcript thereof, or a variant or derivative thereof. None of the previously known reports show that change in the expression of the gene or the transcript thereof can serve as a marker for dementia.

The 410th target gene is the hsa-miR-1233-5p gene, a congener thereof, a transcript thereof, or a variant or derivative thereof. None of the previously known reports show that change in the expression of the gene or the transcript thereof can serve as a marker for dementia.

The 411st target gene is the hsa-miR-6836-3p gene, a congener thereof, a transcript thereof, or a variant or derivative thereof. None of the previously known reports show that change in the expression of the gene or the transcript thereof can serve as a marker for dementia.

The 412nd target gene is the hsa-miR-6769a-5p gene, a congener thereof, a transcript thereof, or a variant or derivative thereof. None of the previously known reports show that change in the expression of the gene or the transcript thereof can serve as a marker for dementia.

The 413rd target gene is the hsa-miR-4532 gene, a congener thereof, a transcript thereof, or a variant or derivative thereof. None of the previously known reports show that change in the expression of the gene or the transcript thereof can serve as a marker for dementia.

The 414th target gene is the hsa-miR-365a-5p gene, a congener thereof, a transcript thereof, or a variant or derivative thereof. None of the previously known reports show that change in the expression of the gene or the transcript thereof can serve as a marker for dementia.

The 415th target gene is the hsa-miR-1231 gene, a congener thereof, a transcript thereof, or a variant or derivative thereof. None of the previously known reports show that change in the expression of the gene or the transcript thereof can serve as a marker for dementia.

The 416th target gene is the hsa-miR-1228-5p gene, a congener thereof, a transcript thereof, or a variant or derivative thereof. None of the previously known reports show that change in the expression of the gene or the transcript thereof can serve as a marker for dementia.

The 417th target gene is the hsa-miR-4430 gene, a congener thereof, a transcript thereof, or a variant or derivative thereof. None of the previously known reports show that change in the expression of the gene or the transcript thereof can serve as a marker for dementia.

The 418th target gene is the hsa-miR-296-3p gene, a congener thereof, a transcript thereof, or a variant or derivative thereof. None of the previously known reports show that change in the expression of the gene or the transcript thereof can serve as a marker for dementia.

The 419th target gene is the hsa-miR-1237-5p gene, a congener thereof, a transcript thereof, or a variant or derivative thereof. None of the previously known reports show that change in the expression of the gene or the transcript thereof can serve as a marker for dementia.

The 420th target gene is the hsa-miR-4466 gene, a congener thereof, a transcript thereof, or a variant or derivative thereof. None of the previously known reports show that change in the expression of the gene or the transcript thereof can serve as a marker for dementia.

The 421st target gene is the hsa-miR-6789-5p gene, a congener thereof, a transcript thereof, or a variant or derivative thereof. None of the previously known reports show that change in the expression of the gene or the transcript thereof can serve as a marker for dementia.

The 422nd target gene is the hsa-miR-4632-5p gene, a congener thereof, a transcript thereof, or a variant or derivative thereof. None of the previously known reports show that change in the expression of the gene or the transcript thereof can serve as a marker for dementia.

The 423rd target gene is the hsa-miR-4745-5p gene, a congener thereof, a transcript thereof, or a variant or derivative thereof. None of the previously known reports show that change in the expression of the gene or the transcript thereof can serve as a marker for dementia.

The 424th target gene is the hsa-miR-4665-5p gene, a congener thereof, a transcript thereof, or a variant or derivative thereof. None of the previously known reports show that change in the expression of the gene or the transcript thereof can serve as a marker for dementia.

The 425th target gene is the hsa-miR-6807-5p gene, a congener thereof, a transcript thereof, or a variant or derivative thereof. None of the previously known reports show that change in the expression of the gene or the transcript thereof can serve as a marker for dementia.

The 426th target gene is the hsa-miR-7114-5p gene, a congener thereof, a transcript thereof, or a variant or derivative thereof. None of the previously known reports show that change in the expression of the gene or the transcript thereof can serve as a marker for dementia.

The 427th target gene is the hsa-miR-150-3p gene, a congener thereof, a transcript thereof, or a variant or derivative thereof. The previously known report shows that change in the expression of the gene or the transcript thereof can serve as a marker for dementia (Patent Literature 1).

The 428th target gene is the hsa-miR-423-5p gene, a congener thereof, a transcript thereof, or a variant or derivative thereof. The previously known report shows that change in the expression of the gene or the transcript thereof can serve as a marker for dementia (Patent Literature 1).

The 429th target gene is the hsa-miR-575 gene, a congener thereof, a transcript thereof, or a variant or derivative thereof. The previously known report shows that change in the expression of the gene or the transcript thereof can serve as a marker for dementia (Patent Literature 2).

The 430th target gene is the hsa-miR-671-5p gene, a congener thereof, a transcript thereof, or a variant or derivative thereof. The previously known report shows that change in the expression of the gene or the transcript thereof can serve as a marker for dementia (Patent Literature 3).

The 431st target gene is the hsa-miR-939-5p gene, a congener thereof, a transcript thereof, or a variant or derivative thereof. The previously known report shows that change in the expression of the gene or the transcript thereof can serve as a marker for dementia (Patent Literature 4).

The 432nd target gene is the hsa-miR-3665 gene, a congener thereof, a transcript thereof, or a variant or derivative thereof. The previously known report shows that change in the expression of the gene or the transcript thereof can serve as a marker for dementia (Patent Literature 5).

The 433rd target gene is the hsa-miR-516a-5p gene, a congener thereof, a transcript thereof, or a variant or derivative thereof. None of the previously known reports show that change in the expression of the gene or the transcript thereof can serve as a marker for dementia.

The 434th target gene is the hsa-miR-769-3p gene, a congener thereof, a transcript thereof, or a variant or derivative thereof. None of the previously known reports show that change in the expression of the gene or the transcript thereof can serve as a marker for dementia.

The 435th target gene is the hsa-miR-3692-5p gene, a congener thereof, a transcript thereof, or a variant or derivative thereof. None of the previously known reports show that change in the expression of the gene or the transcript thereof can serve as a marker for dementia.

The 436th target gene is the hsa-miR-3945 gene, a congener thereof, a transcript thereof, or a variant or derivative thereof. None of the previously known reports show that change in the expression of the gene or the transcript thereof can serve as a marker for dementia.

The 437th target gene is the hsa-miR-4433a-3p gene, a congener thereof, a transcript thereof, or a variant or derivative thereof. None of the previously known reports show that change in the expression of the gene or the transcript thereof can serve as a marker for dementia.

The 438th target gene is the hsa-miR-4485-3p gene, a congener thereof, a transcript thereof, or a variant or derivative thereof. None of the previously known reports show that change in the expression of the gene or the transcript thereof can serve as a marker for dementia.

The 439th target gene is the hsa-miR-6831-5p gene, a congener thereof, a transcript thereof, or a variant or derivative thereof. None of the previously known reports show that change in the expression of the gene or the transcript thereof can serve as a marker for dementia.

The 440th target gene is the hsa-miR-519c-5p gene, a congener thereof, a transcript thereof, or a variant or derivative thereof. None of the previously known reports show that change in the expression of the gene or the transcript thereof can serve as a marker for dementia.

The 441st target gene is the hsa-miR-551b-5p gene, a congener thereof, a transcript thereof, or a variant or derivative thereof. None of the previously known reports show that change in the expression of the gene or the transcript thereof can serve as a marker for dementia.

The 442nd target gene is the hsa-miR-1343-3p gene, a congener thereof, a transcript thereof, or a variant or derivative thereof. None of the previously known reports show that change in the expression of the gene or the transcript thereof can serve as a marker for dementia.

The 443rd target gene is the hsa-miR-4286 gene, a congener thereof, a transcript thereof, or a variant or derivative thereof. None of the previously known reports show that change in the expression of the gene or the transcript thereof can serve as a marker for dementia.

The 444th target gene is the hsa-miR-4634 gene, a congener thereof, a transcript thereof, or a variant or derivative thereof. None of the previously known reports show that change in the expression of the gene or the transcript thereof can serve as a marker for dementia.

The 445th target gene is the hsa-miR-4733-3p gene, a congener thereof, a transcript thereof, or a variant or derivative thereof. None of the previously known reports show that change in the expression of the gene or the transcript thereof can serve as a marker for dementia.

The 446th target gene is the hsa-miR-6086 gene, a congener thereof, a transcript thereof, or a variant or derivative thereof. None of the previously known reports show that change in the expression of the gene or the transcript thereof can serve as a marker for dementia.

The 447th target gene is the hsa-miR-30d-5p gene, a congener thereof, a transcript thereof, or a variant or derivative thereof. The previously known report shows that change in the expression of the gene or the transcript thereof can serve as a marker for dementia (U.S. Patent Application Publication No. 2010/279292).

The 448th target gene is the hsa-miR-30b-3p gene, a congener thereof, a transcript thereof, or a variant or derivative thereof. The previously known report shows that change in the expression of the gene or the transcript thereof can serve as a marker for dementia (U.S. Patent Application Publication No. 2010/279292).

The 449th target gene is the hsa-miR-92a-3p gene, a congener thereof, a transcript thereof, or a variant or derivative thereof. The previously known report shows that change in the expression of the gene or the transcript thereof can serve as a marker for dementia (Patent Literature 1).

The 450th target gene is the hsa-miR-371b-5p gene, a congener thereof, a transcript thereof, or a variant or derivative thereof. The previously known report shows that change in the expression of the gene or the transcript thereof can serve as a marker for dementia (Schipper HM et al., Gene Regulation and Systems Biology, 2007, vol. 1, pp. 263-74).

The 451 st target gene is the hsa-miR-486-5p gene, a congener thereof, a transcript thereof, or a variant or derivative thereof. The previously known report shows that change in the expression of the gene or the transcript thereof can serve as a marker for dementia (Patent Literature 1).

In one embodiment, the present invention relates to a marker(s) for detecting dementia or diagnosing dementia, which comprises at least one selected from the above target nucleic acid(s).

In one embodiment, the present invention relates to use of at least one selected from the above target nucleic acid(s) for detecting dementia or diagnosing dementia.

In one embodiment, the present invention relates to a marker(s) for predicting dementia, which comprises at least one target nucleic acid selected from the group of the above (iii).

In one embodiment, the present invention relates to use of at least one target nucleic acid which is selected from the group of the above (iii), for predicting dementia.


2. Nucleic acid Probe(s) or Primer(s) for Detection of Dementia

A nucleic acid probe(s) or primer(s) capable of being used for detection of dementia or diagnosis of dementia according to the present invention enables qualitative and/or quantitative measurement of the presence, expression level(s), or abundance of target nucleic acid(s) for dementia: human-derived miR-4274, miR-4272, miR-4728-5p, miR-4443, miR-4506, miR-6773-5p, miR-4662a-5p, miR-3184-3p, miR-4281, miR-320d, miR-6729-3p, miR-5192, miR-6853-5p, miR-1234-3p, miR-1233-3p, miR-4539, miR-3914, miR-4738-5p, miR-548au-3p, miR-1539, miR-4720-3p, miR-365b-5p, miR-4486, miR-1227-5p, miR-4667-5p, miR-6088, miR-6820-5p, miR-4505, miR-548q, miR-4658, miR-450a-5p, miR-1260b, miR-3677-5p, miR-6777-3p, miR-6826-3p, miR-6832-3p, miR-4725-3p, miR-7161-3p, miR-2277-5p, miR-7110-3p, miR-4312, miR-4461, miR-6766-5p, miR-1266-3p, miR-6729-5p, miR-526b-3p, miR-519e-5p, miR-512-5p, miR-5088-5p, miR-1909-3p, miR-6511a-5p, miR-4734, miR-936, miR-1249-3p, miR-6777-5p, miR-4487, miR-3155a, miR-563, miR-4741, miR-6788-5p, miR-4433b-5p, miR-323a-5p, miR-6811-5p, miR-6721-5p, miR-5004-5p, miR-6509-3p, miR-648, miR-3917, miR-6087, miR-1470, miR-586, miR-3150a-5p, miR-105-3p, miR-7973, miR-1914-5p, miR-4749-3p, miR-15b-5p, miR-1289, miR-4433a-5p, miR-3666, miR-3186-3p, miR-4725-5p, miR-4488, miR-4474-3p, miR-6731-3p, miR-4640-3p, miR-202-5p, miR-6816-5p, miR-638, miR-6821-5p, miR-1247-3p, miR-6765-5p, miR-6800-5p, miR-3928-3p, miR-3940-5p, miR-3960, miR-6775-5p, miR-3178, miR-1202, miR-6790-5p, miR-4731-3p, miR-2681-3p, miR-6758-5p, miR-8072, miR-518d-3p, miR-3606-3p, miR-4800-5p, miR-1292-3p, miR-6784-3p, miR-4450, miR-6132, miR-4716-5p, miR-6860, miR-1268b, miR-378d, miR-4701-5p, miR-4329, miR-185-3p, miR-552-3p, miR-1273g-5p, miR-6769b-3p, miR-520a-3p, miR-4524b-5p, miR-4291, miR-6734-3p, miR-143-5p, miR-939-3p, miR-6889-3p, miR-6842-3p, miR-4511, miR-4318, miR-4653-5p, miR-6867-3p, miR-133b, miR-3196, miR-193b-3p, miR-3162-3p, miR-6819-3p, miR-1908-3p, miR-6786-5p, miR-3648, miR-4513, miR-3652, miR-4640-5p, miR-6871-5p, miR-7845-5p, miR-3138, miR-6884-5p, miR-4653-3p, miR-636, miR-4652-3p, miR-6823-5p, miR-4502, miR-7113-5p, miR-8087, miR-7154-3p, miR-5189-5p, miR-1253, miR-518c-5p, miR-7151-5p, miR-3614-3p, miR-4727-5p, miR-3682-5p, miR-5090, miR-337-3p, miR-488-5p, miR-100-5p, miR-4520-3p, miR-373-3p, miR-6499-5p, miR-3909, miR-32-5p, miR-302a-3p, miR-4686, miR-4659a-3p, miR-4287, miR-1301-5p, miR-593-3p, miR-517a-3p, miR-517b-3p, miR-142-3p, miR-1185-2-3p, miR-602, miR-527, miR-518a-5p, miR-4682, miR-28-5p, miR-4252, miR-452-5p, miR-525-5p, miR-3622a-3p, miR-6813-3p, miR-4769-3p, miR-5698, miR-1915-3p, miR-1343-5p, miR-6861-5p, miR-6781-5p, miR-4508, miR-6743-5p, miR-6726-5p, miR-4525, miR-4651, miR-6813-5p, miR-5787, miR-1290, miR-6075, miR-4758-5p, miR-4690-5p, miR-762, miR-371a-5p, miR-6765-3p, miR-6784-5p, miR-6778-5p, miR-6875-5p, miR-4534, miR-4721, miR-6756-5p, miR-615-5p, miR-6727-5p, miR-6887-5p, miR-8063, miR-6880-5p, miR-6805-3p, miR-4726-5p, miR-4710, miR-7111-5p, miR-3619-3p, miR-6795-5p, miR-1254, miR-1233-5p, miR-6836-3p, miR-6769a-5p, miR-4532, miR-365a-5p, miR-1231, miR-1228-5p, miR-4430, miR-296-3p, miR-1237-5p, miR-4466, miR-6789-5p, miR-4632-5p, miR-4745-5p, miR-4665-5p, miR-6807-5p and miR-7114-5p, miR-516a-5p, miR-769-3p, miR-3692-5p, miR-3945, miR-4433a-3p, miR-4485-3p, miR-6831-5p, miR-519c-5p, miR-551b-5p, miR-1343-3p, miR-4286, miR-4634, miR-4733-3p, miR-6086, miR-1225-3p, miR-3184-5p, miR-665, miR-211-5p, miR-1247-5p, miR-3656, miR-149-5p, miR-744-5p, miR-345-5p, miR-150-5p, miR-191-3p, miR-651-5p, miR-34a-5p, miR-409-5p, miR-369-5p, miR-1915-5p, miR-204-5p, miR-137, miR-382-5p, miR-517-5p, miR-532-5p, miR-22-5p, miR-1237-3p, miR-1224-3p, miR-625-3p, miR-328-3p, miR-122-5p, miR-202-3p, miR-4781-5p, miR-718, miR-342-3p, miR-26b-3p, miR-140-3p, miR-200a-3p, miR-378a-3p, miR-484, miR-296-5p, miR-205-5p, miR-431-5p, miR-150-3p, miR-423-5p, miR-575, miR-671-5p, miR-939-5p and miR-3665, miR-30d-5p, miR-30b-3p, miR-92a-3p, miR-371b-5p, miR-486-5p, miR-1471, miR-1538, miR-449b-3p, miR-1976, miR-4268, miR-4279, miR-3620-3p, miR-3944-3p, miR-3156-3p, miR-3187-5p, miR-4685-3p, miR-4695-3p, miR-4697-3p, miR-4713-5p, miR-4723-3p, miR-371b-3p, miR-3151-3p, miR-3192-3p, miR-6728-3p, miR-6736-3p, miR-6740-3p, miR-6741-3p, miR-6743-3p, miR-6747-3p, miR-6750-3p, miR-6754-3p, miR-6759-3p, miR-6761-3p, miR-6762-3p, miR-6769a-3p, miR-6776-3p, miR-6778-3p, miR-6779-3p, miR-6786-3p, miR-6787-3p, miR-6792-3p, miR-6794-3p, miR-6801-3p, miR-6802-3p, miR-6803-3p, miR-6804-3p, miR-6810-5p, miR-6823-3p, miR-6825-3p, miR-6829-3p, miR-6833-3p, miR-6834-3p, miR-6780b-3p, miR-6845-3p, miR-6862-3p, miR-6865-3p, miR-6870-3p, miR-6875-3p, miR-6877-3p, miR-6879-3p, miR-6882-3p, miR-6885-3p, miR-6886-3p, miR-6887-3p, miR-6890-3p, miR-6893-3p, miR-6894-3p, miR-7106-3p, miR-7109-3p, miR-7114-3p, miR-7155-5p, miR-7160-5p, miR-615-3p, miR-920, miR-1825, miR-675-3p, miR-1910-5p, miR-2278, miR-2682-3p, miR-3122, miR-3151-5p, miR-3175, miR-4323, miR-4326, miR-4284, miR-3605-3p, miR-3622b-5p, miR-3646, miR-3158-5p, miR-4722-3p, miR-4728-3p, miR-4747-3p, miR-4436b-5p, miR-5196-3p, miR-5589-5p, miR-345-3p, miR-642b-5p, miR-6716-3p, miR-6511b-3p, miR-208a-5p, miR-6726-3p, miR-6744-5p, miR-6782-3p, miR-6789-3p, miR-6797-3p, miR-6800-3p, miR-6806-5p, miR-6824-3p, miR-6837-5p, miR-6846-3p, miR-6858-3p, miR-6859-3p, miR-6861-3p, miR-6880-3p, miR-7111-3p, miR-7152-5p, miR-642a-5p, miR-657, miR-1236-3p, miR-764, miR-4314, miR-3675-3p, miR-5703, miR-3191-5p, miR-6511a-3p, miR-6809-3p, miR-6815-5p, miR-6857-3p, miR-6878-3p, miR-766-3p, miR-1229-3p, miR-1306-5p, miR-210-5p, miR-198, miR-485-3p, miR-668-3p, miR-532-3p, miR-877-3p, miR-1238-3p, miR-3130-5p, miR-4298, miR-4290, miR-3943, miR-346 or miR-767-3p, or any combination thereof, any congener thereof, any transcript thereof, or any variant or derivative thereof.

In one embodiment, a nucleic acid probe(s) or primer(s) capable of being used for detection of dementia or diagnosis of dementia enables qualitative and/or quantitative measurement of the presence, expression level(s), or abundance of target nucleic acid(s) for dementia: human-derived miR-5698, miR-1915-3p, miR-1343-5p, miR-6861-5p, miR-6781-5p, miR-4508, miR-6743-5p, miR-6726-5p, miR-4525, miR-4651, miR-6813-5p, miR-5787, miR-1290, miR-6075, miR-4758-5p, miR-4690-5p, miR-762, miR-371a-5p, miR-6765-3p, miR-6784-5p, miR-6778-5p, miR-6875-5p, miR-4534, miR-4721, miR-6756-5p, miR-615-5p, miR-6727-5p, miR-6887-5p, miR-8063, miR-6880-5p, miR-6805-3p, miR-4726-5p, miR-4710, miR-7111-5p, miR-3619-3p, miR-6795-5p, miR-1254, miR-1233-5p, miR-6836-3p, miR-6769a-5p, miR-4532, miR-365a-5p, miR-1231, miR-1228-5p, miR-4430, miR-296-3p, miR-1237-5p, miR-4466, miR-6789-5p, miR-4632-5p, miR-4745-5p, miR-4665-5p, miR-6807-5p and miR-7114-5p, miR-150-3p, miR-423-5p, miR-575, miR-671-5p, miR-939-5p, miR-1225-3p, miR-3184-5p, or miR-3665, or any combination thereof, any congener thereof, any transcript thereof, or any variant or derivative thereof.

The expression level(s) of the above target nucleic acid(s) in samples (herein also referred to as "dementia subject samples") from patients having dementia (herein also referred to as "dementia patients") may be increased or decreased (hereinafter, also referred to as an "increase/decrease"), depending on the kind(s) of the target nucleic acid(s), than samples (herein also referred to as "non-dementia subject samples") from subjects with normal cognitive functions who do not have dementia (herein also referred to as "non-dementia subjects," which includes patients who do not have dementia, but have diseases other than dementia). Thus, a kit or device of the present invention can be effectively used for detection of dementia by measuring the expression level(s) of the above target nucleic acid(s) in body fluid derived from a subject (e.g., a human) suspected of having dementia and in body fluids derived from non-dementia subjects and then comparing the expression level(s) therebetween.

A nucleic acid probe(s) or primer(s) capable of being used in the present invention is a nucleic acid probe(s) capable of specifically binding to a polynucleotide(s) consisting of a nucleotide sequence(s) represented by at least one selected from SEQ ID NOs: 1 to 210, 374, 1315 to 1350, and 1435 to 1448 or to a complementary strand(s) of the polynucleotide(s), or a primer(s) for amplifying a polynucleotide(s) consisting of a nucleotide sequence(s) represented by at least one selected from SEQ ID NOs: 1 to 210, 374, 1315 to 1350, and 1435 to 1448.

The nucleic acid probes or primers capable of being used in the present invention may further comprise a nucleic acid probe(s) capable of specifically binding to a polynucleotide(s) consisting of a nucleotide sequence(s) represented by at least one selected from SEQ ID NOs: 211 to 249, and 1351 to 1356, and 1449 to 1453 or to a complementary strand(s) of the polynucleotide(s), or a primer(s) for amplifying a polynucleotide(s) consisting of a nucleotide sequence(s) represented by at least one selected from SEQ ID NOs: 211 to 249, and 1351 to 1356, and 1449 to 1453.

A nucleic acid probe(s) or primer(s) capable of being used in the present invention is or may further comprise a nucleic acid probe(s) capable of specifically binding to a polynucleotide(s) consisting of a nucleotide sequence(s) represented by at least one selected from SEQ ID NOs: 250 to 373 or to a complementary strand(s) of the polynucleotide(s), or a primer(s) for amplifying a polynucleotide(s) consisting of a nucleotide sequence(s) represented by at least one selected from SEQ ID NOs: 250 to 373.

The nucleic acid probes or primers capable of being used in the present invention may further comprise a nucleic acid probe(s) capable of specifically binding to a polynucleotide(s) consisting of a nucleotide sequence(s) represented by at least one selected from SEQ ID NOs: 375 to 390 or to a complementary strand(s) of the polynucleotide(s), or a primer(s) for amplifying a polynucleotide(s) consisting of a nucleotide sequence(s) represented by at least one selected from SEQ ID NOs: 375 to 390.

In a preferred embodiment of the method according to preferred embodimentthe present invention, the above nucleic acid probe(s) or primer(s) includes any combination of one or more polynucleotides selected from a group of polynucleotides comprising a nucleotide sequence(s) represented by any of SEQ ID NOs: 1 to 1314, 1315 to 1434, and 1435 to 1505 and a nucleotide sequence(s) derived from the nucleotide sequence(s) by the replacement of u with t and a group of polynucleotides complementary thereto, a group of polynucleotides hybridizing under stringent conditions

## EP 4 697 021 A2

(described below) to DNA comprising a nucleotide sequence(s) complementary to the former nucleotide sequence(s) and a group of polynucleotides complementary thereto, and a group of polynucleotides comprising 15 or more, preferably 17 or more consecutive nucleotides in the nucleotide sequence(s) of these polynucleotide groups. These polynucleotides can be used as nucleic acid probes and primers for detecting target nucleic acids, namely the above dementia markers.

Further, specific examples of the nucleic acid probe(s) or primer(s) capable of being used in the present invention include one or more polynucleotides selected from the group consisting of any of the following polynucleotides (a) to (e):

(a) a polynucleotide consisting of a nucleotide sequence represented by any of SEQ ID NOs: 1 to 210, 374, 1315 to 1350, and 1435 to 1448 or a nucleotide sequence derived from the nucleotide sequence by the replacement of u with t, a variant thereof, a derivative thereof, or a fragment thereof comprising 15 or more consecutive nucleotides,
(b) a polynucleotide comprising a nucleotide sequence represented by any of SEQ ID NOs: 1 to 210, 374, 1315 to 1350, and 1435 to 1448,
(c) a polynucleotide consisting of a nucleotide sequence complementary to a nucleotide sequence represented by any of SEQ ID NOs: 1 to 210, 374, 1315 to 1350, and 1435 to 1448 or a nucleotide sequence derived from the nucleotide sequence by the replacement of u with t, a variant thereof, a derivative thereof, or a fragment thereof comprising 15 or more consecutive nucleotides,
(d) a polynucleotide comprising a nucleotide sequence complementary to a nucleotide sequence represented by any of SEQ ID NOs: 1 to 210, 374, 1315 to 1350, and 1435 to 1448 or a nucleotide sequence derived from the nucleotide sequence by the replacement of u with t, and
(e) a polynucleotide hybridizing under stringent conditions to any of the polynucleotides (a) to (d).

In addition to at least one polynucleotide selected from any of the above polynucleotides (a) to (e), the nucleic acid probe(s) or primer(s) capable of being used in the present invention can further comprise a polynucleotide represented by any of the following (f) to (j), (k) to (o), and (p) to (t):

(f) a polynucleotide consisting of a nucleotide sequence represented by any of SEQ ID NOs: 211 to 249, 1351 to 1356, and 1449 to 1453 or a nucleotide sequence derived from the nucleotide sequence by the replacement of u with t, a variant thereof, a derivative thereof, or a fragment thereof comprising 15 or more consecutive nucleotides,
(g) a polynucleotide comprising a nucleotide sequence represented by any of SEQ ID NOs: 211 to 249, 1351 to 1356, and 1449 to 1453,
(h) a polynucleotide consisting of a nucleotide sequence complementary to a nucleotide sequence represented by any of SEQ ID NOs: 211 to 249, 1351 to 1356, and 1449 to 1453 or a nucleotide sequence derived from the nucleotide sequence by the replacement of u with t, a variant thereof, a derivative thereof, or a fragment thereof comprising 15 or more consecutive nucleotides,
(i) a polynucleotide comprising a nucleotide sequence complementary to a nucleotide sequence represented by any of SEQ ID NOs: 211 to 249, 1351 to 1356, and 1449 to 1453 or a nucleotide sequence derived from the nucleotide sequence by the replacement of u with t, and
(j) a polynucleotide hybridizing under stringent conditions to any of the polynucleotides (f) to (i),
(k) a polynucleotide consisting of a nucleotide sequence represented by any of SEQ ID NOs: 250 to 373 or a nucleotide sequence derived from the nucleotide sequence by the replacement of u with t, a variant thereof, a derivative thereof, or a fragment thereof comprising 15 or more consecutive nucleotides,
(l) a polynucleotide comprising a nucleotide sequence represented by any of SEQ ID NOs: 250 to 373,
(m) a polynucleotide consisting of a nucleotide sequence complementary to a nucleotide sequence represented by any of SEQ ID NOs: 250 to 373 or a nucleotide sequence derived from the nucleotide sequence by the replacement of u with t, a variant thereof, a derivative thereof, or a fragment thereof comprising 15 or more consecutive nucleotides,
(n) a polynucleotide comprising a nucleotide sequence complementary to a nucleotide sequence represented by any of SEQ ID NOs: 250 to 373 or a nucleotide sequence derived from the nucleotide sequence by the replacement of u with t, and
(o) a polynucleotide hybridizing under stringent conditions to any of the polynucleotides (k) to (n),
(p) a polynucleotide consisting of a nucleotide sequence represented by any of SEQ ID NOs: 375 to 390 or a nucleotide sequence derived from the nucleotide sequence by the replacement of u with t, a variant thereof, a derivative thereof, or a fragment thereof comprising 15 or more consecutive nucleotides,
(q) a polynucleotide comprising a nucleotide sequence represented by any of SEQ ID NOs: 375 to 390,
(r) a polynucleotide consisting of a nucleotide sequence complementary to a nucleotide sequence represented by any of SEQ ID NOs: 375 to 390 or a nucleotide sequence derived from the nucleotide sequence by the replacement of u with t, a variant thereof, a derivative thereof, or a fragment thereof comprising 15 or more consecutive nucleotides,
(s) a polynucleotide comprising a nucleotide sequence complementary to a nucleotide sequence represented by any of SEQ ID NOs: 375 to 390 or a nucleotide sequence derived from the nucleotide sequence by the replacement of u

**EP 4 697 021 A2**

with t, and

(t) a polynucleotide hybridizing under stringent conditions to any of the polynucleotides (p) to (s).

Further, examples of the nucleic acid probe(s) or primer(s) capable of being used in the present invention include one or more polynucleotides, which may be optionally combined with a polynucleotide represented by any of the above (p) to (s), selected from the group consisting of any of the following polynucleotides (u) to (y):

(u) a polynucleotide consisting of a nucleotide sequence represented by any of SEQ ID NOs: 3, 8, 15, 26, 34, 40, 53, 69, 99, 101, 107, 109, 112, 125, 128, 133, 191 to 194, 212, and 250 to 374 or a nucleotide sequence derived from the nucleotide sequence by the replacement of u with t, a variant thereof, a derivative thereof, or a fragment thereof comprising 15 or more consecutive nucleotides,

(v) a polynucleotide comprising a nucleotide sequence represented by any of SEQ ID NOs: 3, 8, 15, 26, 34, 40, 53, 69, 99, 101, 107, 109, 112, 125, 128, 133, 191 to 194, 212, and 250 to 374,

(w) a polynucleotide consisting of a nucleotide sequence complementary to a nucleotide sequence represented by any of SEQ ID NOs: 3, 8, 15, 26, 34, 40, 53, 69, 99, 101, 107, 109, 112, 125, 128, 133, 191 to 194, 212, and 250 to 374 or a nucleotide sequence derived from the nucleotide sequence by the replacement of u with t, a variant thereof, a derivative thereof, or a fragment thereof comprising 15 or more consecutive nucleotides,

(x) a polynucleotide comprising a nucleotide sequence complementary to a nucleotide sequence represented by any of SEQ ID NOs: 3, 8, 15, 26, 34, 40, 53, 69, 99, 101, 107, 109, 112, 125, 128, 133, 191 to 194, 212, and 250 to 374 or a nucleotide sequence derived from the nucleotide sequence by the replacement of u with t, and

(y) a polynucleotide hybridizing under stringent conditions to any of the polynucleotides (u) to (x).

In one embodiment, a nucleic acid probe(s) or primer(s) capable of being used in the present invention is a nucleic acid probe(s) capable of specifically binding to a polynucleotide(s) consisting of a nucleotide sequence(s) represented by at least one selected from SEQ ID NOs: 194 to 210, 374, 1315 to 1350, and 1435 to 1448 or to a complementary strand(s) of the polynucleotide(s), or a primer(s) for amplifying a polynucleotide(s) consisting of a nucleotide sequence(s) represented by at least one selected from SEQ ID NOs: 194 to 210, 374, 1315 to 1350, and 1435 to 1448.

A nucleic acid probe(s) or primer(s) capable of being used in the present invention is a nucleic acid probe(s) capable of specifically binding to a polynucleotide(s) consisting of a nucleotide sequence(s) represented by at least one selected from SEQ ID NOs: 211 to 212, 1351 to 1356, and 1449 to 1453 or to a complementary strand(s) of the polynucleotide(s), or a primer(s) for amplifying a polynucleotide(s) consisting of a nucleotide sequence(s) represented by at least one selected from SEQ ID NOs: 211 to 212, 1351 to 1356, and 1449 to 1453.

In a preferred embodiment of the method according to the present invention, the above nucleic acid probe(s) or primer(s) comprises any combination of one or more polynucleotides selected from a group of polynucleotides comprising a nucleotide sequence(s) represented by any of SEQ ID NOs: 194 to 212, 374, 398, 490, 591, 593 to 609, 766, 1015 to 1017, 1019 to 1024, 1026 to 1031, 1285 to 1286, and 1315 to 1434 and a nucleotide sequence(s) derived from the nucleotide sequence(s) by the replacement of u with t and a group of polynucleotides complementary thereto, a group of polynucleotides hybridizing under stringent conditions (described below) to DNA comprising a nucleotide sequence(s) complementary to the former nucleotide sequence(s) and a group of polynucleotides complementary thereto, and a group of polynucleotides comprising 15 or more, preferably 17 or more consecutive nucleotides in the nucleotide sequence(s) of these polynucleotide groups. These polynucleotides can be used as nucleic acid probes and primers for detecting target nucleic acids, namely the above dementia markers.

In one embodiment, examples of the nucleic acid probe(s) or primer(s) capable of being used in the present invention include one or more polynucleotides selected from the group consisting of any of the following polynucleotides (a) to (e):

(a) a polynucleotide consisting of a nucleotide sequence represented by any of SEQ ID NOs: 194 to 210, 374, and 1315 to 1350 or a nucleotide sequence derived from the nucleotide sequence by the replacement of u with t, a variant thereof, a derivative thereof, or a fragment thereof comprising 15 or more consecutive nucleotides,

(b) a polynucleotide comprising a nucleotide sequence represented by any of SEQ ID NOs: 194 to 210, 374, and 1315 to 1350,

(c) a polynucleotide consisting of a nucleotide sequence complementary to a nucleotide sequence represented by any of SEQ ID NOs: 194 to 210, 374, and 1315 to 1350 or a nucleotide sequence derived from the nucleotide sequence by the replacement of u with t, a variant thereof, a derivative thereof, or a fragment thereof comprising 15 or more consecutive nucleotides,

(d) a polynucleotide comprising a nucleotide sequence complementary to a nucleotide sequence represented by any of SEQ ID NOs: 194 to 210, 374, and 1315 to 1350 or a nucleotide sequence derived from the nucleotide sequence by the replacement of u with t, and

(e) a polynucleotide hybridizing under stringent conditions to any of the polynucleotides (a) to (d).

In one embodiment, in addition to at least one polynucleotide selected from any of the above polynucleotides (a) to (e), the nucleic acid probe(s) or primer(s) capable of being used in the present invention may further comprise a polynucleotide represented by any of the following (f) to (j):

(f) a polynucleotide consisting of a nucleotide sequence represented by any of SEQ ID NOs: 211 to 212 and 1351 to 1356 or a nucleotide sequence derived from the nucleotide sequence by the replacement of u with t, a variant thereof, a derivative thereof, or a fragment thereof comprising 15 or more consecutive nucleotides,
(g) a polynucleotide comprising a nucleotide sequence represented by any of SEQ ID NOs: 211 to 212 and 1351 to 1356,
(h) a polynucleotide consisting of a nucleotide sequence complementary to a nucleotide sequence represented by any of SEQ ID NOs: 211 to 212 and 1351 to 1356 or a nucleotide sequence derived from the nucleotide sequence by the replacement of u with t, a variant thereof, a derivative thereof, or a fragment thereof comprising 15 or more consecutive nucleotides,
(i) a polynucleotide comprising a nucleotide sequence complementary to a nucleotide sequence represented by any of SEQ ID NOs: 211 to 212 and 1351 to 1356 or a nucleotide sequence derived from the nucleotide sequence by the replacement of u with t, and
(j) a polynucleotide hybridizing under stringent conditions to any of the polynucleotides (f) to (i).

The above polynucleotides or fragments thereof used in the present invention may each be DNA or RNA.
The above polynucleotide(s) capable of being used in the present invention may be prepared using a general technique such as DNA recombination technology, a PCR method, or a method using an automated DNA/RNA synthesizer.
As the DNA recombination technology or the PCR method, it is possible to use techniques described in, for instance, Ausubel et al., Current Protocols in Molecular Biology, John Willey & Sons, US(1993); and Sambrook et al., Molecular Cloning A Laboratory Manual, Cold Spring Harbor Laboratory Press, US (1989).
The human-derived miR-4274, miR-4272, miR-4728-5p, miR-4443, miR-4506, miR-6773-5p, miR-4662a-5p, miR-3184-3p, miR-4281, miR-320d, miR-6729-3p, miR-5192, miR-6853-5p, miR-1234-3p, miR-1233-3p, miR-4539, miR-3914, miR-4738-5p, miR-548au-3p, miR-1539, miR-4720-3p, miR-365b-5p, miR-4486, miR-1227-5p, miR-4667-5p, miR-6088, miR-6820-5p, miR-4505, miR-548q, miR-4658, miR-450a-5p, miR-1260b, miR-3677-5p, miR-6777-3p, miR-6826-3p, miR-6832-3p, miR-4725-3p, miR-7161-3p, miR-2277-5p, miR-7110-3p, miR-4312, miR-4461, miR-6766-5p, miR-1266-3p, miR-6729-5p, miR-526b-3p, miR-519e-5p, miR-512-5p, miR-5088-5p, miR-1909-3p, miR-6511a-5p, miR-4734, miR-936, miR-1249-3p, miR-6777-5p, miR-4487, miR-3155a, miR-563, miR-4741, miR-6788-5p, miR-4433b-5p, miR-323a-5p, miR-6811-5p, miR-6721-5p, miR-5004-5p, miR-6509-3p, miR-648, miR-3917, miR-6087, miR-1470, miR-586, miR-3150a-5p, miR-105-3p, miR-7973, miR-1914-5p, miR-4749-3p, miR-15b-5p, miR-1289, miR-4433a-5p, miR-3666, miR-3186-3p, miR-4725-5p, miR-4488, miR-4474-3p, miR-6731-3p, miR-4640-3p, miR-202-5p, miR-6816-5p, miR-638, miR-6821-5p, miR-1247-3p, miR-6765-5p, miR-6800-5p, miR-3928-3p, miR-3940-5p, miR-3960, miR-6775-5p, miR-3178, miR-1202, miR-6790-5p, miR-4731-3p, miR-2681-3p, miR-6758-5p, miR-8072, miR-518d-3p, miR-3606-3p, miR-4800-5p, miR-1292-3p, miR-6784-3p, miR-4450, miR-6132, miR-4716-5p, miR-6860, miR-1268b, miR-378d, miR-4701-5p, miR-4329, miR-185-3p, miR-552-3p, miR-1273g-5p, miR-6769b-3p, miR-520a-3p, miR-4524b-5p, miR-4291, miR-6734-3p, miR-143-5p, miR-939-3p, miR-6889-3p, miR-6842-3p, miR-4511, miR-4318, miR-4653-5p, miR-6867-3p, miR-133b, miR-3196, miR-193b-3p, miR-3162-3p, miR-6819-3p, miR-1908-3p, miR-6786-5p, miR-3648, miR-4513, miR-3652, miR-4640-5p, miR-6871-5p, miR-7845-5p, miR-3138, miR-6884-5p, miR-4653-3p, miR-636, miR-4652-3p, miR-6823-5p, miR-4502, miR-7113-5p, miR-8087, miR-7154-3p, miR-5189-5p, miR-1253, miR-518c-5p, miR-7151-5p, miR-3614-3p, miR-4727-5p, miR-3682-5p, miR-5090, miR-337-3p, miR-488-5p, miR-100-5p, miR-4520-3p, miR-373-3p, miR-6499-5p, miR-3909, miR-32-5p, miR-302a-3p, miR-4686, miR-4659a-3p, miR-4287, miR-1301-5p, miR-593-3p, miR-517a-3p, miR-517b-3p, miR-142-3p, miR-1185-2-3p, miR-602, miR-527, miR-518a-5p, miR-4682, miR-28-5p, miR-4252, miR-452-5p, miR-525-5p, miR-3622a-3p, miR-6813-3p, miR-4769-3p, miR-5698, miR-1915-3p, miR-1343-5p, miR-6861-5p, miR-6781-5p, miR-4508, miR-6743-5p, miR-6726-5p, miR-4525, miR-4651, miR-6813-5p, miR-5787, miR-1290, miR-6075, miR-4758-5p, miR-4690-5p, miR-762, miR-1225-3p, miR-3184-5p, miR-665, miR-211-5p, miR-1247-5p, miR-3656, miR-149-5p, miR-744-5p, miR-345-5p, miR-150-5p, miR-191-3p, miR-651-5p, miR-34a-5p, miR-409-5p, miR-369-5p, miR-1915-5p, miR-204-5p, miR-137, miR-382-5p, miR-517-5p, miR-532-5p, miR-22-5p, miR-1237-3p, miR-1224-3p, miR-625-3p, miR-328-3p, miR-122-5p, miR-202-3p, miR-4781-5p, miR-718, miR-342-3p, miR-26b-3p, miR-140-3p, miR-200a-3p, miR-378a-3p, miR-484, miR-296-5p, miR-205-5p, miR-431-5p, miR-1471, miR-1538, miR-449b-3p, miR-1976, miR-4268, miR-4279, miR-3620-3p, miR-3944-3p, miR-3156-3p, miR-3187-5p, miR-4685-3p, miR-4695-3p, miR-4697-3p, miR-4713-5p, miR-4723-3p, miR-371b-3p, miR-3151-3p, miR-3192-3p, miR-6728-3p, miR-6736-3p, miR-6740-3p, miR-6741-3p, miR-6743-3p, miR-6747-3p, miR-6750-3p, miR-6754-3p, miR-6759-3p, miR-6761-3p, miR-6762-3p, miR-6769a-3p, miR-6776-3p, miR-6778-3p, miR-6779-3p, miR-6786-3p, miR-6787-3p, miR-6792-3p, miR-6794-3p, miR-6801-3p, miR-6802-3p, miR-6803-3p, miR-6804-3p, miR-6810-5p, miR-6823-3p, miR-6825-3p, miR-6829-3p, miR-6833-3p, miR-6834-3p, miR-6780b-3p, miR-6845-3p, miR-6862-3p, miR-6865-3p, miR-6870-3p, miR-6875-3p, miR-6877-3p, miR-6879-3p, miR-6882-3p, miR-6885-3p, miR-6886-3p, miR-6887-3p, miR-6890-3p, miR-6893-3p, miR-6894-3p, miR-7106-3p, miR-7109-3p, miR-7114-3p, miR-7155-5p, miR-7160-5p, miR-615-3p, miR-920, miR-1825, miR-675-3p, miR-1910-5p, miR-2278, miR-2682-3p, miR-3122, miR-3151-5p, miR-3175, miR-4323, miR-4326, miR-4284, miR-3605-3p, miR-3622b-5p, miR-3646, miR-3158-5p, miR-4722-3p, miR-4728-3p, miR-4747-3p, miR-4436b-5p, miR-5196-3p, miR-5589-5p, miR-345-3p, miR-642b-5p, miR-6716-3p, miR-6511b-3p, miR-208a-5p, miR-6726-3p, miR-6744-5p, miR-6782-3p, miR-6789-3p, miR-6797-3p, miR-6800-3p, miR-6806-5p, miR-6824-3p, miR-6837-5p, miR-6846-3p, miR-6858-3p, miR-6859-3p, miR-6861-3p, miR-6880-3p, miR-7111-3p, miR-7152-5p, miR-642a-5p, miR-657, miR-1236-3p, miR-764, miR-4314, miR-3675-3p, miR-5703, miR-3191-5p, miR-6511a-3p, miR-6809-3p, miR-6815-5p, miR-6857-3p, miR-6878-3p, miR-371a-5p, miR-766-3p, miR-1229-3p, miR-1306-5p, miR-210-5p, miR-198, miR-485-3p, miR-668-3p, miR-532-3p, miR-877-3p, miR-1238-3p, miR-3130-5p, miR-4298, miR-4290, miR-3943, miR-346 and miR-767-3p, miR-6765-3p, miR-6784-5p, miR-6778-5p, miR-6875-5p, miR-4534, miR-4721, miR-6756-5p, miR-615-5p, miR-6727-5p, miR-6887-5p, miR-8063, miR-6880-5p, miR-6805-3p, miR-4726-5p, miR-4710, miR-7111-5p, miR-3619-3p, miR-6795-5p, miR-1254, miR-1233-5p, miR-6836-3p, miR-6769a-5p, miR-4532, miR-365a-5p, miR-1231, miR-1228-5p, miR-4430, miR-296-3p, miR-1237-5p, miR-4466, miR-6789-5p, miR-4632-5p, miR-4745-5p, miR-4665-5p, miR-6807-5p and miR-7114-5p, miR-516a-5p, miR-769-3p, miR-3692-5p, miR-3945, miR-4433a-3p, miR-4485-3p, miR-6831-5p, miR-519c-5p, miR-551b-5p, miR-1343-3p, miR-4286, miR-4634, miR-4733-3p, miR-6086, miR-30d-5p, miR-30b-3p, miR-92a-3p, miR-371b-5p, and miR-486-5p, which are represented by SEQ ID NOs: 1 to 210, 211 to 249, 250 to 374, 375 to 390, 1315 to 1350, 1435 to 1448, and 1449 to 1453, are known, and methods for obtaining them are also known, as described above. Therefore, a polynucleotide that can be used as a nucleic acid probe or a primer in the present invention can be produced via cloning the gene.

Moreover, the followings are also known, and methods for obtaining them are also known, as described above: human-derived hsa-mir-4274, hsa-mir-4272, hsa-mir-4728, hsa-mir-4443, hsa-mir-4506, hsa-mir-6773, hsa-mir-4662a, hsa-mir-3184, hsa-mir-4281, hsa-mir-320d-1, hsa-mir-320d-2, hsa-mir-6729, hsa-mir-5192, hsa-mir-6853, hsa-mir-1234, hsa-mir-1233-1, hsa-mir-1233-2, hsa-mir-4539, hsa-mir-3914-1, hsa-mir-3914-2, hsa-mir-4738, hsa-mir-548au, hsa-mir-1539, hsa-mir-4720, hsa-mir-365b, hsa-mir-4486, hsa-mir-1227, hsa-mir-4667, hsa-mir-6088, hsa-mir-6820, hsa-mir-4505, hsa-mir-548q, hsa-mir-4658, hsa-mir-450a-1, hsa-mir-450a-2, hsa-mir-1260b, hsa-mir-3677, hsa-mir-6777, hsa-mir-6826, hsa-mir-6832, hsa-mir-4725, hsa-mir-7161, hsa-mir-2277, hsa-mir-7110, hsa-mir-4312, hsa-mir-4461, hsa-mir-6766, hsa-mir-1266, hsa-mir-526b, hsa-mir-519e, hsa-mir-512-1, hsa-mir-512-2, hsa-mir-5088, hsa-mir-1909, hsa-mir-6511a-1, hsa-mir-6511a-2, hsa-mir-6511a-3, hsa-mir-6511a-4, hsa-mir-4734, hsa-mir-936, hsa-mir-1249, hsa-mir-4487, hsa-mir-3155a, hsa-mir-563, hsa-mir-4741, hsa-mir-6788, hsa-mir-4433b, hsa-mir-323a, hsa-mir-6811, hsa-mir-6721, hsa-mir-5004, hsa-mir-6509, hsa-mir-648, hsa-mir-3917, hsa-mir-6087, hsa-mir-1470, hsa-mir-586, hsa-mir-3150a, hsa-mir-105-1, hsa-mir-105-2, hsa-mir-7973-1, hsa-mir-7973-2, hsa-mir-1914, hsa-mir-4749, hsa-mir-15b, hsa-mir-1289-1, hsa-mir-1289-2, hsa-mir-4433a, hsa-mir-3666, hsa-mir-3186, hsa-mir-4488, hsa-mir-4474, hsa-mir-6731, hsa-mir-4640, hsa-mir-202, hsa-mir-6816, hsa-mir-638, hsa-mir-6821, hsa-mir-1247, hsa-mir-6765, hsa-mir-6800, hsa-mir-3928, hsa-mir-3940, hsa-mir-3960, hsa-mir-6775, hsa-mir-3178, hsa-mir-1202, hsa-mir-6790, hsa-mir-4731, hsa-mir-2681, hsa-mir-6758, hsa-mir-8072, hsa-mir-518d, hsa-mir-3606, hsa-mir-4800, hsa-mir-1292, hsa-mir-6784, hsa-mir-4450, hsa-mir-6132, hsa-mir-4716, hsa-mir-6860, hsa-mir-1268b, hsa-mir-378d-2, hsa-mir-378d-1, hsa-mir-4701, hsa-mir-4329, hsa-mir-185, hsa-mir-552, hsa-mir-1273g, hsa-mir-6769b, hsa-mir-520a, hsa-mir-4524b, hsa-mir-4291, hsa-mir-6734, hsa-mir-143, hsa-mir-939, hsa-mir-6889, hsa-mir-6842, hsa-mir-4511, hsa-mir-4318, hsa-mir-4653, hsa-mir-6867, hsa-mir-133b, hsa-mir-3196, hsa-mir-193b, hsa-mir-3162, hsa-mir-6819, hsa-mir-1908, hsa-mir-6786, hsa-mir-3648-1, hsa-mir-3648-2, hsa-mir-4513, hsa-mir-3652, hsa-mir-6871, hsa-mir-7845, hsa-mir-3138, hsa-mir-6884, hsa-mir-636, hsa-mir-4652, hsa-mir-6823, hsa-mir-4502, hsa-mir-7113, hsa-mir-8087, hsa-mir-7154, hsa-mir-5189, hsa-mir-1253, hsa-mir-518c, hsa-mir-7151, hsa-mir-3614, hsa-mir-4727, hsa-mir-3682, hsa-mir-5090, hsa-mir-337, hsa-mir-488, hsa-mir-100, hsa-mir-4520-1, hsa-mir-373, hsa-mir-6499, hsa-mir-3909, hsa-mir-32, hsa-mir-302a, hsa-mir-4686, hsa-mir-4659a, hsa-mir-4287, hsa-mir-1301, hsa-mir-593, hsa-mir-517a, hsa-mir-517b, hsa-mir-142, hsa-mir-1185-2, hsa-mir-602, hsa-mir-527, hsa-mir-518a-1, hsa-mir-518a-2, hsa-mir-4682, hsa-mir-28, hsa-mir-4252, hsa-mir-452, hsa-mir-525, hsa-mir-3622a, hsa-mir-6813, hsa-mir-4769, hsa-mir-5698, hsa-mir-1915, hsa-mir-1343, hsa-mir-6861, hsa-mir-6781, hsa-mir-4508, hsa-mir-6743, hsa-mir-6726, hsa-mir-4525, hsa-mir-4651, hsa-mir-5787, hsa-mir-1290, hsa-mir-6075, hsa-mir-4758, hsa-mir-4690, hsa-mir-762, hsa-mir-1225, hsa-mir-665, hsa-mir-211, hsa-mir-3656, hsa-mir-149, hsa-mir-744, hsa-mir-345, hsa-mir-150, hsa-mir-191, hsa-mir-651, hsa-mir-34a, hsa-mir-409, hsa-mir-369, hsa-mir-204, hsa-mir-137, hsa-mir-382, hsa-mir-517c, hsa-mir-532, hsa-mir-22, hsa-mir-1237, hsa-mir-1224, hsa-mir-625, hsa-mir-328, hsa-mir-122, hsa-mir-4781, hsa-mir-718, hsa-mir-342, hsa-mir-26b, hsa-mir-140, hsa-mir-200a, hsa-mir-378a, hsa-mir-484, hsa-mir-296, hsa-mir-205, hsa-mir-431, hsa-mir-1471, hsa-mir-1538, hsa-mir-449b, hsa-mir-1976, hsa-mir-4268, hsa-mir-4279, hsa-mir-3620, hsa-mir-3944, hsa-mir-3156-1, hsa-mir-3156-2, hsa-mir-3187, hsa-mir-4685, hsa-mir-4695, hsa-mir-4697, hsa-mir-4713, hsa-mir-4723, hsa-mir-371b, hsa-mir-3151, hsa-mir-3192, hsa-mir-6728, hsa-mir-6736, hsa-mir-6740, hsa-mir-6741, hsa-mir-6747, hsa-mir-6750, hsa-mir-6754, hsa-mir-6759, hsa-mir-6761, hsa-mir-6762, hsa-mir-6769a, hsa-mir-6776, hsa-mir-6778, hsa-mir-6779, hsa-mir-6787, hsa-mir-6792, hsa-mir-6794, hsa-mir-6801, hsa-mir-6802, hsa-mir-6803, hsa-mir-6804, hsa-mir-6810, hsa-mir-6825, hsa-mir-6829, hsa-mir-6833, hsa-mir-6834, hsa-mir-6780b, hsa-mir-6845, hsa-mir-6862-1, hsa-mir-6862-2, hsa-mir-6865, hsa-mir-6870, hsa-mir-6875, hsa-mir-6877, hsa-mir-6879, hsa-mir-6882, hsa-mir-6885, hsa-mir-6886, hsa-mir-6887, hsa-mir-6890, hsa-mir-6893, hsa-mir-6894, hsa-mir-7106, hsa-mir-7109, hsa-mir-7114, hsa-mir-7155, hsa-mir-7160, hsa-mir-615, hsa-mir-920, hsa-mir-1825, hsa-mir-675, hsa-mir-1910, hsa-mir-2278, hsa-mir-2682, hsa-mir-3122, hsa-mir-3175, hsa-mir-4323, hsa-mir-4326, hsa-mir-4284, hsa-mir-3605, hsa-mir-3622b, hsa-mir-3646, hsa-mir-3158-1, hsa-mir-3158-2, hsa-mir-4722, hsa-mir-4747, hsa-mir-4436b-1, hsa-mir-4436b-2, hsa-mir-5196, hsa-mir-5589, hsa-mir-642b, hsa-mir-6716, hsa-mir-6511b-1, hsa-mir-6511b-2, hsa-mir-208a, hsa-mir-6744, hsa-mir-6782, hsa-mir-6789, hsa-mir-6797, hsa-mir-6806, hsa-mir-6824, hsa-mir-6837, hsa-mir-6846, hsa-mir-6858, hsa-mir-6859-1, hsa-mir-6859-2, hsa-mir-6859-3, hsa-mir-6859-4, hsa-mir-6880, hsa-mir-7111, hsa-mir-7152, hsa-mir-642a, hsa-mir-657, hsa-mir-1236, hsa-mir-764, hsa-mir-4314, hsa-mir-3675, hsa-mir-5703, hsa-mir-3191, hsa-mir-6809, hsa-mir-6815, hsa-mir-6857, hsa-mir-6878, hsa-mir-371a, hsa-mir-766, hsa-mir-1229, hsa-mir-1306, hsa-mir-210, hsa-mir-198, hsa-mir-485, hsa-mir-668, hsa-mir-877, hsa-mir-1238, hsa-mir-3130-1, hsa-mir-3130-2, hsa-mir-4298, hsa-mir-4290, hsa-mir-3943, hsa-mir-346, hsa-mir-767, hsa-mir-516a-1, hsa-mir-769, hsa-mir-3692, hsa-mir-3945, hsa-mir-4433a, hsa-mir-4485, hsa-mir-6831, hsa-mir-519c, hsa-mir-551b, hsa-mir-1343, hsa-mir-4286, hsa-mir-4634, hsa-mir-4733, hsa-mir-6086, hsa-mir-30d, hsa-mir-30b, hsa-mir-92a-1, hsa-mir-371b, hsa-mir-486-1, isomiR sequence 1 of SEQ ID NO: 3, isomiR sequence 2 of SEQ ID NO: 3, isomiR sequence 1 of SEQ ID NO: 4, isomiR sequence 2 of SEQ ID NO: 4, isomiR sequence 1 of SEQ ID NO: 5, isomiR sequence 2 of SEQ ID NO: 5, isomiR sequence 1 of SEQ ID NO: 6, isomiR sequence 2 of SEQ ID NO: 6, isomiR sequence 1 of SEQ ID NO: 7, isomiR sequence 1 of SEQ ID NO: 8, isomiR sequence 1 of SEQ ID NO: 10, isomiR sequence 2 of SEQ ID NO: 10, isomiR sequence 1 of SEQ ID NO: 11, isomiR sequence 1 of SEQ ID NO: 12, isomiR sequence 1 of SEQ ID NO: 13, isomiR sequence 1 of SEQ ID NO: 15, isomiR sequence 2 of SEQ ID NO: 15, isomiR sequence 1 of SEQ ID NO: 16, isomiR sequence 1 of SEQ ID NO: 17, isomiR sequence 1 of SEQ ID NO: 19, isomiR sequence 1 of SEQ ID NO: 22, isomiR sequence 2 of SEQ ID NO: 22, isomiR sequence 1 of SEQ ID NO: 23, isomiR sequence 1 of SEQ ID NO: 25, isomiR sequence 2 of SEQ ID NO: 25, isomiR sequence 1 of SEQ ID NO: 26, isomiR sequence 2 of SEQ ID NO: 26, isomiR sequence 1 of SEQ ID NO: 28, isomiR sequence 1 of SEQ ID NO: 29, isomiR sequence 2 of SEQ ID NO: 29, isomiR sequence 1 of SEQ ID NO: 30, isomiR sequence 1 of SEQ ID NO: 31, isomiR sequence 2 of SEQ ID NO: 31, isomiR sequence 1 of SEQ ID NO: 32, isomiR sequence 2 of SEQ ID NO: 32, isomiR sequence 1 of SEQ ID NO: 33, isomiR sequence 1 of SEQ ID NO: 34, isomiR sequence 1 of SEQ ID NO: 35, isomiR sequence 1 of SEQ ID NO: 36, isomiR sequence 1 of SEQ ID NO: 37, isomiR sequence 2 of SEQ ID NO: 37, isomiR sequence 1 of SEQ ID NO: 39, isomiR sequence 2 of SEQ ID NO: 39, isomiR sequence 1 of SEQ ID NO: 40, isomiR sequence 2 of SEQ ID NO: 40, isomiR sequence 1 of SEQ ID NO: 41, isomiR sequence 1 of SEQ ID NO: 44, isomiR sequence 1 of SEQ ID NO: 47, isomiR sequence 1 of SEQ ID NO: 48, isomiR sequence 1 of SEQ ID NO: 49, isomiR sequence 2 of SEQ ID NO: 49, isomiR sequence 1 of SEQ ID NO: 50, isomiR sequence 2 of SEQ ID NO: 50, isomiR sequence 1 of SEQ ID NO: 51, isomiR sequence 2 of SEQ ID NO: 51, isomiR sequence 1 of SEQ ID NO: 52, isomiR sequence 1 of SEQ ID NO: 54, isomiR sequence 2 of SEQ ID NO: 54, isomiR sequence 1 of SEQ ID NO: 56, isomiR sequence 2 of SEQ ID NO: 56, isomiR sequence 1 of SEQ ID NO: 57, isomiR sequence 2 of SEQ ID NO: 57, isomiR sequence 1 of SEQ ID NO: 59, isomiR sequence 2 of SEQ ID NO: 59, isomiR sequence 1 of SEQ ID NO: 60, isomiR sequence 2 of SEQ ID NO: 60, isomiR sequence 1 of SEQ ID NO: 61, isomiR sequence 2 of SEQ ID NO: 61, isomiR sequence 1 of SEQ ID NO: 62, isomiR sequence 2 of SEQ ID NO: 62, isomiR sequence 1 of SEQ ID NO: 63, isomiR sequence 2 of SEQ ID NO: 63, isomiR sequence 1 of SEQ ID NO: 64, isomiR sequence 2 of SEQ ID NO: 64, isomiR sequence 1 of SEQ ID NO: 65, isomiR sequence 1 of SEQ ID NO: 66, isomiR sequence 1 of SEQ ID NO: 67, isomiR sequence 1 of SEQ ID NO: 68, isomiR sequence 2 of SEQ ID NO: 68, isomiR sequence 1 of SEQ ID NO: 69, isomiR sequence 2 of SEQ ID NO: 69, isomiR sequence 1 of SEQ ID NO: 70, isomiR sequence 1 of SEQ ID NO: 71, isomiR sequence 1 of SEQ ID NO: 72, isomiR sequence 2 of SEQ ID NO: 72, isomiR sequence 1 of SEQ ID NO: 73, isomiR sequence 2 of SEQ ID NO: 73, isomiR sequence 1 of SEQ ID NO: 75, isomiR sequence 1 of SEQ ID NO: 77, isomiR sequence 2 of SEQ ID NO: 77, isomiR sequence 1 of SEQ ID NO: 78, isomiR sequence 1 of SEQ ID NO: 79, isomiR sequence 1 of SEQ ID NO: 81, isomiR sequence 2 of SEQ ID NO: 81, isomiR sequence 1 of SEQ ID NO: 82, isomiR sequence 1 of SEQ ID NO: 83, isomiR sequence 2 of SEQ ID NO: 83, isomiR sequence 1 of SEQ ID NO: 84, isomiR sequence 1 of SEQ ID NO: 85, isomiR sequence 1 of SEQ ID NO: 86, isomiR sequence 1 of SEQ ID NO: 87, isomiR sequence 2 of SEQ ID NO: 87, isomiR sequence 1 of SEQ ID NO: 89, isomiR sequence 2 of SEQ ID NO: 89, isomiR sequence 1 of SEQ ID NO: 91, isomiR sequence 2 of SEQ ID NO: 91, isomiR sequence 1 of SEQ ID NO: 94, isomiR sequence 2 of SEQ ID NO: 94, isomiR sequence 1 of SEQ ID NO: 95, isomiR sequence 2 of SEQ ID NO: 95, isomiR sequence 1 of SEQ ID NO: 96, isomiR sequence 2 of SEQ ID NO: 96, isomiR sequence 1 of SEQ ID NO: 98, isomiR sequence 2 of SEQ ID NO: 98, isomiR sequence 1 of SEQ ID NO: 99, isomiR sequence 1 of SEQ ID NO: 100, isomiR sequence 1 of SEQ ID NO: 101, isomiR sequence 1 of SEQ ID NO: 102, isomiR sequence 1 of SEQ ID NO: 103, isomiR sequence 2 of SEQ ID NO: 103, isomiR sequence 1 of SEQ ID NO: 105, isomiR sequence 1 of SEQ ID NO: 107, isomiR sequence 1 of SEQ ID NO: 109, isomiR sequence 1 of SEQ ID NO: 111, isomiR sequence 1 of SEQ ID NO: 112, isomiR sequence 2 of SEQ ID NO: 112, isomiR sequence 1 of SEQ ID NO: 113, isomiR sequence 1 of SEQ ID NO: 114, isomiR sequence 2 of SEQ ID NO: 114, isomiR sequence 1 of SEQ ID NO: 115, isomiR sequence 2 of SEQ ID NO: 115, isomiR sequence 1 of SEQ ID NO: 116, isomiR sequence 2 of SEQ ID NO: 116, isomiR sequence 1 of SEQ ID NO: 118, isomiR sequence 2 of SEQ ID NO: 118, isomiR sequence 1 of SEQ ID NO: 119, isomiR sequence 1 of SEQ ID NO: 121, isomiR sequence 1 of SEQ ID NO: 122, isomiR sequence 1 of SEQ ID NO: 125, isomiR sequence 1 of SEQ ID NO: 126, isomiR sequence 2 of SEQ ID NO: 126, isomiR sequence 1 of SEQ ID NO: 127, isomiR sequence 2 of SEQ ID NO: 127, isomiR sequence 1 of SEQ ID NO: 128, isomiR sequence 1 of SEQ ID NO: 129, isomiR sequence 1 of SEQ ID NO: 130, isomiR sequence 2 of SEQ ID NO: 130, isomiR sequence 1 of SEQ ID NO: 133, isomiR sequence 1 of SEQ ID NO: 134, isomiR sequence 2 of SEQ ID NO: 134, isomiR sequence 1 of SEQ ID NO: 135, isomiR sequence 2 of SEQ ID NO: 135, isomiR sequence 1 of SEQ ID NO: 136, isomiR sequence 2 of SEQ ID NO: 136, isomiR sequence 1 of SEQ ID NO: 137, isomiR sequence 1 of SEQ ID NO: 138, isomiR sequence 1 of SEQ ID NO: 139, isomiR sequence 2 of SEQ ID NO: 139, isomiR sequence 1 of SEQ ID NO: 141, isomiR sequence 2 of SEQ ID NO: 141, isomiR sequence 1 of SEQ ID NO: 142, isomiR sequence 2 of SEQ ID NO: 142, isomiR sequence 1 of SEQ ID NO: 143, isomiR sequence 2 of SEQ ID NO: 143, isomiR sequence 1 of SEQ ID NO: 144, isomiR sequence 2 of SEQ ID NO: 144, isomiR sequence 1 of SEQ ID NO: 145, isomiR sequence 2 of SEQ ID NO: 145, isomiR sequence 1 of SEQ ID NO: 147, isomiR sequence 2 of SEQ ID NO: 147, isomiR sequence 1 of SEQ ID NO: 148, isomiR sequence 2 of SEQ ID NO: 148, isomiR sequence 1 of SEQ ID NO: 149, isomiR sequence 2 of SEQ ID NO: 149, isomiR sequence 1 of SEQ ID NO: 150, isomiR sequence 2 of SEQ ID NO: 150, isomiR sequence 1 of SEQ ID NO: 151, isomiR sequence 2 of SEQ ID NO: 151, isomiR sequence 1 of SEQ ID NO: 152, isomiR sequence 2 of SEQ ID NO: 152, isomiR sequence 1 of SEQ ID NO: 153, isomiR sequence 2 of SEQ ID NO: 153, isomiR sequence 1 of SEQ ID NO: 154, isomiR sequence 2 of SEQ ID NO: 154, isomiR sequence 1 of SEQ ID NO: 157, isomiR sequence 2 of SEQ ID NO: 157, isomiR sequence 1 of SEQ ID NO: 158, isomiR sequence 1 of SEQ ID NO: 159, isomiR sequence 1 of SEQ ID NO: 161, isomiR sequence 2 of SEQ ID NO: 161, isomiR sequence 1 of SEQ ID NO: 162, isomiR sequence 1 of SEQ ID NO: 163, isomiR sequence 2 of SEQ ID NO: 163, isomiR sequence 1 of SEQ ID NO: 164, isomiR sequence 2 of SEQ ID NO: 164, isomiR sequence 1 of SEQ ID NO: 165, isomiR sequence 2 of SEQ ID NO: 165, isomiR sequence 1 of SEQ ID NO: 166, isomiR sequence 1 of SEQ ID NO: 167, isomiR sequence 2 of SEQ ID NO: 167, isomiR sequence 1 of SEQ ID NO: 168, isomiR sequence 2 of SEQ ID NO: 168, isomiR sequence 1 of SEQ ID NO: 169, isomiR sequence 1 of SEQ ID NO: 170, isomiR sequence 2 of SEQ ID NO: 170, isomiR sequence 1 of SEQ ID NO: 171, isomiR sequence 2 of SEQ ID NO: 171, isomiR sequence 1 of SEQ ID NO: 172, isomiR sequence 2 of SEQ ID NO: 172, isomiR sequence 1 of SEQ ID NO: 173, isomiR sequence 2 of SEQ ID NO: 173, isomiR sequence 1 of SEQ ID NO: 175, isomiR sequence 2 of SEQ ID NO: 175, isomiR sequence 1 of SEQ ID NO: 177, isomiR sequence 2 of SEQ ID NO: 177, isomiR sequence 1 of SEQ ID NO: 179, isomiR sequence 2 of SEQ ID NO: 179, isomiR sequence 1 of SEQ ID NO: 180, isomiR sequence 2 of SEQ ID NO: 180, isomiR sequence 1 of SEQ ID NO: 181, isomiR sequence 2 of SEQ ID NO: 181, isomiR sequence 1 of SEQ ID NO: 182, isomiR sequence 2 of SEQ ID NO: 182, isomiR sequence 1 of SEQ ID NO: 185, isomiR sequence 1 of SEQ ID NO: 187, isomiR sequence 2 of SEQ ID NO: 187, isomiR sequence 1 of SEQ ID NO: 189, isomiR sequence 2 of SEQ ID NO: 189, isomiR sequence 1 of SEQ ID NO: 190, isomiR sequence 1 of SEQ ID NO: 191, isomiR sequence 2 of SEQ ID NO: 191, isomiR sequence 1 of SEQ ID NO: 192, isomiR sequence 1 of SEQ ID NO: 194, isomiR sequence 2 of SEQ ID NO: 194, isomiR sequence 1 of SEQ ID NO: 195, isomiR sequence 2 of SEQ ID NO: 195, isomiR sequence 1 of SEQ ID NO: 199, isomiR sequence 2 of SEQ ID NO: 199, isomiR sequence 1 of SEQ ID NO: 202, isomiR sequence 2 of SEQ ID NO: 202, isomiR sequence 1 of SEQ ID NO: 203, isomiR sequence 1 of SEQ ID NO: 205, isomiR sequence 2 of SEQ ID NO: 205, isomiR sequence 1 of SEQ ID NO: 206, isomiR sequence 2 of SEQ ID NO: 206, isomiR sequence 1 of SEQ ID NO: 208, isomiR sequence 2 of SEQ ID NO: 208, isomiR sequence 1 of SEQ ID NO: 209, isomiR sequence 2 of SEQ ID NO: 209, isomiR sequence 1 of SEQ ID NO: 213, isomiR sequence 2 of SEQ ID NO: 213, isomiR sequence 1 of SEQ ID NO: 214, isomiR sequence 2 of SEQ ID NO: 214, isomiR sequence 1 of SEQ ID NO: 215, isomiR sequence 2 of SEQ ID NO: 215, isomiR sequence 1 of SEQ ID NO: 216, isomiR sequence 2 of SEQ ID NO: 216, isomiR sequence 1 of SEQ ID NO: 217, isomiR sequence 2 of SEQ ID NO: 217, isomiR sequence 1 of SEQ ID NO: 218, isomiR sequence 2 of SEQ ID NO: 218, isomiR sequence 1 of SEQ ID NO: 219, isomiR sequence 2 of SEQ ID NO: 219, isomiR sequence 1 of SEQ ID NO: 220, isomiR sequence 2 of SEQ ID NO: 220, isomiR sequence 1 of SEQ ID NO: 221, isomiR sequence 2 of SEQ ID NO: 221, isomiR sequence 1 of SEQ ID NO: 222, isomiR sequence 2 of SEQ ID NO: 222, isomiR sequence 1 of SEQ ID NO: 223, isomiR sequence 2 of SEQ ID NO: 223, isomiR sequence 1 of SEQ ID NO: 224, isomiR sequence 2 of SEQ ID NO: 224, isomiR sequence 1 of SEQ ID NO: 225, isomiR sequence 2 of SEQ ID NO: 225, isomiR sequence 1 of SEQ ID NO: 226, isomiR sequence 2 of SEQ ID NO: 226, isomiR sequence 1 of SEQ ID NO: 227, isomiR sequence 2 of SEQ ID NO: 227, isomiR sequence 1 of SEQ ID NO: 228, isomiR sequence 2 of SEQ ID NO: 228, isomiR sequence 1 of SEQ ID NO: 229, isomiR sequence 2 of SEQ ID NO: 229, isomiR sequence 1 of SEQ ID NO: 230, isomiR sequence 1 of SEQ ID NO: 231, isomiR sequence 2 of SEQ ID NO: 231, isomiR sequence 1 of SEQ ID NO: 232, isomiR sequence 2 of SEQ ID NO: 232, isomiR sequence 1 of SEQ ID NO: 233, isomiR sequence 2 of SEQ ID NO: 233, isomiR sequence 1 of SEQ ID NO: 234, isomiR sequence 2 of SEQ ID NO: 234, isomiR sequence 1 of SEQ ID NO: 235, isomiR sequence 2 of SEQ ID NO: 235, isomiR sequence 1 of SEQ ID NO: 236, isomiR sequence 2 of SEQ ID NO: 236, isomiR sequence 1 of SEQ ID NO: 237, isomiR sequence 2 of SEQ ID NO: 237, isomiR sequence 1 of SEQ ID NO: 238, isomiR sequence 2 of SEQ ID NO: 238, isomiR sequence 1 of SEQ ID NO: 239, isomiR sequence 2 of SEQ ID NO: 239, isomiR sequence 1 of SEQ ID NO: 241, isomiR sequence 2 of SEQ ID NO: 241, isomiR sequence 1 of SEQ ID NO: 242, isomiR sequence 2 of SEQ ID NO: 242, isomiR sequence 1 of SEQ ID NO: 243, isomiR sequence 2 of SEQ ID NO: 243, isomiR sequence 1 of SEQ ID NO: 244, isomiR sequence 2 of SEQ ID NO: 244, isomiR sequence 1 of SEQ ID NO: 245, isomiR sequence 2 of SEQ ID NO: 245, isomiR sequence 1 of SEQ ID NO: 246, isomiR sequence 2 of SEQ ID NO: 246, isomiR sequence 1 of SEQ ID NO: 247, isomiR sequence 2 of SEQ ID NO: 247, isomiR sequence 1 of SEQ ID NO: 248, isomiR sequence 2 of SEQ ID NO: 248, isomiR sequence 1 of SEQ ID NO: 249, isomiR sequence 2 of SEQ ID NO: 249, isomiR sequence 1 of SEQ ID NO: 251, isomiR sequence 2 of SEQ ID NO: 251, isomiR sequence 1 of SEQ ID NO: 252, isomiR sequence 2 of SEQ ID NO: 252, isomiR sequence 1 of SEQ ID NO: 253, isomiR sequence 2 of SEQ ID NO: 253, isomiR sequence 1 of SEQ ID NO: 255, isomiR sequence 1 of SEQ ID NO: 256, isomiR sequence 2 of SEQ ID NO: 256, isomiR sequence 1 of SEQ ID NO: 257, isomiR sequence 2 of SEQ ID NO: 257, isomiR sequence 1 of SEQ ID NO: 259, isomiR sequence 2 of SEQ ID NO: 259, isomiR sequence 1 of SEQ ID NO: 260, isomiR sequence 2 of SEQ ID NO: 260, isomiR sequence 1 of SEQ ID NO: 261, isomiR sequence 2 of SEQ ID NO: 261, isomiR sequence 1 of SEQ ID NO: 262, isomiR sequence 2 of SEQ ID NO: 262, isomiR sequence 1 of SEQ ID NO: 263, isomiR sequence 2 of SEQ ID NO: 263, isomiR sequence 1 of SEQ ID NO: 264, isomiR sequence 2 of SEQ ID NO: 264, isomiR sequence 1 of SEQ ID NO: 265, isomiR sequence 1 of SEQ ID NO: 268, isomiR sequence 1 of SEQ ID NO: 269, isomiR sequence 2 of SEQ ID NO: 269, isomiR sequence 1 of SEQ ID NO: 270, isomiR sequence 2 of SEQ ID NO: 270, isomiR sequence 1 of SEQ ID NO: 271, isomiR sequence 2 of SEQ ID NO: 271, isomiR sequence 1 of SEQ ID NO: 272, isomiR sequence 2 of SEQ ID NO: 272, isomiR sequence 1 of SEQ ID NO: 273, isomiR sequence 2 of SEQ ID NO: 273, isomiR sequence 1 of SEQ ID NO: 274, isomiR sequence 2 of SEQ ID NO: 274, isomiR sequence 1 of SEQ ID NO: 275, isomiR sequence 2 of SEQ ID NO: 275, isomiR sequence 1 of SEQ ID NO: 276, isomiR sequence 1 of SEQ ID NO: 277, isomiR sequence 2 of SEQ ID NO: 277, isomiR sequence 1 of SEQ ID NO: 278, isomiR sequence 2 of SEQ ID NO: 278, isomiR sequence 1 of SEQ ID NO: 279, isomiR sequence 1 of SEQ ID NO: 280, isomiR sequence 2 of SEQ ID NO: 280, isomiR sequence 1 of SEQ ID NO: 281, isomiR sequence 2 of SEQ ID NO: 281, isomiR sequence 1 of SEQ ID NO: 282, isomiR sequence 2 of SEQ ID NO: 282, isomiR sequence 1 of SEQ ID NO: 283, isomiR sequence 2 of SEQ ID NO: 283, isomiR sequence 1 of SEQ ID NO: 284, isomiR sequence 2 of SEQ ID NO: 284, isomiR sequence 1 of SEQ ID NO: 286, isomiR sequence 2 of SEQ ID NO: 286, isomiR sequence 1 of SEQ ID NO: 287, isomiR sequence 2 of SEQ ID NO: 287, isomiR sequence 1 of SEQ ID NO: 288, isomiR sequence 2 of SEQ ID NO: 288, isomiR sequence 1 of SEQ ID NO: 289, isomiR sequence 2 of SEQ ID NO: 289, isomiR sequence 1 of SEQ ID NO: 290, isomiR sequence 1 of SEQ ID NO: 291, isomiR sequence 2 of SEQ ID NO: 291, isomiR sequence 1 of SEQ ID NO: 292, isomiR sequence 1 of SEQ ID NO: 293, isomiR sequence 2 of SEQ ID NO: 293, isomiR sequence 1 of SEQ ID NO: 295, isomiR sequence 2 of SEQ ID NO: 295, isomiR sequence 1 of SEQ ID NO: 296, isomiR sequence 2 of SEQ ID NO: 296, isomiR sequence 1 of SEQ ID NO: 297, isomiR sequence 2 of SEQ ID NO: 297, isomiR sequence 1 of SEQ ID NO: 298, isomiR sequence 1 of SEQ ID NO: 299, isomiR sequence 2 of SEQ ID NO: 299, isomiR sequence 1 of SEQ ID NO: 300, isomiR sequence 2 of SEQ ID NO: 300, isomiR sequence 1 of SEQ ID NO: 301, isomiR sequence 1 of SEQ ID NO: 302, isomiR sequence 2 of SEQ ID NO: 302, isomiR sequence 1 of SEQ ID NO: 303, isomiR sequence 1 of SEQ ID NO: 304, isomiR sequence 2 of SEQ ID NO: 304, isomiR sequence 1 of SEQ ID NO: 305, isomiR sequence 2 of SEQ ID NO: 305, isomiR sequence 1 of SEQ ID NO: 306, isomiR sequence 2 of SEQ ID NO: 306, isomiR sequence 1 of SEQ ID NO: 307, isomiR sequence 1 of SEQ ID NO: 308, isomiR sequence 2 of SEQ ID NO: 308, isomiR sequence 1 of SEQ ID NO: 309, isomiR sequence 1 of SEQ ID NO: 310, isomiR sequence 1 of SEQ ID NO: 311, isomiR sequence 2 of SEQ ID NO: 311, isomiR sequence 1 of SEQ ID NO: 312, isomiR sequence 1 of SEQ ID NO: 313, isomiR sequence 2 of SEQ ID NO: 313, isomiR sequence 1 of SEQ ID NO: 314, isomiR sequence 2 of SEQ ID NO: 314, isomiR sequence 1 of SEQ ID NO: 315, isomiR sequence 1 of SEQ ID NO: 316, isomiR sequence 2 of SEQ ID NO: 316, isomiR sequence 1 of SEQ ID NO: 317, isomiR sequence 2 of SEQ ID NO: 317, isomiR sequence 1 of SEQ ID NO: 320, isomiR sequence 2 of SEQ ID NO: 320, isomiR sequence 1 of SEQ ID NO: 321, isomiR sequence 2 of SEQ ID NO: 321, isomiR sequence 1 of SEQ ID NO: 322, isomiR sequence 2 of SEQ ID NO: 322, isomiR sequence 1 of SEQ ID NO: 323, isomiR sequence 2 of SEQ ID NO: 323, isomiR sequence 1 of SEQ ID NO: 324, isomiR sequence 2 of SEQ ID NO: 324, isomiR sequence 1 of SEQ ID NO: 325, isomiR sequence 2 of SEQ ID NO: 325, isomiR sequence 1 of SEQ ID NO: 326, isomiR sequence 2 of SEQ ID NO: 326, isomiR sequence 1 of SEQ ID NO: 327, isomiR sequence 1 of SEQ ID NO: 328, isomiR sequence 2 of SEQ ID NO: 328, isomiR sequence 1 of SEQ ID NO: 329, isomiR sequence 2 of SEQ ID NO: 329, isomiR sequence 1 of SEQ ID NO: 330, isomiR sequence 2 of SEQ ID NO: 330, isomiR sequence 1 of SEQ ID NO: 331, isomiR sequence 1 of SEQ ID NO: 332, isomiR sequence 1 of SEQ ID NO: 333, isomiR sequence 2 of SEQ ID NO: 333, isomiR sequence 1 of SEQ ID NO: 335, isomiR sequence 2 of SEQ ID NO: 335, isomiR sequence 1 of SEQ ID NO: 338, isomiR sequence 2 of SEQ ID NO: 338, isomiR sequence 1 of SEQ ID NO: 340, isomiR sequence 2 of SEQ ID NO: 340, isomiR sequence 1 of SEQ ID NO: 341, isomiR sequence 2 of SEQ ID NO: 341, isomiR sequence 1 of SEQ ID NO: 342, isomiR sequence 2 of SEQ ID NO: 342, isomiR sequence 1 of SEQ ID NO: 343, isomiR sequence 2 of SEQ ID NO: 343, isomiR sequence 1 of SEQ ID NO: 345, isomiR sequence 2 of SEQ ID NO: 345, isomiR sequence 1 of SEQ ID NO: 347, isomiR sequence 2 of SEQ ID NO: 347, isomiR sequence 1 of SEQ ID NO: 348, isomiR sequence 2 of SEQ ID NO: 348, isomiR sequence 1 of SEQ ID NO: 349, isomiR sequence 2 of SEQ ID NO: 349, isomiR sequence 1 of SEQ ID NO: 350, isomiR sequence 1 of SEQ ID NO: 352, isomiR sequence 2 of SEQ ID NO: 352, isomiR sequence 1 of SEQ ID NO: 353, isomiR sequence 2 of SEQ ID NO: 353, isomiR sequence 1 of SEQ ID NO: 354, isomiR sequence 1 of SEQ ID NO: 355, isomiR sequence 2 of SEQ ID NO: 355, isomiR sequence 1 of SEQ ID NO: 356, isomiR sequence 2 of SEQ ID NO: 356, isomiR sequence 1 of SEQ ID NO: 357, isomiR sequence 2 of SEQ ID NO: 357, isomiR sequence 1 of SEQ ID NO: 359, isomiR sequence 2 of SEQ ID NO: 359, isomiR sequence 1 of SEQ ID NO: 361, isomiR sequence 2 of SEQ ID NO: 361, isomiR sequence 1 of SEQ ID NO: 362, isomiR sequence 1 of SEQ ID NO: 363, isomiR sequence 2 of SEQ ID NO: 363, isomiR sequence 1 of SEQ ID NO: 367, isomiR sequence 2 of SEQ ID NO: 367, isomiR sequence 1 of SEQ ID NO: 368, isomiR sequence 2 of SEQ ID NO: 368, isomiR sequence 1 of SEQ ID NO: 369, isomiR sequence 2 of SEQ ID NO: 369, isomiR sequence 1 of SEQ ID NO: 371, isomiR sequence 2 of SEQ ID NO: 371, isomiR sequence 1 of SEQ ID NO: 372, isomiR sequence 2 of SEQ ID NO: 372, isomiR sequence 1 of SEQ ID NO: 373, isomiR sequence 1 of SEQ ID NO: 374, isomiR sequence 2 of SEQ ID NO: 374, isomiR sequence 1 of SEQ ID NO: 375, isomiR sequence 2 of SEQ ID NO: 375, isomiR sequence 1 of SEQ ID NO: 376, isomiR sequence 2 of SEQ ID NO: 376, isomiR sequence 1 of SEQ ID NO: 377, isomiR sequence 2 of SEQ ID NO: 377, isomiR sequence 1 of SEQ ID NO: 378, isomiR sequence 2 of SEQ ID NO: 378, isomiR sequence 1 of SEQ ID NO: 379, isomiR sequence 2 of SEQ ID NO: 379, isomiR sequence 1 of SEQ ID NO: 380, isomiR sequence 2 of SEQ ID NO: 380, isomiR sequence 1 of SEQ ID NO: 381, isomiR sequence 2 of SEQ ID NO: 381, isomiR sequence 1 of SEQ ID NO: 382, isomiR sequence 2 of SEQ ID NO: 382, isomiR sequence 1 of SEQ ID NO: 383, isomiR sequence 2 of SEQ ID NO: 383, isomiR sequence 1 of SEQ ID NO: 385, isomiR sequence 2 of SEQ ID NO: 385, isomiR sequence 1 of SEQ ID NO: 386, isomiR sequence 2 of SEQ ID NO: 386, isomiR sequence 1 of SEQ ID NO: 388, isomiR sequence 2 of SEQ ID NO: 388, isomiR sequence 1 of SEQ ID NO: 389, isomiR sequence 2 of SEQ ID NO: 389, isomiR sequence 1 of SEQ ID NO: 390, isomiR sequence 2 of SEQ ID NO: 390, isomiR sequence 1 of SEQ ID NO: 1435, isomiR sequence 2 of SEQ ID NO: 1435, isomiR sequence 1 of SEQ ID NO: 1436, isomiR sequence 2 of SEQ ID NO: 1436, isomiR sequence 1 of SEQ ID NO: 1438, isomiR sequence 2 of SEQ ID NO: 1438, isomiR sequence 1 of SEQ ID NO: 1439, isomiR sequence 2 of SEQ ID NO: 1439, isomiR sequence 1 of SEQ ID NO: 1440, isomiR sequence 2 of SEQ ID NO: 1440, isomiR sequence 1 of SEQ ID NO: 1441, isomiR sequence 2 of SEQ ID NO: 1441, isomiR sequence 1 of SEQ ID NO: 1446, isomiR sequence 2 of SEQ ID NO: 1446, isomiR sequence 1 of SEQ ID NO: 1448, isomiR sequence 2 of SEQ ID NO: 1448, isomiR sequence 1 of SEQ ID NO: 1449, isomiR sequence 2 of SEQ ID NO: 1449, isomiR sequence 1 of SEQ ID NO: 1451, isomiR sequence 2 of SEQ ID NO: 1451, isomiR sequence 1 of SEQ ID NO: 1452, isomiR sequence 2 of SEQ ID NO: 1452, isomiR sequence 1 of SEQ ID NO: 1455, isomiR sequence 1 of SEQ ID NO: 1458, isomiR sequence 2 of SEQ ID NO: 1458, isomiR sequence 1 of SEQ ID NO: 1460, isomiR sequence 2 of SEQ ID NO: 1460, isomiR sequence 1 of SEQ ID NO: 1461, isomiR sequence 2 of SEQ ID NO: 1461, isomiR sequence 1 of SEQ ID NO: 1462, isomiR sequence 2 of SEQ ID NO: 1462, isomiR sequence 1 of SEQ ID NO: 1463, and isomiR sequence 2 of SEQ ID NO: 1463, which are represented by SEQ ID NOs: 391 to 1314 and 1454 to 1472. Therefore, a polynucleotide that can be used as a nucleic acid probe or a primer in the present invention can be produced via cloning the gene.

Furthermore, the followings are also known, and methods for obtaining them are also known, as described above: human-derived hsa-miR-6765-3p, hsa-miR-6784-5p, hsa-miR-6778-5p, hsa-miR-6875-5p, hsa-miR-4534, hsa-miR-4721, hsa-miR-6756-5p, hsa-miR-615-5p, hsa-miR-6727-5p, hsa-miR-6887-5p, hsa-miR-8063, hsa-miR-6880-5p, hsa-miR-6805-3p, hsa-miR-4726-5p, hsa-miR-4710, hsa-miR-7111-5p, hsa-miR-3619-3p, hsa-miR-6795-5p, hsa-miR-1254, hsa-miR-1233-5p, hsa-miR-6836-3p, hsa-miR-6769a-5p, hsa-miR-4532, hsa-mir-365a-5p, hsa-miR-1231, hsa-miR-1228-5p, hsa-miR-4430, hsa-miR-296-3p, hsa-miR-1237-5p, hsa-miR-4466, hsa-miR-6789-5p, hsa-miR-4632-5p, hsa-miR-4745-5p, hsa-miR-4665-5p, hsa-miR-6807-5p, hsa-miR-7114-5p, hsa-miR-150-3p, hsa-miR-423-5p, hsa-miR-575, hsa-miR-671-5p, hsa-miR-939-5p, hsa-miR-3665, hsa-mir-6784, hsa-mir-6778, hsa-mir-6875, hsa-mir-4534, hsa-mir-4721, hsa-mir-6756, hsa-mir-615, hsa-mir-6727, hsa-mir-6887, hsa-mir-8063, hsa-mir-6880, hsa-mir-6805, hsa-mir-4726, hsa-mir-4710, hsa-mir-7111, hsa-mir-3619, hsa-mir-6795, hsa-mir-1254-1, hsa-mir-1254-2, hsa-mir-1233-1, hsa-mir-1233-2, hsa-mir-6836, hsa-mir-6769a, hsa-mir-4532, hsa-mir-365a, hsa-mir-1231, hsa-mir-1228, hsa-mir-4430, hsa-mir-296, hsa-mir-1237, hsa-mir-4466, hsa-mir-6789, hsa-mir-4632, hsa-mir-4745, hsa-mir-4665, hsa-mir-6807, hsa-mir-7114, hsa-mir-150, hsa-mir-423, hsa-mir-575, hsa-mir-671, hsa-mir-939, hsa-mir-3665, isomiR example-1 of SEQ ID NO: 1320, isomiR example-1 of SEQ ID NO: 1322, isomiR example-2 of SEQ ID NO: 1322, isomiR example-1 of SEQ ID NO: 1328, isomiR example-2 of SEQ ID NO: 1328, isomiR example-2 of SEQ ID NO: 1329, isomiR example-1 of SEQ ID NO: 1333, isomiR example-2 of SEQ ID NO: 1333, isomiR example-1 of SEQ ID NO: 1334, isomiR example-2 of SEQ ID NO: 1334, isomiR example-1 of SEQ ID NO: 1337, isomiR example-2 of SEQ ID NO: 1337, isomiR example-1 of SEQ ID NO: 1338, isomiR example-2 of SEQ ID NO: 1338, isomiR example-1 of SEQ ID NO: 1340, isomiR example-2 of SEQ ID NO: 1340, isomiR example-1 of SEQ ID NO: 1341, isomiR example-2 of SEQ ID NO: 1341, isomiR example-2 of SEQ ID NO: 1342, isomiR example-2 of SEQ ID NO: 1343, isomiR example-1 of SEQ ID NO: 1344, isomiR example-2 of SEQ ID NO: 1344, isomiR example-2 of SEQ ID NO: 1346, isomiR example-2 of SEQ ID NO: 1347, isomiR example-2 of SEQ ID NO: 1348, isomiR example-1 of SEQ ID NO: 1351, isomiR example-2 of SEQ ID NO: 1351, isomiR example-1 of SEQ ID NO: 1352, isomiR example-2 of SEQ ID NO: 1352, isomiR example-1 of SEQ ID NO: 1354, isomiR example-2 of SEQ ID NO: 1354, isomiR example-1 of SEQ ID NO: 1355, isomiR example-2 of SEQ ID NO: 1355, isomiR example-1 of SEQ ID NO: 1356, and isomiR example-2 of SEQ ID NO: 1356, which are represented by SEQ ID NOs: 1351 to 1434. Therefore, a polynucleotide that can be used as a nucleic acid probe or a primer in the present invention can be produced via cloning the gene.

Such a nucleic acid probe(s) or primer(s) may be chemically synthesized using an automated DNA synthesizer. A phosphoramidite process is commonly used for this synthesis, and this process can be used to automatically synthesize a single-stranded DNA with up to about 100 nucleotides in length. The automated DNA synthesizer is commercially available from, for instance, Polygen, Inc., ABI, Inc., or Applied BioSystems, Inc.

Alternatively, a polynucleotide(s) of the present invention may be prepared by cDNA cloning. For the cDNA cloning technology, a microRNA Cloning Kit Wako, for instance, can be utilized.

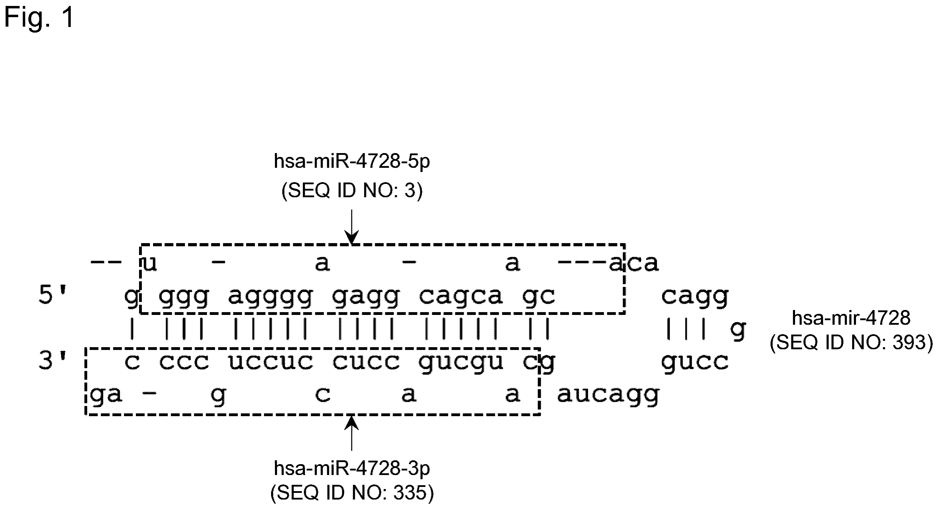

The sequence of a nucleic acid probe or primer for detection of a polynucleotide consisting of a nucleotide sequence represented by any of SEQ ID NOs: 1 to 210, 211 to 249, 250 to 374, 375 to 390, 1435 to 1448, and 1449 to 1453 is not present *in vivo* as an miRNA or any precursor thereof. For instance, the nucleotide sequences represented by SEQ ID NO: 3 and miR-4728-5p are generated from the precursor represented by SEQ ID NO: 393. This precursor has a hairpin-like structure as shown in Fig. 1, and the nucleotide sequences represented by SEQ ID NO: 3 and miR-4728-5p have mismatch nucleotides therebetween. Accordingly, a nucleotide sequence perfectly complementary to the nucleotide sequence represented by SEQ ID NO: 3 or miR-4728-5p is not naturally occurring *in vivo*. Thus, the nucleic acid probe(s) or primer(s) for detecting a nucleotide sequence represented by any of SEQ ID NOs: 1 to 210, 211 to 249, 250 to 374, 375 to 390, 1435 to 1448, and 1449 to 1453 may have an artificial nucleotide sequence not present *in vivo*.

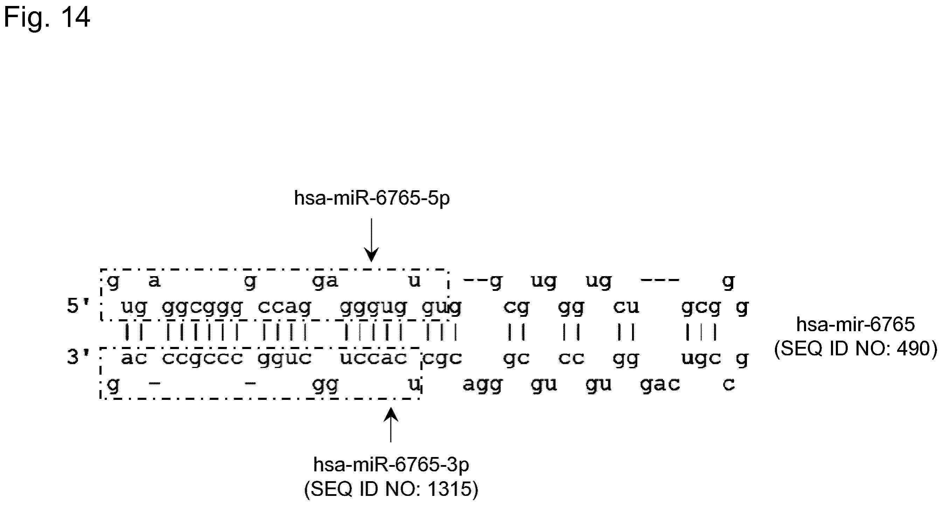

In addition, for instance, the nucleotide sequences represented by SEQ ID NO: 1315 and miR-6765-5p are generated from the precursor represented by SEQ ID NO: 490. This precursor has a hairpin-like structure as shown in Fig. 14, and the nucleotide sequences represented by SEQ ID NO: 1315 and miR-6765-5p have mismatch nucleotides therebetween. Accordingly, a nucleotide sequence perfectly complementary to the nucleotide sequence represented by SEQ ID NO: 1315 or miR-6765-5p is not naturally occurring *in vivo*. Thus, the nucleic acid probe(s) or primer(s) for detecting a nucleotide sequence represented by any of SEQ ID NOs: 194 to 210, 374, and 1315 to 1350 and 211 to 212, and 1351 to 1356 may have an artificial nucleotide sequence not present *in vivo*.

3. Kit or Device for Detection of Dementia

The present invention provides a kit or device for detection of dementia, comprising one or more polynucleotide(s) (which may include any variant(s), fragment(s), or derivative(s)) capable of being used as a nucleic acid probe(s) or primer(s) for measuring a target nucleic acid(s) as a dementia marker(s) in the present invention.

A target nucleic acid(s) as a dementia marker(s) according to the present invention is preferably at least one selected from the following group A.

Group A:

miR-4274, miR-4272, miR-4728-5p, miR-4443, miR-4506, miR-6773-5p, miR-4662a-5p, miR-3184-3p, miR-4281, miR-320d, miR-6729-3p, miR-5192, miR-6853-5p, miR-1234-3p, miR-1233-3p, miR-4539, miR-3914, miR-4738-5p, miR-548au-3p, miR-1539, miR-4720-3p, miR-365b-5p, miR-4486, miR-1227-5p, miR-4667-5p, miR-6088, miR-6820-5p, miR-4505, miR-548q, miR-4658, miR-450a-5p, miR-1260b, miR-3677-5p, miR-6777-3p, miR-6826-3p, miR-6832-3p, miR-4725-3p, miR-7161-3p, miR-2277-5p, miR-7110-3p, miR-4312, miR-4461, miR-6766-5p, miR-1266-3p, miR-6729-5p, miR-526b-3p, miR-519e-5p, miR-512-5p, miR-5088-5p, miR-1909-3p, miR-6511a-5p, miR-4734, miR-936, miR-1249-3p, miR-6777-5p, miR-4487, miR-3155a, miR-563, miR-4741, miR-6788-5p, miR-4433b-5p, miR-323a-5p, miR-6811-5p, miR-6721-5p, miR-5004-5p, miR-6509-3p, miR-648, miR-3917, miR-6087, miR-1470, miR-586, miR-3150a-5p, miR-105-3p, miR-7973, miR-1914-5p, miR-4749-3p, miR-15b-5p, miR-1289, miR-4433a-5p, miR-3666, miR-3186-3p, miR-4725-5p, miR-4488, miR-4474-3p, miR-6731-3p, miR-4640-3p, miR-202-5p, miR-6816-5p, miR-638, miR-6821-5p, miR-1247-3p, miR-6765-5p, miR-6800-5p, miR-3928-3p, miR-3940-5p, miR-3960, miR-6775-5p, miR-3178, miR-1202, miR-6790-5p, miR-4731-3p, miR-2681-3p, miR-6758-5p, miR-8072, miR-518d-3p, miR-3606-3p, miR-4800-5p, miR-1292-3p, miR-6784-3p, miR-4450, miR-6132, miR-4716-5p, miR-6860, miR-1268b, miR-378d, miR-4701-5p, miR-4329, miR-185-3p, miR-552-3p, miR-1273g-5p, miR-6769b-3p, miR-520a-3p, miR-4524b-5p, miR-4291, miR-6734-3p, miR-143-5p, miR-939-3p, miR-6889-3p, miR-6842-3p, miR-4511, miR-4318, miR-4653-5p, miR-6867-3p, miR-133b, miR-3196, miR-193b-3p, miR-3162-3p, miR-6819-3p, miR-1908-3p, miR-6786-5p, miR-3648, miR-4513, miR-3652, miR-4640-5p, miR-6871-5p, miR-7845-5p, miR-3138, miR-6884-5p, miR-4653-3p, miR-636, miR-4652-3p, miR-6823-5p, miR-4502, miR-7113-5p, miR-8087, miR-7154-3p, miR-5189-5p, miR-1253, miR-518c-5p, miR-7151-5p, miR-3614-3p, miR-4727-5p, miR-3682-5p, miR-5090, miR-337-3p, miR-488-5p, miR-100-5p, miR-4520-3p, miR-373-3p, miR-6499-5p, miR-3909, miR-32-5p, miR-302a-3p, miR-4686, miR-4659a-3p, miR-4287, miR-1301-5p, miR-593-3p, miR-517a-3p, miR-517b-3p, miR-142-3p, miR-1185-2-3p, miR-602, miR-527, miR-518a-5p, miR-4682, miR-28-5p, miR-4252, miR-452-5p, miR-525-5p, miR-3622a-3p, miR-6813-3p, miR-4769-3p, miR-5698, miR-1915-3p, miR-1343-5p, miR-6861-5p, miR-6781-5p, miR-4508, miR-6743-5p, miR-6726-5p, miR-4525, miR-4651, miR-6813-5p, miR-5787, miR-1290, miR-6075, miR-4758-5p, miR-4690-5p, miR-762, miR-371a-5p, miR-6765-3p, miR-6784-5p, miR-6778-5p, miR-6875-5p, miR-4534, miR-4721, miR-6756-5p, miR-615-5p, miR-6727-5p, miR-6887-5p, miR-8063, miR-6880-5p, miR-6805-3p, miR-4726-5p, miR-4710, miR-7111-5p, miR-3619-3p, miR-6795-5p, miR-1254, miR-1233-5p, miR-6836-3p, miR-6769a-5p, miR-4532, miR-365a-5p, miR-1231, miR-1228-5p, miR-4430, miR-296-3p, miR-1237-5p, miR-4466, miR-6789-5p, miR-4632-5p, miR-4745-5p, miR-4665-5p, miR-6807-5p and miR-7114-5p, miR-516a-5p, miR-769-3p, miR-3692-5p, miR-3945, miR-4433a-3p, miR-4485-3p, miR-6831-5p, miR-519c-5p, miR-551b-5p, miR-1343-3p, miR-4286, miR-4634, miR-4733-3p, and miR-6086.

An additional target nucleic acid(s) capable of being optionally used for the measurement is preferably selected from the following group B, C, or D.

Group B:

miR-1225-3p, miR-3184-5p, miR-665, miR-211-5p, miR-1247-5p, miR-3656, miR-149-5p, miR-744-5p, miR-345-5p, miR-150-5p, miR-191-3p, miR-651-5p, miR-34a-5p, miR-409-5p, miR-369-5p, miR-1915-5p, miR-204-5p, miR-137, miR-382-5p, miR-517-5p, miR-532-5p, miR-22-5p, miR-1237-3p, miR-1224-3p, miR-625-3p, miR-328-3p, miR-122-5p, miR-202-3p, miR-4781-5p, miR-718, miR-342-3p, miR-26b-3p, miR-140-3p, miR-200a-3p, miR-378a-3p, miR-484, miR-296-5p, miR-205-5p, miR-431-5p, miR-150-3p, miR-423-5p, miR-575, miR-671-5p, miR-939-5p and miR-3665, miR-30d-5p, miR-30b-3p, miR-92a-3p, miR-371b-5p, and miR-486-5p.

Group C:

miR-1471, miR-1538, miR-449b-3p, miR-1976, miR-4268, miR-4279, miR-3620-3p, miR-3944-3p, miR-3156-3p, miR-3187-5p, miR-4685-3p, miR-4695-3p, miR-4697-3p, miR-4713-5p, miR-4723-3p, miR-371b-3p, miR-3151-3p, miR-3192-3p, miR-6728-3p, miR-6736-3p, miR-6740-3p, miR-6741-3p, miR-6743-3p, miR-6747-3p, miR-6750-3p, miR-6754-3p, miR-6759-3p, miR-6761-3p, miR-6762-3p, miR-6769a-3p, miR-6776-3p, miR-6778-3p, miR-6779-3p, miR-6786-3p, miR-6787-3p, miR-6792-3p, miR-6794-3p, miR-6801-3p, miR-6802-3p, miR-6803-3p, miR-6804-3p, miR-6810-5p, miR-6823-3p, miR-6825-3p, miR-6829-3p, miR-6833-3p, miR-6834-3p, miR-6780b-3p, miR-6845-3p, miR-6862-3p, miR-6865-3p, miR-6870-3p, miR-6875-3p, miR-6877-3p, miR-6879-3p, miR-6882-3p, miR-6885-3p, miR-6886-3p, miR-6887-3p, miR-6890-3p, miR-6893-3p, miR-6894-3p, miR-7106-3p, miR-7109-3p, miR-7114-3p, miR-7155-5p, miR-7160-5p, miR-615-3p, miR-920, miR-1825, miR-675-3p, miR-1910-5p, miR-2278, miR-2682-3p, miR-3122, miR-3151-5p, miR-3175, miR-4323, miR-4326, miR-4284, miR-3605-3p, miR-3622b-5p, miR-3646, miR-3158-5p, miR-4722-3p, miR-4728-3p, miR-4747-3p, miR-4436b-5p, miR-5196-3p, miR-5589-5p, miR-345-3p, miR-642b-5p, miR-6716-3p, miR-6511b-3p, miR-208a-5p, miR-6726-3p, miR-6744-5p, miR-6782-3p, miR-6789-3p, miR-6797-3p, miR-6800-3p, miR-6806-5p, miR-6824-3p, miR-6837-5p, miR-6846-3p, miR-6858-3p, miR-6859-3p, miR-6861-3p, miR-6880-3p, miR-7111-3p, miR-7152-5p, miR-642a-5p, miR-657, miR-1236-3p, miR-764, miR-4314, miR-3675-3p, miR-5703, miR-3191-5p, miR-6511a-3p, miR-6809-3p, miR-6815-5p, miR-6857-3p, miR-6878-3p.

Group D:

miR-766-3p, miR-1229-3p, miR-1306-5p, miR-210-5p, miR-198, miR-485-3p, miR-668-3p, miR-532-3p, miR-877-3p, miR-1238-3p, miR-3130-5p, miR-4298, miR-4290, miR-3943, miR-346, miR-767-3p.

In one embodiment, a target nucleic acid(s) as a dementia marker(s) according to the present invention is at least one selected from the following group E.

Group E:

at least one polynucleotide selected from the group consisting of miR-4728-5p, miR-3184-3p, miR-1233-3p, miR-6088, miR-6777-3p, miR-7110-3p, miR-936, miR-6087, miR-1202, miR-4731-3p, miR-4800-5p, miR-6784-3p, miR-4716-5p, miR-6734-3p, miR-6889-3p, miR-6867-3p, miR-3622a-3p, miR-6813-3p, miR-4769-3p, miR-5698, miR-3184-5p, miR-1471, miR-1538, miR-449b-3p, miR-1976, miR-4268, miR-4279, miR-3620-3p, miR-3944-3p, miR-3156-3p, miR-3187-5p, miR-4685-3p, miR-4695-3p, miR-4697-3p, miR-4713-5p, miR-4723-3p, miR-371b-3p, miR-3151-3p, miR-3192-3p, miR-6728-3p, miR-6736-3p, miR-6740-3p, miR-6741-3p, miR-6743-3p, miR-6747-3p, miR-6750-3p, miR-6754-3p, miR-6759-3p, miR-6761-3p, miR-6762-3p, miR-6769a-3p, miR-6776-3p, miR-6778-3p, miR-6779-3p, miR-6786-3p, miR-6787-3p, miR-6792-3p, miR-6794-3p, miR-6801-3p, miR-6802-3p, miR-6803-3p, miR-6804-3p, miR-6810-5p, miR-6823-3p, miR-6825-3p, miR-6829-3p, miR-6833-3p, miR-6834-3p, miR-6780b-3p, miR-6845-3p, miR-6862-3p, miR-6865-3p, miR-6870-3p, miR-6875-3p, miR-6877-3p, miR-6879-3p, miR-6882-3p, miR-6885-3p, miR-6886-3p, miR-6887-3p, miR-6890-3p, miR-6893-3p, miR-6894-3p, miR-7106-3p, miR-7109-3p, miR-7114-3p, miR-7155-5p, miR-7160-5p, miR-615-3p, miR-920, miR-1825, miR-675-3p, miR-1910-5p, miR-2278, miR-2682-3p, miR-3122, miR-3151-5p, miR-3175, miR-4323, miR-4326, miR-4284, miR-3605-3p, miR-3622b-5p, miR-3646, miR-3158-5p, miR-4722-3p, miR-4728-3p, miR-4747-3p, miR-4436b-5p, miR-5196-3p, miR-5589-5p, miR-345-3p, miR-642b-5p, miR-6716-3p, miR-6511b-3p, miR-208a-5p, miR-6726-3p, miR-6744-5p, miR-6782-3p, miR-6789-3p, miR-6797-3p, miR-6800-3p, miR-6806-5p, miR-6824-3p, miR-6837-5p, miR-6846-3p, miR-6858-3p, miR-6859-3p, miR-6861-3p, miR-6880-3p, miR-7111-3p, miR-7152-5p, miR-642a-5p, miR-657, miR-1236-3p, miR-764, miR-4314, miR-3675-3p, miR-5703, miR-3191-5p, miR-6511a-3p, miR-6809-3p, miR-6815-5p, miR-6857-3p, miR-6878-3p, and miR-371a-5p.

The target nucleic acid(s) selected from group E may be used in combination with one or more target nucleic acids arbitrarily selected from the group A, B, and D.

The target nucleic acid(s) selected from group E can be used for prediction of dementia in addition to detection of dementia. The term "prediction of dementia" used herein means predicting the possibility that a subject without currently having dementia will have dementia in the future. The detection or prediction of dementia may be distinctively carried out using different thresholds (e.g., a lower threshold for prediction than for detection) or carried out by using the same threshold (such that a subject without dementia detected is determined to be likely to have dementia). The prediction of dementia is useful because it enables a prophylactic treatment for dementia to be implemented in a subject who has been determined to be likely to have dementia.

A kit or device of the present invention comprises a nucleic acid(s) capable of specifically binding to the above target nucleic acid(s) as dementia marker(s), preferably, at least one polynucleotide selected from the polynucleotides described in the above groups or a variant thereof.

Specifically, a kit or device of the present invention may comprise at least one of a polynucleotide comprising (or consisting of) a nucleotide sequence represented by any of SEQ ID NOs: 1 to 210, 211 to 249, 250 to 374, 375 to 390, 1315 to 1350, 1351 to 1356, 1435 to 1448, and 1449 to 1453 or a nucleotide sequence derived from the nucleotide sequence by the replacement of u with t or a polynucleotide comprising (consisting of) a complementary sequence thereof, or a polynucleotide hybridizing under stringent conditions to any of the polynucleotides, or a variant or fragment thereof comprising 15 or more consecutive nucleotides of any of the polynucleotide sequences.

A fragment(s) that can be comprised in a kit or device of the present invention may be, for instance, one or more polynucleotides and preferably at least two polynucleotides selected from the group consisting of the following (1) to (8):

(1) a polynucleotide comprising a sequence of 15 or more consecutive nucleotides in a nucleotide sequence derived from the nucleotide sequence represented by any of SEQ ID NOs: 1 to 210 by the replacement of u with t or in a complementary sequence thereof;
(2) a polynucleotide comprising a sequence of 15 or more consecutive nucleotides in a nucleotide sequence derived from the nucleotide sequence represented by any of SEQ ID NOs: 211 to 249 by the replacement of u with t or in a complementary sequence thereof;
(3) a polynucleotide comprising a sequence of 15 or more consecutive nucleotides in a nucleotide sequence derived from the nucleotide sequence represented by any of SEQ ID NOs: 250 to 374 by the replacement of u with t or in a complementary sequence thereof;
(4) a polynucleotide comprising a sequence of 15 or more consecutive nucleotides in a nucleotide sequence derived from the nucleotide sequence represented by any of SEQ ID NOs: 375 to 390 by the replacement of u with t or in a complementary sequence thereof;
(5) a polynucleotide comprising a sequence of 15 or more consecutive nucleotides in a nucleotide sequence derived from the nucleotide sequence represented by any of SEQ ID NOs: 391 to 782 by the replacement of u with t or in a complementary sequence thereof;
(6) a polynucleotide comprising a sequence of 15 or more consecutive nucleotides in a nucleotide sequence derived from the nucleotide sequence represented by any of SEQ ID NOs: 783 to 1314 by the replacement of u with t or in a complementary sequence thereof;
(7) a polynucleotide comprising a sequence of 15 or more consecutive nucleotides in a nucleotide sequence derived from the nucleotide sequence represented by any of SEQ ID NOs: 1315 to 1434 by the replacement of u with t or in a complementary sequence thereof; and
(8) a polynucleotide comprising a sequence of 15 or more consecutive nucleotides in a nucleotide sequence derived from the nucleotide sequence represented by any of SEQ ID NOs: 1434 to 1505 by the replacement of u with t or in a complementary sequence thereof.

In a preferred embodiment, the polynucleotide is a polynucleotide consisting of a nucleotide sequence represented by any of SEQ ID NOs: 1 to 210, 374, 1315 to 1350, and 1435 to 1448 or a nucleotide sequence derived from the nucleotide sequence by the replacement of u with t or a polynucleotide consisting of a complementary sequence thereof, or a polynucleotide hybridizing under stringent conditions to any of the polynucleotides, or a variant comprising a sequence of 15 or more, preferably 17 or more, and more preferably 19 or more consecutive nucleotides of any of the polynucleotides.

In addition, in a preferred embodiment, the polynucleotide is a polynucleotide consisting of a nucleotide sequence represented by any of SEQ ID NOs: 1 to 210, 211 to 249, 250 to 374, and 375 to 390, 391 to 782, 783 to 1314, 1315 to 1350, 1351 to 1356, 1435 to 1448, 1449 to 1453, and 1454 to 1505 or a nucleotide sequence derived from the nucleotide sequence by the replacement of u with t or a polynucleotide consisting of a complementary sequence thereof, or a polynucleotide hybridizing under stringent conditions to any of the polynucleotides, or a variant comprising 15 or more, preferably 17 or more, and more preferably 19 or more consecutive nucleotides of any of the polynucleotides.

In a preferred embodiment, the fragment may be a polynucleotide comprising 15 or more, preferably 17 or more, and more preferably 19 or more consecutive nucleotides.

In the present invention, the size of the polynucleotide fragment is represented by the number of nucleotides in a range of, for instance, from 15 to less than the total number of consecutive nucleotides in the nucleotide sequence of each polynucleotide, from 17 to less than the total number of nucleotides in the sequence, or from 19 to less than the total number of nucleotides in the sequence.

Specific examples of a combination of the above polynucleotides as target nucleic acids in a kit or device of the present invention include a combination of 1, 2, 3, 4, 5, 6, 7, 8, 9, 10, or more polynucleotides selected from polynucleotides consisting of nucleotide sequences represented by SEQ ID NOs: 1 to 210, 211 to 249, 250 to 374, and 375 to 390, 1435 to 1448, and 1449 to 1453 listed in Table 1-1 (provided that at least one selected from polynucleotides consisting of nucleotide sequences represented by SEQ ID NOs: 1 to 210 and 250 to 374 is included). This is just an example and all the other various possible combinations are included in the present invention.

As a combination of target nucleic acids in a kit or device for discriminating between dementia patients and non-dementia subjects in the present invention, for instance, it is desirable to combine two or more polynucleotides listed in Table 1-1 (provided that at least one selected from polynucleotides consisting of nucleotide sequences represented by SEQ ID NOs: 1 to 210 and 250 to 374 is included). Specific examples include any combination of at least 2 and preferably 3 to 110 polynucleotides selected from polynucleotides consisting of nucleotide sequences represented by SEQ ID NOs: 1 to 210, 211 to 249, 250 to 374, 375 to 390, 1315 to 1350, 1351 to 1356, 1435 to 1448, and 1449 to 1453, provided that at least one selected from polynucleotides consisting of nucleotide sequences represented by SEQ ID NOs: 1 to 210 is included.

The following shows, as non-limiting examples, combinations comprising the above polynucleotide(s) as a target nucleic acid(s). For instance, the target nucleic acids may be combined as follows.

As a combination of target nucleic acids in a kit or device for discriminating between Alzheimer's dementia patients and non-dementia subjects in the present invention, it is desirable to combine 2 or more and preferably 3 to 110 polynucleotides listed in Table 1-1 (provided that at least one selected from polynucleotides consisting of nucleotide sequences represented by SEQ ID NOs: 1 to 210 and 250 to 374 is included). Specific examples include any combination of 2 or more polynucleotides selected from polynucleotides consisting of nucleotide sequences represented by SEQ ID NOs: 1 to 23, 26, 34, 40, 47, 49, 54, 63, 72, 77, 78, 83, 87, 90, 91, 99, 101, 104, 133, 145, 146, 152 to 156, 191, 192 and 194, 200, 206, 212, 214, 215, 250 to 316, and 375 to 378. Preferred examples include any combination of 2 or more polynucleotides selected from polynucleotides consisting of nucleotide sequences represented by SEQ ID NOs: 1 to 13, 15, 16, 18 to 20, 23, 26, 49, 72, 77, 83, 101, 152, 153, 155, 156, 192 and 194, 200, 214, and 215.

In addition, as a combination of target nucleic acids in a kit or device for discriminating between vascular dementia patients and non-dementia subjects in the present invention, for instance, it is desirable to combine 2 or more and preferably 3 to 30 polynucleotides listed in Table 1-1 (provided that at least one selected from polynucleotides consisting of nucleotide sequences represented by SEQ ID NOs: 1 to 210 is included). Specific examples include any combination of 2 or more polynucleotides selected from polynucleotides consisting of nucleotide sequences represented by SEQ ID NOs: 3, 4, 8, 15, 16, 23 to 45, 59, 69, 79, 80, 83, 88, 89, 91, 99, 101, 109, 110 to 112, 125, 133, 140, 141, 191 to 193 and 194, 197, 199, 201, 203, 204, 206 to 209, 214, 216 to 218, 233, 252, 255, 258, 261 to 264, 266, 267, 271, 277, 278, 282, 284, 287, 289, 290, 293, 294, 297, 298, 301, 303, 306, 307, 310, 312 to 314, 320, 321, 323, 327, 329, 335, 336, 338, 342, 343, 345, 348, 349, 351, 352, 354, 359, 361 to 373, 375, 376, 380 to 383, 385, and 387 to 390. Preferred examples include any combination of 2 or more polynucleotides selected from polynucleotides consisting of nucleotide sequences represented by SEQ ID NOs: 3, 4, 8, 16, 23 to 30, 32, 34, 36, 37, 42, 43, 45, 79, 88, 89, 99, 111, 125 and 194, 197, 199, 201, 203, 204, 206, 207, 214, and 216 to 218.

In addition, as a combination of target nucleic acids in a kit or device for discriminating between Lewy body dementia patients and non-dementia subjects in the present invention, for instance, it is desirable to combine 2 or more and preferably 3 to 30 polynucleotides listed in Table 1-1 (provided that at least one selected from polynucleotides consisting of nucleotide sequences represented by SEQ ID NOs: 1 to 210 is included). Specific examples include any combination of 2 or more polynucleotides selected from polynucleotides consisting of nucleotide sequences represented by SEQ ID NOs: 1, 3, 4, 8, 11, 13, 15, 16, 26, 29, 30, 32 to 34, 36, 37, 45 to 68, 83, 87, 90 to 99, 101, 107, 109, 112 to 114, 116, 128, 133, 134, 157 to 159, 191, 192 and 194, 196, 197, 200, 204, 206, 207, 209, 210, 214, 216, 218 to 222, 234, 247, 250, 253 to 258, 261, 262, 264 to 267, 271, 277, 279, 282, 283, 284, 287, 289, 290, 293, 294, 298, 299, 301 to 303, 306, 308, 310, 312 to 314, 316 to 360, 374 to 377, and 379 to 388. Preferred examples include any combination of 2 or more polynucleotides selected from polynucleotides consisting of nucleotide sequences represented by SEQ ID NOs: 3, 4, 8, 11, 13, 16, 26, 29, 32, 36, 37, 45 to 49, 52 to 55, 60, 64, 68, 91, 116, 134 and 194, 197, 200, 207, 209, 214, 216, 219, 220, 221, and 247.

In addition, as a combination of target nucleic acids in a kit or device for discriminating between normal pressure hydrocephalus patients and non-dementia subjects in the present invention, for instance, it is desirable to combine 2 or more and preferably 3 to 30 polynucleotides listed in Table 1-1 (provided that at least one selected from polynucleotides consisting of nucleotide sequences represented by SEQ ID NOs: 1 to 210 is included). Specific examples include any combination of 2 or more polynucleotides selected from polynucleotides consisting of nucleotide sequences represented by SEQ ID NOs: 1, 3, 8, 10, 11, 16, 26, 30, 34, 37, 41, 43, 45, 49, 52 to 54, 60, 66, 75, 76, 83 to 86, 88, 94, 98, 99, 104, 116, 134, 136, 139 to 144, 150, 151, 176, 183 to 190 and 195, 200, 203, 205, 211, 212, 214, 215, 220, 225, 226, 230, 234, 240, 245, and 246. Preferred examples include any combination of 2 or more polynucleotides selected from polynucleotides consisting of nucleotide sequences represented by SEQ ID NOs: 1, 3, 8, 10, 11, 16, 26, 30, 34, 37, 41, 45, 49, 52, 54, 60, 66, 75, 76, 83 to 85, 88, 94, 104, 136, 140, 151, 176, 184 to 186 and 203, 211, 212, 220, 226, 230, 234, and 245.

In addition, as a combination of target nucleic acids in a kit or device for discriminating between frontotemporal lobar degeneration patients and non-dementia subjects in the present invention, for instance, it is desirable to combine 2 or more and preferably 3 to 30 polynucleotides listed in Table 1-1 (provided that at least one selected from polynucleotides consisting of nucleotide sequences represented by SEQ ID NOs: 1 to 210 is included). Specific examples include any combination of 2 or more polynucleotides selected from polynucleotides consisting of nucleotide sequences represented by SEQ ID NOs: 3, 10, 13, 24, 26, 29, 30, 33, 37, 43, 49, 53, 69 to 74, 81 to 83, 94, 98, 99, 115 to 117, 120, 121, 125, 128, 133, 135, 147 to 149, 153, 160 to 182, 191, 193 and 197, 200, 202, 213, 217, 223 to 225, 227, 228, 236, 238, 241 to 243, 248, and 249. Preferred examples include any combination of 2 or more polynucleotides selected from polynucleotides consisting of nucleotide sequences represented by SEQ ID NOs: 3, 10, 13, 24, 26, 29, 30, 33, 37, 43, 69 to 74, 82, 98, 99, 116, 117, 120, 121, 125, 147, 148, 161 to 163, 166, 167, 169, 175, 176 and 197, 200, 202, 223 to 225, 227, 236, 241, 242, 248, and 249.

In addition, as a combination of target nucleic acids in a kit or device for discriminating between Alzheimer's dementia patients and non-dementia subjects or for predicting Alzheimer's dementia patients in the present invention, for instance, it is desirable to combine SEQ ID NOs: 8, 15, 34, 40, 133, 191, 212, 250 to 316, and 375 to 378.

In addition, as a combination of target nucleic acids in a kit or device for discriminating between vascular dementia patients and non-dementia subjects in the present invention, for instance, it is desirable to combine SEQ ID NOs: 3, 8, 15, 26, 34, 69, 99, 101, 109, 112, 125, 133, 191 to 194, 252, 255, 258, 261 to 264, 266, 267, 271, 277, 278, 282, 284, 287, 289, 290, 293, 294, 297, 298, 301, 303, 306, 307, 310, 312 to 314, 320, 321, 323, 327, 329, 335, 336, 338, 342, 343, 345, 348, 349, 351, 352, 354, 359, 361 to 373 and 375, 376, 380 to 383, 385, and 387 to 390.

In addition, as a combination of target nucleic acids in a kit or device for discriminating between Lewy body dementia patients and non-dementia subjects in the present invention, for instance, it is desirable to combine SEQ ID NOs: 3, 8, 15, 26, 34, 53, 99, 101, 107, 109, 112, 128, 133, 191, 192, 194, 250, 253 to 258, 261, 262, 264 to 267, 271, 277, 279, 282 to 284, 287, 289, 290, 293, 294, 298, 299, 301 to 303, 306, 308, 310, 312 to 314, 316 to 360 and 374 to 377, and 379 to 388.

In one embodiment, a target nucleic acid(s) as a dementia marker(s) according to the present invention is preferably at least one selected from the following group A'.

Group A':

miR-5698, miR-1915-3p, miR-1343-5p, miR-6861-5p, miR-6781-5p, miR-4508, miR-6743-5p, miR-6726-5p, miR-4525, miR-4651, miR-6813-5p, miR-5787, miR-1290, miR-6075, miR-4758-5p, miR-4690-5p, miR-762, miR-371a-5p, miR-6765-3p, miR-6784-5p, miR-6778-5p, miR-6875-5p, miR-4534, miR-4721, miR-6756-5p, miR-615-5p, miR-6727-5p, miR-6887-5p, miR-8063, miR-6880-5p, miR-6805-3p, miR-4726-5p, miR-4710, miR-7111-5p, miR-3619-3p, miR-6795-5p, miR-1254, miR-1233-5p, miR-6836-3p, miR-6769a-5p, miR-4532, miR-365a-5p, miR-1231, miR-1228-5p, miR-4430, miR-296-3p, miR-1237-5p, miR-4466, miR-6789-5p, miR-4632-5p, miR-4745-5p, miR-4665-5p, miR-6807-5p, miR-7114-5p.

In one embodiment, an additional target nucleic acid(s) capable of being optionally used for the measurement is preferably selected from the following group B'.

Group B':

miR-1225-3p, miR-3184-5p, miR-150-3p, miR-423-5p, miR-575, miR-671-5p, miR-939-5p, miR-3665.

In one embodiment, a kit or device of the present invention comprises a nucleic acid(s) capable of specifically binding to the above target nucleic acids as dementia markers, preferably at least one polynucleotide selected from the polynucleotides described in the above two groups or a variant thereof.

In one embodiment, a kit or device of the present invention may comprise at least one of a polynucleotide comprising (or consisting of) a nucleotide sequence represented by any of SEQ ID NOs: 194 to 210, 374, and 1315 to 1350, and 211 to 212, and 1351 to 1356 or a nucleotide sequence derived from the nucleotide sequence by the replacement of u with t or a polynucleotide comprising (or consisting of) a complementary sequence thereof, or a polynucleotide hybridizing under stringent conditions to any of the polynucleotides, or a variant or fragment thereof comprising 15 or more consecutive nucleotides of any of the polynucleotide sequences.

In one embodiment, a fragment(s) that can be comprised in a kit or device of the present invention may be, for instance, one or more polynucleotides and preferably at least two polynucleotides selected from the group consisting of the following (1) to (4):

(1) a polynucleotide comprising a sequence of 15 or more consecutive nucleotides in a nucleotide sequence derived from the nucleotide sequence represented by any of SEQ ID NOs: 194 to 210, 374, and 1315 to 1350 by the replacement of u with t or in a complementary sequence thereof;

(2) a polynucleotide comprising a sequence of 15 or more consecutive nucleotides in a nucleotide sequence derived from the nucleotide sequence represented by any of SEQ ID NOs: 211 to 212 and 1351 to 1356 by the replacement of u with t or in a complementary sequence thereof;

(3) a polynucleotide comprising a sequence of 15 or more consecutive nucleotides in a nucleotide sequence derived from the nucleotide sequence represented by any of SEQ ID NOs: 398, 490, 591, 593 to 609, and 1357 to 1399 by the replacement of u with t or in a complementary sequence thereof; and

(4) a polynucleotide comprising a sequence of 15 or more consecutive nucleotides in a nucleotide sequence derived from the nucleotide sequence represented by any of SEQ ID NOs: 1015 to 1017, 1019 to 1024, 1026 to 1031, 1285 to 1286, and 1400 to 1434 by the replacement of u with t or in a complementary sequence thereof.

In a preferred embodiment, the polynucleotide is a polynucleotide consisting of a nucleotide sequence represented by any of SEQ ID NOs: 194 to 210, 374, and 1315 to 1350 or a nucleotide sequence derived from the nucleotide sequence by the replacement of u with t or a polynucleotide consisting of a complementary sequence thereof, or a polynucleotide hybridizing under stringent conditions to any of the polynucleotides, or a variant comprising a sequence of 15 or more, preferably 17 or more, and more preferably 19 or more consecutive nucleotides of any of the polynucleotides.

In addition, in a preferred embodiment, the polynucleotide is a polynucleotide consisting of a nucleotide sequence represented by any of SEQ ID NOs: 211 to 212 and 1351 to 1356, 398, 490, 591, 593 to 609, 1357 to 1399, 1015 to 1017, 1019 to 1024, 1026 to 1031, 1285 to 1286, and 1400 to 1434 or a nucleotide sequence derived from the nucleotide sequence by the replacement of u with t or a polynucleotide consisting of a complementary sequence thereof, or a polynucleotide hybridizing under stringent conditions to any of the polynucleotides, or a variant comprising 15 or more, preferably 17 or more, and more preferably 19 or more consecutive nucleotides of any of the polynucleotides.

In a preferred embodiment, the fragment may be a polynucleotide comprising 15 or more, preferably 17 or more, and more preferably 19 or more consecutive nucleotides.

In the present invention, the size of the polynucleotide fragment is represented by the number of nucleotides in a range of, for instance, from 15 to less than the total number of consecutive nucleotides in the nucleotide sequence of each polynucleotide, from 17 to less than the total number of nucleotides in the sequence, or from 19 to less than the total number of nucleotides in the sequence.

Specific examples of a combination of the above polynucleotides as target nucleic acids in a kit or device of the present invention include a combination of 1, 2, 3, 4, 5, 6, 7, 8, 9, 10, or more polynucleotides selected from polynucleotides consisting of nucleotide sequences represented by SEQ ID NOs: 194 to 210, 374, and 1315 to 1350, and 211 to 212, and 1351 to 1356 listed in Table 1-2 (provided that at least one selected from polynucleotides consisting of nucleotide sequences represented by SEQ ID NOs: 194 to 210, 374, and 1315 to 1350 is included). This is just an example and all the other various possible combinations are included in the present invention.

As a combination of target nucleic acids in a kit or device for discriminating between dementia patients and non-dementia subjects in the present invention, for instance, it is desirable to combine two or more polynucleotides listed in Table 1-2 (provided that at least one selected from polynucleotides consisting of nucleotide sequences represented by SEQ ID NOs: 194 to 210, 374, and 1315 to 1350 is included). Specific examples include any combination of at least 2, preferably 20 to 60, and more preferably 40 to 55 polynucleotides selected from polynucleotides consisting of nucleotide sequences represented by SEQ ID NOs: 194 to 210, 374, and 1315 to 1350, and 211 to 212, and 1351 to 1356, provided that at least one selected from polynucleotides consisting of nucleotide sequences represented by SEQ ID NOs: 194 to 210, 374, and 1315 to 1350 is included.

The following shows, as non-limiting examples, combinations comprising the above polynucleotide(s) as a target nucleic acid(s). Examples of a combination of target nucleic acids include any combination comprising a polynucleotide consisting of SEQ ID NO: 1315. Examples of a combination of two target nucleic acids include any combination comprising two polynucleotides consisting of SEQ ID NOs: 1317 and 195. Examples of a combination of three target nucleic acids include any combination comprising three polynucleotides consisting of SEQ ID NOs: 1315, 1316, and 194. Examples of a combination of 20 target nucleic acids include any combination comprising 20 polynucleotides consisting of SEQ ID NOs: 1315, 1316, 194, 195, 1318, 1319, 197, 1320 to 1325, 199, 1331, 200, 1335, 1352, 1354, and 1356; or of SEQ ID NOs: 1315, 1316, 194, 195, 197, 1321, 1322, 1326, 1327, 199, 1331, 200, 202, 1333, 1335, 206, 1349, 1352, 1355, and 212. Examples of a combination of 42 target nucleic acids include any combination comprising 42 polynucleotides consisting of SEQ ID NOs: 194 to 205, 1315 to 1339, 1352 to 1355, and 1356. Examples of a combination of 51 target nucleic acids include any combination comprising 51 polynucleotides consisting of SEQ ID NOs: 1315, 1316, 194, 195, 1318, 1319, 197, 1321 to 1323, 1326, 1327, 198, 199, 1329, 1331, 200, 202, 1333, 203, 1335, 204 to 212, 374, and 1337 to 1356.

In addition, as a combination of target nucleic acids in a kit or device for discriminating between Alzheimer's dementia patients and non-dementia subjects in the present invention, it is desirable to combine 2 or more, preferably 20 to 60, and more preferably 40 to 55 polynucleotides listed in Table 1-2 (provided that at least one selected from polynucleotides consisting of nucleotide sequences represented by SEQ ID NOs: 194 to 210, 374, and 1315 to 1350 is included). Specific examples include any combination of at least 2 polynucleotides selected from polynucleotides consisting of nucleotide sequences represented by SEQ ID NOs: 194 to 210, 374, and 1315 to 1350, and 211 to 212, and 1351 to 1356, provided that at least one selected from polynucleotides consisting of nucleotide sequences represented by SEQ ID NOs: 194 to 210, 374, and 1315 to 1350 is included.

In addition, as a combination of target nucleic acids in a kit or device for discriminating between vascular dementia patients and non-dementia subjects in the present invention, it is desirable to combine 2 or more, preferably 20 to 60, and more preferably 40 to 55 polynucleotides listed in Table 1-2 (provided that at least one selected from polynucleotides consisting of nucleotide sequences represented by SEQ ID NOs: 194 to 210, 374, and 1315 to 1350 is included). Specific examples include any combination of at least 2 polynucleotides selected from polynucleotides consisting of nucleotide sequences represented by SEQ ID NOs: 194 to 210, 374, and 1315 to 1350, and 211 to 212, and 1351 to 1356, provided that at least one selected from polynucleotides consisting of nucleotide sequences represented by SEQ ID NOs: 194 to 210, 374, and 1315 to 1350 is included.

In addition, as a combination of target nucleic acids in a kit or device for discriminating between Lewy body dementia patients and non-dementia subjects in the present invention, it is desirable to combine 2 or more, preferably 20 to 60, and more preferably 40 to 55 polynucleotides listed in Table 1-2 (provided that at least one selected from polynucleotides consisting of nucleotide sequences represented by SEQ ID NOs: 194 to 210, 374, and 1315 to 1350 is included). Specific examples include any combination of at least 2 polynucleotides selected from polynucleotides consisting of nucleotide sequences represented by SEQ ID NOs: 194 to 210, 374, and 1315 to 1350, and 211 to 212, and 1351 to 1356, provided that at least one selected from polynucleotides consisting of a nucleotide sequence represented by SEQ ID NOs: 194 to 210, 374, and 1315 to 1350 is included.

In addition, as a combination of target nucleic acids in a kit or device for discriminating between normal pressure hydrocephalus patients and non-dementia subjects in the present invention, it is desirable to combine 2 or more, preferably 20 to 60, and more preferably 40 to 55 polynucleotides listed in Table 1-2 (provided that at least one selected from polynucleotides consisting of nucleotide sequences represented by SEQ ID NOs: 194 to 210, 374, and 1315 to 1350 is included). Specific examples include any combination of at least 2 polynucleotides selected from polynucleotides consisting of nucleotide sequences represented by SEQ ID NOs: 194 to 210, 374, and 1315 to 1350, and 211 to 212, and 1351 to 1356, provided that at least one selected from polynucleotides consisting of nucleotide sequences represented by SEQ ID NOs: 194 to 210, 374, and 1315 to 1350 is included.

In addition, as a combination of target nucleic acids in a kit or device for discriminating between frontotemporal lobar degeneration patients and non-dementia subjects in the present invention, it is desirable to combine 2 or more, preferably 20 to 60, and more preferably 40 to 55 polynucleotides listed in Table 1-2 (provided that at least one selected from polynucleotides consisting of nucleotide sequences represented by SEQ ID NOs: 194 to 210, 374, and 1315 to 1350 is included). Specific examples include any combination of at least 2 polynucleotides selected from polynucleotides consisting of nucleotide sequences represented by SEQ ID NOs: 194 to 210, 374, and 1315 to 1350, and 211 to 212, and 1351 to 1356, provided that at least one selected from polynucleotides consisting of nucleotide sequences represented by SEQ ID NOs: 194 to 210, 374, and 1315 to 1350 is included.

A kit or device of the present invention may comprise, in addition to the above-described polynucleotide(s) in the present invention (which may include any variant(s), fragment(s), or derivative(s)), a polynucleotide(s) known to be able to detect dementia or a polynucleotide(s) that will be discovered in the future.

The kit or device of the present invention may be used in combination with, in addition to the above-described polynucleotides according to the present invention, a known marker(s) for detection of dementia, such as amyloid β-protein or tau protein, and/or an imaging test, such as PET, that visualizes the marker(s).

The kit of the present invention may be used in combination with, in addition to the above-described polynucleotides according to the present invention, a neuropsychological examination or test such as MMSE.

These polynucleotides, or variants thereof or fragments thereof contained in the kit of the present invention may be packaged in different containers either individually or in any combination.

The kit of the present invention may comprise a kit for extracting nucleic acids (e.g., total RNA) from body fluids, cells, or tissues, a fluorescent material for labeling, an enzyme and a medium for nucleic acid amplification, an instruction manual, etc.

The device of the present invention may be a device for measuring a dementia marker(s), in which nucleic acids such as the above-described polynucleotide(s), variant(s), derivative(s), or fragment(s) thereof according to the present invention are, for instance, bonded or attached onto a solid phase. Examples of the material for the solid phase include plastics, paper, glass, and silicon. The material for the solid phase is preferably a plastic from the viewpoint of easy processability. The solid phase has any shape and is, for example, square, round, reed-shaped, or film-shaped. The device of the present invention includes, for example, a device for measurement by a hybridization technique. Specific examples thereof include blotting devices and nucleic acid arrays (e.g., microarrays, DNA chips, and RNA chips).

The nucleic acid array technique is a technique which involves bonding or attaching the nucleic acids one by one by use of a method [e.g., a method of spotting the nucleic acids using a high-density dispenser called spotter or arrayer onto the surface of the solid phase surface-treated, if necessary, by coating with L-lysine or the introduction of a functional group such as an amino group or a carboxyl group, a method of spraying the nucleic acids onto the solid phase using an inkjet which injects very small liquid droplets by a piezoelectric element or the like from a nozzle, or a method of sequentially synthesizing nucleotides on the solid phase] to prepare an array such as a chip and measuring target nucleic acids through the use of hybridization using this array.

The kit or device of the present invention comprises nucleic acids capable of specifically binding to at least one, preferably at least two, more preferably at least three, most preferably at least five to all polynucleotides selected from the above-mentioned dementia marker miRNAs of the group A, or to a complementary strand(s) of the polynucleotide(s), respectively. The kit or device of the present invention can optionally further comprise nucleic acids capable of specifically binding to at least one, preferably at least two, more preferably at least three, most preferably at least five to all polynucleotides selected from the above-mentioned dementia marker miRNAs of the group B, or to a complementary strand(s) of the polynucleotide(s), respectively.

The kit or device of the present invention can be used for detecting dementia as described in Section 4 below.

4. Method for Detecting Dementia

The present invention relates to a method for detecting dementia, comprising measuring, *in vitro,* an expression level(s) of 1 or more, 2 or more, 3 or more, 10 or more, preferably 3 to 30, or 3 to 110 genes comprising target gene(s) selected from group A (optionally in combination with target gene(s) selected from groups B to D) or target gene(s) selected from group E (optionally in combination with target gene(s) selected from group A, B, or D) in a sample as described in the Section "3. Kit or Device for Detection of Dementia"; and evaluating *in vitro* whether or not a subject has dementia using the measured expression level(s) (and optionally a control expression level(s) in a healthy subject(s) as likewise measured). In the method, it is possible to determine that a subject has dementia by using the expression level(s) of the above-mentioned genes in a sample from a subject suspected of having dementia and control expression levels of the genes in samples from non-dementia subjects, e.g., if there is a difference in the expression level(s) of the target nucleic acid(s) in the samples when comparing the expression level(s) between the subjects, in which the sample(s) may be, for instance, blood, serum, plasma or the like collected from the subject suspected of having dementia and the non-dementia subjects.

In addition, the present invention relates to a method for predicting dementia, comprising measuring, *in vitro,* an expression level(s) of 1 or more, 2 or more, 3 or more, 10 or more, preferably 3 to 30, or 3 to 110 genes comprising target gene(s) selected from group E (optionally in combination with target gene(s) selected from group A, B, or D) as described in the Section "3. Kit or Device for Detection of Dementia"; and predicting *in vitro* the possibility for a subject to develop dementia in the future by using the measured expression level(s) (and optionally control expression level(s) in healthy subjects as likewise measured). In the method, it is possible to predict that a subject is likely to have dementia by using the expression level(s) of the above-mentioned genes in a sample from a subject with the possibility of developing dementia in the future and control expression levels of the genes in samples from non-dementia subjects, e.g., if there is a difference in the expression level(s) of the target nucleic acid(s) in the samples when comparing the expression level(s) between the subjects, in which the samples may be, for instance, blood, serum, plasma or the like collected from the subject with the possibility of developing dementia in the future and the non-dementia subjects.

In one embodiment, the present invention provides a method for detecting dementia, comprising: measuring *in vitro* an expression level(s) of 1 or more, 2 or more, 3 or more, 10 or more, preferably 20 to 60, and more preferably 40 to 55 genes selected from genes including dementia-derived genes represented by miR-5698, miR-1915-3p, miR-1343-5p, miR-6861-5p, miR-6781-5p, miR-4508, miR-6743-5p, miR-6726-5p, miR-4525, miR-4651, miR-6813-5p, miR-5787, miR-1290, miR-6075, miR-4758-5p, miR-4690-5p, miR-762, miR-371a-5p, miR-6765-3p, miR-6784-5p, miR-6778-5p, miR-6875-5p, miR-4534, miR-4721, miR-6756-5p, miR-615-5p, miR-6727-5p, miR-6887-5p, miR-8063, miR-6880-5p, miR-6805-3p, miR-4726-5p, miR-4710, miR-7111-5p, miR-3619-3p, miR-6795-5p, miR-1254, miR-1233-5p, miR-6836-3p, miR-6769a-5p, miR-4532, miR-365a-5p, miR-1231, miR-1228-5p, miR-4430, miR-296-3p, miR-1237-5p, miR-4466, miR-6789-5p, miR-4632-5p, miR-4745-5p, miR-4665-5p, miR-6807-5p, and miR-7114-5p and optionally further comprising dementia-derived genes represented by miR-1225-3p, miR-3184-5p, miR-150-3p, miR-423-5p, miR-575, miR-671-5p, miR-939-5p, and miR-3665 in a sample by using a kit or device (comprising the above nucleic acid(s) capable of being used in the present invention) according to the present invention as described in the above Section 3; and determining that a subject has dementia by using the expression level(s) of the above-mentioned genes in a sample from a subject suspected of having dementia and control expression levels of the genes in samples from non-dementia subjects, e.g., if there is a difference in the expression level(s) of the target nucleic acid(s) in the samples when comparing both the expression level(s) between the subject(s), in which the sample(s) may be, for instance, blood, serum, plasma or the like collected from the subject suspected of having dementia and the non-dementia subjects.

The above-mentioned method of the present invention enables less-invasive, highly sensitive and specific early diagnosis of at least one dementia selected from Alzheimer's dementia, vascular dementia, Lewy body dementia, normal pressure hydrocephalus, frontotemporal lobar degeneration, senile dementia of the neurofibrillary tangle type, dementia resulting from other diseases (e.g., infectious diseases), alcoholic dementia, vitamin deficiency dementia, or the like, mixed dementia of any of these types, or type-unknown or unspecified dementia. This allows for an early medical intervention and suppression of disease progression and further makes it possible to monitor exacerbation of the disease and monitor effectiveness of surgical treatment or medication.

For the method for extracting the dementia-derived gene(s) from the sample such as blood, serum, or plasma according to the present invention, it is particularly preferred to add a reagent for RNA extraction in 3D-Gene (registered trademark) RNA extraction reagent from liquid sample kit (Toray Industries, Inc., Japan) for preparation of a sample. Alternatively, a general acidic phenol method (acid guanidinium-phenol-chloroform (AGPC)) may be used, or Trizol (registered trademark) (Life Technologies Corp.) may be used. Alternatively, for preparation of a sample, a reagent for RNA extraction containing acidic phenol, such as Trizol (Life Technologies Corp.) or Isogen (Nippon Gene Co., Ltd., Japan) may be added. Alternatively, a kit such as miRNeasy (registered trademark) Mini Kit (Qiagen N.V.) may be used. However, the method according to the present invention is not limited thereto.

The present invention also provides use of a dementia-derived miRNA gene(s) in a sample from a subject for *in vitro* detection of an expression product(s) thereof.

The present invention also provides use of the kit or the device of the present invention for detecting *in vitro* an expression product(s) of a dementia-derived miRNA gene(s) in a sample from a subject.

A technique to be used for carrying out the method of the present invention is not limited and, for instance, the kit or device of the present invention (comprising the above nucleic acid(s) capable of being used in the present invention) as described in the above Section 3 may be used for carrying out the method.

In the method of the present invention, the kit or device described above comprising a single polynucleotide or any possible combination of polynucleotides that can be used in the present invention as described above, can be used.

In the detection or (genetic) diagnosis of dementia according to the present invention, a polynucleotide(s) contained in the kit or device of the present invention can be used as a probe(s) or a primer(s). In the case of using the polynucleotide(s) as a primer(s), TaqMan (registered trademark) MicroRNA Assays from Life Technologies Corp., miScript PCR System from Qiagen N.V., or the like can be used. However, though the method according to the present invention is not limited thereto.

In the method of the present invention, measurement of the gene expression levels can be performed according to a routine technique of a method known in the art for specifically detecting particular genes, for example, a hybridization technique such as Northern blot, Southern blot, *in situ* hybridization, Northern hybridization, or Southern hybridization; a quantitative amplification technique such as quantitative RT-PCR; or a method with a next-generation sequencer. A body fluid such as blood, serum, plasma, or urine from a subject is collected as a sample to be assayed depending on the type of the detection method to be used. Alternatively, total RNA prepared from such a body fluid by the method described above may be used, and various polynucleotides including cDNA prepared from the RNA may be used.

The method of the present invention is useful for the diagnosis of dementia or the detection of the presence or absence of dementia. Specifically, the detection of dementia according to the present invention can be performed by detecting *in vitro* an expression level(s) of a gene(s) which is detected using the nucleic acid probe(s) or the primer(s) contained in the kit or the device according to the present invention, in a sample such as blood, serum, plasma, or urine from a subject suspected of having dementia. If the expression level(s) of a polynucleotide(s) consisting of a nucleotide sequence(s) represented by at least one selected from SEQ ID NOs: 1 to 210, 374, 1315 to 1350, and 1435 to 1448 and optionally a nucleotide sequence(s) represented by one or more of SEQ ID NOs: 211 to 249, 1351 to 1356, and 1449 to 1453, or optionally a nucleotide sequence(s) represented by at least one selected from SEQ ID NOs: 250 to 373, and optionally a nucleotide sequence(s) represented by one or more selected from SEQ ID NOs: 375 to 390 in a sample such as blood, serum, plasma, or urine of a subject suspected of having dementia, is statistically significantly higher compared to an expression level(s) thereof in a sample such as blood, serum, or plasma, or urine of a subject without dementia, the former subject can be evaluated as having dementia.

In one embodiment, if the expression level(s) of a polynucleotide(s) consisting of a nucleotide sequence(s) represented by at least one selected from SEQ ID NOs: 194 to 210, 374, and 1315 to 1350 and optionally a nucleotide sequence(s) represented by one or more selected from SEQ ID NOs: 211 to 212 and 1351 to 1356 in a sample such as blood, serum, plasma, or urine of a subject suspected of having dementia, is statistically significantly higher compared to an expression level(s) thereof in a sample such as blood, serum, or plasma, or urine of a subject without dementia, the former subject can be evaluated as having dementia.

Regarding the method of the present invention, the method for detecting the absence of dementia or the presence of dementia for a sample from a subject comprises collecting a body fluid such as blood, serum, plasma, or urine of the subject, and measuring the expression level(s) of the target gene(s) (or target nucleic acid(s)) contained therein using one or more polynucleotides (including variant(s), fragment(s), or derivative(s)) selected from the groups of polynucleotides according to the present invention, thereby evaluating the presence or absence of dementia or detecting dementia. The method for detecting dementia according to the present invention can also be used to evaluate or diagnose, for example, the presence or absence of amelioration of the disease or the degree of amelioration thereof in a dementia patient when a dementia-related therapeutic drug which is known in the art or on a development stage (including Aricept, GE Aricept, memantine, galantamine, rivastigmine, and combination drugs thereof, as non-limiting examples) is administered to the patient for treatment or amelioration of the disease.

The method of the present invention may comprise, for example, the following steps (a), (b), and (c):

(a) contacting *in vitro* a sample from a subject with a polynucleotide(s) contained in a kit or device of the present invention;
(b) measuring an expression level(s) of target nucleic acid(s) in the sample using the polynucleotide(s) as a nucleic acid probe(s) or primer(s); and
(c) evaluating the presence or absence of dementia (or mutant protein(s)) in the subject on the basis of the measurement results in step (b).

In a preferred embodiment of the method according to the present invention, the present invention provides a method for detecting dementia, comprising measuring an expression level(s) of a target nucleic acid(s) in a sample from a subject using a nucleic acid(s) capable of specifically binding to 1, preferably 2 or more, 3 or more, 10 or more, and more preferably 3 to 30 polynucleotides selected from the group consisting of miR-4274, miR-4272, miR-4728-5p, miR-4443, miR-4506, miR-6773-5p, miR-4662a-5p, miR-3184-3p, miR-4281, miR-320d, miR-6729-3p, miR-5192, miR-6853-5p, miR-1234-3p, miR-1233-3p, miR-4539, miR-3914, miR-4738-5p, miR-548au-3p, miR-1539, miR-4720-3p, miR-365b-5p, miR-4486, miR-1227-5p, miR-4667-5p, miR-6088, miR-6820-5p, miR-4505, miR-548q, miR-4658, miR-450a-5p, miR-1260b, miR-3677-5p, miR-6777-3p, miR-6826-3p, miR-6832-3p, miR-4725-3p, miR-7161-3p, miR-2277-5p, miR-7110-3p, miR-4312, miR-4461, miR-6766-5p, miR-1266-3p, miR-6729-5p, miR-526b-3p, miR-519e-5p, miR-512-5p, miR-5088-5p, miR-1909-3p, miR-6511a-5p, miR-4734, miR-936, miR-1249-3p, miR-6777-5p, miR-4487, miR-3155a, miR-563, miR-4741, miR-6788-5p, miR-4433b-5p, miR-323a-5p, miR-6811-5p, miR-6721-5p, miR-5004-5p, miR-6509-3p, miR-648, miR-3917, miR-6087, miR-1470, miR-586, miR-3150a-5p, miR-105-3p, miR-7973, miR-1914-5p, miR-4749-3p, miR-15b-5p, miR-1289, miR-4433a-5p, miR-3666, miR-3186-3p, miR-4725-5p, miR-4488, miR-4474-3p, miR-6731-3p, miR-4640-3p, miR-202-5p, miR-6816-5p, miR-638, miR-6821-5p, miR-1247-3p, miR-6765-5p, miR-6800-5p, miR-3928-3p, miR-3940-5p, miR-3960, miR-6775-5p, miR-3178, miR-1202, miR-6790-5p, miR-4731-3p, miR-2681-3p, miR-6758-5p, miR-8072, miR-518d-3p, miR-3606-3p, miR-4800-5p, miR-1292-3p, miR-6784-3p, miR-4450, miR-6132, miR-4716-5p, miR-6860, miR-1268b, miR-378d, miR-4701-5p, miR-4329, miR-185-3p, miR-552-3p, miR-1273g-5p, miR-6769b-3p, miR-520a-3p, miR-4524b-5p, miR-4291, miR-6734-3p, miR-143-5p, miR-939-3p, miR-6889-3p, miR-6842-3p, miR-4511, miR-4318, miR-4653-5p, miR-6867-3p, miR-133b, miR-3196, miR-193b-3p, miR-3162-3p, miR-6819-3p, miR-1908-3p, miR-6786-5p, miR-3648, miR-4513, miR-3652, miR-4640-5p, miR-6871-5p, miR-7845-5p, miR-3138, miR-6884-5p, miR-4653-3p, miR-636, miR-4652-3p, miR-6823-5p, miR-4502, miR-7113-5p, miR-8087, miR-7154-3p, miR-5189-5p, miR-1253, miR-518c-5p, miR-7151-5p, miR-3614-3p, miR-4727-5p, miR-3682-5p, miR-5090, miR-337-3p, miR-488-5p, miR-100-5p, miR-4520-3p, miR-373-3p, miR-6499-5p, miR-3909, miR-32-5p, miR-302a-3p, miR-4686, miR-4659a-3p, miR-4287, miR-1301-5p, miR-593-3p, miR-517a-3p, miR-517b-3p, miR-142-3p, miR-1185-2-3p, miR-602, miR-527, miR-518a-5p, miR-4682, miR-28-5p, miR-4252, miR-452-5p, miR-525-5p, miR-3622a-3p, miR-6813-3p, and miR-4769-3p, miR-5698, miR-1915-3p, miR-1343-5p, miR-6861-5p, miR-6781-5p, miR-4508, miR-6743-5p, miR-6726-5p, miR-4525, miR-4651, miR-6813-5p, miR-5787, miR-1290, miR-6075, miR-4758-5p, miR-4690-5p, miR-762, miR-371a-5p, miR-6765-3p, miR-6784-5p, miR-6778-5p, miR-6875-5p, miR-4534, miR-4721, miR-6756-5p, miR-615-5p, miR-6727-5p, miR-6887-5p, miR-8063, miR-6880-5p, miR-6805-3p, miR-4726-5p, miR-4710, miR-7111-5p, miR-3619-3p, miR-6795-5p, miR-1254, miR-1233-5p, miR-6836-3p, miR-6769a-5p, miR-4532, miR-365a-5p, miR-1231, miR-1228-5p, miR-4430, miR-296-3p, miR-1237-5p, miR-4466, miR-6789-5p, miR-4632-5p, miR-4745-5p, miR-4665-5p, miR-6807-5p and miR-7114-5p, miR-516a-5p, miR-769-3p, miR-3692-5p, miR-3945, miR-4433a-3p, miR-4485-3p, miR-6831-5p, miR-519c-5p, miR-551b-5p, miR-1343-3p, miR-4286, miR-4634, miR-4733-3p, and miR-6086, or to a complementary strand(s) of the polynucleotide(s); and evaluating *in vitro* whether or not the subject has dementia by using the measured expression level(s) and a control expression level(s) in a subject(s) without dementia as likewise measured.

The term "evaluating" as used herein is evaluation support based on results of *in vitro* examination, not physician's judgment.

As described above, in a preferred embodiment of the method according to the present invention, miR-5698 is hsa-miR-5698, miR-1915-3p is hsa-miR-1915-3p, miR-1343-5p is hsa-miR-1343-5p, miR-6861-5p is hsa-miR-6861-5p, miR-6781-5p is hsa-miR-6781-5p, miR-4508 is hsa-miR-4508, miR-6743-5p is hsa-miR-6743-5p, miR-6726-5p is hsa-miR-6726-5p, miR-4525 is hsa-miR-4525, miR-4651 is hsa-miR-4651, miR-6813-5p is hsa-miR-6813-5p, miR-5787 is hsa-miR-5787, miR-1290 is hsa-miR-1290, miR-6075 is hsa-miR-6075, miR-4758-5p is hsa-miR-4758-5p, miR-4690-5p is hsa-miR-4690-5p, miR-762 is hsa-miR-762, miR-4274 is hsa-miR-4274, miR-4272 is hsa-miR-4272, miR-4728-5p is hsa-miR-4728-5p, miR-4443 is hsa-miR-4443, miR-4506 is hsa-miR-4506, miR-6773-5p is hsa-miR-6773-5p, miR-4662a-5p is hsa-miR-4662a-5p, miR-3184-3p is hsa-miR-3184-3p, miR-4281 is hsa-miR-4281, miR-320d is hsa-miR-320d, miR-6729-3p is hsa-miR-6729-3p, miR-5192 is hsa-miR-5192, miR-6853-5p is hsa-miR-6853-5p, miR-1234-3p is hsa-miR-1234-3p, miR-1233-3p is hsa-miR-1233-3p, miR-4539 is hsa-miR-4539, miR-3914 is hsa-miR-3914, miR-4738-5p is hsa-miR-4738-5p, miR-548au-3p is hsa-miR-548au-3p, miR-1539 is hsa-miR-1539, miR-4720-3p is hsa-miR-4720-3p, miR-365b-5p is hsa-miR-365b-5p, miR-4486 is hsa-miR-4486, miR-1227-5p is hsa-miR-1227-5p, miR-4667-5p is hsa-miR-4667-5p, miR-6088 is hsa-miR-6088, miR-6820-5p is hsa-miR-6820-5p, miR-4505 is hsa-miR-4505, miR-548q is hsa-miR-548q, miR-4658 is hsa-miR-4658, miR-450a-5p is hsa-miR-450a-5p, miR-1260b is hsa-miR-1260b, miR-3677-5p is hsa-miR-3677-5p, miR-6777-3p is hsa-miR-6777-3p, miR-6826-3p is hsa-miR-6826-3p, miR-6832-3p is hsa-miR-6832-3p, miR-4725-3p is hsa-miR-4725-3p, miR-7161-3p is hsa-miR-7161-3p, miR-2277-5p is hsa-miR-2277-5p, miR-7110-3p is hsa-miR-7110-3p, miR-4312 is hsa-miR-4312, miR-4461 is hsa-miR-4461, miR-6766-5p is hsa-miR-6766-5p, miR-1266-3p is hsa-miR-1266-3p, miR-6729-5p is hsa-miR-6729-5p, miR-526b-3p is hsa-miR-526b-3p, miR-519e-5p is hsa-miR-519e-5p, miR-512-5p is hsa-miR-512-5p, miR-5088-5p is hsa-miR-5088-5p, miR-1909-3p is hsa-miR-1909-3p, miR-6511a-5p is hsa-miR-6511a-5p, miR-4734 is hsa-miR-4734, miR-936 is hsa-miR-936, miR-1249-3p is hsa-miR-1249-3p, miR-6777-5p is hsa-miR-6777-5p, miR-4487 is hsa-miR-4487, miR-3155a is hsa-miR-3155a, miR-563 is hsa-miR-563, miR-4741 is hsa-miR-4741, miR-6788-5p is hsa-miR-6788-5p, miR-4433b-5p is hsa-miR-4433b-5p, miR-323a-5p is hsa-miR-323a-5p, miR-6811-5p is hsa-miR-6811-5p, miR-6721-5p is hsa-miR-6721-5p, miR-5004-5p is hsa-miR-5004-5p, miR-6509-3p is hsa-miR-6509-3p, miR-648 is hsa-miR-648, miR-3917 is hsa-miR-3917, miR-6087 is hsa-miR-6087, miR-1470 is hsa-miR-1470, miR-586 is hsa-miR-586, miR-3150a-5p is hsa-miR-3150a-5p, miR-105-3p is hsa-miR-105-3p, miR-7973 is hsa-miR-7973, miR-1914-5p is hsa-miR-1914-5p, miR-4749-3p is hsa-miR-4749-3p, miR-15b-5p is hsa-miR-15b-5p, miR-1289 is hsa-miR-1289, miR-4433a-5p is hsa-miR-4433a-5p, miR-3666 is hsa-miR-3666, miR-3186-3p is hsa-miR-3186-3p, miR-4725-5p is hsa-miR-4725-5p, miR-4488 is hsa-miR-4488, miR-4474-3p is hsa-miR-4474-3p, miR-6731-3p is hsa-miR-6731-3p, miR-4640-3p is hsa-miR-4640-3p, miR-202-5p is hsa-miR-202-5p, miR-6816-5p is hsa-miR-6816-5p, miR-638 is hsa-miR-638, miR-6821-5p is hsa-miR-6821-5p, miR-1247-3p is hsa-miR-1247-3p, miR-6765-5p is hsa-miR-6765-5p, miR-6800-5p is hsa-miR-6800-5p, miR-3928-3p is hsa-miR-3928-3p, miR-3940-5p is hsa-miR-3940-5p, miR-3960 is hsa-miR-3960, miR-6775-5p is hsa-miR-6775-5p, miR-3178 is hsa-miR-3178, miR-1202 is hsa-miR-1202, miR-6790-5p is hsa-miR-6790-5p, miR-4731-3p is hsa-miR-4731-3p, miR-2681-3p is hsa-miR-2681-3p, miR-6758-5p is hsa-miR-6758-5p, miR-8072 is hsa-miR-8072, miR-518d-3p is hsa-miR-518d-3p, miR-3606-3p is hsa-miR-3606-3p, miR-4800-5p is hsa-miR-4800-5p, miR-1292-3p is hsa-miR-1292-3p, miR-6784-3p is hsa-miR-6784-3p, miR-4450 is hsa-miR-4450, miR-6132 is hsa-miR-6132, miR-4716-5p is hsa-miR-4716-5p, miR-6860 is hsa-miR-6860, miR-1268b is hsa-miR-1268b, miR-378d is hsa-miR-378d, miR-4701-5p is hsa-miR-4701-5p, miR-4329 is hsa-miR-4329, miR-185-3p is hsa-miR-185-3p, miR-552-3p is hsa-miR-552-3p, miR-1273g-5p is hsa-miR-1273g-5p, miR-6769b-3p is hsa-miR-6769b-3p, miR-520a-3p is hsa-miR-520a-3p, miR-4524b-5p is hsa-miR-4524b-5p, miR-4291 is hsa-miR-4291, miR-6734-3p is hsa-miR-6734-3p, miR-143-5p is hsa-miR-143-5p, miR-939-3p is hsa-miR-939-3p, miR-6889-3p is hsa-miR-6889-3p, miR-6842-3p is hsa-miR-6842-3p, miR-4511 is hsa-miR-4511, miR-4318 is hsa-miR-4318, miR-4653-5p is hsa-miR-4653-5p, miR-6867-3p is hsa-miR-6867-3p, miR-133b is hsa-miR-133b, miR-3196 is hsa-miR-3196, miR-193b-3p is hsa-miR-193b-3p, miR-3162-3p is hsa-miR-3162-3p, miR-6819-3p is hsa-miR-6819-3p, miR-1908-3p is hsa-miR-1908-3p, miR-6786-5p is hsa-miR-6786-5p, miR-3648 is hsa-miR-3648, miR-4513 is hsa-miR-4513, miR-3652 is hsa-miR-3652, miR-4640-5p is hsa-miR-4640-5p, miR-6871-5p is hsa-miR-6871-5p, miR-7845-5p is hsa-miR-7845-5p, miR-3138 is hsa-miR-3138, miR-6884-5p is hsa-miR-6884-5p, miR-4653-3p is hsa-miR-4653-3p, miR-636 is hsa-miR-636, miR-4652-3p is hsa-miR-4652-3p, miR-6823-5p is hsa-miR-6823-5p, miR-4502 is hsa-miR-4502, miR-7113-5p is hsa-miR-7113-5p, miR-8087 is hsa-miR-8087, miR-7154-3p is hsa-miR-7154-3p, miR-5189-5p is hsa-miR-5189-5p, miR-1253 is hsa-miR-1253, miR-518c-5p is hsa-miR-518c-5p, miR-7151-5p is hsa-miR-7151-5p, miR-3614-3p is hsa-miR-3614-3p, miR-4727-5p is hsa-miR-4727-5p, miR-3682-5p is hsa-miR-3682-5p, miR-5090 is hsa-miR-5090, miR-337-3p is hsa-miR-337-3p, miR-488-5p is hsa-miR-488-5p, miR-100-5p is hsa-miR-100-5p, miR-4520-3p is hsa-miR-4520-3p, miR-373-3p is hsa-miR-373-3p, miR-6499-5p is hsa-miR-6499-5p, miR-3909 is hsa-miR-3909, miR-32-5p is hsa-miR-32-5p, miR-302a-3p is hsa-miR-302a-3p, miR-4686 is hsa-miR-4686, miR-4659a-3p is hsa-miR-4659a-3p, miR-4287 is hsa-miR-4287, miR-1301-5p is hsa-miR-1301-5p, miR-593-3p is hsa-miR-593-3p, miR-517a-3p is hsa-miR-517a-3p, miR-517b-3p is hsa-miR-517b-3p, miR-142-3p is hsa-miR-142-3p, miR-1185-2-3p is hsa-miR-1185-2-3p, miR-602 is hsa-miR-602, miR-527 is hsa-miR-527, miR-518a-5p is hsa-miR-518a-5p, miR-4682 is hsa-miR-4682, miR-28-5p is hsa-miR-28-5p, miR-4252 is hsa-miR-4252, miR-452-5p is hsa-miR-452-5p, miR-525-5p is hsa-miR-525-5p, miR-3622a-3p is hsa-miR-3622a-3p, miR-6813-3p is hsa-miR-6813-3p, miR-4769-3p is hsa-miR-4769-3p, miR-371a-5p is hsa-miR-371a-5p, miR-6765-3p is hsa-miR-6765-3p, miR-6784-5p is hsa-miR-6784-5p, miR-6778-5p is hsa-miR-6778-5p, miR-6875-5p is hsa-miR-6875-5p, miR-4534 is hsa-miR-4534, miR-4721 is hsa-miR-4721, miR-6756-5p is hsa-miR-6756-5p, miR-615-5p is hsa-miR-615-5p, miR-6727-5p is hsa-miR-6727-5p, miR-6887-5p is hsa-miR-6887-5p, miR-8063 is hsa-miR-8063, miR-6880-5p is hsa-miR-6880-5p, miR-6805-3p is hsa-miR-6805-3p, miR-4726-5p is hsa-miR-4726-5p, miR-4710 is hsa-miR-4710, miR-7111-5p is hsa-miR-7111-5p, miR-3619-3p is hsa-miR-3619-3p, miR-6795-5p is hsa-miR-6795-5p, miR-1254 is hsa-miR-1254, miR-1233-5p is hsa-miR-1233-5p, miR-6836-3p is hsa-miR-6836-3p, miR-6769a-5p is hsa-miR-6769a-5p, miR-4532 is hsa-miR-4532, miR-365a-5p is hsa-miR-365a-5p, miR-1231 is hsa-miR-1231, miR-1228-5p is hsa-miR-1228-5p, miR-4430 is hsa-miR-4430, miR-296-3p is hsa-miR-296-3p, miR-1237-5p is hsa-miR-1237-5p, miR-4466 is hsa-miR-4466, miR-6789-5p is hsa-miR-6789-5p, miR-4632-5p is hsa-miR-4632-5p, miR-4745-5p is hsa-miR-4745-5p, miR-4665-5p is hsa-miR-4665-5p, miR-6807-5p is hsa-miR-6807-5p, miR-7114-5p is hsa-miR-7114-5p, miR-516a-5p is hsa-miR-516a-5p, miR-769-3p is hsa-miR-769-3p, miR-3692-5p is hsa-miR-3692-5p, miR-3945 is hsa-miR-3945, miR-4433a-3p is hsa-miR-4433a-3p, miR-4485-3p is hsa-miR-4485-3p, miR-6831-5p is hsa-miR-6831-5p, miR-519c-5p is hsa-miR-519c-5p, miR-551b-5p is hsa-miR-551b-5p, miR-1343-3p is hsa-miR-1343-3p, miR-4286 is hsa-miR-4286, miR-4634 is hsa-miR-4634, miR-4733-3p is hsa-miR-4733-3p, and miR-6086 is hsa-miR-6086.

In addition, in a preferred embodiment of the method according to the present invention, a nucleic acid(s) (specifically, a probe(s) or primer(s)) is selected from the group consisting of polynucleotides shown in any of the following group (a) to (e):

(a) a polynucleotide consisting of a nucleotide sequence represented by any of SEQ ID NOs: 1 to 210, 374, 1315 to 1350, and 1435 to 1448 or a nucleotide sequence derived from the nucleotide sequence by the replacement of u with t, a variant thereof, a derivative thereof, or a fragment thereof comprising 15 or more consecutive nucleotides,
(b) a polynucleotide comprising a nucleotide sequence represented by any of SEQ ID NOs: 1 to 210, 374, 1315 to 1350, and 1435 to 1448,
(c) a polynucleotide consisting of a nucleotide sequence complementary to a nucleotide sequence represented by any of SEQ ID NOs: 1 to 210, 374, 1315 to 1350, and 1435 to 1448 or a nucleotide sequence derived from the nucleotide sequence by the replacement of u with t, a variant thereof, a derivative thereof, or a fragment thereof comprising 15 or more consecutive nucleotides,
(d) a polynucleotide comprising a nucleotide sequence complementary to a nucleotide sequence represented by any of SEQ ID NOs: 1 to 210, 374, 1315 to 1350, and 1435 to 1448 or a nucleotide sequence derived from the nucleotide sequence by the replacement of u with t, and
(e) a polynucleotide hybridizing under stringent conditions to any of the polynucleotides (a) to (d).

A target nucleic acid(s) in the method of the present invention may further comprise at least one polynucleotide selected from the group consisting of miR-1225-3p, miR-3184-5p, miR-665, miR-211-5p, miR-1247-5p, miR-3656, miR-149-5p, miR-744-5p, miR-345-5p, miR-150-5p, miR-191-3p, miR-651-5p, miR-34a-5p, miR-409-5p, miR-369-5p, miR-1915-5p, miR-204-5p, miR-137, miR-382-5p, miR-517-5p, miR-532-5p, miR-22-5p, miR-1237-3p, miR-1224-3p, miR-625-3p, miR-328-3p, miR-122-5p, miR-202-3p, miR-4781-5p, miR-718, miR-342-3p, miR-26b-3p, miR-140-3p, miR-200a-3p, miR-378a-3p, miR-484, miR-296-5p, miR-205-5p, and miR-431-5p, miR-150-3p, miR-423-5p, miR-575, miR-671-5p, miR-939-5p, and miR-3665, miR-30d-5p, miR-30b-3p, miR-92a-3p, miR-371b-5p, miR-486-5p, or miR-1471, miR-1538, miR-449b-3p, miR-1976, miR-4268, miR-4279, miR-3620-3p, miR-3944-3p, miR-3156-3p, miR-3187-5p, miR-4685-3p, miR-4695-3p, miR-4697-3p, miR-4713-5p, miR-4723-3p, miR-371b-3p, miR-3151-3p, miR-3192-3p, miR-6728-3p, miR-6736-3p, miR-6740-3p, miR-6741-3p, miR-6743-3p, miR-6747-3p, miR-6750-3p, miR-6754-3p, miR-6759-3p, miR-6761-3p, miR-6762-3p, miR-6769a-3p, miR-6776-3p, miR-6778-3p, miR-6779-3p, miR-6786-3p, miR-6787-3p, miR-6792-3p, miR-6794-3p, miR-6801-3p, miR-6802-3p, miR-6803-3p, miR-6804-3p, miR-6810-5p, miR-6823-3p, miR-6825-3p, miR-6829-3p, miR-6833-3p, miR-6834-3p, miR-6780b-3p, miR-6845-3p, miR-6862-3p, miR-6865-3p, miR-6870-3p, miR-6875-3p, miR-6877-3p, miR-6879-3p, miR-6882-3p, miR-6885-3p, miR-6886-3p, miR-6887-3p, miR-6890-3p, miR-6893-3p, miR-6894-3p, miR-7106-3p, miR-7109-3p, miR-7114-3p, miR-7155-5p, miR-7160-5p, miR-615-3p, miR-920, miR-1825, miR-675-3p, miR-1910-5p, miR-2278, miR-2682-3p, miR-3122, miR-3151-5p, miR-3175, miR-4323, miR-4326, miR-4284, miR-3605-3p, miR-3622b-5p, miR-3646, miR-3158-5p, miR-4722-3p, miR-4728-3p, miR-4747-3p, miR-4436b-5p, miR-5196-3p, miR-5589-5p, miR-345-3p, miR-642b-5p, miR-6716-3p, miR-6511b-3p, miR-208a-5p, miR-6726-3p, miR-6744-5p, miR-6782-3p, miR-6789-3p, miR-6797-3p, miR-6800-3p, miR-6806-5p, miR-6824-3p, miR-6837-5p, miR-6846-3p, miR-6858-3p, miR-6859-3p, miR-6861-3p, miR-6880-3p, miR-7111-3p, miR-7152-5p, miR-642a-5p, miR-657, miR-1236-3p, miR-764, miR-4314, miR-3675-3p, miR-5703, miR-3191-5p, miR-6511a-3p, miR-6809-3p, miR-6815-5p, miR-6857-3p, and miR-6878-3p, or miR-766-3p, miR-1229-3p, miR-1306-5p, miR-210-5p, miR-198, miR-485-3p, miR-668-3p, miR-532-3p, miR-877-3p, miR-1238-3p, miR-3130-5p, miR-4298, miR-4290, miR-3943, miR-346, and miR-767-3p or a nucleic acid(s) capable of specifically binding to the polynucleotide(s).

In a preferred embodiment of the method according to the present invention, miR-1225-3p is hsa-miR-1225-3p, miR-3184-5p is hsa-miR-3184-5p, miR-665 is hsa-miR-665, miR-211-5p is hsa-miR-211-5p, miR-1247-5p is hsa-miR-1247-5p, miR-3656 is hsa-miR-3656, miR-149-5p is hsa-miR-149-5p, miR-744-5p is hsa-miR-744-5p, miR-345-5p is hsa-miR-345-5p, miR-150-5p is hsa-miR-150-5p, miR-191-3p is hsa-miR-191-3p, miR-651-5p is hsa-miR-651-5p, miR-34a-5p is hsa-miR-34a-5p, miR-409-5p is hsa-miR-409-5p, miR-369-5p is hsa-miR-369-5p, miR-1915-5p is hsa-miR-1915-5p, miR-204-5p is hsa-miR-204-5p, miR-137 is hsa-miR-137, miR-382-5p is hsa-miR-382-5p, miR-517-5p is hsa-miR-517-5p, miR-532-5p is hsa-miR-532-5p, miR-22-5p is hsa-miR-22-5p, miR-1237-3p is hsa-miR-1237-3p, miR-1224-3p is hsa-miR-1224-3p, miR-625-3p is hsa-miR-625-3p, miR-328-3p is hsa-miR-328-3p, miR-122-5p is hsa-miR-122-5p, miR-202-3p is hsa-miR-202-3p, miR-4781-5p is hsa-miR-4781-5p, miR-718 is hsa-miR-718, miR-342-3p is hsa-miR-342-3p, miR-26b-3p is hsa-miR-26b-3p, miR-140-3p is hsa-miR-140-3p, miR-200a-3p is hsa-miR-200a-3p, miR-378a-3p is hsa-miR-378a-3p, miR-484 is hsa-miR-484, miR-296-5p is hsa-miR-296-5p, miR-205-5p is hsa-miR-205-5p, miR-431-5p is hsa-miR-431-5p, miR-150-3p is hsa-miR-150-3p, miR-423-5p is hsa-miR-423-5p, miR-575 is hsa-miR-575, miR-671-5p is hsa-miR-671-5p, miR-939-5p is hsa-miR-939-5p, miR-3665 is hsa-miR-3665, miR-30d-5p is hsa-miR-30d-5p, miR-30b-3p is hsa-miR-30b-3p, miR-92a-3p is hsa-miR-92a-3p, miR-371b-5p is hsa-miR-371b-5p, miR-486-5p is hsa-miR-486-5p, miR-1471 is hsa-miR-1471, miR-1538 is hsa-miR-1538, miR-449b-3p is hsa-miR-449b-3p, miR-1976 is hsa-miR-1976, miR-4268 is hsa-miR-4268, miR-4279 is hsa-miR-4279, miR-3620-3p is hsa-miR-3620-3p, miR-3944-3p is hsa-miR-3944-3p, miR-3156-3p is hsa-miR-3156-3p, miR-3187-5p is hsa-miR-3187-5p, miR-4685-3p is hsa-miR-4685-3p, miR-4695-3p is hsa-miR-4695-3p, miR-4697-3p is hsa-miR-4697-3p, miR-4713-5p is hsa-miR-4713-5p, miR-4723-3p is hsa-miR-4723-3p, miR-371b-3p is hsa-miR-371b-3p, miR-3151-3p is hsa-miR-3151-3p, miR-3192-3p is hsa-miR-3192-3p, miR-6728-3p is hsa-miR-6728-3p, miR-6736-3p is hsa-miR-6736-3p, miR-6740-3p is hsa-miR-6740-3p, miR-6741-3p is hsa-miR-6741-3p, miR-6743-3p is hsa-miR-6743-3p, miR-6747-3p is hsa-miR-6747-3p, miR-6750-3p is hsa-miR-6750-3p, miR-6754-3p is hsa-miR-6754-3p, miR-6759-3p is hsa-miR-6759-3p, miR-6761-3p is hsa-miR-6761-3p, miR-6762-3p is hsa-miR-6762-3p, miR-6769a-3p is hsa-miR-6769a-3p, miR-6776-3p is hsa-miR-6776-3p, miR-6778-3p is hsa-miR-6778-3p, miR-6779-3p is hsa-miR-6779-3p, miR-6786-3p is hsa-miR-6786-3p, miR-6787-3p is hsa-miR-6787-3p, miR-6792-3p is hsa-miR-6792-3p, miR-6794-3p is hsa-miR-6794-3p, miR-6801-3p is hsa-miR-6801-3p, miR-6802-3p is hsa-miR-6802-3p, miR-6803-3p is hsa-miR-6803-3p, miR-6804-3p is hsa-miR-6804-3p, miR-6810-5p is hsa-miR-6810-5p, miR-6823-3p is hsa-miR-6823-3p, miR-6825-3p is hsa-miR-6825-3p, miR-6829-3p is hsa-miR-6829-3p, miR-6833-3p is hsa-miR-6833-3p, miR-6834-3p is hsa-miR-6834-3p, miR-6780b-3p is hsa-miR-6780b-3p, miR-6845-3p is hsa-miR-6845-3p, miR-6862-3p is hsa-miR-6862-3p, miR-6865-3p is hsa-miR-6865-3p, miR-6870-3p is hsa-miR-6870-3p, miR-6875-3p is hsa-miR-6875-3p, miR-6877-3p is hsa-miR-6877-3p, miR-6879-3p is hsa-miR-6879-3p, miR-6882-3p is hsa-miR-6882-3p, miR-6885-3p is hsa-miR-6885-3p, miR-6886-3p is hsa-miR-6886-3p, miR-6887-3p is hsa-miR-6887-3p, miR-6890-3p is hsa-miR-6890-3p, miR-6893-3p is hsa-miR-6893-3p, miR-6894-3p is hsa-miR-6894-3p, miR-7106-3p is hsa-miR-7106-3p, miR-7109-3p is hsa-miR-7109-3p, miR-7114-3p is hsa-miR-7114-3p, miR-7155-5p is hsa-miR-7155-5p, miR-7160-5p is hsa-miR-7160-5p, miR-615-3p is hsa-miR-615-3p, miR-920 is hsa-miR-920, miR-1825 is hsa-miR-1825, miR-675-3p is hsa-miR-675-3p, miR-1910-5p is hsa-miR-1910-5p, miR-2278 is hsa-miR-2278, miR-2682-3p is hsa-miR-2682-3p, miR-3122 is hsa-miR-3122, miR-3151-5p is hsa-miR-3151-5p, miR-3175 is hsa-miR-3175, miR-4323 is hsa-miR-4323, miR-4326 is hsa-miR-4326, miR-4284 is hsa-miR-4284, miR-3605-3p is hsa-miR-3605-3p, miR-3622b-5p is hsa-miR-3622b-5p, miR-3646 is hsa-miR-3646, miR-3158-5p is hsa-miR-3158-5p, miR-4722-3p is hsa-miR-4722-3p, miR-4728-3p is hsa-miR-4728-3p, miR-4747-3p is hsa-miR-4747-3p, miR-4436b-5p is hsa-miR-4436b-5p, miR-5196-3p is hsa-miR-5196-3p, miR-5589-5p is hsa-miR-5589-5p, miR-345-3p is hsa-miR-345-3p, miR-642b-5p is hsa-miR-642b-5p, miR-6716-3p is hsa-miR-6716-3p, miR-6511b-3p is hsa-miR-6511b-3p, miR-208a-5p is hsa-miR-208a-5p, miR-6726-3p is hsa-miR-6726-3p, miR-6744-5p is hsa-miR-6744-5p, miR-6782-3p is hsa-miR-6782-3p, miR-6789-3p is hsa-miR-6789-3p, miR-6797-3p is hsa-miR-6797-3p, miR-6800-3p is hsa-miR-6800-3p, miR-6806-5p is hsa-miR-6806-5p, miR-6824-3p is hsa-miR-6824-3p, miR-6837-5p is hsa-miR-6837-5p, miR-6846-3p is hsa-miR-6846-3p, miR-6858-3p is hsa-miR-6858-3p, miR-6859-3p is hsa-miR-6859-3p, miR-6861-3p is hsa-miR-6861-3p, miR-6880-3p is hsa-miR-6880-3p, miR-7111-3p is hsa-miR-7111-3p, miR-7152-5p is hsa-miR-7152-5p, miR-642a-5p is hsa-miR-642a-5p, miR-657 is hsa-miR-657, miR-1236-3p is hsa-miR-1236-3p, miR-764 is hsa-miR-764, miR-4314 is hsa-miR-4314, miR-3675-3p is hsa-miR-3675-3p, miR-5703 is hsa-miR-5703, miR-3191-5p is hsa-miR-3191-5p, miR-6511a-3p is hsa-miR-6511a-3p, miR-6809-3p is hsa-miR-6809-3p, miR-6815-5p is hsa-miR-6815-5p, miR-6857-3p is hsa-miR-6857-3p, miR-6878-3p is hsa-miR-6878-3p, miR-766-3p is hsa-miR-766-3p, miR-1229-3p is hsa-miR-1229-3p, miR-1306-5p is hsa-miR-1306-5p, miR-210-5p is hsa-miR-210-5p, miR-198 is hsa-miR-198, miR-485-3p is hsa-miR-485-3p, miR-668-3p is hsa-miR-668-3p, miR-532-3p is hsa-miR-532-3p, miR-877-3p is hsa-miR-877-3p, miR-1238-3p is hsa-miR-1238-3p, miR-3130-5p is hsa-miR-3130-5p, miR-4298 is hsa-miR-4298, miR-4290 is hsa-miR-4290, miR-3943 is hsa-miR-3943, miR-346 is hsa-miR-346, and miR-767-3p is hsa-miR-767-3p.

Further, in one embodiment, the expression level(s) of the polynucleotide(s) are measured by using nucleic acid(s) capable of specifically binding to the polynucleotide(s) or to the complementary strand(s) of the polynucleotide(s), and the nucleic acid(s) are polynucleotide(s) selected from the group consisting of the following polynucleotides (f) to (j), (k) to (o), and (p) to (t):

(f) a polynucleotide consisting of a nucleotide sequence represented by any of SEQ ID NOs: 211 to 249, 1351 to 1356, and 1449 to 1453 or a nucleotide sequence derived from the nucleotide sequence by the replacement of u with t, a variant thereof, a derivative thereof, or a fragment thereof comprising 15 or more consecutive nucleotides,

(g) a polynucleotide comprising a nucleotide sequence represented by any of SEQ ID NOs: 211 to 249, 1351 to 1356, and 1449 to 1453,

(h) a polynucleotide consisting of a nucleotide sequence complementary to a nucleotide sequence represented by any of SEQ ID NOs: 211 to 249, 1351 to 1356, and 1449 to 1453 or a nucleotide sequence derived from the nucleotide sequence by the replacement of u with t, a variant thereof, a derivative thereof, or a fragment thereof comprising 15 or more consecutive nucleotides,

(i) a polynucleotide comprising a nucleotide sequence complementary to a nucleotide sequence represented by any of SEQ ID NOs: 211 to 249, 1351 to 1356, and 1449 to 1453 or a nucleotide sequence derived from the nucleotide sequence by the replacement of u with t, and

(j) a polynucleotide hybridizing under stringent conditions to any of the polynucleotides (f) to (i),

(k) a polynucleotide consisting of a nucleotide sequence represented by any of SEQ ID NOs: 250 to 373 or a nucleotide sequence derived from the nucleotide sequence by the replacement of u with t, a variant thereof, a derivative thereof, or a fragment thereof comprising 15 or more consecutive nucleotides,

(1) a polynucleotide comprising a nucleotide sequence represented by any of SEQ ID NOs: 250 to 373,

(m) a polynucleotide consisting of a nucleotide sequence complementary to a nucleotide sequence represented by any of SEQ ID NOs: 250 to 373 or a nucleotide sequence derived from the nucleotide sequence by the replacement of u with t, a variant thereof, a derivative thereof, or a fragment thereof comprising 15 or more consecutive nucleotides,

(n) a polynucleotide comprising a nucleotide sequence complementary to a nucleotide sequence represented by any of SEQ ID NOs: 250 to 373 or a nucleotide sequence derived from the nucleotide sequence by the replacement of u with t, and

(o) a polynucleotide hybridizing under stringent conditions to any of the polynucleotides (k) to (n), and

(p) a polynucleotide consisting of a nucleotide sequence represented by any of SEQ ID NOs: 375 to 390 or a nucleotide sequence derived from the nucleotide sequence by the replacement of u with t, a variant thereof, a derivative thereof, or a fragment thereof comprising 15 or more consecutive nucleotides,

(q) a polynucleotide comprising a nucleotide sequence represented by any of SEQ ID NOs: 375 to 390,

(r) a polynucleotide consisting of a nucleotide sequence complementary to a nucleotide sequence represented by any of SEQ ID NOs: 375 to 390 or a nucleotide sequence derived from the nucleotide sequence by the replacement of u with t, a variant thereof, a derivative thereof, or a fragment thereof comprising 15 or more consecutive nucleotides,

(s) a polynucleotide comprising a nucleotide sequence complementary to a nucleotide sequence represented by any of SEQ ID NOs: 375 to 390 or a nucleotide sequence derived from the nucleotide sequence by the replacement of u with t, and

(t) a polynucleotide hybridizing under stringent conditions to any of the polynucleotides (p) to (s).

In one embodiment of the method according to the present invention, the present invention provides a method for detecting dementia, comprising measuring an expression level(s) of a target nucleic acid(s) in a sample from a subject by using a nucleic acid(s) capable of specifically binding to 1, preferably 2 or more, 3 or more, 10 or more, more preferably 20 to 60, and still more preferably 40 to 55 polynucleotides selected from the group consisting of miR-6765-3p, miR-6784-5p, miR-5698, miR-6778-5p, miR-1915-3p, miR-6875-5p, miR-1343-5p, miR-4534, miR-6861-5p, miR-4721, miR-6756-5p, miR-615-5p, miR-6727-5p, miR-6887-5p, miR-8063, miR-6880-5p, miR-6805-3p, miR-6781-5p, miR-4508, miR-4726-5p, miR-4710, miR-7111-5p, miR-3619-3p, miR-6743-5p, miR-6795-5p, miR-6726-5p, miR-4525, miR-1254, miR-1233-5p, miR-4651, miR-6836-3p, miR-6769a-5p, miR-6813-5p, miR-4532, miR-365a-5p, miR-1231, miR-5787, miR-1290, miR-1228-5p, miR-371a-5p, miR-4430, miR-296-3p, miR-6075, miR-1237-5p, miR-4758-5p, miR-4690-5p, miR-4466, miR-6789-5p, miR-4632-5p, miR-4745-5p, miR-4665-5p, miR-6807-5p, miR-762, and miR-7114-5p; and evaluating *in vitro* whether or not the subject has dementia by using the measured expression level(s) and a control expression level(s) in a subject(s) without dementia as likewise measured.

In an embodiment of the method according to the present invention, miR-6765-3p is hsa-miR-6765-3p, miR-6784-5p is hsa-miR-6784-5p, miR-5698 is hsa-miR-5698, miR-6778-5p is hsa-miR-6778-5p, miR-1915-3p is hsa-miR-1915-3p, miR-6875-5p is hsa-miR-6875-5p, miR-1343-5p is hsa-miR-1343-5p, miR-4534 is hsa-miR-4534, miR-6861-5p is hsa-miR-6861-5p, miR-4721 is hsa-miR-4721, miR-6756-5p is hsa-miR-6756-5p, miR-615-5p is hsa-miR-615-5p, miR-6727-5p is hsa-miR-6727-5p, miR-6887-5p is hsa-miR-6887-5p, miR-8063 is hsa-miR-8063, miR-6880-5p is hsa-miR-6880-5p, miR-6805-3p is hsa-miR-6805-3p, miR-6781-5p is hsa-miR-6781-5p, miR-4508 is hsa-miR-4508, miR-4726-5p is hsa-miR-4726-5p, miR-4710 is hsa-miR-4710, miR-7111-5p is hsa-miR-7111-5p, miR-3619-3p is hsa-miR-3619-3p, miR-6743-5p is hsa-miR-6743-5p, miR-6795-5p is hsa-miR-6795-5p, miR-6726-5p is hsa-miR-6726-5p, miR-4525 is hsa-miR-4525, miR-1254 is hsa-miR-1254, miR-1233-5p is hsa-miR-1233-5p, miR-4651 is hsa-miR-4651, miR-6836-3p is hsa-miR-6836-3p, miR-6769a-5p is hsa-miR-6769a-5p, miR-6813-5p is hsa-miR-6813-5p, miR-4532 is hsa-miR-4532, miR-365a-5p is hsa-miR-365a-5p, miR-1231 is hsa-miR-1231, miR-5787 is hsa-miR-5787, miR-1290 is hsa-miR-1290, miR-1228-5p is hsa-miR-1228-5p, miR-371a-5p is hsa-miR-371a-5p, miR-4430 is hsa-miR-4430, miR-296-3p is hsa-miR-296-3p, miR-6075 is hsa-miR-6075, miR-1237-5p is hsa-miR-1237-5p, miR-4758-5p is hsa-miR-4758-5p, miR-4690-5p is hsa-miR-4690-5p, miR-4466 is hsa-miR-4466, miR-6789-5p is hsa-miR-6789-5p, miR-4632-5p is hsa-miR-4632-5p, miR-4745-5p is hsa-miR-4745-5p, miR-4665-5p is hsa-miR-4665-5p, miR-6807-5p is hsa-miR-6807-5p, miR-762 is hsa-miR-762, and miR-7114-5p is hsa-miR-7114-5p.

In addition, in one embodiment of the method according to the present invention, a nucleic acid(s) (specifically, a probe(s) or primer(s)) is a polynucleotide(s) selected from the group consisting of polynucleotides shown in any of the following group (a) to (e):

(a) a polynucleotide consisting of a nucleotide sequence represented by any of SEQ ID NOs: 194 to 210, 374, and 1315 to 1350 or a nucleotide sequence derived from the nucleotide sequence by the replacement of u with t, a variant thereof, a derivative thereof, or a fragment thereof comprising 15 or more consecutive nucleotides,

(b) a polynucleotide comprising a nucleotide sequence represented by any of SEQ ID NOs: 194 to 210, 374, and 1315 to 1350,

(c) a polynucleotide consisting of a nucleotide sequence complementary to a nucleotide sequence represented by any of SEQ ID NOs: 194 to 210, 374, and 1315 to 1350 or a nucleotide sequence derived from the nucleotide sequence by the replacement of u with t, a variant thereof, a derivative thereof, or a fragment thereof comprising 15 or more consecutive nucleotides,

(d) a polynucleotide comprising a nucleotide sequence complementary to a nucleotide sequence represented by any of SEQ ID NOs: 194 to 210, 374, and 1315 to 1350 or a nucleotide sequence derived from the nucleotide sequence by the replacement of u with t, and

(e) a polynucleotide hybridizing under stringent conditions to any of the polynucleotides (a) to (d).

In one embodiment, a target nucleic acid(s) in a method according to the present invention may further comprise a nucleic acid(s) capable of specifically binding to at least one polynucleotide selected from the group consisting of miR-1225-3p, miR-3184-5p, miR-150-3p, miR-423-5p, miR-575, miR-671-5p, miR-939-5p, and miR-3665.

In one embodiment of the method according to the present invention, miR-150-3p is hsa-miR-150-3p, miR-423-5p is hsa-miR-423-5p, miR-575 is hsa-miR-575, miR-671-5p is hsa-miR-671-5p, miR-939-5p is hsa-miR-939-5p, miR-1225-3p is hsa-miR-1225-3p, miR-3184-5p is hsa-miR-3184-5p, and miR-3665 is hsa-miR-3665.

Further, in one embodiment of the method according to the present invention, the nucleic acid(s) is a polynucleotide(s) selected from the group consisting of polynucleotides shown in any of the following group (f) to (j):

(f) a polynucleotide consisting of a nucleotide sequence represented by any of SEQ ID NOs: 211 to 212 and 1351 to 1356 or a nucleotide sequence derived from the nucleotide sequence by the replacement of u with t, a variant thereof, a derivative thereof, or a fragment thereof comprising 15 or more consecutive nucleotides,

(g) a polynucleotide comprising a nucleotide sequence represented by any of SEQ ID NOs: 211 to 212 and 1351 to 1356,

(h) a polynucleotide consisting of a nucleotide sequence complementary to a nucleotide sequence represented by any of SEQ ID NOs: 211 to 212 and 1351 to 1356 or a nucleotide sequence derived from the nucleotide sequence by the replacement of u with t, a variant thereof, a derivative thereof, or a fragment thereof comprising 15 or more consecutive nucleotides,

(i) a polynucleotide comprising a nucleotide sequence complementary to a nucleotide sequence represented by any of SEQ ID NOs: 211 to 212 and 1351 to 1356 or a nucleotide sequence derived from the nucleotide sequence by the replacement of u with t, and

(j) a polynucleotide hybridizing under stringent conditions to any of the polynucleotides (f) to (i).

Examples of the sample used in the method of the present invention can include samples prepared from living tissues (preferably brain tissues or nerve tissues) or body fluids such as blood, serum, plasma, and urine from subjects. Specifically, for example, an RNA-containing sample prepared from the tissue, a polynucleotide-containing sample further prepared therefrom, a body fluid such as blood, serum, plasma, or urine, a portion or the whole of a living tissue collected from the subject by biopsy or the like, or a living tissue excised by surgery can be used, and the sample for measurement can be prepared therefrom.

As used herein, the subject refers to a mammal, for example, a human, a monkey, a mouse, or a rat, without any limitation, and is preferably a human.

The steps of the method of the present invention can be changed depending on the type of the sample to be measured.

In the case of using RNA as an analyte, the method for detecting dementia (abnormal protein) can comprise, for example, the following steps (a), (b), and (c):

(a) binding RNA prepared from a sample from a subject (wherein, for example, the 3' end of the RNA may be polyadenylated for quantitative RT-PCR in step (b) or any sequence may be added to one or both ends of the RNA by ligation) or a complementary polynucleotides (cDNA) transcribed from the RNA to a polynucleotide(s) in the kit of the present invention;

(b) measuring the sample-derived RNA or the cDNA synthesized from the RNA, which has been bound to the polynucleotide(s), by hybridization using the polynucleotide(s) as a nucleic acid probe(s) or by quantitative RT-PCR using the polynucleotide(s) as a primer(s); and

(c) evaluating the presence or absence of dementia (or dementia-derived gene(s)) on the basis of the measurement results of step (b).

For example, various hybridization methods can be used for measuring the expression level(s) of a target gene(s) according to the present invention. For example, Northern blot, Southern blot, DNA chip analysis, *in situ* hybridization, Northern hybridization, or Southern hybridization can be used as such a hybridization method. PCR such as quantitative RT-PCR or next-generation sequencing can also be used in combination with a hybridization method, or as an alternative thereof.

In the case of using the Northern blot, the presence or absence of expression of each gene or the expression level thereof in the RNA can be detected or measured by using, for example, the nucleic acid probe(s) that can be used in the present invention. Specific examples thereof can include a method which comprises labeling the nucleic acid probe (or a complementary strand) with a radioisotope ($^{32}$P, $^{33}$P, $^{35}$S, etc.), a fluorescent material, or the like, hybridizing the labeled product with the living tissue-derived RNA from a subject, which is transferred to a nylon membrane or the like according to a routine method, and then detecting and measuring a signal derived from the label (radioisotope or fluorescent material) on the formed DNA/RNA duplex using a radiation detector (examples thereof can include BAS-1800 II (Fujifilm Corp.)) or a fluorescence detector (examples thereof can include STORM 865 (GE Healthcare Japan Corp.)).

In the case of using the quantitative RT-PCR, the presence or absence of expression of each gene or the expression level thereof in the RNA can be detected or measured by using, for example, the primer(s) that can be used in the present invention. Specific examples thereof can include a method which comprises recovering the living tissue-derived RNA from a subject, polyadenylating the 3'-end, preparing cDNAs from the polyadenylated RNA according to a routine method, and performing PCR according to a routine method by hybridizing a pair of primers (consisting of a positive strand and a reverse strand each binding to the cDNA) which could be contained in the kit or device for detection of the present invention with the cDNA such that the region of each target gene marker can be amplified with the cDNA as a template, to detect the obtained single-stranded or double-stranded DNA. The method for detecting the single-stranded or double-stranded DNA can include a method of performing the PCR using the primers labeled in advance with a radioisotope or a fluorescent material, a method of electrophoresing the PCR product on an agarose gel and staining the double-stranded DNA with ethidium bromide or the like for detection, and a method of transferring the produced single-stranded or double-stranded DNA to a nylon membrane or the like according to a routine method and hybridizing the single-stranded or double-stranded DNA with a labeled nucleic acid probe for detection.

In the case of using the nucleic acid array analysis, an RNA chip or a DNA chip in which the kit or device for detection in the present invention is attached as nucleic acid probes (single-stranded or double-stranded) to a substrate (solid phase), for example, is used. Regions having the attached nucleic acid probes are referred to as probe spots, and regions having no attached nucleic acid probe are referred to as blank spots. A group of genes immobilized on a solid-phase substrate is generally called a nucleic acid chip, a nucleic acid array, a microarray, or the like. The DNA or RNA array includes a DNA or RNA macroarray and a DNA or RNA microarray. In the present specification, the term "chip" includes these arrays. 3D-Gene (registered trademark) Human miRNA Oligo chip (Toray Industries, Inc., Japan) can be used as the DNA chip, though the DNA chip is not limited thereto.

Examples of the measurement using the DNA chip can include, but are not limited to, a method of detecting and measuring a signal derived from the label on the kit or device for detection using an image detector (examples thereof can include Typhoon 9410 (GE Healthcare) and 3D-Gene (registered trademark) scanner (Toray Industries, Inc., Japan)).

The "stringent conditions" used herein are, as mentioned above, conditions under which a nucleic acid probe hybridizes to its target sequence to a detectably larger extent (e.g., a measurement value equal to or larger than "(a mean of background measurement values) + (a standard error of the background measurement values) x 2)") than that for other sequences.

The stringent conditions are defined by hybridization and subsequent washing. Examples of the hybridization conditions include, but not limited to, 30°C to 60°C for 1 to 24 hours in a solution containing SSC, a surfactant, formamide, dextran sulfate, a blocking agent(s), etc. In this context, $1 \times$ SSC is an aqueous solution (pH 7.0) containing 150 mM sodium chloride and 15 mM sodium citrate. The surfactant includes, for example, SDS (sodium dodecyl sulfate), Triton, or Tween. The hybridization conditions more preferably comprise 3-10 $\times$ SSC and 0.1-1% SDS. Examples of the conditions for the washing, following the hybridization, which is another condition to define the stringent conditions, can include conditions comprising continuous washing at 30°C in a solution containing 0.5 $\times$ SSC and 0.1% SDS, at 30°C in a solution containing 0.2 $\times$ SSC and 0.1% SDS, and at 30°C in a 0.05 $\times$ SSC solution. It is desirable that the complementary strand should maintain its hybridized state with a target plus strand even by washing under such conditions. Specifically, examples of such a complementary strand can include a strand consisting of a nucleotide sequence in a completely complementary relationship with the nucleotide sequence of the target plus (+) strand, and a strand consisting of a nucleotide sequence having at least 80%, preferably at least 85%, more preferably at least 90% or at least 95% homology to the strand.

Other examples of the "stringent conditions" for the hybridization are described in, for example, Sambrook, J. & Russel, D., Molecular Cloning, A LABORATORY MANUAL, Cold Spring Harbor Laboratory Press, published on January 15, 2001, Vol. 1, 7.42 to 7.45 and Vol. 2, 8.9 to 8.17, and can be used in the present invention.

Examples of the conditions for carrying out PCR using polynucleotide fragments in the kit of the present invention as primers include treatment for approximately 15 seconds to 1 minute at 5 to 10°C plus a Tm value calculated from the sequences of the primers, using a PCR buffer having composition such as 10 mM Tris-HCL (pH 8.3), 50 mM KCl, and 1 to 2 mM MgCl$_2$. Examples of the method for calculating such a Tm value include Tm value = 2 $\times$ (the number of adenine residues + the number of thymine residues) + 4 $\times$ (the number of guanine residues + the number of cytosine residues).

In the case of using the quantitative RT-PCR, a commercially available kit for measurement specially designed for quantitatively measuring miRNA, such as TaqMan (registered trademark) MicroRNA Assays (Life Technologies Corp.), LNA (registered trademark)-based MicroRNA PCR (Exiqon), or Ncode (registered trademark) miRNA qRT-PCT kit (Invitrogen Corp.) may be used.

In the method of the present invention, measurement of the gene expression level(s) may be performed with a sequencer, in addition to hybridization methods described above. In use of a sequencer, any of DNA sequencers of the first generation based on Sanger method, the second generation with shorter read size, and the third generation with longer read size can be used (herein referred to as "next-generation sequencer", including sequencers of the second generation and the third generation). For example, a commercially available measurement kit specifically designed for measuring miRNA using Miseq, Hiseq, or NexSeq (Illumina, Inc.); Ion Proton, Ion PGM, or Ion S5/S5 XL (Thermo Fisher Scientific Inc.); PacBio RS II or Sequel (Pacific Biosciences of California, Inc.); MinION (Oxford Nanopore Technologies Ltd.) exemplified in use of a Nanopore sequencer; or the like may be used.

Next-generation sequencing is a method of obtaining sequence information using a next-generation sequencer, and characterized by being capable of simultaneously performing a huge number of sequencing reactions compared to Sanger method (e.g., Rick Kamps et al., Int. J. Mol. Sci., 2017, 18(2), p.308 and Int. Neurourol. J., 2016, 20 (Suppl.2), S76-83). Examples of next-generation sequencing steps for miRNA include, but not limited to, the following steps: at first, adaptor sequences having predetermined nucleotide sequences are attached, and all RNAs are reverse-transcribed into cDNAs before or after attachment of the sequences. After the reverse transcription, cDNAs derived from specific target miRNAs may be amplified or concentrated by PCR or the like or with a probe or the like, for analyzing the target miRNA before sequencing steps. Subsequent sequencing steps varies in detail depending on the type of a next-generation sequencer, but typically, a sequencing reaction is performed by linking to a substrate via an adaptor sequence and further using the adaptor sequence as a priming site. See details of the sequencing reaction, for example, in Rick Kamps et al. (see supra). Finally, the data are outputted. This step provides a collection of sequence information (reads) obtained by the sequencing reaction. For example, next-generation sequencing can identify a target miRNA(s) based on the sequence information, and measure the expression level thereof based on the number of reads having the sequences of the target miRNA(s).

For the calculation of gene expression levels, statistical treatment described in, for example, Statistical analysis of gene expression microarray data (Speed T., Chapman and Hall/CRC), and A beginner's guide Microarray gene expression data analysis (Causton H. C. *et al.,* Blackwell publishing) can be used in the present invention, though the calculation method is not limited thereto. For example, twice, preferably 3 times, more preferably 6 times the standard deviation of the measurement values of the blank spots are added to the average measurement value of the blank spots on the DNA chip, and probe spots having a signal value equal to or larger than the resulting value can be regarded as detection spots. Alternatively, the average measurement value of the blank spots is regarded as a background and can be subtracted from the measurement values of the probe spots to determine gene expression levels. A missing value for a gene expression level can be excluded from the analyte, preferably replaced with the smallest value of the gene expression level in each DNA chip, or more preferably replaced with a value obtained by subtracting 0.1 from a logarithmic value of the smallest value of the gene expression level. In order to eliminate low-signal genes, only a gene(s) having a gene expression level of $2^6$, preferably $2^8$, more preferably $2^{10}$ or larger in 20% or more, preferably 50% or more, more preferably 80% or more of the number of measurement samples can be selected as the analyte(s). Examples of the normalization of the gene expression level include, but are not limited to, global normalization and quantile normalization (Bolstad, B.M. *et al.,* 2003, Bioinformatics, Vol. 19, p. 185-193).

The present invention also provides a method for detecting dementia (or assisting the detection thereof) in a subject, comprising measuring a target genes or gene expression levels in a sample from the subject; and assigning the expression levels of the target genes in the sample from the subject to a discriminant (discriminant function), which is prepared using gene expression levels of a sample(s) from a subject(s) (or a patient(s)) known to have dementia and a sample(s) from a subject(s) without dementia, as a training sample(s), and which can distinguishably discriminate the presence or absence of dementia, thereby evaluating the presence or absence of dementia.

Specifically, the present invention further provides the method comprising a first step of measuring *in vitro* expression levels of target genes, which are known to determine or evaluate whether a subject has dementia and/or not, in a plurality of samples; a second step of preparing a discriminant using the measurement values of the expression levels of the target genes obtained in the first step as training samples; a third step of measuring *in vitro* the expression levels of the target genes in a sample from the subject in the same manner as in the first step; and a fourth step of assigning the measurement values of the expression levels of the target genes obtained in the third step to the discriminant obtained in the second step, and determining or evaluating whether or not the subject has dementia on the basis of the results obtained from the discriminant. The above target genes are those that can be detected, for example, by the polynucleotides, the polynucleotides contained in the kit or chip, and variants thereof or fragments thereof.

The discriminant herein can be prepared by use of any discriminant analysis method, based on which a discriminant that distinguishably discriminates the presence or absence of dementia can be prepared, such as linear discriminant analysis such as Fisher's discriminant analysis, nonlinear discriminant analysis based on the Mahalanobis' distance, neural network, Support Vector Machine (SVM) such as C-SVC, logistic regression analysis (especially, logistic regression analysis using the LASSO (Least Absolute Shrinkage and Selection Operator) method, ridge regression, or logistic regression using elastic net and multiple logistic regression using principal component analysis), k-nearest neighbor method, or decision tree, though the analysis method is not limited to these specific examples.

When a clustering boundary is a straight line or a hyperplane, the linear discriminant analysis is a method for determining the belonging of a cluster using Formula 1 as a discriminant. In Formula 1, x represents an explanatory variable, w represents a coefficient of the explanatory variable, and $w_0$ represents a constant term.

$$f(x) = w_0 + \sum_{i=1}^{n} w_i x_i \qquad \text{Formula 1}$$

Values obtained from the discriminant are referred to as discriminant scores. The measurement values of a newly offered data set can be assigned as explanatory variables to the discriminant to determine clusters by the signs of the discriminant scores.

The Fisher's discriminant analysis, a type of linear discriminant analysis, is a dimensionality reduction method for selecting a dimension suitable for discriminating classes, and constructs a highly discriminating synthetic variable by focusing on the variance of the synthetic variables and minimizing the variance of data having the same label (Venables, W. N. et al., Modern Applied Statistics with S. Fourth edition. Springer, 2002). In the Fisher's discriminant analysis, direction w of projection is determined so as to maximize Formula 2. In this formula, $\mu$ represents an average input, $n_g$ represents the number of data belonging to class g, and $\mu_g$ represents an average input of the data belonging to class g. The numerator and the denominator are the interclass variance and the intraclass variance, respectively, when each of data is projected in the direction of the vector w. Discriminant coefficient wi is determined by maximizing this ratio (Takafumi Kanamori et al., "Pattern Recognition", KYORITSU SHUPPAN CO., LTD. (Tokyo, Japan) (2009); Richard O. et al., Pattern Classification, Second Edition., Wiley-Interscience, 2000).

$$J(w) = \frac{\sum_{g=1}^{G} n_g \left( w^T \mu_g - w^T \mu \right) \left( w^T \mu_g - w^T \mu \right)^T}{\sum_{g=1}^{G} \sum_{i:y_i=g} \left( w^T x_i - w^T \mu_g \right) \left( w^T x_i - w^T \mu_g \right)} \qquad \text{Formula 2}$$

$$subject\ to \quad \mu = \sum_{i=1}^{n} \frac{x_i}{n}, \quad \mu_g = \sum_{i:u_i=g}^{n} \frac{x_i}{n_g} \quad .$$

The Mahalanobis' distance is calculated according to Formula 3 in consideration of data correlation and can be used as nonlinear discriminant analysis for determining a cluster in which a data point belongs to, based on a short Mahalanobis' distance from the data point to that cluster. In Formula 3, $\mu$ represents a central vector of each cluster, and $S^{-1}$ represents an inverse matrix of the variance-covariance matrix of the cluster. The central vector is calculated from explanatory variable x, and an average vector, a median value vector, or the like can be used.

$$D(x, \mu) = \left\{ (x - \mu)' S^{-1} (x - \mu) \right\}^{\frac{1}{2}} \qquad \text{Formula 3}$$

SVM is a discriminant analysis method devised by V. Vapnik (The Nature of Statistical Leaning Theory, Springer, 1995). Particular data points of a data set having known classes are defined as explanatory variables, and classes are defined as objective variables. A boundary plane called hyperplane for correctly classifying the data set into the known classes is determined, and a discriminant for data classification is determined using the boundary plane. Then, the measurement values of a newly offered data set can be assigned as explanatory variables to the discriminant to determine classes. In this respect, the result of the discriminant analysis may be classes, may be a probability of being classified into correct classes, or may be the distance from the hyperplane. In SVM, a method of nonlinearly converting a feature vector to a high dimension and performing linear discriminant analysis in the space is known as a method for tackling nonlinear problems. An expression in which an inner product of two factors in a nonlinearly mapped space is expressed only by inputs in their original spaces is called kernel. Examples of the kernel can include a linear kernel, an RBF (Radial Basis Function) kernel, and a Gaussian kernel. While highly dimensional mapping is performed according to the kernel, the optimum discriminant, *i.e.,* a discriminant, can be actually constructed by mere calculation according to the kernel, which avoids calculating features in the mapped space (e.g., Hideki Aso et al., Frontier of Statistical Science 6 "Statistics of pattern recognition and learning - New concepts and approaches", Iwanami Shoten, Publishers (Tokyo, Japan) (2004); Nello Cristianini et al., Introduction to SVM, Kyoritsu Shuppan Co., Ltd. (Tokyo, Japan) (2008)).

C-support vector classification (C-SVC), a type of SVM, comprises preparing a hyperplane by training a data set with the explanatory variables of two groups and classifying an unknown data set into either of the groups (C. Cortes et al., 1995, Machine Learning, Vol. 20, p. 273-297).

Exemplary calculation of the C-SVC discriminant that can be used in the method of the present invention will be given below. First, all subjects are divided into two groups, i.e., a group of dementia patients and a group of subjects without dementia. Diagnostic brain imaging (e.g., MRI, CT, SPECT) can be used as a reference of determining whether or not a subject has dementia.

Next, a data set consisting of comprehensive gene expression levels of serum-derived samples of the two divided groups (hereinafter, this data set is referred to as a training cohort) is prepared, and a C-SVC discriminant is determined by using genes found to differ clearly in their gene expression levels between the two groups as explanatory variables and this grouping as objective variables (e.g., -1 and +1). An optimizing objective function is represented by Formula 4 wherein e represents all input vectors, y represents an objective variable, a represents a Lagrange's undetermined multiplier vector, Q represents a positive definite matrix, and C represents a parameter for adjusting constrained conditions.

$$\min_{a} \quad \frac{1}{2} a^T Q a - e^T a$$

Formula 4

$$\text{subject to} \quad y^T a = 0, \quad 0 \le a_i \le C, \quad i = 1, \dots, l,$$

Formula 5 is a finally obtained discriminant, and a group in which the data point belongs to can be determined on the basis of the sign of a value obtained according to the discriminant. In this formula, x represents a support vector, y represents a label indicating the belonging of a group, a represents the corresponding coefficient, b represents a constant term, and K represents a kernel function.

$$f(x) = \text{sgn}\left( \sum_{i=1}^{l} y_i a_i K(x_i, x) + b \right)$$

Formula 5

For example, an RBF kernel defined by Formula 6 can be used as the kernel function. In this formula, x represents a support vector, and y represents a kernel parameter for adjusting the complexity of the hyperplane.

$$K(x_i, x_j) = \exp\left( -r \|x_i - x_j\|^2 \right) \quad r < 0$$

Formula 6

Logistic regression is a multivariate analysis method in which one category variable (binary variable) is used as an objective variable to predict the probability of occurrence by using multiple explanatory variables, and is expressed in the following formula 7.

$$\text{logit}(\text{prob}(y_i = 1)) = \beta_0 + \sum_{j=1}^{p} \beta_j x_j$$

Formula 7

The LASSO (Least Absolute Shrinkage and Selection Operator) method is one of techniques for selecting and adjusting variables when multiple observed variables are present, and was proposed by Tibshirani (Tibshirani R., 1996, J R Stat Soc Ser B, vol.58, p267-88). Ridge regression is the oldest regularization technique, and was proposed by Hoerl (Hoerl, A.E., 1970, Technometrics., vol.12, p.55-67). Elastic net refers to a model in which the LASSO method and ridge regression is linearly combined and was proposed by Zou (Zou, H., 2005, J R Stat Soc Ser B, vol.67, p.301-320). The LASSO method is characterized in that when regression coefficients are estimated, penalties are imposed, so that overfitting to a model is reduced and some of the regression coefficients are then estimated as 0. In ridge regression, coefficients in a model can be estimated and even if the explanatory variable is larger than the number of samples, the variable larger than the number of samples can be selected. When there is a strong correlation between explanatory variables, only one of the variables with a high correlation remains in the LASSO method and the rest are estimated to 0. By contrast, in ridge regression, both can be selected. In elastic net, explanatory variables that have a high correlation and are difficult to be selected in the LASSO method can be properly selected to create a model with reduced dimensions. In logistic regression using the LASSO method, regression coefficients are estimated so as to maximize a log-likelihood function expressed in Formula 8.

$$\frac{1}{N} \sum_{i=1}^{N} \left( y_i (\beta_0 + x_i^T \beta) - \log\left(1 + e^{(\beta_0 + x_i^T \beta)}\right) \right) - \lambda \sum_{j=1}^{p} |\beta_j|$$

Formula 8

Principal component analysis method is a technique in which correlation between variables of quantitative data described using many variables is excluded and analysis is conducted, with as small information loss as possible, by converging into a small number of synthetic variables without any correlation, and was proposed by Pearson (PEARSON, K., 1901, Philosophical Magazine, series 6, vol.2(11), p559-572) and Hotelling (HOTELLING, H., 1933, Journal of Educational Psychology, vol.24, p417-441, p498-520). The degree of contribution to a certain data point can be expressed by principal component scores obtained after the principal component analysis.

The method of the present invention can comprise, for example, the following steps (a), (b), and (c):

(a) measuring an expression level(s) of a target gene(s) in samples already known to be from dementia patients and to be from subjects without dementia, using polynucleotide(s), a kit, or a DNA chip for detection according to the present invention;

(b) preparing the discriminants of Formulas 1 to 3, 5 and 6 described above from the measurement values of the expression level(s) measured in step (a); and

(c) measuring an expression level(s) of the target gene(s) in a sample from a subject using the polynucleotide(s), the kit, or the DNA chip for diagnosis (detection) according to the present invention, and assigning the obtained measurement value(s) to the discriminants prepared in step (b), and determining or evaluating whether or not the subject has dementia on the basis of the obtained results, or evaluating the expression level(s) derived from a dementia patient by comparison with a control from a subject(s) without dementia.

In this context, in the discriminants of Formulas 1 to 3, 5 and 6, x represents an explanatory variable and includes a value obtained by measuring a polynucleotide(s) selected from the polynucleotides described in Section 2 above, or a fragment thereof. Specifically, the explanatory variable of the present invention for discriminating between a dementia patient(s) and subject(s) without dementia is a gene expression level(s) selected from, for example, the following:
gene expression level(s) in sera of a dementia patient and a subject without dementia as measured by any DNA comprising 15 or more consecutive nucleotides in the nucleotide sequence represented by any of SEQ ID NOs: 1 to 210, 211 to 249, 250 to 374, 375 to 390, 1315 to 1350, 1351 to 1356, 1435 to 1448, and 1449 to 1453 or in a complementary sequence thereof.

In addition, in one embodiment, an explanatory variable for discriminating between the subjects is a gene expression level(s) selected from, for instance, the following:
gene expression level(s) in sera of a dementia patient and a subject without dementia as measured by any DNA comprising 15 or more consecutive nucleotides in the nucleotide sequence represented by any of SEQ ID NOs: 194 to 210, 374, and 1315 to 1350, and 211 to 212, and 1351 to 1356 or in a complementary sequence thereof.

As described above, for the method for determining or evaluating whether or not a subject has dementia using a sample from the subject, it is necessary to use a discriminant employing one or more gene expression levels as an explanatory variable(s). In particular, for enhancing the accuracy of the discriminant using a single gene expression level alone, it is necessary to use a gene having a clear difference in the expression level between two groups consisting of a group of dementia patients and a group of subjects with normal cognitive functions, in a discriminant.

Specifically, the gene that is used for an explanatory variable of a discriminant is preferably determined as follows. First, using comprehensive gene expression levels of a group of dementia patients and comprehensive gene expression levels of a group of test subjects without dementia, both of which are in a training cohort, as a data set, the degree of difference in the expression level of each gene between the two groups is obtained by use of, for example, the P value of a parametric analysis such as t-test, the P value of a nonparametric analysis such as the Mann-Whitney's U test or the P value of the Wilcoxon test.

The gene can be regarded as being statistically significant when the critical rate (significance level) as the P value obtained by the test is smaller than, for example, 5%, 1%, or 0.01%.

In order to correct an increased probability of type I error attributed to the repetition of a test, a method known in the art, for example, Bonferroni or Holm method, can be used for the correction (e.g., Yasushi Nagata et al., "Basics of statistical multiple comparison methods", Scientist Press Co., Ltd. (Tokyo, Japan) (2007)). As an example of the Bonferroni correction, for example, the P value obtained by a test is multiplied by the number of repetitions of the test, *i.e.,* the number of genes used in the analysis, and the obtained value can be compared with a desired significance level to suppress a probability of causing type I error in the whole test.

Instead of the test, the absolute value (fold change) of an expression ratio of a median value of each gene expression level between gene expression levels of a group of dementia patients and gene expression levels of a group of test subjects without dementia may be calculated to select a gene that is used for an explanatory variable in a discriminant. Alternatively, ROC curves may be prepared using gene expression levels of a group of dementia patients and a group of test subjects without dementia, and a gene that is used for an explanatory variable in a discriminant can be selected on the basis of an AUROC value.

Next, a discriminant that can be calculated by various methods described above is prepared using any number of genes having large difference in their gene expression levels determined here. Examples of the method for constructing a discriminant that produces the largest discrimination accuracy include a method of constructing a discriminant in every combination of genes that satisfy the significance level being P value, and a method of repetitively evaluating the genes for use in the preparation of a discriminant while increasing the number of genes one by one in a descending order of difference in gene expression level (Furey TS. et al., 2000, Bioinformatics., Vol. 16, p. 906-14). To the discriminant, the gene expression level of another independent dementia patient or a test subject without dementia is assigned as an explanatory variable to calculate discrimination results of the group to which the independent dementia patient or the test subject without dementia belongs. Specifically, the gene set for diagnosis found and the discriminant constructed using the gene set for diagnosis can be evaluated in an independent sample cohort to find more universal gene set for diagnosis that can detect dementia and a more universal method for discriminating dementia.

In preparing a discriminant using expression levels of a plurality of genes as an explanatory variable, it is not necessary to select a gene having a clear difference in expression level between the group of dementia patients and the group of test subjects without dementia as described above. Specifically, if expression levels of a plurality of genes are used in combination even though the expression levels of individual genes do not clearly differ, a discriminant having high discriminant performance can be obtained, as the case may be. Because of this, it is possible to utilize a method of directly searching for a discriminant having high discriminant performance without beforehand selecting the gene to be employed in the discriminant.

Split-sample method is preferably used for evaluating the performance (generality) of the discriminant. Specifically, a data set is divided into a training cohort and a validation cohort, and gene selection by a statistical test and discriminant preparation are performed using the training cohort. Accuracy, sensitivity, and specificity are calculated using a result of discriminating a validation cohort according to the discriminant, and a true group to which the validation cohort belongs, to evaluate the performance of the discriminant. On the other hand, instead of dividing a data set, the gene selection by a statistical test and discriminant preparation may be performed using all of samples, and accuracy, sensitivity, and specificity can be calculated by the discriminant using a newly prepared sample cohort for evaluation of the performance of the discriminant.

The present invention provides a polynucleotide(s) for diagnosis or detection of a disease as being useful for diagnosing and treating dementia, a method for detecting dementia using the polynucleotide(s), and a kit and device for detecting dementia, comprising the polynucleotide(s).

A gene set for diagnosis may be set as, for instance, any combination selected from a group of one or more polynucleotides based on a nucleotide sequence(s) represented by any of SEQ ID NOs: 1 to 210 as described above, and optionally further comprising at least one polynucleotide based on a nucleotide sequence(s) represented by any of SEQ ID NOs: 194 to 210, 211 to 249, 250 to 374, and 375 to 390. Then, a discriminant is constructed using the expression levels of the gene set for diagnosis in samples from dementia patients as a result of tissue diagnosis and samples from subjects without dementia. As a result, whether or not a subject, from which an unknown sample is provided, has dementia can be discriminated by measuring expression levels of the gene set for diagnosis in the unknown sample.

In one embodiment, a gene set for diagnosis is set as any combination selected from a group of one or more polynucleotides based on a nucleotide sequence(s) represented by any of SEQ ID NOs: 194 to 210, 374, and 1315 to 1350 as described above and optionally further comprising at least one polynucleotide based on a nucleotide sequence(s) represented by any of SEQ ID NOs: 211 to 212 and 1351 to 1356. Then, a discriminant is constructed using the expression levels of the gene set for diagnosis in samples from dementia patients as a result of tissue diagnosis and samples from subjects without dementia. As a result, whether or not a subject, from which an unknown sample is provided, has dementia can be discriminated by measuring expression levels of the gene set for diagnosis in the unknown sample.

EXAMPLES

The present invention is described further specifically with reference to Examples below. However, the scope of the present invention is not intended to be limited by these Examples.

[Reference Example 1]

<Collection of samples>

Sera were collected using VENOJECT II vacuum blood collecting tube VP-AS109K60 (Terumo Corp. (Japan)) from a total of 1,606 people including 1,496 dementia patients (patients having diagnosed definitely as dementia by doctors) and 110 non-dementia subjects after obtainment of informed consent (Table 2).

[Table 2]

| Subject's disease type | | | Number of samples | |
|---|---|---|---|---|
| Dementia patients | 1 | Alzheimer's dementia | 1147 | 1496 |
| | 2 | Vascular dementia | 80 | |
| | 3 | Lewy body dementia | 159 | |
| | 4 | Frontotemporal lobar degeneration | 40 | |
| | 5 | Normal pressure hydrocephalus | 70 | |
| Non-dementia subjects | 6 | Subjects with normal cognitive functions | 110 | |
| Total | | | 1606 | |
| Each disease type number corresponds to the same numeral on the abscissa in Figs. 2 to 12. | | | | |

<Extraction of Total RNA>

Total RNA was obtained, using a reagent for RNA extraction in 3D-Gene (registered trademark) RNA extraction reagent from liquid sample kit (Toray Industries, Inc. (Japan)) according to the protocol provided by the manufacturer, from 300 μL of the serum sample obtained from each of the total of 1,606 people

<Measurement of Gene Expression Level>

MiRNA in the total RNA, which was obtained from the serum sample of each of the total of 1,606 people, was fluorescently labeled by use of 3D-Gene (registered trademark) miRNA Labeling kit (Toray Industries, Inc.) according to the protocol provided by the manufacturer. The oligo DNA chip used was 3D-Gene (registered trademark) Human miRNA Oligo chip (Toray Industries, Inc.) with attached probes having sequences complementary to 2,565 miRNAs among the miRNAs registered in miRBase Release 21. Hybridization under stringent conditions and washing following the hybridization were performed according to the protocol provided by the manufacturer. The DNA chip was scanned using 3D-Gene (registered trademark) scanner (Toray Industries, Inc.) to obtain images. Fluorescence intensity on the image was digitized using 3D-Gene (registered trademark) Extraction (Toray Industries, Inc.). The digitized fluorescence intensity was converted to a logarithmic value having a base of 2 and used as a gene expression level, from which a blank value was subtracted. A missing value was replaced with a signal value 0.1. As a result, the comprehensive gene expression levels of the miRNAs in the sera were obtained for the above 1,606 people. Subsequently, samples used for discriminant analysis of dementia were extracted. First, dementia patients were assigned to a positive group and non-dementia subjects were assigned to a negative group. Next, each cohort was sorted into a training cohort and a validation cohort as described in each Example below. Calculation and statistical analysis using the digitized gene expression levels of the miRNAs were carried out using R language 3.3.1 (R Core Team (2016) R: A language and environment for statistical computing. R Foundation for Statistical Computing, Vienna, Austria. URL https://www.R-project.org/.) and MASS package 7.3.45 (Venables, W.N. & Ripley, B.D. (2002) Modern Applied Statistics with S. Fourth Edition. Springer, New York. ISBN 0-387-95457-0).

[Example 1]

In Example 1, analysis was conducted by logistic regression using the LASSO method.

[Example 1-1: Search for Alzheimer's Dementia Markers]

To search for markers that can discriminate between patients with Alzheimer's dementia, a type of dementia and non-dementia subjects, 75 samples from the respective Alzheimer's dementia patients or non-dementia subjects were sorted into a training cohort (B in Table 3-1) and the rest samples were sorted into a validation cohort (C in Table 3-1).

[Table 3-1]

| Subject's disease type | | | A. All samples | | Example 1-1 | | Example 1-2 | |
|---|---|---|---|---|---|---|---|---|
| | | | | | B. Training samples | C. Validation samples | D. Training samples | E. Validation samples |
| Dementia patients | 1 | Alzheimer's dementia | 1147 | 1496 | 75 | 1072 | - | - |
| | 2 | Vascular dementia | 80 | | - | - | 50 | 30 |
| | 3 | Lewy body dementia | 159 | | - | - | - | - |
| | 4 | Frontotemporal lobar degeneration | 40 | | - | - | - | - |
| | 5 | Normal pressure hydrocephalus | 70 | | - | - | - | - |
| Non-dementia subjects | 6 | Subjects with normal cognitive functions | 110 | | 75 | 35 | 50 | 60 |
| Total | | | 1606 | | | | | |
| Each disease type number corresponds to the same numeral on the abscissa in Figs. 2 to 12. | | | | | | | | |

[Table 3-2]

| Subject's disease type | | | A. All samples | | Example 1-3 | | Example 1-4 | |
|---|---|---|---|---|---|---|---|---|
| | | | | | F Training samples | G. Validation samples | H. Training samples | I. Validation samples |
| Dementia patients | 1 | Alzheimer's dementia | 1147 | 1496 | - | - | - | - |
| | 2 | Vascular dementia | 80 | | 54 | 26 | - | - |
| | 3 | Lewy body dementia | 159 | | - | - | 75 | 84 |
| | 4 | Frontotemporal lobar degeneration | 40 | | - | - | - | - |
| | 5 | Normal pressure hydrocephalus | 70 | | - | - | - | - |
| Non-dementia subjects | 6 | Subjects with normal cognitive functions | 110 | | 74 | 36 | 75 | 35 |
| Total | | | 1606 | | | | | |
| Each disease type number corresponds to the same numeral on the abscissa in Figs. 2 to 12. | | | | | | | | |

[Table 3-3]

| Subject's disease type | | | A. All samples | | Example 1-5 | | Example 1-6 | |
|---|---|---|---|---|---|---|---|---|
| | | | | | J Training samples | K Validation samples | L. Training samples | M Validation samples |
| Dementia patients | 1 | Alzheimer's dementia | 1147 | 1496 | - | - | - | - |
| | 2 | Vascular dementia | 80 | | - | - | - | - |
| | 3 | Lewy body dementia | 159 | | 106 | 53 | - | - |
| | 4 | Frontotemporal lobar degeneration | 40 | | - | - | 30 | 10 |
| | 5 | Normal pressure hydrocephalus | 70 | | - | - | - | - |
| Non-dementia subjects | 6 | Subjects with normal cognitive functions | 110 | | 74 | 36 | 30 | 80 |
| Total | | | 1606 | | | | | |
| Each disease type number corresponds to the same numeral on the abscissa in Figs. 2 to 12. | | | | | | | | |

[Table 3-4]

| Subject's disease type | | | A. All samples | | Example 1-7 | | Example 1-8 | |
|---|---|---|---|---|---|---|---|---|
| | | | | | N Training samples | O Validation samples | P. Training samples | Q Validation samples |
| Dementia patients | 1 | Alzheimer's dementia | 1147 | 1496 | - | - | - | - |
| | 2 | Vascular dementia | 80 | | - | - | - | - |
| | 3 | Lewy body dementia | 159 | | - | - | - | - |
| | 4 | Frontotemporal lobar degeneration | 40 | | - | - | - | - |
| | 5 | Normal pressure hydrocephalus | 70 | | 50 | 20 | 47 | 23 |
| Non-dementia subjects | 6 | Subjects with normal cognitive functions | 110 | | 50 | 60 | 74 | 36 |
| Total | | | 1606 | | | | | |
| Each disease type number corresponds to the same numeral on the abscissa in Figs. 2 to 12. | | | | | | | | |

Meanwhile, in this Example, a discriminant formula with six gene markers was prepared using the above training cohort, and discriminant performance was then evaluated in the above validation cohort.

Specifically, first, miRNA expression levels obtained in the above Reference Example 1 were together normalized in accordance with quantile normalization. Further, in order to obtain a more highly reliable diagnostic marker, only 317 genes having a gene expression level of $2^6$ or more in 50% or more samples in either one of a group of dementia patients or a group of non-dementia subjects were analyzed. Next, a combination of the measurement values for the gene expression levels of 6 out of 317 miRNAs was subjected to logistic regression to construct a discriminant formula for discriminating the presence or absence of dementia. Further, this constructed discriminant formula was used to calculate the accuracy, the sensitivity, and the specificity in the validation cohort. Then, discriminant performance was validated in independent samples.

This resulted in a discriminant formula with an accuracy of 76%, a sensitivity of 77%, and a specificity of 49% in the validation cohort when the 6 miRNAs designated in Table 4 were used as discriminant markers (Table 4).

[Example 1-2: Search for Vascular Dementia Markers - 1]

To search for markers that can discriminate between patients with vascular dementia, a type of dementia and non-dementia subjects, 50 samples from the respective vascular dementia patients or non-dementia subjects were sorted into a training cohort (D in Table 3-1) and the rest samples were sorted into a validation cohort (E in Table 3-1).

Like Example 1-1, a combination of 13 miRNAs was used to construct a discriminant formula for discriminating the presence or absence of dementia, and the discriminant formula was validated.

This resulted in a discriminant formula with an accuracy of 79%, a sensitivity of 87%, and a specificity of 75% in the validation cohort when the 13 miRNAs designated in Table 4 were used as discriminant markers (Table 4).

[Example 1-3: Search for Vascular Dementia Markers - 2]

In order to sort as many samples as possible into a training cohort and search for markers with high accuracy, about 2/3 of samples from the respective vascular dementia patients or non-dementia subjects were sorted into a training cohort (F inTable 3-2) and the rest samples were sorted into a validation cohort (G inTable 3-2).

Like Example 1-1, a combination of 17 miRNAs was used to construct a discriminant formula for discriminating the presence or absence of dementia, and the discriminant formula was validated.

This resulted in a discriminant formula with an accuracy of 84%, a sensitivity of 73%, and a specificity of 92% in the validation cohort when the 17 miRNAs designated in Table 4 were used as discriminant markers (Table 4).

Sorting many samples into the training cohort resulted in the increased accuracy by 5% and the increased specificity by 17% compared to those in Example 1-2. However, because the number of positive samples was less than the number of negative samples in the training cohort, the sensitivity was decreased by 14%.

[Example 1-4: Search for Lewy Body Dementia Markers - 1]

To search for markers that can discriminate between patients with Lewy body dementia, a type of dementia and non-dementia subjects, 75 samples from the respective Lewy body dementia patients or non-dementia subjects were sorted into a training cohort (H in Table 3-2) and the rest samples were sorted into a validation cohort (I in Table 3-2).

Like Example 1-1, a combination of 4 miRNAs was used to construct a discriminantformula for discriminating the presence or absence of dementia, and the discriminant formula was validated.

This resulted in a discriminant formula with an accuracy of 82%, a sensitivity of 94%, and a specificity of 51% in the validation cohort when the 4 miRNAs designated in Table 4 were used as discriminant markers (Table 4).

[Example 1-5: Search for Lewy Body Dementia Markers - 2]

In order to sort as many samples as possible into a training cohort and search for markers with high accuracy, about 2/3 of samples from the respective Lewy body dementia patients or non-dementia subjects were sorted into a training cohort (J in Table 3-3) and the rest samples were sorted into a validation cohort (K in Table 3-3).

Like Example 1-1, a combination of 20 miRNAs was used to construct a discriminant formula for discriminating the presence or absence of dementia, and the discriminant formula was validated.

This resulted in a discriminant formula with an accuracy of 87%, a sensitivity of 96%, and a specificity of 72% in the validation cohort when the 20 miRNAs designated in Table 4 were used as discriminant markers (Table 4).

Sorting many samples into the training cohort resulted in the increased accuracy by 5%, the increased sensitivity by 2%, and the increased specificity by 21% compared to those in Example 1-4.

[Example 1-6: Search for Frontotemporal Lobar Degeneration Markers]

To search for markers that can discriminate between patients with frontotemporal lobar degeneration, a type of dementia and non-dementia subjects, 30 samples from the respective frontotemporal lobar degeneration patients or non-dementia subjects were sorted into a training cohort (L in Table 3-3) and the rest samples were sorted into a validation cohort (M in Table 3-3).

Like Example 1-1, a combination of 4 miRNAs was used to construct a discriminant formula for discriminating the presence or absence of dementia, and the discriminant formula was validated.

This resulted in a discriminant formula with an accuracy of 62%, a sensitivity of 90%, and a specificity of 59% in the validation cohort when the 4 miRNAs designated in Table 4 were used as discriminant markers (Table 4).

[Example 1-7: Search for Normal Pressure Hydrocephalus Markers - 1]

To search for markers that can discriminate between patients with normal pressure hydrocephalus, a type of dementia and non-dementia subjects, 30 samples from the respective normal pressure hydrocephalus patients or non-dementia subjects were sorted into a training cohort (N in Table 3-4) and the rest samples were sorted into a validation cohort (O in Table 3-4).

Like Example 1-1, a combination of 6 miRNAs was used to construct a discriminant formula for discriminating the presence or absence of dementia, and the discriminant formula was validated.

This resulted in a discriminant formula with an accuracy of 73%, a sensitivity of 75%, and a specificity of 72% in the validation cohort when the 6 miRNAs designated in Table 4 were used as discriminant markers (Table 4).

[Example 1-8: Search for Normal Pressure Hydrocephalus Markers - 2]

In order to sort as many samples as possible into a training cohort and search for markers with high accuracy, about 2/3 of samples from the respective normal pressure hydrocephalus patients or non-dementia subjects were sorted into a training cohort (P in Table 3-4) and the rest samples were sorted into a validation cohort (Q in Table 3-4).

Like Example 1-1, a combination of 18 miRNAs was used to construct a discriminant formula for discriminating the presence or absence of dementia, and the discriminant formula was validated.

This resulted in a discriminant formula with an accuracy of 78%, a sensitivity of 65%, and a specificity of 86% in the validation cohort when the 18 miRNAs designated in Table 4 were used as discriminant markers (Table 4).

[Table 4]

| Example No. | Number of miRNAs | SEQ ID NOs: | Training cohort | | | Validation cohort | | |
|---|---|---|---|---|---|---|---|---|
| | | | Accuracy (%) | Sensitivity (%) | Specificity (%) | Accuracy (%) | Sensitivity (%) | Specificity (%) |
| Example 1-1 | 6 | 3, 26, 54, 91, 99,206 | 76 | 81 | 71 | 76 | 77 | 49 |
| Example 1-2 | 13 | 4, 24, 25, 26, 28, 37, 45, 199, 201, 203, 204, 206, 216 | 87 | 90 | 84 | 79 | 87 | 75 |
| Example 1-3 | 17 | 3, 4, 23, 24, 26, 28, 32, 37, 45, 83, 88, 89, 199, 204, 206, 208, 216 | 84 | 78 | 89 | 84 | 73 | 92 |
| Example 1-4 | 4 | 3, 26, 90, 91 | 85 | 93 | 76 | 82 | 94 | 51 |
| Example 1-5 | 20 | 3, 4, 11, 26, 45, 55, 83, 91, 92, 93, 94, 95, 96, 97, 98, 196, 197, 204, 206, 210 | 87 | 99 | 70 | 87 | 96 | 72 |
| Example 1-6 | 4 | 26, 69, 135, 213 | 73 | 80 | 67 | 62 | 90 | 59 |

(continued)

| Example No. | Number of miRNAs | SEQ ID NOs: | Training cohort | | | Validation cohort | | |
|---|---|---|---|---|---|---|---|---|
| | | | Accuracy (%) | Sensitivity (%) | Specificity (%) | Accuracy (%) | Sensitivity (%) | Specificity (%) |
| Example 1-7 | 6 | 3, 26, 54, 203, 211, 212 | 79 | 90 | 68 | 73 | 75 | 72 |
| Example 1-8 | 18 | 3, 26, 45, 52, 54, 88, 94, 98, 140, 141, 142, 143, 144, 200, 203, 205, 211, 212 | 86 | 81 | 89 | 78 | 65 | 86 |

Sorting many samples into the training cohort resulted in the increased accuracy by 5% and the increased specificity by 14% compared to those in Example 1-7. However, because the number of positive samples was less than the number of negative samples in the training cohort, the sensitivity was decreased by 10%.

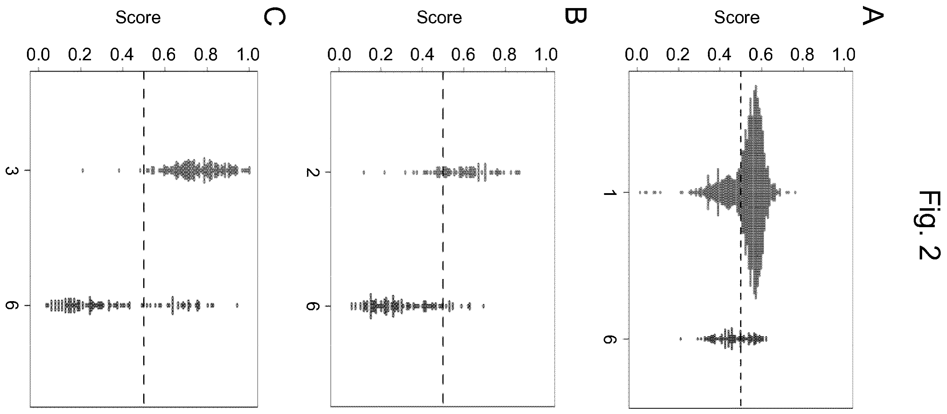

Fig. 2 shows plots of discriminant scores obtained in Examples 1-1, 1-3, and 1-5. Fig. 2A is a plot of discriminant scores for Alzheimer's dementia as described in Example 1-1; Fig. 2B is a plot of discriminant scores for vascular dementia as described in Example 1-3; and Fig. 2C is a plot of discriminant scores for Lewy body dementia as described in Example 1-5. The discriminant scores range from 0 to 1. The dotted line in each panel depicts a discriminant boundary (a discriminant score of 0.5) that discriminates the presence or absence of dementia. The measurement values for the expression levels of the above 6 (Fig. 2A), 17 (Fig. 2B), or 20 (Fig. 2C) miRNAs in sera from dementia patients or non-dementia subjects selected as each training cohort were used to prepare a discriminant formula by logistic regression analysis using the LASSO method. The ordinate represents a discriminant score obtained from the discriminant formula, and the abscissa represents each cohort.

On the abscissa of Fig. 2, the numeral 1 represents Alzheimer's dementia, the numeral 2 represents vascular dementia, the numeral 3 represents Lewy body dementia, and the numeral 6 represents non-dementia subjects (the disease type numbers in Table 2 and 3 are the same as the numerals on the abscissa in each panel).

[Example 2]

In Example 1, a plurality of marker sets having excellent discriminant performance having a sensitivity or a specificity of more than 80% were obtained. Thus, in Example 2, deepening Example 1, a goal was set to investigate whether or not any marker set having both sensitivity and specificity of more than 70% or 80% was obtained by varying the cohort sorting.

Accordingly, samples from each of the positive group or the negative group were divided into 5 groups, and 4/5 of the divided groups were assigned to a training cohort and 1/5 thereof was assigned to a validation cohort. The validation cohort was changed five times. Whenever the validation cohort was changed, a discriminant formula was constructed using the training cohort like Example 1-1 by logistic regression using the LASSO method. Then, the performance was evaluated on the validation cohort. Further, the dividing methods were changed five times.

[Example 2-1: Search for Alzheimer's Dementia Markers]

One thousand and one hundred forty seven (1,147) samples from patients with Alzheimer's dementia, a type of dementia, and 110 samples from non-dementia subjects were used for analysis. As a result, any discriminant formula with both sensitivity and specificity of more than 80% in the validation cohort was not obtained, but 3 different discriminant formulae with both sensitivity and specificity of more than 70% were obtained.

In the case of using, as the 1st discriminant markers, 10 miRNAs designated in Table 5, a discriminant formula with an AUC of 0.824 and a sensitivity of 81% and a specificity of 79% in the validation cohort was obtained.

In the case of using, as the 2nd discriminant markers, 12 miRNAs designated in Table 5, a discriminant formula with an AUC of 0.803 and a sensitivity of 80% and a specificity of 74% in the validation cohort was obtained.

In the case of using, as the 3rd discriminant markers, 19 miRNAs designated in Table 5, a discriminant formula with an AUC of 0.831 and a sensitivity of 76% and a specificity of 77% in the validation cohort was obtained (Table 5).

Varying the cohort made it possible to discover discriminant markers that made the sensitivity comparable or higher and the specificity improved by maximum 30% compared to that in Example 1-1.

[Example 2-2: Search for Vascular Dementia Markers]

Eighty (80) samples from patients with vascular dementia, a type of dementia, and 110 samples from non-dementia subjects were used for analysis. As a result, 8 different discriminant formulae with sensitivity and specificity of more than 80% in the validation cohort were obtained.

In the case of using, as the 4th discriminant markers, 8 miRNAs designated in Table 5, a discriminant formula with an AUC of 0.892 and a sensitivity of 83% and a specificity of 80% in the validation cohort was obtained.

In the case of using, as the 5th discriminant markers, 10 miRNAs designated in Table 5, a discriminant formula with an AUC of 0.939 and a sensitivity of 87% and a specificity of 91% in the validation cohort was obtained.

In the case of using, as the 6th discriminant markers, 18 miRNAs designated in Table 5, a discriminant formula with an AUC of 0.929 and a sensitivity of 80% and a specificity of 86% in the validation cohort was obtained.

In the case of using, as the 7th discriminant markers, 15 miRNAs designated in Table 5, a discriminant formula with an AUC of 0.898 and a sensitivity of 92% and a specificity of 80% in the validation cohort was obtained.

In the case of using, as the 8th discriminant markers, 14 miRNAs designated in Table 5, a discriminant formula with an AUC of 0.900 and a sensitivity of 83% and a specificity of 85% in the validation cohort was obtained.

In the case of using, as the 9th discriminant markers, 11 miRNAs designated in Table 5, a discriminant formula with an AUC of 0.950 and a sensitivity of 82% and a specificity of % in the validation cohort was obtained.

In the case of using, as the 10th discriminant markers, 11 miRNAs designated in Table 5, a discriminant formula with an AUC of 0.810 and a sensitivity of 82% and a specificity of 81% in the validation cohort was obtained.

In the case of using, as the 11th discriminant markers, 13 miRNAs designated in Table 5, a discriminant formula with an AUC of 0.884 and a sensitivity of 81% and a specificity of 86% in the validation cohort was obtained (Table 5).

In Examples 1-2 and 1-3, either the sensitivity or the specificity was 80% or less. However, varying the cohort made it possible to discover discriminant markers with both the sensitivity and the specificity more than 80%.

[Example 2-3: Search for Lewy Body Dementia Markers]

One hundred fifty nine (159) samples from patients with Lewy body dementia, a type of dementia, and 110 samples from non-dementia subjects were used for analysis. As a result, 6 different discriminant formulae with sensitivity and specificity of more than 80% in the validation cohort were obtained.

In the case of using, as the 12th discriminant markers, 17 miRNAs designated in Table 5, a discriminant formula with an AUC of 0.929 and a sensitivity of 94% and a specificity of 80% in the validation cohort was obtained.

In the case of using, as the 13th discriminant markers, 14 miRNAs designated in Table 5, a discriminant formula with an AUC of 0.927 and a sensitivity of 93% and a specificity of 85% in the validation cohort was obtained.

In the case of using, as the 14th discriminant markers, 18 miRNAs designated in Table 5, a discriminant formula with an AUC of 0.879 and a sensitivity of 87% and a specificity of 83% in the validation cohort was obtained.

In the case of using, as the 15th discriminant markers, 28 miRNAs designated in Table 5, a discriminant formula with an AUC of 0.885 and a sensitivity of 84% and a specificity of 83% in the validation cohort was obtained.

In the case of using, as the 16th discriminant markers, 18 miRNAs designated in Table 5, a discriminant formula with an AUC of 0.890 and a sensitivity of 88% and a specificity of 80% in the validation cohort was obtained.

In the case of using, as the 17th discriminant markers, 16 miRNAs designated in Table 5, a discriminant formula with an AUC of 0.872 and a sensitivity of 85% and a specificity of 82% in the validation cohort was obtained (Table 5).

In Examples 1-4 and 1-5, the specificity was 80% or less. However, varying the cohort made it possible to discover discriminant markers with both the sensitivity and the specificity more than 80%.

[Example 2-4: Search for Frontotemporal Lobar Degeneration Markers]

Fouty (40) samples from patients with frontotemporal lobar degeneration, a type of dementia, and 110 samples from non-dementia subjects were used for analysis. As a result, any discriminant formula with both sensitivity and specificity of more than 80% in the validation cohort was not obtained, but 2 different discriminant formulae with both sensitivity and specificity of more than 70% were obtained.

In the case of using, as the 18th discriminant markers, 3 miRNAs designated in Table 5, a discriminant formula with an AUC of 0.886 and a sensitivity of 88% and a specificity of 77% in the validation cohort was obtained.

In the case of using, as the 19th discriminant markers, 12 miRNAs designated in Table 5, a discriminant formula with an AUC of 0.784 and a sensitivity of 75% and a specificity of 73% in the validation cohort was obtained (Table 5).

In Example 1-6, the specificity was as low as 59%. However, varying the cohort made it possible to discover discriminant markers with both the sensitivity and the specificity more than 70%.

[Example 2-5: Search for Normal Pressure Hydrocephalus Markers]

Seventy (70) samples from patients with frontotemporal lobar degeneration, a type of dementia, and 110 samples from non-dementia subjects were used for analysis. As a result, 3 different discriminant formulae with sensitivity and specificity of more than 80% in the validation cohort were obtained.

In the case of using, as the 20th discriminant markers, 6 miRNAs designated in Table 5, a discriminant formula with an AUC of 0.894 and a sensitivity of 88% and a specificity of 85% in the validation cohort was obtained.

In the case of using, as the 21st discriminant markers, 6 miRNAs designated in Table 5, a discriminant formula with an AUC of 0.903 and a sensitivity of 100% and a specificity of 82% in the validation cohort was obtained.

In the case of using, as the 22nd discriminant markers, 8 miRNAs designated in Table 5, a discriminant formula with an AUC of 0.816 and a sensitivity of 81% and a specificity of 80% in the validation cohort was obtained (Table 5).

[Table 5]

| Example No. | | Number of miRNAs | SEQ ID NOs | AUC | Sensitivity | Specificity |
|---|---|---|---|---|---|---|
| Example 2-1 | 1st | 10 | 1, 2, 3, 4, 5, 6, 7, 8, 200, 214 | 0.824 | 81 | 79 |
| | 2nd | 12 | 1, 2, 3, 4, 5, 9, 10, 11, 12, 13, 214, 215 | 0.803 | 80 | 74 |
| | 3rd | 19 | 3, 4, 5, 6, 7, 8, 10, 13, 14, 15, 16, 17, 18, 19, 20, 21, 22, 200, 214 | 0.831 | 76 | 77 |
| Example 2-2 | 4th | 8 | 4, 16, 23, 24, 25, 26, 197, 216 | 0.892 | 83 | 80 |
| | 5th | 10 | 3, 4, 16, 23, 26, 27, 28, 29, 214, 217 | 0.939 | 87 | 91 |
| | 6th | 18 | 4, 16, 24, 26, 29, 30, 31, 32, 33, 34, 35, 36, 37, 197, 201, 214, 217, 218 | 0.929 | 80 | 86 |
| | 7th | 15 | 3, 4, 16, 25, 26, 28, 34, 37, 38, 39, 40, 197, 207, 214, 218 | 0.898 | 92 | 80 |
| | 8th | 14 | 4, 16, 23, 24, 26, 28, 29, 32, 36, 37, 41, 42, 207, 214 | 0.900 | 83 | 85 |
| | 9th | 11 | 4, 16, 23, 24, 26, 36, 37, 42, 207, 214, 217 | 0.950 | 82 | 91 |
| | 10th | 11 | 3,4, 23, 25, 26, 28, 36, 43, 214, 216, 218 | 0.810 | 82 | 81 |
| | 11th | 13 | 3, 4, 16, 25, 26, 28, 29, 36, 44, 45, 197, 217, 218 | 0.884 | 81 | 86 |
| Example 2-3 | 12th | 17 | 3, 4, 16, 26, 29, 45, 46, 47, 48, 49, 50, 51, 52, 53, 214, 216, 218 | 0.929 | 94 | 80 |
| | 13th | 14 | 3, 4, 11, 16, 26, 29, 45, 48, 49, 54, 55, 200, 214, 219 | 0.927 | 93 | 85 |
| | 14th | 18 | 3, 11, 16, 26, 29, 45, 46, 48, 49, 52, 55, 56, 57, 194, 209, 214, 216, 220 | 0.879 | 87 | 83 |
| | 15th | 28 | 3, 4, 11, 16, 26, 29, 30, 36, 46, 47, 48, 58, 59, 60, 61, 62, 63, 64, 65, 66, 197, 200, 207, 209, 216, 219, 220, 221 | 0.885 | 84 | 83 |
| | 16th | 18 | 3, 13, 16, 26, 29, 45, 47, 48, 49, 54, 60, 64, 194, 214, 216, 219, 220, 222 | 0.890 | 88 | 80 |
| | 17th | 16 | 3, 8, 16, 26, 33, 45, 48, 49, 54, 55, 67, 68, 194, 214, 219, 220 | 0.872 | 85 | 82 |

(continued)

| Example No. | | Number of miRNAs | SEQ ID NOs | AUC | Sensitivity | Specificity |
|---|---|---|---|---|---|---|
| Example 2-4 | 18th | 3 | 26, 69, 70 | 0.886 | 88 | 77 |
| | 19th | 12 | 26, 30, 37, 53, 70, 71, 72, 73, 74, 223, 224, 225 | 0.784 | 75 | 73 |
| Example 2-5 | 20th | 6 | 3, 11, 16, 26, 75, 226 | 0.894 | 88 | 85 |
| | 21st | 6 | 3, 26, 34, 75, 76, 226 | 0.903 | 100 | 82 |
| | 22nd | 8 | 3, 11, 26, 34, 52, 75, 203, 220 | 0.816 | 81 | 80 |

In Examples 1-7 and 1-8, the sensitivity was 80% or less. However, varying the cohort made it possible to discover discriminant markers with both the sensitivity and the specificity more than 80%.

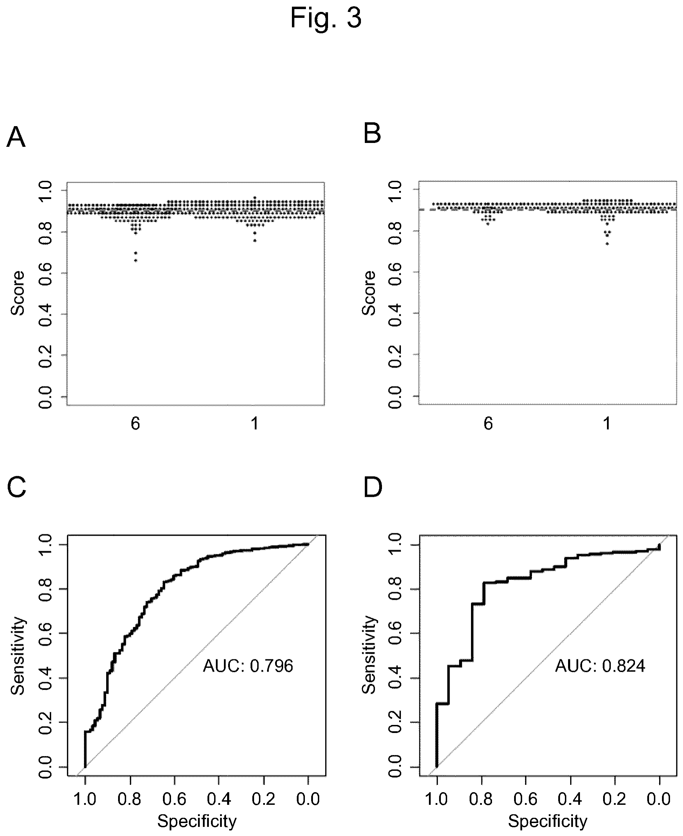

Figs. 3 to 7 show plots of discriminant scores and ROC (Receiver Operating Characteristic) curves as obtained in Examples 2-1 to 2-5.

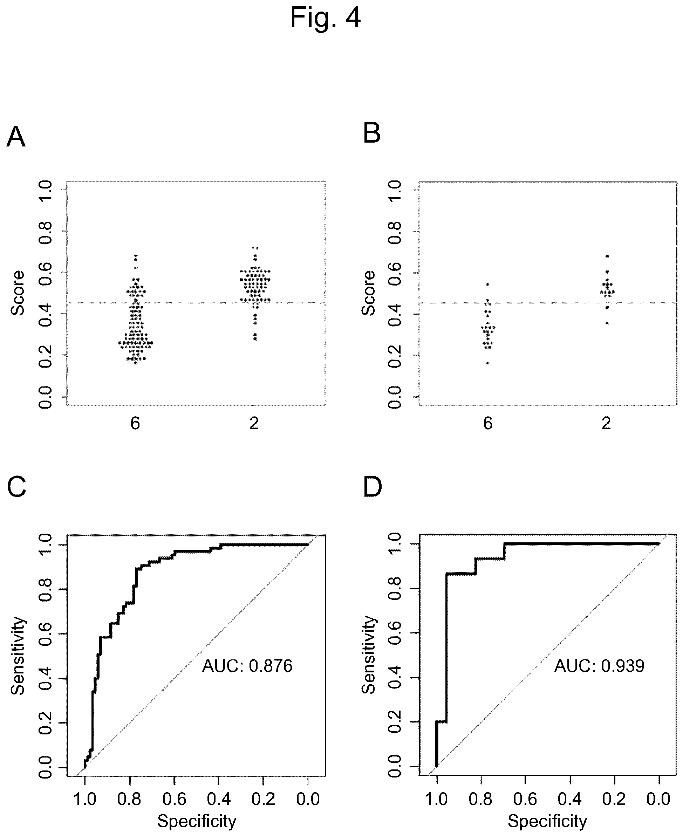

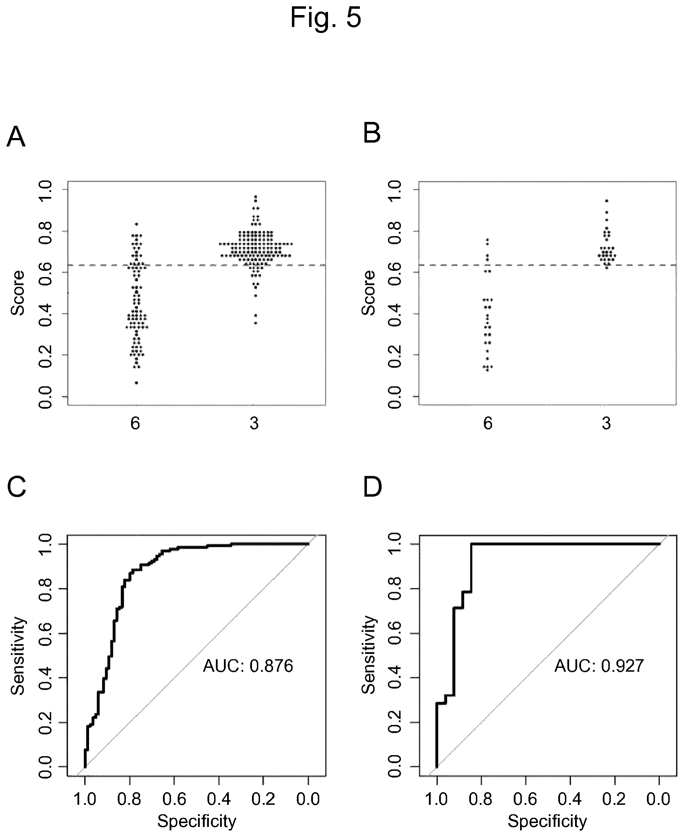

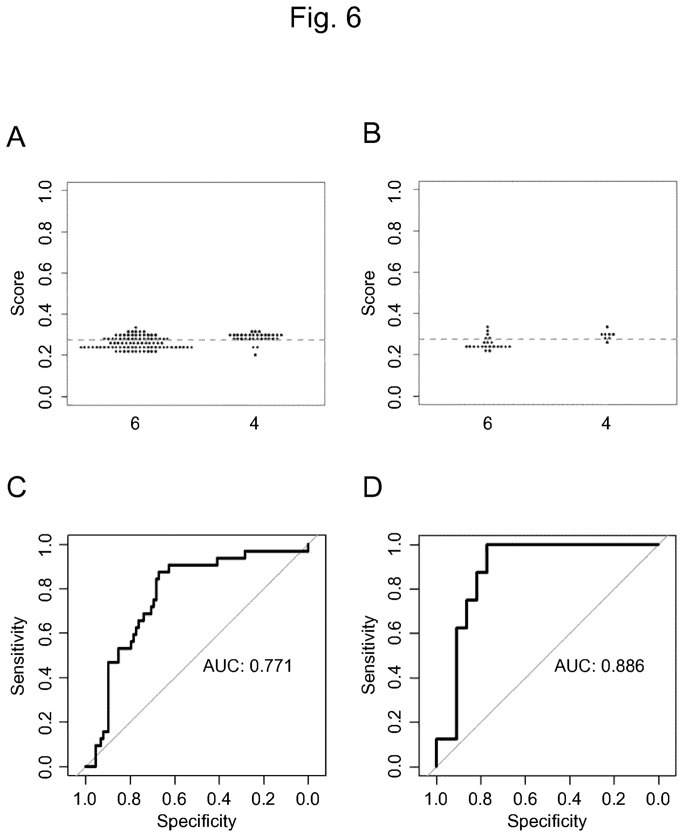

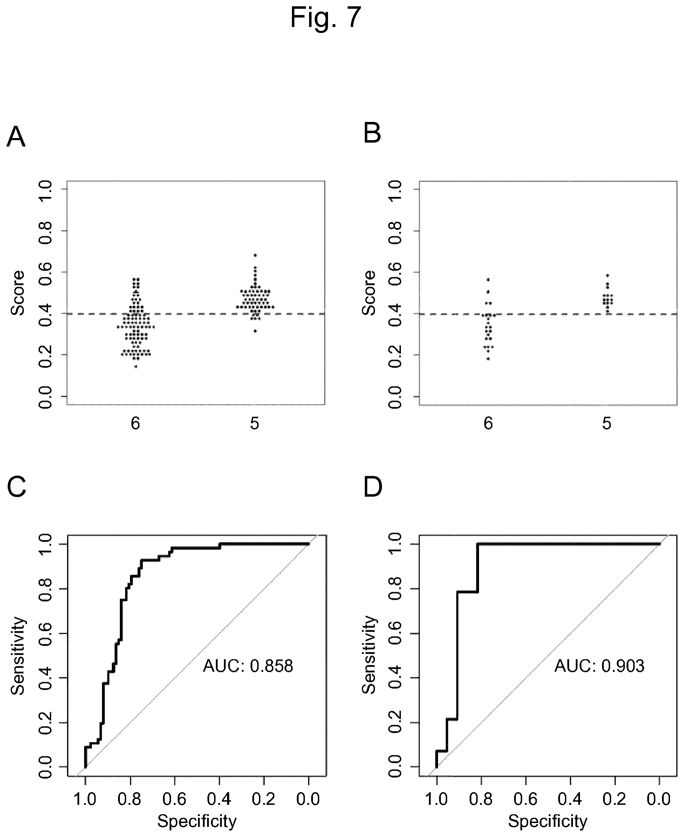

Fig. 3 shows plots of discriminant scores and ROC curves for Alzheimer's dementia described in Example 2-1. Fig. 4 shows plots of discriminant scores and ROC curves for vascular dementia described in Example 2-2. Fig. 5 shows plots of discriminant scores and ROC curves for Lewy body dementia described in Example 2-3. Fig. 6 shows plots of discriminant scores and ROC curves for frontotemporal lobar degeneration described in Example 2-4. Fig. 7 shows plots of discriminant scores and ROC curves for normal pressure hydrocephalus described in Example 2-5. In each figure, panel A is a plot of discriminant scores in a training cohort; panel B is a plot of discriminant scores in a validation cohort; panel C is a ROC curve for the training cohort; and panel D is a ROC curve for the validation cohort.

The discriminant scores in each plot of discriminant scores range from 0 to 1. The dotted line in each panel depicts a discriminant boundary that discriminates the presence or absence of dementia. Logistic regression analysis using the LASSO method was used to prepare a discriminant formula from the measurement values for the expression levels of miRNAs that are the 1st (Fig. 3), the 5th (Fig. 4), the 13th (Fig. 5), the 18th (Fig. 6), or the 21st (Fig. 7) discriminant markers in sera from dementia patients and non-dementia subjects selected as the training cohort. The ordinate represents a discriminant score obtained from the discriminant formula, and the abscissa represents each cohort.

MiRNA markers obtained in each Example were used to calculate the sensitivity as the ordinate and the specificity as the abscissa expressed in each ROC curve. Early detection and early treatment of dementia require diagnosis without misidentification. Meanwhile, once diagnosed as dementia, the disease patients receive a large social impact. Accordingly, it is also important to be able to give a diagnosis with good accuracy and without false positives. At the time of selection of a discriminant formula and markers for discriminating the presence or absence of dementia, a desired combination can be selected, depending on prioritized performance, from the ROC curves shown in Figs. 3 to 7.

On the abscissa of Figs. 3 to 7, the numeral 1 represents Alzheimer's dementia, the numeral 2 represents vascular dementia, the numeral 3 represents Lewy body dementia, the numeral 4 represents frontotemporal lobar degeneration, the numeral 5 represents normal pressure hydrocephalus, and the numeral 6 represents non-dementia subjects (the disease type numbers in Table 2 and 3 are the same as the numerals on the abscissa in each panel).

[Example 3]

Example 3 had the following two objects.

Object 1: To conduct analysis, as a supplement of Example 2, by deepening Example 1 by varying, like Example 2, the cohort sorting while the statistical technique was changed from that in Example 2.
Object 2: To examine whether a marker set with sensitivity and specificity of more than 70% or 80% was obtained even when the number of markers used in the discriminant formula was reduced to 5.

Specifically, using a training cohort, the resulting markers with high correlation were excluded. Next, every combination of 5 out of the top 20 markers ranked by the P value was analyzed by Fisher's discriminant analysis to construct a discriminant formula. Then, the performance was evaluated in a validation cohort. The P value was calculated as follows. First, miRNA expression levels in dementia patients and non-dementia subjects as obtained in the above Reference Example 1 were together normalized in accordance with quantile normalization. Next, in order to evaluate a more highly reliable diagnostic marker, only a gene having a gene expression level of $2^6$ or more in 50% or more samples in either one of a group of dementia patients or a group of non-dementia subjects was selected. Further, to evaluate a gene having a statistically significant difference in the gene expression level between the group of dementia patients and the group of non-dementia subjects, an equal-variance-assumed two-sided t test was conducted to calculate the P value, which was corrected by Bonferroni correction. Table 6 lists the top markers ranked by the P value and used in the discriminant formula (Table 6).

[Table 6]

| SEQ ID NO | Name of gene | Selection Count |
|---|---|---|
| 26 | hsa-miR-6088 | 53 |
| 3 | hsa-miR-4728-5p | 43 |
| 4 | hsa-miR-4443 | 39 |
| 214 | hsa-miR-211-5p | 34 |
| 49 | hsa-miR-5088-5p | 27 |
| 16 | hsa-miR-4539 | 26 |
| 11 | hsa-miR-6729-3p | 23 |
| 8 | hsa-miR-3184-3p | 20 |
| 37 | hsa-miR-4725-3p | 17 |
| 70 | hsa-miR-1470 | 14 |
| 75 | hsa-miR-1914-5p | 14 |
| 30 | hsa-miR-4658 | 12 |
| 226 | hsa-miR-1915-5p | 12 |
| 30 | hsa-miR-4658 | 12 |
| 200 | hsa-miR-6743-5p | 10 |
| 52 | hsa-miR-4734 | 10 |
| 45 | hsa-miR-6729-5p | 10 |
| 34 | hsa-miR-6777-3p | 10 |
| 83 | hsa-miR-4488 | 9 |
| 5 | hsa-miR-4506 | 9 |
| 203 | hsa-miR-4651 | 9 |
| 54 | hsa-miR-1249-3p | 8 |
| 220 | hsa-miR-150-5p | 7 |
| 115 | hsa-miR-378d | 7 |
| 10 | hsa-miR-320d | 6 |
| 218 | hsa-miR-744-5p | 6 |
| 99 | hsa-miR-1202 | 5 |
| 32 | hsa-miR-1260b | 5 |
| 236 | hsa-miR-328-3p | 5 |
| 23 | hsa-miR-4486 | 5 |
| 116 | hsa-miR-4701-5p | 5 |
| 69 | hsa-miR-6087 | 5 |
| 85 | hsa-miR-6731-3p | 5 |
| 43 | hsa-miR-6766-5p | 5 |

(continued)

| SEQ ID NO | Name of gene | Selection Count |
|---|---|---|
| 121 | hsa-miR-6769b-3p | 5 |
| 36 | hsa-miR-6832-3p | 5 |
| 13 | hsa-miR-6853-5p | 5 |
| 228 | hsa-miR-137 | 4 |
| 227 | hsa-miR-204-5p | 4 |
| 98 | hsa-miR-3178 | 4 |
| 9 | hsa-miR-4281 | 4 |
| 207 | hsa-miR-6075 | 4 |
| 234 | hsa-miR-1224-3p | 3 |
| 78 | hsa-miR-1289 | 3 |
| 20 | hsa-miR-1539 | 3 |
| 219 | hsa-miR-345-5p | 3 |
| 80 | hsa-miR-3666 | 3 |
| 1 | hsa-miR-4274 | 3 |
| 28 | hsa-miR-4505 | 3 |
| 112 | hsa-miR-4716-5p | 3 |
| 24 | hsa-miR-1227-5p | 2 |
| 114 | hsa-miR-1268b | 2 |
| 126 | hsa-miR-143-5p | 2 |
| 217 | hsa-miR-149-5p | 2 |
| 136 | hsa-miR-193b-3p | 2 |
| 79 | hsa-miR-4433a-5p | 2 |
| 84 | hsa-miR-4474-3p | 2 |
| 25 | hsa-miR-4667-5p | 2 |
| 82 | hsa-miR-4725-5p | 2 |
| 101 | hsa-miR-4731-3p | 2 |
| 122 | hsa-miR-520a-3p | 2 |
| 89 | hsa-miR-638 | 2 |
| 129 | hsa-miR-6842-3p | 2 |
| 128 | hsa-miR-6889-3p | 2 |
| 104 | hsa-miR-8072 | 2 |
| 53 | hsa-miR-936 | 2 |
| 100 | hsa-miR-6790-5p | 1 |
| 102 | hsa-miR-2681-3p | 1 |
| 87 | hsa-miR-202-5p | 1 |
| 229 | hsa-miR-382-5p | 1 |
| 103 | hsa-miR-6758-5p | 1 |
| 230 | hsa-miR-517-5p | 1 |
| 47 | hsa-miR-519e-5p | 1 |

(continued)

| SEQ ID NO | Name of gene | Selection Count |
|---|---|---|
| 105 | hsa-miR-518d-3p | 1 |
| 231 | hsa-miR-532-5p | 1 |
| 72 | hsa-miR-3150a-5p | 1 |
| 106 | hsa-miR-3606-3p | 1 |
| 77 | hsa-miR-15b-5p | 1 |
| 41 | hsa-miR-4312 | 1 |
| 233 | hsa-miR-1237-3p | 1 |
| 109 | hsa-miR-6784-3p | 1 |
| 110 | hsa-miR-4450 | 1 |
| 29 | hsa-miR-548q | 1 |
| 111 | hsa-miR-6132 | 1 |
| 94 | hsa-miR-3928-3p | 1 |
| 42 | hsa-miR-4461 | 1 |
| 198 | hsa-miR-6781-5p | 1 |
| 201 | hsa-miR-6726-5p | 1 |
| 113 | hsa-miR-6860 | 1 |
| 48 | hsa-miR-512-5p | 1 |
| 235 | hsa-miR-625-3p | 1 |
| 46 | hsa-miR-526b-3p | 1 |
| 237 | hsa-miR-122-5p | 1 |
| 117 | hsa-miR-4329 | 1 |
| 118 | hsa-miR-185-3p | 1 |
| 33 | hsa-miR-3677-5p | 1 |
| 119 | hsa-miR-552-3p | 1 |
| 120 | hsa-miR-1273$_9$-5p | 1 |
| 123 | hsa-miR-4524b-5p | 1 |
| 238 | hsa-miR-202-3p | 1 |
| 124 | hsa-miR-4291 | 1 |
| 125 | hsa-miR-6734-3p | 1 |
| 239 | hsa-miR-4781-5p | 1 |
| 127 | hsa-miR-939-3p | 1 |
| 240 | hsa-miR-718 | 1 |
| 241 | hsa-miR-342-3p | 1 |
| 130 | hsa-miR-4511 | 1 |
| 131 | hsa-miR-4318 | 1 |
| 242 | hsa-miR-26b-3p | 1 |
| 81 | hsa-miR-3186-3p | 1 |
| 243 | hsa-miR-140-3p | 1 |
| 132 | hsa-miR-4653-5p | 1 |

(continued)

| SEQ ID NO | Name of gene | Selection Count |
|---|---|---|
| 133 | hsa-miR-6867-3p | 1 |
| 244 | hsa-miR-200a-3p | 1 |
| 134 | hsa-miR-133b | 1 |
| 245 | hsa-miR-378a-3p | 1 |
| 135 | hsa-miR-3196 | 1 |
| 137 | hsa-miR-3162-3p | 1 |
| 194 | hsa-miR-5698 | 1 |
| 66 | hsa-miR-6509-3p | 1 |
| 246 | hsa-miR-484 | 1 |
| 138 | hsa-miR-6819-3p | 1 |
| 139 | hsa-miR-1908-3p | 1 |
| 86 | hsa-miR-4640-3p | 1 |
| 215 | hsa-miR-1247-5p | 1 |

[Example 3-1: Search for Alzheimer's Dementia Markers]

One thousand and one hundred forty seven (1,147) samples from patients with Alzheimer's dementia, a type of dementia, and 110 samples from non-dementia subjects were used for analysis. As a result, any discriminant formula with both sensitivity and specificity of more than 80% in the validation cohort was not obtained, but 4 different discriminant formulae with both sensitivity and specificity of more than 70% were obtained.

In the case of using, as the 23rd discriminant markers, 5 miRNAs designated in Table 7, a discriminant formula with an AUC of 0.845 and a sensitivity of 76% and a specificity of 74% in the validation cohort was obtained.

In the case of using, as the 24th discriminant markers, 5 miRNAs designated in Table 7, a discriminant formula with an AUC of 0.763 and a sensitivity of 79% and a specificity of 76% in the validation cohort was obtained.

In the case of using, as the 25th discriminant markers, 5 miRNAs designated in Table 7, a discriminant formula with an AUC of 0.804 and a sensitivity of 76% and a specificity of 75% in the validation cohort was obtained.

In the case of using, as the 26th discriminant markers, 5 miRNAs designated in Table 7, a discriminant formula with an AUC of 0.755 and a sensitivity of 75% and a specificity of 74% in the validation cohort was obtained (Table 7).

In Example 1-1, the specificity was as low as 49%. However, varying the cohort made it possible to discover discriminant markers with both the sensitivity and the specificity more than 70% even when the number of markers was reduced to 5.

[Example 3-2: Search for Vascular Dementia Markers]

EIghty (80) samples from patients with vascular dementia, a type of dementia, and 110 samples from non-dementia subjects were used for analysis. As a result, 2 different discriminant formulae with sensitivity and specificity of more than 80% in the validation cohort were obtained.

In the case of using, as the 27th discriminant markers, 5 miRNAs designated in Table 7, a discriminant formula with an AUC of 0.942 and a sensitivity of 89% and a specificity of 80% in the validation cohort was obtained.

In the case of using, as the 28th discriminant markers, 5 miRNAs designated in Table 7, a discriminant formula with an AUC of 0.938 and a sensitivity of 88% and a specificity of 100% in the validation cohort was obtained (Table 7).

In Examples 1-2 and 1-3, either the sensitivity or the specificity was 80% or less. However, varying the cohort made it possible to discover discriminant markers with both the sensitivity and the specificity more than 80% even when the number of markers was reduced to 5.

[Example 3-3: Search for Lewy Body Dementia Markers]

One hundred fifty nine (159) samples from patients with Lewy body dementia, a type of dementia, and 110 samples from non-dementia subjects were used for analysis. As a result, 2 different discriminant formulae with sensitivity and specificity of more than 80% in the validation cohort were obtained.

In the case of using, as the 29th discriminant markers, 5 miRNAs designated in Table 7, a discriminant formula with an AUC of 0.864 and a sensitivity of 87% and a specificity of 83% in the validation cohort was obtained.

In the case of using, as the 30th discriminant markers, 5 miRNAs designated in Table 7, a discriminant formula with an AUC of 0.862 and a sensitivity of 85% and a specificity of 80% in the validation cohort was obtained (Table 7).

In Examples 1-4 and 1-5, the specificity was 80% or less. However, varying the cohort made it possible to discover discriminant markers with both the sensitivity and the specificity more than 80% even when the number of markers was reduced to 5.

[Example 3-4: Search for Frontotemporal Lobar Degeneration Markers]

Forty (40) samples from patients with frontotemporal lobar degeneration, a type of dementia, and 110 samples from non-dementia subjects were used for analysis. As a result, any discriminant formula with both sensitivity and specificity of more than 80% in the validation cohort was not obtained, but 2 different discriminant formulae with both sensitivity and specificity of more than 70% were obtained.

In the case of using, as the 31st discriminant markers, 5 miRNAs designated in Table 7, a discriminant formula with an AUC of 0.801 and a sensitivity of 88% and a specificity of 73% in the validation cohort was obtained.

In the case of using, as the 32nd discriminant markers, 5 miRNAs designated in Table 7, a discriminant formula with an AUC of 0.630 and a sensitivity of 70% and a specificity of 70% in the validation cohort was obtained (Table 7).

In Example 1-6, the specificity was as low as 59%. However, varying the cohort made it possible to discover discriminant markers with both the sensitivity and the specificity more than 70% even when the number of markers was reduced to 5.

[Example 3-5: Search for Normal Pressure Hydrocephalus Markers]

Seventy (70) samples from patients with normal pressure hydrocephalus, a type of dementia, and 110 samples from non-dementia subjects were used for analysis. As a result, 3 different discriminant formulae with sensitivity and specificity of more than 80% in the validation cohort were obtained.

In the case of using, as the 33rd discriminant markers, 5 miRNAs designated in Table 7, a discriminant formula with an AUC of 0.897 and a sensitivity of 81% and a specificity of 95% in the validation cohort was obtained.

In the case of using, as the 34th discriminant markers, 5 miRNAs designated in Table 7, a discriminant formula with an AUC of 0.867 and a sensitivity of 87% and a specificity of 86% in the validation cohort was obtained.

In the case of using, as the 35th discriminant markers, 5 miRNAs designated in Table 7, a discriminant formula with an AUC of 0.948 and a sensitivity of 100% and a specificity of 94% in the validation cohort was obtained (Table 7).

[Table 7]

| Example No. | | Number of miRNAs | SEQ ID NOs | AUC | Sensitivity | Specificity |
|---|---|---|---|---|---|---|
| Example 3-1 | 23rd | 5 | 3, 4, 11, 16, 214 | 0.845 | 76 | 74 |
| | 24th | 5 | 3, 4, 11, 49, 214 | 0.763 | 79 | 76 |
| | 25th | 5 | 4, 5, 9, 11, 72 | 0.804 | 76 | 75 |
| | 26th | 5 | 1, 3, 49, 77, 78 | 0.755 | 75 | 74 |
| Example 3-2 | 27th | 5 | 8, 16, 26, 79, 218 | 0.942 | 89 | 80 |
| | 28th | 5 | 4, 16, 26, 36, 80 | 0.938 | 88 | 100 |
| Example 3-3 | 29th | 5 | 26, 37, 45, 46, 54 | 0.864 | 87 | 83 |
| | 30th | 5 | 26, 37, 45, 49, 54 | 0.862 | 85 | 80 |
| Example 3-4 | 31st | 5 | 10, 13, 37, 70, 227 | 0.801 | 88 | 73 |
| | 32nd | 5 | 10, 81, 82, 200, 228 | 0.630 | 70 | 70 |
| Example 3-5 | 33rd | 5 | 34, 49, 83, 203, 226 | 0.897 | 81 | 95 |
| | 34th | 5 | 3, 8, 75, 84, 226 | 0.867 | 87 | 86 |
| | 35th | 5 | 3, 8, 75, 85, 86 | 0.948 | 100 | 94 |

In Examples 1-7 and 1-8, the sensitivity was 80% or less. However, varying the cohort made it possible to discover discriminant markers with both the sensitivity and the specificity more than 80% even when the number of markers was reduced to 5.

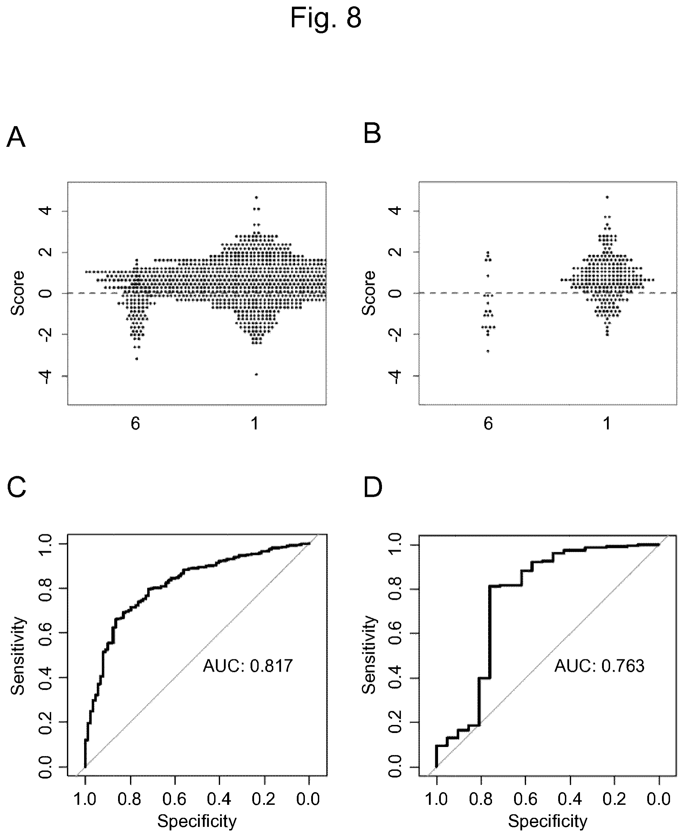

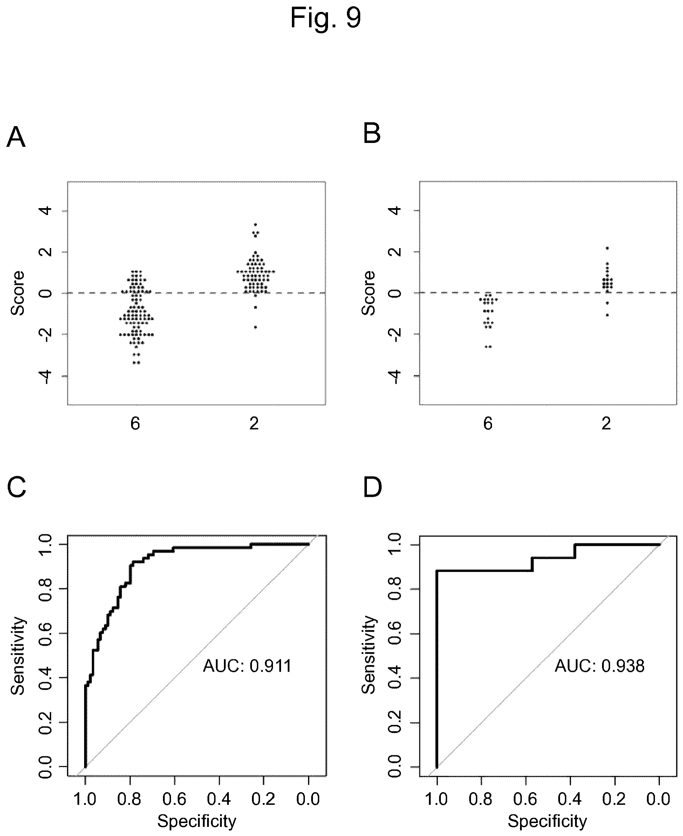

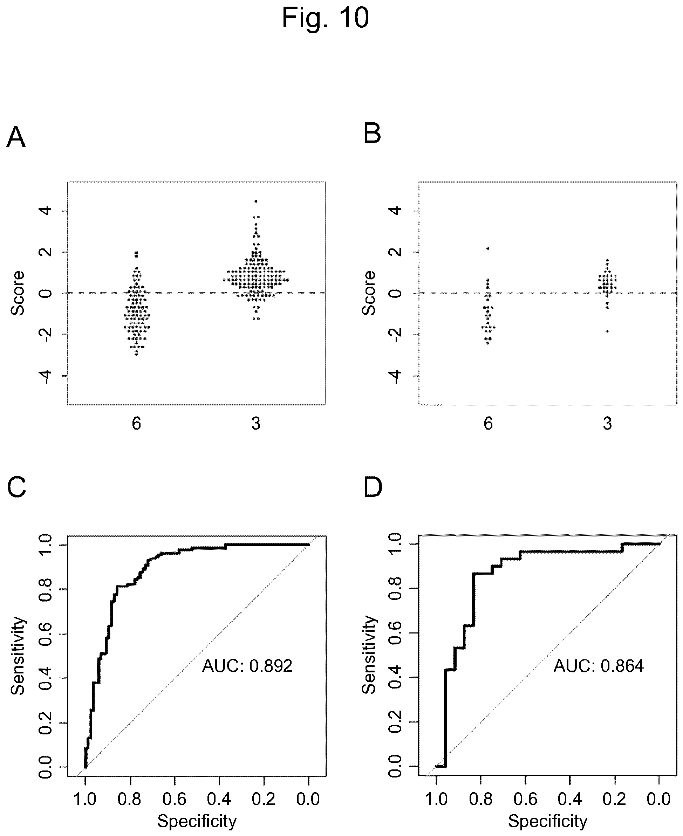

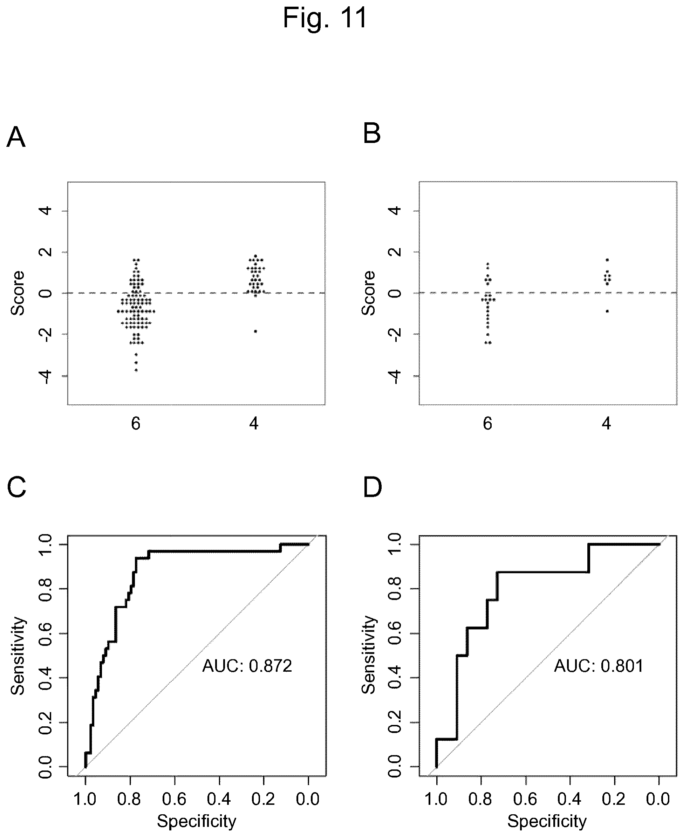

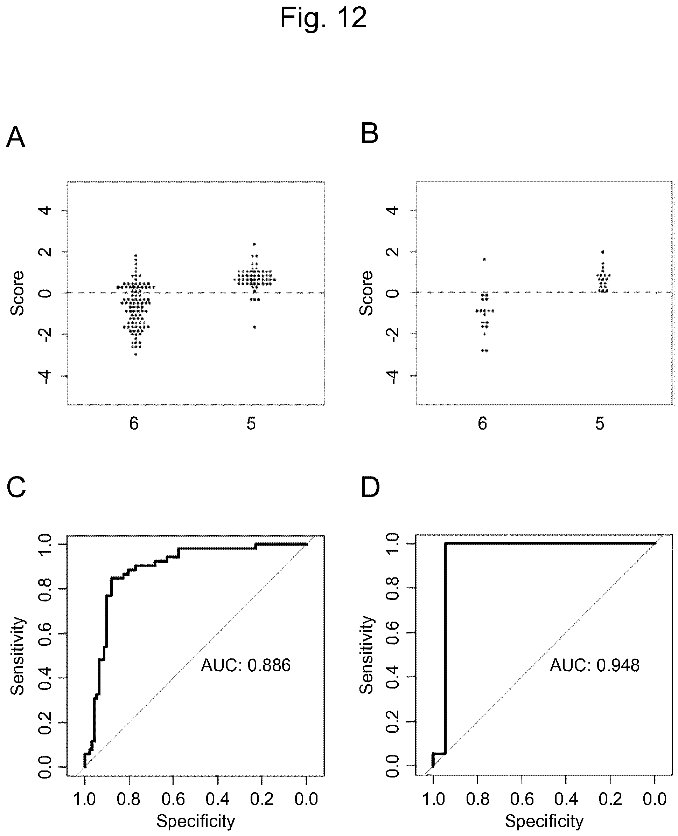

Figs. 8 to 12 show plots of discriminant scores and ROC curves as obtained in Examples 3-1 to 3-5. Fig. 8 shows plots of discriminant scores and ROC curves for Alzheimer's dementia described in Example 3-1. Fig. 9 shows plots of discriminant scores and ROC curves for vascular dementia described in Example 3-2. Fig. 10 shows plots of discriminant scores and ROC curves for Lewy body dementia described in Example 3-3. Fig. 11 shows plots of discriminant scores and ROC curves for frontotemporal lobar degeneration described in Example 3-4. Fig. 12 shows plots of discriminant scores and ROC curves for normal pressure hydrocephalus described in Example 3-5. In each figure, panel A is a plot of discriminant scores in a training cohort; panel B is a plot of discriminant scores in a validation cohort; panel C is a ROC curve for the training cohort; and panel D is a ROC curve for the validation cohort.

The discriminant scores in each plot of discriminant scores range from -5 to 5. The dotted line (a discriminant score of 0) in each panel depicts a discriminant boundary that discriminates the presence or absence of dementia. The discriminant scores were calculated by assigning the measurement values for the miRNA expression levels corresponding to each sample to a discriminant formula prepared using the above respective 5 miRNAs (the respective 24th, 28th, 29th, 31st, or 35th discriminant markers in Examples 3-1 to 3-5). The ordinate represents a discriminant score obtained, and the abscissa represents each cohort. The numerals on the abscissa in Figs. 8 to 12 indicate the dementia disease types, which are the same as in Figs. 3 to 7 (the disease type numbers in Table 2 and 3 are the same as the numerals on the abscissa in each panel).

MiRNA markers obtained in each Example were used to calculate the sensitivity as the ordinate and the specificity as the abscissa expressed in each ROC curve. Early detection and early treatment of dementia require diagnosis without misidentification. Meanwhile, once diagnosed as dementia, the disease patients receive a large social impact. Accordingly, it is also important to be able to give a diagnosis with good accuracy and without false positives. At the time of selection of a discriminant formula and markers for discriminating the presence or absence of dementia, a desired combination can be selected, depending on prioritized performance, from the ROC curves shown in Figs. 8 to 12.

[Example 4]

In Example 4, the examination of the object 2 in Example 3 was further deepened. The object is to examine the validation when the number of markers used in the discriminant formula was reduced to 3. Specifically, using a training cohort, the resulting markers with high correlation were excluded. Next, every combination of 3 out of the top 30 markers ranked by the P value was analyzed by Fisher's discriminant analysis to construct a discriminant formula. Then, the performance was evaluated in a validation cohort. Otherwise, the same procedure as in Example 3 was repeated.

[Example 4-1: Search for Alzheimer's Dementia Markers]

One thousand and one hundred forty seven (1,147) samples from patients with Alzheimer's dementia, a type of dementia, and 110 samples from non-dementia subjects were used for analysis. Table 8 shows the resulting various 25 different (the 36th to the 60th) combinations and their discriminant performance. Note that only the 55th combination gave a discriminant formula with sensitivity and specificity of more than 70% in the validation cohort (Table 8).

When combinations of 5 markers described in Example 3-1 were used, 4 different discriminant formulae with sensitivity and specificity of more than 70% in the validation cohort were obtained. Even when the number of markers was reduced to 3, some marker combination in the present invention was demonstrated to make it possible to exceed target performance.

[Example 4-2: Search for Vascular Dementia Markers]

Eighty (80) samples from patients with vascular dementia, a type of dementia, and 110 samples from non-dementia subjects were used for analysis. Table 8 shows the resulting various 25 different (the 61st to the 85th) combinations and their discriminant performance. Note that the 80th and 81st combinations gave 2 discriminant formulae with sensitivity and specificity of more than 80% in the validation cohort (Table 8).

When combinations of 5 markers described in Example 3-2 were used, 2 different discriminant formulae with sensitivity and specificity of more than 80% in the validation cohort were obtained. Even when the number of markers was reduced to 3, some marker combination in the present invention was demonstrated to make it possible to exceed target performance.

[Example 4-3: Search for Lewy Body Dementia Markers]

One hundred fifty nine (159) samples from patients with Lewy body dementia, a type of dementia, and 110 samples from non-dementia subjects were used for analysis. Table 8 shows the resulting various 25 different (the 86th to

the 110th) combinations and their discriminant performance. Note that the 87th and 101st combinations gave 2 discriminant formulae with sensitivity and specificity of more than 80% in the validation cohort (Table 8).

When combinations of 5 markers described in Example 3-3 were used, 2 different discriminant formulae with sensitivity and specificity of more than 80% in the validation cohort were obtained. Even when the number of markers was reduced to 3, some marker combination in the present invention was demonstrated to make it possible to exceed target performance.

[Example 4-4: Search for Frontotemporal Lobar Degeneration Markers]

Forty (40) samples from patients with frontotemporal lobar degeneration, a type of dementia, and 110 samples from non-dementia subjects were used for analysis. Table 8 shows the resulting various 25 different (the 111st to the 135th) combinations and their discriminant performance. Note that only the 133rd combination gave a discriminant formula with sensitivity and specificity of more than 70% in the validation cohort (Table 8).

When combinations of 5 markers described in Example 3-4 were used, 2 different discriminant formulae with sensitivity and specificity of more than 70% in the validation cohort were obtained. Even when the number of markers was reduced to 3, some marker combination in the present invention was demonstrated to make it possible to exceed target performance.

[Example 4-5: Search for Normal Pressure Hydrocephalus Markers]

Seventy (70) samples from patients with normal pressure hydrocephalus, a type of dementia, and 110 samples from non-dementia subjects were used for analysis. Table 8 shows the resulting various 25 different (the 136th to the 160th) combinations and their discriminant performance. Note that the 139th, 145th, 147th, 153rd, and 158th combinations gave 5 discriminant formulae with sensitivity and specificity of more than 80% in the validation cohort (Table 8).

[Table 8]

| Example No. | | Number of miRNAs | SEQ ID NOs | AUC | Sensitivity | Specificity |
|---|---|---|---|---|---|---|
| Example 4-1 | 36th | 3 | 26, 49, 101 | 0.673 | 74 | 62 |
| | 37th | 3 | 3, 4, 5 | 0.662 | 71 | 50 |
| | 38th | 3 | 3, 20, 49 | 0.679 | 76 | 48 |
| | 39th | 3 | 3, 16, 214 | 0.788 | 75 | 68 |
| | 40th | 3 | 3, 9, 11 | 0.683 | 79 | 37 |
| | 41st | 3 | 3, 16, 214 | 0.707 | 74 | 67 |
| | 42nd | 3 | 3, 49, 214 | 0.746 | 75 | 67 |
| | 43rd | 3 | 26, 49, 101 | 0.726 | 72 | 59 |
| | 44th | 3 | 3, 20, 49 | 0.705 | 77 | 60 |
| | 45th | 3 | 8,145,214 | 0.677 | 67 | 58 |
| | 46th | 3 | 3, 49, 215 | 0.753 | 76 | 68 |
| | 47th | 3 | 3, 20, 49 | 0.814 | 78 | 65 |
| | 48th | 3 | 26, 49, 101 | 0.683 | 71 | 56 |
| | 49th | 3 | 3, 49, 104 | 0.706 | 78 | 63 |
| | 50th | 3 | 8, 16, 20 | 0.610 | 72 | 47 |
| | 51st | 3 | 3, 49, 101 | 0.753 | 78 | 64 |
| | 52nd | 3 | 26, 49, 101 | 0.695 | 70 | 61 |
| | 53rd | 3 | 3, 16, 214 | 0.798 | 77 | 69 |
| | 54th | 3 | 23, 26, 214 | 0.664 | 69 | 67 |
| | 55th | 3 | 3, 11, 49 | 0.775 | 77 | 75 |
| | 56th | 3 | 3, 11, 49 | 0.737 | 78 | 64 |
| | 57th | 3 | 1, 3, 49 | 0.750 | 78 | 59 |

(continued)

| Example No. | | Number of miRNAs | SEQ ID NOs | AUC | Sensitivity | Specificity |
|---|---|---|---|---|---|---|
| | 58th | 3 | 8, 49, 214 | 0.664 | 76 | 68 |
| | 59th | 3 | 5, 8, 16 | 0.668 | 74 | 61 |
| | 60th | 3 | 11,146,200 | 0.672 | 68 | 63 |
| Example 4-2 | 61st | 3 | 26,197,218 | 0.886 | 78 | 90 |
| | 62nd | 3 | 16, 26, 32 | 0.832 | 81 | 77 |
| | 63rd | 3 | 26, 203, 218 | 0.745 | 87 | 57 |
| | 64th | 3 | 16, 26, 217 | 0.910 | 87 | 78 |
| | 65th | 3 | 26, 29, 111 | 0.872 | 81 | 73 |
| | 66th | 3 | 26, 28, 233 | 0.800 | 72 | 65 |
| | 67th | 3 | 4, 26, 34 | 0.867 | 78 | 75 |
| | 68th | 3 | 16, 26, 218 | 0.869 | 89 | 75 |
| | 69th | 3 | 16, 26, 218 | 0.907 | 90 | 75 |
| | 70th | 3 | 26, 203, 218 | 0.795 | 69 | 82 |
| | 71st | 3 | 16, 26, 218 | 0.859 | 95 | 74 |
| | 72nd | 3 | 16, 26, 111 | 0.788 | 93 | 57 |
| | 73rd | 3 | 16, 26, 30 | 0.818 | 71 | 79 |
| | 74th | 3 | 16, 26, 32 | 0.893 | 93 | 65 |
| | 75th | 3 | 16, 26, 197 | 0.835 | 59 | 86 |
| | 76th | 3 | 26, 203, 218 | 0.765 | 73 | 61 |
| | 77th | 3 | 26, 217, 218 | 0.832 | 77 | 67 |
| | 78th | 3 | 3, 4, 16 | 0.803 | 92 | 48 |
| | 79th | 3 | 16, 26, 45 | 0.888 | 71 | 91 |
| | 80th | 3 | 26, 28, 42 | 0.903 | 83 | 85 |
| | 81st | 3 | 16, 26, 42 | 0.924 | 88 | 81 |
| | 82nd | 3 | 26, 43, 141 | 0.716 | 73 | 69 |
| | 83rd | 3 | 16, 26, 32 | 0.810 | 88 | 64 |
| | 84th | 3 | 26, 203, 218 | 0.805 | 67 | 76 |
| | 85th | 3 | 26, 28, 217 | 0.864 | 88 | 64 |
| Example 4-3 | 86th | 3 | 11, 26, 49 | 0.753 | 86 | 52 |
| | 87th | 3 | 3, 16, 45 | 0.907 | 88 | 85 |
| | 88th | 3 | 3, 197, 219 | 0.722 | 70 | 71 |
| | 89th | 3 | 3, 16, 26 | 0.740 | 95 | 44 |
| | 90th | 3 | 3, 114, 220 | 0.775 | 79 | 64 |
| | 91st | 3 | 26, 87, 207 | 0.793 | 81 | 65 |
| | 92nd | 3 | 26, 32, 49 | 0.853 | 85 | 75 |
| | 93rd | 3 | 26, 214, 247 | 0.844 | 68 | 83 |
| | 94th | 3 | 26, 54, 220 | 0.815 | 92 | 63 |
| | 95th | 3 | 3, 4, 52 | 0.680 | 77 | 39 |
| | 96th | 3 | 3, 16, 26 | 0.841 | 89 | 68 |

(continued)

| Example No. | | Number of miRNAs | SEQ ID NOs | AUC | Sensitivity | Specificity |
|---|---|---|---|---|---|---|
| | 97th | 3 | 3, 29, 52 | 0.849 | 89 | 77 |
| | 98th | 3 | 26, 47, 52 | 0.681 | 71 | 53 |
| | 99th | 3 | 3, 45, 49 | 0.782 | 88 | 62 |
| | 100th | 3 | 3, 4, 116 | 0.772 | 76 | 68 |
| | 101st | 3 | 26, 32, 46 | 0.849 | 87 | 83 |
| | 102nd | 3 | 3, 49, 220 | 0.693 | 88 | 37 |
| | 103rd | 3 | 26, 45, 214 | 0.756 | 83 | 71 |
| | 104th | 3 | 26, 207, 221 | 0.850 | 77 | 78 |
| | 105th | 3 | 3, 16, 26 | 0.878 | 88 | 75 |
| | 106th | 3 | 26, 47, 207 | 0.803 | 69 | 63 |
| | 107th | 3 | 26, 214, 247 | 0.871 | 94 | 70 |
| | 108th | 3 | 3, 16, 45 | 0.857 | 85 | 74 |
| | 109th | 3 | 26, 45, 220 | 0.688 | 76 | 62 |
| | 110th | 3 | 26, 32, 49 | 0.878 | 83 | 74 |
| Example 4-4 | 111th | 3 | 30, 70, 147 | 0.758 | 86 | 61 |
| | 112th | 3 | 26, 116, 117 | 0.646 | 46 | 63 |
| | 113th | 3 | 69, 148, 236 | 0.835 | 100 | 46 |
| | 114th | 3 | 3, 33, 70 | 0.566 | 56 | 62 |
| | 115th | 3 | 69, 121, 148 | 0.608 | 20 | 84 |
| | 116th | 3 | 69, 98, 236 | 0.704 | 80 | 64 |
| | 117th | 3 | 30, 69, 227 | 0.574 | 50 | 59 |
| | 118th | 3 | 3, 70, 217 | 0.693 | 56 | 86 |
| | 119th | 3 | 115, 121, 238 | 0.605 | 60 | 45 |
| | 120th | 3 | 26, 70, 125 | 0.631 | 50 | 73 |
| | 121 st | 3 | 26, 120, 121 | 0.795 | 63 | 82 |
| | 122nd | 3 | 30, 70, 227 | 0.622 | 46 | 74 |
| | 123rd | 3 | 98, 99, 202 | 0.683 | 75 | 50 |
| | 124th | 3 | 3, 33, 121 | 0.661 | 67 | 57 |
| | 125th | 3 | 10, 30, 70 | 0.716 | 75 | 55 |
| | 126th | 3 | 37, 70, 128 | 0.503 | 57 | 52 |
| | 127th | 3 | 26, 30, 242 | 0.653 | 63 | 77 |
| | 128th | 3 | 82, 83, 120 | 0.600 | 60 | 70 |
| | 129th | 3 | 70, 99, 243 | 0.571 | 57 | 65 |
| | 130th | 3 | 43, 70, 121 | 0.659 | 63 | 73 |
| | 131st | 3 | 30, 37, 227 | 0.642 | 67 | 56 |
| | 132nd | 3 | 30, 70, 133 | 0.702 | 86 | 65 |
| | 133rd | 3 | 43, 70, 121 | 0.869 | 77 | 94 |
| | 134th | 3 | 37, 149, 227 | 0.761 | 50 | 77 |
| | 135th | 3 | 26, 43, 248 | 0.593 | 56 | 67 |

(continued)

| Example No. | | Number of miRNAs | SEQ ID NOs | AUC | Sensitivity | Specificity |
|---|---|---|---|---|---|---|
| Example 4-5 | 136th | 3 | 49, 99, 150 | 0.720 | 64 | 68 |
| | 137th | 3 | 34, 83, 226 | 0.841 | 75 | 75 |
| | 138th | 3 | 8, 75, 203 | 0.775 | 93 | 62 |
| | 139th | 3 | 3, 34, 49 | 0.906 | 86 | 82 |
| | 140th | 3 | 8, 49, 75 | 0.867 | 64 | 91 |
| | 141st | 3 | 26, 34, 49 | 0.850 | 81 | 70 |
| | 142nd | 3 | 3, 49, 85 | 0.756 | 93 | 59 |
| | 143rd | 3 | 3, 34, 226 | 0.708 | 83 | 58 |
| | 144th | 3 | 8, 75, 85 | 0.809 | 85 | 65 |
| | 145th | 3 | 8, 75, 83 | 0.870 | 93 | 81 |
| | 146th | 3 | 3, 10, 49 | 0.765 | 89 | 74 |
| | 147th | 3 | 3, 34, 49 | 0.884 | 80 | 88 |
| | 148th | 3 | 26, 75, 104 | 0.796 | 69 | 65 |
| | 149th | 3 | 8, 75, 203 | 0.829 | 87 | 67 |
| | 150th | 3 | 8, 75, 151 | 0.756 | 85 | 65 |
| | 151st | 3 | 26, 75, 104 | 0.833 | 73 | 68 |
| | 152nd | 3 | 3, 10, 246 | 0.741 | 75 | 65 |
| | 153rd | 3 | 3, 34, 49 | 0.897 | 94 | 80 |
| | 154th | 3 | 1, 49, 83 | 0.732 | 62 | 65 |
| | 155th | 3 | 8, 49, 75 | 0.873 | 57 | 86 |
| | 156th | 3 | 8, 75, 203 | 0.722 | 88 | 50 |
| | 157th | 3 | 34, 83, 226 | 0.724 | 93 | 50 |
| | 158th | 3 | 3, 34, 49 | 0.951 | 89 | 94 |
| | 159th | 3 | 3, 34, 49 | 0.854 | 90 | 62 |
| | 160th | 3 | 34, 37, 49 | 0.757 | 75 | 75 |

When combinations of 5 markers described in Example 3-5 were used, 3 different discriminant formulae with sensitivity and specificity of more than 80% in the validation cohort were obtained. Even when the number of markers was reduced to 3, some marker combination in the present invention was demonstrated to make it possible to give a large number of combinations exhibiting performance exceeding target discriminant performance.

[Example 5]

In Example 5, the examination in Examples 3 and 4 was further deepened in order to examine the variation when the number of markers used in the discriminant formula was increased to 8. Specifically, like Example 2, samples from each of the positive group or the negative group were divided into 5 groups. Then, 4/5 of the divided groups were assigned to a training cohort and 1/5 thereof was assigned to a validation cohort. The validation cohort was changed five times. Whenever the validation cohort was changed, a discriminant formula was constructed using the training cohort like Example 1-1 by logistic regression using the LASSO method. Then, the performance was evaluated on the validation cohort. Further, the dividing method was changed five times.

[Example 5-1: Search for Lewy Body Dementia Markers]

One hundred fifty nine (159) samples from patients with Lewy body dementia, a type of dementia, and 110 samples from non-dementia subjects were used for analysis. Table 9 shows the resulting 3 different (the 161st to the 163rd)

combinations and their discriminant performance. All the 3 combinations each gave a discriminant formula with sensitivity and specificity of more than 70% in the validation cohort (Table 9).

[Example 5-2: Search for Frontotemporal Lobar Degeneration Markers]

Forty (40) samples from patients with frontotemporal lobar degeneration, a type of dementia, and 110 samples from non-dementia subjects were used for analysis. Table 9 shows the resulting 6 different (the 164th to the 169th) combinations and their discriminant performance. The 165th, 166th, and 167th combinations gave 3 different discriminant formulae with sensitivity and specificity of more than 70% in the validation cohort (Table 9).

[Example 5-3: Search for Normal Pressure Hydrocephalus Markers]

Seventy (70) samples from patients with normal pressure hydrocephalus, a type of dementia, and 110 samples from non-dementia subjects were used for analysis. Table 9 shows the resulting 3 different (the 170th to the 172nd) combinations and their discriminant performance. Only the 171st combination gave a discriminant formula with sensitivity and specificity of more than 70% in the validation cohort (Table 9).

[Table 9]

| Example No. | | Number of miRNAs | SEQ ID NOs | AUC | Sensitivity | Specificity |
|---|---|---|---|---|---|---|
| Example 5-1 | 161st | 8 | 3, 4, 16, 26, 49, 54, 207, 219 | 0.854 | 77 | 84 |
| | 162nd | 8 | 3, 4, 16, 26, 49, 53, 194, 214 | 0.846 | 91 | 70 |
| | 163rd | 8 | 3, 4, 26, 49, 194, 214, 219, 220 | 0.858 | 90 | 74 |
| Example 5-2 | 164th | 8 | 26, 70, 117, 147, 160, 161, 197, 225 | 0.679 | 36 | 74 |
| | 165th | 8 | 30, 69, 70, 71, 73, 121, 248, 249 | 0.784 | 100 | 72 |
| | 166th | 8 | 26, 37, 69, 70, 71, 72, 171, 248 | 0.892 | 75 | 91 |
| | 167th | 8 | 26, 30, 70, 71, 72, 175, 193, 197 | 0.841 | 88 | 77 |
| | 168th | 8 | 3, 26, 37, 70, 72, 153, 176, 177 | 0.509 | 43 | 61 |
| | 169th | 8 | 13, 26, 69, 70, 71, 181, 197, 223 | 0.652 | 71 | 61 |
| Example 5-3 | 170th | 8 | 3, 8, 34, 66, 75, 136, 184/185, 203 | 0.806 | 69 | 83 |
| | 171st | 8 | 3, 8, 26, 75, 84, 186, 220, 226 | 0.879 | 73 | 86 |
| | 172nd | 8 | 3, 8, 11, 26, 75, 203, 220, 226 | 0.781 | 94 | 60 |

[Example 6]

In Example 6, the examination in Examples 3 to 5 was further deepened, in order to examine the variation when the number of markers used in the discriminant formula was increased to 9. Specifically, substantially the same procedure as in Example 5 was repeated.

[Example 6-1: Search for Alzheimer's Dementia Markers]

One thousand and one hundred forty seven (1,147) samples from patients with Alzheimer's dementia, a type of dementia, and 110 samples from non-dementia subjects were used for analysis. Table 10 shows the resulting 6 different (the 173rd to the 178th) combinations and their discriminant performance. Regarding the 5 different 173rd, 175th, 176th, 177th, and 178th combinations, some marker set variations with either sensitivity or specificity of more than 70% in the validation cohort were found (Table 10).

[Example 6-2: Search for Vascular Dementia Markers]

Eighty (80) samples from patients with vascular dementia, a type of dementia, and 110 samples from non-dementia subjects were used for analysis. Table 10 shows the resulting 4 different (the 179th to the 182nd) combinations

and their discriminant performance. Regarding the 2 different 179th and 181st combinations, marker set variations with either sensitivity or specificity of more than 80% in the validation cohort were found (Table 10).

[Example 6-3: Search for Lewy Body Dementia Markers]

One hundred fifty nine (159) samples from patients with Lewy body dementia, a type of dementia, and 110 samples from non-dementia subjects were used for analysis. Table 10 shows the resulting 8 different (the 183rd to the 190th) combinations and their discriminant performance. Among them, regarding the 2 different 187th and 190th combinations, marker set variations with sensitivity and specificity of more than 80% in the validation cohort were found (Table 10).

[Example 6-4: Search for Frontotemporal Lobar Degeneration Markers]

Forty (40) samples from patients with frontotemporal lobar degeneration, a type of dementia, and 110 samples from non-dementia subjects were used for analysis. Table 10 shows the resulting 3 different (the 191st to the 193rd) combinations and their discriminant performance. Regarding all the 3 different combinations, marker set variations with either sensitivity or specificity of more than 70% were found (Table 10).

[Example 6-5: Search for Normal Pressure Hydrocephalus Markers]

Seventy (70) samples from patients with normal pressure hydrocephalus, a type of dementia, and 110 samples from non-dementia subjects were used for analysis. Table 10 shows the resulting 8 different (the 194th to the 201st) combinations and their discriminant performance. Among them, regarding the 3 different 194th, 195th, and 198th combinations, marker set variations with sensitivity and specificity of more than 80% in the validation cohort were found (Table 10).

[Table 10]

| Example No. | | Number of miRNAs | SEQ ID NOs | AUC | Sensitivity | Specificity |
|---|---|---|---|---|---|---|
| Example 6-1 | 173rd | 9 | 2, 3, 4, 5, 8, 9, 13, 49, 214 | 0.743 | 71 | 67 |
| | 174th | 9 | 4, 5, 8, 11, 20, 90, 155, 200, 214 | 0.634 | 65 | 65 |
| | 175th | 9 | 3, 4, 5, 7, 8, 9, 20, 200, 214 | 0.699 | 79 | 56 |
| | 176th | 9 | 3, 4, 5, 7, 8, 9, 10, 20, 214 | 0.782 | 80 | 63 |
| | 177th | 9 | 3, 4, 5, 7, 9, 13, 20, 49, 155 | 0.750 | 78 | 55 |
| | 178th | 9 | 1, 3, 4, 5, 8, 10, 11, 152, 200 | 0.714 | 75 | 61 |
| Example 6-2 | 179th | 9 | 3, 16, 23, 26, 32, 45, 89, 110, 218 | 0.833 | 89 | 75 |
| | 180th | 9 | 3, 4, 16, 23, 26, 34, 89, 111, 217 | 0.771 | 60 | 74 |
| | 181st | 9 | 16, 23, 25, 26, 28, 37, 203, 207, 218 | 0.885 | 65 | 95 |
| | 182nd | 9 | 4, 23, 26, 28, 45, 197, 203, 214, 218 | 0.745 | 67 | 74 |
| Example 6-3 | 183rd | 9 | 3, 11, 26, 49, 134, 194, 200, 214, 234 | 0.744 | 89 | 60 |
| | 184th | 9 | 3, 13, 16, 26, 49, 64, 68, 113, 214 | 0.709 | 90 | 56 |
| | 185th | 9 | 3, 16, 26, 29, 49, 52, 53, 214, 220 | 0.844 | 87 | 71 |
| | 186th | 9 | 1, 3, 13, 26, 49, 54, 200, 214, 220 | 0.858 | 96 | 70 |

(continued)

| Example No. | | Number of miRNAs | SEQ ID NOs | AUC | Sensitivity | Specificity |
|---|---|---|---|---|---|---|
| | 187th | 9 | 3, 4, 26, 29, 45, 49, 54, 55, 214 | 0.896 | 93 | 85 |
| | 188th | 9 | 3, 26, 36, 45, 49, 54, 60, 214, 219 | 0.811 | 88 | 67 |
| | 189th | 9 | 3, 4, 26, 49, 194, 200, 214, 219, 220 | 0.703 | 88 | 53 |
| | 190th | 9 | 3, 13, 16, 26, 45, 49, 194, 214, 219 | 0.881 | 94 | 80 |
| Example 6-4 | 191st | 9 | 69, 70, 72, 167, 173, 200, 202, 225, 248 | 0.654 | 75 | 65 |
| | 192nd | 9 | 37, 43, 70, 71, 121, 200, 225, 227, 249 | 0.653 | 50 | 73 |
| | 193rd | 9 | 24, 26, 43, 69, 70, 71,163, 197, 248 | 0.720 | 78 | 48 |
| Example 6-5 | 194th | 9 | 3, 11, 16, 26, 34, 75, 104, 134, 226 | 0.891 | 81 | 85 |
| | 195th | 9 | 3, 26, 34, 75, 76, 104, 136, 215, 226 | 0.899 | 93 | 82 |
| | 196th | 9 | 3, 26, 34, 75, 84, 104, 203, 226, 230 | 0.799 | 77 | 78 |
| | 197th | 9 | 3, 8, 26, 75, 104, 151, 203, 220, 226 | 0.826 | 77 | 78 |
| | 198th | 9 | 3, 11, 26, 34, 45, 52, 75, 203, 220 | 0.806 | 81 | 80 |
| | 199th | 9 | 3, 16, 26, 41, 49, 75, 83, 136, 225 | 0.753 | 85 | 70 |
| | 200th | 9 | 3, 16, 26, 34, 45, 75, 104, 189, 190 | 0.935 | 72 | 94 |
| | 201st | 9 | 3, 26, 37, 49, 75, 84, 104, 203, 245 | 0.778 | 83 | 67 |

[Example 7]

In Example 7, the examination in Examples 3 to 6 was further deepened, in order to examine the validation when the number of markers used in the discriminant formula was increased to 10. Specifically, substantially the same procedure as in Example 5 was repeated.

[Example 7-1: Search for Alzheimer's Dementia Markers]

One thousand and one hundred forty seven (1,147) samples from patients with Alzheimer's dementia, a type of dementia, and 110 samples from non-dementia subjects were used for analysis. Table 11 shows the resulting 17 different (the 202nd to the 218th) combinations and their discriminant performance. Among them, regarding the 3 different 204th, 206th, and 209th combinations, marker set variations with sensitivity and specificity of more than 70% in the validation cohort were found (Table 11).

The number of discriminant formulae with sensitivity and specificity of more than 70% in the validation cohort was 1 for a combination of 3 markers (Example 4-1) and 4 for a combination of 5 markers (Example 3-1). In view of Examples 3 to 7, the discriminant performance of markers in the present invention is not necessarily improved as the number of markers in combination is increased. It can be said that any significant combination of at least 3, more preferably 5, and still more preferably about 10 different markers can elicit high discriminant performance.

[Example 7-2: Search for Vascular Dementia Markers]

Eighty (80) samples from patients with vascular dementia, a type of dementia, and 110 samples from non-dementia subjects were used for analysis. Table 11 shows the resulting 21 different (the 219th to the 239th) combinations and their discriminant performance. Among them, regarding the 9 different 219th, 222nd, 225th, 226th, 232nd, 234th, 235th, 236th, and 239th combinations, marker set variations with sensitivity and specificity of more than 80% in the validation cohort were found (Table 11).

The number of discriminant formulae with sensitivity and specificity of more than 80% in the validation cohort was 2 for a combination of 3 markers (Example 4-2) and 2 for a combination of 5 markers (Example 3-2). In view of Examples 3 to 7, the discriminant performance of markers in the present invention is not necessarily improved as the number of markers in combination is increased. It can be said that any significant combination of at least 3, more preferably 5, and still more preferably about 10 different markers can elicit high discriminant performance.

[Example 7-3: Search for Lewy Body Dementia Markers]

One hundred fifty nine (159) samples from patients with Lewy body dementia, a type of dementia, and 110 samples from non-dementia subjects were used for analysis. Table 11 shows the resulting 14 different (the 240th to the 253rd) combinations and their discriminant performance.

Among them, regarding the 5 different 240th, 243rd, 249th, 251st, and 252nd combinations, marker set variations with sensitivity and specificity of more than 80% in the validation cohort were found (Table 11).

The number of discriminant formulae with sensitivity and specificity of more than 80% in the validation cohort was 2 for a combination of 3 markers (Example 4-3), 2 for a combination of 5 markers (Example 3-3), and 2 for a combination of 9 markers (Example 6-3). In view of Examples 3 to 7, the discriminant performance of markers in the present invention is not necessarily improved as the number of markers in combination is increased. It can be said that any significant combination of at least 3, more preferably 5, and still more preferably about 10 different markers can elicit high discriminant performance.

[Example 7-4: Search for Frontotemporal Lobar Degeneration Markers]

Forty (40) samples from patients with frontotemporal lobar degeneration, a type of dementia, and 110 samples from non-dementia subjects were used for analysis. Table 11 shows the resulting 16 different (the 254th to the 269th) combinations and their discriminant performance. Among them, regarding only the 269th combination, a marker set variation with sensitivity and specificity of more than 70% in the validation cohort were found (Table 11).

The number of discriminant formulae with sensitivity and specificity of more than 70% in the validation cohort was 1 for a combination of 3 markers (Example 4-4), 2 for a combination of 5 markers (Example 3-4), and 3 for a combination of 8 markers (Example 5-2). The discriminant performance of markers in the present invention is not necessarily improved as the number of markers in combination is increased. It can be said that any significant combination of at least 3, more preferably 5, and still more preferably about 10 different markers can elicit high discriminant performance.

[Example 7-5: Search for Normal Pressure Hydrocephalus Markers]

Seventy (70) samples from patients with normal pressure hydrocephalus, a type of dementia, and 110 samples from non-dementia subjects were used for analysis. Table 11 shows the resulting 14 different (the 270th to the 283rd) combinations and their discriminant performance (Table 11).

[Table 11]

| Example No. | | Number of miRNAs | SEQ ID NOs | AUC | Sensitivity | Specificity |
|---|---|---|---|---|---|---|
| Example 7-1 | 202nd | 10 | 3, 4, 5, 7, 8, 10, 13, 20, 83, 214 | 0.686 | 72 | 56 |
| | 203rd | 10 | 3, 4, 5, 8, 9, 18, 20, 192, 200, 214 | 0.665 | 71 | 52 |
| | 204th | 10 | 1, 2, 3, 4, 5, 6, 7, 8, 200, 214 | 0.834 | 80 | 79 |
| | 205th | 10 | 2, 3, 4, 8, 9, 10, 13, 152, 200, 214 | 0.739 | 80 | 48 |
| | 206th | 10 | 1, 3, 4, 5, 8, 83, 152, 194, 200, 214 | 0.776 | 71 | 71 |

(continued)

| Example No. | | Number of miRNAs | SEQ ID NOs | AUC | Sensitivity | Specificity |
|---|---|---|---|---|---|---|
| | 207th | 10 | 3, 4, 5, 13, 20, 26, 63, 152, 153, 200 | 0.671 | 79 | 56 |
| | 208th | 10 | 4, 5, 7, 8, 10, 20, 83, 154, 200, 214 | 0.698 | 80 | 50 |
| | 209th | 10 | 1, 2, 3, 4, 5, 9, 10, 11, 214, 215 | 0.803 | 78 | 74 |
| | 210th | 10 | 2, 3, 5, 7, 8, 12, 18, 20, 200, 214 | 0.767 | 78 | 65 |
| | 211th | 10 | 3, 4, 5, 6, 8, 10, 152, 155, 200, 214 | 0.670 | 73 | 52 |
| | 212th | 10 | 3, 4, 5, 7, 9, 83, 152, 192, 194, 200 | 0.696 | 77 | 52 |
| | 213th | 10 | 1, 3, 5, 10, 11, 13, 18, 20, 156, 200 | 0.653 | 77 | 55 |
| | 214th | 10 | 3, 4, 5, 6, 8, 13, 18, 20, 200, 214 | 0.830 | 80 | 62 |
| | 215th | 10 | 4, 5, 9, 19, 23, 26, 47, 153, 200, 214 | 0.697 | 85 | 57 |
| | 216th | 10 | 2, 3, 4, 5, 9, 18, 20, 49, 200, 214 | 0.802 | 80 | 67 |
| | 217th | 10 | 3, 4, 5, 6, 8, 18, 20, 83, 200, 214 | 0.604 | 64 | 58 |
| | 218th | 10 | 3, 4, 5, 8, 11, 13, 152, 156, 200, 214 | 0.749 | 73 | 58 |
| Example 7-2 | 219th | 10 | 3, 4, 16, 23, 24, 25, 26, 197, 216, 218 | 0.894 | 83 | 85 |
| | 220th | 10 | 3, 4, 16, 26, 32, 37, 45, 140, 194, 218 | 0.810 | 63 | 82 |
| | 221st | 10 | 4, 16, 23, 25, 26, 28, 29, 32, 36, 218 | 0.736 | 73 | 70 |
| | 222nd | 10 | 3, 4, 16, 23, 26, 27, 28, 29, 214, 217 | 0.942 | 87 | 91 |
| | 223rd | 10 | 4, 16, 23, 24, 25, 26, 28, 29, 37, 214 | 0.864 | 81 | 68 |
| | 224th | 10 | 3, 4, 23, 25, 26, 28, 29, 125, 207, 216 | 0.831 | 61 | 85 |
| | 225th | 10 | 4, 8, 16, 25, 26, 34, 37, 59, 203, 214 | 0.892 | 89 | 80 |
| | 226th | 10 | 4, 16, 24, 26, 29, 30, 32, 36, 214, 218 | 0.932 | 80 | 86 |
| | 227th | 10 | 4, 23, 26, 28, 45, 88, 91, 203, 217, 218 | 0.810 | 75 | 77 |
| | 228th | 10 | 3, 4, 16, 23, 26, 28, 37, 45, 214, 218 | 0.845 | 90 | 74 |
| | 229th | 10 | 3, 4, 16, 26, 28, 29, 30, 36, 79, 214 | 0.881 | 79 | 79 |
| | 230th | 10 | 3, 4, 16, 25, 26, 28, 32, 37, 214, 218 | 0.893 | 87 | 74 |
| | 231st | 10 | 3, 4, 16, 26, 34, 36, 37, 45, 99, 218 | 0.770 | 65 | 86 |
| | 232nd | 10 | 3, 4, 16, 25, 26, 28, 37, 207, 214, 218 | 0.898 | 92 | 80 |
| | 233rd | 10 | 4, 16, 23, 25, 26, 29, 36, 45, 216, 218 | 0.882 | 71 | 86 |

(continued)

| Example No. | | Number of miRNAs | SEQ ID NOs | AUC | Sensitivity | Specificity |
|---|---|---|---|---|---|---|
| | 234th | 10 | 4, 16, 23, 24, 26, 28, 29, 32, 207, 214 | 0.906 | 83 | 85 |
| | 235th | 10 | 4, 16, 23, 24, 26, 37, 42, 207, 214, 217 | 0.941 | 88 | 86 |
| | 236th | 10 | 3, 4, 23, 25, 26, 28, 43, 214, 216, 218 | 0.810 | 82 | 81 |
| | 237th | 10 | 3, 4, 16, 26, 28, 32, 45, 201, 209, 214 | 0.798 | 75 | 73 |
| | 238th | 10 | 4, 16, 23, 26, 30, 34, 36, 37, 203, 218 | 0.812 | 67 | 83 |
| | 239th | 10 | 3, 4, 16, 26, 28, 29, 36, 45, 217, 218 | 0.866 | 81 | 82 |
| Example 7-3 | 240th | 10 | 3, 4, 16, 26, 45, 47, 49, 53, 194, 214 | 0.915 | 97 | 80 |
| | 241st | 10 | 3, 8, 11, 16, 26, 45, 49, 53, 197, 219 | 0.788 | 93 | 71 |
| | 242nd | 10 | 3, 4, 26, 45, 47, 194, 207, 214, 219, 220 | 0.834 | 83 | 76 |
| | 243rd | 10 | 3, 4, 16, 26, 49, 54, 157, 194, 207, 214 | 0.859 | 97 | 80 |
| | 244th | 10 | 3, 16, 26, 48, 55, 134, 194, 200, 214, 219 | 0.842 | 84 | 78 |
| | 245th | 10 | 3, 4, 11, 26, 49, 52, 53, 64, 158, 219 | 0.707 | 81 | 57 |
| | 246th | 10 | 3, 16, 26, 49, 52, 53, 55, 194, 207, 214 | 0.803 | 89 | 74 |
| | 247th | 10 | 3, 16, 26, 47, 49, 53, 134, 200, 214, 219 | 0.720 | 94 | 42 |
| | 248th | 10 | 3, 4, 26, 45, 47, 49, 116, 134, 207, 220 | 0.789 | 86 | 68 |
| | 249th | 10 | 3, 11, 16, 26, 29, 46, 49, 53, 55, 214 | 0.850 | 87 | 83 |
| | 250th | 10 | 3, 4, 8, 26, 45, 47, 53, 159, 194, 214 | 0.788 | 97 | 63 |
| | 251st | 10 | 3, 16, 26, 29, 47, 54, 209, 214, 219, 220 | 0.860 | 94 | 83 |
| | 252nd | 10 | 3, 8, 16, 26, 49, 54, 194, 214, 219, 220 | 0.860 | 85 | 82 |
| | 253rd | 10 | 3, 4, 11, 26, 45, 47, 53, 60, 200, 220 | 0.698 | 88 | 57 |
| Example 7-4 | 254th | 10 | 26, 29, 37, 69, 70, 71, 72, 74, 147, 200 | 0.770 | 57 | 65 |
| | 255th | 10 | 26, 37, 69, 70, 71, 74, 162, 197, 248, 249 | 0.818 | 63 | 82 |

(continued)

| Example No. | | Number of miRNAs | SEQ ID NOs | AUC | Sensitivity | Specificity |
|---|---|---|---|---|---|---|
| | 256th | 10 | 3, 33, 69, 70, 72, 73, 163, 164, 225, 241 | 0.651 | 67 | 76 |
| | 257th | 10 | 13, 49, 69, 70, 71, 72, 120, 121, 148, 161 | 0.704 | 60 | 76 |
| | 258th | 10 | 26, 30, 37, 69, 70, 71, 74, 224, 227, 248 | 0.631 | 38 | 59 |
| | 259th | 10 | 37, 70, 71, 72, 121, 165, 166, 167, 223, 248 | 0.741 | 33 | 86 |
| | 260th | 10 | 26, 37, 69, 70, 72, 73, 74, 161, 168, 197 | 0.845 | 60 | 75 |
| | 261st | 10 | 26, 69, 70, 72, 125, 147, 169, 170, 241, 242 | 0.619 | 38 | 73 |
| | 262nd | 10 | 30, 69, 70, 71, 73, 121, 172, 197, 227, 249 | 0.632 | 18 | 63 |
| | 263rd | 10 | 3, 26, 37, 69, 70, 72, 73, 174, 191, 241 | 0.619 | 67 | 71 |
| | 264th | 10 | 26, 30, 69, 71, 73, 94, 121, 178, 236, 248 | 0.676 | 63 | 64 |
| | 265th | 10 | 69, 70, 72, 73, 98, 147, 162, 179/180, 248, 249 | 0.875 | 50 | 85 |
| | 266th | 10 | 13, 26, 30, 37, 70, 71, 72, 121, 227, 236 | 0.605 | 67 | 78 |
| | 267th | 10 | 30, 37, 69, 70, 72, 116, 166, 169, 176, 248 | 0.652 | 57 | 70 |
| | 268th | 10 | 24, 29, 69, 70, 71, 72, 73, 175, 182, 197 | 0.878 | 54 | 100 |
| | 269th | 10 | 26, 30, 37, 70, 71, 73, 74, 223, 224, 225 | 0.761 | 75 | 73 |
| Example 7-5 | 270th | 10 | 3, 8, 49, 75, 84, 94, 176, 203, 220, 226 | 0.753 | 73 | 72 |
| | 271st | 10 | 3, 11, 26, 34, 75, 84, 183, 203, 220, 240 | 0.702 | 87 | 62 |
| | 272nd | 10 | 3, 8, 16, 26, 41, 49, 75, 104, 226, 234 | 0.877 | 86 | 77 |
| | 273rd | 10 | 8, 16, 26, 49, 52, 75, 88, 104, 203, 226 | 0.844 | 63 | 85 |
| | 274th | 10 | 3, 26, 49, 75, 84, 85, 104, 184/185, 226, 230 | 0.740 | 71 | 73 |
| | 275th | 10 | 3, 8, 11, 26, 34, 41, 49, 75, 136, 214 | 0.688 | 83 | 67 |
| | 276th | 10 | 1, 3, 8, 26, 41, 43, 49, 75, 136, 176 | 0.827 | 78 | 70 |
| | 277th | 10 | 3, 26, 30, 34, 49, 66, 75, 140, 187, 245 | 0.881 | 75 | 94 |
| | 278th | 10 | 3, 8, 26, 49, 60, 75, 184/185, 203, 220, 226 | 0.860 | 67 | 81 |

(continued)

| Example No. | | Number of miRNAs | SEQ ID NOs | AUC | Sensitivity | Specificity |
|---|---|---|---|---|---|---|
| | 279th | 10 | 3, 8, 26, 34, 41, 60, 75, 84, 104, 226 | 0.829 | 73 | 84 |
| | 280th | 10 | 3, 16, 26, 75, 85, 104, 188, 195, 226, 234 | 0.816 | 75 | 75 |
| | 281st | 10 | 3, 8, 26, 30, 49, 53, 75, 104, 116, 226 | 0.883 | 71 | 91 |
| | 282nd | 10 | 3, 8, 16, 26, 60, 75, 94, 139, 186, 226 | 0.795 | 93 | 64 |
| | 283rd | 10 | 3, 8, 26, 34, 49, 75, 94, 104, 136, 140 | 0.842 | 90 | 69 |

The number of discriminant formulae with sensitivity and specificity of more than 80% in the validation cohort was 5 for a combination of 3 markers (Example 4-5), 3 for a combination of 5 markers (Example 3-5), 1 for a combination of 8 markers (Example 5-3), and 3 for a combination of 9 markers (Example 6-5). The discriminant performance of markers in the present invention is not necessarily improved as the number of markers is increased. As the number of markers is increased, information that supports any significant markers is increased. Thus, it can be said that any significant combination of at least 3, more preferably 5, and still more preferably about 10 different markers can elicit high discriminant performance.

[Example 8]

In Example 8, the object is to examine whether the sensitivity and specificity were improved, compared to those in Example 3, by increasing the number of markers to the top 30 ranked by the P value for Alzheimer's dementia in Example 3.

Specifically, a training cohort was used, and the resulting markers with high correlation were excluded. Next, every combination of 4 or 5 out of the top 30 markers ranked by the P value was analyzed by Fisher's discriminant analysis to construct a discriminant formula. Then, the performance was evaluated in a validation cohort. Otherwise, substantially the same procedure as in Example 3 was repeated.

[Example 8-1: Search for Alzheimer's Dementia Markers]

One thousand and one hundred forty seven (1,147) samples from patients with Alzheimer's dementia, a type of dementia, and 110 samples from non-dementia subjects were used for analysis. Four (4) out of the top 30 markers ranked by the P value were used. This resulted in 3 different (the 284th to the 286th) discriminant formulae with both sensitivity and specificity of more than 70% in the validation cohort. Table 12 shows these combinations and their discriminant performance.

[Example 8-2: Search for Alzheimer's Dementia Markers]

One thousand and one hundred forty seven (1,147) samples from patients with Alzheimer's dementia, a type of dementia, and 110 samples from non-dementia subjects were used for analysis. Five (5) out of the top 30 markers ranked by the P value were used. This resulted in 3 different (the 287th to the 289th) discriminant formulae with both sensitivity and specificity of more than 70% in the validation cohort. Table 12 shows these combinations and their discriminant performance.

[Table 12]

| Example No. | | Number of miRNAs | SEQ ID NOs | AUC | Sensitivity | Specificity |
|---|---|---|---|---|---|---|
| Example 8-1 | 284th | 4 | 3, 16, 23, 214 | 0.825 | 75 | 74 |
| | 285th | 4 | 3, 16, 23, 214 | 0.774 | 76 | 74 |
| | 286th | 4 | 1, 3, 49, 77 | 0.748 | 74 | 74 |
| Example 8-2 | 287th | 5 | 3, 16, 23, 87, 214 | 0.832 | 75 | 79 |

(continued)

| Example No. | | Number of miRNAs | SEQ ID NOs | AUC | Sensitivity | Specificity |
|---|---|---|---|---|---|---|
| | 288th | 5 | 1, 4, 26, 49, 214 | 0.766 | 79 | 71 |
| | 289th | 5 | 4, 5, 9, 11, 72 | 0.804 | 76 | 75 |

The discriminant performance was comparable even when the number of selected markers used in the discriminant formula was increased from the top 20 ranked by the P value (Example 3) to the top 30 ranked by the P value (Example 8).

From the results of Examples 1 to 8, for Alzheimer's dementia, SEQ ID NOs: 1 to 13, 15, 16, 18 to 20, 23, 26, 49, 72, 77, 83, 101, 152, 153, 155, 156, 192 and 194, 200, 214, and 215 are preferably included; for vascular dementia, SEQ ID NOs: 3, 4, 8, 16, 23 to 30, 32, 34, 36, 37, 42, 43, 45, 79, 88, 89, 99, 111, 125 and 194, 197, 199, 201, 203, 204, 206, 207, 214, and 216 to 218 are preferably included; for Lewy body dementia, SEQ ID NOs: 3, 4, 8, 11, 13, 16, 26, 29, 32, 36, 37, 45 to 49, 52 to 55, 60, 64, 68, 91, 116, 134 and 194, 197, 200, 207, 209, 214, 216, 219, 220, 221, and 247 are preferably included; for frontotemporal lobar degeneration, SEQ ID NOs: 3, 10, 13, 24, 26, 29, 30, 33, 37, 43, 69 to 74, 82, 98, 99, 116, 117, 120, 121, 125, 147, 148, 161 to 163, 166, 167, 169, 175, 176 and 197, 200, 202, 223 to 225, 227, 236, 241, 242, 248, and 249 are preferably included; and for normal pressure hydrocephalus, SEQ ID NOs: 1, 3, 8, 10, 11, 16, 26, 30, 34, 37, 41, 45, 49, 52, 54, 60, 66, 75, 76, 83 to 85, 88, 94, 104, 136, 140, 151, 176, 184 to 186 and 203, 211, 212, 220, 226, 230, 234, and 245 are preferably included.

[Example 9]

In Example 9, the object is to perform analysis while the other statistical technique than those in Examples 1 to 8 was used and to construct any marker set with high discriminant accuracy. Specifically, first, miRNA expression levels obtained in the above Reference Example 1 were together normalized in accordance with global normalization. Next, univariate logistic regression was used to calculate a test statistic (z value) for each miRNA, and any miRNA with a z value larger than a certain cut-off T value was selected. Only the selected miRNAs were used in principal component analysis to calculate principal component scores. A multivariate logistic regression formula was prepared using the top-ranked principal component scores as explanatory variables. All the miRNAs were retrieved while the T value and the principal component scores were changed in this operation, and 10-times cross validation was conducted to optimize the T value and the principal component scores. The optimized T value and principal component scores were used to select a prediction formula. This prediction formula was validated in other independent samples.

[Example 9-1: Search for Alzheimer's Dementia Markers]

In Example 9-1, 1,021 samples from patients with Alzheimer's dementia, a type of dementia, as described in Reference Example 1, and 288 samples from non-dementia subjects including non-dementia subjects described in Reference Example 1 and subjects with disease other than dementia as collected likewise in Reference Example 1 were used for analysis. Among them, 511 samples from Alzheimer's dementia patients and 144 samples from non-dementia subjects were assigned to a training cohort and the rest samples, i.e., 510 samples from Alzheimer's dementia patients and 144 samples from non-dementia subjects were assigned to a validation cohort. When 78 miRNAs represented by SEQ ID NOs: 8, 15, 34, 40, 133, 191, 212, 250 to 316, and 375 to 378 were used as discriminant markers, the T value was set to 4.5, and the 10 principal component scores were used, it was possible to predict Alzheimer's dementia in the validation cohort while the AUC value = 0.874. When the cut-off of prediction index value was set to 0.281, the accuracy was 87%, the sensitivity was 93%, and the specificity was 66% (Table 13).

[Example 9-2: Search for Vascular Dementia Markers]

In Example 9-2, 91 samples including samples from vascular dementia patients described in Reference Example 1 and samples from other vascular dementia patients collected likewise in Reference Example 1, and 288 samples from non-dementia subjects same as those in Example 9-1 were used for analysis. Among them, 46 samples from vascular dementia patients and 144 samples from non-dementia subjects were assigned to a training cohort and the rest samples, i.e., 45 samples from vascular dementia patients and 144 samples from non-dementia subjects were assigned to a validation cohort. When 86 miRNAs represented by SEQ ID NOs: 3, 8, 15, 26, 34, 69, 99, 101, 109, 112, 125, 133, 191 to 194, 252, 255, 258, 261 to 264, 266, 267, 271, 277, 278, 282, 284, 287, 289, 290, 293, 294, 297, 298, 301, 303, 306, 307, 310, 312 to 314, 320, 321, 323, 327, 329, 335, 336, 338, 342, 343, 345, 348, 349, 351, 352, 354, 359, 361 to 373 and 375, 376, 380 to 383, 385, and 387 to 390 were used as discriminant markers, the T value was set to 4.0, and the 10 principal component scores were used, it was possible to predict vascular dementia in the validation cohort while the AUC value= 0.867. When the cut-off of prediction index value was set to 0.761, the accuracy was 84%, the sensitivity was 73%, and the specificity was 87% (Table 13).

[Example 9-3: Search for Lewy Body Dementia Markers]

In Example 9-3, 169 samples including samples from Lewy body dementia patients described in Reference Example 1 and samples from other Lewy body dementia patients collected likewise in Reference Example 1, and 288 samples from non-dementia subjects same as those in Example 9-1 were used for analysis. Among them, 85 samples from Lewy body dementia patients and 144 samples from non-dementia subjects were assigned to a training cohort and the rest samples, i.e., 84 samples from Lewy body dementia patients and 144 samples from non-dementia subjects were assigned to a validation cohort. When 110 miRNAs represented by SEQ ID NOs: 3, 8, 15, 26, 34, 53, 99, 101, 107, 109, 112, 128, 133, 191, 192, 194, 250, 253 to 258, 261, 262, 264 to 267, 271, 277, 279, 282 to 284, 287, 289, 290, 293, 294, 298, 299, 301 to 303, 306, 308, 310, 312 to 314, 316 to 360, 374 to 377, and 379 to 388 were used as discriminant markers, the T value was set to 3.4, and the 9 principal component scores were used, it was possible to predict Lewy body dementia in the validation cohort while the AUC value = 0.870. When the cut-off of prediction index value was set to 0.0392, the accuracy was 83%, the sensitivity was 76%, and the specificity was 86% (Table 13).

[Table 13]

| Example No | T value | Number of principal component scores | Number of miRNAs | AUC | Cut-off of prediction index | Accuracy (%) | Sensitivity (%) | Specificity (%) |
|---|---|---|---|---|---|---|---|---|
| Example 8-1 | 4.5 | 10 | 78 | 0.874 | 0.281 | 87 | 93 | 66 |
| Example 8-2 | 4 | 10 | 86 | 0.867 | -0.761 | 84 | 73 | 87 |
| Example 8-3 | 3.4 | 9 | 110 | 0.870 | 0.0392 | 83 | 76 | 86 |

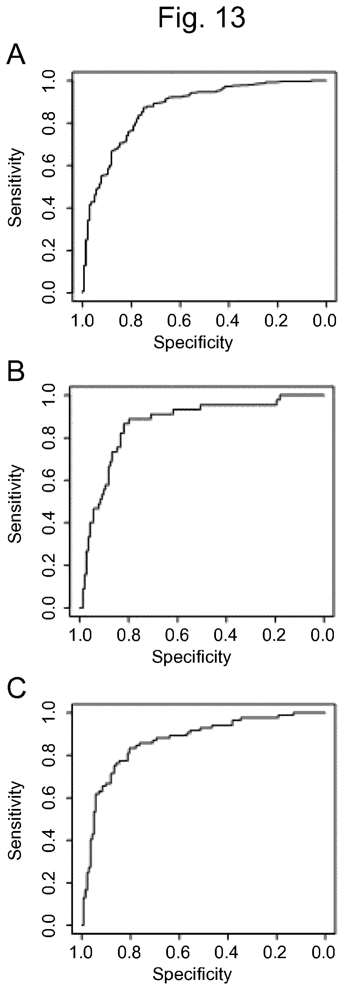

Figs. 13A to 13C show ROC curves as obtained in Examples 9-1 to 9-3. Fig. 13A is for Alzheimer's dementia as described in Example 9-1; Fig. 13B is for vascular dementia as described in Example 9-2; and Fig. 13C is for Lewy body dementia as described in Example 9-3. MiRNA markers obtained in each Example were used to calculate the true positive rate as the ordinate and the false positive rate as the abscissa expressed in each ROC curve. Early detection and early treatment of dementia require diagnosis without misidentification. Meanwhile, once diagnosed as dementia, the disease patients receive a large social impact. Accordingly, it is also important to be able to give a diagnosis with good accuracy and without false positives. At the time of selection of a discriminant formula and markers for discriminating the presence or absence of dementia, a desired combination can be selected, depending on prioritized performance, from the ROC curves shown in Figs. 13A to 13C.

[Example 10]

The discriminant formula prepared in Example 9-1 was used to carry out a progress prediction of whether or not 32 subjects with mild cognitive impairment at a pre-dementia stage progressed into dementia. Among them, 10 cases progressed into Alzheimer's dementia as clinically diagnosed after at least 6 months. When the cut-off of prediction index value was set to 0.281, all the 10 progressed cases were able to be predicted with a sensitivity of 100%. In addition, all the 4 cases that did not progress into Alzheimer's dementia were able to be predicted with a specificity of 100%. Meanwhile, the discriminant results predicted that the rest 18 cases would progress into Alzheimer's dementia. However, when clinically diagnosed, the cases were mild cognitive impairment and were still in follow-up.

[Reference Example 2]

<Collection of samples>

Sera were collected using VENOJECT II vacuum blood collecting tube VP-AS109K60 (Terumo Corp. (Japan)) from a total of 2,978 people including 1,972 dementia patients (patients having diagnosed definitely as dementia by doctors) and 1,006 non-dementia subjects after obtainment of informed consent. The details of the disease types of dementia patients included; 1: 1,147 Alzheimer's dementia patients; 2: 151 patients with Alzheimer's dementia complicated by vascular dementia; 3: 240 patients suspected of Alzheimer's dementia and with Alzheimer's dementia complicated by dementia other than vascular dementia; 4: 80 vascular dementia patients; 5: 85 patients with "other dementia" (dementia resulting from other disease such as alcoholic dementia or vitamin induced dementia and type-unknown or unspecified dementia; the same applies to the following Examples); 6: 159 Lewy body dementia patients; 7: 40 frontotemporal lobar degeneration patients; and 8: 70 normal pressure hydrocephalus patients. In addition, the details of the non-dementia subjects included; 9: 110 subjects without a disease other than dementia; and 10: 896 subjects with a disease other than dementia (Table 14).

[Table 14]

| Subject's disease type | | | Number of samples | |
|---|---|---|---|---|
| Dementia patients | 1 | Alzheimer's dementia | 1147 | 1972 |
| | 2 | Alzheimer's dementia complicated by vascular dementia | 151 | |
| | 3 | Suspected of Alzheimer's dementia and Alzheimer's dementia complicated by dementia other than vascular dementia | 240 | |
| | 4 | Vascular dementia | 80 | |
| | 5 | "Other dementia" (Dementia resulting from another disease and type-unknown or unspecified dementia) | 85 | |
| | 6 | Lewy body dementia | 159 | |
| | 7 | Frontotemporal lobar degeneration | 40 | |
| | 8 | Normal pressure hydrocephalus | 70 | |
| Non-dementia subjects | 9 | Subjects without a disease other than dementia | 110 | 1006 |
| | 10 | Subjects with a disease other than dementia | 896 | |
| Total | | | 2978 | |
| Each disease type number corresponds to the same numeral on the abscissa in Fig. 15 or 16. | | | | |

<Extraction of Total RNA>

Total RNA was obtained, using a reagent for RNA extraction in 3D-Gene (registered trademark) RNA extraction reagent from liquid sample kit (Toray Industries, Inc. (Japan)) according to the protocol provided by the manufacturer, from 300 $\mu$L of the serum sample obtained from each of the total of 2,978 people.

<Measurement of Gene Expression Level>

First, miRNA in the total RNA, which was obtained from the serum sample of each of the total of 2,978 people was fluorescently labeled by use of 3D-Gene (registered trademark) miRNA Labeling kit (Toray Industries, Inc.) according to the protocol provided by the manufacturer. The oligo DNA chip used was 3D-Gene (registered trademark) Human miRNA Oligo chip (Toray Industries, Inc.) with attached probes having sequences complementary to 2,565 miRNAs among the miRNAs registered in miRBase Release 21. Hybridization under stringent conditions and washing following the hybridization were performed according to the protocol provided by the manufacturer. The DNA chip was scanned using 3D-Gene (registered trademark) scanner (Toray Industries, Inc.) to obtain images. Fluorescence intensity was digitized using 3D-Gene (registered trademark) Extraction (Toray Industries, Inc.). The digitized fluorescence intensity was converted to a logarithmic value having a base of 2 and used as a gene expression level, from which a blank value was subtracted. A missing value was replaced with a signal value 0.1. As a result, the comprehensive gene expression levels of the miRNAs in the sera were obtained for the above 2,978 people

Subsequently, samples used for discriminant analysis of dementia were extracted. First, 1,972 dementia patients were assigned to a positive group. Further, 1,006 subjects without dementia (non-dementia subjects) were assigned to a negative group. Next, each group was sorted into a training cohort and a validation cohort as shown in each Example below. Calculation and statistical analysis using the digitized gene expression levels of the miRNAs were carried out using R language 3.3.1 (R Core Team (2016). R: A language and environment for statistical computing. R Foundation for Statistical Computing, Vienna, Austria. URL https://www.R-project.org/.) and MASS package 7.3.45 (Venables, W.N. & Ripley, B.D. (2002) Modern Applied Statistics with S. Fourth Edition. Springer, New York. ISBN 0-387-95457-0).

[Example 11]

To search for markers that can discriminate, with a good balance, between dementia patients and non-dementia subjects, 900 samples from each of the dementia patients and the non-dementia subjects were sorted into a training cohort (B of Table 15), and the rest samples were sorted into a validation cohort (C of Table 15). Specifically, as listed in Table 15, 900 of all the samples from the dementia patients (1: 522 Alzheimer's dementia patients; 2: 73 patients with Alzheimer's dementia complicated by vascular dementia; 3: 99 patients suspected of Alzheimer's dementia and with Alzheimer's dementia complicated by dementia other than vascular dementia; 4: 34 vascular dementia patients; 5: 46 patients with "other dementia"; 6: 72 Lewy body dementia patients; 7: 20 frontotemporal lobar degeneration patients; and 8: 34 normal pressure hydrocephalus patients) and 900 of all the samples from the non-dementia subjects (9: 102 subjects without a disease other than dementia; and 10: 798 subjects with a disease other than dementia) were assigned to a training cohort, and 1072 of all the samples from the dementia patients (1: 625 Alzheimer's dementia patients; 2: 78 patients with Alzheimer's dementia complicated by vascular dementia; 3: 141 patients suspected of Alzheimer's dementia and with Alzheimer's dementia complicated by dementia other than vascular dementia; 4: 46 vascular dementia patients; 5: 39 patients with "other dementia"; 6: 87 Lewy body dementia patients; 7: 20 frontotemporal lobar degeneration patients; and 8: 36 normal pressure hydrocephalus patients) and 106 of all the samples from the non-dementia subjects (9: 8 subjects without a disease other than dementia; and 10: 98 subjects with a disease other than dementia) were assigned to a validation cohort.

[Table 15]

| Subject's disease type | | | A. All samples | | B. Training cohort | | C. Validation cohort | |
|---|---|---|---|---|---|---|---|---|
| Dementia patients | 1 | Alzheimer's dementia | | 1147 | | 522 | | 625 |
| | 2 | Alzheimer's dementia complicated by vascular dementia | | 151 | | 73 | | 78 |
| | 3 | Suspected of Alzheimer's dementia and Alzheimer's dementia complicated by dementia other than vascular dementia | | 240 | | 99 | | 141 |
| | 4 | Vascular dementia | 1972 | 80 | 900 | 34 | 1072 | 46 |
| | 5 | "Other dementia" (Dementia resulting from another disease and type-unknown or unspecified dementia) | | 85 | | 46 | | 39 |
| | 6 | Lewy body dementia | | 159 | | 72 | | 87 |
| | 7 | Frontotemporal lobar degeneration | | 40 | | 20 | | 20 |
| | 8 | Normal pressure hydrocephalus | | 70 | | 34 | | 36 |
| Non-dementia subjects | 9 | Subjects without a disease other than dementia | 1006 | 110 | 900 | 102 | 106 | 8 |
| | 10 | Subjects with a disease other than dementia | | 896 | | 798 | | 98 |
| Each disease type number corresponds to the same numeral on the abscissa in Fig. 15 or 16. | | | | | | | | |

Meanwhile, in this Example, a discriminant formula with three gene markers was prepared using the above training cohort, and discriminant performance was then evaluated in the above validation cohort.

Specifically, first, miRNA expression levels in the training cohort and the validation cohort obtained in the above Reference Example 2 were together normalized in accordance with quantile normalization. Further, in order to obtain a more highly reliable diagnostic marker, only 317 genes having gene expression levels of $2^6$ or more in 50% or more samples in either one of a group of dementia patients or a group of non-dementia subjects were selected as analytes

Next, a combination of the measurement values for the gene expression levels of 3 out of 317 miRNAs was subjected to logistic regression to construct a discriminant formula for discriminating the presence or absence of dementia.

Further, the above-prepared discriminant formula was used to calculate the accuracy, the sensitivity, and the specificity in the validation cohort. Then, discriminant performance was validated in independent samples.

This resulted in a discriminant formula with a sensitivity of 70% and a specificity of 56% in the validation cohort when the 3 miRNAs represented by SEQ ID NOs: 1315 to 1316 and 194 were used as discriminant markers (Table 17).

[Example 12]

Like Example 11, a combination of 11 miRNAs was used to construct a discriminant formula for discriminating the presence or absence of dementia, and the discriminant formula was validated.

This resulted in a discriminant formula with a sensitivity of 70% and a specificity of 60% in the validation cohort when the 11 miRNAs represented by SEQ ID NOs: 1315 to 1316, 194, 195, 1318, 1319, 1320 to 1322, and 1335 were used as discriminant markers (Table 17). When the number of miRNA markers was increased from 3 to 11, an increase in the specificity (a decrease in the false positive) was demonstrated.

[Example 13]

Like Example 1, a combination of 20 miRNAs was used to construct a discriminant formula for discriminating the presence or absence of dementia, and the discriminant formula was validated.

This resulted in a discriminant formula with a sensitivity of 70% and a specificity of 64% in the validation cohort when the 20 miRNAs represented by SEQ ID NOs: 1315, 1316, 194, 195, 1318, 1319, 197, 1320 to 1325, 199, 1331, 200, 1335, 1352, 1354, and 1356 were used as discriminant markers (Table 17). When the number of miRNA markers was increased from 11 to 20, a further increase in the specificity (a decrease in the false positive) was demonstrated.

[Example 14]

Like Example 11, a combination of 26 miRNAs was used to construct a discriminant formula for discriminating the presence or absence of dementia, and the discriminant formula was validated.

This resulted in a discriminant formula with a sensitivity of 70% and a specificity of 66% in the validation cohort when the 26 miRNAs represented by SEQ ID NOs: 1315 to 1325, 194 to 197, 199, 1328, 1331, 200, 201, 1335, 1338, 1352 to 1354, and 1356 were used as discriminant markers (Table 17). When the number of miRNA markers was increased from 20 to 26, a further increase in the specificity (a decrease in the false positive) was demonstrated.

[Example 15]

Like Example 11, a combination of 42 miRNAs was used to construct a discriminant formula for discriminating the presence or absence of dementia, and the discriminant formula was validated.

This resulted in a discriminant formula with a sensitivity of 70% and a specificity of 66% in the validation cohort when the 42 miRNAs represented by SEQ ID NOs: 194 to 205, 1315 to 1339, 1352 to 1355, and 1356 were used as discriminant markers (Table 17). When the number of miRNA markers was increased from 26 to 42, each of the accuracy, the sensitivity, or the specificity was slightly increased in the training cohort. However, the accuracy was slightly decreased in the validation cohort while the sensitivity and the specificity were kept at a high value. This demonstrated that about 26 miRNA markers were at a peak.

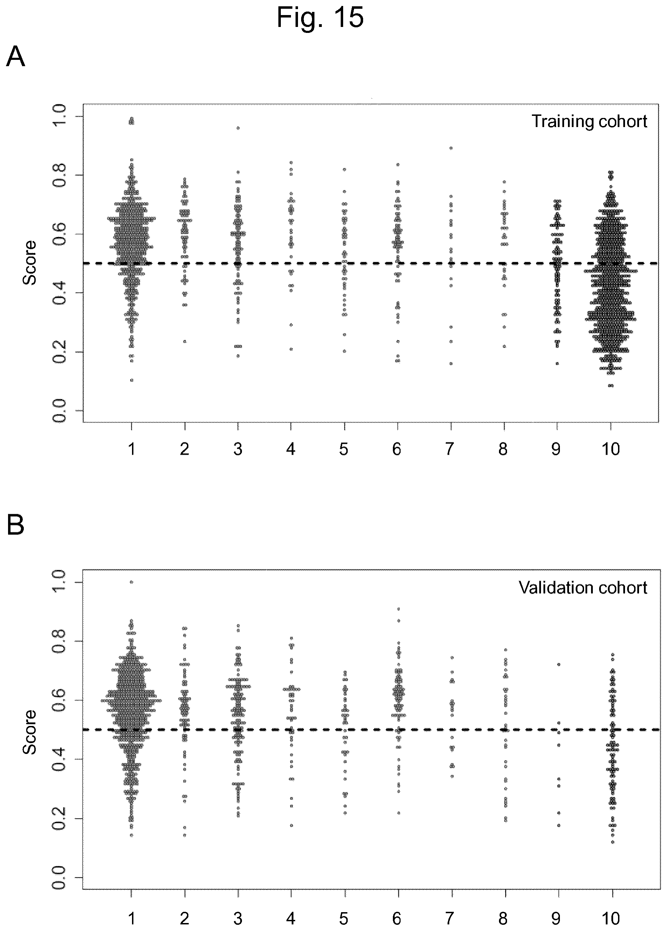

Fig. 15 shows plots of discriminant scores obtained at that time. Fig. 15A is a plot of discriminant scores for the training cohort and Fig. 15B is a plot of discriminant scores for the validation cohort. The discriminant scores range from 0 to 1. The dotted line in each panel depicts a discriminant boundary (a discriminant score of 0.5) that discriminates the presence or absence of dementia. In Fig. 15A, the measurement values for the expression levels of the above 42 miRNAs in sera from (900) dementia patients or (900) non-dementia subjects selected as the training cohort were used to prepare a discriminant formula by logistic regression analysis using the LASSO method. The ordinate represents a discriminant score obtained from the discriminant formula, and the abscissa represents each cohort. The measurement values for the expression levels of the 42 miRNAs in sera from (1072) dementia patients and (106) non-dementia subjects selected as the validation cohort were plugged into the discriminant formula prepared using the training cohort to give discriminant scores as the ordinate and each cohort as the abscissa expressed in Fig. 15B.

Here, regarding the abscissa in each of Fig. 15A or Fig. 15B, the numeral 1 represents Alzheimer's dementia, the numeral 2 represents Alzheimer's dementia complicated by vascular dementia, the numeral 3 represents cases suspected of Alzheimer's dementia and Alzheimer's dementia complicated by dementia other than vascular dementia, the numeral 4 represents vascular dementia, the numeral 5 represents "other dementia" (dementia caused by another disease and dementia of type-unknown or unspecified dementia), the numeral 6 represents Lewy body dementia, the numeral 7 represents frontotemporal lobar degeneration, the numeral 8 represents normal pressure hydrocephalus, the numeral 9 represents non-dementia subjects (subjects without a disease other than dementia), and the numeral 10 represents non-dementia subjects (subjects with a disease other than dementia).

[Example 16]

To search for markers that can discriminate, with good sensitivity, dementia patients in particular, about 2/3 of samples from each group were sorted into a training cohort (B of Table 16) and the rest samples were sorted into a validation cohort (C of Table 16). Specifically, as listed in Table 16, 1,315 of all the samples from the dementia patients (1: 774 Alzheimer's dementia patients; 2: 108 patients with Alzheimer's dementia complicated by vascular dementia; 3: 150 patients suspected of Alzheimer's dementia and with Alzheimer's dementia complicated by dementia other than vascular dementia; 4: 48 vascular dementia patients; 5: 58 patients with "other dementia"; 6: 106 Lewy body dementia patients: 7: 27 frontotemporal lobar degeneration patients; and 8: 44 normal pressure hydrocephalus patients) and 671 of all the samples from the non-dementia subjects (9: 71 subjects without a disease other than dementia; and 10: 600 subjects with a disease other than dementia) were assigned to a training cohort, and 657 of all the samples from the dementia patients (1: 373 Alzheimer's dementia patients; 2: 43 patients with Alzheimer's dementia complicated by vascular dementia; 3: 90 patients suspected of Alzheimer's dementia and with Alzheimer's dementia complicated by dementia other than vascular dementia; 4: 32 vascular dementia patients; 5: 27 patients with "other dementia"; 6: 53 Lewy body dementia patients; 7: 13 frontotemporal lobar degeneration patients,; and 8:26 normal pressure hydrocephalus patients) and 335 of all the samples from the non-dementia subjects (9: 39 subjects without a disease other than dementia; and 10: 296 subjects with a disease other than dementia) were assigned to a validation cohort.

[Table 16]

| Subject's disease type | | | A. All samples | | B. Training cohort | | C. Validation cohort | |
|---|---|---|---|---|---|---|---|---|
| Dementia patients | 1 | Alzheimer's dementia | 1972 | 1147 | 1315 | 774 | 657 | 373 |
| | 2 | Alzheimer's dementia complicated by vascular dementia | | 151 | | 108 | | 43 |
| | 3 | Suspected of Alzheimer's dementia and Alzheimer's dementia complicated by dementia other than vascular dementia | | 240 | | 150 | | 90 |
| | 4 | Vascular dementia | | 80 | | 48 | | 32 |
| | 5 | "Other dementia" (Dementia resulting from another disease and type-unknown or unspecified dementia) | | 85 | | 58 | | 27 |
| | 6 | Lewy body dementia | | 159 | | 106 | | 53 |
| | 7 | Frontotemporal lobar degeneration | | 40 | | 27 | | 13 |
| | 8 | Normal pressure hydrocephalus | | 70 | | 44 | | 26 |
| Non-dementia subjects | 9 | Subjects without a disease other than dementia | 1006 | 110 | 671 | 71 | 335 | 39 |
| | 10 | Subjects with a disease other than dementia | | 896 | | 600 | | 296 |
| Each disease type number corresponds to the same numeral on the abscissa in Fig. 15 or 16. | | | | | | | | |

Meanwhile, in this Example, a discriminant formula with three gene markers was prepared using the above training cohort, and discriminant performance was then evaluated in the above validation cohort.

Specifically, a discriminant formula for discriminating the presence or absence of dementia was constructed by logistic regression using a combination of the measurement values for the expression levels of 3 miRNAs included in the above 317 genes obtained as analytes like Example 11. Further, the above-prepared discriminant formula was used to calculate the accuracy, the sensitivity, and the specificity in the validation cohort. Then, discriminant performance was validated in independent samples.

This resulted in a discriminant formula with a sensitivity of 97% and a specificity of 11% in the validation cohort when the 3 miRNAs represented by SEQ ID NOs: 1315 to 1316 and 194 were used as discriminant markers (Table 17).

[Example 17]

Like Example 16, a combination of 10 miRNAs was used to construct a discriminant formula for discriminating the presence or absence of dementia, and the discriminant formula was validated.

This resulted in a discriminant formula with a sensitivity of 95% and a specificity of 21% in the validation cohort when the 10 miRNAs represented by SEQ ID NOs: 1315 to 1316, 194, 195, 1321, 1322, 1326, 1327, 202, and 1347 were used as discriminant markers (Table 17). When the number of miRNA markers was increased from 3 to 10, an increase in the specificity (a decrease in the false positive) was demonstrated.

[Example 18]

Like Example 16, a combination of 20 miRNAs was used to construct a discriminant formula for discriminating the presence or absence of dementia, and the discriminant formula was validated.

This resulted in a discriminant formula with a sensitivity of 92% and a specificity of 27% in the validation cohort when the 20 miRNAs represented by SEQ ID NOs: 1315, 1316, 194, 195, 197, 1321, 1322, 1326, 1327, 199, 1331, 200, 202, 1333, 1335, 206, 1349, 1352, 1355, and 212 were used as discriminant markers (Table 17). When the number of miRNA markers was increased from 10 to 20, a further increase in the specificity (a decrease in the false positive) was demonstrated.

[Example 19]

Like Example 16, a combination of 39 miRNAs was used to construct a discriminant formula for discriminating the presence or absence of dementia, and the discriminant formula was validated.

This resulted in a discriminant formula with a sensitivity of 90% and a specificity of 33% in the validation cohort when the 39 miRNAs represented by SEQ ID NOs: 1315, 1316, 194, 195, 1318, 1319, 197, 1321 to 1323, 1326, 1327, 198, 1331, 200, 202, 1333, 203, 1335, 1337 to 1339, 1340, 374, 1342, 1343, 208 to 209, 1345, 1347 to 1349, 1351, 1353 to 1356, and 211 to 212 were used as discriminant markers (Table 17). When the number of miRNA markers was increased from 20 to 39, a further increase in the specificity (a decrease in the false positive) was demonstrated.

[Example 20]

Like Example 16, a combination of 51 miRNAs was used to construct a discriminant formula for discriminating the presence or absence of dementia, and the discriminant formula was validated.

This resulted in a discriminant formula with a sensitivity of 90% and a specificity of 35% in the validation cohort when the 51 miRNAs represented by SEQ ID NOs: 1315, 1316, 194, 195, 1318, 1319, 197, 1321 to 1323, 1326, 1327, 198, 199, 1329, 1331, 200, 202, 1333, 203, 1335, 204 to 212, 374, and 1337 to 1356 were used as discriminant markers (Table 17). When the number of miRNA markers was increased from 39 to 51, a further increase in the specificity (a decrease in the false positive) was demonstrated.

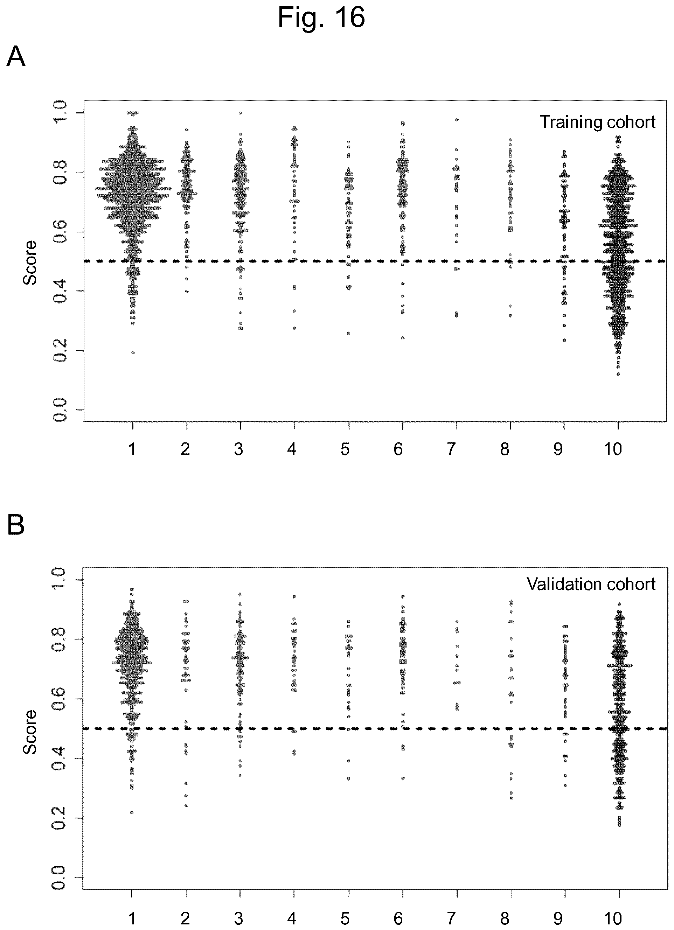

Fig. 16 shows plots of discriminant scores obtained in this Example. Fig. 16A is a plot of discriminant scores for the training cohort and Fig. 16B is a plot of discriminant scores for the validation cohort. The discriminant scores range from 0 to 1. The dotted line in each panel depicts a discriminant boundary (a discriminant score of 0.5) that discriminates the presence or absence of dementia. In Fig. 16A, the measurement values for the expression levels of the above 51 miRNAs in sera from (1,315) dementia patients or (671) non-dementia subjects selected as the training cohort were used to prepare a discriminant formula by logistic regression analysis using the LASSO method. The ordinate represents a discriminant score obtained from the discriminant formula, and the abscissa represents each cohort. The measurement values for the expression levels of the 51 miRNAs in sera from (657) dementia patients and (335) non-dementia subjects selected as the validation cohort were plugged into the discriminant formula prepared using the training cohort to give discriminant scores as the ordinate and each cohort as the abscissa expressed in Fig. 16B.

Here, the numerals 1 to 10 on the abscissa in each of Fig. 16A or 16B denote the respective same dementia disease types as in Fig. 15 (Example 15).

[Table 17]

| Example No. | number of miRNAs | Training cohort | | | Validation cohort | | |
|---|---|---|---|---|---|---|---|
| | | Accuracy (%) | Sensitivity (%) | Specificity (%) | Accuracy (%) | Sensitivity (%) | Specificity (%) |
| Example 11 | 3 | 64 | 71 | 56 | 69 | 70 | 56 |

(continued)

| Example No. | number of miRNAs | Training cohort | | | Validation cohort | | |
|---|---|---|---|---|---|---|---|
| | | Accuracy (%) | Sensitivity (%) | Specificity (%) | Accuracy (%) | Sensitivity (%) | Specificity (%) |
| Example 12 | 11 | 65 | 71 | 58 | 69 | 70 | 60 |
| Example 13 | 20 | 67 | 71 | 62 | 69 | 70 | 64 |
| Example 14 | 26 | 68 | 72 | 63 | 70 | 70 | 66 |
| Example 15 | 42 | 69 | 73 | 64 | 69 | 70 | 66 |
| Example 16 | 3 | 68 | 97 | 10 | 68 | 97 | 11 |
| Example 17 | 10 | 70 | 94 | 22 | 70 | 95 | 21 |
| Example 18 | 20 | 72 | 93 | 29 | 70 | 92 | 27 |
| Example 19 | 39 | 73 | 92 | 36 | 71 | 90 | 33 |
| Example 20 | 51 | 75 | 93 | 39 | 71 | 90 | 35 |
| Example 22 | 2 | 62 | 70 | 54 | 68 | 70 | 52 |
| Example 23 | 1 | 62 | 69 | 54 | 67 | 69 | 53 |

[Example 21]

Early detection and early treatment of dementia require diagnosis without misidentification. Meanwhile, once diagnosed as dementia, the disease patients receive a large social impact. Accordingly, it is also important to be able to give a diagnosis with good accuracy and without false positives. In view of the above Examples 11 to 20, some combination of 1, 2 or more, preferably 20 to 60, and more preferably 40 to 55 polynucleotides from all the nucleotide sequences represented by SEQ ID NOs: 1 to 54, 55 to 62, and 127 to 178 can be said to be genes that can discriminate, with high accuracy, between dementia patients and subjects without dementia.

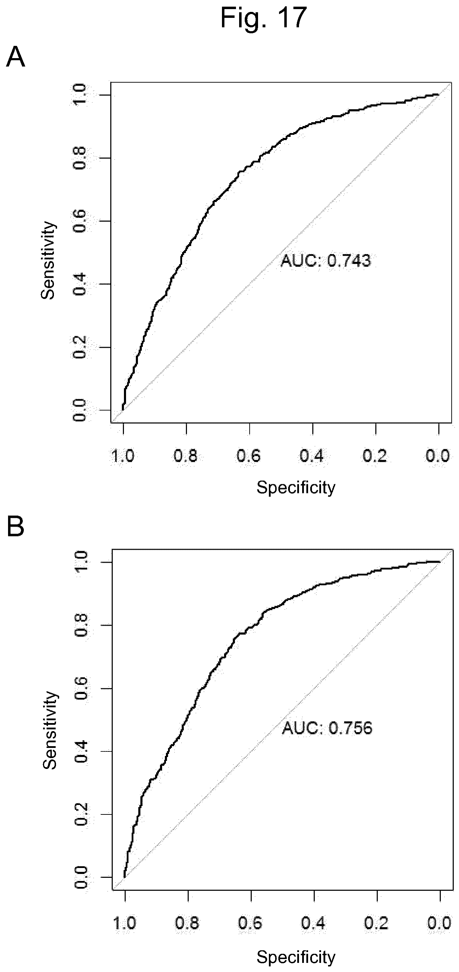

Fig. 17A is a ROC (Receiver Operating Characteristic) curve while the ordinate denotes the sensitivity and the abscissa denotes the specificity as calculated using 42 miRNA markers (SEQ ID NOs: 194 to 205, 1315 to 1339, 1352 to 1355, and 1356) obtained in Example 15. In Fig. 17B, 51 miRNA markers (SEQ ID NOs: 1315, and 1316, 194, 195, 1318, 1319, 197, 1321 to 1323, 1326, 1327, 198, 199, 1329, 1331, 200, 202, 1333, 203, 1335, 204 to 212, 374, 1337 to 1356) obtained in Example 20 were used to calculate the sensitivity as the ordinate and the specificity as the abscissa expressed in a ROC curve. At the time of selection of a discriminant formula and markers for discriminating the presence or absence of dementia, a desired combination can be selected, depending on prioritized performance, from the ROC curves shown in Fig. 17.

[Example 22]

Like Example 11, a combination of 2 miRNAs was used to construct a discriminant formula for discriminating the presence or absence of dementia, and the discriminant formula was validated.

This resulted in a discriminant formula with a sensitivity of 70% and a specificity of 52% in the validation cohort when the 2 miRNAs represented by SEQ ID NOs: 1317 and 195 were used as discriminant markers (Table 17).

[Example 23]

Like Example 11, one miRNA was used to construct a discriminant formula for discriminating the presence or absence of dementia, and the discriminant formula was validated.

This resulted in a discriminant formula with a sensitivity of 69% and a specificity of 53% in the validation cohort when the one miRNA represented by SEQ ID NO: 1315 was used as a discriminant marker (Table 17).

[Example 24]

In Example 24, in order to search for a statistical technique more fit to discriminate dementia, the statistical techniques used in Examples 11 to 23; the LASSO method, elastic net, and ridge regression were compared. For this comparison, analysis was conducted using 2,177 samples from dementia patients including samples from the dementia patients described in Reference Example 2 and samples from dementia patients newly collected like in Reference Example 2. In addition, 756 samples from non-dementia subjects including the samples from the non-dementia subjects described in Reference Example 2 and samples from non-dementia subjects newly collected like in Reference Example 2 were used. Specifically, first, miRNA expression levels obtained in the above Reference Example 2 were together normalized in accordance with global normalization. Next, the samples were divided, and 3/4 of all the samples were assigned to a training cohort and the rest 1/4 were assigned to a validation cohort. Further the training cohort was divided into 5 groups. Then, the p value for each miRNA was calculated by multivariate logistic regression using each of the LASSO method, elastic net, or ridge regression. After the number of variables was increased while each miRNA was included according to the order from the top-ranked p value, the optimal number of miRNAs was examined by cross validation between the 5 divided groups.

The results demonstrated that even when any of the analysis techniques was used, the case where the number of miRNAs used was 60 was optimal. Then, the 60 miRNAs with the top-ranked p-values were used to create each dementia discriminant model, and this model was validated in the validation cohort.

As a result, in the case of the LASSO method, the AUC value was 0.772, the accuracy was 73%, the sensitivity was 74%, and the specificity was 70%; in the case of elastic net, the AUC value was 0.773, the accuracy was 74%, the sensitivity was 76%, and the specificity was 68%; and in the case of ridge regression, the AUC value was 0.773, the accuracy was 73%, the sensitivity was 75%, and the specificity was 66%. When attention was paid to the accuracy and the sensitivity, elastic net exhibited the highest discriminant accuracy with a marginal difference (Table 18). In the discriminant model obtained using this elastic net, used were 44 miRNAs of SEQ ID NOs: 68, 99, 100, 194, 197, 200, 212, 255, 263, 265, 294, 302, 315, 342, 344, 1315, 1316, 1318, 1320, 1321, 1322, 1323, 1326, 1331, 1340, 1435, 1436, 1438, 1439, 1440, 1441, 1446, 1448, 1449, 1451, 1452, 1453, 1455, 1456, 1458, 1460, 1461, 1462, and 1463.

[Table 18]

| Example No. | Statistical technique | AUC | Accuracy (%) | Sensitivity (%) | Specificity (%) |
|---|---|---|---|---|---|
| Example 24 | LASSO method | 0.772 | 73 | 74 | 69 |
| | Elastic net | 0.773 | 74 | 76 | 68 |
| | Ridge regression | 0.773 | 73 | 75 | 66 |

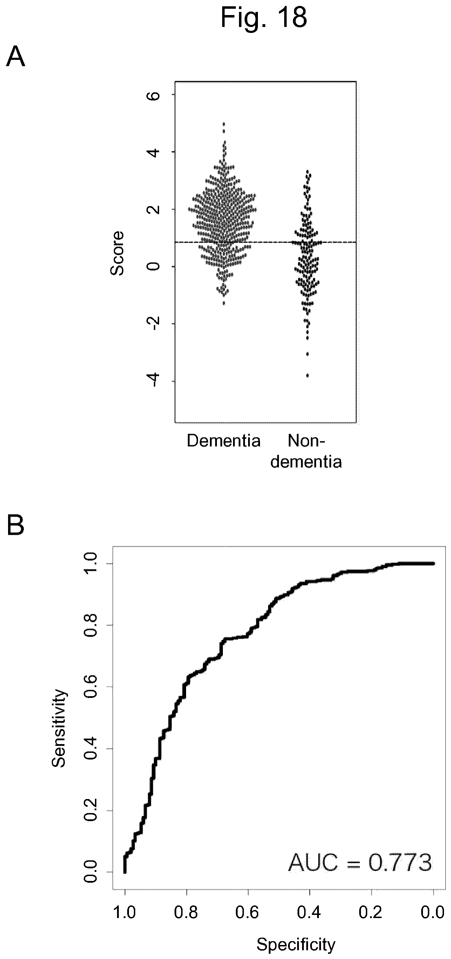

Fig. 18 shows a plot (Fig. 18A) of discriminant scores and a ROC curve (Fig. 18B) as obtained using elastic net. The dotted line in the panel of Fig. 18A depicts a discriminant boundary that discriminates the presence or absence of dementia. A discriminant formula was prepared from measurement values for the expression levels of the above 44 miRNAs by logistic regression analysis using elastic net. The ordinate represents a discriminant score obtained from the discriminant formula, and the abscissa represents each cohort. Fig. 18B is a ROC (Receiver Operating Characteristic) curve while the ordinate denotes the sensitivity and the abscissa denotes the specificity as calculated using the above 44 miRNAs. At the time of selection of a discriminant formula and markers for discriminating the presence or absence of dementia, a desired combination can be selected, depending on prioritized performance, from the ROC curve shown in Fig. 18B.

All the publications, patents, and patent applications cited herein are incorporated herein by reference in the entirety.

The following labelled statements set out further aspects of the present invention:

A1. A kit for detection of dementia, comprising nucleic acid(s) capable of specifically binding to one or more polynucleotide(s) selected from the group consisting of dementia markers: miR-4274, miR-4272, miR-4728-5p, miR-4443, miR-4506, miR-6773-5p, miR-4662a-5p, miR-3184-3p, miR-4281, miR-320d, miR-6729-3p, miR-5192, miR-6853-5p, miR-1234-3p, miR-1233-3p, miR-4539, miR-3914, miR-4738-5p, miR-548au-3p, miR-1539, miR-4720-3p, miR-365b-5p, miR-4486, miR-1227-5p, miR-4667-5p, miR-6088, miR-6820-5p, miR-4505, miR-548q, miR-4658, miR-450a-5p, miR-1260b, miR-3677-5p, miR-6777-3p, miR-6826-3p, miR-6832-3p, miR-4725-3p, miR-7161-3p, miR-2277-5p, miR-7110-3p, miR-4312, miR-4461, miR-6766-5p, miR-1266-3p, miR-6729-5p, miR-526b-3p, miR-519e-5p, miR-512-5p, miR-5088-5p, miR-1909-3p, miR-6511a-5p, miR-4734, miR-936, miR-1249-3p, miR-6777-5p, miR-4487, miR-3155a, miR-563, miR-4741, miR-6788-5p, miR-4433b-5p, miR-323a-5p, miR-6811-5p, miR-6721-5p, miR-5004-5p, miR-6509-3p, miR-648, miR-3917, miR-6087, miR-1470, miR-586, miR-3150a-5p, miR-105-3p, miR-7973, miR-1914-5p, miR-4749-3p, miR-15b-5p, miR-1289, miR-4433a-5p, miR-3666, miR-3186-3p, miR-4725-5p, miR-4488, miR-4474-3p, miR-6731-3p, miR-4640-3p, miR-202-5p, miR-6816-5p, miR-638, miR-6821-5p, miR-1247-3p, miR-6765-5p, miR-6800-5p, miR-3928-3p, miR-3940-5p, miR-3960, miR-6775-5p, miR-3178, miR-1202, miR-6790-5p, miR-4731-3p, miR-2681-3p, miR-6758-5p, miR-8072, miR-518d-3p, miR-3606-3p, miR-4800-5p, miR-1292-3p, miR-6784-3p, miR-4450, miR-6132, miR-4716-5p, miR-6860, miR-1268b, miR-378d, miR-4701-5p, miR-4329, miR-185-3p, miR-552-3p, miR-1273g-5p, miR-6769b-3p, miR-520a-3p, miR-4524b-5p, miR-4291, miR-6734-3p, miR-143-5p, miR-939-3p, miR-6889-3p, miR-6842-3p, miR-4511, miR-4318, miR-4653-5p, miR-6867-3p, miR-133b, miR-3196, miR-193b-3p, miR-3162-3p, miR-6819-3p, miR-1908-3p, miR-6786-5p, miR-3648, miR-4513, miR-3652, miR-4640-5p, miR-6871-5p, miR-7845-5p, miR-3138, miR-6884-5p, miR-4653-3p, miR-636, miR-4652-3p, miR-6823-5p, miR-4502, miR-7113-5p, miR-8087, miR-7154-3p, miR-5189-5p, miR-1253, miR-518c-5p, miR-7151-5p, miR-3614-3p, miR-4727-5p, miR-3682-5p, miR-5090, miR-337-3p, miR-488-5p, miR-100-5p, miR-4520-3p, miR-373-3p, miR-6499-5p, miR-3909, miR-32-5p, miR-302a-3p, miR-4686, miR-4659a-3p, miR-4287, miR-1301-5p, miR-593-3p, miR-517a-3p, miR-517b-3p, miR-142-3p, miR-1185-2-3p, miR-602, miR-527, miR-518a-5p, miR-4682, miR-28-5p, miR-4252, miR-452-5p, miR-525-5p, miR-3622a-3p, miR-6813-3p, miR-4769-3p, miR-5698, miR-1915-3p, miR-1343-5p, miR-6861-5p, miR-6781-5p, miR-4508, miR-6743-5p, miR-6726-5p, miR-4525, miR-4651, miR-6813-5p, miR-5787, miR-1290, miR-6075, miR-4758-5p, miR-4690-5p, miR-762, miR-371a-5p, miR-6765-3p, miR-6784-5p, miR-6778-5p, miR-6875-5p, miR-4534, miR-4721, miR-6756-5p, miR-615-5p, miR-6727-5p, miR-6887-5p, miR-8063, miR-6880-5p, miR-6805-3p, miR-4726-5p, miR-4710, miR-7111-5p, miR-3619-3p, miR-6795-5p, miR-1254, miR-1233-5p, miR-6836-3p, miR-6769a-5p, miR-4532, miR-365a-5p, miR-1231, miR-1228-5p, miR-4430, miR-296-3p, miR-1237-5p, miR-4466, miR-6789-5p, miR-4632-5p, miR-4745-5p, miR-4665-5p, miR-6807-5p, miR-7114-5p, miR-516a-5p, miR-769-3p, miR-3692-5p, miR-3945, miR-4433a-3p, miR-4485-3p, miR-6831-5p, miR-519c-5p, miR-551b-5p, miR-1343-3p, miR-4286, miR-4634, miR-4733-3p, and miR-6086, or to complementary strand(s) of the polynucleotide(s).

A2. The kit according to clause A1, wherein the nucleic acid(s) are polynucleotide(s) selected from the group consisting of the following polynucleotides (a) to (e):

(a) a polynucleotide consisting of a nucleotide sequence represented by any of SEQ ID NOs: 1 to 210, 374, 1315 to 1350, and 1435 to 1448 or a nucleotide sequence derived from the nucleotide sequence by the replacement of u with t, a variant thereof, a derivative thereof, or a fragment thereof comprising 15 or more consecutive nucleotides,
(b) a polynucleotide comprising a nucleotide sequence represented by any of SEQ ID NOs: 1 to 210, 374, 1315 to 1350, and 1435 to 1448,
(c) a polynucleotide consisting of a nucleotide sequence complementary to a nucleotide sequence represented by any of SEQ ID NOs: 1 to 210, 374, 1315 to 1350, and 1435 to 1448 or a nucleotide sequence derived from the nucleotide sequence by the replacement of u with t, a variant thereof, a derivative thereof, or a fragment thereof comprising 15 or more consecutive nucleotides,
(d) a polynucleotide comprising a nucleotide sequence complementary to a nucleotide sequence represented by any of SEQ ID NOs: 1 to 210, 374, 1315 to 1350, and 1435 to 1448 or a nucleotide sequence derived from the nucleotide sequence by the replacement of u with t, and
(e) a polynucleotide hybridizing under stringent conditions to any of the polynucleotides (a) to (d).

A3. The kit according to clause A1 or A2, wherein the kit further comprises nucleic acid(s) capable of specifically binding to one or more polynucleotide(s) selected from the group consisting of other dementia markers: miR-1225-3p, miR-3184-5p, miR-665, miR-211-5p, miR-1247-5p, miR-3656, miR-149-5p, miR-744-5p, miR-345-5p, miR-150-5p, miR-191-3p, miR-651-5p, miR-34a-5p, miR-409-5p, miR-369-5p, miR-1915-5p, miR-204-5p, miR-137, miR-382-5p, miR-517-5p, miR-532-5p, miR-22-5p, miR-1237-3p, miR-1224-3p, miR-625-3p, miR-328-3p, miR-122-5p, miR-202-3p, miR-4781-5p, miR-718, miR-342-3p, miR-26b-3p, miR-140-3p, miR-200a-3p, miR-378a-3p, miR-484, miR-296-5p, miR-205-5p, miR-431-5p, miR-150-3p, miR-423-5p, miR-575, miR-671-5p, miR-939-5p, miR-3665, miR-30d-5p, miR-30b-3p, miR-92a-3p, miR-371b-5p, and miR-486-5p, or to complementary strand(s) of the polynucleotide(s).

A4. The kit according to clause A3, wherein the nucleic acid(s) are polynucleotide(s) selected from the group consisting of the following polynucleotides (f) to (j):

(f) a polynucleotide consisting of a nucleotide sequence represented by any of SEQ ID NOs: 211 to 249, 1351 to 1356, and 1449 to 1453 or a nucleotide sequence derived from the nucleotide sequence by the replacement of u with t, a variant thereof, a derivative thereof, or a fragment thereof comprising 15 or more consecutive nucleotides,
(g) a polynucleotide comprising a nucleotide sequence represented by any of SEQ ID NOs: 211 to 249, 1351 to 1356, and 1449 to 1453,
(h) a polynucleotide consisting of a nucleotide sequence complementary to a nucleotide sequence represented by any of SEQ ID NOs: 211 to 249, 1351 to 1356, and 1449 to 1453 or a nucleotide sequence derived from the nucleotide sequence by the replacement of u with t, a variant thereof, a derivative thereof, or a fragment thereof comprising 15 or more consecutive nucleotides,

(i) a polynucleotide comprising a nucleotide sequence complementary to a nucleotide sequence represented by any of SEQ ID NOs: 211 to 249, 1351 to 1356, and 1449 to 1453 or a nucleotide sequence derived from the nucleotide sequence by the replacement of u with t, and

(j) a polynucleotide hybridizing under stringent conditions to any of the polynucleotides (f) to (i).

A5. The kit according to any one of clauses A1 to A4, wherein the kit further comprises nucleic acid(s) capable of specifically binding to one or more polynucleotide(s) selected from the group consisting of other dementia markers: miR-1471, miR-1538, miR-449b-3p, miR-1976, miR-4268, miR-4279, miR-3620-3p, miR-3944-3p, miR-3156-3p, miR-3187-5p, miR-4685-3p, miR-4695-3p, miR-4697-3p, miR-4713-5p, miR-4723-3p, miR-371b-3p, miR-3151-3p, miR-3192-3p, miR-6728-3p, miR-6736-3p, miR-6740-3p, miR-6741-3p, miR-6743-3p, miR-6747-3p, miR-6750-3p, miR-6754-3p, miR-6759-3p, miR-6761-3p, miR-6762-3p, miR-6769a-3p, miR-6776-3p, miR-6778-3p, miR-6779-3p, miR-6786-3p, miR-6787-3p, miR-6792-3p, miR-6794-3p, miR-6801-3p, miR-6802-3p, miR-6803-3p, miR-6804-3p, miR-6810-5p, miR-6823-3p, miR-6825-3p, miR-6829-3p, miR-6833-3p, miR-6834-3p, miR-6780b-3p, miR-6845-3p, miR-6862-3p, miR-6865-3p, miR-6870-3p, miR-6875-3p, miR-6877-3p, miR-6879-3p, miR-6882-3p, miR-6885-3p, miR-6886-3p, miR-6887-3p, miR-6890-3p, miR-6893-3p, miR-6894-3p, miR-7106-3p, miR-7109-3p, miR-7114-3p, miR-7155-5p, miR-7160-5p, miR-615-3p, miR-920, miR-1825, miR-675-3p, miR-1910-5p, miR-2278, miR-2682-3p, miR-3122, miR-3151-5p, miR-3175, miR-4323, miR-4326, miR-4284, miR-3605-3p, miR-3622b-5p, miR-3646, miR-3158-5p, miR-4722-3p, miR-4728-3p, miR-4747-3p, miR-4436b-5p, miR-5196-3p, miR-5589-5p, miR-345-3p, miR-642b-5p, miR-6716-3p, miR-6511b-3p, miR-208a-5p, miR-6726-3p, miR-6744-5p, miR-6782-3p, miR-6789-3p, miR-6797-3p, miR-6800-3p, miR-6806-5p, miR-6824-3p, miR-6837-5p, miR-6846-3p, miR-6858-3p, miR-6859-3p, miR-6861-3p, miR-6880-3p, miR-7111-3p, miR-7152-5p, miR-642a-5p, miR-657, miR-1236-3p, miR-764, miR-4314, miR-3675-3p, miR-5703, miR-3191-5p, miR-6511a-3p, miR-6809-3p, miR-6815-5p, miR-6857-3p, and miR-6878-3p, or to complementary strand(s) of the polynucleotide(s).

A6. The kit according to clause A5, wherein the nucleic acid(s) are polynucleotide(s) selected from the group consisting of the following polynucleotides (k) to (o):

(k) a polynucleotide consisting of a nucleotide sequence represented by any of SEQ ID NOs: 250 to 373 or a nucleotide sequence derived from the nucleotide sequence by the replacement of u with t, a variant thereof, a derivative thereof, or a fragment thereof comprising 15 or more consecutive nucleotides,

(1) a polynucleotide comprising a nucleotide sequence represented by any of SEQ ID NOs: 250 to 373,

(m) a polynucleotide consisting of a nucleotide sequence complementary to a nucleotide sequence represented by any of SEQ ID NOs: 250 to 373 or a nucleotide sequence derived from the nucleotide sequence by the replacement of u with t, a variant thereof, a derivative thereof, or a fragment thereof comprising 15 or more consecutive nucleotides,

(n) a polynucleotide comprising a nucleotide sequence complementary to a nucleotide sequence represented by any of SEQ ID NOs: 250 to 373 or a nucleotide sequence derived from the nucleotide sequence by the replacement of u with t, and

(o) a polynucleotide hybridizing under stringent conditions to any of the polynucleotides (k) to (n).

A7. The kit according to any one of clauses A1 to A6, wherein the kit further comprises nucleic acid(s) capable of specifically binding to one or more polynucleotide(s) selected from the group consisting of other dementia markers: miR-766-3p, miR-1229-3p, miR-1306-5p, miR-210-5p, miR-198, miR-485-3p, miR-668-3p, miR-532-3p, miR-877-3p, miR-1238-3p, miR-3130-5p, miR-4298, miR-4290, miR-3943, miR-346, and miR-767-3p, or to complementary strand(s) of the polynucleotide(s).

A8. The kit according to clause A7, wherein the nucleic acid(s) are polynucleotide(s) selected from the group consisting of the following polynucleotides (p) to (t):

(p) a polynucleotide consisting of a nucleotide sequence represented by any of SEQ ID NOs: 375 to 390 or a nucleotide sequence derived from the nucleotide sequence by the replacement of u with t, a variant thereof, a derivative thereof, or a fragment thereof comprising 15 or more consecutive nucleotides,

(q) a polynucleotide comprising a nucleotide sequence represented by any of SEQ ID NOs: 375 to 390,

(r) a polynucleotide consisting of a nucleotide sequence complementary to a nucleotide sequence represented by any of SEQ ID NOs: 375 to 390 or a nucleotide sequence derived from the nucleotide sequence by the replacement of u with t, a variant thereof, a derivative thereof, or a fragment thereof comprising 15 or more consecutive nucleotides,

(s) a polynucleotide comprising a nucleotide sequence complementary to a nucleotide sequence represented by any of SEQ ID NOs: 375 to 390 or a nucleotide sequence derived from the nucleotide sequence by the replacement of u with t, and

(t) a polynucleotide hybridizing under stringent conditions to any of the polynucleotides (p) to (s).

A9. A kit for detection or prediction of dementia, comprising nucleic acid(s) I capable of specifically binding to one or more polynucleotide(s) selected from the group consisting of dementia markers: miR-4728-5p, miR-3184-3p, miR-1233-3p, miR-6088, miR-6777-3p, miR-7110-3p, miR-936, miR-6087, miR-1202, miR-4731-3p, miR-4800-5p, miR-6784-3p, miR-4716-5p, miR-6734-3p, miR-6889-3p, miR-6867-3p, miR-3622a-3p, miR-6813-3p, miR-4769-3p, miR-5698, miR-3184-5p, miR-1471, miR-1538, miR-449b-3p, miR-1976, miR-4268, miR-4279, miR-3620-3p, miR-3944-3p, miR-3156-3p, miR-3187-5p, miR-4685-3p, miR-4695-3p, miR-4697-3p, miR-4713-5p, miR-4723-3p, miR-371b-3p, miR-3151-3p, miR-3192-3p, miR-6728-3p, miR-6736-3p, miR-6740-3p, miR-6741-3p, miR-6743-3p, miR-6747-3p, miR-6750-3p, miR-6754-3p, miR-6759-3p, miR-6761-3p, miR-6762-3p, miR-6769a-3p, miR-6776-3p, miR-6778-3p, miR-6779-3p, miR-6786-3p, miR-6787-3p, miR-6792-3p, miR-6794-3p, miR-6801-3p, miR-6802-3p, miR-6803-3p, miR-6804-3p, miR-6810-5p, miR-6823-3p, miR-6825-3p, miR-6829-3p, miR-6833-3p, miR-6834-3p, miR-6780b-3p, miR-6845-3p, miR-6862-3p, miR-6865-3p, miR-6870-3p, miR-6875-3p, miR-6877-3p, miR-6879-3p, miR-6882-3p, miR-6885-3p, miR-6886-3p, miR-6887-3p, miR-6890-3p, miR-6893-3p, miR-6894-3p, miR-7106-3p, miR-7109-3p, miR-7114-3p, miR-7155-5p, miR-7160-5p, miR-615-3p, miR-920, miR-1825, miR-675-3p, miR-1910-5p, miR-2278, miR-2682-3p, miR-3122, miR-3151-5p, miR-3175, miR-4323, miR-4326, miR-4284, miR-3605-3p, miR-3622b-5p, miR-3646, miR-3158-5p, miR-4722-3p, miR-4728-3p, miR-4747-3p, miR-4436b-5p, miR-5196-3p, miR-5589-5p, miR-345-3p, miR-642b-5p, miR-6716-3p, miR-6511b-3p, miR-208a-5p, miR-6726-3p, miR-6744-5p, miR-6782-3p, miR-6789-3p, miR-6797-3p, miR-6800-3p, miR-6806-5p, miR-6824-3p, miR-6837-5p, miR-6846-3p, miR-6858-3p, miR-6859-3p, miR-6861-3p, miR-6880-3p, miR-7111-3p, miR-7152-5p, miR-642a-5p, miR-657, miR-1236-3p, miR-764, miR-4314, miR-3675-3p, miR-5703, miR-3191-5p, miR-6511a-3p, miR-6809-3p, miR-6815-5p, miR-6857-3p, miR-6878-3p, and miR-371a-5p, or to complementary strand(s) of the polynucleotide(s), and

optionally further comprising nucleic acid(s) II capable of specifically binding to one or more polynucleotide(s) selected from the group consisting of: miR-766-3p, miR-1229-3p, miR-1306-5p, miR-210-5p, miR-198, miR-485-3p, miR-668-3p, miR-532-3p, miR-877-3p, miR-1238-3p, miR-3130-5p, miR-4298, miR-4290, miR-3943, miR-346, and miR-767-3p, or to complementary strand(s) of the polynucleotide(s).

A10. The kit according to clause A9, wherein the nucleic acid(s) I are polynucleotide(s) selected from the group consisting of the following polynucleotides (u) to (y):

(u) a polynucleotide consisting of a nucleotide sequence represented by any of SEQ ID NOs: 3, 8, 15, 26, 34, 40, 53, 69, 99, 101, 107, 109, 112, 125, 128, 133, 191 to 194, 212, and 250 to 374 or a nucleotide sequence derived from the nucleotide sequence by the replacement of u with t, a variant thereof, a derivative thereof, or a fragment thereof comprising 15 or more consecutive nucleotides,

(v) a polynucleotide comprising a nucleotide sequence represented by any of SEQ ID NOs: 3, 8, 15, 26, 34, 40, 53, 69, 99, 101, 107, 109, 112, 125, 128, 133, 191 to 194, 212, and 250 to 374,

(w) a polynucleotide consisting of a nucleotide sequence complementary to a nucleotide sequence represented by any of SEQ ID NOs: 3, 8, 15, 26, 34, 40, 53, 69, 99, 101, 107, 109, 112, 125, 128, 133, 191 to 194, 212, and 250 to 374 or a nucleotide sequence derived from the nucleotide sequence by the replacement of u with t, a variant thereof, a derivative thereof, or a fragment thereof comprising 15 or more consecutive nucleotides,

(x) a polynucleotide comprising a nucleotide sequence complementary to a nucleotide sequence represented by any of SEQ ID NOs: 3, 8, 15, 26, 34, 40, 53, 69, 99, 101, 107, 109, 112, 125, 128, 133, 191 to 194, 212, and 250 to 374 or a nucleotide sequence derived from the nucleotide sequence by the replacement of u with t, and

(y) a polynucleotide hybridizing under stringent conditions to any of the polynucleotides (u) to (x),

wherein the nucleic acid(s) II are polynucleotides selected from the group consisting of the following polynucleotides (p) to (t):

(p) a polynucleotide consisting of a nucleotide sequence represented by any of SEQ ID NOs: 375 to 390 or a nucleotide sequence derived from the nucleotide sequence by the replacement of u with t, a variant thereof, a derivative thereof, or a fragment thereof comprising 15 or more consecutive nucleotides,

(q) a polynucleotide comprising a nucleotide sequence represented by any of SEQ ID NOs: 375 to 390,

(r) a polynucleotide consisting of a nucleotide sequence complementary to a nucleotide sequence represented by any of SEQ ID NOs: 375 to 390 or a nucleotide sequence derived from the nucleotide sequence by the replacement of u with t, a variant thereof, a derivative thereof, or a fragment thereof comprising 15 or more consecutive nucleotides,

(s) a polynucleotide comprising a nucleotide sequence complementary to a nucleotide sequence represented by any of SEQ ID NOs: 375 to 390 or a nucleotide sequence derived from the nucleotide sequence by the replacement of u with t, and

(t) a polynucleotide hybridizing under stringent conditions to any of the polynucleotides (p) to (s).

A11. A device for detection of dementia, comprising nucleic acid(s) I capable of specifically binding to one or more polynucleotide(s) selected from the group consisting of dementia markers: miR-4274, miR-4272, miR-4728-5p, miR-4443, miR-4506, miR-6773-5p, miR-4662a-5p, miR-3184-3p, miR-4281, miR-320d, miR-6729-3p, miR-5192, miR-6853-5p, miR-1234-3p, miR-1233-3p, miR-4539, miR-3914, miR-4738-5p, miR-548au-3p, miR-1539, miR-4720-3p, miR-365b-5p, miR-4486, miR-1227-5p, miR-4667-5p, miR-6088, miR-6820-5p, miR-4505, miR-548q, miR-4658, miR-450a-5p, miR-1260b, miR-3677-5p, miR-6777-3p, miR-6826-3p, miR-6832-3p, miR-4725-3p, miR-7161-3p, miR-2277-5p, miR-7110-3p, miR-4312, miR-4461, miR-6766-5p, miR-1266-3p, miR-6729-5p, miR-526b-3p, miR-519e-5p, miR-512-5p, miR-5088-5p, miR-1909-3p, miR-6511a-5p, miR-4734, miR-936, miR-1249-3p, miR-6777-5p, miR-4487, miR-3155a, miR-563, miR-4741, miR-6788-5p, miR-4433b-5p, miR-323a-5p, miR-6811-5p, miR-6721-5p, miR-5004-5p, miR-6509-3p, miR-648, miR-3917, miR-6087, miR-1470, miR-586, miR-3150a-5p, miR-105-3p, miR-7973, miR-1914-5p, miR-4749-3p, miR-15b-5p, miR-1289, miR-4433a-5p, miR-3666, miR-3186-3p, miR-4725-5p, miR-4488, miR-4474-3p, miR-6731-3p, miR-4640-3p, miR-202-5p, miR-6816-5p, miR-638, miR-6821-5p, miR-1247-3p, miR-6765-5p, miR-6800-5p, miR-3928-3p, miR-3940-5p, miR-3960, miR-6775-5p, miR-3178, miR-1202, miR-6790-5p, miR-4731-3p, miR-2681-3p, miR-6758-5p, miR-8072, miR-518d-3p, miR-3606-3p, miR-4800-5p, miR-1292-3p, miR-6784-3p, miR-4450, miR-6132, miR-4716-5p, miR-6860, miR-1268b, miR-378d, miR-4701-5p, miR-4329, miR-185-3p, miR-552-3p, miR-1273g-5p, miR-6769b-3p, miR-520a-3p, miR-4524b-5p, miR-4291, miR-6734-3p, miR-143-5p, miR-939-3p, miR-6889-3p, miR-6842-3p, miR-4511, miR-4318, miR-4653-5p, miR-6867-3p, miR-133b, miR-3196, miR-193b-3p, miR-3162-3p, miR-6819-3p, miR-1908-3p, miR-6786-5p, miR-3648, miR-4513, miR-3652, miR-4640-5p, miR-6871-5p, miR-7845-5p, miR-3138, miR-6884-5p, miR-4653-3p, miR-636, miR-4652-3p, miR-6823-5p, miR-4502, miR-7113-5p, miR-8087, miR-7154-3p, miR-5189-5p, miR-1253, miR-518c-5p, miR-7151-5p, miR-3614-3p, miR-4727-5p, miR-3682-5p, miR-5090, miR-337-3p, miR-488-5p, miR-100-5p, miR-4520-3p, miR-373-3p, miR-6499-5p, miR-3909, miR-32-5p, miR-302a-3p, miR-4686, miR-4659a-3p, miR-4287, miR-1301-5p, miR-593-3p, miR-517a-3p, miR-517b-3p, miR-142-3p, miR-1185-2-3p, miR-602, miR-527, miR-518a-5p, miR-4682, miR-28-5p, miR-4252, miR-452-5p, miR-525-5p, miR-3622a-3p, miR-6813-3p, miR-4769-3p, miR-5698, miR-1915-3p, miR-1343-5p, miR-6861-5p, miR-6781-5p, miR-4508, miR-6743-5p, miR-6726-5p, miR-4525, miR-4651, miR-6813-5p, miR-5787, miR-1290, miR-6075, miR-4758-5p, miR-4690-5p, miR-762, miR-371a-5p, miR-6765-3p, miR-6784-5p, miR-6778-5p, miR-6875-5p, miR-4534, miR-4721, miR-6756-5p, miR-615-5p, miR-6727-5p, miR-6887-5p, miR-8063, miR-6880-5p, miR-6805-3p, miR-4726-5p, miR-4710, miR-7111-5p, miR-3619-3p, miR-6795-5p, miR-1254, miR-1233-5p, miR-6836-3p, miR-6769a-5p, miR-4532, miR-365a-5p, miR-1231, miR-1228-5p, miR-4430, miR-296-3p, miR-1237-5p, miR-4466, miR-6789-5p, miR-4632-5p, miR-4745-5p, miR-4665-5p, miR-6807-5p, miR-7114-5p, miR-516a-5p, miR-769-3p, miR-3692-5p, miR-3945, miR-4433a-3p, miR-4485-3p, miR-6831-5p, miR-519c-5p, miR-551b-5p, miR-1343-3p, miR-4286, miR-4634, miR-4733-3p, and miR-6086, or to complementary strand(s) of the polynucleotide(s).

A12. The device according to clause A11, wherein the nucleic acid(s) are polynucleotide(s) selected from the group consisting of the following polynucleotides (a) to (e):

(a) a polynucleotide consisting of a nucleotide sequence represented by any of SEQ ID NOs: 1 to 210, 374, 1315 to 1350, and 1435 to 1448 or a nucleotide sequence derived from the nucleotide sequence by the replacement of u with t, a variant thereof, a derivative thereof, or a fragment thereof comprising 15 or more consecutive nucleotides,

(b) a polynucleotide comprising a nucleotide sequence represented by any of SEQ ID NOs: 1 to 210, 374, 1315 to 1350, and 1435 to 1448,

(c) a polynucleotide consisting of a nucleotide sequence complementary to a nucleotide sequence represented by any of SEQ ID NOs: 1 to 210, 374, 1315 to 1350, and 1435 to 1448 or a nucleotide sequence derived from the nucleotide sequence by the replacement of u with t, a variant thereof, a derivative thereof, or a fragment thereof comprising 15 or more consecutive nucleotides,

(d) a polynucleotide comprising a nucleotide sequence complementary to a nucleotide sequence represented by any of SEQ ID NOs: 1 to 210, 374, 1315 to 1350, and 1435 to 1448 or a nucleotide sequence derived from the nucleotide sequence by the replacement of u with t, and

(e) a polynucleotide hybridizing under stringent conditions to any of the polynucleotides (a) to (d).

A13. The device according to clause A11 or A12, wherein the device further comprises nucleic acid(s) capable of specifically binding to one or more polynucleotide(s) selected from the group consisting of other dementia markers: miR-1225-3p, miR-3184-5p, miR-665, miR-211-5p, miR-1247-5p, miR-3656, miR-149-5p, miR-744-5p, miR-345-5p, miR-150-5p, miR-191-3p, miR-651-5p, miR-34a-5p, miR-409-5p, miR-369-5p, miR-1915-5p, miR-204-5p, miR-137, miR-382-5p, miR-517-5p, miR-532-5p, miR-22-5p, miR-1237-3p, miR-1224-3p, miR-625-3p, miR-328-3p, miR-122-5p, miR-202-3p, miR-4781-5p, miR-718, miR-342-3p, miR-26b-3p, miR-140-3p, miR-200a-3p, miR-378a-3p, miR-484, miR-296-5p, miR-205-5p, miR-431-5p, miR-150-3p, miR-423-5p, miR-575, miR-671-5p, miR-939-5p, miR-3665, miR-30d-5p, miR-30b-3p, miR-92a-3p, miR-371b-5p, and miR-486-5p, or to complementary strand(s) of the polynucleotide(s).

A14. The device according to clause A13, wherein the nucleic acid(s) are polynucleotide(s) selected from the group consisting of the following polynucleotides (f) to (j):

(f) a polynucleotide consisting of a nucleotide sequence represented by any of SEQ ID NOs: 211 to 249, 1351 to 1356, and 1449 to 1453 or a nucleotide sequence derived from the nucleotide sequence by the replacement of u with t, a variant thereof, a derivative thereof, or a fragment thereof comprising 15 or more consecutive nucleotides,

(g) a polynucleotide comprising a nucleotide sequence represented by any of SEQ ID NOs: 211 to 249, 1351 to 1356, and 1449 to 1453,

(h) a polynucleotide consisting of a nucleotide sequence complementary to a nucleotide sequence represented by any of SEQ ID NOs: 211 to 249, 1351 to 1356, and 1449 to 1453 or a nucleotide sequence derived from the nucleotide sequence by the replacement of u with t, a variant thereof, a derivative thereof, or a fragment thereof comprising 15 or more consecutive nucleotides,

(i) a polynucleotide comprising a nucleotide sequence complementary to a nucleotide sequence represented by any of SEQ ID NOs: 211 to 249, 1351 to 1356, and 1449 to 1453 or a nucleotide sequence derived from the nucleotide sequence by the replacement of u with t, and

(j) a polynucleotide hybridizing under stringent conditions to any of the polynucleotides (f) to (i).

A15. The device according to any one of clauses A11 to A14, wherein the device further comprises nucleic acid(s) capable of specifically binding to one or more polynucleotide(s) selected from the group consisting of other dementia markers: miR-1471, miR-1538, miR-449b-3p, miR-1976, miR-4268, miR-4279, miR-3620-3p, miR-3944-3p, miR-3156-3p, miR-3187-5p, miR-4685-3p, miR-4695-3p, miR-4697-3p, miR-4713-5p, miR-4723-3p, miR-371b-3p, miR-3151-3p, miR-3192-3p, miR-6728-3p, miR-6736-3p, miR-6740-3p, miR-6741-3p, miR-6743-3p, miR-6747-3p, miR-6750-3p, miR-6754-3p, miR-6759-3p, miR-6761-3p, miR-6762-3p, miR-6769a-3p, miR-6776-3p, miR-6778-3p, miR-6779-3p, miR-6786-3p, miR-6787-3p, miR-6792-3p, miR-6794-3p, miR-6801-3p, miR-6802-3p, miR-6803-3p, miR-6804-3p, miR-6810-5p, miR-6823-3p, miR-6825-3p, miR-6829-3p, miR-6833-3p, miR-6834-3p, miR-6780b-3p, miR-6845-3p, miR-6862-3p, miR-6865-3p, miR-6870-3p, miR-6875-3p, miR-6877-3p, miR-6879-3p, miR-6882-3p, miR-6885-3p, miR-6886-3p, miR-6887-3p, miR-6890-3p, miR-6893-3p, miR-6894-3p, miR-7106-3p, miR-7109-3p, miR-7114-3p, miR-7155-5p, miR-7160-5p, miR-615-3p, miR-920, miR-1825, miR-675-3p, miR-1910-5p, miR-2278, miR-2682-3p, miR-3122, miR-3151-5p, miR-3175, miR-4323, miR-4326, miR-4284, miR-3605-3p, miR-3622b-5p, miR-3646, miR-3158-5p, miR-4722-3p, miR-4728-3p, miR-4747-3p, miR-4436b-5p, miR-5196-3p, miR-5589-5p, miR-345-3p, miR-642b-5p, miR-6716-3p, miR-6511b-3p, miR-208a-5p, miR-6726-3p, miR-6744-5p, miR-6782-3p, miR-6789-3p, miR-6797-3p, miR-6800-3p, miR-6806-5p, miR-6824-3p, miR-6837-5p, miR-6846-3p, miR-6858-3p, miR-6859-3p, miR-6861-3p, miR-6880-3p, miR-7111-3p, miR-7152-5p, miR-642a-5p, miR-657, miR-1236-3p, miR-764, miR-4314, miR-3675-3p, miR-5703, miR-3191-5p, miR-6511a-3p, miR-6809-3p, miR-6815-5p, miR-6857-3p, and miR-6878-3p, or to complementary strand(s) of the polynucleotide(s).

A16. The device according to clause A15, wherein the nucleic acid(s) are polynucleotide(s) selected from the group consisting of the following polynucleotides (k) to (o):

(k) a polynucleotide consisting of a nucleotide sequence represented by any of SEQ ID NOs: 250 to 373 or a nucleotide sequence derived from the nucleotide sequence by the replacement of u with t, a variant thereof, a derivative thereof, or a fragment thereof comprising 15 or more consecutive nucleotides,

(l) a polynucleotide comprising a nucleotide sequence represented by any of SEQ ID NOs: 250 to 373,

(m) a polynucleotide consisting of a nucleotide sequence complementary to a nucleotide sequence represented by any of SEQ ID NOs: 250 to 373 or a nucleotide sequence derived from the nucleotide sequence by the replacement of u with t, a variant thereof, a derivative thereof, or a fragment thereof comprising 15 or more consecutive nucleotides,

(n) a polynucleotide comprising a nucleotide sequence complementary to a nucleotide sequence represented by any of SEQ ID NOs: 250 to 373 or a nucleotide sequence derived from the nucleotide sequence by the replacement of u with t, and

(o) a polynucleotide hybridizing under stringent conditions to any of the polynucleotides (k) to (n).

A17. The device according to any one of clauses A11 to A16, wherein the kit further comprises nucleic acid(s) capable of specifically binding to one or more polynucleotide(s) selected from the group consisting of other dementia markers: miR-766-3p, miR-1229-3p, miR-1306-5p, miR-210-5p, miR-198, miR-485-3p, miR-668-3p, miR-532-3p, miR-877-3p, miR-1238-3p, miR-3130-5p, miR-4298, miR-4290, miR-3943, miR-346, and miR-767-3p, or to complementary strand(s) of the polynucleotide(s).

A18. The device according to clause A17, wherein the nucleic acid(s) are polynucleotide(s) selected from the group consisting of the following polynucleotides (p) to (t):

(p) a polynucleotide consisting of a nucleotide sequence represented by any of SEQ ID NOs: 375 to 390 or a nucleotide sequence derived from the nucleotide sequence by the replacement of u with t, a variant thereof, a derivative thereof, or a fragment thereof comprising 15 or more consecutive nucleotides,

(q) a polynucleotide comprising a nucleotide sequence represented by any of SEQ ID NOs: 375 to 390,

(r) a polynucleotide consisting of a nucleotide sequence complementary to a nucleotide sequence represented by any of SEQ ID NOs: 375 to 390 or a nucleotide sequence derived from the nucleotide sequence by the replacement of u with t, a variant thereof, a derivative thereof, or a fragment thereof comprising 15 or more consecutive nucleotides,

(s) a polynucleotide comprising a nucleotide sequence complementary to a nucleotide sequence represented by any of SEQ ID NOs: 375 to 390 or a nucleotide sequence derived from the nucleotide sequence by the replacement of u with t, and

(t) a polynucleotide hybridizing under stringent conditions to any of the polynucleotides (p) to (s).

A19. A device for detection or prediction of dementia, comprising nucleic acid(s) I capable of specifically binding to one or more polynucleotide(s) selected from the group consisting of dementia markers: miR-4728-5p, miR-3184-3p, miR-1233-3p, miR-6088, miR-6777-3p, miR-7110-3p, miR-936, miR-6087, miR-1202, miR-4731-3p, miR-4800-5p, miR-6784-3p, miR-4716-5p, miR-6734-3p, miR-6889-3p, miR-6867-3p, miR-3622a-3p, miR-6813-3p, miR-4769-3p, miR-5698, miR-3184-5p, miR-1471, miR-1538, miR-449b-3p, miR-1976, miR-4268, miR-4279, miR-3620-3p, miR-3944-3p, miR-3156-3p, miR-3187-5p, miR-4685-3p, miR-4695-3p, miR-4697-3p, miR-4713-5p, miR-4723-3p, miR-371b-3p, miR-3151-3p, miR-3192-3p, miR-6728-3p, miR-6736-3p, miR-6740-3p, miR-6741-3p, miR-6743-3p, miR-6747-3p, miR-6750-3p, miR-6754-3p, miR-6759-3p, miR-6761-3p, miR-6762-3p, miR-6769a-3p, miR-6776-3p, miR-6778-3p, miR-6779-3p, miR-6786-3p, miR-6787-3p, miR-6792-3p, miR-6794-3p, miR-6801-3p, miR-6802-3p, miR-6803-3p, miR-6804-3p, miR-6810-5p, miR-6823-3p, miR-6825-3p, miR-6829-3p, miR-6833-3p, miR-6834-3p, miR-6780b-3p, miR-6845-3p, miR-6862-3p, miR-6865-3p, miR-6870-3p, miR-6875-3p, miR-6877-3p, miR-6879-3p, miR-6882-3p, miR-6885-3p, miR-6886-3p, miR-6887-3p, miR-6890-3p, miR-6893-3p, miR-6894-3p, miR-7106-3p, miR-7109-3p, miR-7114-3p, miR-7155-5p, miR-7160-5p, miR-615-3p, miR-920, miR-1825, miR-675-3p, miR-1910-5p, miR-2278, miR-2682-3p, miR-3122, miR-3151-5p, miR-3175, miR-4323, miR-4326, miR-4284, miR-3605-3p, miR-3622b-5p, miR-3646, miR-3158-5p, miR-4722-3p, miR-4728-3p, miR-4747-3p, miR-4436b-5p, miR-5196-3p, miR-5589-5p, miR-345-3p, miR-642b-5p, miR-6716-3p, miR-6511b-3p, miR-208a-5p, miR-6726-3p, miR-6744-5p, miR-6782-3p, miR-6789-3p, miR-6797-3p, miR-6800-3p, miR-6806-5p, miR-6824-3p, miR-6837-5p, miR-6846-3p, miR-6858-3p, miR-6859-3p, miR-6861-3p, miR-6880-3p, miR-7111-3p, miR-7152-5p, miR-642a-5p, miR-657, miR-1236-3p, miR-764, miR-4314, miR-3675-3p, miR-5703, miR-3191-5p, miR-6511a-3p, miR-6809-3p, miR-6815-5p, miR-6857-3p, miR-6878-3p, and miR-371a-5p, or to complementary strand(s) of the polynucleotide(s), and

optionally further comprising nucleic acid(s) II capable of specifically binding to one or more polynucleotide(s) selected from the group consisting of other dementia markers: miR-766-3p, miR-1229-3p, miR-1306-5p, miR-210-5p, miR-198, miR-485-3p, miR-668-3p, miR-532-3p, miR-877-3p, miR-1238-3p, miR-3130-5p, miR-4298, miR-4290, miR-3943, miR-346, and miR-767-3p, or to complementary strand(s) of the polynucleotide(s).

A20. The device according to clause A19, wherein the nucleic acid(s) I are polynucleotide(s) selected from the group consisting of the following polynucleotides (u) to (y):

(u) a polynucleotide consisting of a nucleotide sequence represented by any of SEQ ID NOs: 3, 8, 15, 26, 34, 40, 53, 69, 99, 101, 107, 109, 112, 125, 128, 133, 191 to 194, 212, and 250 to 374 or a nucleotide sequence derived from the nucleotide sequence by the replacement of u with t, a variant thereof, a derivative thereof, or a fragment thereof comprising 15 or more consecutive nucleotides,

(v) a polynucleotide comprising a nucleotide sequence represented by any of SEQ ID NOs: 3, 8, 15, 26, 34, 40, 53, 69, 99, 101, 107, 109, 112, 125, 128, 133, 191 to 194, 212, and 250 to 374,

(w) a polynucleotide consisting of a nucleotide sequence complementary to a nucleotide sequence represented by any of SEQ ID NOs: 3, 8, 15, 26, 34, 40, 53, 69, 99, 101, 107, 109, 112, 125, 128, 133, 191 to 194, 212, and 250 to 374 or a nucleotide sequence derived from the nucleotide sequence by the replacement of u with t, a variant thereof, a derivative thereof, or a fragment thereof comprising 15 or more consecutive nucleotides,

(x) a polynucleotide comprising a nucleotide sequence complementary to a nucleotide sequence represented by any of SEQ ID NOs: 3, 8, 15, 26, 34, 40, 53, 69, 99, 101, 107, 109, 112, 125, 128, 133, 191 to 194, 212, and 250 to 374 or a nucleotide sequence derived from the nucleotide sequence by the replacement of u with t, and

(y) a polynucleotide hybridizing under stringent conditions to any of the polynucleotides (u) to (x),

wherein the nucleic acid(s) II are polynucleotide(s) selected from the group consisting of the following polynucleotides (p) to (t):

(p) a polynucleotide consisting of a nucleotide sequence represented by any of SEQ ID NOs: 375 to 390 or a nucleotide sequence derived from the nucleotide sequence by the replacement of u with t, a variant thereof, a derivative thereof, or a fragment thereof comprising 15 or more consecutive nucleotides,

(q) a polynucleotide comprising a nucleotide sequence represented by any of SEQ ID NOs: 375 to 390,

(r) a polynucleotide consisting of a nucleotide sequence complementary to a nucleotide sequence represented by any of SEQ ID NOs: 375 to 390 or a nucleotide sequence derived from the nucleotide sequence by the replacement of u with t, a variant thereof, a derivative thereof, or a fragment thereof comprising 15 or more consecutive nucleotides,

(s) a polynucleotide comprising a nucleotide sequence complementary to a nucleotide sequence represented by any of SEQ ID NOs: 375 to 390 or a nucleotide sequence derived from the nucleotide sequence by the replacement of u with t, and

(t) a polynucleotide hybridizing under stringent conditions to any of the polynucleotides (p) to (s).

A21. The device according to any one of clauses A11 to A20, wherein the device is a device for measurement by a hybridization technique.

A22. The device according to clause A21, wherein the hybridization technique is a nucleic acid array technique.

A23. A method for detecting dementia, comprising measuring expression level(s) of one or more polynucleotide(s) selected from the group consisting of dementia markers: miR-4274, miR-4272, miR-4728-5p, miR-4443, miR-4506, miR-6773-5p, miR-4662a-5p, miR-3184-3p, miR-4281, miR-320d, miR-6729-3p, miR-5192, miR-6853-5p, miR-1234-3p, miR-1233-3p, miR-4539, miR-3914, miR-4738-5p, miR-548au-3p, miR-1539, miR-4720-3p, miR-365b-5p, miR-4486, miR-1227-5p, miR-4667-5p, miR-6088, miR-6820-5p, miR-4505, miR-548q, miR-4658, miR-450a-5p, miR-1260b, miR-3677-5p, miR-6777-3p, miR-6826-3p, miR-6832-3p, miR-4725-3p, miR-7161-3p, miR-2277-5p, miR-7110-3p, miR-4312, miR-4461, miR-6766-5p, miR-1266-3p, miR-6729-5p, miR-526b-3p, miR-519e-5p, miR-512-5p, miR-5088-5p, miR-1909-3p, miR-6511a-5p, miR-4734, miR-936, miR-1249-3p, miR-6777-5p, miR-4487, miR-3155a, miR-563, miR-4741, miR-6788-5p, miR-4433b-5p, miR-323a-5p, miR-6811-5p, miR-6721-5p, miR-5004-5p, miR-6509-3p, miR-648, miR-3917, miR-6087, miR-1470, miR-586, miR-3150a-5p, miR-105-3p, miR-7973, miR-1914-5p, miR-4749-3p, miR-15b-5p, miR-1289, miR-4433a-5p, miR-3666, miR-3186-3p, miR-4725-5p, miR-4488, miR-4474-3p, miR-6731-3p, miR-4640-3p, miR-202-5p, miR-6816-5p, miR-638, miR-6821-5p, miR-1247-3p, miR-6765-5p, miR-6800-5p, miR-3928-3p, miR-3940-5p, miR-3960, miR-6775-5p, miR-3178, miR-1202, miR-6790-5p, miR-4731-3p, miR-2681-3p, miR-6758-5p, miR-8072, miR-518d-3p, miR-3606-3p, miR-4800-5p, miR-1292-3p, miR-6784-3p, miR-4450, miR-6132, miR-4716-5p, miR-6860, miR-1268b, miR-378d, miR-4701-5p, miR-4329, miR-185-3p, miR-552-3p, miR-1273g-5p, miR-6769b-3p, miR-520a-3p, miR-4524b-5p, miR-4291, miR-6734-3p, miR-143-5p, miR-939-3p, miR-6889-3p, miR-6842-3p, miR-4511, miR-4318, miR-4653-5p, miR-6867-3p, miR-133b, miR-3196, miR-193b-3p, miR-3162-3p, miR-6819-3p, miR-1908-3p, miR-6786-5p, miR-3648, miR-4513, miR-3652, miR-4640-5p, miR-6871-5p, miR-7845-5p, miR-3138, miR-6884-5p, miR-4653-3p, miR-636, miR-4652-3p, miR-6823-5p, miR-4502, miR-7113-5p, miR-8087, miR-7154-3p, miR-5189-5p, miR-1253, miR-518c-5p, miR-7151-5p, miR-3614-3p, miR-4727-5p, miR-3682-5p, miR-5090, miR-337-3p, miR-488-5p, miR-100-5p, miR-4520-3p, miR-373-3p, miR-6499-5p, miR-3909, miR-32-5p, miR-302a-3p, miR-4686, miR-4659a-3p, miR-4287, miR-1301-5p, miR-593-3p, miR-517a-3p, miR-517b-3p, miR-142-3p, miR-1185-2-3p, miR-602, miR-527, miR-518a-5p, miR-4682, miR-28-5p, miR-4252, miR-452-5p, miR-525-5p, miR-3622a-3p, miR-6813-3p, miR-4769-3p, miR-5698, miR-1915-3p, miR-1343-5p, miR-6861-5p, miR-6781-5p, miR-4508, miR-6743-5p, miR-6726-5p, miR-4525, miR-4651, miR-6813-5p, miR-5787, miR-1290, miR-6075, miR-4758-5p, miR-4690-5p, miR-762, miR-371a-5p, miR-6765-3p, miR-6784-5p, miR-6778-5p, miR-6875-5p, miR-4534, miR-4721, miR-6756-5p, miR-615-5p, miR-6727-5p, miR-6887-5p, miR-8063, miR-6880-5p, miR-6805-3p, miR-4726-5p, miR-4710, miR-7111-5p, miR-3619-3p, miR-6795-5p, miR-1254, miR-1233-5p, miR-6836-3p, miR-6769a-5p, miR-4532, miR-365a-5p, miR-1231, miR-1228-5p, miR-4430, miR-296-3p, miR-1237-5p, miR-4466, miR-6789-5p, miR-4632-5p, miR-4745-5p, miR-4665-5p, miR-6807-5p, miR-7114-5p, miR-516a-5p, miR-769-3p, miR-3692-5p, miR-3945, miR-4433a-3p, miR-4485-3p, miR-6831-5p, miR-519c-5p, miR-551b-5p, miR-1343-3p, miR-4286, miR-4634, miR-4733-3p, and miR-6086 in a sample from a subject; and evaluating *in vitro* whether or not the subject has dementia using the measured expression level(s).

A24. The method according to clause A23, comprising plugging the gene expression level(s) of the one or more polynucleotide(s) in the sample from the subject into a discriminant formula capable of discriminating dementia or non-dementia distinctively, wherein the discriminant formula is created by using gene expression levels in samples from subjects known to have dementia and gene expression levels in samples from non-dementia subjects as training samples; and thereby evaluating whether or not the subject has dementia.

A25. The method according to clause A23 or A24, wherein the expression level(s) of the polynucleotide(s) are measured by using nucleic acid(s) capable of specifically binding to the polynucleotide(s) or to complementary strand(s) of the polynucleotide(s), and
wherein the nucleic acid(s) are polynucleotide(s) selected from the group consisting of the following polynucleotides (a) to (e):

(a) a polynucleotide consisting of a nucleotide sequence represented by any of SEQ ID NOs: 1 to 210, 374, 1315 to 1350, and 1435 to 1448 or a nucleotide sequence derived from the nucleotide sequence by the replacement of u with t, a variant thereof, a derivative thereof, or a fragment thereof comprising 15 or more consecutive nucleotides,
(b) a polynucleotide comprising a nucleotide sequence represented by any of SEQ ID NOs: 1 to 210, 374, 1315 to 1350, and 1435 to 1448,
(c) a polynucleotide consisting of a nucleotide sequence complementary to a nucleotide sequence represented by any of SEQ ID NOs: 1 to 210, 374, 1315 to 1350, and 1435 to 1448 or a nucleotide sequence derived from the nucleotide sequence by the replacement of u with t, a variant thereof, a derivative thereof, or a fragment thereof comprising 15 or more consecutive nucleotides,
(d) a polynucleotide comprising a nucleotide sequence complementary to a nucleotide sequence represented by any of SEQ ID NOs: 1 to 210, 374, 1315 to 1350, and 1435 to 1448 or a nucleotide sequence derived from the nucleotide sequence by the replacement of u with t, and
(e) a polynucleotide hybridizing under stringent conditions to any of the polynucleotides (a) to (d).

A26. The method according to any one of clauses A23 to A25, further comprising measuring expression level(s) of one or more polynucleotide(s) selected from the group consisting of other dementia markers: miR-1225-3p, miR-3184-5p, miR-665, miR-211-5p, miR-1247-5p, miR-3656, miR-149-5p, miR-744-5p, miR-345-5p, miR-150-5p, miR-191-3p, miR-651-5p, miR-34a-5p, miR-409-5p, miR-369-5p, miR-1915-5p, miR-204-5p, miR-137, miR-382-5p, miR-517-5p, miR-532-5p, miR-22-5p, miR-1237-3p, miR-1224-3p, miR-625-3p, miR-328-3p, miR-122-5p, miR-202-3p, miR-4781-5p, miR-718, miR-342-3p, miR-26b-3p, miR-140-3p, miR-200a-3p, miR-378a-3p, miR-484, miR-296-5p, miR-205-5p, miR-431-5p, miR-150-3p, miR-423-5p, miR-575, miR-671-5p, miR-939-5p, miR-3665, miR-30d-5p, miR-30b-3p, miR-92a-3p, miR-371b-5p, and miR-486-5p.

A27. The method according to clause A26, wherein the expression level(s) of the polynucleotide(s) are measured by using nucleic acid(s) capable of specifically binding to the polynucleotide(s) or to complementary strand(s) of the polynucleotide(s), and
wherein the nucleic acid(s) are polynucleotide(s) selected from the group consisting of the following polynucleotides (f) to (j):

(f) a polynucleotide consisting of a nucleotide sequence represented by any of SEQ ID NOs: 211 to 249, 1351 to 1356, and 1449 to 1453 or a nucleotide sequence derived from the nucleotide sequence by the replacement of u with t, a variant thereof, a derivative thereof, or a fragment thereof comprising 15 or more consecutive nucleotides,

(g) a polynucleotide comprising a nucleotide sequence represented by any of SEQ ID NOs: 211 to 249, 1351 to 1356, and 1449 to 1453,

(h) a polynucleotide consisting of a nucleotide sequence complementary to a nucleotide sequence represented by any of SEQ ID NOs: 211 to 249, 1351 to 1356, and 1449 to 1453 or a nucleotide sequence derived from the nucleotide sequence by the replacement of u with t, a variant thereof, a derivative thereof, or a fragment thereof comprising 15 or more consecutive nucleotides,

(i) a polynucleotide comprising a nucleotide sequence complementary to a nucleotide sequence represented by any of SEQ ID NOs: 211 to 249, 1351 to 1356, and 1449 to 1453 or a nucleotide sequence derived from the nucleotide sequence by the replacement of u with t, and

(j) a polynucleotide hybridizing under stringent conditions to any of the polynucleotides (f) to (i).

A28. The method according to any one of clauses A23 to A27, further comprising measuring expression level(s) of one or more polynucleotide(s) selected from the group consisting of other dementia markers: miR-1471, miR-1538, miR-449b-3p, miR-1976, miR-4268, miR-4279, miR-3620-3p, miR-3944-3p, miR-3156-3p, miR-3187-5p, miR-4685-3p, miR-4695-3p, miR-4697-3p, miR-4713-5p, miR-4723-3p, miR-371b-3p, miR-3151-3p, miR-3192-3p, miR-6728-3p, miR-6736-3p, miR-6740-3p, miR-6741-3p, miR-6743-3p, miR-6747-3p, miR-6750-3p, miR-6754-3p, miR-6759-3p, miR-6761-3p, miR-6762-3p, miR-6769a-3p, miR-6776-3p, miR-6778-3p, miR-6779-3p, miR-6786-3p, miR-6787-3p, miR-6792-3p, miR-6794-3p, miR-6801-3p, miR-6802-3p, miR-6803-3p, miR-6804-3p, miR-6810-5p, miR-6823-3p, miR-6825-3p, miR-6829-3p, miR-6833-3p, miR-6834-3p, miR-6780b-3p, miR-6845-3p, miR-6862-3p, miR-6865-3p, miR-6870-3p, miR-6875-3p, miR-6877-3p, miR-6879-3p, miR-6882-3p, miR-6885-3p, miR-6886-3p, miR-6887-3p, miR-6890-3p, miR-6893-3p, miR-6894-3p, miR-7106-3p, miR-7109-3p, miR-7114-3p, miR-7155-5p, miR-7160-5p, miR-615-3p, miR-920, miR-1825, miR-675-3p, miR-1910-5p, miR-2278, miR-2682-3p, miR-3122, miR-3151-5p, miR-3175, miR-4323, miR-4326, miR-4284, miR-3605-3p, miR-3622b-5p, miR-3646, miR-3158-5p, miR-4722-3p, miR-4728-3p, miR-4747-3p, miR-4436b-5p, miR-5196-3p, miR-5589-5p, miR-345-3p, miR-642b-5p, miR-6716-3p, miR-6511b-3p, miR-208a-5p, miR-6726-3p, miR-6744-5p, miR-6782-3p, miR-6789-3p, miR-6797-3p, miR-6800-3p, miR-6806-5p, miR-6824-3p, miR-6837-5p, miR-6846-3p, miR-6858-3p, miR-6859-3p, miR-6861-3p, miR-6880-3p, miR-7111-3p, miR-7152-5p, miR-642a-5p, miR-657, miR-1236-3p, miR-764, miR-4314, miR-3675-3p, miR-5703, miR-3191-5p, miR-6511a-3p, miR-6809-3p, miR-6815-5p, miR-6857-3p, and miR-6878-3p.

A29. The method according to clause A28, wherein the expression level(s) of the polynucleotide(s) are measured by using nucleic acid(s) capable of specifically binding to the polynucleotide(s) or to complementary strand(s) of the polynucleotide(s), and

wherein the nucleic acid(s) are polynucleotide(s) selected from the group consisting of the following polynucleotides (k) to (o):

(k) a polynucleotide consisting of a nucleotide sequence represented by any of SEQ ID NOs: 250 to 373 or a nucleotide sequence derived from the nucleotide sequence by the replacement of u with t, a variant thereof, a derivative thereof, or a fragment thereof comprising 15 or more consecutive nucleotides,

(l) a polynucleotide comprising a nucleotide sequence represented by any of SEQ ID NOs: 250 to 373,

(m) a polynucleotide consisting of a nucleotide sequence complementary to a nucleotide sequence represented by any of SEQ ID NOs: 250 to 373 or a nucleotide sequence derived from the nucleotide sequence by the replacement of u with t, a variant thereof, a derivative thereof, or a fragment thereof comprising 15 or more consecutive nucleotides,

(n) a polynucleotide comprising a nucleotide sequence complementary to a nucleotide sequence represented by any of SEQ ID NOs: 250 to 373 or a nucleotide sequence derived from the nucleotide sequence by the replacement of u with t, and

(o) a polynucleotide hybridizing under stringent conditions to any of the polynucleotides (k) to (n).

A30. The method according to any one of clauses A23 to A29, further comprising measuring expression level(s) of one or more polynucleotide(s) selected from the group consisting of other dementia markers: miR-766-3p, miR-1229-3p, miR-1306-5p, miR-210-5p, miR-198, miR-485-3p, miR-668-3p, miR-532-3p, miR-877-3p, miR-1238-3p, miR-3130-5p, miR-4298, miR-4290, miR-3943, miR-346, and miR-767-3p.

A31. The method according to clause A30, wherein the expression level(s) of the polynucleotide(s) are measured by using nucleic acid(s) capable of specifically binding to the polynucleotide(s) or to the complementary strand(s) of the polynucleotide(s), and

wherein the nucleic acid(s) are polynucleotide(s) selected from the group consisting of the following polynucleotides (p) to (t):

(p) a polynucleotide consisting of a nucleotide sequence represented by any of SEQ ID NOs: 375 to 390 or a nucleotide sequence derived from the nucleotide sequence by the replacement of u with t, a variant thereof, a derivative thereof, or a fragment thereof comprising 15 or more consecutive nucleotides,

(q) a polynucleotide comprising a nucleotide sequence represented by any of SEQ ID NOs: 375 to 390,

(r) a polynucleotide consisting of a nucleotide sequence complementary to a nucleotide sequence represented by any of SEQ ID NOs: 375 to 390 or a nucleotide sequence derived from the nucleotide sequence by the replacement of u with t, a variant thereof, a derivative thereof, or a fragment thereof comprising 15 or more consecutive nucleotides,

(s) a polynucleotide comprising a nucleotide sequence complementary to a nucleotide sequence represented by any of SEQ ID NOs: 375 to 390 or a nucleotide sequence derived from the nucleotide sequence by the replacement of u with t, and

(t) a polynucleotide hybridizing under stringent conditions to any of the polynucleotides (p) to (s).

A32. A method for detecting or predicting dementia, comprising:

measuring expression level(s) of one or more polynucleotide(s) I selected from the group consisting of dementia markers: miR-4728-5p, miR-3184-3p, miR-1233-3p, miR-6088, miR-6777-3p, miR-7110-3p, miR-936, miR-6087, miR-1202, miR-4731-3p, miR-4800-5p, miR-6784-3p, miR-4716-5p, miR-6734-3p, miR-6889-3p, miR-6867-3p, miR-3622a-3p, miR-6813-3p, miR-4769-3p, miR-5698, miR-3184-5p, miR-1471, miR-1538, miR-449b-3p, miR-1976, miR-4268, miR-4279, miR-3620-3p, miR-3944-3p, miR-3156-3p, miR-3187-5p, miR-4685-3p, miR-4695-3p, miR-4697-3p, miR-4713-5p, miR-4723-3p, miR-371b-3p, miR-3151-3p, miR-3192-3p, miR-6728-3p, miR-6736-3p, miR-6740-3p, miR-6741-3p, miR-6743-3p, miR-6747-3p, miR-6750-3p, miR-6754-3p, miR-6759-3p, miR-6761-3p, miR-6762-3p, miR-6769a-3p, miR-6776-3p, miR-6778-3p, miR-6779-3p, miR-6786-3p, miR-6787-3p, miR-6792-3p, miR-6794-3p, miR-6801-3p, miR-6802-3p, miR-6803-3p, miR-6804-3p, miR-6810-5p, miR-6823-3p, miR-6825-3p, miR-6829-3p, miR-6833-3p, miR-6834-3p, miR-6780b-3p, miR-6845-3p, miR-6862-3p, miR-6865-3p, miR-6870-3p, miR-6875-3p, miR-6877-3p, miR-6879-3p, miR-6882-3p, miR-6885-3p, miR-6886-3p, miR-6887-3p, miR-6890-3p, miR-6893-3p, miR-6894-3p, miR-7106-3p, miR-7109-3p, miR-7114-3p, miR-7155-5p, miR-7160-5p, miR-615-3p, miR-920, miR-1825, miR-675-3p, miR-1910-5p, miR-2278, miR-2682-3p, miR-3122, miR-3151-5p, miR-3175, miR-4323, miR-4326, miR-4284, miR-3605-3p, miR-3622b-5p, miR-3646, miR-3158-5p, miR-4722-3p, miR-4728-3p, miR-4747-3p, miR-4436b-5p, miR-5196-3p, miR-5589-5p, miR-345-3p, miR-642b-5p, miR-6716-3p, miR-6511b-3p, miR-208a-5p, miR-6726-3p, miR-6744-5p, miR-6782-3p, miR-6789-3p, miR-6797-3p, miR-6800-3p, miR-6806-5p, miR-6824-3p, miR-6837-5p, miR-6846-3p, miR-6858-3p, miR-6859-3p, miR-6861-3p, miR-6880-3p, miR-7111-3p, miR-7152-5p, miR-642a-5p, miR-657, miR-1236-3p, miR-764, miR-4314, miR-3675-3p, miR-5703, miR-3191-5p, miR-6511a-3p, miR-6809-3p, miR-6815-5p, miR-6857-3p, miR-6878-3p, and miR-371a-5p, further optionally measuring expression level(s) of one or more polynucleotide(s) II selected from the group consisting of other dementia markers: miR-766-3p, miR-1229-3p, miR-1306-5p, miR-210-5p, miR-198, miR-485-3p, miR-668-3p, miR-532-3p, miR-877-3p, miR-1238-3p, miR-3130-5p, miR-4298, miR-4290, miR-3943, miR-346, and miR-767-3p, and evaluating *in vitro* whether or not the subject has dementia or predicting *in vitro* the possibility for the subject to develop dementia using the measured expression level(s).

A33. The method according to clause A32, wherein the expression level(s) of the polynucleotide(s) I are measured by using nucleic acid(s) capable of specifically binding to the polynucleotide(s) I or to complementary strand(s) of the polynucleotide(s),

wherein the nucleic acid(s) are polynucleotide(s) selected from the group consisting of the following polynucleotides (u) to (y):

(u) a polynucleotide consisting of a nucleotide sequence represented by any of SEQ ID NOs: 3, 8, 15, 26, 34, 40, 53, 69, 99, 101, 107, 109, 112, 125, 128, 133, 191 to 194, 212, and 250 to 374 or a nucleotide sequence derived from the nucleotide sequence by the replacement of u with t, a variant thereof, a derivative thereof, or a fragment thereof comprising 15 or more consecutive nucleotides,

(v) a polynucleotide comprising a nucleotide sequence represented by any of SEQ ID NOs: 3, 8, 15, 26, 34, 40, 53, 69, 99, 101, 107, 109, 112, 125, 128, 133, 191 to 194, 212, and 250 to 374,

(w) a polynucleotide consisting of a nucleotide sequence complementary to a nucleotide sequence represented by any of SEQ ID NOs: 3, 8, 15, 26, 34, 40, 53, 69, 99, 101, 107, 109, 112, 125, 128, 133, 191 to 194, 212, and 250 to 374 or a nucleotide sequence derived from the nucleotide sequence by the replacement of u with t, a variant thereof, a derivative thereof, or a fragment thereof comprising 15 or more consecutive nucleotides,

(x) a polynucleotide comprising a nucleotide sequence complementary to a nucleotide sequence represented by any of SEQ ID NOs: 3, 8, 15, 26, 34, 40, 53, 69, 99, 101, 107, 109, 112, 125, 128, 133, 191 to 194, 212, and 250 to 374 or a nucleotide sequence derived from the nucleotide sequence by the replacement of u with t, and

(y) a polynucleotide hybridizing under stringent conditions to any of the polynucleotides (u) to (x),

wherein the expression level(s) of the polynucleotide(s) II are measured by using nucleic acid(s) capable of specifically binding to the polynucleotide(s) II or to complementary strand(s) of the polynucleotide(s), wherein the nucleic acid(s) are polynucleotide(s) selected from the group consisting of the following polynucleotides (p) to (t):

(p) a polynucleotide consisting of a nucleotide sequence represented by any of SEQ ID NOs: 375 to 390 or a nucleotide sequence derived from the nucleotide sequence by the replacement of u with t, a variant thereof, a derivative thereof, or a fragment thereof comprising 15 or more consecutive nucleotides,

(q) a polynucleotide comprising a nucleotide sequence represented by any of SEQ ID NOs: 375 to 390,

(r) a polynucleotide consisting of a nucleotide sequence complementary to a nucleotide sequence represented by any of SEQ ID NOs: 375 to 390 or a nucleotide sequence derived from the nucleotide sequence by the replacement of u with t, a variant thereof, a derivative thereof, or a fragment thereof comprising 15 or more consecutive nucleotides,

(s) a polynucleotide comprising a nucleotide sequence complementary to a nucleotide sequence represented by any of SEQ ID NOs: 375 to 390 or a nucleotide sequence derived from the nucleotide sequence by the replacement of u with t, and

(t) a polynucleotide hybridizing under stringent conditions to any of the polynucleotides (p) to (s).

A34. The method according to any one of clauses A23 to A33, wherein the expression level(s) of the target gene(s) in the sample from the subject are measured by using the kit according to any one of claims 1 to 10 or the device according to any one of claims 11 to 22.

A35. The method according to any one of clauses A23 to A34, the subject is a human.

A36. The method according to any one of clauses A23 to A35, the sample is blood, serum, or plasma.

The following numbered clauses contain further statements of various aspects of the present invention

1. Use of a kit for detection of dementia, comprising nucleic acids capable of specifically binding to polynucleotides dementia markers miR-4486, miR-6088 and miR-211-5p, or to complementary strands of the polynucleotides.

2. Use of a device for detection of dementia, comprising nucleic acids capable of specifically binding to polynucleotide dementia markers miR-4486, miR-6088 and miR-211-5p, or to complementary strands of the polynucleotides.

3. The use of the kit according to clause 1 or the use of the device according to clause 2, wherein the nucleic acids are polynucleotides selected from the group consisting of the following polynucleotides (a) to (e):

(a) a polynucleotide consisting of a nucleotide sequence represented by SEQ ID NO: 23, 26 or 214, or a nucleotide sequence derived from the nucleotide sequence by the replacement of u with t, or a fragment thereof comprising 15 or more consecutive nucleotides,

(b) a polynucleotide comprising a nucleotide sequence represented by SEQ ID NO: 23, 26 or 214, or a nucleotide sequence derived from the nucleotide sequence by the replacement of u with t,

(c) a polynucleotide consisting of a nucleotide sequence complementary to a nucleotide sequence represented by SEQ ID NO: 23, 26 or 214, or a nucleotide sequence derived from the nucleotide sequence by the replacement of u with t, or a fragment thereof comprising 15 or more consecutive nucleotides,

(d) a polynucleotide comprising a nucleotide sequence complementary to a nucleotide sequence represented by SEQ ID NO: 23, 26 or 214, or a nucleotide sequence derived from the nucleotide sequence by the replacement of u with t, and

(e) a polynucleotide hybridizing under stringent conditions to any of the polynucleotides (a) to (d).

4. The use of the kit according to clause 1 or 3 or the use of the device according to clause 2 or 3, wherein the kit or the device further comprises nucleic acid(s) capable of specifically binding to one or more polynucleotide(s) selected from the group consisting of other dementia markers: miR-4274, miR-4272, miR-4443, miR-4506, miR-6773-5p, miR-4662a-5p, miR-3184-3p, miR-4281, miR-320d, miR-6729-3p, miR-5192, miR-6853-5p, miR-1234-3p, miR-1233-3p, miR-4539, miR-3914, miR-4738-5p, miR-548au-3p, miR-1539, miR-4720-3p, miR-365b-5p, miR-1227-5p, miR-4667-5p, miR-6820-5p, miR-4505, miR-548q, miR-4658, miR-450a-5p, miR-1260b, miR-3677-5p, miR-6777-3p, miR-6826-3p, miR-6832-3p, miR-4725-3p, miR-7161-3p, miR-2277-5p, miR-7110-3p, miR-4312, miR-4461, miR-6766-5p, miR-1266-3p, miR-6729-5p, miR-526b-3p, miR-519e-5p, miR-512-5p, miR-5088-5p, miR-1909-3p, miR-6511a-5p, miR-4734, miR-936, miR-1249-3p, miR-6777-5p, miR-4487, miR-3155a, miR-563, miR-4741, miR-6788-5p, miR-4433b-5p, miR-323a-5p, miR-6811-5p, miR-6721-5p, miR-5004-5p, miR-6509-3p, miR-648, miR-3917, miR-6087, miR-1470, miR-586, miR-3150a-5p, miR-105-3p, miR-7973, miR-1914-5p, miR-4749-3p, miR-15b-5p, miR-1289, miR-4433a-5p, miR-3666, miR-3186-3p, miR-4725-5p, miR-4488, miR-4474-3p, miR-6731-3p, miR-4640-3p, miR-202-5p, miR-6816-5p, miR-638, miR-6821-5p, miR-1247-3p, miR-6765-5p, miR-6800-5p, miR-3928-3p, miR-3940-5p, miR-3960, miR-6775-5p, miR-3178, miR-1202, miR-6790-5p, miR-4731-3p, miR-2681-3p, miR-6758-5p, miR-8072, miR-518d-3p, miR-3606-3p, miR-4800-5p, miR-1292-3p, miR-6784-3p, miR-4450, miR-6132, miR-4716-5p, miR-6860, miR-1268b, miR-378d, miR-4701-5p, miR-4329, miR-185-3p, miR-552-3p, miR-1273g-5p, miR-6769b-3p, miR-520a-3p, miR-4524b-5p, miR-4291, miR-6734-3p, miR-143-5p, miR-939-3p, miR-6889-3p, miR-6842-3p, miR-4511, miR-4318, miR-4653-5p, miR-6867-3p, miR-133b, miR-3196, miR-193b-3p, miR-3162-3p, miR-6819-3p, miR-1908-3p, miR-6786-5p, miR-3648, miR-4513, miR-3652, miR-4640-5p, miR-6871-5p, miR-7845-5p, miR-3138, miR-6884-5p, miR-4653-3p, miR-636, miR-4652-3p, miR-6823-5p, miR-4502, miR-7113-5p, miR-8087, miR-7154-3p, miR-5189-5p, miR-1253, miR-518c-5p, miR-7151-5p, miR-3614-3p, miR-4727-5p, miR-3682-5p, miR-5090, miR-337-3p, miR-488-5p, miR-100-5p, miR-4520-3p, miR-373-3p, miR-6499-5p, miR-3909, miR-32-5p, miR-302a-3p, miR-4686, miR-4659a-3p, miR-4287, miR-1301-5p, miR-593-3p, miR-517a-3p, miR-517b-3p, miR-142-3p, miR-1185-2-3p, miR-602, miR-527, miR-518a-5p, miR-4682, miR-28-5p, miR-4252, miR-452-5p, miR-525-5p, miR-3622a-3p, miR-6813-3p, miR-4769-3p, miR-5698, miR-1915-3p, miR-1343-5p, miR-6861-5p, miR-6781-5p, miR-4508, miR-6743-5p, miR-6726-5p, miR-4525, miR-4651, miR-6813-5p, miR-5787, miR-1290, miR-6075, miR-4758-5p, miR-4690-5p, miR-762, miR-371a-5p, miR-6765-3p, miR-6784-5p, miR-6778-5p, miR-6875-5p, miR-4534, miR-4721, miR-6756-5p, miR-615-5p, miR-6727-5p, miR-6887-5p, miR-8063, miR-6880-5p, miR-6805-3p, miR-4726-5p, miR-4710, miR-7111-5p, miR-3619-3p, miR-6795-5p, miR-1254, miR-1233-5p, miR-6836-3p, miR-6769a-5p, miR-4532, miR-365a-5p, miR-1231, miR-1228-5p, miR-4430, miR-296-3p, miR-1237-5p, miR-4466, miR-6789-5p, miR-4632-5p, miR-4745-5p, miR-4665-5p, miR-6807-5p, miR-7114-5p, miR-516a-5p, miR-769-3p, miR-3692-5p, miR-3945, miR-4433a-3p, miR-4485-3p, miR-6831-5p, miR-519c-5p, miR-551b-5p, miR-1343-3p, miR-4286, miR-4634, miR-4733-3p, and miR-6086, or to complementary strand(s) of the polynucleotide(s), and
wherein the kit or the device optionally further comprises nucleic acid(s) capable of specifically binding to one or more polynucleotide(s) selected from the group consisting of other dementia markers: miR-1225-3p, miR-3184-5p, miR-665, miR-1247-5p, miR-3656, miR-149-5p, miR-744-5p, miR-345-5p, miR-150-5p, miR-191-3p, miR-651-5p, miR-34a-5p, miR-409-5p, miR-369-5p, miR-1915-5p, miR-204-5p, miR-137, miR-382-5p, miR-517-5p, miR-532-5p, miR-22-5p, miR-1237-3p, miR-1224-3p, miR-625-3p, miR-328-3p, miR-122-5p, miR-202-3p, miR-4781-5p, miR-718, miR-342-3p, miR-26b-3p, miR-140-3p, miR-200a-3p, miR-378a-3p, miR-484, miR-296-5p, miR-205-5p, miR-431-5p, miR-150-3p, miR-423-5p, miR-575, miR-671-5p, miR-939-5p, miR-3665, miR-30d-5p, miR-30b-3p, miR-92a-3p, miR-371b-5p, miR-486-5p, miR-1471, miR-1538, miR-449b-3p, miR-1976, miR-4268, miR-4279, miR-3620-3p, miR-3944-3p, miR-3156-3p, miR-3187-5p, miR-4685-3p, miR-4695-3p, miR-4697-3p, miR-4713-5p, miR-4723-3p, miR-371b-3p, miR-3151-3p, miR-3192-3p, miR-6728-3p, miR-6736-3p, miR-6740-3p, miR-6741-3p, miR-6743-3p, miR-6747-3p, miR-6750-3p, miR-6754-3p, miR-6759-3p, miR-6761-3p, miR-6762-3p, miR-6769a-3p, miR-6776-3p, miR-6778-3p, miR-6779-3p, miR-6786-3p, miR-6787-3p, miR-6792-3p, miR-6794-3p, miR-6801-3p, miR-6802-3p, miR-6803-3p, miR-6804-3p, miR-6810-5p, miR-6823-3p, miR-6825-3p, miR-6829-3p, miR-6833-3p, miR-6834-3p, miR-6780b-3p, miR-6845-3p, miR-6862-3p, miR-6865-3p, miR-6870-3p, miR-6875-3p, miR-6877-3p, miR-6879-3p, miR-6882-3p, miR-6885-3p, miR-6886-3p, miR-6887-3p, miR-6890-3p, miR-6893-3p, miR-6894-3p, miR-7106-3p, miR-7109-3p, miR-7114-3p, miR-7155-5p, miR-7160-5p, miR-615-3p, miR-920, miR-1825, miR-675-3p, miR-1910-5p, miR-2278, miR-2682-3p, miR-3122, miR-3151-5p, miR-3175, miR-4323, miR-4326, miR-4284, miR-3605-3p, miR-3622b-5p, miR-3646, miR-3158-5p, miR-4722-3p, miR-4728-3p, miR-4747-3p, miR-4436b-5p, miR-5196-3p, miR-5589-5p, miR-345-3p, miR-642b-5p, miR-6716-3p, miR-6511b-3p, miR-208a-5p, miR-6726-3p, miR-6744-5p, miR-6782-3p, miR-6789-3p, miR-6797-3p, miR-6800-3p, miR-6806-5p, miR-6824-3p, miR-6837-5p, miR-6846-3p, miR-6858-3p, miR-6859-3p, miR-6861-3p, miR-6880-3p, miR-7111-3p, miR-7152-5p, miR-642a-5p, miR-657, miR-1236-3p, miR-764, miR-4314, miR-3675-3p, miR-5703, miR-3191-5p, miR-6511a-3p, miR-6809-3p, miR-6815-5p, miR-6857-3p, miR-6878-3p, miR-766-3p, miR-1229-3p, miR-1306-5p, miR-210-5p, miR-198, miR-485-3p, miR-668-3p, miR-532-3p, miR-877-3p, miR-1238-3p, miR-3130-5p, miR-4298, miR-4290, miR-3943, miR-346, and miR-767-3p or to complementary strand(s) of the polynucleotide(s).

5. The use of the kit according to clause 4 or the use of the device according to clause 4, wherein the nucleic acid(s) is(are) polynucleotide(s) selected from the group consisting of the following polynucleotides (a') to (e') and (f) to (t):

(a') a polynucleotide consisting of a nucleotide sequence represented by any of SEQ ID NOs: 1, 2, 4 to 22, 24, 25, 27 to 210, 374, 1315 to 1350, and 1435 to 1448 or a nucleotide sequence derived from the nucleotide sequence by the replacement of u with t, or a fragment thereof comprising 15 or more consecutive nucleotides,

(b') a polynucleotide comprising a nucleotide sequence represented by any of SEQ ID NOs: 1, 2, 4 to 22, 24, 25, 27 to 210, 374, 1315 to 1350, and 1435 to 1448, or a nucleotide sequence derived from the nucleotide sequence by the replacement of u with t,

(c') a polynucleotide consisting of a nucleotide sequence complementary to a nucleotide sequence represented by any of SEQ ID NOs: 1, 2, 4 to 22, 24, 25, 27 to 210, 374, 1315 to 1350, and 1435 to 1448 or a nucleotide sequence derived from the nucleotide sequence by the replacement of u with t, or a fragment thereof comprising 15 or more consecutive nucleotides,

(d') a polynucleotide comprising a nucleotide sequence complementary to a nucleotide sequence represented by any of SEQ ID NOs: 1, 2, 4 to 22, 24, 25, 27 to 210, 374, 1315 to 1350, and 1435 to 1448 or a nucleotide sequence derived from the nucleotide sequence by the replacement of u with t,

(e') a polynucleotide hybridizing under stringent conditions to any of the polynucleotides (a') to (d),

(f) a polynucleotide consisting of a nucleotide sequence represented by any of SEQ ID NOs: 211 to 213, 215 to 249, 1351 to 1356, and 1449 to 1453 or a nucleotide sequence derived from the nucleotide sequence by the replacement of u with t, or a fragment thereof comprising 15 or more consecutive nucleotides,

(g) a polynucleotide comprising a nucleotide sequence represented by any of SEQ ID NOs: 211 to 213, 215 to 249, 1351 to 1356, and 1449 to 1453, or a nucleotide sequence derived from the nucleotide sequence by the replacement of u with t,

(h) a polynucleotide consisting of a nucleotide sequence complementary to a nucleotide sequence represented by any of SEQ ID NOs: 211 to 213, 215 to 249, 1351 to 1356, and 1449 to 1453 or a nucleotide sequence derived from the nucleotide sequence by the replacement of u with t, or a fragment thereof comprising 15 or more consecutive nucleotides,

(i) a polynucleotide comprising a nucleotide sequence complementary to a nucleotide sequence represented by any of SEQ ID NOs: 211 to 213, 215 to 249, 1351 to 1356, and 1449 to 1453 or a nucleotide sequence derived from the nucleotide sequence by the replacement of u with t,

(j) a polynucleotide hybridizing under stringent conditions to any of the polynucleotides (f) to (i),

(k) a polynucleotide consisting of a nucleotide sequence represented by any of SEQ ID NOs: 250 to 373 or a nucleotide sequence derived from the nucleotide sequence by the replacement of u with t, or a fragment thereof comprising 15 or more consecutive nucleotides,

(l) a polynucleotide comprising a nucleotide sequence represented by any of SEQ ID NOs: 250 to 373, or a nucleotide sequence derived from the nucleotide sequence by the replacement of u with t,

(m) a polynucleotide consisting of a nucleotide sequence complementary to a nucleotide sequence represented by any of SEQ ID NOs: 250 to 373 or a nucleotide sequence derived from the nucleotide sequence by the replacement of u with t, or a fragment thereof comprising 15 or more consecutive nucleotides,

(n) a polynucleotide comprising a nucleotide sequence complementary to a nucleotide sequence represented by any of SEQ ID NOs: 250 to 373 or a nucleotide sequence derived from the nucleotide sequence by the replacement of u with t,

(o) a polynucleotide hybridizing under stringent conditions to any of the polynucleotides (k) to (n),

(p) a polynucleotide consisting of a nucleotide sequence represented by any of SEQ ID NOs: 375 to 390 or a nucleotide sequence derived from the nucleotide sequence by the replacement of u with t, or a fragment thereof comprising 15 or more consecutive nucleotides,

(q) a polynucleotide comprising a nucleotide sequence represented by any of SEQ ID NOs: 375 to 390, or a nucleotide sequence derived from the nucleotide sequence by the replacement of u with t,

(r) a polynucleotide consisting of a nucleotide sequence complementary to a nucleotide sequence represented by any of SEQ ID NOs: 375 to 390 or a nucleotide sequence derived from the nucleotide sequence by the replacement of u with t, or a fragment thereof comprising 15 or more consecutive nucleotides,

(s) a polynucleotide comprising a nucleotide sequence complementary to a nucleotide sequence represented by any of SEQ ID NOs: 375 to 390 or a nucleotide sequence derived from the nucleotide sequence by the replacement of u with t, and

(t) a polynucleotide hybridizing under stringent conditions to any of the polynucleotides (p) to (s).

6. The use of the device according to any one of clauses 2 to 5, wherein the device is a device for measurement by a hybridization technique.

7. The use of the device according to clause 6, wherein the hybridization technique is a nucleic acid array technique.

8. A method for detecting dementia, comprising measuring expression levels of polynucleotides of dementia markers miR-4486, miR-6088 and miR-211-5p in a sample from a subject; and evaluating *in vitro* whether or not the subject has dementia using the measured expression levels.

9. A method according to clause 8, further comprising measuring expression level(s) of one or more polynucleotide(s) selected from the group consisting of other dementia markers: miR-4274, miR-4272, miR-4443, miR-4506, miR-6773-5p, miR-4662a-5p,-miR-3184-3p, miR-4281, miR-320d, miR-6729-3p, miR-5192, miR-6853-5p, miR-1234-3p, miR-1233-3p, miR-4539, miR-3914, miR-4738-5p, miR-548au-3p, miR-1539, miR-4720-3p, miR-365b-5p, miR-1227-5p, miR-4667-5p, miR-6820-5p, miR-4505, miR-548q, miR-4658, miR-450a-5p, miR-1260b, miR-3677-5p, miR-6777-3p, miR-6826-3p, miR-6832-3p, miR-4725-3p, miR-7161-3p, miR-2277-5p, miR-7110-3p, miR-4312, miR-4461, miR-6766-5p, miR-1266-3p, miR-6729-5p, miR-526b-3p, miR-519e-5p, miR-512-5p, miR-5088-5p, miR-1909-3p, miR-6511a-5p, miR-4734, miR-936, miR-1249-3p, miR-6777-5p, miR-4487, miR-3155a, miR-563, miR-4741, miR-6788-5p, miR-4433b-5p, miR-323a-5p, miR-6811-5p, miR-6721-5p, miR-5004-5p, miR-6509-3p, miR-648, miR-3917, miR-6087, miR-1470, miR-586, miR-3150a-5p, miR-105-3p, miR-7973, miR-1914-5p, miR-4749-3p, miR-15b-5p, miR-1289, miR-4433a-5p, miR-3666, miR-3186-3p, miR-4725-5p, miR-4488, miR-4474-3p, miR-6731-3p, miR-4640-3p, miR-202-5p, miR-6816-5p, miR-638, miR-6821-5p, miR-1247-3p, miR-6765-5p, miR-6800-5p, miR-3928-3p, miR-3940-5p, miR-3960, miR-6775-5p, miR-3178, miR-1202, miR-6790-5p, miR-4731-3p, miR-2681-3p, miR-6758-5p, miR-8072, miR-518d-3p, miR-3606-3p, miR-4800-5p, miR-1292-3p, miR-6784-3p, miR-4450, miR-6132, miR-4716-5p, miR-6860, miR-1268b, miR-378d, miR-4701-5p, miR-4329, miR-185-3p, miR-552-3p, miR-1273g-5p, miR-6769b-3p, miR-520a-3p, miR-4524b-5p, miR-4291, miR-6734-3p, miR-143-5p, miR-939-3p, miR-6889-3p, miR-6842-3p, miR-4511, miR-4318, miR-4653-5p, miR-6867-3p, miR-133b, miR-3196, miR-193b-3p, miR-3162-3p, miR-6819-3p, miR-1908-3p, miR-6786-5p, miR-3648, miR-4513, miR-3652, miR-4640-5p, miR-6871-5p, miR-7845-5p, miR-3138, miR-6884-5p, miR-4653-3p, miR-636, miR-4652-3p, miR-6823-5p, miR-4502, miR-7113-5p, miR-8087, miR-7154-3p, miR-5189-5p, miR-1253, miR-518c-5p, miR-7151-5p, miR-3614-3p, miR-4727-5p, miR-3682-5p, miR-5090, miR-337-3p, miR-488-5p, miR-100-5p, miR-4520-3p, miR-373-3p, miR-6499-5p, miR-3909, miR-32-5p, miR-302a-3p, miR-4686, miR-4659a-3p, miR-4287, miR-1301-5p, miR-593-3p, miR-517a-3p, miR-517b-3p, miR-142-3p, miR-1185-2-3p, miR-602, miR-527, miR-518a-5p, miR-4682, miR-28-5p, miR-4252, miR-452-5p, miR-525-5p, miR-3622a-3p, miR-6813-3p, miR-4769-3p, miR-5698, miR-1915-3p, miR-1343-5p, miR-6861-5p, miR-6781-5p, miR-4508, miR-6743-5p, miR-6726-5p, miR-4525, miR-4651, miR-6813-5p, miR-5787, miR-1290, miR-6075, miR-4758-5p, miR-4690-5p, miR-762, miR-371a-5p, miR-6765-3p, miR-6784-5p, miR-6778-5p, miR-6875-5p, miR-4534, miR-4721, miR-6756-5p, miR-615-5p, miR-6727-5p, miR-6887-5p, miR-8063, miR-6880-5p, miR-6805-3p, miR-4726-5p, miR-4710, miR-7111-5p, miR-3619-3p, miR-6795-5p, miR-1254, miR-1233-5p, miR-6836-3p, miR-6769a-5p, miR-4532, miR-365a-5p, miR-1231, miR-1228-5p, miR-4430, miR-296-3p, miR-1237-5p, miR-4466, miR-6789-5p, miR-4632-5p, miR-4745-5p, miR-4665-5p, miR-6807-5p, miR-7114-5p, miR-516a-5p, miR-769-3p, miR-3692-5p, miR-3945, miR-4433a-3p, miR-4485-3p, miR-6831-5p, miR-519c-5p, miR-551b-5p, miR-1343-3p, miR-4286, miR-4634, miR-4733-3p, and miR-6086 in a sample from a subject; and evaluating *in vitro* whether or not the subject has dementia using the measured expression level(s).

10. The method according to clause 8 or 9, comprising plugging the gene expression levels of the three or more polynucleotides in the sample from the subject into a discriminant formula capable of discriminating dementia or non-dementia distinctively, wherein the discriminant formula is created by using gene expression levels in samples from subjects known to have dementia and gene expression levels in samples from non-dementia subjects as training samples; and thereby evaluating whether or not the subject has dementia.

11. The method according to any one of clauses 8 to 10, wherein the expression level(s) of the polynucleotide(s) is(are) measured by using nucleic acid(s) capable of specifically binding to the polynucleotide(s) or to complementary strand(s) of the polynucleotide(s), and the nucleic acid(s) is(are) polynucleotide(s) selected from the group consisting of the following polynucleotides (a) to (e):

(a) a polynucleotide consisting of a nucleotide sequence represented by SEQ ID NO: 23, 26 or 214, or a nucleotide sequence derived from the nucleotide sequence by the replacement of u with t, or a fragment thereof comprising 15 or more consecutive nucleotides,

(b) a polynucleotide comprising a nucleotide sequence represented by SEQ ID NO: 23, 26 or 214, or a nucleotide sequence derived from the nucleotide sequence by the replacement of u with t,

(c) a polynucleotide consisting of a nucleotide sequence complementary to a nucleotide sequence represented by SEQ ID NO: 23, 26 or 214, or a nucleotide sequence derived from the nucleotide sequence by the replacement of u with t, or a fragment thereof comprising 15 or more consecutive nucleotides,

(d) a polynucleotide comprising a nucleotide sequence complementary to a nucleotide sequence represented by SEQ ID NO: 23, 26 or 214, or a nucleotide sequence derived from the nucleotide sequence by the replacement of u with t, and

(e) a polynucleotide hybridizing under stringent conditions to any of the polynucleotides (a) to (d), and

wherein the expression level(s) of the polynucleotide(s) is(are) optionally measured by using nucleic acid(s) capable of specifically binding to the polynucleotide(s) or to complementary strand(s) of the polynucleotide(s), and the nucleic acid(s) is(are) polynucleotide(s) selected from the group consisting of the following polynucleotides (a') to (e'): (a') a polynucleotide consisting of a nucleotide sequence represented by any of SEQ ID NOs: 1, 2, 4 to 22, 24, 25, 27 to 210, 374, 1315 to 1350, and 1435 to 1448 or a nucleotide sequence derived from the nucleotide sequence by the replacement of u with t, or a fragment thereof comprising 15 or more consecutive nucleotides,

(b') a polynucleotide comprising a nucleotide sequence represented by any of SEQ ID NOs: 1, 2, 4 to 22, 24, 25, 27 to 210, 374, 1315 to 1350, and 1435 to 1448, or a nucleotide sequence derived from the nucleotide sequence by the replacement of u with t,

(c') a polynucleotide consisting of a nucleotide sequence complementary to a nucleotide sequence represented by any of SEQ ID NOs: 1, 2, 4 to 22, 24, 25, 27 to 210, 374, 1315 to 1350, and 1435 to 1448 or a nucleotide sequence derived from the nucleotide sequence by the replacement of u with t, or a fragment thereof comprising 15 or more consecutive nucleotides, (d') a polynucleotide comprising a nucleotide sequence complementary to a nucleotide sequence represented by any of SEQ ID NOs: 1, 2, 4 to 22, 24, 25, 27 to 210, 374, 1315 to 1350, and 1435 to 1448 or a nucleotide sequence derived from the nucleotide sequence by the replacement of u with t, and

(e') a polynucleotide hybridizing under stringent conditions to any of the polynucleotides (a') to (d').

12. The method according to any one of clauses 8 to 11, further comprising measuring expression level(s) of one or more polynucleotide(s) selected from the group consisting of other dementia markers: miR-1225-3p, miR-3184-5p, miR-665, miR-1247-5p, miR-3656, miR-149-5p, miR-744-5p, miR-345-5p, miR-150-5p, miR-191-3p, miR-651-5p, miR-34a-5p, miR-409-5p, miR-369-5p, miR-1915-5p, miR-204-5p, miR-137, miR-382-5p, miR-517-5p, miR-532-5p, miR-22-5p, miR-1237-3p, miR-1224-3p, miR-625-3p, miR-328-3p, miR-122-5p, miR-202-3p, miR-4781-5p, miR-718, miR-342-3p, miR-26b-3p, miR-140-3p, miR-200a-3p, miR-378a-3p, miR-484, miR-296-5p, miR-205-5p, miR-431-5p, miR-150-3p, miR-423-5p, miR-575, miR-671-5p, miR-939-5p, miR-3665, miR-30d-5p, miR-30b-3p, miR-92a-3p, miR-371b-5p, miR-486-5p, miR-1471, miR-1538, miR-449b-3p, miR-1976, miR-4268, miR-4279, miR-3620-3p, miR-3944-3p, miR-3156-3p, miR-3187-5p, miR-4685-3p, miR-4695-3p, miR-4697-3p, miR-4713-5p, miR-4723-3p, miR-371b-3p, miR-3151-3p, miR-3192-3p, miR-6728-3p, miR-6736-3p, miR-6740-3p, miR-6741-3p, miR-6743-3p, miR-6747-3p, miR-6750-3p, miR-6754-3p, miR-6759-3p, miR-6761-3p, miR-6762-3p, miR-6769a-3p, miR-6776-3p, miR-6778-3p, miR-6779-3p, miR-6786-3p, miR-6787-3p, miR-6792-3p, miR-6794-3p, miR-6801-3p, miR-6802-3p, miR-6803-3p, miR-6804-3p, miR-6810-5p, miR-6823-3p, miR-6825-3p, miR-6829-3p, miR-6833-3p, miR-6834-3p, miR-6780b-3p, miR-6845-3p, miR-6862-3p, miR-6865-3p, miR-6870-3p, miR-6875-3p, miR-6877-3p, miR-6879-3p, miR-6882-3p, miR-6885-3p, miR-6886-3p, miR-6887-3p, miR-6890-3p, miR-6893-3p, miR-6894-3p, miR-7106-3p, miR-7109-3p, miR-7114-3p, miR-7155-5p, miR-7160-5p, miR-615-3p, miR-920, miR-1825, miR-675-3p, miR-1910-5p, miR-2278, miR-2682-3p, miR-3122, miR-3151-5p, miR-3175, miR-4323, miR-4326, miR-4284, miR-3605-3p, miR-3622b-5p, miR-3646, miR-3158-5p, miR-4722-3p, miR-4728-3p, miR-4747-3p, miR-4436b-5p, miR-5196-3p, miR-5589-5p, miR-345-3p, miR-642b-5p, miR-6716-3p, miR-6511b-3p, miR-208a-5p, miR-6726-3p, miR-6744-5p, miR-6782-3p, miR-6789-3p, miR-6797-3p, miR-6800-3p, miR-6806-5p, miR-6824-3p, miR-6837-5p, miR-6846-3p, miR-6858-3p, miR-6859-3p, miR-6861-3p, miR-6880-3p, miR-7111-3p, miR-7152-5p, miR-642a-5p, miR-657, miR-1236-3p, miR-764, miR-4314, miR-3675-3p, miR-5703, miR-3191-5p, miR-6511a-3p, miR-6809-3p, miR-6815-5p, miR-6857-3p, miR-6878-3p, and miR-766-3p, miR-1229-3p, miR-1306-5p, miR-210-5p, miR-198, miR-485-3p, miR-668-3p, miR-532-3p, miR-877-3p, miR-1238-3p, miR-3130-5p, miR-4298, miR-4290, miR-3943, miR-346, and miR-767-3p.

13. The method according to clause 12, wherein the expression level(s) of the polynucleotide(s) is(are) measured by using nucleic acid(s) capable of specifically binding to the polynucleotide(s) or to complementary strand(s) of the polynucleotide(s), and

wherein the nucleic acid(s) is(are) polynucleotide(s) selected from the group consisting of the following polynucleotides (f) to (t):

(f) a polynucleotide consisting of a nucleotide sequence represented by any of SEQ ID NOs: 211 to 213, 215 to 249, 1351 to 1356, and 1449 to 1453 or a nucleotide sequence derived from the nucleotide sequence by the replacement of u with t, or a fragment thereof comprising 15 or more consecutive nucleotides,

(g) a polynucleotide comprising a nucleotide sequence represented by any of SEQ ID NOs: 211 to 213, 215 to 249, 1351 to 1356, and 1449 to 1453, or a nucleotide sequence derived from the nucleotide sequence by the replacement of u with t,

(h) a polynucleotide consisting of a nucleotide sequence complementary to a nucleotide sequence represented by any of SEQ ID NOs: 211 to 213, 215 to 249, 1351 to 1356, and 1449 to 1453 or a nucleotide sequence derived from the nucleotide sequence by the replacement of u with t, or a fragment thereof comprising 15 or more consecutive nucleotides,

(i) a polynucleotide comprising a nucleotide sequence complementary to a nucleotide sequence represented by any of SEQ ID NOs: 211 to 213, 215 to 249, 1351 to 1356, and 1449 to 1453 or a nucleotide sequence derived from the nucleotide sequence by the replacement of u with t,

(j) a polynucleotide hybridizing under stringent conditions to any of the polynucleotides (f) to (i),

(k) a polynucleotide consisting of a nucleotide sequence represented by any of SEQ ID NOs: 250 to 373 or a nucleotide sequence derived from the nucleotide sequence by the replacement of u with t, or a fragment thereof comprising 15 or more consecutive nucleotides,

(l) a polynucleotide comprising a nucleotide sequence represented by any of SEQ ID NOs: 250 to 373, or a nucleotide sequence derived from the nucleotide sequence by the replacement of u with t,

(m) a polynucleotide consisting of a nucleotide sequence complementary to a nucleotide sequence represented by any of SEQ ID NOs: 250 to 373 or a nucleotide sequence derived from the nucleotide sequence by the replacement of u with t, or a fragment thereof comprising 15 or more consecutive nucleotides,

(n) a polynucleotide comprising a nucleotide sequence complementary to a nucleotide sequence represented by any of SEQ ID NOs: 250 to 373 or a nucleotide sequence derived from the nucleotide sequence by the replacement of u with t,

(o) a polynucleotide hybridizing under stringent conditions to any of the polynucleotides (k) to (n),

(p) a polynucleotide consisting of a nucleotide sequence represented by any of SEQ ID NOs: 375 to 390 or a nucleotide sequence derived from the nucleotide sequence by the replacement of u with t, or a fragment thereof comprising 15 or more consecutive nucleotides,

(q) a polynucleotide comprising a nucleotide sequence represented by any of SEQ ID NOs: 375 to 390 or a nucleotide sequence derived from the nucleotide sequence by the replacement of u with t,

(r) a polynucleotide consisting of a nucleotide sequence complementary to a nucleotide sequence represented by any of SEQ ID NOs: 375 to 390 or a nucleotide sequence derived from the nucleotide sequence by the replacement of u with t, or a fragment thereof comprising 15 or more consecutive nucleotides,

(s) a polynucleotide comprising a nucleotide sequence complementary to a nucleotide sequence represented by any of SEQ ID NOs: 375 to 390 or a nucleotide sequence derived from the nucleotide sequence by the replacement of u with t, and

(t) a polynucleotide hybridizing under stringent conditions to any of the polynucleotides (p) to (s).

14. The method according to any one of clauses 8 to 13, wherein the expression level(s) of the target gene(s) in the sample from the subject is(are) measured by use of the kit according to any one of clauses 1 and 3 to 5 or the device according to any one of clauses 2 to 7.

15. The method according to any one of clauses 8 to 14, wherein the sample is blood, serum, or plasma.

16. Use of dementia markers miR-4486, miR-6088 and miR-211-5p in an in vitro method of detection or prediction of dementia.

17. The use according to clause 16, wherein miR-4486, miR-6088 and miR-211-5p are used together with at least one or more polynucleotide(s) selected from the group consisting of dementia markers: miR-4274, miR-4272, miR-4443, miR-4506, miR-6773-5p, miR-4662a-5p, miR-3184-3p, miR-4281, miR-320d, miR-6729-3p, miR-5192, miR-6853-5p, miR-1234-3p, miR-1233-3p, miR-4539, miR-3914, miR-4738-5p, miR-548au-3p, miR-1539, miR-4720-3p, miR-365b-5p, miR-1227-5p, miR-4667-5p, miR-6820-5p, miR-4505, miR-548q, miR-4658, miR-450a-5p, miR-1260b, miR-3677-5p, miR-6777-3p, miR-6826-3p, miR-6832-3p, miR-4725-3p, miR-7161-3p, miR-2277-5p, miR-7110-3p, miR-4312, miR-4461, miR-6766-5p, miR-1266-3p, miR-6729-5p, miR-526b-3p, miR-519e-5p, miR-512-5p, miR-5088-5p, miR-1909-3p, miR-6511a-5p, miR-4734, miR-936, miR-1249-3p, miR-6777-5p, miR-4487, miR-3155a, miR-563, miR-4741, miR-6788-5p, miR-4433b-5p, miR-323a-5p, miR-6811-5p, miR-6721-5p, miR-5004-5p, miR-6509-3p, miR-648, miR-3917, miR-6087, miR-1470, miR-586, miR-3150a-5p, miR-105-3p, miR-7973, miR-1914-5p, miR-4749-3p, miR-15b-5p, miR-1289, miR-4433a-5p, miR-3666, miR-3186-3p, miR-4725-5p, miR-4488, miR-4474-3p, miR-6731-3p, miR-4640-3p, miR-202-5p, miR-6816-5p, miR-638, miR-6821-5p, miR-1247-3p, miR-6765-5p, miR-6800-5p, miR-3928-3p, miR-3940-5p, miR-3960, miR-6775-5p, miR-3178, miR-1202, miR-6790-5p, miR-4731-3p, miR-2681-3p, miR-6758-5p, miR-8072, miR-518d-3p, miR-3606-3p, miR-4800-5p, miR-1292-3p, miR-6784-3p, miR-4450, miR-6132, miR-4716-5p, miR-6860, miR-1268b, miR-378d, miR-4701-5p, miR-4329, miR-185-3p, miR-552-3p, miR-1273g-5p, miR-6769b-3p, miR-520a-3p, miR-4524b-5p, miR-4291, miR-6734-3p, miR-143-5p, miR-939-3p, miR-6889-3p, miR-6842-3p, miR-4511, miR-4318, miR-4653-5p, miR-6867-3p, miR-133b, miR-3196, miR-193b-3p, miR-3162-3p, miR-6819-3p, miR-1908-3p, miR-6786-5p, miR-3648, miR-4513, miR-3652, miR-4640-5p, miR-6871-5p, miR-7845-5p, miR-3138, miR-6884-5p, miR-4653-3p, miR-636, miR-4652-3p, miR-6823-5p, miR-4502, miR-7113-5p, miR-8087, miR-7154-3p, miR-5189-5p, miR-1253, miR-518c-5p, miR-7151-5p, miR-3614-3p, miR-4727-5p, miR-3682-5p, miR-5090, miR-337-3p, miR-488-5p, miR-100-5p, miR-4520-3p, miR-373-3p, miR-6499-5p, miR-3909, miR-32-5p, miR-302a-3p, miR-4686, miR-4659a-3p, miR-4287, miR-1301-5p, miR-593-3p, miR-517a-3p, miR-517b-3p, miR-142-3p, miR-1185-2-3p, miR-602, miR-527, miR-518a-5p, miR-4682, miR-28-5p, miR-4252, miR-452-5p, miR-525-5p, miR-3622a-3p, miR-6813-3p, miR-4769-3p, miR-5698, miR-1915-3p, miR-1343-5p, miR-6861-5p, miR-6781-5p, miR-4508, miR-6743-5p, miR-6726-5p, miR-4525, miR-4651, miR-6813-5p, miR-5787, miR-1290, miR-6075, miR-4758-5p, miR-4690-5p, miR-762, miR-371a-5p, miR-6765-3p, miR-6784-5p, miR-6778-5p, miR-6875-5p, miR-4534, miR-4721, miR-6756-5p, miR-615-5p, miR-6727-5p, miR-6887-5p, miR-8063, miR-6880-5p, miR-6805-3p, miR-4726-5p, miR-4710, miR-7111-5p, miR-3619-3p, miR-6795-5p, miR-1254, miR-1233-5p, miR-6836-3p, miR-6769a-5p, miR-4532, miR-365a-5p, miR-1231, miR-1228-5p, miR-4430, miR-296-3p, miR-1237-5p, miR-4466, miR-6789-5p, miR-4632-5p, miR-4745-5p, miR-4665-5p, miR-6807-5p, miR-7114-5p, miR-516a-5p, miR-769-3p, miR-3692-5p, miR-3945, miR-4433a-3p, miR-4485-3p, miR-6831-5p, miR-519c-5p, miR-551b-5p, miR-1343-3p, miR-4286, miR-4634, miR-4733-3p, and miR-6086, and

optionally wherein miR-4486, miR-6088 and miR-211-5p are used together with at least one or more polynucleotide(s) selected from the group consisting of other dementia markers: miR-1225-3p, miR-3184-5p, miR-665, miR-1247-5p, miR-3656, miR-149-5p, miR-744-5p, miR-345-5p, miR-150-5p, miR-191-3p, miR-651-5p, miR-34a-5p, miR-409-5p, miR-369-5p, miR-1915-5p, miR-204-5p, miR-137, miR-382-5p, miR-517-5p, miR-532-5p, miR-22-5p, miR-1237-3p, miR-1224-3p, miR-625-3p, miR-328-3p, miR-122-5p, miR-202-3p, miR-4781-5p, miR-718, miR-342-3p, miR-26b-3p, miR-140-3p, miR-200a-3p, miR-378a-3p, miR-484, miR-296-5p, miR-205-5p, miR-431-5p, miR-150-3p, miR-423-5p, miR-575, miR-671-5p, miR-939-5p, miR-3665, miR-30d-5p, miR-30b-3p, miR-92a-3p, miR-371b-5p, miR-486-5p, miR-1471, miR-1538, miR-449b-3p, miR-1976, miR-4268, miR-4279, miR-3620-3p, miR-3944-3p, miR-3156-3p, miR-3187-5p, miR-4685-3p, miR-4695-3p, miR-4697-3p, miR-4713-5p, miR-4723-3p, miR-371b-3p, miR-3151-3p, miR-3192-3p, miR-6728-3p, miR-6736-3p, miR-6740-3p, miR-6741-3p, miR-6743-3p, miR-6747-3p, miR-6750-3p, miR-6754-3p, miR-6759-3p, miR-6761-3p, miR-6762-3p, miR-6769a-3p, miR-6776-3p, miR-6778-3p, miR-6779-3p, miR-6786-3p, miR-6787-3p, miR-6792-3p, miR-6794-3p, miR-6801-3p, miR-6802-3p, miR-6803-3p, miR-6804-3p, miR-6810-5p, miR-6823-3p, miR-6825-3p, miR-6829-3p, miR-6833-3p, miR-6834-3p, miR-6780b-3p, miR-6845-3p, miR-6862-3p, miR-6865-3p, miR-6870-3p, miR-6875-3p, miR-6877-3p, miR-6879-3p, miR-6882-3p, miR-6885-3p, miR-6886-3p, miR-6887-3p, miR-6890-3p, miR-6893-3p, miR-6894-3p, miR-7106-3p, miR-7109-3p, miR-7114-3p, miR-7155-5p, miR-7160-5p, miR-615-3p, miR-920, miR-1825, miR-675-3p, miR-1910-5p, miR-2278, miR-2682-3p, miR-3122, miR-3151-5p, miR-3175, miR-4323, miR-4326, miR-4284, miR-3605-3p, miR-3622b-5p, miR-3646, miR-3158-5p, miR-4722-3p, miR-4728-3p, miR-4747-3p, miR-4436b-5p, miR-5196-3p, miR-5589-5p, miR-345-3p, miR-642b-5p, miR-6716-3p, miR-6511b-3p, miR-208a-5p, miR-6726-3p, miR-6744-5p, miR-6782-3p, miR-6789-3p, miR-6797-3p, miR-6800-3p, miR-6806-5p, miR-6824-3p, miR-6837-5p, miR-6846-3p, miR-6858-3p, miR-6859-3p, miR-6861-3p, miR-6880-3p, miR-7111-3p, miR-7152-5p, miR-642a-5p, miR-657, miR-1236-3p, miR-764, miR-4314, miR-3675-3p, miR-5703, miR-3191-5p, miR-6511a-3p, miR-6809-3p, miR-6815-5p, miR-6857-3p, miR-6878-3p, miR-766-3p, miR-1229-3p, miR-1306-5p, miR-210-5p, miR-198, miR-485-3p, miR-668-3p, miR-532-3p, miR-877-3p, miR-1238-3p, miR-3130-5p, miR-4298, miR-4290, miR-3943, miR-346, and miR-767-3p.

## Claims

1. Use of a kit for detection of dementia, comprising nucleic acids capable of specifically binding to polynucleotides dementia markers miR-4486, miR-6088 and miR-211-5p, or to complementary strands of the polynucleotides.

2. Use of a device for detection of dementia, comprising nucleic acids capable of specifically binding to polynucleotide dementia markers miR-4486, miR-6088 and miR-211-5p, or to complementary strands of the polynucleotides.

3. The use of the kit according to claim 1 or the use of the device according to claim 2, wherein the nucleic acids are polynucleotides selected from the group consisting of the following polynucleotides (a) to (e):

    (a) a polynucleotide consisting of a nucleotide sequence represented by SEQ ID NO: 23, 26 or 214, or a nucleotide sequence derived from the nucleotide sequence by the replacement of u with t, or a fragment thereof comprising 15 or more consecutive nucleotides,
    (b) a polynucleotide comprising a nucleotide sequence represented by SEQ ID NO: 23, 26 or 214, or a nucleotide sequence derived from the nucleotide sequence by the replacement of u with t,
    (c) a polynucleotide consisting of a nucleotide sequence complementary to a nucleotide sequence represented by SEQ ID NO: 23, 26 or 214, or a nucleotide sequence derived from the nucleotide sequence by the replacement of u with t, or a fragment thereof comprising 15 or more consecutive nucleotides,
    (d) a polynucleotide comprising a nucleotide sequence complementary to a nucleotide sequence represented by SEQ ID NO: 23, 26 or 214, or a nucleotide sequence derived from the nucleotide sequence by the replacement of u with t, and
    (e) a polynucleotide hybridizing under stringent conditions to any of the polynucleotides (a) to (d).

4. The use of the kit according to claim 1 or 3 or the use of the device according to claim 2 or 3, wherein the kit or the device further comprises nucleic acid(s) capable of specifically binding to one or more polynucleotide(s) selected from the group consisting of other dementia markers: miR-4274, miR-4272, miR-4443, miR-4506, miR-6773-5p, miR-4662a-5p, miR-3184-3p, miR-4281, miR-320d, miR-6729-3p, miR-5192, miR-6853-5p, miR-1234-3p, miR-1233-3p, miR-4539, miR-3914, miR-4738-5p, miR-548au-3p, miR-1539, miR-4720-3p, miR-365b-5p, miR-1227-5p, miR-4667-5p, miR-6820-5p, miR-4505, miR-548q, miR-4658, miR-450a-5p, miR-1260b, miR-3677-5p, miR-6777-3p, miR-6826-3p, miR-6832-3p, miR-4725-3p, miR-7161-3p, miR-2277-5p, miR-7110-3p, miR-4312, miR-4461, miR-6766-5p, miR-1266-3p, miR-6729-5p, miR-526b-3p, miR-519e-5p, miR-512-5p, miR-5088-5p, miR-1909-3p, miR-6511a-5p, miR-4734, miR-936, miR-1249-3p, miR-6777-5p, miR-4487, miR-3155a, miR-563, miR-4741, miR-6788-5p, miR-4433b-5p, miR-323a-5p, miR-6811-5p, miR-6721-5p, miR-5004-5p, miR-6509-3p, miR-648, miR-3917, miR-6087, miR-1470, miR-586, miR-3150a-5p, miR-105-3p, miR-7973, miR-1914-5p, miR-4749-3p, miR-15b-5p, miR-1289, miR-4433a-5p, miR-3666, miR-3186-3p, miR-4725-5p, miR-4488, miR-4474-3p, miR-6731-3p, miR-4640-3p, miR-202-5p, miR-6816-5p, miR-638, miR-6821-5p, miR-1247-3p, miR-6765-5p, miR-6800-5p, miR-3928-3p, miR-3940-5p, miR-3960, miR-6775-5p, miR-3178, miR-1202, miR-6790-5p, miR-4731-3p, miR-2681-3p, miR-6758-5p, miR-8072, miR-518d-3p, miR-3606-3p, miR-4800-5p, miR-1292-3p, miR-6784-3p, miR-4450, miR-6132, miR-4716-5p, miR-6860, miR-1268b, miR-378d, miR-4701-5p, miR-4329, miR-185-3p, miR-552-3p, miR-1273g-5p, miR-6769b-3p, miR-520a-3p, miR-4524b-5p, miR-4291, miR-6734-3p, miR-143-5p, miR-939-3p, miR-6889-3p, miR-6842-3p, miR-4511, miR-4318, miR-4653-5p, miR-6867-3p, miR-133b, miR-3196, miR-193b-3p, miR-3162-3p, miR-6819-3p, miR-1908-3p, miR-6786-5p, miR-3648, miR-4513, miR-3652, miR-4640-5p, miR-6871-5p, miR-7845-5p, miR-3138, miR-6884-5p, miR-4653-3p, miR-636, miR-4652-3p, miR-6823-5p, miR-4502, miR-7113-5p, miR-8087, miR-7154-3p, miR-5189-5p, miR-1253, miR-518c-5p, miR-7151-5p, miR-3614-3p, miR-4727-5p, miR-3682-5p, miR-5090, miR-337-3p, miR-488-5p, miR-100-5p, miR-4520-3p, miR-373-3p, miR-6499-5p, miR-3909, miR-32-5p, miR-302a-3p, miR-4686, miR-4659a-3p, miR-4287, miR-1301-5p, miR-593-3p, miR-517a-3p, miR-517b-3p, miR-142-3p, miR-1185-2-3p, miR-602, miR-527, miR-518a-5p, miR-4682, miR-28-5p, miR-4252, miR-452-5p, miR-525-5p, miR-3622a-3p, miR-6813-3p, miR-4769-3p, miR-5698, miR-1915-3p, miR-1343-5p, miR-6861-5p, miR-6781-5p, miR-4508, miR-6743-5p, miR-6726-5p, miR-4525, miR-4651, miR-6813-5p, miR-5787, miR-1290, miR-6075, miR-4758-5p, miR-4690-5p, miR-762, miR-371a-5p, miR-6765-3p, miR-6784-5p, miR-6778-5p, miR-6875-5p, miR-4534, miR-4721, miR-6756-5p, miR-615-5p, miR-6727-5p, miR-6887-5p, miR-8063, miR-6880-5p, miR-6805-3p, miR-4726-5p, miR-4710, miR-7111-5p, miR-3619-3p, miR-6795-5p, miR-1254, miR-1233-5p, miR-6836-3p, miR-6769a-5p, miR-4532, miR-365a-5p, miR-1231, miR-1228-5p, miR-4430, miR-296-3p, miR-1237-5p, miR-4466, miR-6789-5p, miR-4632-5p, miR-4745-5p, miR-4665-5p, miR-6807-5p, miR-7114-5p, miR-516a-5p, miR-769-3p, miR-3692-5p, miR-3945, miR-4433a-3p, miR-4485-3p, miR-6831-5p, miR-519c-5p, miR-551b-5p, miR-1343-3p, miR-4286, miR-4634, miR-4733-3p, and miR-6086, or to complementary strand(s) of the polynucleotide(s), and

wherein the kit or the device optionally further comprises nucleic acid(s) capable of specifically binding to one or more polynucleotide(s) selected from the group consisting of other dementia markers: miR-1225-3p, miR-3184-5p, miR-665, miR-1247-5p, miR-3656, miR-149-5p, miR-744-5p, miR-345-5p, miR-150-5p, miR-191-3p, miR-651-5p, miR-34a-5p, miR-409-5p, miR-369-5p, miR-1915-5p, miR-204-5p, miR-137, miR-382-5p, miR-517-5p, miR-532-5p, miR-22-5p, miR-1237-3p, miR-1224-3p, miR-625-3p, miR-328-3p, miR-122-5p, miR-202-3p, miR-4781-5p, miR-718, miR-342-3p, miR-26b-3p, miR-140-3p, miR-200a-3p, miR-378a-3p, miR-484, miR-296-5p, miR-205-5p, miR-431-5p, miR-150-3p, miR-423-5p, miR-575, miR-671-5p, miR-939-5p, miR-3665, miR-30d-5p, miR-30b-3p, miR-92a-3p, miR-371b-5p, miR-486-5p, miR-1471, miR-1538, miR-449b-3p, miR-1976, miR-4268, miR-4279, miR-3620-3p, miR-3944-3p, miR-3156-3p, miR-3187-5p, miR-4685-3p, miR-4695-3p, miR-4697-3p, miR-4713-5p, miR-4723-3p, miR-371b-3p, miR-3151-3p, miR-3192-3p, miR-6728-3p, miR-6736-3p, miR-6740-3p, miR-6741-3p, miR-6743-3p, miR-6747-3p, miR-6750-3p, miR-6754-3p, miR-6759-3p, miR-6761-3p, miR-6762-3p, miR-6769a-3p, miR-6776-3p, miR-6778-3p, miR-6779-3p, miR-6786-3p, miR-6787-3p, miR-6792-3p, miR-6794-3p, miR-6801-3p, miR-6802-3p, miR-6803-3p, miR-6804-3p, miR-6810-5p, miR-6823-3p, miR-6825-3p, miR-6829-3p, miR-6833-3p, miR-6834-3p, miR-6780b-3p, miR-6845-3p, miR-6862-3p, miR-6865-3p, miR-6870-3p, miR-6875-3p, miR-6877-3p, miR-6879-3p, miR-6882-3p, miR-6885-3p, miR-6886-3p, miR-6887-3p, miR-6890-3p, miR-6893-3p, miR-6894-3p, miR-7106-3p, miR-7109-3p, miR-7114-3p, miR-7155-5p, miR-7160-5p, miR-615-3p, miR-920, miR-1825, miR-675-3p, miR-1910-5p, miR-2278, miR-2682-3p, miR-3122, miR-3151-5p, miR-3175, miR-4323, miR-4326, miR-4284, miR-3605-3p, miR-3622b-5p, miR-3646, miR-3158-5p, miR-4722-3p, miR-4728-3p, miR-4747-3p, miR-4436b-5p, miR-5196-3p, miR-5589-5p, miR-345-3p, miR-642b-5p, miR-6716-3p, miR-6511b-3p, miR-208a-5p, miR-6726-3p, miR-6744-5p, miR-6782-3p, miR-6789-3p, miR-6797-3p, miR-6800-3p, miR-6806-5p, miR-6824-3p, miR-6837-5p, miR-6846-3p, miR-6858-3p, miR-6859-3p, miR-6861-3p, miR-6880-3p, miR-7111-3p, miR-7152-5p, miR-642a-5p, miR-657, miR-1236-3p, miR-764, miR-4314, miR-3675-3p, miR-5703, miR-3191-5p, miR-6511a-3p, miR-6809-3p, miR-6815-5p, miR-6857-3p, miR-6878-3p, miR-766-3p, miR-1229-3p, miR-1306-5p, miR-210-5p, miR-198, miR-485-3p, miR-668-3p, miR-532-3p, miR-877-3p, miR-1238-3p, miR-3130-5p, miR-4298, miR-4290, miR-3943, miR-346, and miR-767-3p or to complementary strand(s) of the polynucleotide(s).

5. The use of the kit according to claim 4 or the use of the device according to claim 4, wherein the nucleic acid(s) is(are) polynucleotide(s) selected from the group consisting of the following polynucleotides (a') to (e') and (f) to (t):

(a') a polynucleotide consisting of a nucleotide sequence represented by any of SEQ ID NOs: 1, 2, 4 to 22, 24, 25, 27 to 210, 374, 1315 to 1350, and 1435 to 1448 or a nucleotide sequence derived from the nucleotide sequence by the replacement of u with t, or a fragment thereof comprising 15 or more consecutive nucleotides,
(b') a polynucleotide comprising a nucleotide sequence represented by any of SEQ ID NOs: 1, 2, 4 to 22, 24, 25, 27 to 210, 374, 1315 to 1350, and 1435 to 1448, or a nucleotide sequence derived from the nucleotide sequence by the replacement of u with t,
(c') a polynucleotide consisting of a nucleotide sequence complementary to a nucleotide sequence represented by any of SEQ ID NOs: 1, 2, 4 to 22, 24, 25, 27 to 210, 374, 1315 to 1350, and 1435 to 1448 or a nucleotide sequence derived from the nucleotide sequence by the replacement of u with t, or a fragment thereof comprising 15 or more consecutive nucleotides,
(d') a polynucleotide comprising a nucleotide sequence complementary to a nucleotide sequence represented by any of SEQ ID NOs: 1, 2, 4 to 22, 24, 25, 27 to 210, 374, 1315 to 1350, and 1435 to 1448 or a nucleotide sequence derived from the nucleotide sequence by the replacement of u with t,
(e') a polynucleotide hybridizing under stringent conditions to any of the polynucleotides (a') to (d'),
(f) a polynucleotide consisting of a nucleotide sequence represented by any of SEQ ID NOs: 211 to 213, 215 to 249, 1351 to 1356, and 1449 to 1453 or a nucleotide sequence derived from the nucleotide sequence by the replacement of u with t, or a fragment thereof comprising 15 or more consecutive nucleotides,
(g) a polynucleotide comprising a nucleotide sequence represented by any of SEQ ID NOs: 211 to 213, 215 to 249, 1351 to 1356, and 1449 to 1453, or a nucleotide sequence derived from the nucleotide sequence by the replacement of u with t,
(h) a polynucleotide consisting of a nucleotide sequence complementary to a nucleotide sequence represented by any of SEQ ID NOs: 211 to 213, 215 to 249, 1351 to 1356, and 1449 to 1453 or a nucleotide sequence derived from the nucleotide sequence by the replacement of u with t, or a fragment thereof comprising 15 or more consecutive nucleotides,
(i) a polynucleotide comprising a nucleotide sequence complementary to a nucleotide sequence represented by any of SEQ ID NOs: 211 to 213, 215 to 249, 1351 to 1356, and 1449 to 1453 or a nucleotide sequence derived from the nucleotide sequence by the replacement of u with t,
(j) a polynucleotide hybridizing under stringent conditions to any of the polynucleotides (f) to (i),

(k) a polynucleotide consisting of a nucleotide sequence represented by any of SEQ ID NOs: 250 to 373 or a nucleotide sequence derived from the nucleotide sequence by the replacement of u with t, or a fragment thereof comprising 15 or more consecutive nucleotides,

(l) a polynucleotide comprising a nucleotide sequence represented by any of SEQ ID NOs: 250 to 373, or a nucleotide sequence derived from the nucleotide sequence by the replacement of u with t,

(m) a polynucleotide consisting of a nucleotide sequence complementary to a nucleotide sequence represented by any of SEQ ID NOs: 250 to 373 or a nucleotide sequence derived from the nucleotide sequence by the replacement of u with t, or a fragment thereof comprising 15 or more consecutive nucleotides,

(n) a polynucleotide comprising a nucleotide sequence complementary to a nucleotide sequence represented by any of SEQ ID NOs: 250 to 373 or a nucleotide sequence derived from the nucleotide sequence by the replacement of u with t,

(o) a polynucleotide hybridizing under stringent conditions to any of the polynucleotides (k) to (n),

(p) a polynucleotide consisting of a nucleotide sequence represented by any of SEQ ID NOs: 375 to 390 or a nucleotide sequence derived from the nucleotide sequence by the replacement of u with t, or a fragment thereof comprising 15 or more consecutive nucleotides,

(q) a polynucleotide comprising a nucleotide sequence represented by any of SEQ ID NOs: 375 to 390, or a nucleotide sequence derived from the nucleotide sequence by the replacement of u with t,

(r) a polynucleotide consisting of a nucleotide sequence complementary to a nucleotide sequence represented by any of SEQ ID NOs: 375 to 390 or a nucleotide sequence derived from the nucleotide sequence by the replacement of u with t, or a fragment thereof comprising 15 or more consecutive nucleotides,

(s) a polynucleotide comprising a nucleotide sequence complementary to a nucleotide sequence represented by any of SEQ ID NOs: 375 to 390 or a nucleotide sequence derived from the nucleotide sequence by the replacement of u with t, and

(t) a polynucleotide hybridizing under stringent conditions to any of the polynucleotides (p) to (s).

6. The use of the device according to any one of claims 2 to 5, wherein the device is a device for measurement by a hybridization technique.

7. The use of the device according to claim 6, wherein the hybridization technique is a nucleic acid array technique.

8. A method for detecting dementia, comprising measuring expression levels of polynucleotides of dementia markers miR-4486, miR-6088 and miR-211-5p in a sample from a subject; and evaluating *in vitro* whether or not the subject has dementia using the measured expression levels.

9. A method according to claim 8, further comprising measuring expression level(s) of one or more polynucleotide(s) selected from the group consisting of other dementia markers: miR-4274, miR-4272, miR-4443, miR-4506, miR-6773-5p, miR-4662a-5p, miR-3184-3p, miR-4281, miR-320d, miR-6729-3p, miR-5192, miR-6853-5p, miR-1234-3p, miR-1233-3p, miR-4539, miR-3914, miR-4738-5p, miR-548au-3p, miR-1539, miR-4720-3p, miR-365b-5p, miR-1227-5p, miR-4667-5p, miR-6820-5p, miR-4505, miR-548q, miR-4658, miR-450a-5p, miR-1260b, miR-3677-5p, miR-6777-3p, miR-6826-3p, miR-6832-3p, miR-4725-3p, miR-7161-3p, miR-2277-5p, miR-7110-3p, miR-4312, miR-4461, miR-6766-5p, miR-1266-3p, miR-6729-5p, miR-526b-3p, miR-519e-5p, miR-512-5p, miR-5088-5p, miR-1909-3p, miR-6511a-5p, miR-4734, miR-936, miR-1249-3p, miR-6777-5p, miR-4487, miR-3155a, miR-563, miR-4741, miR-6788-5p, miR-4433b-5p, miR-323a-5p, miR-6811-5p, miR-6721-5p, miR-5004-5p, miR-6509-3p, miR-648, miR-3917, miR-6087, miR-1470, miR-586, miR-3150a-5p, miR-105-3p, miR-7973, miR-1914-5p, miR-4749-3p, miR-15b-5p, miR-1289, miR-4433a-5p, miR-3666, miR-3186-3p, miR-4725-5p, miR-4488, miR-4474-3p, miR-6731-3p, miR-4640-3p, miR-202-5p, miR-6816-5p, miR-638, miR-6821-5p, miR-1247-3p, miR-6765-5p, miR-6800-5p, miR-3928-3p, miR-3940-5p, miR-3960, miR-6775-5p, miR-3178, miR-1202, miR-6790-5p, miR-4731-3p, miR-2681-3p, miR-6758-5p, miR-8072, miR-518d-3p, miR-3606-3p, miR-4800-5p, miR-1292-3p, miR-6784-3p, miR-4450, miR-6132, miR-4716-5p, miR-6860, miR-1268b, miR-378d, miR-4701-5p, miR-4329, miR-185-3p, miR-552-3p, miR-1273g-5p, miR-6769b-3p, miR-520a-3p, miR-4524b-5p, miR-4291, miR-6734-3p, miR-143-5p, miR-939-3p, miR-6889-3p, miR-6842-3p, miR-4511, miR-4318, miR-4653-5p, miR-6867-3p, miR-133b, miR-3196, miR-193b-3p, miR-3162-3p, miR-6819-3p, miR-1908-3p, miR-6786-5p, miR-3648, miR-4513, miR-3652, miR-4640-5p, miR-6871-5p, miR-7845-5p, miR-3138, miR-6884-5p, miR-4653-3p, miR-636, miR-4652-3p, miR-6823-5p, miR-4502, miR-7113-5p, miR-8087, miR-7154-3p, miR-5189-5p, miR-1253, miR-518c-5p, miR-7151-5p, miR-3614-3p, miR-4727-5p, miR-3682-5p, miR-5090, miR-337-3p, miR-488-5p, miR-100-5p, miR-4520-3p, miR-373-3p, miR-6499-5p, miR-3909, miR-32-5p, miR-302a-3p, miR-4686, miR-4659a-3p, miR-4287, miR-1301-5p, miR-593-3p, miR-517a-3p, miR-517b-3p, miR-142-3p, miR-1185-2-3p, miR-602, miR-527, miR-518a-5p, miR-4682, miR-28-5p, miR-4252, miR-452-5p, miR-525-5p, miR-3622a-3p, miR-6813-3p, miR-4769-3p, miR-5698, miR-1915-3p, miR-1343-5p, miR-6861-5p, miR-6781-5p, miR-4508, miR-6743-5p, miR-6726-5p, miR-4525, miR-4651, miR-6813-5p, miR-5787, miR-1290, miR-6075, miR-4758-5p, miR-4690-5p, miR-762, miR-371a-5p, miR-6765-3p, miR-6784-5p, miR-6778-5p, miR-6875-5p, miR-4534, miR-4721, miR-6756-5p, miR-615-5p, miR-6727-5p, miR-6887-5p, miR-8063, miR-6880-5p, miR-6805-3p, miR-4726-5p, miR-4710, miR-7111-5p, miR-3619-3p, miR-6795-5p, miR-1254, miR-1233-5p, miR-6836-3p, miR-6769a-5p, miR-4532, miR-365a-5p, miR-1231, miR-1228-5p, miR-4430, miR-296-3p, miR-1237-5p, miR-4466, miR-6789-5p, miR-4632-5p, miR-4745-5p, miR-4665-5p, miR-6807-5p, miR-7114-5p, miR-516a-5p, miR-769-3p, miR-3692-5p, miR-3945, miR-4433a-3p, miR-4485-3p, miR-6831-5p, miR-519c-5p, miR-551b-5p, miR-1343-3p, miR-4286, miR-4634, miR-4733-3p, and miR-6086 in a sample from a subject; and evaluating *in vitro* whether or not the subject has dementia using the measured expression level(s).

10. The method according to claim 8 or 9, comprising plugging the gene expression levels of the three or more polynucleotides in the sample from the subject into a discriminant formula capable of discriminating dementia or non-dementia distinctively, wherein the discriminant formula is created by using gene expression levels in samples from subjects known to have dementia and gene expression levels in samples from non-dementia subjects as training samples; and thereby evaluating whether or not the subject has dementia.

11. The method according to any one of claims 8 to 10, wherein the expression level(s) of the polynucleotide(s) is(are) measured by using nucleic acid(s) capable of specifically binding to the polynucleotide(s) or to complementary strand(s) of the polynucleotide(s), and the nucleic acid(s) is(are) polynucleotide(s) selected from the group consisting of the following polynucleotides (a) to (e):

(a) a polynucleotide consisting of a nucleotide sequence represented by SEQ ID NO: 23, 26 or 214, or a nucleotide sequence derived from the nucleotide sequence by the replacement of u with t, or a fragment thereof comprising 15 or more consecutive nucleotides,
(b) a polynucleotide comprising a nucleotide sequence represented by SEQ ID NO: 23, 26 or 214, or a nucleotide sequence derived from the nucleotide sequence by the replacement of u with t,
(c) a polynucleotide consisting of a nucleotide sequence complementary to a nucleotide sequence represented by SEQ ID NO: 23, 26 or 214, or a nucleotide sequence derived from the nucleotide sequence by the replacement of u with t, or a fragment thereof comprising 15 or more consecutive nucleotides,
(d) a polynucleotide comprising a nucleotide sequence complementary to a nucleotide sequence represented by SEQ ID NO: 23, 26 or 214, or a nucleotide sequence derived from the nucleotide sequence by the replacement of u with t, and
(e) a polynucleotide hybridizing under stringent conditions to any of the polynucleotides (a) to (d), and

wherein the expression level(s) of the polynucleotide(s) is(are) optionally measured by using nucleic acid(s) capable of specifically binding to the polynucleotide(s) or to complementary strand(s) of the polynucleotide(s), and the nucleic acid(s) is(are) polynucleotide(s) selected from the group consisting of the following polynucleotides (a') to (e'):

(a') a polynucleotide consisting of a nucleotide sequence represented by any of SEQ ID NOs: 1, 2, 4 to 22, 24, 25, 27 to 210, 374, 1315 to 1350, and 1435 to 1448 or a nucleotide sequence derived from the nucleotide sequence by the replacement of u with t, or a fragment thereof comprising 15 or more consecutive nucleotides,
(b') a polynucleotide comprising a nucleotide sequence represented by any of SEQ ID NOs: 1, 2, 4 to 22, 24, 25, 27 to 210, 374, 1315 to 1350, and 1435 to 1448, or a nucleotide sequence derived from the nucleotide sequence by the replacement of u with t,
(c') a polynucleotide consisting of a nucleotide sequence complementary to a nucleotide sequence represented by any of SEQ ID NOs: 1, 2, 4 to 22, 24, 25, 27 to 210, 374, 1315 to 1350, and 1435 to 1448 or a nucleotide sequence derived from the nucleotide sequence by the replacement of u with t, or a fragment thereof comprising 15 or more consecutive nucleotides,
(d') a polynucleotide comprising a nucleotide sequence complementary to a nucleotide sequence represented by any of SEQ ID NOs: 1, 2, 4 to 22, 24, 25, 27 to 210, 374, 1315 to 1350, and 1435 to 1448 or a nucleotide sequence derived from the nucleotide sequence by the replacement of u with t, and
(e') a polynucleotide hybridizing under stringent conditions to any of the polynucleotides (a') to (d').

12. The method according to any one of claims 8 to 11, further comprising measuring expression level(s) of one or more polynucleotide(s) selected from the group consisting of other dementia markers: miR-1225-3p, miR-3184-5p, miR-665, miR-1247-5p, miR-3656, miR-149-5p, miR-744-5p, miR-345-5p, miR-150-5p, miR-191-3p, miR-651-5p, miR-34a-5p, miR-409-5p, miR-369-5p, miR-1915-5p, miR-204-5p, miR-137, miR-382-5p, miR-517-5p, miR-532-5p, miR-22-5p, miR-1237-3p, miR-1224-3p, miR-625-3p, miR-328-3p, miR-122-5p, miR-202-3p, miR-4781-5p, miR-718, miR-342-3p, miR-26b-3p, miR-140-3p, miR-200a-3p, miR-378a-3p, miR-484, miR-296-5p, miR-205-5p, miR-431-5p, miR-150-3p, miR-423-5p, miR-575, miR-671-5p, miR-939-5p, miR-3665, miR-30d-5p, miR-30b-3p, miR-92a-3p, miR-371b-5p, miR-486-5p, miR-1471, miR-1538, miR-449b-3p, miR-1976, miR-4268, miR-4279, miR-3620-3p, miR-3944-3p, miR-3156-3p, miR-3187-5p, miR-4685-3p, miR-4695-3p, miR-4697-3p, miR-4713-5p, miR-4723-3p, miR-371b-3p, miR-3151-3p, miR-3192-3p, miR-6728-3p, miR-6736-3p, miR-6740-3p, miR-6741-3p, miR-6743-3p, miR-6747-3p, miR-6750-3p, miR-6754-3p, miR-6759-3p, miR-6761-3p, miR-6762-3p, miR-6769a-3p, miR-6776-3p, miR-6778-3p, miR-6779-3p, miR-6786-3p, miR-6787-3p, miR-6792-3p, miR-6794-3p, miR-6801-3p, miR-6802-3p, miR-6803-3p, miR-6804-3p, miR-6810-5p, miR-6823-3p, miR-6825-3p, miR-6829-3p, miR-6833-3p, miR-6834-3p, miR-6780b-3p, miR-6845-3p, miR-6862-3p, miR-6865-3p, miR-6870-3p, miR-6875-3p, miR-6877-3p, miR-6879-3p, miR-6882-3p, miR-6885-3p, miR-6886-3p, miR-6887-3p, miR-6890-3p, miR-6893-3p, miR-6894-3p, miR-7106-3p, miR-7109-3p, miR-7114-3p, miR-7155-5p, miR-7160-5p, miR-615-3p, miR-920, miR-1825, miR-675-3p, miR-1910-5p, miR-2278, miR-2682-3p, miR-3122, miR-3151-5p, miR-3175, miR-4323, miR-4326, miR-4284, miR-3605-3p, miR-3622b-5p, miR-3646, miR-3158-5p, miR-4722-3p, miR-4728-3p, miR-4747-3p, miR-4436b-5p, miR-5196-3p, miR-5589-5p, miR-345-3p, miR-642b-5p, miR-6716-3p, miR-6511b-3p, miR-208a-5p, miR-6726-3p, miR-6744-5p, miR-6782-3p, miR-6789-3p, miR-6797-3p, miR-6800-3p, miR-6806-5p, miR-6824-3p, miR-6837-5p, miR-6846-3p, miR-6858-3p, miR-6859-3p, miR-6861-3p, miR-6880-3p, miR-7111-3p, miR-7152-5p, miR-642a-5p, miR-657, miR-1236-3p, miR-764, miR-4314, miR-3675-3p, miR-5703, miR-3191-5p, miR-6511a-3p, miR-6809-3p, miR-6815-5p, miR-6857-3p, miR-6878-3p, and miR-766-3p, miR-1229-3p, miR-1306-5p, miR-210-5p, miR-198, miR-485-3p, miR-668-3p, miR-532-3p, miR-877-3p, miR-1238-3p, miR-3130-5p, miR-4298, miR-4290, miR-3943, miR-346, and miR-767-3p.

13. The method according to claim 12, wherein the expression level(s) of the polynucleotide(s) is(are) measured by using nucleic acid(s) capable of specifically binding to the polynucleotide(s) or to complementary strand(s) of the polynucleotide(s), and
wherein the nucleic acid(s) is(are) polynucleotide(s) selected from the group consisting of the following polynucleotides (f) to (t):

(f) a polynucleotide consisting of a nucleotide sequence represented by any of SEQ ID NOs: 211 to 213, 215 to 249, 1351 to 1356, and 1449 to 1453 or a nucleotide sequence derived from the nucleotide sequence by the replacement of u with t, or a fragment thereof comprising 15 or more consecutive nucleotides,
(g) a polynucleotide comprising a nucleotide sequence represented by any of SEQ ID NOs: 211 to 213, 215 to 249, 1351 to 1356, and 1449 to 1453, or a nucleotide sequence derived from the nucleotide sequence by the replacement of u with t,
(h) a polynucleotide consisting of a nucleotide sequence complementary to a nucleotide sequence represented by any of SEQ ID NOs: 211 to 213, 215 to 249, 1351 to 1356, and 1449 to 1453 or a nucleotide sequence derived from the nucleotide sequence by the replacement of u with t, or a fragment thereof comprising 15 or more consecutive nucleotides,
(i) a polynucleotide comprising a nucleotide sequence complementary to a nucleotide sequence represented by any of SEQ ID NOs: 211 to 213, 215 to 249, 1351 to 1356, and 1449 to 1453 or a nucleotide sequence derived from the nucleotide sequence by the replacement of u with t,
(j) a polynucleotide hybridizing under stringent conditions to any of the polynucleotides (f) to (i),
(k) a polynucleotide consisting of a nucleotide sequence represented by any of SEQ ID NOs: 250 to 373 or a nucleotide sequence derived from the nucleotide sequence by the replacement of u with t, or a fragment thereof comprising 15 or more consecutive nucleotides,
(l) a polynucleotide comprising a nucleotide sequence represented by any of SEQ ID NOs: 250 to 373, or a nucleotide sequence derived from the nucleotide sequence by the replacement of u with t,
(m) a polynucleotide consisting of a nucleotide sequence complementary to a nucleotide sequence represented by any of SEQ ID NOs: 250 to 373 or a nucleotide sequence derived from the nucleotide sequence by the replacement of u with t, or a fragment thereof comprising 15 or more consecutive nucleotides,
(n) a polynucleotide comprising a nucleotide sequence complementary to a nucleotide sequence represented by any of SEQ ID NOs: 250 to 373 or a nucleotide sequence derived from the nucleotide sequence by the replacement of u with t,
(o) a polynucleotide hybridizing under stringent conditions to any of the polynucleotides (k) to (n),
(p) a polynucleotide consisting of a nucleotide sequence represented by any of SEQ ID NOs: 375 to 390 or a nucleotide sequence derived from the nucleotide sequence by the replacement of u with t, or a fragment thereof comprising 15 or more consecutive nucleotides,

(q) a polynucleotide comprising a nucleotide sequence represented by any of SEQ ID NOs: 375 to 390 or a nucleotide sequence derived from the nucleotide sequence by the replacement of u with t,

(r) a polynucleotide consisting of a nucleotide sequence complementary to a nucleotide sequence represented by any of SEQ ID NOs: 375 to 390 or a nucleotide sequence derived from the nucleotide sequence by the replacement of u with t, or a fragment thereof comprising 15 or more consecutive nucleotides,

(s) a polynucleotide comprising a nucleotide sequence complementary to a nucleotide sequence represented by any of SEQ ID NOs: 375 to 390 or a nucleotide sequence derived from the nucleotide sequence by the replacement of u with t, and

(t) a polynucleotide hybridizing under stringent conditions to any of the polynucleotides (p) to (s).

14. The method according to any one of claims 8 to 13, wherein the expression level(s) of the target gene(s) in the sample from the subject is(are) measured by use of the kit according to any one of claims 1 and 3 to 5 or the device according to any one of claims 2 to 7.

15. The method according to any one of claims 8 to 14, the sample is blood, serum, or plasma.

16. Use of dementia markers miR-4486, miR-6088 and miR-211-5p in an in vitro method of detection or prediction of dementia.

17. The use according to claim 16, wherein miR-4486, miR-6088 and miR-211-5p are used together with at least one or more polynucleotide(s) selected from the group consisting of dementia markers: miR-4274, miR-4272, miR-4443, miR-4506, miR-6773-5p, miR-4662a-5p, miR-3184-3p, miR-4281, miR-320d, miR-6729-3p, miR-5192, miR-6853-5p, miR-1234-3p, miR-1233-3p, miR-4539, miR-3914, miR-4738-5p, miR-548au-3p, miR-1539, miR-4720-3p, miR-365b-5p, miR-1227-5p, miR-4667-5p, miR-6820-5p, miR-4505, miR-548q, miR-4658, miR-450a-5p, miR-1260b, miR-3677-5p, miR-6777-3p, miR-6826-3p, miR-6832-3p, miR-4725-3p, miR-7161-3p, miR-2277-5p, miR-7110-3p, miR-4312, miR-4461, miR-6766-5p, miR-1266-3p, miR-6729-5p, miR-526b-3p, miR-519e-5p, miR-512-5p, miR-5088-5p, miR-1909-3p, miR-6511a-5p, miR-4734, miR-936, miR-1249-3p, miR-6777-5p, miR-4487, miR-3155a, miR-563, miR-4741, miR-6788-5p, miR-4433b-5p, miR-323a-5p, miR-6811-5p, miR-6721-5p, miR-5004-5p, miR-6509-3p, miR-648, miR-3917, miR-6087, miR-1470, miR-586, miR-3150a-5p, miR-105-3p, miR-7973, miR-1914-5p, miR-4749-3p, miR-15b-5p, miR-1289, miR-4433a-5p, miR-3666, miR-3186-3p, miR-4725-5p, miR-4488, miR-4474-3p, miR-6731-3p, miR-4640-3p, miR-202-5p, miR-6816-5p, miR-638, miR-6821-5p, miR-1247-3p, miR-6765-5p, miR-6800-5p, miR-3928-3p, miR-3940-5p, miR-3960, miR-6775-5p, miR-3178, miR-1202, miR-6790-5p, miR-4731-3p, miR-2681-3p, miR-6758-5p, miR-8072, miR-518d-3p, miR-3606-3p, miR-4800-5p, miR-1292-3p, miR-6784-3p, miR-4450, miR-6132, miR-4716-5p, miR-6860, miR-1268b, miR-378d, miR-4701-5p, miR-4329, miR-185-3p, miR-552-3p, miR-1273g-5p, miR-6769b-3p, miR-520a-3p, miR-4524b-5p, miR-4291, miR-6734-3p, miR-143-5p, miR-939-3p, miR-6889-3p, miR-6842-3p, miR-4511, miR-4318, miR-4653-5p, miR-6867-3p, miR-133b, miR-3196, miR-193b-3p, miR-3162-3p, miR-6819-3p, miR-1908-3p, miR-6786-5p, miR-3648, miR-4513, miR-3652, miR-4640-5p, miR-6871-5p, miR-7845-5p, miR-3138, miR-6884-5p, miR-4653-3p, miR-636, miR-4652-3p, miR-6823-5p, miR-4502, miR-7113-5p, miR-8087, miR-7154-3p, miR-5189-5p, miR-1253, miR-518c-5p, miR-7151-5p, miR-3614-3p, miR-4727-5p, miR-3682-5p, miR-5090, miR-337-3p, miR-488-5p, miR-100-5p, miR-4520-3p, miR-373-3p, miR-6499-5p, miR-3909, miR-32-5p, miR-302a-3p, miR-4686, miR-4659a-3p, miR-4287, miR-1301-5p, miR-593-3p, miR-517a-3p, miR-517b-3p, miR-142-3p, miR-1185-2-3p, miR-602, miR-527, miR-518a-5p, miR-4682, miR-28-5p, miR-4252, miR-452-5p, miR-525-5p, miR-3622a-3p, miR-6813-3p, miR-4769-3p, miR-5698, miR-1915-3p, miR-1343-5p, miR-6861-5p, miR-6781-5p, miR-4508, miR-6743-5p, miR-6726-5p, miR-4525, miR-4651, miR-6813-5p, miR-5787, miR-1290, miR-6075, miR-4758-5p, miR-4690-5p, miR-762, miR-371a-5p, miR-6765-3p, miR-6784-5p, miR-6778-5p, miR-6875-5p, miR-4534, miR-4721, miR-6756-5p, miR-615-5p, miR-6727-5p, miR-6887-5p, miR-8063, miR-6880-5p, miR-6805-3p, miR-4726-5p, miR-4710, miR-7111-5p, miR-3619-3p, miR-6795-5p, miR-1254, miR-1233-5p, miR-6836-3p, miR-6769a-5p, miR-4532, miR-365a-5p, miR-1231, miR-1228-5p, miR-4430, miR-296-3p, miR-1237-5p, miR-4466, miR-6789-5p, miR-4632-5p, miR-4745-5p, miR-4665-5p, miR-6807-5p, miR-7114-5p, miR-516a-5p, miR-769-3p, miR-3692-5p, miR-3945, miR-4433a-3p, miR-4485-3p, miR-6831-5p, miR-519c-5p, miR-551b-5p, miR-1343-3p, miR-4286, miR-4634, miR-4733-3p, and miR-6086, and

optionally wherein miR-4486, miR-6088 and miR-211-5p are used together with at least one or more polynucleotide(s) selected from the group consisting of other dementia markers: miR-1225-3p, miR-3184-5p, miR-665, miR-1247-5p, miR-3656, miR-149-5p, miR-744-5p, miR-345-5p, miR-150-5p, miR-191-3p, miR-651-5p, miR-34a-5p, miR-409-5p, miR-369-5p, miR-1915-5p, miR-204-5p, miR-137, miR-382-5p, miR-517-5p, miR-532-5p, miR-22-5p, miR-1237-3p, miR-1224-3p, miR-625-3p, miR-328-3p, miR-122-5p, miR-202-3p, miR-4781-5p, miR-718, miR-342-3p, miR-26b-3p, miR-140-3p, miR-200a-3p, miR-378a-3p, miR-484, miR-296-5p, miR-205-5p, miR-431-5p, miR-150-3p, miR-423-5p, miR-575, miR-671-5p, miR-939-5p, miR-3665, miR-30d-5p, miR-30b-3p, miR-92a-3p, miR-371b-5p, miR-486-5p, miR-1471, miR-1538, miR-449b-3p, miR-1976, miR-4268, miR-4279, miR-3620-3p, miR-3944-3p, miR-3156-3p, miR-3187-5p, miR-4685-3p, miR-4695-3p, miR-4697-3p, miR-4713-5p, miR-4723-3p, miR-371b-3p, miR-3151-3p, miR-3192-3p, miR-6728-3p, miR-6736-3p, miR-6740-3p, miR-6741-3p, miR-6743-3p, miR-6747-3p, miR-6750-3p, miR-6754-3p, miR-6759-3p, miR-6761-3p, miR-6762-3p, miR-6769a-3p, miR-6776-3p, miR-6778-3p, miR-6779-3p, miR-6786-3p, miR-6787-3p, miR-6792-3p, miR-6794-3p, miR-6801-3p, miR-6802-3p, miR-6803-3p, miR-6804-3p, miR-6810-5p, miR-6823-3p, miR-6825-3p, miR-6829-3p, miR-6833-3p, miR-6834-3p, miR-6780b-3p, miR-6845-3p, miR-6862-3p, miR-6865-3p, miR-6870-3p, miR-6875-3p, miR-6877-3p, miR-6879-3p, miR-6882-3p, miR-6885-3p, miR-6886-3p, miR-6887-3p, miR-6890-3p, miR-6893-3p, miR-6894-3p, miR-7106-3p, miR-7109-3p, miR-7114-3p, miR-7155-5p, miR-7160-5p, miR-615-3p, miR-920, miR-1825, miR-675-3p, miR-1910-5p, miR-2278, miR-2682-3p, miR-3122, miR-3151-5p, miR-3175, miR-4323, miR-4326, miR-4284, miR-3605-3p, miR-3622b-5p, miR-3646, miR-3158-5p, miR-4722-3p, miR-4728-3p, miR-4747-3p, miR-4436b-5p, miR-5196-3p, miR-5589-5p, miR-345-3p, miR-642b-5p, miR-6716-3p, miR-6511b-3p, miR-208a-5p, miR-6726-3p, miR-6744-5p, miR-6782-3p, miR-6789-3p, miR-6797-3p, miR-6800-3p, miR-6806-5p, miR-6824-3p, miR-6837-5p, miR-6846-3p, miR-6858-3p, miR-6859-3p, miR-6861-3p, miR-6880-3p, miR-7111-3p, miR-7152-5p, miR-642a-5p, miR-657, miR-1236-3p, miR-764, miR-4314, miR-3675-3p, miR-5703, miR-3191-5p, miR-6511a-3p, miR-6809-3p, miR-6815-5p, miR-6857-3p, miR-6878-3p, miR-766-3p, miR-1229-3p, miR-1306-5p, miR-210-5p, miR-198, miR-485-3p, miR-668-3p, miR-532-3p, miR-877-3p, miR-1238-3p, miR-3130-5p, miR-4298, miR-4290, miR-3943, miR-346, and miR-767-3p.

# Fig. 1

hsa-miR-4728-5p
(SEQ ID NO: 3)

hsa-mir-4728
(SEQ ID NO: 393)

hsa-miR-4728-3p
(SEQ ID NO: 335)

# Fig. 2

# Fig. 3

# Fig. 4

# Fig. 5

# Fig. 6

# Fig. 7

# Fig. 8

# Fig. 9

A

B

C

AUC: 0.911

D

AUC: 0.938

# Fig. 10

# Fig. 11

# Fig. 12

A

B

C

D

AUC: 0.886

AUC: 0.948

## Fig. 13

A

B

C

EP 4 697 021 A2

Fig. 14

hsa-miR-6765-5p

hsa-mir-6765
(SEQ ID NO: 490)

hsa-miR-6765-3p
(SEQ ID NO: 1315)

# Fig. 15

A

B

# Fig. 16

A

B

# Fig. 17

A

B

## Fig. 18

A

B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2017184642 A
- US 20130184331
- US 20140206777
- US 20140120545
- US 20170002348
- WO 2017084770 A
- WO 2017059094 A
- JP 2018023283 A
- JP 2018085652 A
- JP 2018138487 A
- US 20050261218
- US 20140303025
- US 20130040303
- US 20160273040
- WO 2017186719 A
- US 2010279292

### Non-patent literature cited in the description

- **SIRANJEEVI N. et al.** *Oncotarget*, 05 February 2017, vol. 8 (10), 16122-16143
- **ALIFIYA KAPASI et al.** *Acta Neuropathol*, 2017, vol. 134, 171-186
- **ZHENG ZHANG et al.** *J. Comput. Biol.*, 2000, vol. 7, 203-214
- **ALTSCHUL, S.F. et al.** *Journal of Molecular Biology*, 1990, vol. 215, 403-410
- **PEARSON, W.R. et al.** *Proc. Natl. Acad. Sci. U.S.A.*, 1988, vol. 85, 2444-2448
- **NIELSEN, P.E. et al.** *Science*, 1991, vol. 254, 1497-500
- **OBIKA, S. et al.** *Tetrahedron Lett.*, 1998, vol. 39, 5401-5404
- **GOFF LA et al.** *PLoS One*, 2009, vol. 4, e7192
- **PERSSON H et al.** *Cancer Res.*, 2011, vol. 71, 78-86
- **JIMA DD et al.** *Blood*, 2010, vol. 116, e118-e127
- **LADEWIG E et al.** *Genome Res.*, 2012, vol. 22, 1634-1645
- **STARK MS et al.** *PLoS One*, 2010, vol. 5, e9685
- **FRIEDLANDER MR et al.** *Nat. Biotechnol.*, 2008, vol. 26, 407-415
- **SCHOTTE D et al.** *Leukemia*, 2011, vol. 25, 1389-1399
- **LADEWIG E et al.** *Genome Res*, 2012, vol. 22, 1634-1645
- **BEREZIKOV E et al.** *Mol. Cell*, 2007, vol. 28, 328-336
- **CREIGHTON CJ et al.** *PLoS One*, 2010, vol. 5, e9637
- **FRIEDLANDER MR et al.** *Nucleic Acids Res.*, 2012, vol. 40, 37-52

- **AZUMA-MUKAI, A et al.** *Proc. Natl. Acad. Sci. U.S.A.*, 2008, vol. 105, 7964-7969
- **XIE X et al.** *Nature*, 2005, vol. 434, 338-345
- **YOO JK et al.** *Stem Cells Dev.*, 2012, vol. 21, 2049-2057
- **WYMAN SK et al.** *PLoS One*, 2009, vol. 4, e5311
- **VAZ C et al.** *BMC Genomics*, 2010, vol. 11, 288
- **MEUNIER J et al.** *Genome Res.*, 2013, vol. 23, 34-45
- **NYGAARD S et al.** *BMC Med. Genomics*, 2009, vol. 2, 35
- **MORIN RD et al.** *Genome Res*, 2008, vol. 18, 610-621
- **BENTWICH I et al.** *Nat. Genet.*, 2005, vol. 37, 766-770
- **BENTWICH I et al.** *Nat. Genet*, 2005, vol. 37, 766-770
- **DING N et al.** *J. Radiat. Res.*, 2011, vol. 52, 425-432
- **BAR M et al.** *Stem Cells*, 2008, vol. 26, 2496-2505
- **JOYCE CE et al.** *Hum. Mol. Genet*, 2011, vol. 20, 4025-4040
- **LUI WO et al.** *Cancer Res.*, 2007, vol. 67, 6031-6043
- **CUMMINS JM et al.** *Proc. Natl. Acad. Sci. U.S.A.*, 2006, vol. 103, 3687-3692
- **PLE H et al.** *PLoS One*, 2012, vol. 7, e50746
- **KIM J et al.** *Proc. Natl. Acad. Sci. U.S.A.*, 2004, vol. 101, 360-365
- **LI Y et al.** *Gene*, 2012, vol. 497, 330-335
- **HANSEN TB et al.** *RNA Biol.*, 2011, vol. 8, 378-383
- **YOO JK et al.** *Stem Cells Dev*, 2012, vol. 21, 2049-2057
- **KAWAJI H et al.** *BMC Genomics*, 2008, vol. 9, 157
- **MOURELATOS Z et al.** *Genes Dev*, 2002, vol. 16, 720-728
- **VELTHUT-MEIKAS A et al.** *Mol. Endocrinol*, 2013, vol. 27, 1128-1141
- **LAGOS-QUINTANA M et al.** *Curr. Biol.*, 2002, vol. 12, 735-739
- **LIAO JY et al.** *PLoS One*, 2010, vol. 5, e10563
- **HU R et al.** *J. Biol. Chem.*, 2011, vol. 286, 12328-12339
- **MARTON S et al.** *Leukemia*, 2008, vol. 22, 330-338
- **WANG HJ et al.** *Shock*, 2013, vol. 39, 480-487
- **WITTEN D et al.** *BMC Biol.*, 2010, vol. 8, 58
- **DANNEMANN M et al.** *Genome Biol. Evol.*, 2012, vol. 4, 552-564
- **BEREZIKOV E et al.** *Genome Res.*, 2006, vol. 16, 1289-1298
- **LAGOS-QUINTANA M et al.** *RNA*, 2003, vol. 9, 175-179
- **RESHMI G et al.** *Genomics*, 2011, vol. 97, 333-340
- **TANDON M et al.** *Oral Dis*, 2012, vol. 18, 127-131
- **LIM LP et al.** *Science*, 2003, vol. 299, 1540
- **MEIRI E et al.** *Nucleic Acids Res.*, 2010, vol. 38, 6234-6246
- **MEUNIER J et al.** *Genome Res*, 2013, vol. 23, 34-45
- **MORIN RD et al.** *Genome Res.*, 2008, vol. 18, 610-621
- **WITTEN D et al.** *BMC Biol*, 2010, vol. 8, 58
- **MOURELATOS Z et al.** *Genes Dev.*, 2002, vol. 16, 720-728
- **SUH MR et al.** *Dev. Biol.*, 2004, vol. 270, 488-498
- **LAGOS-QUINTANA M et al.** *Science*, 2001, vol. 294, 853-858
- **HOUBAVIY HB et al.** *Dev. Cell*, 2003, vol. 5, 351-358
- **LAGOS-QUINTANA M et al.** *Curr. Biol*, 2002, vol. 12, 735-739
- **ALTUVIA Y et al.** *Nucleic Acids Res.*, 2005, vol. 33, 2697-2706
- **WATAHIKI A et al.** *PLoS One*, 2011, vol. 6, e24950
- **YOO H et al.** *Biochem. Biophys. Res. Commun.*, 2011, vol. 415, 567-572
- **VOELLENKLE C et al.** *RNA*, 2012, vol. 18, 472-484
- **BEREZIKOV E et al.** *Genome Res*, 2006, vol. 16, 1289-1298
- **KIM J et al.** *Proc. Natl. Acad. Sci.U.S.A.*, 2004, vol. 101, 360-365
- **CUMMINS JM et al.** *Proc. Natl. Acad. Sci., U.S.A.*, 2006, vol. 103, 3687-3692
- **DOSTIE J et al.** *RNA*, 2003, vol. 9, 180-186
- **KIM J et al.** *Proc. Natl. Acad. Sci., U.S.A.*, 2004, vol. 101, 360-365
- **ARTZI S et al.** *BMC Bioinformatics*, 2008, vol. 9, 39
- **KASASHIMA K et al.** *Biochem. Biophys. Res. Commun*, 2004, vol. 322, 403-410

- **FU H et al.** *FEBS Lett.*, 2005, vol. 579, 3849-3854
- **HOUBAVIY HB et al.** *Dev. Cell.*, 2003, vol. 5, 351-358
- **AZUMA, MUKAI, A et al.** *Proc. Natl. Acad. Sci. U.S.A.*, 2008, vol. 105, 7964-7969
- **SCHOTTE D et al.** *Leukemia*, 2009, vol. 23, 313-322
- **WITTEN D et al.** *BMC Bio*, 2010, vol. 8, 58
- **NOVOTNY GW et al.** *Int. J. Androl.*, 2007, vol. 30, 316-326
- **CAI X et al.** *RNA*, 2007, vol. 13, 313-316
- **KASASHIMA K et al.** *Biochem. Biophys. Res. Commun.*, 2004, vol. 322, 403-410
- **MORIN RD. et al.** *Genome Res*, 2008, vol. 18, 610-621
- **SATOH J. et al.** *Biomark Insights.*, 2015, vol. 10, 21-23
- **SCHIPPER HM et al.** *Gene Regulation and Systems Biology*, 2007, vol. 1, 263-74
- **AUSUBEL et al.** Current Protocols in Molecular Biology. John Willey & Sons, 1993
- **SAMBROOK et al.** Molecular Cloning A Laboratory Manua. Cold Spring Harbor Laboratory Press, 1989
- Molecular Cloning. **SAMBROOK, J.** ; **RUSSEL, D**. A LABORATORY MANUAL. Cold Spring Harbor Laboratory Press, 15 January 2001, vol. 1,2, 7.42-7.45, 8.9-8.17
- **RICK KAMPS et al.** *Int. J. Mol. Sci.*, 2017, vol. 18 (2), 308
- *Int. Neurourol. J.*, 2016, vol. 20 (2), 76-83
- **VENABLES, W. N et al.** Modern Applied Statistics. Springer, 2002
- **TAKAFUMI KANAMORI et al.** Pattern Recognition. KYORITSU SHUPPAN CO., LTD., 2009
- **RICHARD O. et al.** Pattern Classification. Wiley-Interscience, 2000
- **V. VAPNIK**. The Nature of Statistical Leaning Theory. Springer, 1995
- Statistics of pattern recognition and learning - New concepts and approaches. **HIDEKI ASO et al.** Frontier of Statistical Science 6. Publishers, 2004
- **NELLO CRISTIANINI et al.** Introduction to SVM. Kyoritsu Shuppan Co., Ltd, 2008
- **C. CORTES et al.** *Machine Learning*, 1995, vol. 20, 273-297
- **TIBSHIRANI R**. *J R Stat Soc Ser B*, 1996, vol. 58, 267-88
- **HOERL, A.E.** *Technometrics*, 1970, vol. 12, 55-67
- **ZOU, H.** *J R Stat Soc Ser B*, 2005, vol. 67, 301-320
- **PEARSON, K**. *Philosophical Magazine*, 1901, vol. 2 (11), 559-572
- **HOTELLING, H.** *Journal of Educational Psychology*, 1933, vol. 24, 417-441, 498-520
- **YASUSHI NAGATA et al.** Basics of statistical multiple comparison methods. Scientist Press Co., Ltd., 2007
- **FUREY TS. et al.** *Bioinformatics*, 2000, vol. 16, 906-14
- **R CORE TEAM**. R: A language and environment for statistical computing. R Foundation for Statistical Computing, 2016
- **VENABLES, W.N.** ; **RIPLEY, B.D.** Modern Applied Statistics. Springer, 2002